# EUROPEAN PATENT APPLICATION

(11) **EP 4 685 161 A2**
(43) Date of publication of application: **28.01.2026**
(21) Application number: 25205599.1
(22) Date of filing: 25.10.2019
(51) Int. Cl.: C07K 16/32

(54) **ONCOLYTIC VIROTHERAPY AND IMMUNOTHERAPY**

(30) Priority: 25.10.2018 US 201862750402 P
(62) Divisional of application: 23186335.8
(71) Applicant: Baylor College of Medicine, Houston, TX 77030 (US)
(72) Inventor: SUZUKI, Masataka, Houston, 77030 (US)
(74) Representative: J A Kemp LLP

(57) **Abstract**

Methods of treating a cancer, comprising administering to a subject: (i) a virus comprising nucleic acid encoding an antigen-binding molecule comprising:(a) an antigen-binding moiety specific for an immune cell surface molecule, and (b) an antigen-binding moiety specific for a cancer cell antigen; and (ii) an oncolytic virus, and/or (iii) at least one cell comprising a chimeric antigen receptor (CAR) specific for a cancer cell antigen are disclosed. Also disclosed are articles and compositions for use in such methods.

## Description

This application claims priority from US 62/750,402 filed 25 October 2018, the contents and elements of which are herein incorporated by reference for all purposes.

### Technical Field

The present disclosure relates at least to the fields of cell biology, molecular biology, immunology, virology, and medicine, including cancer therapy. In particular embodiments the disclosure relates to combination treatments involving the use of oncolytic virotherapy and immunotherapy.

### Background

### Oncolytic virotherapy for squamous cell carcinoma of the head and neck (HNSCC)

HNSCC is the sixth leading cancer by incidence worldwide. Treatment of locally advanced, recurrent and metastatic HNSCC is often limited by an unfavorable efficacy to toxicity ratio and median survival for patients with metastatic disease remains less than one year (Zandberg and Strome, Oral Oncology (2014) 50: 627-632). Since HNSCC is a locoregional disease that presents at or close to the surface of the body, it is amenable to initial intratumoral injection of adenoviral vectors (Ads) to prompt a locoregional and even a systemic anti-tumor immune response (Liu et al., Nature Clinical Practice Oncology (2007) 4: 101-117). Several clinical trials of conditionally-replicating Ads (OncAds) or replication-deficient Ads encoding a therapeutic transgene have demonstrated the safety and feasibility of Ad gene therapy for HNSCC, but failed to show improved overall survival since intensive local treatment, even when combined with chemo/radiotherapy, did not prevent metastasis to distant sites (Liu *et al., supra*). OncAds are generally administered intratumorally, and poorly re-target to metastasized tumors (Koksi et al., Molecular Therapy: The Journal of the American Society of Gene Therapy (2015) 23:1641-1652).

### OncAd with helper-dependent Ad (HDAd) expressing immunomodulatory molecules

Adenoviral-based vectors (Ads) can infect a range of malignant cells and express high levels of lytic antigens and immunogenic transgenes, making them attractive as agents for cancer gene therapy (Cerullo et al., Advances in Cancer Research (2012) 115, 265-318). OncAds selectively replicate in cancer cells and are commonly used Ad-based vectors in clinical trials for cancer gene therapy. However, OncAds have a limited coding capacity for transgenes (~1.5 kb). Helper-dependent Ads (HDAds) are devoid of viral coding sequences, enabling a cargo capacity of up to 34 kb for insertion of multiple transgenes in a single vector (Suzuki et al., Human Gene Therapy (2010) 21; 120-126). Since HDAd vector DNA encodes packaging signals, the OncAd replication machinery acts in *trans* to replicate and package both OncAd and HDAd within infected tumor cells, leading to multiple cycles of production and release of both the oncolytic virus and the transgenes encoded by the HDAd (combinatorial adenoviral vectors: CAd-VEC; Farzad et al., Molecular Therapy - Oncolytics (2014) 1, 14008).

### CAR T-cell therapy

The use of T-cells as agents for cancer therapy has recently been facilitated by the expression of cancer cell antigen-directed chimeric antigen receptors (CARs; reviewed in Kershaw et al., Nature (2013) 13: 525-541). CAR-modified T-cells have shown promise for the treatment of hematological malignancies (Garfall et al., The New England Journal of Medicine (2015) 373:1040-1047), but have been less effective in treating solid tumors, which may in part be a consequence of the highly immunosuppressive nature of the solid tumor microenvironment (Quail et al., Nature Medicine (2013) 19:1423-1437). Due to immunosuppressive mechanisms at tumor site CAR T-cells fail to expand and persist long term despite the expression of one or two costimulatory endodomains.

### Bispecific T cell Engagers (BiTEs)

Bispecific T cell engagers are a class of antigen-binding molecule which are useful to enhance a subject's immune response to cells expressing given target antigen. BiTEs comprise an antigen-binding moiety specific for a target antigen connected via a linker to an immune cell surface protein (typically CD3). The BiTEs promote effector immune cell activity directed against cells expressing the target antigen by physically linking immune cells (T cells) to cells expressing the target antigen, thereby stimulating T cell activation, cytokine production and killing of the cell expressing the target antigen. BiTEs that target cancer cell antigens are reviewed e.g. in Huhels et al., Immunol Cell Biol. (2015) 93(3): 290-296.

The present disclosure provides a solution to a long-felt need for effective cancer therapies, including combinatorial cancer therapies.

### Brief Summary

In a first aspect, the present disclosure provides a combination of:
(i) an antigen-binding molecule comprising: (a) an antigen-binding moiety specific for an immune cell surface molecule, and (b) an antigen-binding moiety specific for a cancer cell antigen, or nucleic acid encoding said antigen-binding molecule; and
(ii) an oncolytic virus.

Also provided is a combination of:
(i) an antigen-binding molecule comprising: (a) an antigen-binding moiety specific for an immune cell surface molecule, and (b) an antigen-binding moiety specific for a cancer cell antigen, or nucleic acid encoding said antigen-binding molecule; and
(ii) at least one cell comprising a chimeric antigen receptor (CAR) specific for a cancer cell antigen.

Also provided is a combination of:
(i) an antigen-binding molecule comprising: (a) an antigen-binding moiety specific for an immune cell surface molecule, and (b) an antigen-binding moiety specific for a cancer cell antigen, or nucleic acid encoding said antigen-binding molecule;
(ii) an oncolytic virus; and
(iii) at least one cell comprising a chimeric antigen receptor (CAR) specific for a cancer cell antigen.

The present disclosure also provides a method of treating a cancer, comprising administering to a subject:
(i) an antigen-binding molecule comprising: (a) an antigen-binding moiety specific for an immune cell surface molecule, and (b) an antigen-binding moiety specific for a cancer cell antigen, or nucleic acid encoding said antigen-binding molecule; and
(ii) an oncolytic virus, and/or (iii) at least one cell comprising a chimeric antigen receptor (CAR) specific for a cancer cell antigen.

In a related aspect, the present disclosure provides a combination of:
(i) an antigen-binding molecule comprising: (a) an antigen-binding moiety specific for an immune cell surface molecule, and (b) an antigen-binding moiety specific for a cancer cell antigen, or nucleic acid encoding said antigen-binding molecule; and
(ii) an oncolytic virus, and/or (iii) at least one cell comprising a chimeric antigen receptor (CAR) specific for a cancer cell antigen, for use in a method of treating a cancer.

Also provided is the use of:
(i) an antigen-binding molecule comprising: (a) an antigen-binding moiety specific for an immune cell surface molecule, and (b) an antigen-binding moiety specific for a cancer cell antigen, or nucleic acid encoding said antigen-binding molecule; and
(ii) an oncolytic virus, and/or (iii) at least one cell comprising a chimeric antigen receptor (CAR) specific for a cancer cell antigen, in the manufacture of a medicament for use in a method of treating a cancer.

In some embodiments the nucleic acid encoding the antigen-binding molecule is comprised within a virus. In some embodiments the virus encoding the antigen-binding molecule is a helper-dependent adenovirus (HDAd).

Also provided is a method of treating a cancer, comprising administering to a subject:
(i) a virus comprising nucleic acid encoding an antigen-binding molecule comprising: (a) an antigen-binding moiety specific for an immune cell surface molecule, and (b) an antigen-binding moiety specific for a cancer cell antigen; and
(ii) an oncolytic virus, and/or (iii) at least one cell comprising a chimeric antigen receptor (CAR) specific for a cancer cell antigen.

Also provided is a combination of:
(i) a virus comprising nucleic acid encoding an antigen-binding molecule comprising: (a) an antigen-binding moiety specific for an immune cell surface molecule, and (b) an antigen-binding moiety specific for a cancer cell antigen; and
(ii) an oncolytic virus, and/or (iii) at least one cell comprising a chimeric antigen receptor (CAR) specific for a cancer cell antigen, for use in a method of treating a cancer.

Also provided is the use of:
(i) a virus comprising nucleic acid encoding an antigen-binding molecule comprising: (a) an antigen-binding moiety specific for an immune cell surface molecule, and (b) an antigen-binding moiety specific for a cancer cell antigen; and
(ii) an oncolytic virus, and/or (iii) at least one cell comprising a chimeric antigen receptor (CAR) specific for a cancer cell antigen, in the manufacture of a medicament for use in a method of treating a cancer.

In some embodiments in accordance with various aspects of the present disclosure the CAR and the antigen-binding moiety capable of binding to a cancer cell antigen are specific for non-identical cancer cell antigens. In some embodiments the antigen-binding molecule comprises (a) a heavy chain variable region (VH) and a light chain variable region (VL) specific for an immune cell surface molecule associated via a linker sequence to (b) VH and a VL specific for a cancer cell antigen. In some embodiments the immune cell surface molecule is a CD3-TCR complex polypeptide. In some embodiments the cancer cell antigen is selected from CD44v6, HER2, CD19, PSCA, p53, CEA, GP100, EGFR, hTERT, NY-ESO1, MAGE-A3, mesothelin and MUC-1.

In some embodiments the virus comprising nucleic acid encoding an antigen-binding molecule additionally comprises nucleic acid encoding an immunomodulatory factor which is an agonist of an effector immune response or an antagonist of an immunoregulatory response. In some embodiments the virus comprising nucleic acid encoding an antigen-binding molecule additionally comprises nucleic acid encoding IL-12 and/or an antagonist anti-PD-L1 antibody. In some embodiments the virus comprising nucleic acid encoding an antigen-binding molecule is a helper-dependent adenovirus (HDAd). In some embodiments the virus comprising nucleic acid encoding an antigen-binding molecule comprises nucleic acid encoding an enzyme capable of catalysing conversion of a non-toxic factor to a cytotoxic form. In some embodiments the enzyme is selected from: thymidine kinase, cytosine deaminase, nitroreductase, cytochrome P450, carboxypeptidase G2, purine nucleoside phosphorylase, horseradish peroxidase and carboxylesterase.

In some embodiments the cell comprising a CAR is specific for the oncolytic virus. In some embodiments the cell comprising a CAR is a T cell. In some embodiments the oncolytic virus is an oncolytic adenovirus (OncAd). In some embodiments the oncolytic virus is derived from adenovirus 5 (Ad5). In some embodiments the oncolytic virus encodes an E1A protein which displays reduced binding to Rb protein as compared to E1A protein encoded by Ad5. In some embodiments the oncolytic virus encodes an E1A protein lacking the amino acid sequence LTCHEACF (SEQ ID NO:105). In some embodiments the oncolytic virus encodes an E1A protein comprising, or consisting of, the amino acid sequence SEQ ID NO:104.

In some embodiments the oncolytic virus comprises nucleic acid having one or more binding sites for one or more transcription factors. In some embodiments the oncolytic virus comprises nucleic acid having one or more binding sites for STAT1.

In some embodiments the method of treating a cancer comprises:
(a) isolating at least one cell from a subject;
(b) modifying the at least one cell to express or comprise a CAR specific for a cancer cell antigen, or a nucleic acid encoding a CAR specific for a cancer cell antigen,
(c) optionally expanding the modified at least one cell, and;
(d) administering the modified at least one cell to a subject.

In some embodiments the method of treating a cancer comprises:
(a) isolating immune cells from a subject;
(b) generating or expanding a population of immune cells specific for an oncolytic virus by a method comprising: stimulating the immune cells by culture in the presence of antigen presenting cells (APCs) presenting a peptide of the oncolytic virus, and;
(c) administering at least one immune cell specific for the oncolytic virus to a subject.

In some embodiments the cancer is selected from head and neck cancer, head and neck squamous cell carcinoma (HNSCC), nasopharyngeal carcinoma (NPC), oropharyngeal carcinoma (OPC), prostate carcinoma, pancreatic carcinoma, cervical carcinoma (CC), gastric carcinoma (GC), hepatocellular carcinoma (HCC) and lung cancer.

Also provided is a helper-dependent adenovirus (HDAd) comprising nucleic acid encoding an antigen-binding molecule comprising: (a) an antigen-binding moiety specific for an immune cell surface molecule, and (b) an antigen-binding moiety specific for a cancer cell antigen.

Also provided is a combination, comprising:
(i) a helper-dependent adenovirus (HDAd) comprising nucleic acid encoding an antigen-binding molecule comprising: (a) an antigen-binding moiety specific for an immune cell surface molecule, and (b) an antigen-binding moiety specific for a cancer cell antigen; and
(ii) an oncolytic virus, and/or (iii) at least one cell comprising a chimeric antigen receptor (CAR) specific for a cancer cell antigen.

In some embodiments the antigen-binding molecule comprises (a) a single-chain variable fragment (scFv) specific for an immune cell surface molecule associated via a linker to (b) a scFv specific for a cancer cell antigen. In some embodiments the immune cell surface molecule is a CD3-TCR complex polypeptide. In some embodiments the cancer cell antigen is selected from CD44v6, CD19, HER2, PSCA, p53, CEA, GP100, EGFR, hTERT, NY-ESO1, MAGE-A3, mesothelin and MUC-1. In some embodiments the HdAd additionally comprises nucleic acid encoding an immunomodulatory factor which is an agonist of an effector immune response or an antagonist of an immunoregulatory response. In some embodiments the HdAd additionally comprises nucleic acid encoding IL-12 and/or an antagonist anti-PD-L1 antibody. In some embodiments the HdAd additionally comprises nucleic acid encoding an enzyme capable of catalysing conversion of a non-toxic factor to a cytotoxic form. In some embodiments the enzyme is selected from: thymidine kinase, cytosine deaminase, nitroreductase, cytochrome P450, carboxypeptidase G2, purine nucleoside phosphorylase, horseradish peroxidase and carboxylesterase.

Also provided is an antigen-binding molecule, optionally isolated or man-made, comprising: (a) an antigen-binding moiety specific for an immune cell surface molecule, and (b) an antigen-binding moiety specific for a cancer cell antigen. In some embodiments the antigen-binding molecule comprises (a) a heavy chain variable region (VH) and a light chain variable region (VL) specific for an immune cell surface molecule associated via a linker sequence to (b) VH and a VL specific for a cancer cell antigen. In some embodiments the immune cell surface molecule is a CD3-TCR complex polypeptide. In some embodiments the cancer cell antigen is selected from CD44v6, HER2, CD19, PSCA, p53, CEA, GP100, EGFR, hTERT, NY-ESO1, MAGE-A3, mesothelin and MUC-1.

Also provided is a nucleic acid, or a plurality of nucleic acids, optionally isolated or man-made, encoding the helper-dependent adenovirus (HDAd), the components of the combination, or the antigen-binding molecule according to the present disclosure.

Also provided is a nucleic acid, or a plurality of nucleic acids, optionally isolated or man-made, encoding: (i) an antigen-binding molecule comprising: (a) an antigen-binding moiety specific for an immune cell surface molecule, and (b) an antigen-binding moiety specific for a cancer cell antigen, (ii) IL-12, and/or an antagonist anti-PD-L1 antibody.

Also provided is a cell comprising the helper-dependent adenovirus (HDAd), the components of the combination, the antigen-binding molecule, or the nucleic acid or plurality of nucleic acids according to the present disclosure.

Also provided is a pharmaceutical composition comprising the helper-dependent adenovirus (HDAd), the components of the combination, the antigen-binding molecule, the nucleic acid or plurality of nucleic acids, or the cell according to the present disclosure and a pharmaceutically acceptable carrier, diluent, excipient or adjuvant.

Also provided is a method of treating cancer comprising administering to a subject the helper-dependent adenovirus (HDAd), the combination, the antigen-binding molecule, the nucleic acid or plurality of nucleic acids, the cell, or the pharmaceutical composition according to the present disclosure.

Also provided is the helper-dependent adenovirus (HDAd), the combination, the antigen-binding molecule, the nucleic acid or plurality of nucleic acids, the cell, or the pharmaceutical composition according to the present disclosure, for use in a method of treating a cancer.

Also provided is the use of the helper-dependent adenovirus (HDAd), the combination, the antigen-binding molecule, the nucleic acid or plurality of nucleic acids, the cell, or the pharmaceutical composition according to the present disclosure, in the manufacture of a medicament for treating a cancer.

In some embodiments in accordance with various aspects of the present disclosure the cancer is selected from head and neck cancer, head and neck squamous cell carcinoma (HNSCC), nasopharyngeal carcinoma (NPC), prostate carcinoma, pancreatic carcinoma, cervical carcinoma (CC), oropharyngeal carcinoma (OPC), gastric carcinoma (GC), hepatocellular carcinoma (HCC) and lung cancer.

Also provided is a kit of parts comprising a predetermined quantity of the helper-dependent adenovirus (HDAd), the components of the combination, the antigen-binding molecule, the nucleic acid or plurality of nucleic acids, the cell, or the pharmaceutical composition according to the present disclosure.

### Detailed Description

The present disclosure is concerned with the combined use of multiple therapeutic agents for the treatment of cancer. The therapeutic agents are combined to provide an improved treatment effect as compared to the effect seen when any one of the agents is used alone. In certain embodiments, the agents act in an additive or synergistic manner to treat the cancer.

### Antigen-binding molecule

Aspects of the present disclosure employ an antigen-binding molecule comprising: (a) an antigen-binding moiety specific for an immune cell surface molecule, and (b) an antigen-binding moiety specific for a cancer cell antigen, or nucleic acid encoding said antigen-binding molecule. In some aspects, the present disclosure employs a virus comprising nucleic acid encoding an antigen-binding molecule comprising: (a) an antigen-binding moiety specific for an immune cell surface molecule, and (b) an antigen-binding moiety specific for a cancer cell antigen.

An "antigen-binding molecule" refers to a molecule which is capable of binding to a target antigen, and encompasses antibodies and antibody fragments (e.g. Fv, scFv, Fab, scFab, F(ab')₂, Fab₂, diabodies, triabodies, scFv-Fc, minibodies, single domain antibodies (e.g. VhH), etc.), as long as they display binding to the relevant target molecule(s).

The antigen-binding molecule of the present disclosure comprises antigen-binding moieties specific for particular target antigen(s). In some embodiments, an antigen-binding moiety comprises an antibody heavy chain variable region (VH) and an antibody light chain variable region (VL) of an antibody capable of specific binding to the target antigen. In some embodiments, the antigen-binding moiety comprises or consists of an aptamer capable of binding to the target antigen, e.g. a nucleic acid aptamer (reviewed, for example, in Zhou and Rossi Nat Rev Drug Discov. 2017 16(3):181-202). In some embodiments, the antigen-binding moiety comprises or consists of a antigen-binding peptide/polypeptide, e.g. a peptide aptamer, thioredoxin, monobody, anticalin, Kunitz domain, avimer, knottin, fynomer, atrimer, DARPin, affibody, nanobody (i.e. a single-domain antibody (sdAb)) affilin, armadillo repeat protein (ArmRP), OBody or fibronectin - reviewed e.g. in Reverdatto et al., Curr Top Med Chem. 2015; 15(12): 1082-1101, which is hereby incorporated by reference in its entirety (see also e.g. Boersma et al., J Biol Chem (2011) 286:41273-85 and Emanuel et al., Mabs (2011) 3:38-48).

An antigen-binding molecule may be, or may comprise, an antigen-binding polypeptide, or an antigen-binding polypeptide complex. An antigen-binding molecule may comprise more than one polypeptide which together form the antigen-binding molecule. The polypeptides may associate covalently or non-covalently. In some embodiments the polypeptides form part of a larger polypeptide comprising the polypeptides (e.g. in the case of scFv comprising VH and VL, or in the case of scFab comprising VH-CH1 and VL-CL).

The antigen-binding moieties of the present disclosure may be designed and prepared using the sequences of monoclonal antibodies (mAbs) capable of binding to the relevant target antigens. Antigen-binding regions of antibodies, such as single chain variable fragment (scFv), Fab and F(ab')₂ fragments may also be used/provided. An "antigen-binding region" is any fragment of an antibody which is capable of binding to the target for which the given antibody is specific. An antigen-binding moiety according to the present disclosure may comprise or consist of the antigen-binding region of an antibody specific for a given target.

Antibodies generally comprise six complementarity-determining regions CDRs; three in the heavy chain variable (VH) region: HC-CDR1, HC-CDR2 and HC-CDR3, and three in the light chain variable (VL) region: LC-CDR1, LC-CDR2, and LC-CDR3. The six CDRs together define the paratope of the antibody, which is the part of the antibody which binds to the target antigen.

The VH region and VL region comprise framework regions (FRs) either side of each CDR, which provide a scaffold for the CDRs. From N-terminus to C-terminus, VH regions comprise the following structure: N term-[HC-FR1]-[HC-CDR1]-[HC-FR2]-[HC-CDR2]-[HC-FR3]-[HC-CDR3]-[HC-FR4]-C term; and VL regions comprise the following structure: N term-[LC-FR1]-[LC-CDR1]-[LC-FR2]-[LC-CDR2]-[LC-FR3]-[LC-CDR3]-[LC-FR4]-C term.

There are several different conventions for defining antibody CDRs and FRs, such as those described in Kabat et al., Sequences of Proteins of Immunological Interest, 5th Ed. Public Health Service, National Institutes of Health, Bethesda, MD (1991), Chothia et al., J. Mol. Biol. 196:901-917 (1987), and VBASE2, as described in Retter et al., Nucl. Acids Res. (2005) 33 (suppl 1): D671-D674. The CDRs and FRs of the VH regions and VL regions of the antibody clones described herein were defined according to the international IMGT (ImMunoGeneTics) information system (LeFranc et al., Nucleic Acids Res. (2015) 43 (Database issue):D413-22), which uses the IMGT V-DOMAIN numbering rules as described in Lefranc et al., Dev. Comp. Immunol. (2003) 27:55-77.

The antigen-binding moieties of the present disclosure generally comprise the VH and VL of an antibody capable of specific binding to the target antigen. An antigen-binding moiety formed by the combination of a VH and a VL may be referred to as an Fv.

In some embodiments the VH and VL of an Fv are provided on the same polypeptide chain, and are joined by a linker sequence. That is, in some embodiment the antigen-binding moiety comprises or consists of single chain Fv (scFv) specific capable of specific binding to the target antigen.

The antigen-binding molecule of the present disclosure is multispecific. That is, it displays specific binding to more than one target antigen. In some embodiments the antigen-binding molecule is bispecific. In some embodiments the antigen-binding molecule of the present disclosure comprises at least two, non-identical antigen-binding moieties.

The antigen-binding molecule of the present disclosure comprises (a) an antigen-binding moiety specific for an immune cell surface molecule, and (b) an antigen-binding moiety specific for a cancer cell antigen. In some embodiments the antigen-binding molecule comprises (a) an scFv specific for an immune cell surface molecule, and (b) an scFv specific for a cancer cell antigen.

In some embodiments the antigen-binding moieties of the antigen-binding molecule are joined by a linker sequence.

Linker sequences are known to the skilled person, and are described, for example in Chen et al., Adv Drug Deliv Rev (2013) 65(10): 1357-1369, which is hereby incorporated by reference in its entirety. In some embodiments, a linker sequence may be a flexible linker sequence. Flexible linker sequences allow for relative movement of the amino acid sequences which are linked by the linker sequence. Flexible linkers are known to the skilled person, and several are identified in Chen et al., Adv Drug Deliv Rev (2013) 65(10): 1357-1369. Flexible linker sequences often comprise high proportions of glycine and/or serine residues. In some embodiments, a linker sequence comprises at least one glycine residue and/or at least one serine residue. In some embodiments a linker sequence consists of glycine and serine residues. In some embodiments the linker sequence comprises, or consists, of the amino acid sequence shown in one of SEQ ID NOs:109 to 112.

In some embodiments, the antigen-binding moiety specific for an immune cell surface molecule comprises the CDRs of an antigen-binding molecule which is capable of binding to the immune cell surface molecule. In some embodiments the antigen-binding moiety specific for an immune cell surface molecule comprises the VH region and the VL region of an antigen-binding molecule which is capable of binding to the immune cell surface molecule. In some embodiments the antigen-binding moiety specific for an immune cell surface molecule comprises scFv capable of binding to the immune cell surface molecule.

In some embodiments, the antigen-binding moiety specific for a cancer cell antigen comprises the CDRs of an antigen-binding molecule which is capable of binding to the cancer cell antigen. In some embodiments the antigen-binding moiety specific for a cancer cell antigen comprises the VH region and the VL region of an antigen-binding molecule which is capable of binding to the cancer cell antigen. In some embodiments the antigen-binding moiety specific for a cancer cell antigen comprises scFv capable of binding to the cancer cell antigen.

It will be appreciated that the antigen-binding molecule is a multispecific antigen-binding molecule. Multispecific (e.g. bispecific) antigen-binding molecules may be provided in any suitable format, such as those formats described in described in Brinkmann and Kontermann MAbs (2017) 9(2): 182-212, which is hereby incorporated by reference in its entirety.

The antigen-binding molecule of the present disclosure may a bispecific T cell engager (BiTE). The structure and function of BiTEs is reviewed e.g. in Huhels et al., Immunol Cell Biol. (2015) 93(3): 290-296, which is hereby incorporated by reference in its entirety. Typically, a BiTE molecule comprises an scFv specific for a target antigen joined by a linker sequence to an scFv specific a CD3 polypeptide. The BiTE potentiates T cell activity directed against cells expressing the target protein.

In some embodiments the antigen-binding molecule comprises or consists of a tandem scFv, a diabody, a Fab₂ or a Triomab. In some embodiments the antigen-binding molecule comprises or consists of a tandem scFv.

### Immune cell surface molecules

An immune cell surface molecule may be any peptide/polypeptide, glycoprotein, lipoprotein, glycan, glycolipid, lipid, or fragment thereof expressed at or on the cell surface of an immune cell. In some embodiments, the part of the immune cell surface molecule which is bound by the antigen-binding molecule of the present disclosure is on the external surface of the immune cell (i.e. is extracellular). The immune cell surface molecule may be expressed at the cell surface of any immune cell.

In some embodiments, the immune cell may be a cell of hematopoietic origin, e.g. a neutrophil, eosinophil, basophil, dendritic cell, lymphocyte, or monocyte. The lymphocyte may be e.g. a T cell, B cell, natural killer (NK) cell, NKT cell or innate lymphoid cell (ILC), or a precursor thereof (e.g. a thymocyte or pre-B cell).

In some embodiments, the immune cell surface molecule is a molecule expressed at the surface of a T cell, e.g. CD8+ T cell or a CD4+ T cell. In some embodiments, the immune cell surface molecule is a molecule expressed at the surface of a cytotoxic T cell (e.g. a cytotoxic T lymphocyte (CTL)), a virus-specific T cell (VST), a T helper cell (e.g. a Th1, Th2, Th9, Th17, Th22 or Tfh cell), a regulatory T cell (Treg), a central memory cell (Tcm), or an effector memory cell (Tem).

In some embodiments the immune cell surface molecule is selected from: a CD3-TCR complex polypeptide, CD3ε, CD3γ, CD3δ, CD3ζ, CD3η, TCRα, TCRβ, TCRγ, TCRδ, CD4, CD8, CCR5, CCR7, CD2, CD7, a costimulatory molecule, CD27, CD28, OX40, 4-1BB, ICOS, a checkpoint inhibitor, PD-1, CTLA-4, LAG-3, TIM-3, TIGIT or BTLA.

In some embodiments the immune cell surface molecule is a CD3-TCR complex polypeptide. In some embodiments the immune cell surface molecule is a CD3 polypeptide (e.g. CD3ε, CD3γ, CD3δ, CD3ζ or CD3η). In some embodiments the immune cell surface molecule is CD3ε.

In some embodiments, an antigen-binding moiety specific for an immune cell surface molecule according to the present disclosure comprises:
a VL domain comprising:
   LC-CRD1: SEQ ID NO:75;
   LC-CRD2: SEQ ID NO:76;
   LC-CRD3: SEQ ID NO:77;
and a VH domain comprising:
   HC-CRD1: SEQ ID NO:78;
   HC-CRD2: SEQ ID NO:79;
   HC-CRD3: SEQ ID NO:80.

In some embodiments an antigen-binding moiety specific for an immune cell surface molecule comprises a VL comprising, or consisting of, an amino acid sequence having at least 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99% or having 100% sequence identity to SEQ ID NO:81, and a VH comprising, or consisting of, an amino acid sequence having at least 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99% or having 100% sequence identity to SEQ ID NO:82.

### Cancer cell antigens

The antigen-binding molecule of the present disclosure comprises an antigen-binding moiety specific for a cancer cell antigen. Also, the chimeric antigen receptor (CAR) of the present disclosure is specific for a cancer cell antigen.

As used herein, a "cancer cell antigen" is an antigen which is expressed or over-expressed by a cancer cell. A cancer cell antigen may be any peptide/polypeptide, glycoprotein, lipoprotein, glycan, glycolipid, lipid, or fragment thereof. A cancer cell antigen's expression may be associated with a cancer. A cancer cell antigen may be abnormally expressed by a cancer cell (e.g. the cancer cell antigen may be expressed with abnormal localisation), or may be expressed with an abnormal structure by a cancer cell. A cancer cell antigen may be capable of eliciting an immune response.

In some embodiments, the antigen is expressed at the cell surface of the cancer cell (i.e. the cancer cell antigen is a cancer cell surface antigen). In some embodiments, the part of the cancer cell antigen which is bound by an antigen-binding moiety specific for a cancel cell antigen according to the present disclosur is displayed on the external surface of the cancer cell (i.e. is extracellular). In some embodiments, the antigen is anchored to the cell membrane, e.g. via a transmembrane domain or other membrane anchor (e.g. a lipid anchor such as a GPI anchor). In some embodiments, the cancer cell antigen is expressed at the cell surface (i.e. is expressed in or at the cell membrane) of a cancerous cell, but may be expressed inside the cell (i.e. is expressed inside comparable non-cancerous cells).

The cancer cell antigen may be a cancer-associated antigen. In some embodiments the cancer cell antigen is an antigen whose expression is associated with the development, progression and/or severity of symptoms of a cancer. The cancer-associated antigen may be associated with the cause or pathology of the cancer, or may be expressed abnormally as a consequence of the cancer. In some embodiments, the antigen is an antigen whose expression is upregulated (e.g. at the RNA and/or protein level) by cells of a cancer, e.g. as compared to the level of expression of by comparable non-cancerous cells (e.g. non-cancerous cells derived from the same tissue/cell type).

In some embodiments, the cancer-associated antigen may be preferentially expressed by cancerous cells, and not expressed by comparable non-cancerous cells (e.g. non-cancerous cells derived from the same tissue/cell type). In some embodiments, the cancer-associated antigen may be the product of a mutated oncogene or mutated tumor suppressor gene. In some embodiments, the cancer-associated antigen may be the product of an overexpressed cellular protein, a cancer antigen produced by an oncogenic virus, an oncofetal antigen, or a cell surface glycolipid or glycoprotein.

Cancer cell antigens are reviewed by Zarour HM, DeLeo A, Finn OJ, et al. Categories of Tumor Antigens. In: Kufe DW, Pollock RE, Weichselbaum RR, et al., editors. Holland-Frei Cancer Medicine. 6th edition. Hamilton (ON): BC Decker; 2003. Cancer cell antigens include oncofetal antigens: CEA, Immature laminin receptor, TAG-72; oncoviral antigens such as HPV E6 and E7; overexpressed proteins: BING-4, calcium-activated chloride channel 2, cyclin-B1, 9D7, Ep-CAM, EphA3, HER2/neu, telomerase, mesothelin, SAP-1, survivin; cancer-testis antigens: BAGE, CAGE, GAGE, MAGE, SAGE, XAGE, CT9, CT10, NY-ESO-1, PRAME, SSX-2; lineage restricted antigens: MART1, Gp100, tyrosinase, TRP-1/2, MC1R, prostate specific antigen; mutated antigens: β-catenin, BRCA1/2, CDK4, CML66, Fibronectin, MART-2, p53, Ras, TGF-βRII; post-translationally altered antigens: MUC1, idiotypic antigens: Ig, TCR. Other cancer cell antigens include heat-shock protein 70 (HSP70), heat-shock protein 90 (HSP90), glucose-regulated protein 78 (GRP78), vimentin, nucleolin, feto-acinar pancreatic protein (FAPP), alkaline phosphatase placental-like 2 (ALPPL-2), siglec-5, stress-induced phosphoprotein 1 (STIP1), protein tyrosine kinase 7 (PTK7), and cyclophilin B.

In some embodiments the cancer cell antigen is HER2. Human epidermal growth factor receptor 2 (HER2; also known e.g. as ERBB2, CD340 and NEU) is the protein identified by UniProt P04626-1 (v1). In this specification "HER2" refers to HER2 from any species and includes HER2 isoforms (e.g. P04626-1, P04626-3, P04626-4, P04626-5 or P04626-6), fragments, variants (including mutants) or homologues from any species.

HER2 is overexpressed/amplified in a range of cancers, including breast cancer, ovarian cancer, bladder cancer, salivary gland cancer, endometrial cancer, pancreatic cancer and non-small-cell lung cancer (NSCLC) - see e.g. Scholl, et al., Annals of Oncology 2001, 12 (suppl_1): S81-S87.

As used herein, a "fragment", "variant" or "homologue" of a protein may optionally be characterised as having at least 60%, preferably one of 70%, 75%, 80%, 85%, 90%, 91%, 92%, 93%, 94%, 95%, 96%, 97%, 98%, 99% or 100% amino acid sequence identity to the amino acid sequence of the reference protein (e.g. a reference isoform). In some embodiments fragments, variants, isoforms and homologues of a reference protein may be characterised by ability to perform a function performed by the reference protein.

In some embodiments the cancer cell antigen is CD44v6. CD44v6 refers to an isoform of CD44 obtained by alternative splicing, wherein exons 6 to 10 and 12 to 15 are missing. That is, the coding sequence for CD44v6 is comprised of exons 1 to 5, 11 and 16 to 20. In this specification "CD44v6" refers to CD44v6 from any species and includes fragments, variants (including mutants) or homologues from any species. Human CD44v6 has the amino acid sequence shown in SEQ ID NO:130.

CD44v6 is a cancer cell antigen which is abundantly expressed in head and neck squamous cell carcinomas (HNSCC), and several Phase I clinical trials for the use of anti-CD44v6 IgG bivatuzumab to treat head and neck cancer have been performed - see e.g. Riechelmann et al., Oral Oncol. (2008) 44(9):823-9; Tijink et al., Clin Cancer Res. (2006) 12(20 Pt 1):6064-72; Börjesson et al., Clin Cancer Res. (2003) 9(10 Pt 2):3961S-72S; and Postema et al., J Nucl Med. (2003) 44(10):1690-9. Bivatuzumab has also been investigated for the treatment of breast cancer - see e.g. Rupp et al., Anticancer Drugs (2007) 18(4):477-85. CD44v6 expression has also been shown to be associated with proliferation, invasion, adhesion, metastasis, chemo-/radioresistance, and the induction of EMT as well as the activation PI3K/Akt/mTOR and Wnt signaling pathways in prostate cancer (see Ni et al., Prostate (2014) 74(6):602-17), and CD44v6 is expressed by aggressive prostate cancer cells; positive staining for this marker is significantly higher in late stage, metastatic and higher-grade prostate cancer samples (see Peng et al., Oncotarget (2017) 8(49):86747-86768). CD44v6 has been suggested to be a useful marker for poor prognosis in pancreatic cancer (Gotoda et al., Jpn J Cancer Res. (1998) 89(10):1033-40). CD44v6 is also a marker of constitutive and reprogrammed cancer stem cells driving colon cancer metastasis (Todaro et al., Cell Stem Cell (2014) 14(3):342-56), and has been shown to be an important regulator of tumorigenesis, angiogenesis, and survival in gastric carcinoma (Xu et al., Oncotarget. (2017) 8:45848-45861). Also, CD44v6 expression levels are associated with epithelial ovarian cancer progression, metastasis and relapse (Shi et al. BMC Cancer (2013) 13:182).

T cells engineered to express a CD44v6-specific CAR have been demonstrated to mediate potent antitumor effects against acute myeloid leukemia and multiple myeloma - see e.g. Casucci et al., Blood (2013) 122:3461-3472.

In some embodiments the cancer cell antigen is CD19. CD19 is the protein identified by UniProt P15391-1 (v6). In this specification "CD19" refers to CD19 from any species and includes CD19 isoforms (e.g. P15391-2), fragments, variants (including mutants) or homologues from any species.

CD19 is a marker of B cells, and is a useful target for the treatment of e.g. B cell lymphomas, acute lymphoblastic leukemia (ALL), and chronic lymphocytic leukemia (CLL) - see e.g. Wang et al., Exp Hematol Oncol. (2012) 1:36.

In embodiments of the present disclosure where an antigen-binding molecule and a cell comprising a CAR are employed together, the antigen-binding molecule and CAR may be specific for the same cancer cell antigen, or may be specific for different cancer cell antigens. In some embodiments the antigen-binding molecule and CAR are specific for different cancer cell antigens. That is (i) the antigen-binding moiety specific for a cancer cell antigen of the antigen-binding molecule, and (ii) the antigen-binding moiety of the CAR, may be specific for the same or different cancer cell antigens.

In some embodiments of the present disclosure where an antigen-binding molecule and a cell comprising a CAR are employed together, the antigen-binding molecule is specific for CD44v6 and the CAR is specific for HER2. In some embodiments the antigen-binding molecule is specific for CD19 and the CAR is specific for HER2. In some embodiments the antigen-binding molecule is specific for HER2 and the CAR is specific for HER2.

In some embodiments, an antigen-binding moiety specific for a cancer cell antigen according to the present disclosure comprises:
a VL domain comprising:
   LC-CRD1: SEQ ID NO:9;
   LC-CRD2: SEQ ID NO:10;
   LC-CRD3: SEQ ID NO:11;
and a VH domain comprising:
   HC-CRD1: SEQ ID NO:12;
   HC-CRD2: SEQ ID NO:13;
   HC-CRD3: SEQ ID NO:14.

In some embodiments an antigen-binding moiety specific for a cancer cell antigen comprises a VL comprising, or consisting of, an amino acid sequence having at least 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99% or having 100% sequence identity to SEQ ID NO:15, and a VH comprising, or consisting of, an amino acid sequence having at least 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99% or having 100% sequence identity to SEQ ID NO:16.

In some embodiments, an antigen-binding moiety specific for a cancer cell antigen according to the present disclosure comprises:
a VL domain comprising:
   LC-CRD1: SEQ ID NO:17;
   LC-CRD2: SEQ ID NO:18;
   LC-CRD3: SEQ ID NO:19;
and a VH domain comprising:
   HC-CRD1: SEQ ID NO:20;
   HC-CRD2: SEQ ID NO:21;
   HC-CRD3: SEQ ID NO:22.

In some embodiments an antigen-binding moiety specific for a cancer cell antigen comprises a VL comprising, or consisting of, an amino acid sequence having at least 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99% or having 100% sequence identity to SEQ ID NO:23, and a VH comprising, or consisting of, an amino acid sequence having at least 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99% or having 100% sequence identity to SEQ ID NO:24.

In some embodiments, an antigen-binding moiety specific for a cancer cell antigen according to the present disclosure comprises:
a VL domain comprising:
   LC-CRD1: SEQ ID NO:25;
   LC-CRD2: SEQ ID NO:26;
   LC-CRD3: SEQ ID NO:27;
and a VH domain comprising:
   HC-CRD1: SEQ ID NO:28;
   HC-CRD2: SEQ ID NO:29;
   HC-CRD3: SEQ ID NO:30.

In some embodiments an antigen-binding moiety specific for a cancer cell antigen comprises a VL comprising, or consisting of, an amino acid sequence having at least 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99% or having 100% sequence identity to SEQ ID NO:31 and a VH comprising, or consisting of, an amino acid sequence having at least 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99% or having 100% sequence identity to SEQ ID NO:32.

In some embodiments, an antigen-binding moiety specific for a cancer cell antigen according to the present disclosure comprises:
a VL domain comprising:
   LC-CRD1: SEQ ID NO:33;
   LC-CRD2: SEQ ID NO:34;
   LC-CRD3: SEQ ID NO:35;
and a VH domain comprising:
   HC-CRD1: SEQ ID NO:36;
   HC-CRD2: SEQ ID NO:37;
   HC-CRD3: SEQ ID NO:38.

In some embodiments an antigen-binding moiety specific for a cancer cell antigen comprises a VL comprising, or consisting of, an amino acid sequence having at least 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99% or having 100% sequence identity to SEQ ID NO:39, and a VH comprising, or consisting of, an amino acid sequence having at least 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99% or having 100% sequence identity to SEQ ID NO:40.

In some embodiments, an antigen-binding moiety specific for a cancer cell antigen according to the present disclosure comprises:
a VL domain comprising:
   LC-CRD1: SEQ ID NO:66;
   LC-CRD2: SEQ ID NO:67;
   LC-CRD3: SEQ ID NO:68;
and a VH domain comprising:
   HC-CRD1: SEQ ID NO:69;
   HC-CRD2: SEQ ID NO:70;
   HC-CRD3: SEQ ID NO:71.

In some embodiments an antigen-binding moiety specific for a cancer cell antigen comprises a VL comprising, or consisting of, an amino acid sequence having at least 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99% or having 100% sequence identity to SEQ ID NO:72, and a VH comprising, or consisting of, an amino acid sequence having at least 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99% or having 100% sequence identity to SEQ ID NO:73.

In some embodiments, an antigen-binding moiety specific for a cancer cell antigen according to the present disclosure comprises:
a VL domain comprising:
   LC-CRD1: SEQ ID NO:85;
   LC-CRD2: SEQ ID NO:86;
   LC-CRD3: SEQ ID NO:87;
and a VH domain comprising:
   HC-CRD1: SEQ ID NO:88;
   HC-CRD2: SEQ ID NO:89;
   HC-CRD3: SEQ ID NO:90.

In some embodiments an antigen-binding moiety specific for a cancer cell antigen comprises a VL comprising, or consisting of, an amino acid sequence having at least 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99% or having 100% sequence identity to SEQ ID NO:91, and a VH comprising, or consisting of, an amino acid sequence having at least 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99% or having 100% sequence identity to SEQ ID NO:92.

In some embodiments, an antigen-binding moiety specific for a cancer cell antigen according to the present disclosure comprises:
a VL domain comprising:
   LC-CRD1: SEQ ID NO:95;
   LC-CRD2: SEQ ID NO:96;
   LC-CRD3: SEQ ID NO:97;
and a VH domain comprising:
   HC-CRD1: SEQ ID NO:98;
   HC-CRD2: SEQ ID NO:99;
   HC-CRD3: SEQ ID NO:100.

In some embodiments an antigen-binding moiety specific for a cancer cell antigen comprises a VL comprising, or consisting of, an amino acid sequence having at least 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99% or having 100% sequence identity to SEQ ID NO:101, and a VH comprising, or consisting of, an amino acid sequence having at least 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99% or having 100% sequence identity to SEQ ID NO:102.

### Nucleic acid encoding the antigen-binding molecule

Aspects of the present disclosure employ nucleic acid encoding an antigen-binding molecule comprising: (a) an antigen-binding moiety specific for an immune cell surface molecule, and (b) an antigen-binding moiety specific for a cancer cell antigen.

In some aspects, the nucleic acid is comprised within a virus. That is, the virus comprises nucleic acid encoding the antigen-binding molecule. In such embodiments, the virus acts as a vector for delivering the antigen-binding molecule.

Any virus capable of introducing nucleic acid into a cell (e.g. a primary human immune cell) may be used. Suitable viruses include gammaretrovirus (e.g. murine Leukemia virus (MLV)-derived vectors), lentivirus, adenovirus, adeno-associated virus, vaccinia virus and herpesvirus, *e.g.* as described in Maus et al., Annu Rev Immunol (2014) 32:189-225 or Morgan and Boyerinas, Biomedicines 2016 4, 9, which are both hereby incorporated by reference in its entirety. In some embodiments, the virus comprising nucleic acid encoding an immunomodulatory factor is, or is derived from, an adenovirus, lentivirus, retrovirus, or herpesvirus.

In some embodiments, the virus comprises nucleic acid encoding an antigen-binding molecule described hereinabove. In some embodiments, the virus further comprises nucleic acid encoding one or more immunomodulatory factors described hereinbelow.

In some embodiments, the virus further comprises nucleic acid encoding a further antigen-binding molecule comprising: (a) an antigen-binding moiety specific for an immune cell surface molecule, and (b) an antigen-binding moiety specific for a cancer cell antigen. That is, in some embodiments the virus comprises nucleic acid encoding more than one antigen-binding molecule comprising: (a) an antigen-binding moiety specific for an immune cell surface molecule, and (b) an antigen-binding moiety specific for a cancer cell antigen. In some embodiments the virus comprises nucleic acid encoding, e.g. 2, 3, 4 or 5 such antigen-binding molecules.

In some embodiments the encoded antigen-binding molecules are non-identical. In embodiments wherein the virus comprises nucleic acid encoding more than one antigen-binding molecule comprising: (a) an antigen-binding moiety specific for an immune cell surface molecule, and (b) an antigen-binding moiety specific for a cancer cell antigen, the antigen-binding moieties specific for a cancer cell antigen may are non-identical. For example, in some embodiments the virus encodes an antigen-binding molecule comprising an antigen-binding moiety specific for CD44v6, and an antigen-binding molecule comprising an antigen-binding moiety specific for HER2.

In some embodiments the virus comprises nucleic acid encoding further functional sequence(s). For example, the virus may comprise nucleic acid encoding a protein(s) for reducing growth/proliferation/survival of infected cells, or protein(s) for rendering infected cells sensitive to treatment with a given agent, or protein(s) for disrupting tumour structure (e.g. enzymes for digesting tumour matrix) to facilitate immune cell infiltration.

In some embodiments the virus comprises nucleic acid encoding an enzyme capable of catalysing conversion of a non-toxic factor to a cytotoxic form. The enzyme may catalyse conversion of a non-toxic prodrug into its active, cytotoxic form.

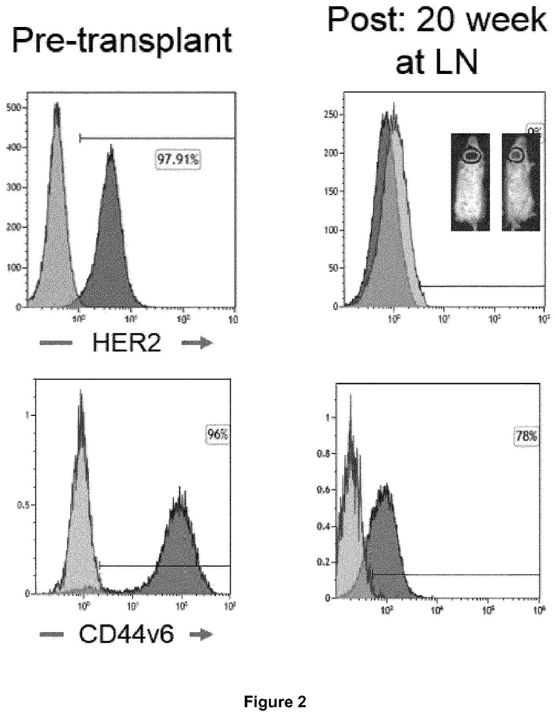

Enzyme/prodrug systems are well known in the art and include those described in Malekshah et al. Curr Pharmacol Rep. (2016) 2(6): 299-308 which is hereby incorporated by reference in its entirety. Examples of non-toxic prodrugs, their active cytotoxic forms and enzymes capable of catalysing conversion of the non-toxic prodrugs to their active cytotoxic forms are shown in Figure 2 of Malekshah et al.

In some embodiments the virus comprises nucleic acid encoding a thymidine kinase, cytosine deaminase, nitroreductase, cytochrome P450, carboxypeptidase G2, purine nucleoside phosphorylase, horseradish peroxidase and/or carboxylesterase. In some embodiments the virus comprises nucleic acid encoding an amino acid sequence which comprises, or consists of, an amino acid sequence having at least 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99% or having 100% sequence identity to SEQ ID NO:42.

For example, the virus may comprise nucleic acid encoding thymidine kinase for rendering cells expressing the virus sensitive to treatment with ganciclovir (GCV), aciclovir (ACV) and/or valaciclovir. The virus may comprise nucleic acid encoding cytosine deaminase for rendering cells expressing the virus sensitive to treatment with 5-fluorocytosine (5-FC), which is converted by cytosine deaminase to 5-fluorouracil (5-FU). The virus may comprise nucleic acid encoding nitroreductase for rendering cells expressing the virus sensitive to treatment with CB1954, nitro-CBI-DEI and/or PR-104A. The virus may comprise nucleic acid encoding cytochrome P450 for rendering cells expressing the virus sensitive to treatment with oxazaphosphorine (e.g. cyclophosphamide or ifosfamide). The virus may comprise nucleic acid encoding carboxypeptidase G2 for rendering cells expressing the virus sensitive to treatment with nitrogen mustard based drugs (e.g. CMDA or ZD2767P). The virus may comprise nucleic acid encoding purine nucleoside phosphorylase for rendering cells expressing the virus sensitive to treatment with 6-methylpurine 2-deoxyriboside and/or fludarabine (e.g. 6-methylpurine-2'-deoxyriboside (MeP-dR), 2-F-2'-deoxyadenosine (F-dAdo) or arabinofuranosyl-2-F-adenine monophosphate (F-araAMP). The virus may comprise nucleic acid encoding horseradish peroxidase for rendering cells expressing the virus sensitive to treatment with indole-3-acetic acid (IAA). The virus may comprise nucleic acid encoding carboxylesterase for rendering cells expressing the virus sensitive to treatment with irinotecan.

In some embodiments the virus may comprise nucleic acid encoding antagonist of a growth factor.

In some embodiments, the virus may be a helper-dependent adenovirus (HDAd). HDAds are reviewed, for example, in Rosewell et al., J Genet Syndr Gene Ther (2011) Suppl 5:001, which is hereby incorporated by reference in its entirety.

HDAds are devoid of viral protein coding sequences, and therefore possess a large capacity (up to 37 Kb) for transduction of a coding sequence of interest. HDAds are non-integrating, and are able to efficiently transduce a wide variety of cell types independently of the cell cycle, and mediate long-term transgene expression without chronic toxicity.

HDAds comprise only the cis acting viral elements required for genomic replication (inverted terminal repeats (ITRs)) and encapsidation (ψ), and are therefore dependent on helper virus for propagation. When a cell is infected with both the helper virus and the HDAd, the helper virus replication machinery acts in *trans* to replicate and package HDAd.

In particular embodiments of the present disclosure, the oncolytic virus is an OncAd and the virus comprising nucleic acid encoding an immunomodulatory factor is a HDAd, and the OncAd and HDAd are able to co-infect and replicate in cells of a cancer.

Dependence of the HDAd on help from the OncAd provides highly localised expression of the immunomodulatory factor(s). That is, because the HDAd is only able to propagate in cells co-infected with the OncAd, and in turn because the OncAd is selective for replication in cancerous cells, expression of the factor(s) encoded by the HDAd is restricted to cancerous cells/tissue, minimising side effects.

Furthermore, because OncAd and HDAd efficiently target and infect tumour cells, expression of immunomodulatory factor(s) in those cells can change the normally immunosuppressive tumour microenvironment to provide conditions promoting the activation, recruitment (i.e. tumour penetration/infiltration), proliferation, activity and/or survival of effector immune cells.

In particular, in the context of the present disclosure wherein the CAR-T cells, expression of immunomodulatory factor(s) encoded by the virus (e.g. HDAd) provide for enhanced activation, recruitment, proliferation, activity and/or survival of the CAR-T cells.

In embodiments wherein the virus encodes more than one antigen-binding molecule as described herein, and/or one more immunomodulatory factors, the coding sequences may be provided in the same or different expression cassettes. That is, expression of the coding sequences may be under the control of the same regulatory sequences or different regulatory sequences.

In some embodiments the wherein the virus encodes more than one antigen-binding molecule as described herein, and/or one more immunomodulatory factors, the antigen-binding molecule(s)/factors may be expressed as a fusion protein.

In some embodiments, the virus encoding an antigen-binding molecule comprising: (a) an antigen-binding moiety specific for an immune cell surface molecule, and (b) an antigen-binding moiety specific for a cancer cell antigen or the present disclosure comprises, or consists of, or consists essentially of, a nucleic acid sequence having at least 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99% or having 100% sequence identity to SEQ ID NO:121, 122, 123, 124 or 125, or an equivalent sequence as a result of codon degeneracy.

### Immunomodulatory factors

In some embodiments, a virus encoding an antigen-binding molecule comprising: (a) an antigen-binding moiety specific for an immune cell surface molecule, and (b) an antigen-binding moiety specific for a cancer cell antigen according to the present disclosure additionally encodes an immunomodulatory factor. In some embodiments, a virus comprises nucleic acid encoding one or more immunomodulatory factor(s).

Immunomodulatory factor(s) according to the present disclosure are preferably selected to facilitate the immune response to a cancer in a subject, in particular the cell-mediated immune response. In some embodiments, the immunomodulatory factor(s) provide favourable conditions for the activation, recruitment, proliferation, activity and/or survival of effector immune cells (*e.g.* CTLs, T_{H}1 cells, NK cells or NKT cells).

In some embodiments, the immunomodulatory factor may be an agonist of an effector immune response. An agonist of an effector immune response may be, *e.g.* a cytokine or chemokine promoting activation, recruitment, proliferation, activity and/or survival of effector immune cells (*e.g.* IL-2, IL-7, IL-17, IL-12, IL-21, IL-15, MIP-1α or RANTES), agonist antibody for a costimulatory receptor (*e.g.* 4-1BB, OX40, CD28, CD27, ICOS, CD30 or GITR), or ligand for a costimulatory receptor (*e.g.* 4-1BBL, OX40L, CD80, CD86, CD70, ICOSL, CD30L or GITRL). In some embodiments, the agonist of an effector immune response may be an antagonist of an immune checkpoint inhibitor, or an antagonist of ligand for immune checkpoint inhibitor, *e.g.* antagonist antibody to PD-L1, PD-L2, PD-1, CTLA-4, LAG-3, TIM-3, Gal-9, TIGIT, VISTA or BTLA, or an antagonist of a cytokine/chemokine which is an antagonist of an effector immune response, *e.g.* TGFβ (*i.e.* antagonist anti-TGFβ antibody or soluble/decoy TGFβ receptor). In some embodiments, an agonist of an effector immune response may be a molecule for engaging and co-opting bystander effector immune cells such as T cells and NK cells.

In some embodiments, the immunomodulatory factor may be an antagonist of an immunoregulatory response, *e.g.* an antagonist of a cytokine/chemokine promoting activation, recruitment, proliferation, activity and/or survival of immunoregulatory cells such as regulatory T cells (Tregs) and/or myeloid-derived suppressor cells (MDSCs), *e.g.* CCL9, CXCL10, CCL20, CCL22.

In some embodiments the immunomodulatory factor is IL-12. In some embodiments the immunomodulatory factor comprises, or consists of, an amino acid sequence having at least 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99% or having 100% sequence identity to SEQ ID NO:41.

In some embodiments the immunomodulatory factor is an antagonist of PD-1/PD-L1 signalling. In some embodiments the antagonist of PD-1/PD-L1 signalling is an anti-PD-L1 antibody.

In some embodiments the anti-PD-L1 antibody comprises an antigen-binding moiety comprising:
a VL domain comprising:
   LC-CRD1: SEQ ID NO:53;
   LC-CRD2: SEQ ID NO:54;
   LC-CRD3: SEQ ID NO:55;
and a VH domain comprising:
   HC-CRD1: SEQ ID NO:56;
   HC-CRD2: SEQ ID NO:57;
   HC-CRD3: SEQ ID NO:58.

In some embodiments the anti-PD-L1 antibody comprises an antigen-binding moiety comprising a VL comprising, or consisting of, an amino acid sequence having at least 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99% or having 100% sequence identity to SEQ ID NO:59, and a VH comprising, or consisting of, an amino acid sequence having at least 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99% or having 100% sequence identity to SEQ ID NO:60.

### Oncolytic virus

Aspects of the present disclosure employ oncolytic virus. Oncolytic viruses and their use to treat cancer is reviewed, for example, in Chiocca and Rabkin Cancer Immunol Res (2014) 2(4): 295-300, which is hereby incorporated by reference in its entirety.

Oncolytic viruses replicate in, and cause lysis of, cancer cells. Often they are selective for cancer cells over non-cancerous cells; for example, oncolytic viruses commonly replicate in dividing cells in preference to non-dividing cells. Oncolytic viruses are therefore useful to selectively kill cancer cells and destroy tumours, without causing substantial damage to normal, non-cancerous cells/tissue.

Oncolytic virotherapy is associated with several advantages features. Oncolytic viruses often target several oncogenic pathways and use multiple mechanisms for cytotoxicity, minimising the chances of resistance arising. As noted above, because oncolytic viruses replicate selectively in tumours and are non-pathogenic they display minimal toxicity. Virus dose in the tumour also increases over time due to replication of the virus, and the oncolytic viruses can also be manipulated genetically to improve safety, *e.g.* by engineering sensitivity to a drug.

There are two main classes of oncolytic virus:
(i) viruses that naturally replicate preferentially in cancer cells, and which are non-pathogenic in humans often due to elevated sensitivity to innate antiviral signalling or dependence on oncogenic signalling pathways, including autonomous parvoviruses, myxoma virus (MYXV; poxvirus), Newcastle disease virus (NDV; paramyxovirus), reovirus, and Seneca valley virus (SVV; picornavirus); and
(ii) viruses that are genetically-manipulated, *e.g.* with mutations/deletions in genes required for replication in normal, but not cancer cells, including adenovirus (Ad), herpes simplex virus (HSV), vaccinia virus (VV), and vesicular stomatitis virus (VSV; rhabdovirus); or viruses that are genetically-manipulated for use as vaccine vectors including measles virus (MV; paramyxovirus), poliovirus (PV; picornavirus), and VV (poxvirus).

Genetic manipulation can include insertion/alteration of functional sequences to provide enhanced selectivity for cancer cells, safety, and/or to modify virus tropism.

For example, oncolytic virus may by genetically engineered to introduce tissue-specific internal ribosome entry sites (IRESs) only permitting viral translation in target cells, and/or to introduce miRNAs/miRNA response elements (MREs); differential miRNA expression between healthy cells or certain tissues vs. tumor cells allows viruses to be detargeted from healthy cells/tissues. Oncolytic virus may also by engineered to place transcription of the viral genome under the control of a cell- or tissue-specific regulatory region, such as promoter/enhancers (*e.g.* tumour cell-specific promoter). In some embodiments, the oncolytic virus according to the present disclosure may comprise one or more modifications for such purpose.

Virus may also be modified for transductional targeting, *e.g.* through modification of virus receptors/coat proteins to target tumour cells and/or detarget healthy cells/tissues.

Oncolytic viruses may be administered in such a way as to minimise anti-oncolytic virus responses (*e.g.* neutralisation by anti-virus antibodies) in the subject and sequestration in the liver, and to maximise tumour delivery, as described in Chiocca and Rabkin, *supra.* For example, oncolytic virus may be administered in a cell carrier, *e.g.* in mesenchymal stromal cells, myeloid-derived suppresser cells (MDSCs), neural stem cells, T cells, cytokine-induced killer cells, or irradiated tumor cells, or can be coated in nanoparticles.

In some embodiments, the oncolytic virus of the present disclosure is, or is derived from, an adenovirus (Ad), herpes simplex virus (HSV), vaccinia virus (VV), vesicular stomatitis virus (VSV); autonomous parvovirus, myxoma virus (MYXV), Newcastle disease virus (NDV), reovirus, Seneca valley virus (SVV) morbillivirus virus, retrovirus, influenza virus, Sindbis virus (SINV) or poxvirus, as examples. In some embodiments, the oncolytic virus is not vaccinia virus. In some embodiments, the oncolytic virus is not vaccinia virus JX-594.

As used herein, an oncolytic virus which is "derived from" a reference virus comprises a nucleic acid sequence or amino acid sequence which is possessed by the reference virus. In some embodiments an oncolytic virus which is "derived from" a reference virus comprises one or more genes possessed by the reference virus. In some embodiments an oncolytic virus which is "derived from" encodes one or more proteins encoded by the reference virus.

In some embodiments, an oncolytic virus which is derived from a reference virus may comprise nucleic acid sequence encoding one or more functional elements of the reference virus. A "functional element" may *e.g.* be a transcriptional regulator (*e.g.* a promoter/enhancer), a regulator of post-transcriptional processing, a translational regulator, a regulator of post-transcriptional processing, a response element, a repeat sequence, or a viral protein. In some embodiments, an oncolytic virus which is derived from a reference virus may comprise one or more genes of, or proteins encoded by, the reference virus.

In some embodiments the oncolytic virus of the present disclosure is, or is derived from, an adenovirus (OncAd). OncAds are reviewed *e.g.* in Larson et al., Oncotarget. (2015) 6(24): 19976-19989, which is hereby incorporated by reference in its entirety.

In some embodiments the OncAd is, or is derived from, a species A, B, C, D, E, F or G human adenovirus (i.e. HAdV-A, HAdV-B, HAdV-C, HAdV-D, HAdV-E, HAdV-F or HAdV-G). In some embodiments the OncAd is, or is derived from, a species C human adenovirus. In some embodiments the OncAd is, or is derived from, Ad5, Ad2, Ad1, Ad6 or Ad57.

In some embodiments the OncAd is a conditionally replicating adenovirus (or CRAd).

In some embodiments the OncAd has reduced ability to infect, replicate in and/or lyse non-cancerous cells (as compared to the ability to infect/replicate in and/or lyse equivalent cancerous cells), for example as a consequence of a genetic modification of the adenovirus from which the OncAd is derived.

In some embodiments the oncolytic virus comprises a modification to one or more protein encoding sequences. In some embodiments, the modification alters the production or activity of the encoded protein. In some embodiments, the modification is a truncation or deletion of the protein.

In some embodiments, the OncAd comprises modification to an adenovirus early protein. In some embodiments, the modification is to the region encoding E1A protein. In some embodiments, the OncAd encodes an E1A protein having reduced ability to bind to Rb protein as compared to wildtype E1A protein (*e.g.* E1A encoded by the adenovirus from which the OncAd is derived). In some embodiments the OncAd encodes an E1A protein lacking the amino acid sequence LTCHEACF (SEQ ID NO:105). An example of an OncAd comprising encodes an E1A protein lacking the amino acid sequence LTCHEACF (SEQ ID NO:105) is Onc5/3Ad2E1Δ24 shown in SEQ ID NO:104.

In some embodiments the oncolytic virus encodes an E1A protein comprising, or consisting of or consisting essentially of, an amino acid sequence having at least 80%, 81%, 82%, 83%, 84%, 85%, 86%, 87%, 88%, 89%, 90%, 91%, 92%, 93%, 94%, 95%, 96%, 97%, 98%, 99% or having 100% sequence identity to SEQ ID NO:104.

In some embodiments the oncolytic virus according to the present disclosure comprises, or consists of, or consists essentially of, a nucleic acid sequence having at least 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99% or having 100% sequence identity to SEQ ID NO:126 or an equivalent sequence as a result of codon degeneracy.

In some embodiments, the oncolytic virus comprises a nucleic acid sequence providing one or more binding sites for one or more transcription factors. In some embodiments, the transcription factor is an activating transcription factor (*i.e.* a transcriptional activator). The one or more binding sites for one or more transcription factors are preferably provided upstream of (*i.e.* 5' to) to nucleic acid sequence encoding one or more functional elements (*e.g.* viral proteins).

In some embodiments, the transcription factor is a transcription factor having increased expression, or increased activity, in cancerous cells as compared to comparable non-cancerous cells (*e.g.* non-cancerous cells derived from the same tissue/cell type).

Herein, "expression" may refer to gene expression or protein expression. Gene expression can be measured by various means known to those skilled in the art, for example by measuring levels of mRNA by quantitative real-time PCR (qRT-PCR), or by reporter-based methods. Similarly, protein expression can be measured by various methods well known in the art, *e.g.* by antibody-based methods, for example by western blot, immunohistochemistry, immunocytochemistry, flow cytometry, ELISA, ELISPOT, or reporter-based methods.

An example of an OncAd comprising one or more binding sites for one or more transcription factors is ICOVIR15 described in Rojas et al. 2010 Mol Ther 18 1960-1971, which is hereby incorporated by reference its entirety. ICOVIR15 comprises 8 binding sites for the transcription factor E2F.

In some embodiments the oncolytic virus comprises one or more binding sites for a transcription factor whose gene or protein expression, or activity in a cell, is upregulated in response to a factor produced or expressed by an immune cell. In some embodiments, a factor produced or expressed by an immune cell may at least one cytokine/chemokine produced by, or a protein expressed at the cell surface of, an effector immune cell, *e.g.* CD8+ cytotoxic T lymphocyte (CTL), CD4+ T helper 1 (T_{H}1) cell, natural killer (NK) cell or natural killer T (NKT) cell.

In some embodiments, the oncolytic virus of the present disclosure comprises one or more binding sites for a STAT transcription factor. In some embodiments, the oncolytic virus comprises one or more binding sites for a STAT1. An ICOSTAT OncAd described herein possesses 8 binding sites for STAT1, and STAT1 is known to be upregulated by IFNγ. In particular embodiments, ICOSTAT is a particularly effective treatment for a cancer because the host's immune response to the cancer cells will promote the replication of the oncolytic virus *in situ.*

In some embodiments, the oncolytic virus comprises more than one binding site for a STAT1, *e.g.* at least 2, 3, 4, 5, 6, 7, 8, 9, or 10 binding sites for STAT1. In some embodiments, a binding site for STAT1 may comprise or consist of or consist essentially of the sequence TTCCGGGAA (SEQ ID NO:128), or TTCTCGGAA (SEQ ID NO:129). In some embodiments, the oncolytic virus of the present disclosure comprises one or more copies of the sequence TTCCGGGAA (SEQ ID NO:128) or TTCTCGGAA (SEQ ID NO:129).

In some embodiments the oncolytic virus according to the present disclosure comprises, or consists of, or consists essentially of, a nucleic acid sequence having at least 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99% or having 100% sequence identity to SEQ ID NO:127 or an equivalent sequence as a result of codon degeneracy.

### Chimeric Antigen Receptors (CARs) and CAR-expressing cells

The present disclosure employs immune cells comprising a chimeric antigen receptor (CAR). The CAR of the present disclosure comprises an antigen-binding moiety specific for a cancer cell antigen. The cancer cell antigen may be a cancer cell antigen as described hereinabove.

Chimeric Antigen Receptors (CARs) are recombinant receptors that provide both antigen-binding and immune cell activating functions. CAR structure and engineering is reviewed, for example, in Dotti et al., Immunol Rev (2014) 257(1), hereby incorporated by reference in its entirety. CARs comprise an antigen-binding moiety linked to a cell membrane anchor region and a signaling region. An optional hinge region may provide separation between the antigen-binding moiety and cell membrane anchor region, and may act as a flexible linker.

The antigen-binding moiety of a CAR may be based on the antigen-binding moiety of an antibody which is specific for the antigen to which the CAR is targeted, or other agent capable of binding to the target. For example, the antigen-binding moiety of a CAR may comprise amino acid sequences for the complementarity-determining regions (CDRs) or complete light chain and heavy chain variable region amino acid sequences of an antibody which binds specifically to the target protein. Antigen-binding moieties of CARs may target antigen based on other protein:protein interaction, such as ligand:receptor binding; for example an IL-13Rα2-targeted CAR has been developed using an antigen-binding moiety based on IL-13 (see *e.g.* Kahlon et al. 2004 Cancer Res 64(24): 9160-9166).

In some embodiments the CAR of the present disclosure comprise an antigen-binding moiety specific for a cancer cell antigen as described herein.

The antigen-binding moiety of the CAR may be provided with any suitable format, *e.g.* scFv, Fab, *etc.* In some embodiments, the antigen-binding moiety of the CAR comprises or consists of a cancer cell antigen-binding scFv.

The cell membrane anchor region is provided between the antigen-binding moiety and the signalling region of the CAR. The cell membrane anchor region provides for anchoring the CAR to the cell membrane of a cell expressing a CAR, with the antigen-binding moiety in the extracellular space, and signalling region inside the cell. Suitable transmembrane domains include transmembrane region derived from CD28, CD3-ζ, CD4 or CD8.

In some embodiments the cell membrane anchor region comprises, or consists of or consists essentially of, an amino acid sequence having at least 80%, 85%, 90%, 91%, 92%, 93%, 94%, 95%, 96%, 97%, 98%, 99% or 100% sequence identity to SEQ ID NO:5.

The signalling region of a CAR allows for activation of the T cell. The CAR signalling regions may comprise the amino acid sequence of the intracellular domain of CD3-ζ, which provides immunoreceptor tyrosine-based activation motifs (ITAMs) for phosphorylation and activation of the CAR-expressing T cell. Signalling regions comprising sequences of other ITAM-containing proteins have also been employed in CARs, such as domains comprising the ITAM containing region of FcγRI (Haynes et al., 2001 J Immunol 166(1):182-187). CARs comprising a signalling region derived from the intracellular domain of CD3-ζ are often referred to as first generation CARs.

In some embodiments the signaling region comprises, or consists of, an amino acid sequence having at least 80%, 85%, 90%, 91%, 92%, 93%, 94%, 95%, 96%, 97%, 98%, 99% or 100% sequence identity to SEQ ID NO:7.

Signalling regions of CARs may also comprise co-stimulatory sequences derived from the signalling region of co-stimulatory molecules, to facilitate activation of CAR-expressing T cells upon binding to the target protein. Suitable co-stimulatory molecules include at least CD28, OX40, 4-1BB, ICOS and CD27. CARs having a signalling region including additional co-stimulatory sequences are often referred to as second generation CARs.

In some cases CARs are engineered to provide for co-stimulation of different intracellular signalling pathways. For example, signalling associated with CD28 costimulation preferentially activates the phosphatidylinositol 3-kinase (P13K) pathway, whereas the 4-1BB-mediated signalling is through TNF receptor associated factor (TRAF) adaptor proteins. Signalling regions of CARs therefore sometimes contain co-stimulatory sequences derived from signalling regions of more than one co-stimulatory molecule. CARs comprising a signalling region with multiple co-stimulatory sequences are often referred to as third generation CARs.

In some embodiments, the CAR of the present disclosure comprises one or more co-stimulatory sequences comprising or consisting of or consisting essentially of an amino acid sequence which comprises, consists of or consists essentially of, or is derived from, the amino acid sequence of the intracellular domain of one or more of CD28, OX40, 4-1BB, ICOS and CD27.

In some embodiments the signaling region comprises, or consists of, an amino acid sequence having at least 80%, 85%, 90%, 91%, 92%, 93%, 94%, 95%, 96%, 97%, 98%, 99% or 100% sequence identity to SEQ ID NO:6.

An optional hinge region may provide separation between the antigen-binding moiety and the transmembrane domain, and may act as a flexible linker. Hinge regions may be flexible domains allowing the binding moiety to orient in different directions. Hinge regions may be derived from IgG1 or the CH₂CH₃ region of immunoglobulin. In some embodiments, the CAR of the present disclosure comprises a hinge region comprising or consisting of or consisting essentially of an amino acid sequence which comprises, consists of or consists essentially of, or is derived from, the amino acid sequence of the hinge region of IgG1 or the CH₂CH₃ region of immunoglobulin.

In some embodiments the hinge region comprises, or consists of, an amino acid sequence having at least 85%, 90%, 91%, 92%, 93%, 94%, 95%, 96%, 97%, 98%, 99% or 100% sequence identity to SEQ ID NO:8.

In some embodiments the CAR comprises, or consists of, an amino acid sequence having at least 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99% or having 100% sequence identity to SEQ ID NO:1, 2, 3 or 4.

The present disclosure also provides a cell comprising or expressing a CAR according to the present disclosure. Also provided is a cell comprising or expressing a nucleic acid encoding a CAR according to the disclosure. Engineering of CARs into T cells may be performed during culture, *in vitro,* for transduction and expansion, such as happens during expansion of T cells for adoptive T cell therapy. Methods for engineering immune cells to express CARs are known to the skilled person and are described e.g. in Wang and Rivière Mol Ther Oncolytics. (2016) 3:16015, which is hereby incorporated by reference in its entirety. It will be appreciated that "at least one cell" encompasses plural cells, e.g. populations of such cells.

The cell comprising or expressing a CAR according to the present disclosure may be a eukaryotic cell, *e.g.* a mammalian cell. The mammal may be a human, or a non-human mammal (*e.g.* rabbit, guinea pig, rat, mouse or other rodent (including any animal in the order Rodentia), cat, dog, pig, sheep, goat, cattle (including cows, *e.g.* dairy cows, or any animal in the order Bos), horse (including any animal in the order Equidae), donkey, and non-human primate).

In some embodiments, the cell may be from, or may have been obtained from, a human subject. Where the CAR-expressing cell is to be used in the treatment of a subject, the cell may be from the subject to be treated with the CAR-expressing cell (*i.e.* the cell may be autologous), or the cell may be from a different subject (*i.e.* the cell may be allogeneic).

The cell may be an immune cell. The cell may be a cell of hematopoietic origin, *e.g.* a neutrophil, eosinophil, basophil, dendritic cell, lymphocyte, or monocyte. The lymphocyte may be *e.g.* a T cell, B cell, NK cell, NKT cell or innate lymphoid cell (ILC), or a precursor thereof. The cell may express *e.g.* CD3 polypeptides (*e.g.* CD3γ CD3ε CD3ζ or CD3δ), TCR polypeptides (TCRα or TCRβ), CD27, CD28, CD4 or CD8.

In some embodiments, the cell is a T cell. In some embodiments, the T cell is a CD3+ T cell. In some embodiments, the T cell is a CD3+, CD8+ T cell. In some embodiments, the T cell is a cytotoxic T cell (*e.g.* a cytotoxic T lymphocyte (CTL)).

The use of CAR T-cells is associated with advantages that they can be systemically administered, and will home to both primary and metastasized tumors (Manzo et al., Human Molecular Genetics (2015) R67-73).

In some embodiments, the cell is an antigen-specific T cell. In embodiments herein, an "antigen-specific" T cell is a cell which displays certain functional properties of a T cell in response to the antigen for which the T cell is specific, or a cell expressing said antigen. In some embodiments, the properties are functional properties associated with effector T cells, *e.g.* cytotoxic T cells.

In some embodiments, an antigen-specific T cell may display one or more of the following properties: cytotoxicity, *e.g.* to a cell comprising/expressing antigen for which the T cell is specific; proliferation, IFNγ expression, CD107a expression, IL-2 expression, TNFα expression, perforin expression, granzyme expression, granulysin expression, and/or FAS ligand (FASL) expression, *e.g.* in response to antigen for which the T cell is specific or a cell comprising/expressing antigen for which the T cell is specific. Antigen-specific T cells comprise a TCR capable of recognising a peptide of the antigen for which the T cell is specific when presented by the appropriate MHC molecule. Antigen-specific T cells may be CD4+ T cells and/or CD8+ T cells.

In some embodiments, the antigen for which the T cell is specific may be a peptide or polypeptide of a virus, *e.g.* Adenovirus, Cytomegalovius (CMV), Epstein-Barr virus (EBV), human papilloma virus (HPV), influenza virus, measles virus, hepatitis B virus (HBV), hepatitis C virus (HCV), human immunodeficiency virus (HIV), lymphocytic choriomeningitis virus (LCMV), or herpes simplex virus (HSV).

A T cell which is specific for an antigen of a virus may be referred to herein as a virus-specific T cell (VST). VSTs may be CD4+ T cells (e.g. T_{H} cells) and/or CD8+ T cells (e.g. CTLs). A T cell which is specific for an antigen of a particular virus may be described as being being specific for the relevant virus; for example, a T cell which is specific for an antigen of an Adenovris may be referred to as an Adenovirus-specific T cell, or "AdVST". The use of virus-specific T cells for the generation of CAR-T cells is associated with the advantage that whilst naïve T cells may have limited long-term persistence after infusion, virus-specific T-cells (VSTs) derived from the memory compartment, and genetically-modified VSTs have been shown to persist for over 10 years after infusion in stem cell transplant recipients (Cruz et al., Cytotherapy (2010) 12:743-749). For example, VSTs expressing GD2.CARs have been shown to persist long-term after infusion and produce complete tumor responses in patients with low tumor burden (Sun et al., Journal for Immunotherapy of Cancer (2015) 3:5 and Pule et al., Nature Medicine (2008) 14: 1264-1270).

In some embodiments the cell comprising/expressing the CAR is a virus-specific T cell (VST, e.g. a virus-specific CD4+ T cell (e.g. T_{H} cell) and/or a virus-specific CD8+ T cell (e.g. CTL). In some embodiments the CAR-expressing cell is an Adenovirus-specific T cell (AdVST), Cytomegalovius-specific T cell (CMVST), Epstein-Barr virus-specific T cell (EBVST), influenza virus-specific T cell, measles virus-specific T cell, hepatitis B virus-specific T cell (HBVST), hepatitis C virus-specific T cell (HCVST), human immunodeficiency virus-specific T cell (HIVST), lymphocytic choriomeningitis virus-specific T cell (LCMVST), Herpes simplex virus-specific T cell (HSVST) or human papilloma virus (HPVST).

In some embodiments the cell comprising/expressing the CAR is an oncolytic virus-specific immune cell (e.g. an oncolytic virus-specific T cell), e.g. as described herein.

Any cells of the disclosure may be included in an isolated population of cells that may or may not be homogeneous. In specific embodiments, the cell population has a majority of cells that are immune cells specific for an oncolytic virus and/or that express a CAR. The cells in the cell population may comprise an oncolytic adenovirus (OncAd), a helper-dependent adenovirus (HDAd), a chimeric antigen receptor (CAR) and/or nucleic acid or plurality of nucleic acids that encodes one or more of the OncAd, HDAd, and/or CAR. In particular embodiments, the cell population has at least 70, 75, 80, 85, 90, 91, 92, 93, 94, 95, 96, 97, 98, or 99% of cells that comprise an oncolytic adenovirus (OncAd), a helper-dependent adenovirus (HDAd), a chimeric antigen receptor (CAR) and/or nucleic acid or plurality of nucleic acids that encodes one or more of the OncAd, HDAd, and/or CAR.

### Additional Sequences

The polypeptides of the present disclosure (i.e. the antigen-binding molecules (e.g. BiTEs), CARs, immunomodulatory factors, fusion proteins) may additionally comprise further amino acids or sequences of amino acids, e.g. to facilitate expression, folding, trafficking, processing, purification and/or detection.

The polypeptides of the present disclosure may additionally comprise a signal peptide (also known as a leader sequence or signal sequence). Signal peptides normally consist of a sequence of 5-30 hydrophobic amino acids, which form a single alpha helix. Secreted proteins and proteins expressed at the cell surface often comprise signal peptides. The signal peptide may be present at the N-terminus, and may be present in the newly synthesised polypeptide. Signal peptides are often removed by cleavage, and thus are not comprised in the mature polypeptide. Signal peptides are known for many proteins, and are recorded in databases such as GenBank, UniProt, Swiss-Prot, TrEMBL, Protein Information Resource, Protein Data Bank, Ensembl, and InterPro, and/or can be identified/predicted e.g. using amino acid sequence analysis tools such as SignalP (Petersen et al., 2011 Nature Methods 8: 785-786) or Signal-BLAST (Frank and Sippl, 2008 Bioinformatics 24: 2172-2176. In some embodiments a polypeptide according to the present disclosure comprises an amino acid sequence having at least 85%, 90%, 91%, 92%, 93%, 94%, 95%, 96%, 97%, 98%, 99% or 100% sequence identity to SEQ ID NO:106, 107 or 108.

The polypeptides of the present disclosure may comprise one or more linker sequences (e.g. flexible linker sequences), e.g. as described hereinabove. In some embodiments a polypeptide according to the present disclosure comprises an amino acid sequence having at least 85%, 90%, 91%, 92%, 93%, 94%, 95%, 96%, 97%, 98%, 99% or 100% sequence identity to SEQ ID NO:109, 110, 111 or 112.

The polypeptides of the present disclosure may comprise one or more detectable moieties. A detectable moiety may be e.g. a fluorescent, lunminescent, immuno-detectable, radio, chemical, nucleic acid or enzymatic moiety. In some embodiments the polypeptides comprise a sequence encoding a HA, His, (e.g. 6XHis), Myc, GST, MBP, FLAG, E, or Biotin tag, optionally at the N- or C- terminus of the polypeptide. In some embodiments a polypeptide according to the present disclosure comprises an amino acid sequence having at least 85%, 90%, 91%, 92%, 93%, 94%, 95%, 96%, 97%, 98%, 99% or 100% sequence identity to SEQ ID NO:113 or 114.

The polypeptide(s) of the present disclosure may comprise one or more cleavable linker sequences. That is, the polypeptide(s) may comprise sequence(s) of amino acids which are capable of being cleaved. For example, a cleavable linker sequence may comprise a sequence capable of acting as a substrate for an enzyme capable of cleaving peptide bonds - i.e. a cleavage site. Many such cleavage sites are known to and can be employed by the person skilled in the art of molecular biology. In some embodiments, a cleavable linker sequence may comprise an autocleavage site. Autocleavage sites are automatically cleaved without the need for treatment with enzymes. An example of an autocleavage site is an amino acid sequence conforming to the 2A cleavage sequence consensus shown in SEQ ID NO:116, which is cleaved at "G/P". SEQ ID NO:115 is an example of cleavable linker sequence comprising a the 2A cleavage sequence conforming to the 2A cleavage sequence consensus shown in SEQ ID NO:116. In some embodiments a polypeptide according to the present disclosure comprises an amino acid sequence having at least 85%, 90%, 91%, 92%, 93%, 94%, 95%, 96%, 97%, 98%, 99% or 100% sequence identity to SEQ ID NO:115 or 116.

### Oncolytic virus-specific immune cells

Aspects of the present disclosure employ oncolytic virus-specific immune cells (also referred to herein as immune cells specific for an oncolytic virus). Oncolytic virus-specific immune cells express/comprise a receptor capable of recognising a peptide of an antigen of an oncolytic virus (e.g. when presented by an MHC molecule). The immune cell may express/comprise such a receptor as a result of expression of endogenous nucleic acid encoding such antigen receptor, or as a result of having been engineered to express such a receptor.

In some embodiments an oncolytic virus-specific immune cell may be a cell of hematopoietic origin, *e.g.* a neutrophil, eosinophil, basophil, dendritic cell, lymphocyte, or monocyte. The lymphocyte may be *e.g.* a T cell, B cell, NK cell, NKT cell or innate lymphoid cell (ILC), or a precursor thereof. The cell may express *e.g.* CD3 polypeptides (*e.g.* CD3γ CD3ε CD3ζ or CD3δ), TCR polypeptides (TCRα or TCRβ), CD27, CD28, CD4 or CD8. In some embodiments, the oncolytic virus-specific immune cell is a T cell, e.g. a CD3+ T cell. In some embodiments, the T cell is a CD3+, CD4+ T cell. In some embodiments, the T cell is a CD3+, CD8+ T cell. In some embodiments, the T cell is a T helper cell (T_{H} cell)). In some embodiments, the T cell is a cytotoxic T cell (e.g. a cytotoxic T lymphocyte (CTL)).

The oncolytic virus-specific immune cell (e.g. oncolytic virus-specific T cell) may be specific for an oncolytic virus as described herein. That is to say, the oncolytic virus-specific immune cell may be specific for one or more antigens of an oncolytic virus described herein.

Methods for generating/expanding populations of immune cells specific for antigen(s) of interest and/or a virus of interest are well known in the art, and are described e.g. in Wang and Rivière Cancer Gene Ther. (2015) 22(2):85-94, which is hereby incorporated by reference in its entirety.

Such methods may involve contacting heterogeneous populations of immune cells (e.g. peripheral blood mononuclear cells (PBMCs), peripheral blood lymphocytes (PBLs) tumor-infiltrating lymphocytes (TILs)) with one or more peptides of the antigen(s) of interest, or cells comprising/expressing the antigen(s)/peptides. Cells comprising/expressing the antigen(s)/peptides may do so as a consequence of infection with the virus comprising/encoding the antigen(s), uptake by the cell of the antigen(s)/peptides thereof or expression of the antigen(s)/peptides thereof. The presentation is typically in the context of an MHC molecule at the cell surface of the antigen-presenting cell.

Cells comprising/expressing the antigen(s)/peptides may have been contacted ("pulsed") with peptides of the antigen(s) according to methods well known to the skilled person. Antigenic peptides may be provided in a library of peptide mixtures (corresponding to one or more antigens), which may be referred to as pepmixes. Peptides of pepmixes may e.g. be overlapping peptides of 8-20 amino acids in length, and may cover all or part of the amino acid sequence of the relevant antigen.

Cells within the population of immune cells comprising receptors specific for the peptide(s) may be activated (and stimulated to proliferate), following recognition of peptide(s) of the antigen(s) presented by antigen-presenting cells (APCs) in the context of appropriate costimulatory signals. It will be appreciated that "an immune cell specific for an oncolytic virus" encompasses plural cells, e.g. populations of such cells. Such populations may be generated/expanded *in vitro* and/or *ex vivo.*

In some embodiments, an immune cell specific for an oncolytic virus is specific for an oncolytic adenovirus (OncAd), e.g. an OncAd as described herein. In some embodiments, an immune cell specific for an oncolytic virus is specific for an antigen of an OncAd. In some embodiments, the antigen is, or is derived from, an OncAd protein, e.g. a protein encoded by an early gene (e.g. E1 (e.g. E1A, E1B), E2 (e.g. E2A, E2B), E3 or E4), a protein encoded by a late gene (e.g. L1, L2, L3, L4 or L5), a protein

encoded by IX, or a protein encoded by IVa2. In some embodiments, the antigen is, or is derived from, an OncAd hexon and/or penton.

In some embodiments in accordance with various aspects of the present disclosure an immune cell specific for a virus may be generated/expanded (or may have been generated/expanded) by a method comprising: stimulating a population of immune cells by culture in the presence of antigen presenting cells (APCs) presenting a peptide of the virus.

In some embodiments an immune cell specific for an oncolytic virus according to the present disclosure is prepared by a method employing a PepMix comprising a mixture of overlappying peptides corresponding to Human Adenovirus 3 hexon and/or a PepMix comprising a mixture of overlappying peptides corresponding to Human Adenovirus 5 penton.

In some embodiments the oncolytic virus-specific immune cell expresses/comprises a CAR, e.g. a CAR as described herein. The oncolytic virus-specific immune cell may be engineered to express a CAR e.g. by transfection/transduction of the oncolytic virus-specific immune cell with nucleic acid encoding a CAR.

### Combinations of the disclosure

Aspects of the present invention include compositions and methods comprising/employing (i) a virus comprising nucleic acid encoding an antigen-binding molecule comprising: (a) an antigen-binding moiety specific for an immune cell surface molecule, and (b) an antigen-binding moiety specific for a cancer cell antigen; and (ii) an oncolytic virus. Also provided are compositions and methods comprising/employing (i) a virus comprising nucleic acid encoding an antigen-binding molecule comprising: (a) an antigen-binding moiety specific for an immune cell surface molecule, and (b) an antigen-binding moiety specific for a cancer cell antigen; and (ii) at least one cell comprising a chimeric antigen receptor (CAR) specific for a cancer cell antigen. Also provided are compositions and methods comprising/employing (i) a virus comprising nucleic acid encoding an antigen-binding molecule comprising: (a) an antigen-binding moiety specific for an immune cell surface molecule, and (b) an antigen-binding moiety specific for a cancer cell antigen; (ii) an oncolytic virus; and (iii) at least one cell comprising a chimeric antigen receptor (CAR) specific for a cancer cell antigen.

In some embodiments in accordance with various aspects described herein the cell comprising/expressing the CAR is specific for the oncolytic virus employed (e.g. comprises antigen receptor (e.g. TCR) specific for an antigen of the oncolytic virus). That is to say, in some embodiments the oncolytic virus and the specificity of the cell comprising/expressing the CAR are matched. By way of example, in some embodiments the oncolytic virus is an adenovirus, and the CAR-expressing cell comprising/expressing a CAR is an Adenovirus-specific T cell.

Similarly, in various aspects described herein an oncolytic virus is employed in combination with an immune cell specific for the oncolytic virus (i.e. the same oncolytic virus).

"Combinations" as referred to herein encompass products and compositions (e.g. pharmaceutical compositions) comprising the components of the combination. "Combinations" also encompass therapeutic regimens employing the components of the combination.

In some embodiments the components of a combination are provided in separate compositions. In some embodiments more than one component of a combination is provided in a composition. In some embodiments the components of a combination are provided in one composition.

Similarly, in some embodiments the components of a combination are administered separately. In some embodiments a component of a combination is administered with another component of the combination. In some embodiments the components of a combination are administered together.

By way of illustration, in the example of a combination comprising an oncolytic virus, a virus comprising nucleic acid encoding an antigen-binding molecule comprising: (a) an antigen-binding moiety specific for an immune cell surface molecule, and (b) an antigen-binding moiety specific for a cancer cell antigen, and at least one cell comprising a CAR specific for a cancer cell antigen, the oncolytic virus and the virus comprising nucleic acid encoding an antigen-binding molecule comprising: (a) an antigen-binding moiety specific for an immune cell surface molecule, and (b) an antigen-binding moiety specific for a cancer cell antigen may be administered together, and the at least one cell comprising a CAR specific for a cancer cell antigen may be administered separately (e.g. subsequently).

Where components of a combination are administered together administration may be simultaneous administration as described hereinbelow. Where components of a combination are administered separately, administration may be simultaneous administration or sequential administration, as described hereinbelow. In cases wherein components of a combination are administered separately, the administration of the separate components may or may not be administered *via* the same administration routes.

### Functional properties

The articles of the present disclosure (e.g. viruses (e.g. oncolytic viruses, HDAds), antigen-binding molecules (e.g. BiTEs), CARs, immunomodulatory factors (e.g. IL-12, anti-PD-L1 minibody), nucleic acids, cells, compositions and combinations) of the present disclosure may be defined by reference to one of more functional properties. The articles may be evaluated for the functional properties, for example, by analysis as described in the experimental examples.

In some embodiments, a virus comprising nucleic acid encoding an antigen-binding molecule comprising: (a) an antigen-binding moiety specific for an immune cell surface molecule, and (b) an antigen-binding moiety specific for a cancer cell antigen according to the present disclosure may possess one or more of the following functional properties:
- ability to cause/increase cell killing of cancer cells;
- reduced ability to cause/increase cell killing of non-cancerous cells as compared to the ability to cause/increase cell killing of cancer cells.

In some embodiments, an antigen-binding molecule comprising: (a) an antigen-binding moiety specific for an immune cell surface molecule, and (b) an antigen-binding moiety specific for a cancer cell antigen according to the present disclosure may possess one or more of the following functional properties:
- ability to bind to the cancer cell antigen for which the antigen-binding molecule comprise a specific antigen-binding moiety;
- ability to bind to cells expressing the cancer cell antigen for which the antigen-binding molecule comprise a specific antigen-binding moiety;
- ability to bind to the immune cell surface molecule for which the antigen-binding molecule comprise a specific antigen-binding moiety;
- ability to bind to cells expressing the immune cell surface molecule for which the antigen-binding molecule comprise a specific antigen-binding moiety;
- ability to cause/increase killing of cells expressing the cancer cell antigen for which the antigen-binding molecule comprise a specific antigen-binding moiety, e.g. by immune cells expressing the immune cell surface molecule for which the antigen-binding molecule comprise a specific antigen-binding moiety.

In some embodiments, an oncolytic virus according to the present disclosure may possess one or more of the following functional properties:
- ability to replicate in, and/or cause cell killing of, cancer cells;
- reduced ability to replicate in and/or cause cell killing of, non-cancerous cells as compared to the ability to replicate in, and/or cause cell killing of, cancer cells;
- comparable or improved ability to cause cell killing of cancer cells as compared to the ability of one or more oncolytic viruses known in the art;
- ability to help replication of helper-dependent adenovirus (HDAd);
- comparable or improved ability to replicate in cancer cells as compared to the ability of one or more oncolytic viruses known in the art.

In some embodiments, a cell comprising a chimeric antigen receptor (CAR) specific for a cancer cell antigen according to the present disclosure may possess one or more of the following functional properties:
- ability to bind to the cancer cell antigen for which the CAR is specific;
- ability to bind to cells expressing the cancer cell antigen for which the CAR is specific;
- ability to cause cell killing of cells expressing the cancer cell antigen for which the CAR is specific;
- reduced ability to cause cell killing of cells not expressing the cancer cell antigen for which the CAR is specific as compared to the ability to cause cell killing of cells expressing the cancer cell antigen for which the CAR is specific.

In some embodiments the combination of a virus comprising nucleic acid encoding an antigen-binding molecule comprising: (a) an antigen-binding moiety specific for an immune cell surface molecule, and (b) an antigen-binding moiety specific for a cancer cell antigen and an oncolytic virus may possess one or more of the following functional properties:
- improved ability to cause cell killing of cancer cells as compared to the ability to cause cell killing of cancer cells by either component used alone.
- ability to cause cell killing of cancer cells which is synergistic (i.e. super-additive) as compared to the ability to cause cell killing of cancer cells by the components used alone.

In some embodiments the combination of a virus comprising nucleic acid encoding an antigen-binding molecule comprising: (a) an antigen-binding moiety specific for an immune cell surface molecule, and (b) an antigen-binding moiety specific for a cancer cell antigen and at least one cell comprising a CAR specific for a cancer cell antigen may possess one or more of the following functional properties:
- improved ability to cause cell killing of cancer cells as compared to the ability to cause cell killing of cancer cells by either component used alone.
- ability to cause cell killing of cancer cells which is synergistic (i.e. super-additive) as compared to the ability to cause cell killing of cancer cells by the components used alone.

In some embodiments the combination of a virus comprising nucleic acid encoding an antigen-binding molecule comprising: (a) an antigen-binding moiety specific for an immune cell surface molecule, and (b) an antigen-binding moiety specific for a cancer cell antigen, an oncolytic virus and at least one cell comprising a CAR specific for a cancer cell antigen may possess one or more of the following functional properties:
- improved ability to cause cell killing of cancer cells as compared to the ability to cause cell killing of cancer cells by any one of the components use alone, or by any two of the components used in combination.
- ability to cause cell killing of cancer cells which is synergistic (i.e. super-additive) as compared to the ability to cause cell killing of cancer cells by the components used alone.

Analysis of the ability to cause cell killing of cancer cells may be assessed e.g. *in vitro,* by analysis of number/viability of cancer cells. Analysis of the ability to cause cell killing of cancer cells may also be analysed *in vivo* in an appropriate model, e.g. by analysis of number of cancer cells, tumor size/volume and/or some other correlate of the number of cancer cells (e.g. disease progression, severity of symptoms of the cancer etc.).

### Articles of the disclosure, compositions and kits

The present disclosure also provides a virus comprising nucleic acid encoding an antigen-binding molecule comprising: (a) an antigen-binding moiety specific for an immune cell surface molecule, and (b) an antigen-binding moiety specific for a cancer cell antigen, optionally isolated. Also provided is a nucleic acid encoding the virus, optionally isolated. Also provided is a cell comprising the virus, or comprising nucleic acid encoding the virus, optionally isolated. Also provided is a composition comprising the cell, nucleic acid or virus.

The present disclosure also provides an oncolytic virus according to the present disclosure, optionally isolated. Also provided is a nucleic acid encoding the oncolytic virus, optionally isolated. Also provided is a cell comprising the oncolytic virus, or comprising nucleic acid encoding the oncolytic virus, optionally isolated. Also provided is a composition comprising the cell, nucleic acid or oncolytic virus.

The present disclosure also provides an antigen-binding molecule comprising: (a) an antigen-binding moiety specific for an immune cell surface molecule, and (b) an antigen-binding moiety specific for a cancer cell antigen, optionally isolated. Also provided is a nucleic acid encoding the antigen-binding molecule, optionally isolated. Also provided is a cell comprising the antigen-binding molecule, or comprising nucleic acid encoding the antigen-binding molecule, optionally isolated. Also provided is a composition comprising the cell, nucleic acid or antigen-binding molecule.

The present disclosure also provides a chimeric antigen receptor (CAR) as described herein, optionally isolated. Also provided is a nucleic acid encoding the CAR, optionally isolated. Also provided is a cell comprising the CAR, or comprising nucleic acid encoding the CAR, optionally isolated. Also provided is a composition comprising the cell, nucleic acid or CAR.

The present disclosure also provides an immunomodulatory factor (e.g. IL-12, anti-PD-L1 minibody), optionally isolated. Also provided is a nucleic acid encoding the immunomodulatory factor, optionally isolated. Also provided is a cell comprising the immunomodulatory factor, or comprising nucleic acid encoding the immunomodulatory factor, optionally isolated. Also provided is a composition comprising the cell, nucleic acid or immunomodulatory factor.

The virus (e.g. oncolytic virus, HDAd), antigen-binding molecule (e.g. BiTE), CAR, immunomodulatory factor (e.g. IL-12, anti-PD-L1 minibody), nucleic acid/plurality, cell/plurality, or combination according to the present disclosure may be formulated as pharmaceutical compositions for clinical use and may comprise a pharmaceutically acceptable carrier, diluent, excipient or adjuvant. Combinations of the present disclosure may be provided as a single composition, or may be provided as plural compositions comprising the components of the combination.

In accordance with the present disclosure methods are also provided for the production of pharmaceutically useful compositions, such methods of production may comprise one or more steps selected from: isolating a virus (e.g. oncolytic virus, HDAd), antigen-binding molecule (e.g. BiTE), CAR, immunomodulatory factor (e.g. IL-12, anti-PD-L1 minibody), nucleic acid/plurality, cell/plurality, composition or combination as described herein; and/or mixing a virus (e.g. oncolytic virus, HDAd), antigen-binding molecule (e.g. BiTE), CAR, immunomodulatory factor (e.g. IL-12, anti-PD-L1 minibody), nucleic acid/plurality, cell/plurality, composition or combination as described herein with a pharmaceutically acceptable carrier, adjuvant, excipient or diluent.

For example, a further aspect of the present disclosure relates to a method of formulating or producing a medicament or pharmaceutical composition for use in the treatment of a cancer, the method comprising formulating a pharmaceutical composition or medicament by mixing a virus (e.g. oncolytic virus, HDAd), antigen-binding molecule (e.g. BiTE), CAR, immunomodulatory factor (e.g. IL-12, anti-PD-L1 minibody), nucleic acid/plurality, cell/plurality, composition or combination as described herein with a pharmaceutically acceptable carrier, adjuvant, excipient or diluent.

The present disclosure also provides a kit of parts comprising one or more of a virus (e.g. oncolytic virus, HDAd), antigen-binding molecule (e.g. BiTE), CAR, immunomodulatory factor (e.g. IL-12, anti-PD-L1 minibody), nucleic acid/plurality, cell/plurality, composition or combination according to the present disclosure. In some embodiments the kit may have at least one container having a predetermined quantity of an article of the present disclosure. The kit may have containers containing individual components of the combinations of the present disclosure, or may have containers containing combinations of the components of the combinations of the present disclosure.

The kit may be provided with instructions for administration to a patient in order to treat a specified cancer. The article(s) may be formulated so as to be suitable for injection or infusion to a tumor or to the blood.

In some embodiments the kit may comprise materials for producing a cell according to the present disclosure. For example, the kit may comprise materials for modifying a cell to express or comprise a virus or an antigen/peptide thereof, CAR or nucleic acid/plurality of nucleic acids according to the present disclosure, or materials for introducing into a cell the virus or an antigen/peptide thereof or nucleic acid/plurality of nucleic acids according to the present disclosure. The kit may comprise materials for producing an immune cell specific for an oncolytic virus; for example, the kit may comprise pepmixes of one or more antigens of the oncolytic virus.

In some embodiments the kit may further comprise at least one container having a predetermined quantity of another therapeutic agent (*e.g.* anti-infective agent or chemotherapy agent). In such embodiments, the kit may also comprise a second medicament or pharmaceutical composition such that the two medicaments or pharmaceutical compositions may be administered simultaneously or separately such that they provide a combined treatment for the cancer. The therapeutic agent may also be formulated so as to be suitable for injection or infusion to a tumor or to the blood.

### Therapeutic applications

Aspects of the present disclosure are concerned with the use of (i) a virus comprising nucleic acid encoding an antigen-binding molecule comprising: (a) an antigen-binding moiety specific for an immune cell surface molecule, and (b) an antigen-binding moiety specific for a cancer cell antigen, (ii) an oncolytic virus, and/or (iii) at least one cell comprising a chimeric antigen receptor (CAR) specific for a cancer cell antigen, in the treatment of a cancer in a subject.

Accordingly, the present disclosure provides a method of treating a cancer, comprising administering to a subject: (i) a virus comprising nucleic acid encoding an antigen-binding molecule comprising: (a) an antigen-binding moiety specific for an immune cell surface molecule, and (b) an antigen-binding moiety specific for a cancer cell antigen, (ii) an oncolytic virus, and/or (iii) at least one cell comprising a chimeric antigen receptor (CAR) specific for a cancer cell antigen.

The present disclosure also provides (i) a virus comprising nucleic acid encoding an antigen-binding molecule comprising: (a) an antigen-binding moiety specific for an immune cell surface molecule, and (b) an antigen-binding moiety specific for a cancer cell antigen, (ii) an oncolytic virus, and/or (iii) at least one cell comprising a chimeric antigen receptor (CAR) specific for a cancer cell antigen, for use in a method of treating a cancer. Also provided is the use of (i) a virus comprising nucleic acid encoding an antigen-binding molecule comprising: (a) an antigen-binding moiety specific for an immune cell surface molecule, and (b) an antigen-binding moiety specific for a cancer cell antigen, (ii) an oncolytic virus, and/or (iii) at least one cell comprising a chimeric antigen receptor (CAR) specific for a cancer cell antigen; in the manufacture of a medicament for treating a cancer.

'Treatment' may, for example, be reduction in the development or progression of a cancer, alleviation of the symptoms of a cancer or reduction in the pathology of a cancer. Treatment or alleviation of a cancer may be effective to prevent progression of the cancer, *e.g.* to prevent worsening of the condition or to slow the rate of development of a more severe disease state. In some embodiments treatment or alleviation may lead to an improvement in the cancer, *e.g.* a reduction in the symptoms of the cancer or reduction in some other correlate of the severity/activity of the cancer. Prevention of a cancer may refer to prevention of a worsening of the condition or prevention of the development of the cancer, *e.g.* preventing an early stage cancer developing to a later stage.

In some embodiments, the treatment may be aimed at reducing the number of cells of the cancer or the amount of tissue comprising cancerous cells in the subject. In some embodiments, the treatment may be aimed at reducing the size of and/or preventing the growth of a tumor in the subject.

In some embodiments, the treatment comprises administering a virus comprising nucleic acid encoding an antigen-binding molecule comprising: (a) an antigen-binding moiety specific for an immune cell surface molecule, and (b) an antigen-binding moiety specific for a cancer cell antigen according to the present disclosure, to a subject. In some embodiments, the treatment comprises administering a cell or population of cells comprising or encoding a virus comprising nucleic acid encoding an antigen-binding molecule comprising: (a) an antigen-binding moiety specific for an immune cell surface molecule, and (b) an antigen-binding moiety specific for a cancer cell antigen according to the present disclosure, to a subject. In some embodiments, the treatment is aimed at providing the subject with an antigen-binding molecule comprising according to the present disclosure.

In some embodiments, the treatment comprises administering an oncolytic virus according to the present disclosure to a subject. In some embodiments, the treatment comprises administering a cell or population of cells comprising or encoding an oncolytic virus according to the present disclosure to a subject.

In some embodiments, the treatment comprises administering a cell comprising a CAR described herein to a subject. In some embodiments, the treatment may comprise modifying a cell or population of cells to comprise/express a CAR according to the present disclosure. In some embodiments, the treatment may comprise administering to a subject a cell or population of cells modified to comprise/express a CAR of the present disclosure. In some embodiments, the treatment is aimed at providing the subject with an immune cell or population of immune cells which having specificity for a cancer cell antigen, e.g. by administering a CAR-expressing cell according to the present disclosure, or generating a CAR-expressing cell according to the present disclosure.

In some embodiments, the treatment may comprise administering to a subject an immune cell/population of immune cells specific for an oncolytic virus according to the present disclosure. In some embodiments, the treatment is aimed at providing the subject with an immune cell/population of immune cells having specificity for an oncolytic virus. In some embodiments, the treatment may comprise generating/expanding a population of immune cells specific for an oncolytic virus according to the present disclosure.

In some embodiments, the treatment may comprise administering to a subject an immune cell/population of immune cells specific for an oncolytic virus according to the present disclosure, modified to comprise/express a CAR according to the present disclosure. In some embodiments, the treatment is aimed at providing the subject with an immune cell/population of immune cells having specificity for an oncolytic virus also having specificity for a cancer cell antigen. In some embodiments, the treatment may comprise generating/expanding a population of immune cells specific for an oncolytic virus according to the present disclosure, and modifying a cell or cells of the population to comprise/express a CAR according to the present disclosure.

The subject to be treated may be any animal or human. The subject is preferably mammalian, more preferably human. The subject may be a non-human mammal, but is more preferably human. The subject may be male or female or of any gender. The subject may be a patient. A subject may have been diagnosed with a cancer requiring treatment, may be suspected of having such a cancer, or may be at risk of developing such a cancer.

In some embodiments, the cancer to be treated comprises cells expressing a cancer cell antigen, e.g. a cancer cell antigen as described herein. In some embodiments, the cells express the cancer cell antigen at the cell surface. In some embodiments, the cancer to be treated is or comprises a tumor comprising cells expressing a cancer cell antigen, e.g. a cancer cell antigen as described herein.

In some embodiments, the cancer to be treated comprises cells expressing a cancer cell antigen for which the CAR comprises a specific antigen-binding moiety. In some embodiments, the cancer to be treated comprises cells expressing a cancer cell antigen for which the antigen-binding molecule comprises a specific antigen-binding moiety.

In embodiments wherein the methods employ a virus encoding an antigen-binding molecule comprising an antigen-binding moiety specific for a cancer cell antigen and a CAR specific for a non-identical cancer cell antigen, the cancer to be treated may comprise cells expressing both of the cancer cell antigens.

In some embodiments, the cancer over-expresses the cancer cell antigen. Overexpression of a cancer cell antigen can be determined by detection of a level of expression of the cancer cell antigen which is greater than the level of expression by equivalent non-cancerous cells/non-tumor tissue. In some embodiments the cancer is a cancer expressing CD44v6, *e.g.* a cancer expressing CD44v6 at the cell surface. In some embodiments the cancer is selected from head and neck squamous cell carcinoma (HNSCC), prostate cancer, pancreatic cancer, breast cancer, colon cancer, gastric carcinoma, ovarian cancer, acute myeloid leukemia and multiple myeloma.

In some embodiments the cancer is a cancer expressing HER2, *e.g.* a cancer expressing HER2 at the cell surface. In some embodiments, the cancer over-expresses HER2. Overexpression of HER2 can be determined by detection of a level of expression of HER2 which is greater than the level of expression of HER2 by equivalent non-cancerous cells/non-tumor tissue. In some embodiments the cancer is selected from breast cancer, ovarian cancer, bladder cancer, salivary gland cancer, endometrial cancer, pancreatic cancer and non-small-cell lung cancer (NSCLC).

In some embodiments the cancer is a cancer expressing CD19, *e.g.* a cancer expressing CD19 at the cell surface. In some embodiments the cancer is selected from B cell lymphoma, acute lymphoblastic leukemia (ALL), and chronic lymphocytic leukemia (CLL).

In some embodiments, the subject to be treated according to the present disclosure is selected for treatment on the basis detection of expression/overexpression of the cancer cell antigen by a cancer cell or tumour obtained from the subject.

Expression of a given cancer cell antigen may be determined by any suitable means. Expression may be gene expression or protein expression. Gene expression can be determined e.g. by detection of mRNA encoding the cancer cell antigen, for example by quantitative real-time PCR (qRT-PCR). Protein expression can be determined e.g. by detection of the cancer cell antigen, for example by antibody-based methods, for example by western blot, immunohistochemistry, immunocytochemistry, flow cytometry, or ELISA.

The cancer to be treated/prevented in accordance with the present disclosure may be any unwanted cell proliferation (or any disease manifesting itself by unwanted cell proliferation), neoplasm or tumor. The cancer may be benign or malignant and may be primary or secondary (metastatic). The cancer may be resistant (initially or following treatment) and/or the cancer may be recurring. A neoplasm or tumor may be any abnormal growth or proliferation of cells and may be located in any tissue. The cancer may be of tissues/cells derived from *e.g.* the adrenal gland, adrenal medulla, anus, appendix, bladder, blood, bone, bone marrow, brain, breast, cecum, central nervous system (including or excluding the brain) cerebellum, cervix, colon, duodenum, endometrium, epithelial cells (*e.g.* renal epithelia), gallbladder, oesophagus, glial cells, heart, ileum, jejunum, kidney, lacrimal glad, larynx, liver, lung, lymph, lymph node, lymphoblast, maxilla, mediastinum, mesentery, myometrium, nasopharynx, omentum, oral cavity, ovary, pancreas, parotid gland, peripheral nervous system, peritoneum, pleura, prostate, salivary gland, sigmoid colon, skin, small intestine, soft tissues, spleen, stomach, testis, thymus, thyroid gland, tongue, tonsil, trachea, uterus, vulva, white blood cells.

The cancer to be treated/prevented may be any kind of cancer, including any one of an acute lymphoblastic leukemia (ALL), acute myeloid leukemia (AML), adrenocortical carcinoma, AIDS-related cancer (*e.g.* Kaposi sarcoma, AIDS-related lymphoma, primary CNS lymphoma), anal cancer, appendix cancer, astrocytoma, basal cell carcinoma of the skin, bile duct cancer (*e.g.* cholangiocarcinoma), bladder cancer, bone cancer (*e.g.* Ewing sarcoma, osteosarcoma, malignant fibrous histiocytoma), brain tumor, breast cancer, bronchial tumor, Burkitt lymphoma, carcinoid tumor, carcinoma of unknown primary, cardiac tumor, central nervous system cancer (*e.g.* atypical teratoid/rhabdoid tumor, embryonal tumor, germ cell tumor, primary CNS lymphoma), cervical cancer, chordoma, chronic lymphocytic leukemia (CLL), chronic myelogenous leukemia (CML), chronic myeloproliferative neoplasm, colorectal cancer, craniopharyngioma, cutaneous T-cell lymphoma (*e.g.* mycosis fungoides, Sézary syndrome), ductal carcinoma in situ (DCIS), endometrial cancer (uterine cancer), ependymoma, esophageal cancer, esthesioneuroblastoma, extracranial germ cell tumor, extragonadal germ cell tumor, eye cancer (*e.g.* intraocular melanoma, retinoblastoma) fallopian tube cancer, malignant fibrous histiocytoma of bone, gallbladder cancer, gastric (stomach) cancer, gastrointestinal carcinoid tumor, gastrointestinal stromal tumor (GIST), ovarian germ cell tumor, testicular cancer, gestational trophoblastic disease, hairy cell leukemia, head and neck cancer, heart tumor, hepatocellular (liver) cancer, histiocytosis, Langerhans cell, Hodgkin lymphoma, hypopharyngeal cancer, islet cell tumor (pancreatic neuroendocrine tumor), kidney (renal cell) cancer, laryngeal cancer, papillomatosis, leukemia, lip and oral cavity cancer, lung cancer (non-small cell lung cancer (NSCLC) and small cell lung cancer (SCLC)) lymphoma, male breast cancer, melanoma, Merkel cell carcinoma, mesothelioma, metastatic cancer, metastatic squamous neck cancer with occult primary, midline tract carcinoma involving NUT gene, mouth cancer, multiple endocrine neoplasia syndromes, multiple myeloma/plasma cell neoplasms, mycosis fungoides, myelodysplastic syndrome, myelodysplastic/myeloproliferative neoplasm, myelogenous leukemia, chronic myeloid leukemia, acute myeloid leukemia (AML), nasal cavity and paranasal sinus cancer, nasopharyngeal cancer, neuroblastoma, non-Hodgkin lymphoma, oral cancer, lip and oral cavity cancer, oropharyngeal cancer, osteosarcoma, ovarian cancer, pancreatic cancer, papillomatosis, paraganglioma, paranasal sinus cancer, nasal cavity cancer, parathyroid cancer, penile cancer, pharyngeal cancer, pheochromocytoma, pituitary tumor, plasma cell neoplasm/multiple myeloma, pleuropulmonary blastoma, pregnancy and breast cancer, primary peritoneal cancer, prostate cancer, rectal cancer, recurrent cancer, retinoblastoma, rhabdomyosarcoma, salivary gland cancer, vascular tumor, uterine sarcoma, skin cancer, small intestine cancer, squamous cell carcinoma of the skin, T-cell lymphoma, throat cancer, thymoma, thymic carcinoma, thyroid cancer, transitional cell cancer of the renal pelvis and ureter, urethral cancer, vaginal cancer, vulvar cancer or Wilms tumor.

In some embodiments the cancer is associated with, or caused by, a virus (e.g. Epstein-Barr Vius, human papillomavirus (HPV), hepatitis B virus (HBV), etc.). In some embodiments the cancer is an EBV-positive cancer. In some embodiments the cancer is an HPV-positive cancer. In some embodiments the cancer is an HBV-positive cancer.

In some embodiments the cancer is selected from head and neck squamous cell carcinoma (HNSCC), prostate cancer, pancreatic cancer, breast cancer, colon cancer, gastric carcinoma, ovarian cancer, acute myeloid leukemia and multiple myeloma, bladder cancer, salivary gland cancer, endometrial cancer, non-small-cell lung cancer (NSCLC), B cell lymphoma, acute lymphoblastic leukemia (ALL), and chronic lymphocytic leukemia (CLL).

In some embodiments the cancer is head and neck cancer (e.g. a cancer originating from tissues of the lip, mouth, nose, sinuses, pharynx or larynx), head and neck squamous cell carcinoma (HNSCC), nasopharyngeal carcinoma (NPC; e.g. EBV-positive NPC), oropharyngeal carcinoma (OPC; e.g. HPV-positive OPC), prostate carcinoma, pancreatic carcinoma, cervical carcinoma (e.g. HPV-positive CC), gastric carcinoma (GC; EBV-positive GC), hepatocellular carcinoma (HCC; e.g. HBV-positive HCC), osteosarcoma (OS), ovarian cancer, colorectal cancer, breast cancer (e.g. HER2-positive breast cancer), or lung cancer (e.g. non-small cell lung cancer (NSCLC)).

Methods of medical treatment may also involve *in vivo, ex vivo,* and adoptive immunotherapies, including those using autologous and/or heterologous cells or immortalized cell lines.

### Administration

Administration is preferably in a "therapeutically effective amount", this being sufficient to show benefit to the individual. The actual amount administered, and rate and time-course of administration, will depend on the nature and severity of the disease being treated. Prescription of treatment, *e.g.* decisions on dosage *etc.,* is within the responsibility of general practitioners and other medical doctors, and typically takes account of the condition to be treated, the condition of the individual patient, the site of delivery, the method of administration and other factors known to practitioners. Examples of the techniques and protocols mentioned above can be found in Remington's Pharmaceutical Sciences, 20th Edition, 2000, pub. Lippincott, Williams & Wilkins.

Articles according to the present disclosure (e.g. viruses (e.g. oncolytic viruses, HDAds), antigen-binding molecules (e.g. BiTEs), CARs, immunomodulatory factors (e.g. IL-12, anti-PD-L1 minibody), nucleic acids and cells) may be formulated as pharmaceutical compositions or medicaments for clinical use and may comprise a pharmaceutically acceptable carrier, diluent, excipient or adjuvant. Compositions may be formulated for topical, parenteral, systemic, intracavitary, intravenous, intra-arterial, intramuscular, intrathecal, intraocular, intraconjunctival, intratumoral, subcutaneous, intradermal, intrathecal, oral or transdermal routes of administration which may include injection or infusion. Suitable formulations may comprise the viruses, antigen-binding molecules, CARs, immunomodulatory factors, nucleic acids, or cells in sterile or isotonic medium. Medicaments and pharmaceutical compositions may be formulated in fluid, including gel, form. Fluid formulations may be formulated for administration by injection or infusion (*e.g.* via catheter) to a selected region of the human or animal body.

In some embodiments the viruses, antigen-binding molecules, CARs, immunomodulatory factors, nucleic acids, cells and/or compositions according to the present disclosure may be formulated for intratumoral administration, or for intravenous administration.

Administration of the components of combinations of the present disclosure may be simultaneous or sequential. The present disclosure also contemplates simultaneous or sequential administration of the components of combinations of the present disclosure.

Simultaneous administration refers to administration of the agents together, for example as a pharmaceutical composition containing the agents (i.e. a combined preparation), or immediately after each other and optionally via the same route of administration, e.g. to the same artery, vein or other blood vessel. In particular embodiments, the virus comprising nucleic acid encoding an antigen-binding molecule comprising: (a) an antigen-binding moiety specific for an immune cell surface molecule and an oncolytic virus may be administered simultaneously in a combined preparation. In some embodiments upon simultaneous administration two or more of the agents may be administered via different routes of administration. In some embodiments simultaneous administration refers to administration at the same time, or within e.g. 1 hr, 2 hrs, 3 hrs, 4 hrs, 5 hrs, 6 hrs, 8 hrs, 12 hrs, 24 hrs, 36 hrs or 48 hrs.

Sequential administration refers to administration of one or more of the agents followed after a given time interval by separate administration of another of the agents. It is not required that the two agents are administered by the same route, although this is the case in some embodiments. The time interval may be any time interval, including hours, days, weeks, months, or years. In some embodiments sequential administration refers to administrations separated by a time interval of one of at least 10 min, 30 min, 1 hr, 6 hrs, 8 hrs, 12 hrs, 24 hrs, 36 hrs, 48 hrs, 3 days, 4 days, 5 days, 6 days, 1 week, 2 weeks, 3 weeks, 1 month, 6 weeks, 2 months, 3 months, 4 months, 5 months or 6 months.

In some embodiments, the treatment may further comprise other therapeutic or prophylactic intervention, *e.g.* chemotherapy, immunotherapy, radiotherapy, surgery, vaccination and/or hormone therapy. Such other therapeutic or prophylactic intervention may occur before, during and/or after the therapies encompassed by the disclosure, and the deliveries of the other therapeutic or prophylactic interventions may occur via different administration routes as the therapies of the disclosure. Chemotherapy and radiotherapy respectively refer to treatment of a cancer with a drug or with ionising radiation (*e.g.* radiotherapy using X-rays or γ-rays). The drug may be a chemical entity, *e.g.* small molecule pharmaceutical, antibiotic, DNA intercalator, protein inhibitor (*e.g.* kinase inhibitor), or a biological agent, *e.g.* antibody, antibody fragment, nucleic acid or peptide aptamer, nucleic acid (*e.g.* DNA, RNA), peptide, polypeptide, or protein. The drug may be formulated as a pharmaceutical composition or medicament. The formulation may comprise one or more drugs (*e.g.* one or more active agents) together with one or more pharmaceutically acceptable diluents, excipients or carriers.

The chemotherapy may be administered by one or more routes of administration, *e.g.* parenteral, intravenous injection, oral, subcutaneous, intradermal or intratumoral.

The chemotherapy may be administered according to a treatment regime. The treatment regime may be a pre-determined timetable, plan, scheme or schedule of chemotherapy administration which may be prepared by a physician or medical practitioner and may be tailored to suit the patient requiring treatment.

The treatment regime may indicate one or more of: the type of chemotherapy to administer to the patient; the dose of each drug or radiation; the time interval between administrations; the length of each treatment; the number and nature of any treatment holidays, if any *etc.* For a co-therapy a single treatment regime may be provided which indicates how each drug is to be administered.

Chemotherapeutic drugs and biologics may be selected from: alkylating agents such as cisplatin, carboplatin, mechlorethamine, cyclophosphamide, chlorambucil, ifosfamide; purine or pyrimidine antimetabolites such as azathiopurine or mercaptopurine; alkaloids and terpenoids, such as vinca alkaloids (*e.g.* vincristine, vinblastine, vinorelbine, vindesine), podophyllotoxin, etoposide, teniposide, taxanes such as paclitaxel (TaxolTM), docetaxel; topoisomerase inhibitors such as the type I topoisomerase inhibitors camptothecins irinotecan and topotecan, or the type II topoisomerase inhibitors amsacrine, etoposide, etoposide phosphate, teniposide; antitumor antibiotics (*e.g.* anthracyline antibiotics) such as dactinomycin, doxorubicin (AdriamycinTM), epirubicin, bleomycin, rapamycin; antibody based agents, such as anti-PD-1 antibodies, anti-PD-L1 antibodies, anti-TIM-3 antibodies, anti-CTLA-4, anti-4-1BB, anti-GITR, anti-CD27, anti-BLTA, anti-OX43, anti-VEGF, anti-TNFα, anti-IL-2, antiGpIIb/IIIa, anti-CD-52, anti-CD20, anti-RSV, anti-HER2/neu(erbB2), anti-TNF receptor, anti-EGFR antibodies, monoclonal antibodies or antibody fragments, examples include: cetuximab, panitumumab, infliximab, basiliximab, bevacizumab (Avastin^{®}), abciximab, daclizumab, gemtuzumab, alemtuzumab, rituximab (Mabthera^{®}), palivizumab, trastuzumab, etanercept, adalimumab, nimotuzumab; EGFR inihibitors such as erlotinib, cetuximab and gefitinib; anti-angiogenic agents such as bevacizumab (Avastin^{®}); cancer vaccines such as Sipuleucel-T (Provenge^{®}).

Further chemotherapeutic drugs may be selected from: 13-cis-Retinoic Acid, 2-Chlorodeoxyadenosine, 5-Azacitidine 5-Fluorouracil, 6-Mercaptopurine, 6-Thioguanine, Abraxane, Accutane^{®}, Actinomycin-D Adriamycin^{®}, Adrucil^{®}, Afinitor^{®}, Agrylin^{®}, Ala-Cort^{®}, Aldesleukin, Alemtuzumab, ALIMTA, Alitretinoin, Alkaban-AQ^{®}, Alkeran^{®}, All-transretinoic Acid, Alpha Interferon, Altretamine, Amethopterin, Amifostine, Aminoglutethimide, Anagrelide, Anandron^{®}, Anastrozole, Arabinosylcytosine, Aranesp^{®}, Aredia^{®}, Arimidex^{®}, Aromasin^{®}, Arranon^{®}, Arsenic Trioxide, Asparaginase, ATRA Avastin^{®}, Azacitidine, BCG, BCNU, Bendamustine, Bevacizumab, Bexarotene, BEXXAR^{®}, Bicalutamide, BiCNU, Blenoxane^{®}, Bleomycin, Bortezomib, Busulfan, Busulfex^{®}, Calcium Leucovorin, Campath^{®}, Camptosar^{®}, Camptothecin-11, Capecitabine, Carac^{™}, Carboplatin, Carmustine, Casodex^{®}, CC-5013, CCI-779, CCNU, CDDP, CeeNU, Cerubidine^{®}, Cetuximab, Chlorambucil, Cisplatin, Citrovorum Factor, Cladribine, Cortisone, Cosmegen^{®}, CPT-11, Cyclophosphamide, Cytadren^{®}, Cytarabine Cytosar-U^{®}, Cytoxan^{®}, Dacogen, Dactinomycin, Darbepoetin Alfa, Dasatinib, Daunomycin, Daunorubicin, Daunorubicin Hydrochloride, Daunorubicin Liposomal, DaunoXome^{®}, Decadron, Decitabine, Delta-Cortef^{®}, Deltasone^{®}, Denileukin, Diftitox, DepoCyt^{™}, Dexamethasone, Dexamethasone Acetate, Dexamethasone Sodium Phosphate, Dexasone, Dexrazoxane, DHAD, DIC, Diodex, Docetaxel, Doxil^{®}, Doxorubicin, Doxorubicin Liposomal, Droxia^{™}, DTIC, DTIC-Dome^{®}, Duralone^{®}, Eligard^{™}, Ellence^{™}, Eloxatin^{™}, Elspar^{®}, Emcyt^{®}, Epirubicin, Epoetin Alfa, Erbitux, Erlotinib, Erwinia L-asparaginase, Estramustine, Ethyol Etopophos^{®}, Etoposide, Etoposide Phosphate, Eulexin^{®}, Everolimus, Evista^{®}, Exemestane, Faslodex^{®}, Femara^{®}, Filgrastim, Floxuridine, Fludara^{®}, Fludarabine, Fluoroplex^{®}, Fluorouracil, Fluoxymesterone, Flutamide, Folinic Acid, FUDR^{®}, Fulvestrant, Gefitinib, Gemcitabine, Gemtuzumab ozogamicin, Gleevec^{™}, Gliadel^{®} Wafer, Goserelin, Granulocyte - Colony Stimulating Factor, Granulocyte Macrophage Colony Stimulating Factor, Herceptin ^{®}, Hexadrol, Hexalen^{®}, Hexamethylmelamine, HMM, Hycamtin^{®}, Hydrea^{®}, Hydrocort Acetate^{®}, Hydrocortisone, Hydrocortisone Sodium Phosphate, Hydrocortisone Sodium Succinate, Hydrocortone Phosphate, Hydroxyurea, Ibritumomab, Ibritumomab Tiuxetan, Idamycin^{®}, Idarubicin, Ifex^{®}, IFN-alpha, Ifosfamide, IL-11, IL-2, Imatinib mesylate, Imidazole Carboxamide, Interferon alfa, Interferon Alfa-2b (PEG Conjugate), Interleukin - 2, Interleukin-11, Intron A^{®} (interferon alfa-2b), Iressa^{®}, Irinotecan, Isotretinoin, Ixabepilone, Ixempra^{™}, Kidrolase, Lanacort^{®}, Lapatinib, L-asparaginase, LCR, Lenalidomide, Letrozole, Leucovorin, Leukeran, Leukine^{™}, Leuprolide, Leurocristine, Leustatin^{™}, Liposomal Ara-C, Liquid Pred^{®}, Lomustine, L-PAM, L-Sarcolysin, Lupron^{®}, Lupron Depot^{®}, Matulane^{®}, Maxidex, Mechlorethamine, Mechlorethamine Hydrochloride, Medralone^{®}, Medrol^{®}, Megace^{®}, Megestrol, Megestrol Acetate, Melphalan, Mercaptopurine, Mesna, Mesnex^{™}, Methotrexate, Methotrexate Sodium, Methylprednisolone, Meticorten^{®}, Mitomycin, Mitomycin-C, Mitoxantrone, M-Prednisol^{®}, MTC, MTX, Mustargen^{®}, Mustine, Mutamycin^{®}, Myleran^{®}, Mylocel^{™}, Mylotarg^{®}, Navelbine^{®}, Nelarabine, Neosar^{®}, Neulasta^{™}, Neumega^{®}, Neupogen^{®}, Nexavar^{®}, Nilandron^{®}, Nilutamide, Nipent^{®}, Nitrogen Mustard, Novaldex^{®}, Novantrone^{®}, Octreotide, Octreotide acetate, Oncospar^{®}, Oncovin^{®}, Ontak^{®}, Onxal^{™}, Oprevelkin, Orapred^{®}, Orasone^{®}, Oxaliplatin, Paclitaxel, Paclitaxel Protein-bound, Pamidronate, Panitumumab, Panretin^{®}, Paraplatin^{®}, Pediapred^{®}, PEG Interferon, Pegaspargase, Pegfilgrastim, PEG-INTRON^{™}, PEG-L-asparaginase, PEMETREXED, Pentostatin, Phenylalanine Mustard, Platinol^{®}, Platinol-AQ^{®}, Prednisolone, Prednisone, Prelone^{®}, Procarbazine, PROCRIT^{®}, Proleukin^{®}, Prolifeprospan 20 with Carmustine Implant Purinethol^{®}, Raloxifene, Revlimid^{®}, Rheumatrex^{®}, Rituxan^{®}, Rituximab, Roferon-A^{®} (Interferon Alfa-2a), Rubex^{®}, Rubidomycin hydrochloride, Sandostatin^{®} Sandostatin LAR^{®}, Sargramostim, Solu-Cortef^{®}, Solu-Medrol^{®}, Sorafenib, SPRYCEL^{™}, STI-571, Streptozocin, SU11248, Sunitinib, Sutent^{®}, Tamoxifen, Tarceva^{®}, Targretin^{®}, Taxol^{®}, Taxotere^{®}, Temodar^{®}, Temozolomide, Temsirolimus, Teniposide, TESPA, Thalidomide, Thalomid^{®}, TheraCys^{®}, Thioguanine, Thioguanine Tabloid^{®}, Thiophosphoamide, Thioplex^{®}, Thiotepa, TICE^{®}, Toposar^{®}, Topotecan, Toremifene, Torisel^{®}, Tositumomab, Trastuzumab, Treanda^{®}, Tretinoin, Trexall^{™}, Trisenox^{®}, TSPA, TYKERB^{®}, VCR, Vectibix^{™}, Velban^{®}, Velcade^{®}, VePesid^{®}, Vesanoid^{®}, Viadur^{™}, Vidaza^{®}, Vinblastine, Vinblastine Sulfate, Vincasar Pfs^{®}, Vincristine, Vinorelbine, Vinorelbine tartrate, VLB, VM-26, Vorinostat, VP-16, Vumon^{®}, Xeloda^{®}, Zanosar^{®}, Zevalin^{™}, Zinecard^{®}, Zoladex^{®}, Zoledronic acid, Zolinza, Zometa^{®}.

In embodiments of the present disclosure wherein a nucleic acid/virus encoding an enzyme capable of catalysing conversion of a non-toxic factor to a cytotoxic form is employed, the method may further comprise administration with a prodrug substrate for the enzyme. The prodrug may be administered simultaneously or sequentially to administration of the nucleic acid/virus encoding an enzyme capable of catalysing conversion of a non-toxic factor to a cytotoxic form.

In some embodiments the prodrug is selected from ganciclovir (GCV), aciclovir (ACV) and/or valaciclovir, e.g. where the nucleic acid/virus encodes a thymidine kinase. In some embodiments the prodrug is 5-fluorocytosine (5-FC), e.g. where the nucleic acid/virus encodes a cytosine deaminase. In some embodiments the prodrug is selected from CB1954, nitro-CBI-DEI and/or PR-104A, e.g. where the nucleic acid/virus encodes a nitroreductase. In some embodiments the prodrug is oxazaphosphorine (e.g. cyclophosphamide or ifosfamide), e.g. where the nucleic acid/virus encodes a cytochrome P450. In some embodiments the prodrug is a nitrogen mustard based drug (e.g. CMDA or ZD2767P), e.g. where the nucleic acid/virus encodes a carboxypeptidase G2. In some embodiments the prodrug is 6-methylpurine 2-deoxyriboside and/or fludarabine (e.g. 6-methylpurine-2'-deoxyriboside (MeP-dR), 2-F-2'-deoxyadenosine (F-dAdo) or arabinofuranosyl-2-F-adenine monophosphate (F-araAMP), e.g. where the nucleic acid/virus encodes a purine nucleoside phosphorylase. In some embodiments the prodrug is indole-3-acetic acid (IAA), e.g. where the nucleic acid/virus encodes a horseradish peroxidase. In some embodiments the prodrug is irinotecan, e.g. where the nucleic acid/virus encodes a carboxylesterase.

Multiple doses of the articles of the present disclosure (e.g. viruses (e.g. oncolytic viruses, HDAds), antigen-binding molecules (e.g. BiTEs), CARs, immunomodulatory factors (e.g. IL-12, anti-PD-L1 minibody), nucleic acids, cells compositions, combinations) of the present disclosure may be provided. One or more, or each, of the doses may be accompanied by simultaneous or sequential administration of another therapeutic agent.

Multiple doses may be separated by a predetermined time interval, which may be selected to be one of 1, 2, 3, 4, 5, 6, 7, 8, 9, 10, 11, 12, 13, 14, 15, 16, 17, 18, 19, 20, 21, 22, 23, or more hours or 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, or 31 days, or 1, 2, 3, 4, 5, or 6 months. By way of example, doses may be given once every 7, 14, 21 or 28 days (plus or minus 3, 2, or 1 days).

### Adoptive transfer

In embodiments of the present disclosure, the methods of treatment comprise adoptive transfer of immune cells. Adoptive cell transfer (ACT) generally refers to a process by which cells (*e.g.* immune cells) are obtained from a subject, typically by drawing a blood sample from which the cells are isolated. The cells are then typically treated or altered in some way, and then administered either to the same subject (adoptive transfer is of autologous cells) or to a different subject (adoptive transfer is of allogeneic cells). The treatment is typically aimed at providing population of cells with certain desired characteristics to a subject, or increasing the frequency of cells with such characteristics in that subject. In the present disclosure, adoptive transfer may be performed with the aim of introducing a cell or population of cells into a subject, and/or increasing the frequency of a cell or population of cells in a subject.

In some embodiments, the subject from which the cell is isolated is the subject administered with the modified cell (*i.e.,* adoptive transfer is of autologous cells). In some embodiments, the subject from which the cell is isolated is a different subject to the subject to which the modified cell is administered (*i.e.,* adoptive transfer is of allogeneic cells).

Adoptive transfer of T cells is described, for example, in Kalos and June 2013, Immunity 39(1): 49-60, which is hereby incorporated by reference in its entirety. Adoptive transfer of NK cells is described, for example, in Davis et al. 2015, Cancer J. 21(6): 486-491, which is hereby incorporated by reference in its entirety.

The cell may *e.g.* be a neutrophil, eosinophil, basophil, dendritic cell, lymphocyte, or monocyte. The lymphocyte may be *e.g.* a T cell, B cell, NK cell, NKT cell or innate lymphoid cell (ILC), or a precursor thereof. In some embodiments, the cell is a T cell. In some embodiments, the T cell is a CD3+ T cell. In some embodiments, the T cell is a CD3+, CD4+ T cell. In some embodiments, the T cell is a CD3+, CD8+ T cell. In some embodiments, the T cell is a T helper cell (T_{H} cell)). In some embodiments, the T cell is a cytotoxic T cell (*e.g.* a cytotoxic T lymphocyte (CTL)). In some embodiments, the T cell is a virus-specific T cell. In some embodiments, the T cell is specific for EBV, HPV, HBV, HCV or sHIV.

In some embodiments the cell is an immune cell specific for an oncolytic virus, as described herein. Accordingly, in some embodiments the methods comprise administration of at least one immune cell specific for an oncolytic virus to a subject. In some embodiments, the methods of the disclosure comprise generating/expanding a population of immune cells specific for an oncolytic virus, and administering at least one immune cell specific for the oncolytic virus to a subject.

In some embodiments, the methods comprise:
(a) isolating immune cells from a subject;
(b) generating or expanding a population of immune cells specific for an oncolytic virus by a method comprising: stimulating the immune cells by culture in the presence of antigen presenting cells (APCs) presenting a peptide of the oncolytic virus, and;
(c) administering at least one immune cell specific for the oncolytic virus to a subject.

In some embodiments the method steps for production of an immune cell specific for an oncolytic virus may comprise one or more of: taking a blood sample from a subject; isolating PBMCs from the blood sample; generating/expanding a population of immune cells specific for an oncolytic virus (e.g. by culturing PBMCs in the presence of cells (e.g. APCs) comprising/expressing antigen(s)/peptide(s) of the oncolytic virus); culturing immune cells specific for an oncolytic virus in *in vitro* or *ex vivo* cell culture; collecting immune cells specific for an oncolytic virus; mixing immune cells specific for an oncolytic virus with an adjuvant, diluent, or carrier; administering the modified cell to a subject.

In some embodiments the methods of the present disclosure comprise administering at least one cell comprising a chimeric antigen receptor (CAR) specific for a cancer cell antigen to a subject. In connection with this feature of the disclosure, in some embodiments, the method additionally comprises steps for production of the at least one cell comprising a chimeric antigen receptor (CAR) specific for a cancer cell antigen. The CAR may be a first generation, second generation or third or subsequent generation CAR. The CAR may comprise one, two, three, or more costimulatory domains, for example.

In some embodiments, the methods comprise modifying at least one cell obtained from a subject to express or comprise a CAR according to the disclosure, optionally expanding the modified at least one cell, and administering the modified at least one cell to a subject.

In some embodiments, the methods comprise:
(a) isolating at least one cell from a subject;
(b) modifying the at least one cell to express or comprise a CAR according to the present disclosure, or a nucleic acid encoding a CAR according to the present disclosure,
(c) optionally expanding the modified at least one cell, and;
(d) administering the modified at least one cell to a subject.

In some embodiments the cell comprising/expressing a CAR specific for a cancer cell antigen is an immune cell specific for an oncolytic virus, as described herein. In some embodiments, the methods comprise modifying an immune cell specific for an oncolytic virus to express or comprise a CAR according to the disclosure, optionally expanding the modified immune cell specific for an oncolytic virus, and administering the modified immune cell specific for an oncolytic virus to a subject.

In some embodiments, the methods comprise:
(a) isolating immune cells from a subject;
(b) generating or expanding a population of immune cells specific for an oncolytic virus by a method comprising: stimulating the immune cells by culture in the presence of antigen presenting cells (APCs) presenting a peptide of the oncolytic virus;
(c) modifying at least one immune cell specific for an oncolytic virus to express or comprise a CAR according to the present disclosure, or a nucleic acid encoding a CAR according to the present disclosure,
(d) optionally expanding the modified at least one immune cell specific for an oncolytic virus, and;
(e) administering the modified at least one immune cell specific for an oncolytic virus to a subject.

The at least one cell modified according to the present disclosure can be modified to comprise/express a CAR according to methods well known to the skilled person. The modification may comprise nucleic acid transfer for permanent or transient expression of the transferred nucleic acid. Any suitable genetic engineering platform may be used to modify a cell according to the present disclosure. Suitable methods for modifying a cell include the use of genetic engineering platforms such as gammaretroviral vectors, lentiviral vectors, adenovirus vectors, DNA transfection, transposon-based gene delivery and RNA transfection, for example as described in Maus et al., Annu Rev Immunol (2014) 32:189-225, incorporated by reference hereinabove.

In some embodiments the method steps for production of the at least one cell comprising a chimeric antigen receptor (CAR) specific for a cancer cell antigen may comprise one or more of: taking a blood sample from a subject; isolating and/or expanding at least one cell from the blood sample; culturing the at least one cell in *in vitro* or *ex vivo* cell culture; introducing into the at least one cell a CAR as described herein, or a nucleic acid encoding a CAR as described herein, thereby modifying the at least one cell; expanding the at least one modified cell; collecting the at least one modified cell; mixing the modified cell with an adjuvant, diluent, or carrier; administering the modified cell to a subject.

In some embodiments, the methods may additionally comprise treating the cell to induce/enhance expression of the CAR or nucleic acid encoding the CAR. For example, the nucleic acid may comprise a control element for inducible upregulation of expression of the CAR from the nucleic acid in response to treatment with a particular agent. In some embodiments, treatment may be *in vivo* by administration of the agent to a subject having been administered with a modified cell according to the disclosure. In some embodiments, treatment may be *ex vivo* or *in vitro* by administration of the agent to cells in culture *ex vivo* or *in vitro.*

The skilled person is able to determine appropriate reagents and procedures for adoptive transfer of cells according to the present disclosure, for example by reference to Dai et al., 2016 J Nat Cancer Inst 108(7): djv439, which is incorporated by reference in its entirety.

In a related aspect, the present disclosure provides a method of preparing a modified cell, the method comprising introducing into a cell a CAR according to the present disclosure or a nucleic acid encoding a CAR according to the present disclosure, thereby modifying the at least one cell. The method is preferably performed *in vitro* or ex *vivo.*

### Sequence Identity

Pairwise and multiple sequence alignment for the purposes of determining percent identity between two or more amino acid or nucleic acid sequences can be achieved in various ways known to a person of skill in the art, for instance, using publicly available computer software such as ClustalOmega (Söding, J. 2005, Bioinformatics 21, 951-960), T-coffee (Notredame et al. 2000, J. Mol. Biol. (2000) 302, 205-217), Kalign (Lassmann and Sonnhammer 2005, BMC Bioinformatics, 6(298)) and MAFFT (Katoh and Standley 2013, Molecular Biology and Evolution, 30(4) 772-780 software. When using such software, the default parameters, e.g. for gap penalty and extension penalty, are preferably used.

### Sequences

| **SEQ ID NO.** | **DESCRIPTION** | **SEQUENCE** |
|---|---|---|
| 1 | HER2(C5)-CD28TM,ICD-CD3Z CAR | QVQLQESGPGLVKPSETLSLTCTVSGGSISSSSYYWGWIRQPPGKGLEWIGSIYYSGST YYNPSLKSRVTISVDTSKNQFSLKLSSVTAADTAVYYCARYAPDSSGYLVAFDIWGQGT MVTVSSGGGGSGGGGSGGGGSQTVVTQEPSFSVSPGGTVTLTCGLSSGSVSTGYYPS WYQQTPGQAPRTLIYSTNSRSSGVPDRFSGSILGNKAALTITGAQADDESDYYCVLYMG SGISVFGGGTKLTVLGSEPKSCDKTHTCPTRFWVLVVVGGVLACYSLLVTVAFIIFWVRS KRSRLLHSDYMNMTPRRPGPTRKHYQPYAPPRDFAAYRSRVKFSRSADAPAYQQGQN QLYNELNLGRREEYDVLDKRRGRDPEMGGKPQRRKNPQEGLYNELQKDKMAEAYSEI GMKGERRRGKGHDGLYQGLSTATKDTYDALHMQALPPR |
| 2 | HER2(E4)-CD28TM,ICD-CD3Z CAR | QVQLQQWGAGLLKPSETLSLTCAVYGGSFSGYYWSWIRQPPGKGLEWIGEINHSGSTN YNPSLKSRVTISVDTSKNQFSLKLSSVTTADTAVYYCARMGINSGGYLYGMDVWGQGTT VTVSSGGGGSGGGGSGGGGSQTVVTQEPSFSVSPGGTVTLTCGLSSGSVSTSYYPSW YQQIPGQAPRTLIYTTNIRSSGVPDRFGGSILGNKAALTITGAQAEDESDYYCMLYMGSGI WVFGGGTKLTVLGSEPKSCDKTHTCPTRFWVLVVVGGVLACYSLLVTVAFIIFWVRSKR SRLLHSDYMNMTPRRPGPTRKHYQPYAPPRDFAAYRSRVKFSRSADAPAYQQGQNQL YNELNLGRREEYDVLDKRRGRDPEMGGKPQRRKNPQEGLYNELQKDKMAEAYSEIGM KGERRRGKGHDGLYQGLSTATKDTYDALHMQALPPR |
| 3 | HER2(F1)-CD28TM,ICD-CD3Z CAR | QVQLVESGPGLVKPSGTLSLTCAVSGGSISSSNWWSWVRQPPGKGLEWIGEIYHSGST NYNPSLKSRVTISVDTSKNQFSLKLSSVTAADTAVYYCARMGANSGGYLYGMDVWGQG TTVTVSSGGGGSGGGGSGGGGSQTVVTQEPSFSVSPGGTVTLTCGLSSGSVSTSYYP SWYQQTPGQAPRTLIYSTNTRSSGVPDRFSGSILGNKAALTITGAQADDESDYYCVLYM GSGIWVFGGGTKLTVLGSEPKSCDKTHTCPTRFWVLVVVGGVLACYSLLVTVAFIIFWVR SKRSRLLHSDYMNMTPRRPGPTRKHYQPYAPPRDFAAYRSRVKFSRSADAPAYQQGQ NQLYNELNLGRREEYDVLDKRRGRDPEMGGKPQRRKNPQEGLYNELQKDKMAEAYSE IGMKGERRRGKGHDGLYQGLSTATKDTYDALHMQALPPR |
| 4 | HER2(A3)-CD28TM,ICD-CD3Z CAR | QLVQSGTEVKKPGASVRVSCKSSGYTFTSYYIHWVRQAPGQGLEWMAIINPGNGDTNY AQRFQGRVTMTRDTSTSTVYMELRSLRSDDTAVYFCAREIASYSGSYYDYWGQGTLVT VSSGGGGSGGGGSGGGGSQAVVLQEPSLSVSPGGTVTLTCGLSSGSVSTGHYASWY QQTPGQAPRTLFYNTNTRSSGVPDRFSGSIVGNKAALTITGAQADDESDYYCVLYVGDG IWVFGGGTKLTVLEPKSCDKTHTCPTRFWVLVVVGGVLACYSLLVTVAFIIFWVRSKRSR LLHSDYMNMTPRRPGPTRKHYQPYAPPRDFAAYRSRVKFSRSADAPAYQQGQNQLYN ELNLGRREEYDVLDKRRGRDPEMGGKPQRRKNPQEGLYNELQKDKMAEAYSEIGMKG ERRRGKGHDGLYQGLSTATKDTYDALHMQALPPR |
| 5 | CD28 TMD | FWVLVVVGGVLACYSLLVTVAFIIFWV |
| 6 | CD28 ICD | RSKRSRLLHSDYMNMTPRRPGPTRKHYQPYAPPRDFAAYRS |
| 7 | CD3Z ICD | RVKFSRSADAPAYQQGQNQLYNELNLGRREEYDVLDKRRGRDPEMGGKPQRRKNPQ EGLYNELQKDKMAEAYSEIGMKGERRRGKGHDGLYQGLSTATKDTYDALHMQALPPR |
| 8 | Hinge | EPKSCDKTHTCPTR |
| 9 | HER2(C5) LC-CDR1 | GLSSGSVSTGYYPS |
| 10 | HER2(C5) LC-CDR2 | STNSRSS |
| 11 | HER2(C5) LC-CDR3 | VLYMGSGISV |
| 12 | HER2(C5) HC-CDR1 | SSSYYWG |
| 13 | HER2(C5) HC-CDR2 | SIYYSGSTYYNPSLKS |
| 14 | HER2(C5) HC-CDR3 | YAPDSSGYLVAFDI |
| 15 | HER2(C5) VL | QTVVTQEPSFSVSPGGTVTLTCGLSSGSVSTGYYPSWYQQTPGQAPRTLIYSTNSRSS GVPDRFSGSILGNKAALTITGAQADDESDYYCVLYMGSGISVFGGGTKLTVLGS |
| 16 | HER2(C5) VH | QVQLQESGPGLVKPSETLSLTCTVSGGSISSSSYYWGWIRQPPGKGLEWIGSIYYSGST YYNPSLKSRVTISVDTSKNQFSLKLSSVTAADTAVYYCARYAPDSSGYLVAFDIWGQGT MVTVSS |
| 17 | HER2(E4) LC-CDR1 | GLSSGSVSTSYYPS |
| 18 | HER2(E4) LC-CDR2 | TTNIRSS |
| 19 | HER2(E4) LC-CDR3 | MLYMGSGIWV |
| 20 | HER2(E4) HC-CDR1 | SGYYWS |
| 21 | HER2(E4) HC-CDR2 | EINHSGSTNYNPSLKS |
| 22 | HER2(E4) HC-CDR3 | MGINSGGYLYGMDV |
| 23 | HER2(E4) VL | QTVVTQEPSFSVSPGGTVTLTCGLSSGSVSTSYYPSWYQQIPGQAPRTLIYTTNIRSSGV PDRFGGSILGNKAALTITGAQAEDESDYYCMLYMGSGIWVFGGGTKLTVLGS |
| 24 | HER2(E4) VH | QVQLQQWGAGLLKPSETLSLTCAVYGGSFSGYYWSWIRQPPGKGLEWIGEINHSGSTN YNPSLKSRVTISVDTSKNQFSLKLSSVTTADTAVYYCARMGINSGGYLYGMDVWGQGTT VTVSS |
| 25 | HER2(F1) LC-CDR1 | GLSSGSVSTSYYPS |
| 26 | HER2(F1) LC-CDR2 | STNTRSS |
| 27 | HER2(F1) LC-CDR3 | VLYMGSGIWV |
| 28 | HER2(F1) HC-CDR1 | SSNWWS |
| 29 | HER2(F1) HC-CDR2 | EIYHSGSTNYNPSLKS |
| 30 | HER2(F1) HC-CDR3 | MGANSGGYLYGMDV |
| 31 | HER2(F1) VL | QTVVTQEPSFSVSPGGTVTLTCGLSSGSVSTSYYPSWYQQTPGQAPRTLIYSTNTRSSG VPDRFSGSILGNKAALTITGAQADDESDYYCVLYMGSGIWVFGGGTKLTVLGS |
| 32 | HER2(F1) VH | QVQLVESGPGLVKPSGTLSLTCAVSGGSISSSNWWSWVRQPPGKGLEWIGEIYHSGST NYNPSLKSRVTISVDTSKNQFSLKLSSVTAADTAVYYCARMGANSGGYLYGMDVWGQG TTVTVSS |
| 33 | HER2(A3) LC-CDR1 | GLSSGSVSTGHYAS |
| 34 | HER2(A3) LC-CDR2 | NTNTRSS |
| 35 | HER2(A3) LC-CDR3 | VLYVGDGIWV |
| 36 | HER2(A3) HC-CDR1 | SYYIHWVRQA |
| 37 | HER2(A3) HC-CDR2 | IINPGNGDTNYAQRFQG |
| 38 | HER2(A3) HC-CDR3 | EIASYSGSYYDY |
| 39 | HER2(A3) VL | QAVVLQEPSLSVSPGGTVTLTCGLSSGSVSTGHYASWYQQTPGQAPRTLFYNTNTRSS GVPDRFSGSIVGNKAALTITGAQADDESDYYCVLYVGDGIWVFGGGTKLTVL |
| 40 | HER2(A3) VH | EVQLVQSGTEVKKPGASVRVSCKSSGYTFTSYYIHWVRQAPGQGLEWMAIINPGNGDT NYAQRFQGRVTMTRDTSTSTVYMELRSLRSDDTAVYFCAREIASYSGSYYDYWGQGTL VTVSS |
| 41 | hulL-12p70 | MGHQQLVISWFSLVFLASPLVAIWELKKDVYVVELDWYPDAPGEMVVLTCDTPEEDGIT WTLDQSSEVLGSGKTLTIQVKEFGDAGQYTCHKGGEVLSHSLLLLHKKEDGIWSTDILKD QKEPKNKTFLRCEAKNYSGRFTCWWLTTISTDLTFSVKSSRGSSDPQGVTCGAATLSAE RVRGDNKEYEYSVECQEDSACPAAEESLPIEVMVDAVHKLKYENYTSSFFIRDIIKPDPP KNLQLKPLKNSRQVEVSWEYPDTWSTPHSYFSLTFCVQVQGKSKREKKDRVFTDKTSA TVICRKNASISVRAQDRYYSSSWSEWASVPCSVPGVGVPGVGARNLPVATPDPGMFPC LHHSQNLLRAVSNMLQKARQTLEFYPCTSEEIDHEDITKDKTSTVEACLPLELTKNESCL NSRETSFITNGSCLASRKTSFMMALCLSSIYEDLKMYQVEFKTMNAKLLMDPKRQIFLDQ NMLAVIDELMQALNFNSETVPQKSSLEEPDFYKTKIKLCILLHAFRIRAVTIDRVMSYLNAS |
| 42 | HSV1 TK | MASYPGHQHASAFDQAARSRGHSNRRTALRPRRQQEATEVRPEQKMPTLLRVYIDGP HGMGKTTTTQLLVALGSRDDIVYVPEPMTYWRVLGASETIANIYTTQHRLDQGEISAGDA AVVMTSAQITMGMPYAVTDAVLAPHIGGEAGSSHAPPPALTLIFDRHPIAALLCYPAARYL MGSMTPQAVLAFVALIPPTLPGTNIVLGALPEDRHIDRLAKRQRPGERLDLAMLAAIRRVY GLLANTVRYLQGGGSWREDWGQLSGTAVPPQGAEPQSNAGPRPHIGDTLFTLFRAPEL LAPNGDLYNVFAWALDVLAKRLRPMHVFILDYDQSPAGCRDALLQLTSGMIQTHVTTPG SIPTICDLARTFAREMGEAN |
| 43 | PD-L1(H12_gl) minibody | EVQLVQSGAEVKKPGASVKVSCKASGGTFSSYAISWVRQAPGQGLEWMGRIIPILGIAN YAQKFQGRVTITADKSTSTAYMELSSLRSEDTAVYYCARSGHGYSYGAFDYWGQGTLV TVSSGGGGSGGGGSGGGGSQSVLTQPPSVSGAPGQRVTISCTGSSSNIGAGYDVHWY QQLPGTAPKLLIYGNSNRPSGVPDRFSGSKSGTSASLAITGLQAEDEADYYCQSYDSSL SGSYVVFGGGTKLTVLEAKSCDKTHTCPPCPAPELLGGPSVFLFPPKPKDTLMISRTPEV TCVVVDVSHEDPEVKFNWYVDGVEVHNAKTKPREEQYNSTYRVVSVLTVLHQDWLNG KEYKCKVSNKALPAPIEKTISKAKGQPREPQVYTLPPSRDELTKNQVSLTCLVKGFYPSDI AVEWESNGQPENNYKTTPPVLDSDGSFFLYSKLTVDKSRWQQGNVFSCSVMHEALHN HYTQKSLSLSPGK |
| 44 | PD-L1(H12_gl) LC-CDR1 | TGSSSNIGAGYDVH |
| 45 | PD-L1(H12_gl) LC-CDR2 | GNSNRPS |
| 46 | PD-L1(H12_gl) LC-CDR3 | QSYDSSLSGSYVV |
| 47 | PD-L1(H12_gl) HC-CDR1 | SYAIS |
| 48 | PD-L1(H12_gl) HC-CDR2 | RIIPILGIANYAQKFQG |
| 49 | PD-L1(H12_gl) HC-CDR3 | SGHGYSYGAFDY |
| 50 | PD-L1 (H12_gl) VL | QSVLTQPPSVSGAPGQRVTISCTGSSSNIGAGYDVHWYQQLPGTAPKLLIYGNSNRPSG VPDRFSGSKSGTSASLAITGLQAEDEADYYCQSYDSSLSGSYVVFGGGTKLTVL |
| 51 | PD-L1(H12_gl) VH | EVQLVQSGAEVKKPGASVKVSCKASGGTFSSYAISWVRQAPGQGLEWMGRIIPILGIAN YAQKFQGRVTITADKSTSTAYMELSSLRSEDTAVYYCARSGHGYSYGAFDYWGQGTLV TVSS |
| 52 | PD-L1 (YW243.55.S70) minibody | EVQLVESGGGLVQPGGSLRLSCAASGFTFSDSWIHWVRQAPGKGLEWVAWISPYGGS TYYADSVKGRFTISADTSKNTAYLQMNSLRAEDTAVYYCARRHWPGGFDYWGQGTLVT VSAGGGGSGGGGSGGGGSDIQMTQSPSSLSASVGDRVTITCRASQDVSTAVAWYQQK PGKAPKLLIYSASFLYSGVPSRFSGSGSGTDFTLTISSLQPEDFATYYCQQYLYHPATFG QGTKVEIKREAKSCDKTHTCPPCPAPELLGGPSVFLFPPKPKDTLMISRTPEVTCVVVDV |
| | | SHEDPEVKFNWYVDGVEVHNAKTKPREEQYNSTYRVVSVLTVLHQDWLNGKEYKCKV SNKALPAPIEKTISKAKGQPREPQVYTLPPSRDELTKNQVSLTCLVKGFYPSDIAVEWES NGQPENNYKTTPPVLDSDGSFFLYSKLTVDKSRWQQGNVFSCSVMHEALHNHYTQKSL SLSPGK |
| 53 | PD-L1 (YW243.55.S70) LC-CDR1 | QDVSTA |
| 54 | PD-L1 (YW243.55.S70) LC-CDR2 | SAS |
| 55 | PD-L1 (YW243.55.S70) LC-CDR3 | QQYLYHPAT |
| 56 | PD-L1 (YW243.55.S70) HC-CDR1 | GFTFSDSW |
| 57 | PD-L1 (YW243.55.S70) HC-CDR2 | ISPYGGST |
| 58 | PD-L1 (YW243.55.S70) HC-CDR3 | ARRHWPGGFDY |
| 59 | PD-L1 (YW243.55.S70) VL | |
| 60 | PD-L1 (YW243.55.S70) VH | EVQLVESGGGLVQPGGSLRLSCAASGFTFSDSWIHWVRQAPGKGLEWVAWISPYGGS TYYADSVKGRFTISADTSKNTAYLQMNSLRAEDTAVYYCARRHWPGGFDYWGQGTLVT VS |
| 61 | IgG1 hinge | KSCDKTHTCP |
| 62 | hIgG1 CH2 | PCPAPELLGGPSVFLFPPKPKDTLMISRTPEVTCVVVDVSHEDPEVKFNWYVDGVEVHN AKTKPREEQYNSTYRVVSVLTVLHQDWLNGKEYKCKVSNKALPAPIEKTISKAK |
| 63 | hIgG1 CH3 | GQPREPQVYTLPPSRDELTKNQVSLTCLVKGFYPSDIAVEWESNGQPENNYKTTPPVLD SDGSFFLYSKLTVDKSRWQQGNVFSCSVMHEALHNHYTQKSLSLSPGK |
| 64 | anti-CD44v6 BiTE | EIVLTQSPATLSLSPGERATLSCSASSSINYIYWLQQKPGQAPRILIYLTSNLASGVPARFS GSGSGTDFTLTISSLEPEDFAVYYCLQWSSNPLTFGGGTKVEIKRGGGGSGGGGSGGG GSGGGGSEVQLVESGGGLVKPGGSLRLSCAASGFTFSSYDMSWVRQAPGKGLEWVS TISSGGSYTYYLDSIKGRFTISRDNAKNSLYLQMNSLRAEDTAVYYCARQGLDYWGRGT LVTVSSGGGGGSDIKLQQSGAELARPGASVKMSCKTSGYTFTRYTMHWVKQRPGQGL EWIGYINPSRGYTNYNQKFKDKATLTTDKSSSTAYMQLSSLTSEDSAVYYCARYYDDHY CLDYWGQGTTLTVSSGGGGSGGGGSGGGGSDIQLTQSPAIMSASPGEKVTMTCRASS SVSYMNWYQQKSGTSPKRWIYDTSKVASGVPYRFSGSGSGTSYSLTISSMEAEDAATY YCQQWSSNPLTFGAGTKLELK |
| 65 | anti-CD44v6 (BIWA8) scFv | EIVLTQSPATLSLSPGERATLSCSASSSINYIYWLQQKPGQAPRILIYLTSNLASGVPARFS GSGSGTDFTLTISSLEPEDFAVYYCLQWSSNPLTFGGGTKVEIKRGGGGSGGGGSGGG GSGGGGSEVQLVESGGGLVKPGGSLRLSCAASGFTFSSYDMSWVRQAPGKGLEWVS TISSGGSYTYYLDSIKGRFTISRDNAKNSLYLQMNSLRAEDTAVYYCARQGLDYWGRGT LVTVSS |
| 66 | anti-CD44v6 (BIWA8) LC-CDR1 | SSINY |
| 67 | anti-CD44v6 (BIWA8) LC-CDR2 | LTS |
| 68 | anti-CD44v6 (BIWA8) LC-CDR3 | LQWSSNPLT |
| 69 | anti-CD44v6 (BIWA8) HC-CDR1 | GFTFSSYD |
| 70 | anti-CD44v6 (BIWA8) HC-CDR2 | ISSGGSYT |
| 71 | anti-CD44v6 (BIWA8) HC-CDR3 | ARQGLDY |
| 72 | anti-CD44v6 (BIWA8) VL | EIVLTQSPATLSLSPGERATLSCSASSSINYIYWLQQKPGQAPRILIYLTSNLASGVPARFS GSGSGTDFTLTISSLEPEDFAVYYCLQWSSNPLTFGGGTKVEIK |
| 73 | anti-CD44v6 (BIWA8) VH | EVQLVESGGGLVKPGGSLRLSCAASGFTFSSYDMSWVRQAPGKGLEWVSTISSGGSYT YYLDSIKGRFTISRDNAKNSLYLQMNSLRAEDTAVYYCARQGLDYWGRGTLVTVSS |
| 74 | anti-CD3 (OKT3) scFv | DIKLQQSGAELARPGASVKMSCKTSGYTFTRYTMHWVKQRPGQGLEWIGYINPSRGYT NYNQKFKDKATLTTDKSSSTAYMQLSSLTSEDSAVYYCARYYDDHYCLDYWGQGTTLT VSSGGGGSGGGGSGGGGSDIQLTQSPAIMSASPGEKVTMTCRASSSVSYMNWYQQK SGTSPKRWIYDTSKVASGVPYRFSGSGSGTSYSLTISSMEAEDAATYYCQQWSSNPLTF GAGTKLELK |
| 75 | anti-CD3 (OKT3) LC-CDR1 | SSVSY |
| 76 | anti-CD3 (OKT3) LC-CDR2 | DTS |
| 77 | anti-CD3 (OKT3) LC-CDR3 | QQWSSNPLT |
| 78 | anti-CD3 (OKT3) HC-CDR1 | GYTFTRYT |
| 79 | anti-CD3 (OKT3) HC-CDR2 | INPSRGYT |
| 80 | anti-CD3 (OKT3) HC-CDR3 | ARYYDDHYCLDY |
| 81 | anti-CD3 (OKT3) VL | DIQLTQSPAIMSASPGEKVTMTCRASSSVSYMNWYQQKSGTSPKRWIYDTSKVASGVP YRFSGSGSGTSYSLTISSMEAEDAATYYCQQWSSNPLTFGAGTKLELK |
| 82 | anti-CD3 (OKT3) VH | DIKLQQSGAELARPGASVKMSCKTSGYTFTRYTMHWVKQRPGQGLEWIGYINPSRGYT NYNQKFKDKATLTTDKSSSTAYMQLSSLTSEDSAVYYCARYYDDHYCLDYWGQGTTLT VSS |
| 83 | anti-HER2 BiTE | EVQLQQSGPELKKPGETVKISCKASGYPFTNYGMNWVKQAPGQGLKWMGWINTSTGE STFADDFKGRFDFSLETSANTAYLQINNLKSEDMATYFCARWEVYHGYVPYWGQGTTV TVSSGGGGSGGGGSGGGGSDIQLTQSHKFLSTSVGDRVSITCKASQDVYNAVAWYQQ KPGQSPKLLIYSASSRYTGVPSRFTGSGSGPDFTFTISSVQAEDLAVYFCQQHFRTPFTF GSGTKLEIKALGGGGSDIKLQQSGAELARPGASVKMSCKTSGYTFTRYTMHWVKQRPG QGLEWIGYINPSRGYTNYNQKFKDKATLTTDKSSSTAYMQLSSLTSEDSAVYYCARYYD DHYCLDYWGQGTTLTVSSGGGGSGGGGSGGGGSDIQLTQSPAIMSASPGEKVTMTCR ASSSVSYMNWYQQKSGTSPKRWIYDTSKVASGVPYRFSGSGSGTSYSLTISSMEAEDA ATYYCQQWSSNPLTFGAGTKLELK |
| 84 | anti-HER2 (FRP5) scFv | EVQLQQSGPELKKPGETVKISCKASGYPFTNYGMNWVKQAPGQGLKWMGWINTSTGE STFADDFKGRFDFSLETSANTAYLQINNLKSEDMATYFCARWEVYHGYVPYWGQGTTV TVSSGGGGSGGGGSGGGGSDIQLTQSHKFLSTSVGDRVSITCKASQDVYNAVAWYQQ KPGQSPKLLIYSASSRYTGVPSRFTGSGSGPDFTFTISSVQAEDLAVYFCQQHFRTPFTF GSGTKLEIK |
| 85 | anti-HER2 (FRP5) LC-CDR1 | QDVYNA |
| 86 | anti-HER2 (FRP5) LC-CDR2 | SAS |
| 87 | anti-HER2 (FRP5) LC-CDR3 | QQHFRTPFT |
| 88 | anti-HER2 (FRP5) HC-CDR1 | GYPFTNYG |
| 89 | anti-HER2 (FRP5) HC-CDR2 | INTSTGES |
| 90 | anti-HER2 (FRP5) HC-CDR3 | ARWEVYHGYVPY |
| 91 | anti-HER2 (FRP5) VL | DIQLTQSHKFLSTSVGDRVSITCKASQDVYNAVAWYQQKPGQSPKLLIYSASSRYTGVP SRFTGSGSGPDFTFTISSVQAEDLAVYFCQQHFRTPFTFGSGTKLEIK |
| 92 | anti-HER2 (FRP5) VH | EVQLQQSGPELKKPGETVKISCKASGYPFTNYGMNWVKQAPGQGLKWMGWINTSTGE STFADDFKGRFDFSLETSANTAYLQINNLKSEDMATYFCARWEVYHGYVPYWGQGTTV TVSS |
| 93 | anti-CD19 BiTE | DIQMTQTTSSLSASLGDRVTISCRASQDISKYLNWYQQKPDGTVKLLIYHTSRLHSGVPS RFSGSGSGTDYSLTISNLEQEDIATYFCQQGNTLPYTFGGGTKLEITGGGGSGGGGSGG GGSEVKLQESGPGLVAPSQSLSVTCTVSGVSLPDYGVSWIRQPPRKGLEWLGVIWGSE TTYYNSALKSRLTIIKDNSKSQVFLKMNSLQTDDTAIYYCAKHYYYGGSYAMDYWGQGT SVTVSSGGGGGSDIKLQQSGAELARPGASVKMSCKTSGYTFTRYTMHWVKQRPGQGL EWIGYINPSRGYTNYNQKFKDKATLTTDKSSSTAYMQLSSLTSEDSAVYYCARYYDDHY CLDYWGQGTTLTVSSGGGGSGGGGSGGGGSDIQLTQSPAIMSASPGEKVTMTCRASS SVSYMNWYQQKSGTSPKRWIYDTSKVASGVPYRFSGSGSGTSYSLTISSMEAEDAATY YCQQWSSNPLTFGAGTKLELK |
| 94 | anti-CD19 (FMC63) scFv | DIQMTQTTSSLSASLGDRVTISCRASQDISKYLNWYQQKPDGTVKLLIYHTSRLHSGVPS RFSGSGSGTDYSLTISNLEQEDIATYFCQQGNTLPYTFGGGTKLEITGGGGSGGGGSGG GGSEVKLQESGPGLVAPSQSLSVTCTVSGVSLPDYGVSWIRQPPRKGLEWLGVIWGSE TTYYNSALKSRLTIIKDNSKSQVFLKMNSLQTDDTAIYYCAKHYYYGGSYAMDYWGQGT SVTVSS |
| 95 | anti-CD19 (FMC63) LC-CDR1 | QDISKY |
| 96 | anti-CD19 (FMC63) LC-CDR2 | HTS |
| 97 | anti-CD19 (FMC63) LC-CDR3 | QQGNTLPYT |
| 98 | anti-CD19 (FMC63) HC-CDR1 | GVSLPDYG |
| 99 | anti-CD19 (FMC63) HC-CDR2 | IWGSETT |
| 100 | anti-CD19 (FMC63) HC-CDR3 | AKHYYYGGSYAMDY |
| 101 | anti-CD19 (FMC63) VL | DIQMTQTTSSLSASLGDRVTISCRASQDISKYLNWYQQKPDGTVKLLIYHTSRLHSGVPS RFSGSGSGTDYSLTISNLEQEDIATYFCQQGNTLPYTFGGGTKLEIT |
| 102 | anti-CD19 (FMC63) VH | EVKLQESGPGLVAPSQSLSVTCTVSGVSLPDYGVSWIRQPPRKGLEWLGVIWGSETTY YNSALKSRLTIIKDNSKSQVFLKMNSLQTDDTAIYYCAKHYYYGGSYAMDYWGQGTSVT VSS |
| 103 | HER2 BiTE_CD44 v6BiTE fusion | EIVLTQSPATLSLSPGERATLSCSASSSINYIYWLQQKPGQAPRILIYLTSNLASGVPARFS GSGSGTDFTLTISSLEPEDFAVYYCLQWSSNPLTFGGGTKVEIKRGGGGSGGGGSGGG GSGGGGSEVQLVESGGGLVKPGGSLRLSCAASGFTFSSYDMSWVRQAPGKGLEWVS TISSGGSYTYYLDSIKGRFTISRDNAKNSLYLQMNSLRAEDTAVYYCARQGLDYWGRGT LVTVSSGGGGGSDIKLQQSGAELARPGASVKMSCKTSGYTFTRYTMHWVKQRPGQGL EWIGYINPSRGYTNYNQKFKDKATLTTDKSSSTAYMQLSSLTSEDSAVYYCARYYDDHY CLDYWGQGTTLTVSSGGGGSGGGGSGGGGSDIQLTQSPAIMSASPGEKVTMTCRASS SVSYMNWYQQKSGTSPKRWIYDTSKVASGVPYRFSGSGSGTSYSLTISSMEAEDAATY YCQQWSSNPLTFGAGTKLELKSEGRGSLLTCGDVEENPGPSMDWIWRILFLVGAATGA HSEVQLQQSGPELKKPGETVKISCKASGYPFTNYGMNWVKQAPGQGLKWMGWINTST GESTFADDFKGRFDFSLETSANTAYLQINNLKSEDMATYFCARWEVYHGYVPYWGQGT TVTVSSGGGGSGGGGSGGGGSDIQLTQSHKFLSTSVGDRVSITCKASQDVYNAVAWY QQKPGQSPKLLIYSASSRYTGVPSRFTGSGSGPDFTFTISSVQAEDLAVYFCQQHFRTP FTFGSGTKLEIKALGGGGSDIKLQQSGAELARPGASVKMSCKTSGYTFTRYTMHWVKQ RPGQGLEWIGYINPSRGYTNYNQKFKDKATLTTDKSSSTAYMQLSSLTSEDSAVYYCAR YYDDHYCLDYWGQGTTLTVSSGGGGSGGGGSGGGGSDIQLTQSPAIMSASPGEKVTM TCRASSSVSYMNWYQQKSGTSPKRWIYDTSKVASGVPYRFSGSGSGTSYSLTISSMEA EDAATYYCQQWSSNPLTFGAGTKLELK |
| 104 | Ad2 E1AΔ24 | MRHIICHGGVITEEMAASLLDQLIEEVLADNLPPPSHFEPPTLHELYDLDVTAPEDPNEEA VSQIFPESVMLAVQEGIDLFTFPPAPGSPEPPHLSRQPEQPEQRALGPVSMPNLVPEVID PPSDDEDEEGEEFVLDYVEHPGHGCRSCHYHRRNTGDPDIMCSLCYMRTCGMFVYSP VSEPEPEPEPEPEPEPARPTRRPKLVPAILRRPTSPVSRECNSSTDSCDSGPSNTPPEIHPV VPLCPIKPVAVRVGGRRQAVECIEDLLNESGQPLDLSCKRPRP |
| 105 | Amino acids 121-128 of Ad E1A protein | LTCHEACF |
| 106 | SignalP 1 | MDWIWRILFLVGAATGAHS |
| 107 | SignalP 2 | MEAPAQLLFLLLLWLPDTTG |
| 108 | SignalP 3 | MGHQQLVISWFSLVFLASPLVA |
| 109 | G4S linker | GGGGS |
| 110 | (G4S)2 linker | GGGGSGGGGS |
| 111 | (G4S)3 linker | GGGGSGGGGSGGGGS |
| 112 | (G4S)4 linker | GGGGSGGGGSGGGGSGGGGS |
| 113 | HA tag | YPYDVPDYA |
| 114 | 3xHA tag | YPYDVPDYAGYPYDVPDYAGYPYDVPDYA |
| 115 | T2A peptide | EGRGSLLTCGDVEENPGP |
| 116 | 2A cleavage sequence consensus | X₁EX₂NPGP |
| | | WHEREIN X₁ = V OR I, AND X₂ = S OR E |
| 117 | HER2(C5)-CD28TM,ICD-CD3Z CAR | AAGTTCAGATCAAGGTCAGGAACAGATGGAACAGCTGAATATGGGCCAAACAGGAT ATCTGTGGTAAGCAGTTCCTGCCCCGGCTCAGGGCCAAGAACAGATGGAACAGCTG AATATGGGCCAAACAGGATATCTGTGGTAAGCAGTTCCTGCCCCGGCTCAGGGCCA AGAACAGATGGTCCCCAGATGCGGTCCAGCCCTCAGCAGTTTCTAGAGAACCATCA GATGTTTCCAGGGTGCCCCAAGGACCTGAAATGACCCTGTGCCTTATTTGAACTAAC CAATCAGTTCGCTTCTCGCTTCTGTTCGCGCGCTTCTGCTCCCCGAGCTCAATAAAA GAGCCCACAACCCCTCACTCGGCGCGCCAGTCCTCCGATTGACTGAGTCGCCCGG GTACCCGTGTATCCAATAAACCCTCTTGCAGTTGCATCCGACTTGTGGTCTCGCTGT TCCTTGGGAGGGTCTCCTCTGAGTGATTGACTACCCGTCAGCGGGGGTCTTTCATTT GGGGGCTCGTCCGGGATCGGGAGACCCCTGCCCAGGGACCACCGACCCACCACC GGGAGGTAAGCTGGCCAGCAACTTATCTGTGTCTGTCCGATTGTCTAGTGTCTATGA CTGATTTTATGCGCCTGCGTCGGTACTAGTTAGCTAACTAGCTCTGTATCTGGCGGA CCCGTGGTGGAACTGACGAGTTCGGAACACCCGGCCGCAACCCTGGGAGACGTCC CAGGGACTTCGGGGGCCGTTTTTGTGGCCCGACCTGAGTCCTAAAATCCCGATCGT TTAGGACTCTTTGGTGCACCCCCCTTAGAGGAGGGATATGTGGTTCTGGTAGGAGA CGAGAACCTAAAACAGTTCCCGCCTCCGTCTGAATTTTTGCTTTCGGTTTGGGACCG AAGCCGCGCCGCGCGTCTTGTCTGCTGCAGCATCGTTCTGTGTTGTCTCTGTCTGA CTGTGTTTCTGTATTTGTCTGAAAATATGGGCCCGGGCTAGCCTGTTACCACTCCCT TAAGTTTGACCTTAGGTCACTGGAAAGATGTCGAGCGGATCGCTCACAACCAGTCG GTAGATGTCAAGAAGAGACGTTGGGTTACCTTCTGCTCTGCAGAATGGCCAACCTTT AACGTCGGATGGCCGCGAGACGGCACCTTTAACCGAGACCTCATCACCCAGGTTAA GATCAAGGTCTTTTCACCTGGCCCGCATGGACACCCAGACCAGGTCCCCTACATCG TGACCTGGGAAGCCTTGGCTTTTGACCCCCCTCCCTGGGTCAAGCCCTTTGTACAC CCTAAGCCTCCGCCTCCTCTTCCTCCATCCGCCCCGTCTCTCCCCCTTGAACCTCCT CGTTCGACCCCGCCTCGATCCTCCCTTTATCCAGCCCTCACTCCTTCTCTAGGCGCC CCCATATGGCCATATGAGATCTTATATGGGGCACCCCCGCCCCTTGTAAACTTCCCT GACCCTGACATGACAAGAGTTACTAACAGCCCCTCTCTCCAAGCTCACTTACAGGCT CTCTACTTAGTCCAGCACGAAGTCTGGAGACCTCTGGCGGCAGCCTACCAAGAACA ACTGGACCGACCGGTGGTACCTCACCCTTACCGAGTCGGCGACACAGTGTGGGTCC GCCGACACCAGACTAAGAACCTAGAACCTCGCTGGAAAGGACCTTACACAGTCCTG CTGACCACCCCCACCGCCCTCAAAGTAGACGGCATCGCAGCTTGGATACACGCCGC CCACGTGAAGGCTGCCGACCCCGGGGGTGGACCATGACTCGAGCCATGGATTGGA TCTGGCGCATCCTGTTTCTCGTGGGAGCTGCCACAGGCGCCCATTCTCAGGTTCAG CTGCAAGAGTCTGGCCCTGGCCTGGTCAAGCCTAGCGAAACACTGAGCCTGACCTG TACCGTGTCTGGCGGCAGCATCAGCAGCAGCTCTTACTACTGGGGCTGGATCAGAC AGCCTCCTGGCAAAGGCCTGGAATGGATCGGCTCCATCTACTACAGCGGCAGCACC TACTACAACCCCAGCCTGAAGTCCAGAGTGACCATCAGCGTGGACACCAGCAAGAA CCAGTTCTCCCTGAAGCTGAGCAGCGTGACAGCCGCCGATACAGCCGTGTACTACT GTGCCAGATACGCCCCTGATAGCAGCGGCTACCTGGTGGCCTTTGATATCTGGGGC CAGGGCACAATGGTCACCGTTTCTAGCGGAGGCGGAGGTTCTGGTGGCGGAGGAA GTGGCGGCGGAGGATCTCAGACAGTGGTCACACAAGAGCCCAGCTTCTCCGTGTCT CCTGGCGGAACAGTGACCCTGACATGTGGCCTTAGCTCTGGCTCTGTGTCCACCGG CTACTACCCCAGCTGGTATCAGCAGACACCTGGACAGGCCCCTCGGACACTGATCT ACAGCACCAACAGCAGATCCAGCGGCGTGCCCGATAGATTCAGCGGCTCTATCCTG GGCAACAAGGCCGCACTGACAATCACAGGCGCTCAGGCCGATGACGAGAGCGACT ACTACTGCGTGCTGTACATGGGCAGCGGCATCTCCGTTTTTGGCGGAGGCACAAAG CTGACCGTGCTGGGATCCGAACCAAAGAGTTGCGACAAAACACACACCTGCCCTAC GCGTTTTTGGGTGCTCGTGGTGGTGGGTGGCGTGCTCGCTTGCTACTCACTTCTGG TGACCGTAGCGTTTATCATTTTTTGGGTCAGGAGCAAGCGATCCCGCCTATTGCACA GCGACTACATGAACATGACCCCCCGGCGCCCCGGGCCAACCCGGAAGCACTACCA GCCATATGCGCCTCCCCGCGATTTCGCAGCGTATCGGTCCCGGGTCAAATTTTCAC GGTCCGCTGACGCCCCGGCCTATCAACAGGGCCAGAATCAGCTGTATAATGAATTA AACCTCGGTAGACGCGAGGAGTACGACGTCCTCGACAAGAGAAGGGGGCGCGACC CAGAGATGGGAGGCAAACCGCAGCGCAGGAAGAATCCACAGGAGGGCCTGTACAA CGAATTACAGAAGGACAAGATGGCAGAGGCCTACAGCGAGATAGGAATGAAGGGTG AAAGGCGTCGTGGAAAGGGCCACGATGGGCTTTACCAGGGCCTAAGTACTGCCACA AAAGATACGTATGACGCGCTGCATATGCAAGCCCTCCCCCCCAGGTAAGCATGCAA CCTCGATCCGGATTAGTCCAATTTGTTAAAGACAGGATATCAGTGGTCCAGGCTCTA GTTTTGACTCAACAATATCACCAGCTGAAGCCTATAGAGTACGAGCCATAGATAAAAT AAAAGATTTTATTTAGTCTCCAGAAAAAGGGGGGAATGAAAGACCCCACCTGTAGGT TTGGCAAGCTAGCTTAAGTAACGCCATTTTGCAAGGCATGGAAAAATACATAACTGA GAATAGAGAAGTTCAGATCAAGGTCAGGAACAGATGGAACAGCTGAATATGGGCCA AACAGGATATCTGTGGTAAGCAGTTCCTGCCCCGGCTCAGGGCCAAGAACAGATGG AACAGCTGAATATGGGCCAAACAGGATATCTGTGGTAAGCAGTTCCTGCCCCGGCT CAGGGCCAAGAACAGATGGTCCCCAGATGCGGTCCAGCCCTCAGCAGTTTCTAGAG AACCATCAGATGTTTCCAGGGTGCCCCAAGGACCTGAAATGACCCTGTGCCTTATTT GAACTAACCAATCAGTTCGCTTCTCGCTTCTGTTCGCGCGCTTC |
| 118 | HER2(E4)-CD28TM,ICD-CD3Z CAR | AAGTTCAGATCAAGGTCAGGAACAGATGGAACAGCTGAATATGGGCCAAACAGGAT ATCTGTGGTAAGCAGTTCCTGCCCCGGCTCAGGGCCAAGAACAGATGGAACAGCTG AATATGGGCCAAACAGGATATCTGTGGTAAGCAGTTCCTGCCCCGGCTCAGGGCCA AGAACAGATGGTCCCCAGATGCGGTCCAGCCCTCAGCAGTTTCTAGAGAACCATCA GATGTTTCCAGGGTGCCCCAAGGACCTGAAATGACCCTGTGCCTTATTTGAACTAAC CAATCAGTTCGCTTCTCGCTTCTGTTCGCGCGCTTCTGCTCCCCGAGCTCAATAAAA GAGCCCACAACCCCTCACTCGGCGCGCCAGTCCTCCGATTGACTGAGTCGCCCGG GTACCCGTGTATCCAATAAACCCTCTTGCAGTTGCATCCGACTTGTGGTCTCGCTGT TCCTTGGGAGGGTCTCCTCTGAGTGATTGACTACCCGTCAGCGGGGGTCTTTCATTT GGGGGCTCGTCCGGGATCGGGAGACCCCTGCCCAGGGACCACCGACCCACCACC GGGAGGTAAGCTGGCCAGCAACTTATCTGTGTCTGTCCGATTGTCTAGTGTCTATGA CTGATTTTATGCGCCTGCGTCGGTACTAGTTAGCTAACTAGCTCTGTATCTGGCGGA CCCGTGGTGGAACTGACGAGTTCGGAACACCCGGCCGCAACCCTGGGAGACGTCC CAGGGACTTCGGGGGCCGTTTTTGTGGCCCGACCTGAGTCCTAAAATCCCGATCGT TTAGGACTCTTTGGTGCACCCCCCTTAGAGGAGGGATATGTGGTTCTGGTAGGAGA CGAGAACCTAAAACAGTTCCCGCCTCCGTCTGAATTTTTGCTTTCGGTTTGGGACCG AAGCCGCGCCGCGCGTCTTGTCTGCTGCAGCATCGTTCTGTGTTGTCTCTGTCTGA CTGTGTTTCTGTATTTGTCTGAAAATATGGGCCCGGGCTAGCCTGTTACCACTCCCT TAAGTTTGACCTTAGGTCACTGGAAAGATGTCGAGCGGATCGCTCACAACCAGTCG GTAGATGTCAAGAAGAGACGTTGGGTTACCTTCTGCTCTGCAGAATGGCCAACCTTT AACGTCGGATGGCCGCGAGACGGCACCTTTAACCGAGACCTCATCACCCAGGTTAA GATCAAGGTCTTTTCACCTGGCCCGCATGGACACCCAGACCAGGTCCCCTACATCG TGACCTGGGAAGCCTTGGCTTTTGACCCCCCTCCCTGGGTCAAGCCCTTTGTACAC CCTAAGCCTCCGCCTCCTCTTCCTCCATCCGCCCCGTCTCTCCCCCTTGAACCTCCT CGTTCGACCCCGCCTCGATCCTCCCTTTATCCAGCCCTCACTCCTTCTCTAGGCGCC CCCATATGGCCATATGAGATCTTATATGGGGCACCCCCGCCCCTTGTAAACTTCCCT GACCCTGACATGACAAGAGTTACTAACAGCCCCTCTCTCCAAGCTCACTTACAGGCT CTCTACTTAGTCCAGCACGAAGTCTGGAGACCTCTGGCGGCAGCCTACCAAGAACA ACTGGACCGACCGGTGGTACCTCACCCTTACCGAGTCGGCGACACAGTGTGGGTCC GCCGACACCAGACTAAGAACCTAGAACCTCGCTGGAAAGGACCTTACACAGTCCTG CTGACCACCCCCACCGCCCTCAAAGTAGACGGCATCGCAGCTTGGATACACGCCGC CCACGTGAAGGCTGCCGACCCCGGGGGTGGACCATGACTCGAGCCATGGATTGGA TCTGGCGCATCCTGTTTCTCGTGGGAGCTGCCACAGGCGCCCATTCTCAGGTTCAG CTGCAACAGTGGGGAGCCGGACTGCTGAAGCCTAGCGAAACACTGAGCCTGACCT GTGCCGTGTACGGCGGCAGCTTTAGCGGCTACTACTGGTCCTGGATCAGACAGCCT CCTGGCAAAGGCCTGGAATGGATCGGCGAGATCAATCACAGCGGCAGCACCAACTA CAACCCCAGCCTGAAGTCCAGAGTGACCATCAGCGTGGACACCAGCAAGAACCAGT TCTCCCTGAAGCTGAGCAGCGTGACCACAGCCGATACCGCCGTGTACTACTGTGCC CGGATGGGCATCAATAGCGGCGGCTACCTGTACGGCATGGATGTGTGGGGACAGG GCACCACCGTGACAGTTTCTAGCGGAGGCGGAGGTTCTGGTGGCGGAGGAAGTGG CGGCGGAGGATCTCAGACAGTGGTCACACAAGAGCCCAGCTTCTCCGTGTCTCCTG GCGGAACAGTGACCCTGACATGTGGCCTTAGCAGCGGCTCTGTGTCCACCAGCTAC TACCCTAGCTGGTATCAGCAGATCCCCGGACAGGCCCCTCGGACACTGATCTACAC CACCAACATCAGATCCAGCGGCGTGCCCGATAGATTCGGCGGATCTATCCTGGGCA ACAAGGCCGCACTGACAATCACAGGTGCCCAGGCCGAGGACGAGTCCGACTACTAC TGCATGCTGTACATGGGCAGCGGCATCTGGGTTTTCGGCGGAGGCACAAAGCTGAC CGTTCTGGGATCCGAACCAAAGAGTTGCGACAAAACACACACCTGCCCTACGCGTTT TTGGGTGCTCGTGGTGGTGGGTGGCGTGCTCGCTTGCTACTCACTTCTGGTGACCG TAGCGTTTATCATTTTTTGGGTCAGGAGCAAGCGATCCCGCCTATTGCACAGCGACT ACATGAACATGACCCCCCGGCGCCCCGGGCCAACCCGGAAGCACTACCAGCCATAT GCGCCTCCCCGCGATTTCGCAGCGTATCGGTCCCGGGTCAAATTTTCACGGTCCGC TGACGCCCCGGCCTATCAACAGGGCCAGAATCAGCTGTATAATGAATTAAACCTCGG TAGACGCGAGGAGTACGACGTCCTCGACAAGAGAAGGGGGCGCGACCCAGAGATG GGAGGCAAACCGCAGCGCAGGAAGAATCCACAGGAGGGCCTGTACAACGAATTACA GAAGGACAAGATGGCAGAGGCCTACAGCGAGATAGGAATGAAGGGTGAAAGGCGT CGTGGAAAGGGCCACGATGGGCTTTACCAGGGCCTAAGTACTGCCACAAAAGATAC GTATGACGCGCTGCATATGCAAGCCCTCCCCCCCAGGTAAGCATGCAACCTCGATC CGGATTAGTCCAATTTGTTAAAGACAGGATATCAGTGGTCCAGGCTCTAGTTTTGACT CAACAATATCACCAGCTGAAGCCTATAGAGTACGAGCCATAGATAAAATAAAAGATTT TATTTAGTCTCCAGAAAAAGGGGGGAATGAAAGACCCCACCTGTAGGTTTGGCAAGC TAGCTTAAGTAACGCCATTTTGCAAGGCATGGAAAAATACATAACTGAGAATAGAGAA GTTCAGATCAAGGTCAGGAACAGATGGAACAGCTGAATATGGGCCAAACAGGATAT CTGTGGTAAGCAGTTCCTGCCCCGGCTCAGGGCCAAGAACAGATGGAACAGCTGAA TATGGGCCAAACAGGATATCTGTGGTAAGCAGTTCCTGCCCCGGCTCAGGGCCAAG AACAGATGGTCCCCAGATGCGGTCCAGCCCTCAGCAGTTTCTAGAGAACCATCAGA TGTTTCCAGGGTGCCCCAAGGACCTGAAATGACCCTGTGCCTTATTTGAACTAACCA ATCAGTTCGCTTCTCGCTTCTGTTCGCGCGCTTC |
| 119 | HER2(F1)-CD28TM,ICD-CD3Z CAR | AAGTTCAGATCAAGGTCAGGAACAGATGGAACAGCTGAATATGGGCCAAACAGGAT ATCTGTGGTAAGCAGTTCCTGCCCCGGCTCAGGGCCAAGAACAGATGGAACAGCTG AATATGGGCCAAACAGGATATCTGTGGTAAGCAGTTCCTGCCCCGGCTCAGGGCCA AGAACAGATGGTCCCCAGATGCGGTCCAGCCCTCAGCAGTTTCTAGAGAACCATCA GATGTTTCCAGGGTGCCCCAAGGACCTGAAATGACCCTGTGCCTTATTTGAACTAAC CAATCAGTTCGCTTCTCGCTTCTGTTCGCGCGCTTCTGCTCCCCGAGCTCAATAAAA GAGCCCACAACCCCTCACTCGGCGCGCCAGTCCTCCGATTGACTGAGTCGCCCGG GTACCCGTGTATCCAATAAACCCTCTTGCAGTTGCATCCGACTTGTGGTCTCGCTGT TCCTTGGGAGGGTCTCCTCTGAGTGATTGACTACCCGTCAGCGGGGGTCTTTCATTT GGGGGCTCGTCCGGGATCGGGAGACCCCTGCCCAGGGACCACCGACCCACCACC GGGAGGTAAGCTGGCCAGCAACTTATCTGTGTCTGTCCGATTGTCTAGTGTCTATGA CTGATTTTATGCGCCTGCGTCGGTACTAGTTAGCTAACTAGCTCTGTATCTGGCGGA CCCGTGGTGGAACTGACGAGTTCGGAACACCCGGCCGCAACCCTGGGAGACGTCC CAGGGACTTCGGGGGCCGTTTTTGTGGCCCGACCTGAGTCCTAAAATCCCGATCGT TTAGGACTCTTTGGTGCACCCCCCTTAGAGGAGGGATATGTGGTTCTGGTAGGAGA CGAGAACCTAAAACAGTTCCCGCCTCCGTCTGAATTTTTGCTTTCGGTTTGGGACCG AAGCCGCGCCGCGCGTCTTGTCTGCTGCAGCATCGTTCTGTGTTGTCTCTGTCTGA CTGTGTTTCTGTATTTGTCTGAAAATATGGGCCCGGGCTAGCCTGTTACCACTCCCT TAAGTTTGACCTTAGGTCACTGGAAAGATGTCGAGCGGATCGCTCACAACCAGTCG GTAGATGTCAAGAAGAGACGTTGGGTTACCTTCTGCTCTGCAGAATGGCCAACCTTT AACGTCGGATGGCCGCGAGACGGCACCTTTAACCGAGACCTCATCACCCAGGTTAA GATCAAGGTCTTTTCACCTGGCCCGCATGGACACCCAGACCAGGTCCCCTACATCG TGACCTGGGAAGCCTTGGCTTTTGACCCCCCTCCCTGGGTCAAGCCCTTTGTACAC CCTAAGCCTCCGCCTCCTCTTCCTCCATCCGCCCCGTCTCTCCCCCTTGAACCTCCT CGTTCGACCCCGCCTCGATCCTCCCTTTATCCAGCCCTCACTCCTTCTCTAGGCGCC CCCATATGGCCATATGAGATCTTATATGGGGCACCCCCGCCCCTTGTAAACTTCCCT GACCCTGACATGACAAGAGTTACTAACAGCCCCTCTCTCCAAGCTCACTTACAGGCT CTCTACTTAGTCCAGCACGAAGTCTGGAGACCTCTGGCGGCAGCCTACCAAGAACA ACTGGACCGACCGGTGGTACCTCACCCTTACCGAGTCGGCGACACAGTGTGGGTCC GCCGACACCAGACTAAGAACCTAGAACCTCGCTGGAAAGGACCTTACACAGTCCTG CTGACCACCCCCACCGCCCTCAAAGTAGACGGCATCGCAGCTTGGATACACGCCGC CCACGTGAAGGCTGCCGACCCCGGGGGTGGACCATGACTCGAGCCATGGATTGGA TCTGGCGCATCCTGTTTCTCGTGGGAGCTGCCACAGGCGCCCATTCTCAGGTTCAG CTGGTGGAATCTGGCCCTGGCCTGGTTAAGCCTAGCGGCACACTGTCTCTGACCTG TGCTGTGTCTGGCGGCAGCATCAGCAGCAGCAATTGGTGGTCTTGGGTCCGACAGC CTCCTGGCAAAGGCCTGGAATGGATCGGCGAGATCTACCACAGCGGCAGCACCAAC TACAACCCCAGCCTGAAGTCCAGAGTGACCATCAGCGTGGACACCAGCAAGAACCA GTTCTCCCTGAAGCTGAGCAGCGTGACAGCCGCCGATACAGCCGTGTACTACTGTG CCAGAATGGGAGCCAATAGCGGCGGCTACCTGTACGGCATGGATGTGTGGGGACA GGGCACCACCGTGACAGTTTCTAGCGGAGGCGGAGGTTCTGGTGGCGGAGGAAGT GGCGGCGGAGGATCTCAGACAGTGGTCACACAAGAGCCCAGCTTCTCCGTGTCTCC TGGCGGAACAGTGACCCTGACATGTGGCCTTAGCAGCGGCTCTGTGTCTACCAGCT ACTACCCCTCCTGGTATCAGCAGACCCCTGGACAGGCTCCCCGGACACTGATCTAC TCCACCAACACCAGATCCAGCGGCGTGCCCGATAGATTCTCCGGCTCTATCCTGGG CAACAAGGCCGCACTGACAATCACAGGCGCTCAGGCCGATGACGAGAGCGACTACT ACTGCGTGCTGTACATGGGCAGCGGCATCTGGGTTTTCGGCGGAGGCACAAAGCTG ACCGTTCTGGGATCCGAACCAAAGAGTTGCGACAAAACACACACCTGCCCTACGCG TTTTTGGGTGCTCGTGGTGGTGGGTGGCGTGCTCGCTTGCTACTCACTTCTGGTGA CCGTAGCGTTTATCATTTTTTGGGTCAGGAGCAAGCGATCCCGCCTATTGCACAGCG ACTACATGAACATGACCCCCCGGCGCCCCGGGCCAACCCGGAAGCACTACCAGCC ATATGCGCCTCCCCGCGATTTCGCAGCGTATCGGTCCCGGGTCAAATTTTCACGGT CCGCTGACGCCCCGGCCTATCAACAGGGCCAGAATCAGCTGTATAATGAATTAAAC CTCGGTAGACGCGAGGAGTACGACGTCCTCGACAAGAGAAGGGGGCGCGACCCAG AGATGGGAGGCAAACCGCAGCGCAGGAAGAATCCACAGGAGGGCCTGTACAACGA ATTACAGAAGGACAAGATGGCAGAGGCCTACAGCGAGATAGGAATGAAGGGTGAAA GGCGTCGTGGAAAGGGCCACGATGGGCTTTACCAGGGCCTAAGTACTGCCACAAAA GATACGTATGACGCGCTGCATATGCAAGCCCTCCCCCCCAGGTAAGCATGCAACCT CGATCCGGATTAGTCCAATTTGTTAAAGACAGGATATCAGTGGTCCAGGCTCTAGTT TTGACTCAACAATATCACCAGCTGAAGCCTATAGAGTACGAGCCATAGATAAAATAAA AGATTTTATTTAGTCTCCAGAAAAAGGGGGGAATGAAAGACCCCACCTGTAGGTTTG GCAAGCTAGCTTAAGTAACGCCATTTTGCAAGGCATGGAAAAATACATAACTGAGAA TAGAGAAGTTCAGATCAAGGTCAGGAACAGATGGAACAGCTGAATATGGGCCAAAC AGGATATCTGTGGTAAGCAGTTCCTGCCCCGGCTCAGGGCCAAGAACAGATGGAAC AGCTGAATATGGGCCAAACAGGATATCTGTGGTAAGCAGTTCCTGCCCCGGCTCAG GGCCAAGAACAGATGGTCCCCAGATGCGGTCCAGCCCTCAGCAGTTTCTAGAGAAC CATCAGATGTTTCCAGGGTGCCCCAAGGACCTGAAATGACCCTGTGCCTTATTTGAA CTAACCAATCAGTTCGCTTCTCGCTTCTGTTCGCGCGCTTC |
| 120 | HDAdIL-12_TK_PD-L1 | AAACATCATCAATAATATACCTTATTTTGGATTGAAGCCAATATGATAATGAGGGGGT GGAGTTTGTGACGTGGCGCGGGGCGTGGGAACGGGGCGGGTGACGTAGTAGTGT GGCGGAAGTGTGATGTTGCAAGTGTGGCGGAACACATGTAAGCGACGGATGTGGCA AAAGTGACGTTTTTGGTGTGCGCCGGTGTACACAGGAAGTGACAATTTTCGCGCGG TTTTAGGCGGATGTTGTAGTAAATTTGGGCGTAACCGAGTAAGATTTGGCCATTTTC GCGGGAAAACTGAATAAGAGGAAGTGAAATCTGAATAATTTTGTGTTACTCATAGCG CGTAATATTTGTCTAGGGCCGCGGGGACTTTGACCGTTTACGTGGAGACTCGCCCA GGTGTTTTTCTCAGGTGTTTTCCGCGTTCCGGGTCAAAGTTGGCGTTTTGATATCAA |
| | | GCTTATCGATACCGTAAACAAGTCTTTAATTCAAGCAAGACTTTAACAAGTTAAAAGG AGCTTATGGGTAGGAAGTAGTGTTATGATGTATGGGCATAAAGGGTTTTAATGGGAT AGTGAAAATGTCTATAATAATACTTAAATGGCTGCCCAATCACCTACAGGATTGATGT AAACATGGAAAAGGTCAAAAACTTGGGTCACTAAAATAGATGATTAATGGAGAGGAT GAGGTTGATAGTTAAATGTAGATAAGTGGTCTTATTCTCAATAAAAATGTGAACATAA GGCGAGTTTCTACAAAGATGGACAGGACTCATTCATGAAACAGCAAAAACTGGACAT TTGTTCTAATCTTTGAAGAGTATGAAAAATTCCTATTTTAAAGGTAAAACAGTAACTCA CAGGAAATACCAACCCAACATAAAATCAGAAACAATAGTCTAAAGTAATAAAAATCAA ACGTTTGCACGATCAAATTATGAATGAAATTCACTACTAAAATTCACACTGATTTTGTT TCATCCACAGTGTCAATGTTGTGATGCATTTCAATTGTGTGACACAGGCAGACTGTG GATCAAAAGTGGTTTCTGGTGCGACTTACTCTCTTGAGTATACCTGCAGTCCCCTTTC TTAAGTGTGTTAAAAAAAAAGGGGGATTTCTTCAATTCGCCAATACTCTAGCTCTCCA TGTGCTTTCTAGGAAACAAGTGTTAACCCACCTTATTTGTCAAACCTAGCTCCAAAGG ACTTTTGACTCCCCACAAACCGATGTAGCTCAAGAGAGGGTATCTGTCACCAGTATG TATAGTGAAAAAAGTATCCCAAGTCCCAACAGCAATTCCTAAAAGGAGTTTATTTAAA AAACCACACACACCTGTAAAATAAGTATATATCCTCCAAGGTGACTAGTTTTAAAAAA ACAGTATTGGCTTTGATGTAAAGTACTAGTGAATATGTTAGAAAAATCTCACTGTAAC CAAGTGAAATGAAAGCAAGTATGGTTTGCAGAGATTCAAAGAAAATATAAGAAAACCT ACTGTTGCCACTAAAAAGAATCATATATTAAATATACTCACACAATAGCTCTTCAGTCT GATAAAATCTACAGTCATAGGAATGGATCTATCACTATTTCTATTCAGTGCTTTGATGT AATCCAGCAGGTCAGCAAAGAATTTATAGCCCCCCTTGAGCACACAGAGGGCTACAA TGTGATGGCCTCCCATCTCCTTCATCACATCTCGAGCAAGACGTTCAGTCCTACAGA AATAAAATCAGGAATTTAATAGAAAGTTTCATACATTAAACTTTATAACAAACACCTCT TAGTCATTAAACTTCCACACCAACCTGGGCAATATAGTGAGACCCCATGCCTGCAAA AAAAAAAAAATTAGCCAGGCATGGTAGCATGTACCTGTAGTCCCAGCTACTTGAGAG GTGAGGTGGGAAAATCACTTTAGTGCAGGATGTTGAGGCTGGAGTGAACTGTGATT GTGCCACTGCACTCCAGCCTGGACAATAGAGCAAGACCTTGTCTCAAAAAAATGCAT TAAAAATTTTTTTTAAATCTTCCACGTATCACATCCTTTGCCCTCATGTTTCATAAGGT AAAAAATTTGATACCTTCAAAAAAACCAAGCATACCACTATCATAATTTTTTTTAAATG CAAATAAAAACAAGATACCATTTTCACCTATCAGACTGGCAGGTTCTGATTAAATGAA ATTTTCTGGATAATATACAATATTAAGAGAGACTGTAGAAACTGGGCCAGTGGCTCAT GCCTGTAATCCCAGCACTTTGGGAGGCTGGGTAACATGGCGAACCCTGTTTCTACAA AATAAAAATATTAGCTGGGAGTGGTGGCGCACACCTATAGTCCCAGCTACTCAGGAG GCTGAGGTGGAAGGATCGCTTGAACCCAGGAGGTTGAGACTGCAGTGAACTGTGAT CATTCTGCTGCACTGCACCCCAGCCTGGGCAACAGAGACCTTGTCTCAAAAAAAAAA AAAAAAGAGACAAATTGTGAAGAGAAAGGTACTCTCATATAACATCAGGAGTATAAAA TGATTCAACTTCTTAGAGGAAAATTTGGCAATACCAAAATATTCAATAAACTCTTTCCC CTTGACCCAGAAATTCCACTTGAATAAAGCTGAACAAGTACCAAACATGTAAAAGAAT GTTTCTTCTAGTACAGTCGGTAAGAACAAAATAGTGTCTATCAATAGTGGACTGGTTA AATCAGTTATGGTATCTCCATAAGACAGAATGCTATGCAACCTTTAAAATATATTAGAT AGCTCTAGACACACTAATATTAAAAGTGTCCAATAACATTTAAAACTATACTCATACGT TAAAATATAAATGTATATATGTACTTTTGCATATAGTATACATGCATAGGCCAGTGCTT GAGAAGAAATGTGTACAGAAGGCTGAAAGGAGAGAACTTTAGTCTTCTTGTTTATGG CCTCCATAGTTAGAATATTTTATAACACAAATATTTTGATATTATAATTTTAAAATAAAA ACACAGAATAGCCAGACATACAATGCAAGCATTCAATACCAGGTAAGGTTTTTCACT GTAATTGACTTAACAGAAAATTTTCAAGCTAGATGTGCATAATAATAAAAATCTGACCT TGCCTTCATGTGATTCAGCCCCAGTCCATTACCCTGTTTAGGACTGAGAAATGCAAG ACTCTGGCTAGAGTTCCTTCTTCCATCTCCCTTCAATGTTTACTTTGTTCTGGTCCCT ACAGAGTCCCACTATACCACAACTGATACTAAGTAATTAGTAAGGCCCTCCTCTTTTA TTTTTAATAAAGAAGATTTTAGAAAGCATCAGTTATTTAATAAGTTGGCCTAGTTTATG TTCAAATAGCAAGTACTCAGAACAGCTGCTGATGTTTGAAATTAACACAAGAAAAAGT AAAAAACCTCATTTTAAGATCTTACTTACCTGTCCATAATTAGTCCATGAGGAATAAAC ACCCTTTCCAAATCCTCAGCATAATGATTAGGTATGCAAAATAAATCAAGGTCATAAC CTGGTTCATCATCACTAATCTGAAAAAGAAATATAGCTGTTTCAATGAGAGCATTACA GGATACAAACATTTGATTGGATTAAGATGTTAAAAAATAACCTTAGTCTATCAGAGAA ATTTAGGTGTAAGATGATATTAGTAACTGTTAACTTTGTAGGTATGATAATGAATTATG TAAGAAAACAACAGGCCGGGCGGGTTGGTTCACACGTGTAATCCCAGCACTTTGGG AGGCTGAGGCAGGCAGACTGCCTGAGCTCAGGAGTTCGAGACCAGCCTGGGCAAC ACGGTGAAATCCCGTCTCTACTAAAAATACAAAAAAATTAGCCGGGTGTGGTGACAC ATGCCTGTAGTCCCAGCTACTTGGGAGGCTGAGGCAGGAGAATCACTTGAACCTGG GAGGTGAAGGTTGCAGTGAGCCAAGATGGCACCACTTCACTCCAGCCTGGGAAACA GAGCAAGACTCTGTCTCTGAGCTGAGATGGCACCACTTCACTCCAGCCTGGGAAAC AGAGCAAGACTCTGTCTCAAAAAAAACAAAACACACAAACAAAAAAACAGGCTGGGC GCGGTGGCTCACGCCTGTAATCCCAGCACTTTGGGAGGCCGAGGCGGGTGGATCA CCTGAGGTCAGGAGTTCCAGACCAGCCTTGTCAACATGGTGAAACCTCCCCCCGCC GTCTCTACTAAAAATACAAAAATTAGCCAGGCGTGGTGGCAGGAGCCTGTAATCCCA GCTACTTGGGAGGCTGAGGCAGGAGAATCGCTTGTACCCAGAAGGCAGAGGTTGCA CTGAGCTGAGATGGCACCATTGCACTCCAGCCTGGGGGACAAGAGCGAGATTTCGT CTTTAAAAAACAAAAACAAAACAAAAAACCATGTAACTATATGTCTTAGTCATCTTAGT CAAGAATGTAGAAGTAAAGTGATAAGATATGGAATTTCCTTTAGGTCACAAAGAGAAA |
| | | AAGAAAAATTTTAAAGAGCTAAGACAAACGCAGCAAAATCTTTATATTTAATAATATTC TAAACATGGGTGATGAACATACGGGTATTCATTATACTATTCTCTCCACTTTTGAGTA TGTTTGAAAATTTAGTAAAACAAGTTTTAACACACTGTAGTCTAACAAGATAAAATATC ACACTGAACAGGAAAAACTGGCATGGTGTGGTGGCTCACACTTGTAATCCCAGTGCT TTGGGAGGCTGAGACAGGAGAGTTGCTTGAGGCCAGGAGTTCAAGACCGACATGG GGAATGTAGCAAGACCCCGTCCCTACAAAAAACTTTGTAAAAATTTGCCAGGTATGG TGGTGCATACCTGTAGTCCCAGCTACTCGGGAGGCGGAGGCAGAAGGAATCACTTG AGCCCAGGAGTTTGAGGCTGCAGTGAGCTACGATCATACCACAGCACTCCAGCGTG GACAACAGAGTAAGACCCTATCTCAAAAACAAAACAAAACAAAACAAACAAAAAAAAC CACAAGAAAAACTGCTGGCTGATGCAGCGGCTCATGCCTGTAATCCCAGTATTTTGG GAGGCCCAGGTGGGCGTATCACCTGAGGTCAGGAGTTAGAGACCAGCCTGGCCAA CATGGTGAAACCCCATCTCTACTAAAAATACAAAATTAGCCAGGCATGTGGCACGCG CCTGTAGTCCCAGTTACTGGGAGGCTGAAGCAGGAGGATCACCTGAGCCCGGGAG GTGGAGGTTGCAGTGAGCCGAGATCACACCACTGCACTCCAGCCTGGGTGACACAG CAATACCCTACCTCAAAATAAAAAAGAAAAAGAAAAGAAAAGTTGCTGTCCCCGCTAC CCCAATCCCAAATCCAAACAGCCTCTCTCATCTCACAGTAAGGGGGAAAAATCACCC AAAAAAGCTAAGTGATCTTTTGAAAACCCAAACTCTTAGAAGTCTAAGATTATTATAGT CAACTCATGAAGTGTCATCATAAAAGATACTCTAATATTATTTAAGTAGAACCACATAT TGGTTGTCTTGGTATGTCTAGCCCCTGGCATACAAAATATTTAATAACACTGATATGG TACCTGTGATGTGAAAATGTACTATGAGTACAGCTTTATAAATACTATATATGTACCTA TATACAGAAAAAAATACAACAAAATCATAAAAGCACTTATCTTTGAAAGAGGAGTTAC AGCAATTTTATTTAGTTCTTTATTGCTTTGCTATATATTCTAAATTTTTTTCAATGAATAT ATATCACTTTTAAAAAAATTCAATGGTCTTTCTTATAAATTATCTTTGGCAGCATGCGT TTTTATATATACATATAAAATGTATGGGAAATTTTTAAAGGATACATTAAATTAAAGCAA AATATACAAACAAAAAATCAGAATACAAAAAGATAAAAAGATTGGGAAGGGAGGGAG GGAGTAAGGAGGAAGGGTGGGTGGGTATAGAGAAATATACCAAATAATGGTAAGAA GTGGGGTCTTGACACTTTCTACACTTTTTTTAAATAAAAAAAATTTTTTTCTCTCTCTTT TTTTTTTTTAGAGACGAAGTCTCGCTATGTTGCCCAGGCTGGTCTTGAACTCCTGGG ATCAAGAGATCCTCCTGCCTCAGCCTCCCAAGGTGCTTGGATTACAGGTGTGAGCC ACCACGCCTGGTCACTTTCTACACTTTAATATATATATTTTTTCATTTTCAATGTCATTT TTATTAGTTAATTTATAATACCCATTCACCATTATATTCAAAGTCTATTTGAAGAAATAA ACCAGAAAGAATGAAATACTCTAGCTCACATGCTATTCAATACTAAATTACCTTTCAAA TCACATTCAAGAAGCTGATGATTTAAGCTTTGGCGGTTTCCAATAAATATTGGTCAAA CCATAATTAAATCTCAATATATCAGTTAGTACCTATTGAGCATCTCCTTTTACAACCTA AGCATTGTATTAGGTGCTTAAATACAAGCAGCTTGACTTTTAATACATTTAAAAATACA TATTTAAGACTTAAAATCTTATTTATGGAATTCAGTTATATTTTGAGGTTTCCAGTGCT GAGAAATTTGAGGTTTGTGCTGTCTTTCAGTCCCCAAAGCTCAGTTCTGAGTTCTCA GACTTTGGTGGAACTTCATGTATTGTCAGGTTGGCCCGTAATACCTGTGGGACAACT TCAGCCCCTGTGCACATGGCCAGGAGGCTGGTTGCAAACATTTTCAGGTAGGTGGA CCAGGACATGCCCCTGGTCATGGCCAGGTGGAGGCATAGTGCTATACAGCAGGCA GAAGTCAATATTGATTTGTTTTTAAAGAAACATGTACTACTTTCATAAGCAGAAAAAAT TTCTATTCTTGGGGGAAAAGATTATGCCAGATCCTCTAGGATTAAATGCTGATGCATC TGCTAAACCTTCACATATCAGAACATATTTACTATAGAAAGAATGAAAATGGGACATT TGTGTGTCACCTATGTGAACATTCCAAAAATATTTTACAACAACTAAGTATTTTATAAA TTTTATGAACTGAAATTTAGTTCAAGTTCTAGGAAAATACAAACCTTGCTAGATATTAT AAAAATGATACAATATATATTCATTTCAGGCTCATCAGAATATATCTGTTATCACTTGA CAAGAATGAAAATGCACCATTTTGTAGTGCTTTAAAATCAGGAAGATCCAGAGTACTA AAAATGACTTCTTCCTTGAAGCTTACTCACCAACTTCCTCCCAGTTACTCACTGCTTC TGCCACAAGCATAAACTAGGACCCAGCCAGAACTCCCTTGAAATATACACTTGCAAC GATTACTGCATCTATCAAAATGGTTCAGTGCCTGGCTACAGGTTCTGCAGATCGACT AAGAATTTGAAAAGTCTTGTTTATTTCAAAGGAAGCCCATGTGAATTCTGCCCAGAGT TCATCCCAGATATGCAGTCTAAGAATACAGACAGATCAGCAGAGATGTATTCTAAAAC AGGAATTCTGGCAATATAACAAATTGATTTCCAATCAAAACAGATTTACATACCATACT TATGTCAAGAAGTTGTTTTGTTTTATTGCATCCTAGATTTTATTTTTTTGATTTATGGTT TACTTTAAGCATAAAAAATTTGTCAATACAACTCTTCCCAAAAGGCATAAACAAAAATT CATAAAACTTGCATCACTTGAGATACTTCAGGTATGAATTCACAACTTTGTTACAACTT ACTATATATATGCACACATATATATATATTTGGGTATATTGGGGGGGTTCTAATTTAAG AAATGCATAATTGGCTATAGACAGACAGTTGTCTGGAATGAAAATCAATACTTTTGCT ATAATCGATTACTGAAATAATTTTACTTTCCAGTAAAACTGGCATTATAATTTTTTTTAA TTTTTAAAACTTCATAATTTTTTGCCAGACTGACCCATGTAAACATACAAATTACTAAT AATTATGCACGTCACATCTGTAATAATGGCCTTCATGTAAACATTTTTGTGGTTTACAC ATAAAATCTCTAATTACAAAGCTATATTATCTAAAATTACAGTAAGCAAGAAAATTAAT CCAAGCTAAGACAATACTTGCAACATCAATTCATCATCTGTGACAAGGACTGCTTAAG TCTCTTTGTGGTTAAAAAGGAAAAAAAAAAAAAAGACATGTTGGCCAGATGCGGTGG CTCACACCTGTAATCCCAGCACTTTGGGAGGCTGAGGTGGGCGGATCACCCCTGGC CTGCCCAACATGGTGAAACCCCGTCTCTACTAAAAACACAAAAATTAGCTGGGCGTG GTGGCGGGCGCCTGTAATTCCAGCTACTCGGGAGGCTGAGGCAGGAGAATTGCTA GAACCCAGGAGGCAGAGATTGCAGTGAGCTGAGATTGCACCATTGCACTACAGTCT GGGCAACAAAAGTGAAACTCCATCTTAAAAAAAAAAAGACAATGTTCGTGGGTCCAA ACAAGACTTAATGGAAGTGAGTCTAAAAATGAGCTATGTGGGCCAGGCGTAGTGGCT |
| | | CCCACCTGTAATCCCAGCACTTTGGGAGGCCGAAGCAGGCAGATCATGAGGTCAGG AGATGGAGACCATCCTGGCCAACACGGTGAAATCCTGTCTCTACAAAAATTAGCTGG GCGTGGTGGTGCCTGCCTGTAATCCCAGCTACTCAGAAGGCTCAGGCAGGAGAATC GCTTGAACCAGGGAGTCGGTGGCTAGAGTGAGCCGAGATTTGCATCACTGCACTCC TGCCTGGTGACAGAGCAAGACTCCATCTCAAAAAAAACAAACAAAAATAAAAGATAAA AATGAGCTATGTGAATTAAAAGAGGTATAACAATAGATAAACCATATTTTATTTAATTC CTAGTAATGAGTAATATTTCCAAACTTCTGGAATGGGCAGAAATTGCTAGTTGGCATA TTTTTACCTTTTATATTCAGATACATTAAAATTCTCAAAAAAAAACACCTCAAAGCAGA TGATCCGCCATCTCCTTGGATAATTTGTGTTAACTCAGGATAACAGAAAACCAAAATT ATGAGTTACTGATGCAATATTCCTAAATGTAAAAATAATTAAAGCTAATAGTAGATTCA TCTTCCAATTTCATATCAGTCTTACAAATAAACTACATATATAACTTGCTTGCCTTCCC TTCTGAGGGATAAAGCTGTTAGAAGAATTAAAATCAGCATTCTTGACTATTCAACCAA GGGAGGGATAAATTATTACTCATTCTAGGGACATGGGCTCATAACTACTACATGTGT AAGGACATGAATTTACCCAATATTACAATTTTTCCTTTTATTAGTGTGTACAGTGGAAG AATAGACATGTTCACTCTGGACAAAAAAAAAATTATACTTATCAGTTATCAGAAGCAC AATGCTGAAGACAGTAGTTCCATAACAATTTGAAGTATGTGATCGAACTAGTAGATTA TCTTAGTAGTAGTGAATTATTGTAAATGTTAGTAATTTGGCAGCCACTGGGCAGAAAA ATAAGAATTGAGGCTCAATATTGATATTAATGGTGGTGATTGACACATAAATTTTATCA AGTCTACACAATATAAAATTACAGAAAGGTAGAAGAGTATACCAGTACAACTTCAACA TATCTTCACTACAAGGGAGTAAAATGACATGGCCTAGTTACTATCTAATGAACTGCAG AAAACTAAAAGAAAACTCCAAGGCAACTCTTCTCTGCTGATCTGGTTGGTCCTTTTCC TACCTTTTGCAATACCCAGATACAAACAATGGATAGAAAACAAAGTAGACTTGTAGTA TGCAGGTCACAGTGCTAAATTCACAGAAAGAAACCCCTGAACTGAACTGCTCTATTT CCTGGTGGTCACAAAGAGTAATTCTGGTTTACACCTACAGATTGATGTCAATCTACAC CCTGTTGATAACAGTGTGGCCAAGGACAAAAAAAAGGTGCTCCGTTTTACCAATTCT GTAAAAAATTATTGGCAGGGTAAGCTCGGCTAGGGCAGGATTACATTTCTAGGACTA CCATCCCCGAAATTTAGAAGATATTATATCCACATAAAGCATATCTTTCACATTAATTT GCAAAAATCTAAAAGCTTTTTCTTAGCTCAAGTGTGTCCAAGTTTACCCTGGCAGTTT AAAACGATAGTTACAAGCAGCATGGGTTGTATCAGACACATTTGAGGGCCAATTTCA TGTAAGTGATATTGGGCAAGTTACTTCAACTATCTGTGCCTCCAAGGTCATACTAGTG TTTATTTACCTAAAGGGTACCTGTTATGTAACTTTAGGGTGTTTACATTAGATAATGCC TGCAAAATATTTACTTCAACGCCTAAAACATAGTTAAGTATTCAATAAATACCTACTAT TGTCACTACTAACTTAAAAGTTTAGAGATTAAGAGCAGAATCTGGGGTGAGACAAACT TAGGTTCAAATCCTAGTATTGTTGGGTAATCTTGGGCAAGTTACTTAACCTCTCTGAT TTGTGTAATTTAAAAAATTAGTTAATATACATAACAGGGCTTAGAAGAGTATCTAGCAC ATAGCACCATTTAAGCATTTGTTATTGCTAACATGCAAACAATTTAAGGGAAAGAAAT TTTTTAAAAAGGAAGAGGGATTTGCAAACTAAAAACAATGAGTATCTTATGTTCAAAG AAAACTAACAAACAGCCAGCTCTAGCAATAATTAAATTCACTATATACTGGGGCAGGC ATCACACCCCAAAGCTAAAAGCGTCTACCTAGGCCAGGCACGGTGGCTCATGCCTG TAATCCCAGCACTTTGGGAAGCAGAGGCGGGCAGATCGCTTGAGCTCAGGAGTTCA AGACCAGCCTGGACAACATGGCAAAACACCATCTCTACAAAAAATACAAATATTAGG CCGGGCGCAGTGGCTCACGCCTGTAATCCCAGCACTTTGGGAGGCCAAGGCGGGT GGATCACCTGAGATCAGGAGTTCGAGAGTAGCCTGGCCAACATGGTGAAACCTCGT CTCTATTAAAAATACAAAAAATTAGCCAGGCATGGTGGCAGGCGCCTGTAATCCCAG CTACTCAGGGGGATGAGGTAGGAGAATCGCTTGAACCCGGGAGGCAGAGGTTGCA CTGAGCCGAGATCATGCCACTGTACTCCAGCCCGGGCAACAAGAGCGAAACTCCAT CTCAAAAAATAAATAAATAAATAAATAAAATAAAGTACAAATATTAGCCAGGGATGGT GGTGCGCACCTGTAGTCCCAGCTACTTGGGAGGCTGAAGTGGGAGAATCCCCTGAG CCTGGGGAGAATCACCCGAGCCCGGGAAGTCGAGGCTGCAGTGAGCAGTGATTGT GCCACTGCACTCCATCCTAGGTGACAGAGTGAGACCCTGTCTCAAAAAAAAGAAATT GGCAGAATTAAGTAAGTTGATGTTTAGAGATGAAAAATCAACATTTTTTCCTCAGCAA CTGAATAAAAACAACAGCCACTACCATTTTTTTGAGTACCTATTTGTAGCCTATTTTTT AACTGGTATTACTCGAGAGAGAGAGAGCTAGGTTCGAGACAGAGCTCCTTCTCTTAA TAACTGTATGACCTAGGGTATGTCTGTTAGCCTCTCTGAGGCTTCAAAGGTTCCTCAT CTGTAAAATGGTAATAATCATACCATTGCTACAGGGCTGTTTTGAAGACTAATTAGGA CTATGTAAGTAAACATGATGATGGCTATTATTACTGTTCCCCGCCAGGGGCCATGCA AGGGTTGCTGATTCACATAGACTGTCTTATAATCCTCTCAATAACTCCAAGAGGTAGC CAGCACCTCAGATATACATAAAATGACTTAAGCCCAGAGAGGTGAAGTAAGTTGCCC ACAGCCACACAACTAGTAAATAGCCCAAACAAGCTGGATTCCCAGTTAGACTCCGTT AATAGCACTGCTCTTTACCTTAAGTCATTACAATGCCTAATATGAAATAGAATCGCTT CTTTCTTAGGGTTCAAGTGGTTAATTATTTAATGTATTCATTCAACAAACCATCATCGA GGACCTCTTACAAGCCAAGTACTGTGCTAAGTGCTAGAGTTACGGCGGTGATTCCTG CCCTTAAAAAGTTTTAGTGGGAGAAACAACAGGTAACCAGGTCATTGCCAAAACAAC AAAAATAATCATAATAAAGCAGGCTAAAGCATATTTAACTGGCCGGGGTTTTGACTAT TTTAGCAAGCATGATCAGAACGGTTGAGGAGGGAGGCCAGCAGCTTGGCCGGTTCA ACAAACAAGAAAAAACCAGTGAGGGTGGAGCTAAGATACCAGAGGCTGATTACGGT TAAGAATGTTCTTGAAGGTAAGGACCAGATTCTCATTTTCTATATCCTGGGGCATCGG TCAGCATGGAATCTGGATTCTAGCACATGTGAATTTCGGCTTGAAATGACCTAATGC CTTTTCCCTAGTTCCTTCGTGTGTCAAATACGCATGGTTACCGCTACCAGAGCTGTA GTGGGGCTTCAATGAGGCCATGAGCATCTCCATAAAGATGAACTACAGTGTGTGCAA |
| | | AACTAAAGGCAAAACCTGGTCCCCACACGCCCTCCCAGGTGGTCGCTTTCCGTGCC GAGGCCCCTCCAGAGGTGCCCCGAGAACCTCACCATCGCACCCCAAACTTCCAGG GAAGGGCCTCTCCCGAGAAAGCCCCCACGCCCCCACCCCGCGCCATCATTCCCGA ATCTGCCCTCGGCCCCTCCCCGCAGCACGCTCGCAGGCGGCACATGTCAACCAAAA CGCCATTTCCACCTTCTCTTCCCACACGCAGTCCTCTTTTCCCAGGGCTCCCCCGAG GAGGGACCCACCCCAAACCCCGCCATTCCGTCCTCCCTGCCGCCCTCGCGTGACG TAAAGCCGAACCCGGGAAACTGGCCGCCCCCGCCTGCGGGGTTCCCTGGGCCCGG CCGCTCTAGAACTAGTGGATCCCAATTGAAGGCCTGGTCTAAATGACTCCAAAATCA CCACTTAATTCAAGAGACTGATTTCCCTGAGTCAGGCCCCTTAAAGCAGCTATTTCAA TGGGACAGGGAAACAACCCTAGGATCTGGATTAGAATCACTTGGGGGCTGCCACAC CCCCAGGGCTCTGATCCTGCCCTTCTCCCACACGCACATTCACATACTGCTGCAGTG ACCTTCCATTTCTAATGGGTTCCTGGGCCATCTGTCAGGTATAGGGAATGGAAAAGG GGTTGGGGAGGCTCTGCTTCAGAAAGTTTGTGTCAGGGGCTCCCAGAGCCTCCACA GATAGATAGCAGGGGTCCCCACCCTACCATGGCAGCTATAAATGTGATCAACATTTA TTGGCCTAGGATACAGCAGTTAGCAAAATGCCTGATGTAGTTCCCACTCCGTGGAGG TTGCAGGCTAGCTCTTTCCTAATGAGCTTTACAGCAGAAGCTGTTTTATCGTTAAGTG CCCCACAGAGACACTTTACCAGGAGGCTGGGAGAGTTCTCCAGATTTGGGAGAGGC GCAGAGACAGTGTGTGAGCCGAGCCCTGTCTCAGCAATCCACCTGGAGGAGCTAGA GTATCCTCCTCCCTTTACCATTCAGACCGAGAGAAAAAGCCCAGCTTGTGTGCACCC TCGTGGGGTTAAGGCGAGCTGTTCCTGGTTTAAAGCCTTTCAGTATTTGTTTTGATGT AAGGCTCTGTGGTTTGGGGGGGAACATCTGTAAACATTATTAGTTGATTTGGGGTTT GTCTTTGATGGTTTCTATCTGCAATTATCGTCATGTATATTTAAGTGTCTGTTATAGAA AACCCACACCCACTGTCCTGTAAACTTTTCTCAGTGTCCAGACTTTCTGTAATCACAT TTTAATTGCCACCTCGTATTTCACCTCTACATTTGAAATCTGGCGTCTGTTTCAAGCC AGTGTGTTTTTTCTTCGTTCTGTAATAAACAGCCAGGAGAAAAGTGCCTCTATGTTTT TATTTTTCAAGGGAGTATTCAGTACCTACAAACCCAAGTCAGGAAGCCTGCTAGTGG CTTTGGTTCTTTCAGAGGCTGCTCGATGCCTTGTGTGTCAGAAAGAAAGATTCAGCA GTTTTGCATCATGGCAAAGAAGCCTGTTATTTTGGGGCTCAGCCCCTCATTTTATAGA GGATGAAACAGAGGGGGATGGGAGGTCACAAAGACAACTGCCCCGGGAGCAGGTG TGGGGGAGACTTGCCCTGAGGGTCTAGACGCTCTGCACCACCGTCCTGTCTCCCTT GCTGAAGACCACACATGCCCTTCTTTGACCAGACCCTGCCACCTGATAGGCCAGGA CCTGGTAGGCGGGTACCCAGGTTTCATGGATGGAACCACATCTCCCCAAAAGTGGG GAGGTAGCTACTGGGATGCACGCCTCCCGCCATGTGCTATAGGAGAGCAGCTGAAG CAACAGTTGGGATCAGATGTAGTCACAATTGAATGCATCATCACATTTATCCCTCTAA GTGGCTGGGAGAGTTGATATCCTCATCCCTAAGGTACAAAATGTTCCAATTTGATCA GTGGCTTTCAGGAGCTGAGAAAGGCATGTGCTCTGAGGCAGAGCTGTTATGTCCCG CAGAGCCTAAAAATGCTCTAAGAACATGCTCCCTGCCAAAATTCTCAATGGCTGTGA CAAGGGACAACGATCGACCAATGGGGGTGGAAGCAGACCTCCGCAGTCCAGGGGC CAGAGCTAGGACAGAGGGGTCGGAGAAAGAGTCATTTTCCCAACACTCCAGCTCTT GGCCAGTCCTCACACAGTCCCCTCCTGCTTCCTGCTGAGAGAGATATCCTCATAGGT CTGGGTAAAGTCCTTCAGTCAGCTTTCATTCCCTGTCACCAACTTTGTCTCTGTTCTC CCTGCCCGTCTCAGGCAGCACTCCTCAGGAAACCTCTCCAAGAGCCAGCCTCACTG CAGCGCCCACTATTGTCCCTCTGCCTCAAGTGTCCCATCCATGCCAGGCCCCAGGC AGGCTGCAGCTTTCCCTCAGGGCCACACCAAAGCACTTGGGCTCAGCTGTGCTGTC CCCCTCCATCACTGAGCTCAGGGGCAGCAGGGGTGGGGTGCCAGGAGGCCCATTC ACCCTTCTCTGGCTCTGTGTTGGACCCACCTGCCCAGCCACTGCTGCTTAGAACCTA CCCGCTGGGAAAATGAAGCCCTCCCGGAGGGGCCACCTCAACCTGAGAGCCTCAC GGATCACAGTTGTCCCCACTCAGCTCTGCCAGCCCTCAGAGACCCATAGATAAAAG CTGAGCTTGGCTCGCAGAGCTGGTTCCATCTTCCATTCCCAGAGGGTTCAACTTCCT ACCCCAACCACACAGGGAACCTCAAGGCTGAGCCAGTGTGGGCTGCAGTGCAGAC CAGCTTCCTGGACACGTCCTGCCACCTGACCCCAGGCTGGCCTCACTGCCCCTGGC ACTCCTGACCCTATCCTCATTCCTCCTGGCAGTGCGTGTTCTGCCATTCCGCTTTCC CTTAGCTGTCCTCTCACTGTACTGTCAGCTTCTCCTTTTCCAGGTGCCCCCCAGGGG CTTTCCACATGACCCTGTCACCCCACAGCCCATCCAGCACCAATTCCAGCTCTCTGC CACCCTTCAAAGGAGTGACAGTGCCCTGCTTCACCTCCCACTCACCCCTCAACCCA GAGCAATCTGGCTCCAGTCTTGCCTCCTTCCCCCTAAGTACTCTAGTCACAGTTCCA AATTCCTCCTGGTCATAAAGCCAAATGAAGCTTCCTGGTCCTCAGCGGACTTGCCAC TTCAGCAGTACTGGACTCTCTCCTCCCAGAAACCTGTTTCCCCTTGGCTCCTGGAGC CCACACTCTGCTGGAATCCTTCTGCCTCTCTGGCCTGTAGCCTGGCCCTCTCTCCCA ACCTGAGGTCCATTCTCTCCTGCTCCTCCACAAGATGTTGCTCCTTCCATTACTTCCT CCCTCTCAACCAAAGCTCCTTCATTAGCTCTTTATCTTCTGGTTTCTTCCCCTGGGCA GACGAATGGATTCAAGAGCCTGTGGCCCAGCAGCCCAGCACTCCAGGATCTCAGCA CTTCAGCATCCCAGTACCCTAGCATCTCAATACCCCAGCACCCCAGCACCATAGTAT TCCAGCACCCCATTGTCCAAGCATCTCAGCACTCCAGCATCCCAGCACCCCAACACT CCAGCAGCCCAGAATCTCAGCACCCTAGCACTGCAGCATCTCAGGACCCCAGCACT TCAGCATCCCAGCACACTAGTACTCCAGCATCTCGGCACCCCAGCACCTAGGCATC CCAACACCCAGCACCCCAGCACTTAAGCATCCCACCACTACAGTATCTCAACACTCC AGCACCCCAGCACCATAGTGTTCCAGCACCCCAGCATCCCAACACCCCAGCACTTA AGCATCCCAACACCTCGGCATCCCAACACCCCAGCACTGCAGCATCTCAGCACCTT AGCATCCCAGTGCCCTAGCATCTCAATGCTCCAGCACACCAGTACTACAGTATTCCA |
| | | GCACCCCAGCACTCCAGCATCTCAGCACTGCAGCACTGCAGCACTCCAGCATCCCA AAATCCCAGCATCCCAACACCCCAGCAGACCAGCAGACCAGCATCTCAGCACCGCA GCATCCAAGGACTATCCCAGCATCCCAGCAACCCAGCACCTCAGCATCCCAACACC CCAGCATTTCAGCATGGCAACACCCCAGTACCCCAGCACTTCAGCACCCCAGTATC CCAGCATCTCAGCGACCCAGTATCACAAAACCTCAGCATCCTAGCACCCCAGCACC CCAGCACCTTAGCACCTTAGCATCCCAGCATCTCAGCGCCTCAGCATCTTGATATTC TGGCTGAGGTCAGCGTGGTGTATCTAGTCAGGGTCCTAACTTTCACTTCGCAGGGA AATGCTGCTGGACTGGGTCTCATGTTGGGCTGAAGCTCTCTAGACCCCTTGAAGACA GCATAAAAGAGCTTGGAGACGCTGGGTGTCCCCCATGGAAGAGTTCACTCTCATCC TGCTTTGACAACAGCCTTCTCTGGGGTCCCTCACGGGCCCCTCTTTCTTACTGCAAG TTTGTCTCTGAGAAGACTGTGATGCAGAAGTCACTCAGCTGCCTGTGGCTCCTGAAG AGCTGAAGGTGGAGGCCTGTAGGCCTCCCTATGAGAGGCGCAGAAAAAACCATGAT TGCTAGTGGGGAGGTGCTCCCTCTACAACCCACTCCATAATCTGCCCCCGCCCAGC TCTGAGGCCAGCCCCAGGGGAAAATGCCAGATCCCCAGGGAGGTGTGTGAGACCT CAGGGGCTCCCTCCTCCCTTACAGCAGGCTCAGGCCCCTGGGGGCCTCAGGGCCA AGGTCTGTGGGTAAGCTACTATCTCTCACTTGTCCTCTAGCCACAAAAGCCAGGGAG ATCTGGCAATGGACATGAGGTTCTGAAGAAGCACATATGACTGGCTTCCTAATGCGT GGTTGTTCAGTGATTCAATAAACACGCATGGGCCAGGCATGGGGAAATAGACAAACA TGATCCCCAACCTCTCCCAGAGTGAACTGGGAGGGAGGAGTGTTCATCCCTCAGGA TTACACCAGAGAAACAAACCAGCAGGAGATATATATGGTTTTGGGGGGTCAAGAAAG AGGAAAAACCTGGCAAGGCAAGTCCAAAATCATAGGACAGGCTGTCAGGAAGGGCA GCCTGGAACCTCTCAAGCAGGAGCTGATGCTGCAGTCCACAGGCAGAATTTCTTCTT CCTCGGGGAAATCTCAGCTTTGTTCTTAAGGCCTTTCAACTGATTGGCTGAGGTCTG CCCCTTCCCCCACATTCTCCAGGATAATCTTCCTTACTTAAAGTCAACTATTAATCAC AGCTACAAAATCCCTTCACAGCTACACATAGATCAGTGTTTGATTGACGAACAGCCC CTACAGCCTAGCCAAGTTGACACATAAAACTAACCATCACAGGGGGACAAATGATGT AAACACATCAACAAATAAAACAGTAACAAGTTAAGGTCTATGGAAAAAACACAGAAGG GGCAGAGAGAAAGAAAGCAAGAAGGAGAGTCCCAGTTTGCTAGGGCTTGTGGGAAG TGGGGAGCAGTTCTCTTTAGCTAGGATATTTGGGAAAGGCATATCTGAAGGAGTGAT ATTTGAGCTTAGATTAAAAGATGGGAAGGAGCAAGCCATGCAAAGAGCTAGGATGTT CCAAGCAGAGACGGAACAGCAAGTGCAAATGTCAGGAGGAATAGAAGGAGGCTGGT GGGTGGGGTCCAGTGAGCAAGAGGAGGGCAGGCAGGAGAGGGGATGGGGAGGTG GGCAGGCCCAGACCACCCAGGGCCCTGGAGACTATCCTGATCCAACAAGGGAAGC CTTGAGTCACTTCAGTGTCCATGTGGAGAATGGACCTCAGACTGAATGAGGGAGGC AGTAAGGAGGGCCTCTACCTCCAGGGCTTCGCCCTGTGGACTGCGCATAGACATCT CCAACTCAGAAAGTCTGAACCAAACTTTCCATAGTTCCCCCAAGTCTGGGCATCCTC CTACTCAGTGAAAGGCAGCCATCACACCTCCCTGCCCTGCTCCCGGATGCCCCAAA TCCTCTTGGTCTCCAAGTCCAGAACCTGAGACTTGTCCTTGATGTTTGTCTTTCCCTC ACCCTTTCTGTATTCTGGGAAGATGGGTTTTTTTCCCCCAGATGAATCTGTAAAACTT CTGTGATCACAATAAAAATTCTGGCAGTATTATTTTCTGGAACATGACAAAGTGATTC AAAATTATTTATCTGGAAGACTACAAAACAAGAATAGCCAGGAAATTTCTAAAAAGAA AGAAGAAGGAGGAGGAGAAAGAAGGAGGAGGAAAAGGAGGAGAAGAAGAAAAGAA AAAGAACCAAGAAAGGGTTCTAGCTCTACCAAATATTAAAACATATCATGAAGCTATT TAAAACAATATGGTTGTGGATACTGAAAAAGATGTGAATAAAGTGGAAGGAAAATAAA TAGAAATGCACATGGGGATTGAGACTGTGAAAAAGGCAGCATCTCACATCAGTGAG GGATGTTCAACACCTGGTGTTGGGAAAACTGGCTAGTCATTTAAACCAAACAACTGG GTCCTCTACCTCACTCCTGACATTAAGATACATTTAGATGATTCAAAGAGTAAGACAG AAAAAATAACACGTGAAAACACTATCAGAAAACAACGTGGGCCAGGTGTGGTGGGTC ACGCCTGTAATCCCAGCACTTTGGGAGGCCGAGGCAGACAGATCACCTGAGGTGG GGAGTTCAAGACCAGCCTGACCAACATGGTGAAATCCTGTCTCTACTAAAAATACAA AATTAGCTGAGCGTGGTGGCGCATGCCTGTAATCCCAGCTACTCAGGAGGCCGAGG CAGGAGAATCACTTGAACCTGGGAGGCAGAGGTTGTGGTGAGCCGAGATCACGCCA TTGCACTCCAGCCTGGGCAACAAGAGTGAAAATCCATCTAAAAAAAAAAAAAAAAGC CAAGGTGGATATTTTTATAGTATCAGGGTAGATCAAGCTTCTCCAATCATGACATGAA ACCCAGAAACCATAAAAGAAAAGAATGATAAAATTGCCCACGTAAAGTAAAAAGCTTG CACACAGAAAAACACCATACAGGTTACAAGATGAGCAGCAAAATCAGAGAAAAAACA TTGCAATTCAGGACACACAGAGGCTATTGTTCCTAATATTTAAAAATAAAAGTAGTGG ATTGTCTACAAAAAGATGAAGACAAGAATTTCAGAAAACCAAATACTGCATGTTTTCA CTTACAAGTGGAAGCTAAACACTGAGTACACGTGTACACAAAGAATGGAACCATAGG CCAGGCACCGTGGCTCACGCCTGTAATCCCAGTACTTTGCGAGGCCGAAGCGGGC GGATCACCTGAGGTGAGGAGTTCGAGACCATCCTGGCCAACATGGTGAAACCCAGT CTCTACTAAAAATACAAAAATTAGCCGGGCGTGGTGGTGGGTGCCTGTAATCCCAGC TACTCGGGAGGCTGCGGCAGTAGAATCGCTTGAACCCTGGAGGTGGACCTTGCAGT GAGCCGAGATCGCACCACTGCACTCCAGCCTGGGCAACAGAGTGAGACTCCATCTC AAAAAAAAAAAAAAGGAATAGAACAATAGACACTGGGGCCTACTTGAGGGAGGAGG GTGAGGATCAAAAACCTGCCTATCAGGTACTATGCTTATTACCTGGGTGGTGAAATA ATCTGTACACCAAACCCCAGTGACATGCAATTTACCGATGTAACAAACCTGCCCATG TACCCGCTGAACCTAAAATAAAAGTTGGAAAAAAATATAGAAATTTTCTTTGTAATAGC CAAAAACTGCAAACAGCCCAGGTGTCTATTAGTAGAATGCATAAACAAACTCGGGCA TGTTCATACAATGTAAAACTACTCATCAATAAAAAGTGATACTTCTCAGCAATGAAAA |
| | | GAAACTAGCTACTGATACCAGCTACAACATGGATGGATTTCAAGTGCTTTATGATGA GAGCAAGAAGCCAGACACAAAAGTGTCTATATATATATACAGTATATATACGTATATA TACACATATATACAGTATATATATACATATACATGTATATATATACTGTATATATACTGT ATATATATACACAGTATATATATACATATATACAGTGTATATATACTGTGTATATATACA TGTATATATACTGTGTATATATACATGTATATATACTGTGTATATATACATGTATATATA CTGTGTATATATACATGTATATATATGTATACTGTATATATACTGTATATATATATACAC ATATATACAGTATATATATACAGTATATACTGTATATATACAGTATATACGTGTATATAT ACATATATACAGTATATATGTAAATATACATATATACAGTATATATGTAAATATACATAT ATACATGTATATATATACACTATATATATACATATATAGTGTATATATACATATATACAT GTATATATTTACTATATGATTCCATTTATATAAAGTGCCAAAACAGTCAAAAATAATCT ATGTGGAAAAAATCAACAAAGGGATCCCCCGGGCTGCAGGAATTCGATGGCGCGCC GACGTCGCATGCAGTTAGGGATAACAGGGTAATACGACCATGGCATGTCCTCTAGA CTCGAGCGGCCGCAATAAAATATCTTTATTTTCATTACATCTGTGTGTTGGTTTTTTGT GTGAATCGTAACTAACATACGCTCTCCATCAAAACAAAACGAAACAAAACAAACTAGC AAAATAGGCTGTCCCCAGTGCAAGTGCAGGTGCCAGAACATTTCTCTATCGAAGGAT CTGCGATCGCTCCGGTGCCCGTCAGTGGGCAGAGCGCACATCGCCCACAGTCCCC GAGAAGTTGGGGGGAGGGGTCGGCAATTGAACCGGTGCCTAGAGAAGGTGGCGCG GGGTAAACTGGGAAAGTGATGTCGTGTACTGGCTCCGCCTTTTTCCCGAGGGTGGG GGAGAACCGTATATAAGTGCAGTAGTCGCCGTGAACGTTCTTTTTCGCAACGGGTTT GCCGCCAGAACACAGCTGAAGCTTCGAGGGGCTCGCATCTCTCCTTCACGCGCCCG CCGCCCTACCTGAGGCCGCCATCCACGCCGGTTGAGTCGCGTTCTGCCGCCTCCC GCCTGTGGTGCCTCCTGAACTGCGTCCGCCGTCTAGGTAAGTTTAAAGCTCAGGTC GAGACCGGGCCTTTGTCCGGCGCTCCCTTGGAGCCTACCTAGACTCAGCCGGCTCT CCACGCTTTGCCTGACCCTGCTTGCTCAACTCTACGTCTTTGTTTCGTTTTCTGTTCT GCGCCGTTACAGATCCAAGCTGTGACCGGCGCCTACGTAAGTGATATCTACTAGATT TATCAAAAAGAGTGTTGACTTGTGAGCGCTCACAATTGATACTTAGATTCATCGAGAG GGACACGTCGACTACTAACCTTCTTCTCTTTCCTACAGCTGAGATCACCGGCGAAGG AGGGCCACCATGGGTCACCAGCAGTTGGTCATCTCTTGGTTTTCCCTGGTTTTTCTG GCATCTCCCCTCGTGGCCATATGGGAACTGAAGAAAGATGTTTATGTCGTAGAATTG GATTGGTATCCGGATGCCCCTGGAGAAATGGTGGTCCTCACCTGTGACACCCCTGA AGAAGATGGTATCACCTGGACCTTGGACCAGAGCAGTGAGGTCTTAGGCTCTGGCA AAACCCTGACCATCCAAGTCAAAGAGTTTGGAGATGCTGGCCAGTACACCTGTCACA AAGGAGGCGAGGTTCTAAGCCATTCGCTCCTGCTGCTTCACAAAAAGGAAGATGGA ATTTGGTCCACTGATATTTTAAAGGACCAGAAAGAACCCAAAAATAAGACCTTTCTAA GATGCGAGGCCAAGAATTATTCTGGACGTTTCACCTGCTGGTGGCTGACGACAATCA GTACTGATTTGACATTCAGTGTCAAAAGCAGCAGAGGCTCTTCTGACCCCCAAGGGG TGACGTGCGGAGCTGCTACACTCTCTGCAGAGAGAGTCAGAGGGGACAACAAGGA GTATGAGTACTCAGTGGAGTGCCAGGAGGACAGTGCCTGCCCAGCTGCTGAGGAG AGTCTGCCCATTGAGGTCATGGTGGATGCCGTTCACAAGCTCAAGTATGAAAACTAC ACCAGCAGCTTCTTCATCAGGGACATCATCAAACCTGACCCACCCAAGAACTTGCAG CTGAAGCCATTAAAGAATTCTCGGCAGGTGGAGGTCAGCTGGGAGTACCCTGACAC CTGGAGTACTCCACATTCCTACTTCTCCCTGACATTCTGCGTTCAGGTCCAGGGCAA GAGCAAGAGAGAAAAGAAAGATAGAGTCTTCACGGACAAGACCTCAGCCACGGTCA TCTGCCGCAAAAATGCCAGCATTAGCGTGCGGGCCCAGGACCGCTACTATAGCTCA TCTTGGAGCGAATGGGCATCTGTGCCCTGCAGTGTTCCTGGAGTAGGGGTACCTGG GGTGGGCGCCAGAAACCTCCCCGTGGCCACTCCAGACCCAGGAATGTTCCCATGC CTTCACCACTCCCAAAACCTGCTGAGGGCCGTCAGCAACATGCTCCAGAAGGCCAG ACAAACTCTAGAATTTTACCCTTGCACTTCTGAAGAGATTGATCATGAAGATATCACA AAAGATAAAACCAGCACAGTGGAGGCCTGTTTACCATTGGAATTAACCAAGAATGAG AGTTGCCTAAATTCCAGAGAGACCTCTTTCATAACTAATGGGAGTTGCCTGGCCTCC AGAAAGACCTCTTTTATGATGGCCCTGTGCCTTAGTAGTATTTATGAAGACTTGAAGA TGTACCAGGTGGAGTTCAAGACCATGAATGCAAAGCTGCTGATGGATCCTAAGAGG CAGATCTTTCTAGATCAAAACATGCTGGCAGTTATTGATGAGCTGATGCAGGCCCTG AATTTCAACAGTGAGACTGTGCCACAAAAATCCTCCCTTGAAGAACCGGATTTTTATA AAACTAAAATCAAGCTCTGCATACTTCTTCATGCTTTCAGAATTCGGGCAGTGACTAT TGATAGAGTGATGAGCTATCTGAATGCTTCCTAAAAAGCGAGGTCCCTCCAAACCGT TGTCATTTTTATAAAACTTTGAAATGAGGAAACTTTGATAGGATGTGGATTAAGAACTA GGGAGGGGCTAGCTCGACATGATAAGATACATTGATGAGTTTGGACAAACCACAACT AGAATGCAGTGAAAAAAATGCTTTATTTGTGAAATTTGTGATGCTATTGCTTTATTTGT GAAATTTGTGATGCTATTGCTTTATTTGTAACCATTATAAGCTGCAATAAACAAGTTAA CAACAACAATTGCATTCATTTTATGTTTCAGGTTCAGGGGGAGGTGTGGGAGGTTTT TTAAAGCAAGTAAAACCTCTACAAATGTGGTAGATCCATTTATTAGCTAGGAGTTTCA GAAAAGGGGGCCTGAGTGGCCCCTTTTTTCAACTTAATTAACCTGCAGGGCCTGAAA TAACCTCTGAAAGAGGAACTTGGTTAGGTACCTTCTGAGGCTGAAAGAACCAGCTGT GGAATGTGTGTCAGTTAGGGTGTGGAAAGTCCCCAGGCTCCCCAGCAGGCAGAAGT ATGCAAAGCATGCATCTCAATTAGTCAGCAACCAGGTGTGGAAAGTCCCCAGGCTCC CCAGCAGGCAGAAGTATGCAAAGCATGCATCTCAATTAGTCAGCAACCATAGTCCCA CTAGTTTCATCACCACCGCCACCCCCCCGCCCCCCCGCCATCTGAAAGGGTTCTAG GGGATTTGCAACCTCTCTCGTGTGTTTCTTCTTTCCGAGAAGCGCCGCCACACGAGA AAGCTGGCCGCGAAAGTCGTGCTGGAATCACTTCCAACGAAACCCCAGGCATAGAT |
| | | GGGAAAGGGTGAAGAACACGTTGTCATGGCTACCGTTTCCCCGGTCACGGAATAAA CGCTCTCTAGGATCCGGAAGTAGTTCCGCCGCGACCTCTCTAAAAGGATGGATGTG TTCTCTGCTTACATTCATTGGACGTTTTCCCTTAGAGGCCAAGGCCGCCCAGGCAAA GGGGCGGTCCCACGCGTGAGGGGCCCGCGGAGCCATTTGATTGGAGAAAAGCTGC AAACCCTGACCAATCGGAAGGAGCCACGCTTCGGGCATCGGTCACCGCACCTGGAC AGCTCCGATTGGTGGACTTCCGCCCCCCCTCACGAATCCTCATTGGGTGCCGTGGG TGCGTGGTGCGGCGCGATTGGTGGGTTCATGTTTCCCGTCCCCCGCCCGCGAGAA GTGGGGGTGAAAAGCGGCCCGACCTGCTTGGGGTGTAGTGGGCGGACCGCGCGG CTGGAGGTGTGAGGATCCGAACCCAGGGGTGGGGGGTGGAGGCGGCTCCTGCGA TCGAAGGGGACTTGAGACTCACCGGTCGCACGTCATGAATCTAGAACCATGGCTTC GTACCCCGGCCATCAGCACGCGTCTGCGTTCGACCAGGCTGCGCGTTCTCGCGGC CATAGCAACCGACGTACGGCGTTGCGCCCTCGCCGGCAGCAAGAAGCCACGGAAG TCCGCCCGGAGCAGAAAATGCCCACGCTACTGCGGGTTTATATAGACGGTCCCCAC GGGATGGGGAAAACCACCACCACGCAACTGCTGGTGGCCCTGGGTTCGCGCGACG ATATCGTCTACGTACCCGAGCCGATGACTTACTGGCGGGTGCTGGGGGCTTCCGAG ACAATCGCGAACATCTACACCACACAACACCGCCTTGACCAGGGTGAGATATCGGC CGGGGACGCGGCGGTGGTAATGACAAGCGCCCAGATAACAATGGGCATGCCTTAT GCCGTGACCGACGCCGTTCTGGCTCCTCATATCGGGGGGGAGGCTGGGAGCTCAC ATGCCCCGCCCCCGGCCCTCACCCTCATCTTCGACCGCCATCCCATCGCCGCCCTC CTGTGCTACCCGGCCGCGCGATACCTTATGGGCAGCATGACCCCCCAGGCCGTGC TGGCGTTCGTGGCCCTCATCCCGCCGACCTTGCCCGGCACAAACATCGTGTTGGGG GCCCTTCCGGAGGACAGACACATCGACCGCCTGGCCAAACGCCAGCGCCCCGGCG AGCGGCTTGACCTGGCTATGCTGGCCGCGATTCGCCGCGTTTACGGGCTGCTTGCC AATACGGTGCGGTATCTGCAGGGCGGCGGGTCGTGGCGGGAGGATTGGGGACAGC TTTCGGGGACGGCCGTGCCGCCCCAGGGTGCCGAGCCCCAGAGCAACGCGGGCC CACGACCCCATATCGGGGACACGTTATTTACCCTGTTTCGGGCCCCCGAGTTGCTG GCCCCCAACGGCGACCTGTACAACGTGTTTGCCTGGGCCTTGGACGTCTTGGCCAA ACGCCTCCGTCCCATGCACGTCTTTATCCTGGATTACGACCAATCGCCCGCCGGCT GCCGGGACGCCCTGCTGCAACTTACCTCCGGGATGATCCAGACCCACGTCACCACC CCAGGCTCCATACCGACGATCTGCGACCTGGCGCGCACGTTTGCCCGGGAGATGG GGGAGGCTAACTGAGTATACCCTAGGATTATCCCTAATACCTGCCACCCCACTCTTA ATCAGTGGTGGAAGAACGGTCTCAGAACTGTTTGTTTCAATTGGCCATTTAAGTTTAG TAGTAAAAGACTGGTTAATGATAACAATGCATCGTAAAACCTTCAGAAGGAAAGGAG AATGTTTTGTGGACCACTTTGGTTTTCTTTTTTGCGTGTGGCAGTTTTAAGTTATTAGT TTTTAAAATCAGTACTTTTTAATGGAAACAACTTGACCAAAAATTTGTCACAGAATTTT GAGACCCATTAAAAAAGTTAAATGAGAAACCTGTGTGTTCCTTTGGTCAACACCGAG ACATTTAGGTGAAAGACATCTAATTCTGGTTTTACGAATCTGGAAACTTCTTGAAAAT GTAATTCTTGAGTTAACACTTCTGGGTGGAGAATAGGGTTGTTTTCCCCCCACATAAT TGGAAGGGGAAGGAATATCATTTAAAGCTATGGGAGGGTTTCTTTGATTACAACACT GGAGAGAAATGCAGCATGTTGCTGATTGCCTGTCACTAAAACAGGCCAAAAACTGAG TCCTTGGGTTGCATAGAAAGCTGCCTGCAGGCGTTACATAACTTACGGTAAATGGCC CGCCTGGCTGACCGCCCAACGACCCCCGCCCATTGACGTCAATAATGACGTATGTT CCCATAGTAACGCCAATAGGGACTTTCCATTGACGTCAATGGGTGGAGTATTTACGG TAAACTGCCCACTTGGCAGTACATCAAGTGTATCATATGCCAAGTACGCCCCCTATT GACGTCAATGACGGTAAATGGCCCGCCTGGCATTATGCCCAGTACATGACCTTATG GGACTTTCCTACTTGGCAGTACATCTACGTATTAGTCATCGCTATTACCATGATGATG CGGTTTTGGCAGTACATCAATGGGCGTGGATAGCGGTTTGACTCACGGGGATTTCC AAGTCTCCACCCCATTGACGTCAATGGGAGTTTGTTTTGACTAGTTACCGGCGGAAA CGGTCTCGGGTTGAGAGGTCACCCGAGGGACAGGCAGCTGCTGAACCAATAGGAC CGGCGCACAGGGCGGATGCTGCCCCTCATTGGCGGCCGTTGAGAGTGACCAAGAG CCAATGAGTCAGCCCGGGGGGCGTAGCAGTGACGTAAGTTGCGGAGGAGGCCGCT TCGAATCGGCAGCGGCCAGCTTGGTGGCATGGACCAATCAGCGTCCTCCAACGAG GAGCGCCTTCGCCAATCGGAGGCCTCCACGACGGGGCTGGGGGGAGGGTATATAA GCCGAGTCGGCGGCGGCGCGCTCCACACGGGCCGAGACCACAGCGACGGGAGCG TCTGCCTCTGCGGGGCCGAGAGGTAAGCGCCGCGGCCTGCCCTTTCCAGGCCAAC TCGGAGCCCGTCTCGTGGCTCCGCCTGATCGGGGGCTCCTGTCGCCCTCAGATCG GTCGGAACGCCGTCGCGCTCCGGGACTACAAGCCTGTTGCTGGGCCCGGAGACTG CCGAAGGACCGCTGAGCACTGTCCTCAGCGCCGGCACCATGGATTGGATCTGGCG GATCCTGTTCCTTGTGGGAGCTGCCACAGGCGCCCATTCTGAAGTTCAGCTGGTTC AGTCTGGCGCCGAAGTGAAGAAACCTGGCGCCTCTGTGAAGGTGTCCTGCAAAGCT TCTGGCGGCACCTTCAGCAGCTACGCCATCTCTTGGGTTCGACAGGCCCCTGGACA AGGCCTGGAATGGATGGGCAGAATCATCCCCATCCTGGGAATCGCCAACTACGCCC AGAAATTCCAGGGCAGAGTGACCATCACCGCCGACAAGAGCACAAGCACCGCCTAC ATGGAACTGAGCAGCCTGAGAAGCGAGGACACCGCCGTGTACTACTGTGCCAGAAG CGGCCACGGCTACAGCTACGGCGCCTTTGATTATTGGGGCCAGGGCACCCTGGTCA CCGTTTCTAGCGGAGGCGGAGGTAGTGGTGGCGGAGGTTCAGGCGGCGGAGGATC TCAATCTGTGCTGACACAGCCTCCAAGCGTGTCAGGTGCTCCTGGCCAGAGAGTGA CAATCAGCTGTACAGGCAGCAGCAGCAACATCGGAGCCGGCTATGACGTGCACTGG TATCAGCAGCTGCCTGGCACAGCCCCTAAACTGCTGATCTACGGCAACAGCAACAG ACCCAGCGGCGTGCCCGATAGATTTTCCGGCTCTAAGAGCGGCACAAGCGCCAGC CTGGCTATTACTGGACTGCAGGCCGAGGACGAGGCCGACTACTACTGTCAGAGCTA CGACAGCAGCCTGTCCGGCAGCTACGTTGTGTTTGGCGGCGGAACAAAGCTGACC GTGCTGGAAGCCAAGAGCTGCGACAAGACCCACACCTGTCCTCCATGTCCTGCTCC AGAACTGCTCGGCGGACCTTCCGTGTTCCTGTTTCCTCCAAAGCCTAAGGACACCCT GATGATCAGCAGAACCCCTGAAGTGACCTGCGTGGTGGTGGATGTGTCCCACGAGG ACCCAGAAGTGAAGTTCAATTGGTACGTGGACGGCGTGGAAGTGCACAACGCCAAG ACCAAGCCTAGAGAGGAACAGTACAACAGCACCTACAGAGTGGTGTCCGTGCTGAC AGTGCTGCACCAGGATTGGCTGAACGGCAAAGAGTACAAGTGCAAGGTGTCCAACA AGGCCCTGCCTGCTCCTATCGAGAAAACCATCAGCAAGGCCAAGGGCCAGCCTAGG GAACCCCAGGTTTACACACTGCCACCTAGCAGGGACGAGCTGACCAAGAATCAGGT GTCCCTGACCTGCCTGGTCAAGGGCTTCTACCCTTCCGATATCGCCGTGGAATGGG AGAGCAATGGCCAGCCAGAGAACAACTACAAGACAACCCCTCCTGTGCTGGACAGC GACGGCTCATTCTTCCTGTACTCCAAGCTGACTGTGGACAAGAGCCGGTGGCAGCA GGGCAATGTGTTCAGCTGTAGCGTGATGCACGAGGCCCTGCACAACCACTACACAC AGAAGTCCCTGTCTCTGAGCCCCGGAAAAGGTGGCGGTGGCTCTTACCCTTACGAC GTGCCAGATTACGCCGGCTATCCCTACGATGTGCCTGACTATGCTGGCTACCCCTAT GACGTCCCCGACTACGCTTAACTAGCTACGGAATTCCGGCTAGCTGGCCAGACATG ATAAGATACATTGATGAGTTTGGACAAACCACAACTAGAATGCAGTGAAAAAAATGCT TTATTTGTGAAATTTGTGATGCTATTGCTTTATTTGTAACCATTATAAGCTGCAATAAA CAAGTTAACAACAACAATTGCATTCATTTTATGTTTCAGGTTCAGGGGGAGGTGTGG GAGGTTTTTTAAAGCAAGTAAAACCTCTACAAATGTGGTATGGAAATGTTAATTAACT AGCCATGACCAAAATCCCTTAACGTGAGTTTTCGTTCCACTGAGCGTCAGACCCCGT AGAAAAGATCAAAGGATCTTCTTGAGATCCTTTTTTTCTGCGCGTAATCTGCTGCTTG CAAACAAAAAAACCACCGCTACCAGCGGTGGTTTGTTTGCCGGATCAAGAGCTACCA ACTCTTTTTCCGAAGGTAACTGGCTTCAGCAGAGCGCAGATACCAAATACTGTTCTT CTAGTGTAGCCGTAGTTAGGCCACCACTTCAAGAACTCTGTAGCACCGCCTACATAC CTCGCTCTGCTAATCCTGTTACCAGTGGCTGCTGCCAGTGGCGATAAGTCGTGTCTT ACCGGGTTGGACTCAAGACGATAGTTACCGGATAAGGCGCAGCGGTCGGGCTGAA CGGGGGGTTCGTGCACACAGCCCAGCTTGGAGCGAACGACCTACACCGAACTGAG ATACCTACAGCGTGAGCTATGAGAAAGCGCCACGCTTCCCGAAGGGAGAAAGGCGG ACAGGTATCCGGTAAGCGGCAGGGTCGGAACAGGAGAGCGCACGAGGGAGCTTCC AGGGGGAAACGCCTGGTATCTTTATAGTCCTGTCGGGTTTCGCCACCTCTGACTTGA GCGTCGATTTTTGTGATGCTCGTCAGGGGGGCGGAGCCTATGGAAAAACGCCAGCA ACGCGGCCTTTTTACGGTTCCTGGCCTTTTGCTGGCCTTTTGCTCAGGGTTCGAAAT CGATAAGCTTGGATCCGGAGAGCTCCCAACGCGTCGGCTAGCTAGTAGGGATAACA GGGTAATAAGCGTCGACGGCGCGCCCCTAGGGGCCGGCCTTAATTAAATCAAGCTT ATCGATACCGTCGAACCTCGAGGGGGGGCATCACTCCGCCCTAAAACCTACGTCAC CCGCCCCGTTCCCACGCCCCGCGCCACGTCACAAACTCCACCCCCTCATTATCATA TTGGCTTCAATCCAAAATAAGGTATATTATTGATGATGTTT |
| 121 | HDAdCD44v6BiT E | AAACATCATCAATAATATACCTTATTTTGGATTGAAGCCAATATGATAATGAGGGGGT GGAGTTTGTGACGTGGCGCGGGGCGTGGGAACGGGGCGGGTGACGTAGGTTTTAG GGCGGAGTAACTTGTATGTGTTGGGAATTGTAGTTTTCTTAAAATGGGAAGTGACGT AACGTGGGAAAACGGAAGTGACGATTTGAGGAAGTTGTGGGTTTTTTGGCTTTCGTT TCTGGGCGTAGGTTCGCGTGCGGTTTTCTGGGTGTTTTTTGTGGACTTTAACCGTTA CGTCATTTTTTAGTCCTATATATACTCGCTCTGCACTTGGCCCTTTTTTACACTGTGAC TGATTGAGCTGGTGCCGTGTCGAGTGGTGTTTTTTTAATAGGTTTTCTTTTTTACTGG TAGGCGCGCCGTCGACGCTTATTACCCTGTTATCCCTACTAGCTAGCCGACGCGTT GGGAGCTCTCCGGATCCAAGCTTATCGATTTCGAACCCAAATGGATCTACCACATTT GTAGAGGTTTTACTTGCTTTAAAAAACCTCCCACACCTCCCCCTGAACCTGAAACATA AAATGAATGCAATTGTTGTTGTTAACTTGTTTATTGCAGCTTATAATGGTTACAAATAA AGCAATAGCATCACAAATTTCACAAATAAAGCAATAGCATCACAAATTTCACAAATAA AGCATTTTTTTCACTGCATTCTAGTTGTGGTTTGTCCAAACTCATCAATGTATCTTATC ATGTCGAGCTAGCTAGTCAGGACTTCAGTTCCAGCTTTGTGCCGGCTCCGAAGGTC AGCGGGTTGCTAGACCATTGCTGACAGTAATATGTGGCGGCATCCTCGGCTTCCATA GAGCTGATTGTCAGGCTGTAGCTGGTGCCAGATCCAGATCCGCTGAATCTGTAGGG CACGCCGGAGGCCACTTTGCTTGTGTCGTAGATCCACCGCTTGGGGCTTGTGCCAG ACTTCTGCTGATACCAGTTCATGTAGGACACGCTGCTGCTGGCTCTGCATGTCATGG TCACTTTCTCGCCTGGGGAAGCGCTCATGATGGCGGGGCTTTGTGTCAGCTGAATA TCGGAGCCACCACCACCGCTTCCACCACCACCTGAACCGCCTCCGCCACTAGAAAC TGTCAGGGTTGTGCCCTGGCCCCAGTAATCCAGACAGTAGTGGTCGTCGTAGTACC GGGCACAATAGTACACAGCGCTATCTTCAGAGGTCAGGCTGGACAGCTGCATGTAG GCGGTAGAGCTGCTCTTGTCGGTGGTCAGTGTGGCCTTGTCCTTGAACTTCTGGTT GTAATTGGTGTAGCCCCGGCTGGGGTTGATGTAGCCGATCCATTCCAGTCCTTGGC CAGGCCTCTGCTTGACCCAGTGCATGGTGTACCGGGTGAATGTGTAGCCGCTGGTC TTGCAGGACATCTTCACAGAGGCGCCAGGTCTAGCCAGTTCGGCGCCAGACTGTTG CAGCTTGATATCGGATCCGCCACCTCCGCCAGATGACACTGTGACCAGTGTTCCTCT GCCCCAGTAGTCGAGGCCCTGTCTGGCGCAATAATACACGGCGGTGTCCTCGGCTC TCAGGGAGTTCATCTGCAGGTACAGGCTGTTCTTGGCGTTGTCCCGGCTGATGGTG AATCTGCCCTTGATGCTGTCCAGGTAGTAGGTGTAGCTGCCGCCGCTGCTGATTGT GGACACCCATTCAAGGCCTTTGCCAGGAGCCTGTCGGACCCAGCTCATATCGTAGC |
| | | TGCTGAAGGTGAAGCCGGAAGCGGCACAGCTCAGTCTCAGAGAGCCGCCAGGTTT CACAAGTCCGCCGCCTGATTCCACCAGCTGCACTTCAGATCCACCACCGCCACTAC CTCCGCCTCCGCTTCCTCCGCCGCCAGATCCTCCGCCACCTCTCTTGATTTCCACCT TGGTGCCTCCGCCAAATGTCAGGGGATTGCTGGACCACTGCAGGCAGTAGTACACG GCGAAGTCCTCAGGTTCCAGGCTGCTTATGGTCAGGGTGAAATCGGTGCCGCTGCC AGAGCCAGAAAATCTGGCAGGCACTCCGCTGGCCAGATTGCTGGTCAGGTAGATCA GGATTCTAGGGGCCTGTCCGGGCTTCTGCTGCAGCCAGTAGATGTAGTTGATGGAG CTGCTGGCGCTACAGCTCAGGGTAGCTCTCTCGCCAGGGCTCAGAGACAGTGTGG CTGGAGACTGTGTCAGCACGATCTCGCCGGTGGTATCAGGCAGCCAAAGCAGCAGC AGGAACAGCAGCTGAGCAGGGGCTTCCATGGTGGCCCTCCTTCGCCGGTGATCTCA GCTGTAGGAAAGAGAAGAAGGTTAGTAGTCGACGTGTCCCTCTCGATGAATCTAAGT ATCAATTGTGAGCGCTCACAAGTCAACACTCTTTTTGATAAATCTAGTAGATATCACT TACGTAGGCGCCGGTCACAGCTTGGATCTGTAACGGCGCAGAACAGAAAACGAAAC AAAGACGTAGAGTTGAGCAAGCAGGGTCAGGCAAAGCGTGGAGAGCCGGCTGAGT CTAGGTAGGCTCCAAGGGAGCGCCGGACAAAGGCCCGGTCTCGACCTGAGCTTTAA ACTTACCTAGACGGCGGACGCAGTTCAGGAGGCACCACAGGCGGGAGGCGGCAGA ACGCGACTCAACCGGCGTGGATGGCGGCCTCAGGTAGGGCGGCGGGCGCGTGAA GGAGAGATGCGAGCCCCTCGAAGCTTCAGCTGTGTTCTGGCGGCAAACCCGTTGCG AAAAAGAACGTTCACGGCGACTACTGCACTTATATACGGTTCTCCCCCACCCTCGGG AAAAAGGCGGAGCCAGTACACGACATCACTTTCCCAGTTTACCCCGCGCCACCTTCT CTAGGCACCGGTTCAATTGCCGACCCCTCCCCCCAACTTCTCGGGGACTGTGGGCG ATGTGCGCTCTGCCCACTGACGGGCACCGGAGCGATCGCAGATCCTTCGATAGAGA AATGTTCTGGCACCTGCACTTGCACTGGGGACAGCCTATTTTGCTAGTTTGTTTTGTT TCGTTTTGTTTTGATGGAGAGCGTATGTTAGTTACGATTCACACAAAAAACCAACACA CAGATGTAATGAAAATAAAGATATTTTATTGCGGCCGCTCGAGTCTAGAGGACATGC CATGGTCGTATTACCCTGTTATCCCTAACTGCATGCGACGTCGGCGCGCCATCGAAT TCCTGCAGCCCGGGGGATCCCTTTGTTGATTTTTTCCACATAGATTATTTTTGACTGT TTTGGCACTTTATATAAATGGAATCATATAGTAAATATATACATGTATATATGTATATAT ACACTATATATGTATATATATAGTGTATATATATACATGTATATATGTATATTTACATAT ATACTGTATATATGTATATTTACATATATACTGTATATATGTATATATACACGTATATAC TGTATATATACAGTATATACTGTATATATATACTGTATATATGTGTATATATATATACAG TATATATACAGTATACATATATATACATGTATATATACACAGTATATATACATGTATATA TACACAGTATATATACATGTATATATACACAGTATATATACATGTATATATACACAGTA TATATACACTGTATATATGTATATATATACTGTGTATATATATACAGTATATATACAGTA TATATATACATGTATATGTATATATATACTGTATATATGTGTATATATACGTATATATAC TGTATATATATATAGACACTTTTGTGTCTGGCTTCTTGCTCTCATCATAAAGCACTTGA AATCCATCCATGTTGTAGCTGGTATCAGTAGCTAGTTTCTTTTCATTGCTGAGAAGTA TCACTTTTTATTGATGAGTAGTTTTACATTGTATGAACATGCCCGAGTTTGTTTATGCA TTCTACTAATAGACACCTGGGCTGTTTGCAGTTTTTGGCTATTACAAAGAAAATTTCT ATATTTTTTTCCAACTTTTATTTTAGGTTCAGCGGGTACATGGGCAGGTTTGTTACATC GGTAAATTGCATGTCACTGGGGTTTGGTGTACAGATTATTTCACCACCCAGGTAATA AGCATAGTACCTGATAGGCAGGTTTTTGATCCTCACCCTCCTCCCTCAAGTAGGCCC CAGTGTCTATTGTTCTATTCCTTTTTTTTTTTTTTGAGATGGAGTCTCACTCTGTTGCC CAGGCTGGAGTGCAGTGGTGCGATCTCGGCTCACTGCAAGGTCCACCTCCAGGGTT CAAGCGATTCTACTGCCGCAGCCTCCCGAGTAGCTGGGATTACAGGCACCCACCAC CACGCCCGGCTAATTTTTGTATTTTTAGTAGAGACTGGGTTTCACCATGTTGGCCAG GATGGTCTCGAACTCCTCACCTCAGGTGATCCGCCCGCTTCGGCCTCGCAAAGTAC TGGGATTACAGGCGTGAGCCACGGTGCCTGGCCTATGGTTCCATTCTTTGTGTACAC GTGTACTCAGTGTTTAGCTTCCACTTGTAAGTGAAAACATGCAGTATTTGGTTTTCTG AAATTCTTGTCTTCATCTTTTTGTAGACAATCCACTACTTTTATTTTTAAATATTAGGAA CAATAGCCTCTGTGTGTCCTGAATTGCAATGTTTTTTCTCTGATTTTGCTGCTCATCTT GTAACCTGTATGGTGTTTTTCTGTGTGCAAGCTTTTTACTTTACGTGGGCAATTTTAT CATTCTTTTCTTTTATGGTTTCTGGGTTTCATGTCATGATTGGAGAAGCTTGATCTACC CTGATACTATAAAAATATCCACCTTGGCTTTTTTTTTTTTTTTTAGATGGATTTTCACTC TTGTTGCCCAGGCTGGAGTGCAATGGCGTGATCTCGGCTCACCACAACCTCTGCCT CCCAGGTTCAAGTGATTCTCCTGCCTCGGCCTCCTGAGTAGCTGGGATTACAGGCA TGCGCCACCACGCTCAGCTAATTTTGTATTTTTAGTAGAGACAGGATTTCACCATGTT GGTCAGGCTGGTCTTGAACTCCCCACCTCAGGTGATCTGTCTGCCTCGGCCTCCCA AAGTGCTGGGATTACAGGCGTGACCCACCACACCTGGCCCACGTTGTTTTCTGATA GTGTTTTCACGTGTTATTTTTTCTGTCTTACTCTTTGAATCATCTAAATGTATCTTAATG TCAGGAGTGAGGTAGAGGACCCAGTTGTTTGGTTTAAATGACTAGCCAGTTTTCCCA ACACCAGGTGTTGAACATCCCTCACTGATGTGAGATGCTGCCTTTTTCACAGTCTCA ATCCCCATGTGCATTTCTATTTATTTTCCTTCCACTTTATTCACATCTTTTTCAGTATCC ACAACCATATTGTTTTAAATAGCTTCATGATATGTTTTAATATTTGGTAGAGCTAGAAC CCTTTCTTGGTTCTTTTTCTTTTCTTCTTCTCCTCCTTTTCCTCCTCCTTCTTTCTCCTC CTCCTTCTTCTTTCTTTTTAGAAATTTCCTGGCTATTCTTGTTTTGTAGTCTTCCAGAT AAATAATTTTGAATCACTTTGTCATGTTCCAGAAAATAATACTGCCAGAATTTTTATTG TGATCACAGAAGTTTTACAGATTCATCTGGGGGAAAAAAACCCATCTTCCCAGAATAC AGAAAGGGTGAGGGAAAGACAAACATCAAGGACAAGTCTCAGGTTCTGGACTTGGA GACCAAGAGGATTTGGGGCATCCGGGAGCAGGGCAGGGAGGTGTGATGGCTGCCT |
| | | TTCACTGAGTAGGAGGATGCCCAGACTTGGGGGAACTATGGAAAGTTTGGTTCAGA CTTTCTGAGTTGGAGATGTCTATGCGCAGTCCACAGGGCGAAGCCCTGGAGGTAGA GGCCCTCCTTACTGCCTCCCTCATTCAGTCTGAGGTCCATTCTCCACATGGACACTG AAGTGACTCAAGGCTTCCCTTGTTGGATCAGGATAGTCTCCAGGGCCCTGGGTGGT CTGGGCCTGCCCACCTCCCCATCCCCTCTCCTGCCTGCCCTCCTCTTGCTCACTGG ACCCCACCCACCAGCCTCCTTCTATTCCTCCTGACATTTGCACTTGCTGTTCCGTCT CTGCTTGGAACATCCTAGCTCTTTGCATGGCTTGCTCCTTCCCATCTTTTAATCTAAG CTCAAATATCACTCCTTCAGATATGCCTTTCCCAAATATCCTAGCTAAAGAGAACTGC TCCCCACTTCCCACAAGCCCTAGCAAACTGGGACTCTCCTTCTTGCTTTCTTTCTCTC TGCCCCTTCTGTGTTTTTTCCATAGACCTTAACTTGTTACTGTTTTATTTGTTGATGTG TTTACATCATTTGTCCCCCTGTGATGGTTAGTTTTATGTGTCAACTTGGCTAGGCTGT AGGGGCTGTTCGTCAATCAAACACTGATCTATGTGTAGCTGTGAAGGGATTTTGTAG CTGTGATTAATAGTTGACTTTAAGTAAGGAAGATTATCCTGGAGAATGTGGGGGAAG GGGCAGACCTCAGCCAATCAGTTGAAAGGCCTTAAGAACAAAGCTGAGATTTCCCC GAGGAAGAAGAAATTCTGCCTGTGGACTGCAGCATCAGCTCCTGCTTGAGAGGTTC CAGGCTGCCCTTCCTGACAGCCTGTCCTATGATTTTGGACTTGCCTTGCCAGGTTTT TCCTCTTTCTTGACCCCCCAAAACCATATATATCTCCTGCTGGTTTGTTTCTCTGGTG TAATCCTGAGGGATGAACACTCCTCCCTCCCAGTTCACTCTGGGAGAGGTTGGGGA TCATGTTTGTCTATTTCCCCATGCCTGGCCCATGCGTGTTTATTGAATCACTGAACAA CCACGCATTAGGAAGCCAGTCATATGTGCTTCTTCAGAACCTCATGTCCATTGCCAG ATCTCCCTGGCTTTTGTGGCTAGAGGACAAGTGAGAGATAGTAGCTTACCCACAGAC CTTGGCCCTGAGGCCCCCAGGGGCCTGAGCCTGCTGTAAGGGAGGAGGGAGCCCC TGAGGTCTCACACACCTCCCTGGGGATCTGGCATTTTCCCCTGGGGCTGGCCTCAG AGCTGGGCGGGGGCAGATTATGGAGTGGGTTGTAGAGGGAGCACCTCCCCACTAG CAATCATGGTTTTTTCTGCGCCTCTCATAGGGAGGCCTACAGGCCTCCACCTTCAGC TCTTCAGGAGCCACAGGCAGCTGAGTGACTTCTGCATCACAGTCTTCTCAGAGACAA ACTTGCAGTAAGAAAGAGGGGCCCGTGAGGGACCCCAGAGAAGGCTGTTGTCAAAG CAGGATGAGAGTGAACTCTTCCATGGGGGACACCCAGCGTCTCCAAGCTCTTTTATG CTGTCTTCAAGGGGTCTAGAGAGCTTCAGCCCAACATGAGACCCAGTCCAGCAGCA TTTCCCTGCGAAGTGAAAGTTAGGACCCTGACTAGATACACCACGCTGACCTCAGCC AGAATATCAAGATGCTGAGGCGCTGAGATGCTGGGATGCTAAGGTGCTAAGGTGCT GGGGTGCTGGGGTGCTAGGATGCTGAGGTTTTGTGATACTGGGTCGCTGAGATGCT GGGATACTGGGGTGCTGAAGTGCTGGGGTACTGGGGTGTTGCCATGCTGAAATGCT GGGGTGTTGGGATGCTGAGGTGCTGGGTTGCTGGGATGCTGGGATAGTCCTTGGAT GCTGCGGTGCTGAGATGCTGGTCTGCTGGTCTGCTGGGGTGTTGGGATGCTGGGA TTTTGGGATGCTGGAGTGCTGCAGTGCTGCAGTGCTGAGATGCTGGAGTGCTGGGG TGCTGGAATACTGTAGTACTGGTGTGCTGGAGCATTGAGATGCTAGGGCACTGGGA TGCTAAGGTGCTGAGATGCTGCAGTGCTGGGGTGTTGGGATGCCGAGGTGTTGGG ATGCTTAAGTGCTGGGGTGTTGGGATGCTGGGGTGCTGGAACACTATGGTGCTGGG GTGCTGGAGTGTTGAGATACTGTAGTGGTGGGATGCTTAAGTGCTGGGGTGCTGGG TGTTGGGATGCCTAGGTGCTGGGGTGCCGAGATGCTGGAGTACTAGTGTGCTGGGA TGCTGAAGTGCTGGGGTCCTGAGATGCTGCAGTGCTAGGGTGCTGAGATTCTGGGC TGCTGGAGTGTTGGGGTGCTGGGATGCTGGAGTGCTGAGATGCTTGGACAATGGG GTGCTGGAATACTATGGTGCTGGGGTGCTGGGGTATTGAGATGCTAGGGTACTGGG ATGCTGAAGTGCTGAGATCCTGGAGTGCTGGGCTGCTGGGCCACAGGCTCTTGAAT CCATTCGTCTGCCCAGGGGAAGAAACCAGAAGATAAAGAGCTAATGAAGGAGCTTT GGTTGAGAGGGAGGAAGTAATGGAAGGAGCAACATCTTGTGGAGGAGCAGGAGAG AATGGACCTCAGGTTGGGAGAGAGGGCCAGGCTACAGGCCAGAGAGGCAGAAGGA TTCCAGCAGAGTGTGGGCTCCAGGAGCCAAGGGGAAACAGGTTTCTGGGAGGAGA GAGTCCAGTACTGCTGAAGTGGCAAGTCCGCTGAGGACCAGGAAGCTTCATTTGGC TTTATGACCAGGAGGAATTTGGAACTGTGACTAGAGTACTTAGGGGGAAGGAGGCA AGACTGGAGCCAGATTGCTCTGGGTTGAGGGGTGAGTGGGAGGTGAAGCAGGGCA CTGTCACTCCTTTGAAGGGTGGCAGAGAGCTGGAATTGGTGCTGGATGGGCTGTGG GGTGACAGGGTCATGTGGAAAGCCCCTGGGGGGCACCTGGAAAAGGAGAAGCTGA CAGTACAGTGAGAGGACAGCTAAGGGAAAGCGGAATGGCAGAACACGCACTGCCA GGAGGAATGAGGATAGGGTCAGGAGTGCCAGGGGCAGTGAGGCCAGCCTGGGGT CAGGTGGCAGGACGTGTCCAGGAAGCTGGTCTGCACTGCAGCCCACACTGGCTCA GCCTTGAGGTTCCCTGTGTGGTTGGGGTAGGAAGTTGAACCCTCTGGGAATGGAAG ATGGAACCAGCTCTGCGAGCCAAGCTCAGCTTTTATCTATGGGTCTCTGAGGGCTG GCAGAGCTGAGTGGGGACAACTGTGATCCGTGAGGCTCTCAGGTTGAGGTGGCCC CTCCGGGAGGGCTTCATTTTCCCAGCGGGTAGGTTCTAAGCAGCAGTGGCTGGGCA GGTGGGTCCAACACAGAGCCAGAGAAGGGTGAATGGGCCTCCTGGCACCCCACCC CTGCTGCCCCTGAGCTCAGTGATGGAGGGGGACAGCACAGCTGAGCCCAAGTGCT TTGGTGTGGCCCTGAGGGAAAGCTGCAGCCTGCCTGGGGCCTGGCATGGATGGGA CACTTGAGGCAGAGGGACAATAGTGGGCGCTGCAGTGAGGCTGGCTCTTGGAGAG GTTTCCTGAGGAGTGCTGCCTGAGACGGGCAGGGAGAACAGAGACAAAGTTGGTGA CAGGGAATGAAAGCTGACTGAAGGACTTTACCCAGACCTATGAGGATATCTCTCTCA GCAGGAAGCAGGAGGGGACTGTGTGAGGACTGGCCAAGAGCTGGAGTGTTGGGAA AATGACTCTTTCTCCGACCCCTCTGTCCTAGCTCTGGCCCCTGGACTGCGGAGGTCT GCTTCCACCCCCATTGGTCGATCGTTGTCCCTTGTCACAGCCATTGAGAATTTTGGC |
| | | AGGGAGCATGTTCTTAGAGCATTTTTAGGCTCTGCGGGACATAACAGCTCTGCCTCA GAGCACATGCCTTTCTCAGCTCCTGAAAGCCACTGATCAAATTGGAACATTTTGTAC CTTAGGGATGAGGATATCAACTCTCCCAGCCACTTAGAGGGATAAATGTGATGATGC ATTCAATTGTGACTACATCTGATCCCAACTGTTGCTTCAGCTGCTCTCCTATAGCACA TGGCGGGAGGCGTGCATCCCAGTAGCTACCTCCCCACTTTTGGGGAGATGTGGTTC CATCCATGAAACCTGGGTACCCGCCTACCAGGTCCTGGCCTATCAGGTGGCAGGGT CTGGTCAAAGAAGGGCATGTGTGGTCTTCAGCAAGGGAGACAGGACGGTGGTGCA GAGCGTCTAGACCCTCAGGGCAAGTCTCCCCCACACCTGCTCCCGGGGCAGTTGTC TTTGTGACCTCCCATCCCCCTCTGTTTCATCCTCTATAAAATGAGGGGCTGAGCCCC AAAATAACAGGCTTCTTTGCCATGATGCAAAACTGCTGAATCTTTCTTTCTGACACAC AAGGCATCGAGCAGCCTCTGAAAGAACCAAAGCCACTAGCAGGCTTCCTGACTTGG GTTTGTAGGTACTGAATACTCCCTTGAAAAATAAAAACATAGAGGCACTTTTCTCCTG GCTGTTTATTACAGAACGAAGAAAAAACACACTGGCTTGAAACAGACGCCAGATTTC AAATGTAGAGGTGAAATACGAGGTGGCAATTAAAATGTGATTACAGAAAGTCTGGAC ACTGAGAAAAGTTTACAGGACAGTGGGTGTGGGTTTTCTATAACAGACACTTAAATAT ACATGACGATAATTGCAGATAGAAACCATCAAAGACAAACCCCAAATCAACTAATAAT GTTTACAGATGTTCCCCCCCAAACCACAGAGCCTTACATCAAAACAAATACTGAAAG GCTTTAAACCAGGAACAGCTCGCCTTAACCCCACGAGGGTGCACACAAGCTGGGCT TTTTCTCTCGGTCTGAATGGTAAAGGGAGGAGGATACTCTAGCTCCTCCAGGTGGAT TGCTGAGACAGGGCTCGGCTCACACACTGTCTCTGCGCCTCTCCCAAATCTGGAGA ACTCTCCCAGCCTCCTGGTAAAGTGTCTCTGTGGGGCACTTAACGATAAAACAGCTT CTGCTGTAAAGCTCATTAGGAAAGAGCTAGCGGAGACTGAAAGGTTCGCAAAAGAG ATTAAGAATCACACAAGGCAATAGGATTTTTAGTGAACATAGAAATAAATGGCCAAGT GGTTTTCTATTTGGCATTTGTCAACTTGCACAACAACTCTTGGTCATATCCACATTGC TCATTGCATTAAAACCATAAGCGACTCAGCCACCTAGCTTAACAAGGTATCACTGGA GCAAACAACACGGTCTGCATATTTGTAACATTGTATAATAAACACAAAACAATGCATA GTAAACACAACTCTACTGAAACAAAAGCCGTCGCTTTATTTACAAAGTCACAAAATGA AGTATAAATACTTCTGTCATTAATGTTTAGGAAAACCATTTACAAAATTTTCAAATATG TACACGTAGCTTGAAAAATCACCAGCTTTCCATTTTGTCACAGGTAGAGAGAGGGAT AAGCATGGGCTGACAACACCACTCAAATTGTAACGGGAGACAACTGCGGGTATGGA TCGACACCACTTCCTAGAGTGATGTCACCATGGGGGTTTCTATGGGCATCCTGCTCA GATTTAAAGTGCCCCAGCATCCTGGGTGACTTGCCCAGAATTCTGGGCTGTGGCATT TTGAGCAGCAGCATGCTGTTCCAAAATGTCGTCGATCAGCCTCAAGTTGCACACCCA GTCTTCATCTGGGCTCACACAGGAGCCTTTCAAGAGAGCTTCAATGAAATCTACCTC ATTGCAGTCAGGTGACGAAATCAGATCATTTAGTGGGGGTTGGGGCTGGCGCAAAA AGTCGGCAGGTGGCAGCTCAGGGGGAATATCCGTTCTGTCGAACGGACCTGGGAA CTGGCTGGCAGCAACGGCAGAAGCAGCAGCAGCGGTGGCAGCAGCAGCCACATAG CTTGGTGGCTCGATGCCCTGTATGGGGCTCAGGGGACTAAAGCTGGCCATACCCTG CTGGAGGAACTTGGTGGTGTTTGCTACAGGCACCGGGCCCTGTACCGGGCTCTGCC TGAGGCTCTGGCTGCCCAGCAGGCTGAAGCTGGGGTTGTTGGCCAGGGGCACTTG TGTTCCCATCGCAGCGGGCACTTGTGCCTCCCAATCAGATGGCCTCTGAAGGCAGG CCTGGCCAGAAGGTGAGTGCTGCTGAACGCTATTATCCACTTGGCTGAGGGGTGTT TTCCCCGAAACTGCTGTGGTCACAGCTGCTGCCGCTGTGACCCATGCAGCATTGTT GAACGCAGTGGGCATTCTTGGCACACTAGGCCGTCTGAGCTGGTGGGGACTCAAG GACTGGGTGCCCAGGGAGCTGGGACAGAACCCAGGCAGGGGCACTTCTGGTGGG GTGGCCTTGGGGCTCTGCATATGCTGGCAGACAGAGTCAAGTCTGCCCAGGGGAGT CTGGCCTGAGTGTGAGAGGATGGGACACTGGGGGCTGGAGGTGAAAATTCCTTGC CGCTTCCCCAGAGTTGGTGAGATCACTCCCATGCCCTCGCAGCTCTGGTGCCTGGT GAGTGGGATCATTCCTGGACTCAGATTGTTCTGAAGAAGCCCAGTTCTGGGTGGCAT CAAGTGCTTGCTAGATGGGGGGCTTGCCTTGATCCGGCTACACTTGGAGGTGACTT GTTCTTGGACGGCTACATACAGAAAGAGAGAAGTGGGGATGAGTTCCAAAGGCATC CTCGACTTCGGCTGTGGCCACCGGAGGGTAGCTCCTGGCCCAACACGGACTTCTCA CCTCCCGCCCTTGGCTCTCTACTGAGCTCCCCCCTGCTCCCCAATTCCTCGCCATTC CCCTCATTTCTCTGCCCTCAGCCTGGACTGCAGTTCTTCTGGGAAGCTGCCCCAACT CCCTAGGTCTGTGCTCACCAAGAGCAGATCACACTGGACTGAAATGCCAGCTGATTT GTCTCTTCAAGAAAATTGGAAGCTCCTGGAGGTCAGGGTCCATGTCTGCTTTTACAC TCAGTGCTCTGTATGCAGGCCTGGCACTGCCCACCCTTTGACAGGTGGTGCATATTT TGTAGAAGGAAGGAAGGGGCCAGGTGGGGTGGGCTGGGCTGGTGGCGGGAGCTA GCTCAGCCTCTTAGATTCTCTACCCGATGGATGTGACCTGGGACAGCAAGTGAGTGT GGTGAGTGAGTGCAGACGGTGCTTTGTTCCCCTCTTGTCTCATAGCCTAGATGGCCT CTGAGCCCAGATCTGGGGCTCAGACAACATTTGTTCAACTGAACGGTAATGGGTTTC CTTTCTGAAGGCTGAAATCTGGGAGCTGACATTCTGGACTCCCTGAGTTCTGAAGAG CCTGGGGATGGAGAGACACGGAGCAGAAGATGGAAGGTAGAGTCCCAGGTGCCTA AGATGGGGAATACATCTCCCCTCATTGTCATGAGAGTCCACTCTAGCTGATATCTAC TGTGGCCAATATCTACCGGTACTTTTTTGGGGTGGACACTGAGTCATGCAGCAGTCT TATGGTTTACCCAAGGTCAGGTAGGGGAGACAGTGCAGTCAGAGCACAAGCCCAGT GTGTCTGACCCACCCAAGAATCCATGCTCGTATCTACAAAAATGATTTTTTCTCTTGT AATGGTGCCTAGGTTCTTTTATTATCATGGCATGTGTATGTTTTTCAACTAGGTTACAA TCTGGCCTTATAAGGTTAACCTCCTGGAGGCCACCAGCCTTCCTGAAACTTGTCTGT GCTGTCCCTGCAACTGGAGTGTGCCTGATGTGGCACTCCAGCCTGGACAAGTGGGA |
| | | CACAGACTCCGCTGTTATCAGGCCCAAAGATGTCTTCCATAAGACCAGAAGAGCAAT GGTGTAGAGGTGTCATGGGCTACAATAAAGATGCTGACCTCCTGTCTGAGGGCAAG CAGCCTCTTCTGGCCCTCAGACAAATGCTGAGTGTTCCCAAGACTACCCTCGGCCT GGTCCAATCTCATCCCACTGGTGCGTAAGGGTTGCTGAACTCATGACTTCTTGGCTA GCCTGCAACCTCCACGGAGTGGGAACTACATCAGGCATTTTGCTAACTGCTGTATCC TAGGCCAATAAATGTTGATCACATTTATAGCTGCCATGGTAGGGTGGGGACCCCTGC TATCTATCTGTGGAGGCTCTGGGAGCCCCTGACACAAACTTTCTGAAGCAGAGCCTC CCCAACCCCTTTTCCATTCCCTATACCTGACAGATGGCCCAGGAACCCATTAGAAAT GGAAGGTCACTGCAGCAGTATGTGAATGTGCGTGTGGGAGAAGGGCAGGATCAGA GCCCTGGGGGTGTGGCAGCCCCCAAGTGATTCTAATCCAGATCCTAGGGTTGTTTC CCTGTCCCATTGAAATAGCTGCTTTAAGGGGCCTGACTCAGGGAAATCAGTCTCTTG AATTAAGTGGTGATTTTGGAGTCATTTAGACCAGGCCTTCAATTGGGATCCACTAGTT CTAGAGCGGCCGGGCCCAGGGAACCCCGCAGGCGGGGGCGGCCAGTTTCCCGGG TTCGGCTTTACGTCACGCGAGGGCGGCAGGGAGGACGGAATGGCGGGGTTTGGGG TGGGTCCCTCCTCGGGGGAGCCCTGGGAAAAGAGGACTGCGTGTGGGAAGAGAAG GTGGAAATGGCGTTTTGGTTGACATGTGCCGCCTGCGAGCGTGCTGCGGGGAGGG GCCGAGGGCAGATTCGGGAATGATGGCGCGGGGTGGGGGCGTGGGGGCTTTCTC GGGAGAGGCCCTTCCCTGGAAGTTTGGGGTGCGATGGTGAGGTTCTCGGGGCACC TCTGGAGGGGCCTCGGCACGGAAAGCGACCACCTGGGAGGGCGTGTGGGGACCA GGTTTTGCCTTTAGTTTTGCACACACTGTAGTTCATCTTTATGGAGATGCTCATGGCC TCATTGAAGCCCCACTACAGCTCTGGTAGCGGTAACCATGCGTATTTGACACACGAA GGAACTAGGGAAAAGGCATTAGGTCATTTCAAGCCGAAATTCACATGTGCTAGAATC CAGATTCCATGCTGACCGATGCCCCAGGATATAGAAAATGAGAATCTGGTCCTTACC TTCAAGAACATTCTTAACCGTAATCAGCCTCTGGTATCTTAGCTCCACCCTCACTGGT TTTTTCTTGTTTGTTGAACCGGCCAAGCTGCTGGCCTCCCTCCTCAACCGTTCTGAT CATGCTTGCTAAAATAGTCAAAACCCCGGCCAGTTAAATATGCTTTAGCCTGCTTTAT TATGATTATTTTTGTTGTTTTGGCAATGACCTGGTTACCTGTTGTTTCTCCCACTAAAA CTTTTTAAGGGCAGGAATCACCGCCGTAACTCTAGCACTTAGCACAGTACTTGGCTT GTAAGAGGTCCTCGATGATGGTTTGTTGAATGAATACATTAAATAATTAACCACTTGA ACCCTAAGAAAGAAGCGATTCTATTTCATATTAGGCATTGTAATGACTTAAGGTAAAG AGCAGTGCTATTAACGGAGTCTAACTGGGAATCCAGCTTGTTTGGGCTATTTACTAG TTGTGTGGCTGTGGGCAACTTACTTCACCTCTCTGGGCTTAAGTCATTTTATGTATAT CTGAGGTGCTGGCTACCTCTTGGAGTTATTGAGAGGATTATAAGACAGTCTATGTGA ATCAGCAACCCTTGCATGGCCCCTGGCGGGGAACAGTAATAATAGCCATCATCATGT TTACTTACATAGTCCTAATTAGTCTTCAAAACAGCCCTGTAGCAATGGTATGATTATTA CCATTTTACAGATGAGGAACCTTTGAAGCCTCAGAGAGGCTAACAGACATACCCTAG GTCATACAGTTATTAAGAGAAGGAGCTCTGTCTCGAACCTAGCTCTCTCTCTCTCGA GTAATACCAGTTAAAAAATAGGCTACAAATAGGTACTCAAAAAAATGGTAGTGGCTGT TGTTTTTATTCAGTTGCTGAGGAAAAAATGTTGATTTTTCATCTCTAAACATCAACTTA CTTAATTCTGCCAATTTCTTTTTTTTGAGACAGGGTCTCACTCTGTCACCTAGGATGG AGTGCAGTGGCACAATCACTGCTCACTGCAGCCTCGACTTCCCGGGCTCGGGTGAT TCTCCCCAGGCTCAGGGGATTCTCCCACTTCAGCCTCCCAAGTAGCTGGGACTACA GGTGCGCACCACCATCCCTGGCTAATATTTGTACTTTATTTTATTTATTTATTTATTTA TTTTTTGAGATGGAGTTTCGCTCTTGTTGCCCGGGCTGGAGTACAGTGGCATGATCT CGGCTCAGTGCAACCTCTGCCTCCCGGGTTCAAGCGATTCTCCTACCTCATCCCCCT GAGTAGCTGGGATTACAGGCGCCTGCCACCATGCCTGGCTAATTTTTTGTATTTTTA ATAGAGACGAGGTTTCACCATGTTGGCCAGGCTACTCTCGAACTCCTGATCTCAGGT GATCCACCCGCCTTGGCCTCCCAAAGTGCTGGGATTACAGGCGTGAGCCACTGCGC CCGGCCTAATATTTGTATTTTTTGTAGAGATGGTGTTTTGCCATGTTGTCCAGGCTGG TCTTGAACTCCTGAGCTCAAGCGATCTGCCCGCCTCTGCTTCCCAAAGTGCTGGGAT TACAGGCATGAGCCACCGTGCCTGGCCTAGGTAGACGCTTTTAGCTTTGGGGTGTG ATGCCTGCCCCAGTATATAGTGAATTTAATTATTGCTAGAGCTGGCTGTTTGTTAGTT TTCTTTGAACATAAGATACTCATTGTTTTTAGTTTGCAAATCCCTCTTCCTTTTTAAAAA ATTTCTTTCCCTTAAATTGTTTGCATGTTAGCAATAACAAATGCTTAAATGGTGCTATG TGCTAGATACTCTTCTAAGCCCTGTTATGTATATTAACTAATTTTTTAAATTACACAAAT CAGAGAGGTTAAGTAACTTGCCCAAGATTACCCAACAATACTAGGATTTGAACCTAA GTTTGTCTCACCCCAGATTCTGCTCTTAATCTCTAAACTTTTAAGTTAGTAGTGACAAT AGTAGGTATTTATTGAATACTTAACTATGTTTTAGGCGTTGAAGTAAATATTTTGCAGG CATTATCTAATGTAAACACCCTAAAGTTACATAACAGGTACCCTTTAGGTAAATAAAC ACTAGTATGACCTTGGAGGCACAGATAGTTGAAGTAACTTGCCCAATATCACTTACAT GAAATTGGCCCTCAAATGTGTCTGATACAACCCATGCTGCTTGTAACTATCGTTTTAA ACTGCCAGGGTAAACTTGGACACACTTGAGCTAAGAAAAAGCTTTTAGATTTTTGCAA ATTAATGTGAAAGATATGCTTTATGTGGATATAATATCTTCTAAATTTCGGGGATGGTA GTCCTAGAAATGTAATCCTGCCCTAGCCGAGCTTACCCTGCCAATAATTTTTTACAGA ATTGGTAAAACGGAGCACCTTTTTTTTGTCCTTGGCCACACTGTTATCAACAGGGTGT AGATTGACATCAATCTGTAGGTGTAAACCAGAATTACTCTTTGTGACCACCAGGAAAT AGAGCAGTTCAGTTCAGGGGTTTCTTTCTGTGAATTTAGCACTGTGACCTGCATACTA CAAGTCTACTTTGTTTTCTATCCATTGTTTGTATCTGGGTATTGCAAAAGGTAGGAAA AGGACCAACCAGATCAGCAGAGAAGAGTTGCCTTGGAGTTTTCTTTTAGTTTTCTGC AGTTCATTAGATAGTAACTAGGCCATGTCATTTTACTCCCTTGTAGTGAAGATATGTT |
| | | GAAGTTGTACTGGTATACTCTTCTACCTTTCTGTAATTTTATATTGTGTAGACTTGATA AAATTTATGTGTCAATCACCACCATTAATATCAATATTGAGCCTCAATTCTTATTTTTCT GCCCAGTGGCTGCCAAATTACTAACATTTACAATAATTCACTACTACTAAGATAATCT ACTAGTTCGATCACATACTTCAAATTGTTATGGAACTACTGTCTTCAGCATTGTGCTT CTGATAACTGATAAGTATAATTTTTTTTTTGTCCAGAGTGAACATGTCTATTCTTCCAC TGTACACACTAATAAAAGGAAAAATTGTAATATTGGGTAAATTCATGTCCTTACACAT GTAGTAGTTATGAGCCCATGTCCCTAGAATGAGTAATAATTTATCCCTCCCTTGGTTG AATAGTCAAGAATGCTGATTTTAATTCTTCTAACAGCTTTATCCCTCAGAAGGGAAGG CAAGCAAGTTATATATGTAGTTTATTTGTAAGACTGATATGAAATTGGAAGATGAATCT ACTATTAGCTTTAATTATTTTTACATTTAGGAATATTGCATCAGTAACTCATAATTTTGG TTTTCTGTTATCCTGAGTTAACACAAATTATCCAAGGAGATGGCGGATCATCTGCTTT GAGGTGTTTTTTTTTGAGAATTTTAATGTATCTGAATATAAAAGGTAAAAATATGCCAA CTAGCAATTTCTGCCCATTCCAGAAGTTTGGAAATATTACTCATTACTAGGAATTAAAT AAAATATGGTTTATCTATTGTTATACCTCTTTTAATTCACATAGCTCATTTTTATCTTTT ATTTTTGTTTGTTTTTTTTGAGATGGAGTCTTGCTCTGTCACCAGGCAGGAGTGCAGT GATGCAAATCTCGGCTCACTCTAGCCACCGACTCCCTGGTTCAAGCGATTCTCCTGC CTGAGCCTTCTGAGTAGCTGGGATTACAGGCAGGCACCACCACGCCCAGCTAATTT TTGTAGAGACAGGATTTCACCGTGTTGGCCAGGATGGTCTCCATCTCCTGACCTCAT GATCTGCCTGCTTCGGCCTCCCAAAGTGCTGGGATTACAGGTGGGAGCCACTACGC CTGGCCCACATAGCTCATTTTTAGACTCACTTCCATTAAGTCTTGTTTGGACCCACGA ACATTGTCTTTTTTTTTTTAAGATGGAGTTTCACTTTTGTTGCCCAGACTGTAGTGCAA TGGTGCAATCTCAGCTCACTGCAATCTCTGCCTCCTGGGTTCTAGCAATTCTCCTGC CTCAGCCTCCCGAGTAGCTGGAATTACAGGCGCCCGCCACCACGCCCAGCTAATTT TTGTGTTTTTAGTAGAGACGGGGTTTCACCATGTTGGGCAGGCCAGGGGTGATCCG CCCACCTCAGCCTCCCAAAGTGCTGGGATTACAGGTGTGAGCCACCGCATCTGGCC AACATGTCTTTTTTTTTTTTTTCCTTTTTAACCACAAAGAGACTTAAGCAGTCCTTGTC ACAGATGATGAATTGATGTTGCAAGTATTGTCTTAGCTTGGATTAATTTTCTTGCTTAC TGTAATTTTAGATAATATAGCTTTGTAATTAGAGATTTTATGTGTAAACCACAAAAATG TTTACATGAAGGCCATTATTACAGATGTGACGTGCATAATTATTAGTAATTTGTATGTT TACATGGGTCAGTCTGGCAAAAAATTATGAAGTTTTAAAAATTAAAAAAAATTATAATG CCAGTTTTACTGGAAAGTAAAATTATTTCAGTAATCGATTATAGCAAAAGTATTGATTT TCATTCCAGACAAAAGTCAGAATGAAAGGTAATTTCTCAATACTCTTTCAGATTAATAA AAGTACCTGTAGCGATTTTTATCATTCACAAGTATATCACAAGTAAGTTAGAATTTGA GAACTGTGTTCTAGATCTCTGAGGAGATGCAGTCAGATTTCTGAACTGTCTCAGCAA ATGGTAAGTAACTTAGAGCTAGTAATTAATAACCTGTCCTTTGATTTCTGATTCAGCC AAGAATGGCCATATTTGGGAAAGGCAGATCTGGAGAGTAACCACGTTTTCATTCATT TACCACTTCTAGGCCCCTCCAGAGCTCTCAGATATTTTGGGGTTGAGCCCTTCCCCA AAGCCATACAGGACCTTTTTTTTGTGATCTGTTCTAGCCATTTTTATGTTGGGTGCTT GTTATGGACTGAGCATTTATGTCCTCCCACACCCCCCCCATACCTTTTTTGAAGTCCT AACCCCCAGTGTGATGGTATTTGGAGACAGGGCCTTTGGAAGGTAATTACAGTTAGA AGAAGTCGGGAGGGTTGGGCCCAGGTCTGATTGGATTAGTGCCCTTATATGAAAAG ACACCAGGACGGGCGCAGTGGCTCACACCTGTAATCCCAGCACTTTGGGAGGCCAA GGTGGGTGGATCACGAGGTCAGGAGTTTGAGACCAGCCTGGCCAATGTAGTGAAAC ACCATCTCTACTAAAAATACAAAAATTAGCTGGGTGTGGTAGCGGGCTCCTGTCATC CAAGCTACTCGGGAGGGTGAGGCATGAGAATCACTTGAACCCGGGAGTTGGAGGTT GCAGTGAGCCCAGATTGTGCCACTGTACTCCAGCCTGGGTGACAGAGTGAGACTCT GTCTCAAAAAAGAAAAAAAAAAAAAAAGAGACACCAGAGAGCTTGTTAGAAGAGGTC ATGTGAGCACACAGTTAGAAGACCTTCAAGCCAAAGAAGAGGCCTGAGATTGAAACC TACCTTGCAGGTACCTTAATTTTGGACTTCCCAGCCTCCAAAACTGTGAGAAATAAGT TTCTGTTAAGTCACTCAGTCTGTGGTATTTTGTTATGGCAGCCTGAGCAGGTAGTTGT TCTTTCAGAAGGTGTTGATAATAACCACATGCAACACCAAGTCACAAATAATAAAACA GATGTAACTTATATTCATACAGAAAGTTGGGCACTGCCATTGCCTTGTTGGTTTACAC GGCTGTGCTAGTTCAGTAGCAGAAAGGTGCTGGTCTCCTTTACTCAGTTTACAATCT AGGCAGTAGAATGTAATCACTGCTTTAAACTTGATACTGCTTAGGGAGAGAATCATTG GTGCTGGGTAACTTTGGGTTCTAGGTTTACTTTTTGTGTATATATAACTGTTTTTGGTA AATCACAAGTTTCTGGGCTTGTCGAATTAGATTTTGTTACAGATTATGAGCTTTATTAT GCTATACAGTTAGTTGTATGTATATATGCCTTTCCCACTAGATTTTAAGCTTTTTTTTTTT TTTTTTTTTTGTGACGGAGTCTTGCTCTTGTCGCCCAGGCTGAAGTGGAGTGCAGT GGCACAATCTCGGCTCACTGCAGCCTCCACCTCCTAGGTTCAAGCGATTCTCCTGC CTCGGCCTCCCAAGTAACTGGGACTACAGGCACGTGCCACCACACCCGGCTAATTT TTGTATTTTTTGTAGAGACAGGGTTTCGCCATGTTGGCTAGGCTGGTCTTGAACTTCT GGCCTCAGGTGATCCACCCGCCTCAGCCTCCCAAAGTGCTGGGATTTACAGGCATG AGCCACCACGCCCAGCTATAGCTCTTTAAGGGTTGTAAATTTATAATCATTCTTTTAC TCTCCTGCAAATTCTGTTGCACACTGCCTTAATCAAGGTAGATGCTGAATGCATTTTT GTATAATTGAATATGTTGCAATCCCCAACTCTCTCCAACTGTTCCTGTCAAAGCAGCC ACTGGATTGTTAACTAATCCATATTAGATGGGGTTAATTAATATCAGATGGGACAAGT AAGGGCTAATAAGATTATAGGCCACCAAGTAGATTTCTGTCTAGCTCTTATAGAGATT GAGTTTATTGGACCTGTTTGATAGGAAGTTTTGGTGTTTGGGATGATTAAAACTGAAG TTCCTATTTATTGAATTATACCTATTTATATTATTTCATATCAGTGGTCCACATGCAAGT GAGGCTTCTGAGACAGAGTTTGAGTTCTCTCTTCAACTACCATAACACTTAACCTGTA |
| | | TCTTTTTTTTTTTTTTTTTTTTTAGACAGGAGTCTCGCTCTGTCACTCAGGCTGGAGTG TAGTGGTATGATCTCGGCTCACTGTAACCTCTGCCTCCTGGATTCAAGCAGTTCTCC ATGTCTCAGCCTCCCTAGTAGCTGGGATTACAGGCCTGTGCCACCATGCCTGGCTA ATTTTTTTTTTGTATTTTTAGTAGAGACGGGGTTTTACCACGTTGGCCAGGCTGGTCT CGAACTCTTGACCTCGAGCGATCAACTTGCCTTGGCCTCCCAAAGTGCTGGGATTAC AGGCATGAGCCACAGCGCCCAGCCGTCTTTTTTTTTAAATAGCAATTTAACACTGTTC ACAGTTACTCATGTACATGTCATGCCATCTATTACACTGTAAGTTCTGTGAGGGTAGC TGTATCAAATTTATCTAACTCTCTCTAGTATGCATGACATAGTAAGTATTCAATAAATA TTTGCATATTAGTGATAAGGATACAGGTTCTGAATAGTGGGTCCTTACCATTTAAGAA TTAGTATTTGATGGCCGGGCGGGGTGGCTCACGCCTGTAATCCCAGCACTTTGGGA GGCTGAGGCGGGCGGATCATGAGATCAGGAGATCGAGACCATCCTGGCTAACATG GTGAAATCCCGTCTTTACAAAAAAAATACAAAAGAATTAACCAAGTGTGGTGGTGGG TGCCTGTAGTCCCAGCTACTGCTTTGTGAGGCTGAGGCAGGCAGATCACCTGAGGT GGGAAATTCAAGACCAGCCTGACCAACATGGAGAAACCCCATCTCTACTAAAAATAC AAAATTAGCCGGGCGTGGTGGCGCATGTCTGTAATCCCAGCTACTCGGGAGGCTGA GGCAGGAGAATGGCGTGAACCCGGGAGGCGGAGCTTGCAGTGAGCCAGGATCGC GCCACTGCACTCCAGCCTGGGCGACAGAGCGAGACTCCGTCTCAAAAAAAAAAAAA AAAAAAAAATTAGTATTTGATATTTGATCATTAAATATGAATTAAGAGGACTTAGACTT TTTGTTAAATGTCAAGCTGGGAAAAGTTGTCATTTAAATGAATTGCCTCTTATTTAATT TCGTCTGATGATACATTTTGTTTTTATTTTGTAAAAAATTATTTTTTTTCTTTTTGGAGA CAGGGTCTTGCTCTGTTGCCCAGGCTGGTCACAAACTCCTGACCTCAAGCAATCCTC CTGCCTTAGCCTCCCAAAATGCTGGGATTACAGGCGTGACGACCTCGCCCGGCCTT GTATTATGATACATTTTGAACAACTACAAGTAGACTTGGTATAATGAACCTGCACGTA CCCATTGCCAAGTTCTGACAACTGTCTGTCTATAGCCAATTATGCATTTCTTAAATTA GAACCCCCCCAATATACCCAAATATATATATATGTGTGCATATATATAGTAAGTTGTAA CAAAGTTGTGAATTCATACCTGAAGTATCTCAAGTGATGCAAGTTTTATGAATTTTTGT TTATGCCTTTTGGGAAGAGTTGTATTGACAAATTTTTTATGCTTAAAGTAAACCATAAA TCAAAAAAATAAAATCTAGGATGCAATAAAACAAAACAACTTCTTGACATAAGTATGG TATGTAAATCTGTTTTGATTGGAAATCAATTTGTTATATTGCCAGAATTCCTGTTTTAG AATACATCTCTGCTGATCTGTCTGTATTCTTAGACTGCATATCTGGGATGAACTCTGG GCAGAATTCACATGGGCTTCCTTTGAAATAAACAAGACTTTTCAAATTCTTAGTCGAT CTGCAGAACCTGTAGCCAGGCACTGAACCATTTTGATAGATGCAGTAATCGTTGCAA GTGTATATTTCAAGGGAGTTCTGGCTGGGTCCTAGTTTATGCTTGTGGCAGAAGCAG TGAGTAACTGGGAGGAAGTTGGTGAGTAAGCTTCAAGGAAGAAGTCATTTTTAGTAC TCTGGATCTTCCTGATTTTAAAGCACTACAAAATGGTGCATTTTCATTCTTGTCAAGT GATAACAGATATATTCTGATGAGCCTGAAATGAATATATATTGTATCATTTTTATAATA TCTAGCAAGGTTTGTATTTTCCTAGAACTTGAACTAAATTTCAGTTCATAAAATTTATA AAATACTTAGTTGTTGTAAAATATTTTTGGAATGTTCACATAGGTGACACACAAATGTC CCATTTTCATTCTTTCTATAGTAAATATGTTCTGATATGTGAAGGTTTAGCAGATGCAT CAGCATTTAATCCTAGAGGATCTGGCATAATCTTTTCCCCCAAGAATAGAAATTTTTT CTGCTTATGAAAGTAGTACATGTTTCTTTAAAAACAAATCAATATTGACTTCTGCCTGC TGTATAGCACTATGCCTCCACCTGGCCATGACCAGGGGCATGTCCTGGTCCACCTA CCTGAAAATGTTTGCAACCAGCCTCCTGGCCATGTGCACAGGGGCTGAAGTTGTCC CACAGGTATTACGGGCCAACCTGACAATACATGAAGTTCCACCAAAGTCTGAGAACT CAGAACTGAGCTTTGGGGACTGAAAGACAGCACAAACCTCAAATTTCTCAGCACTGG AAACCTCAAAATATAACTGAATTCCATAAATAAGATTTTAAGTCTTAAATATGTATTTTT AAATGTATTAAAAGTCAAGCTGCTTGTATTTAAGCACCTAATACAATGCTTAGGTTGT AAAAGGAGATGCTCAATAGGTACTAACTGATATATTGAGATTTAATTATGGTTTGACC AATATTTATTGGAAACCGCCAAAGCTTAAATCATCAGCTTCTTGAATGTGATTTGAAA GGTAATTTAGTATTGAATAGCATGTGAGCTAGAGTATTTCATTCTTTCTGGTTTATTTC TTCAAATAGACTTTGAATATAATGGTGAATGGGTATTATAAATTAACTAATAAAAATGA CATTGAAAATGAAAAAATATATATATTAAAGTGTAGAAAGTGACCAGGCGTGGTGGCT CACACCTGTAATCCAAGCACCTTGGGAGGCTGAGGCAGGAGGATCTCTTGATCCCA GGAGTTCAAGACCAGCCTGGGCAACATAGCGAGACTTCGTCTCTAAAAAAAAAAAAG AGAGAGAAAAAAATTTTTTTTATTTAAAAAAAGTGTAGAAAGTGTCAAGACCCCACTT CTTACCATTATTTGGTATATTTCTCTATACCCACCCACCCTTCCTCCTTACTCCCTCCC TCCCTTCCCAATCTTTTTATCTTTTTGTATTCTGATTTTTTGTTTGTATATTTTGCTTTAA TTTAATGTATCCTTTAAAAATTTCCCATACATTTTATATGTATATATAAAAACGCATGCT GCCAAAGATAATTTATAAGAAAGACCATTGAATTTTTTTAAAAGTGATATATATTCATT GAAAAAAATTTAGAATATATAGCAAAGCAATAAAGAACTAAATAAAATTGCTGTAACTC CTCTTTCAAAGATAAGTGCTTTTATGATTTTGTTGTATTTTTTTCTGTATATAGGTACAT ATATAGTATTTATAAAGCTGTACTCATAGTACATTTTCACATCACAGGTACCATATCAG TGTTATTAAATATTTTGTATGCCAGGGGCTAGACATACCAAGACAACCAATATGTGGT TCTACTTAAATAATATTAGAGTATCTTTTATGATGACACTTCATGAGTTGACTATAATA ATCTTAGACTTCTAAGAGTTTGGGTTTTCAAAAGATCACTTAGCTTTTTTGGGTGATTT TTCCCCCTTACTGTGAGATGAGAGAGGCTGTTTGGATTTGGGATTGGGGTAGCGGG GACAGCAACTTTTCTTTTCTTTTTCTTTTTTATTTTGAGGTAGGGTATTGCTGTGTCAC CCAGGCTGGAGTGCAGTGGTGTGATCTCGGCTCACTGCAACCTCCACCTCCCGGGC TCAGGTGATCCTCCTGCTTCAGCCTCCCAGTAACTGGGACTACAGGCGCGTGCCAC ATGCCTGGCTAATTTTGTATTTTTAGTAGAGATGGGGTTTCACCATGTTGGCCAGGCT |
| | | GGTCTCTAACTCCTGACCTCAGGTGATACGCCCACCTGGGCCTCCCAAAATACTGG GATTACAGGCATGAGCCGCTGCATCAGCCAGCAGTTTTTCTTGTGGTTTTTTTTGTTT GTTTTGTTTTGTTTTGTTTTTGAGATAGGGTCTTACTCTGTTGTCCACGCTGGAGTGC TGTGGTATGATCGTAGCTCACTGCAGCCTCAAACTCCTGGGCTCAAGTGATTCCTTC TGCCTCCGCCTCCCGAGTAGCTGGGACTACAGGTATGCACCACCATACCTGGCAAA TTTTTACAAAGTTTTTTGTAGGGACGGGGTCTTGCTACATTCCCCATGTCGGTCTTGA ACTCCTGGCCTCAAGCAACTCTCCTGTCTCAGCCTCCCAAAGCACTGGGATTACAAG TGTGAGCCACCACACCATGCCAGTTTTTCCTGTTCAGTGTGATATTTTATCTTGTTAG ACTACAGTGTGTTAAAACTTGTTTTACTAAATTTTCAAACATACTCAAAAGTGGAGAGA ATAGTATAATGAATACCCGTATGTTCATCACCCATGTTTAGAATATTATTAAATATAAA GATTTTGCTGCGTTTGTCTTAGCTCTTTAAAATTTTTCTTTTTCTCTTTGTGACCTAAA GGAAATTCCATATCTTATCACTTTACTTCTACATTCTTGACTAAGATGACTAAGACATA TAGTTACATGGTTTTTTGTTTTGTTTTTGTTTTTTAAAGACGAAATCTCGCTCTTGTCC CCCAGGCTGGAGTGCAATGGTGCCATCTCAGCTCAGTGCAACCTCTGCCTTCTGGG TACAAGCGATTCTCCTGCCTCAGCCTCCCAAGTAGCTGGGATTACAGGCTCCTGCCA CCACGCCTGGCTAATTTTTGTATTTTTAGTAGAGACGGCGGGGGGAGGTTTCACCAT GTTGACAAGGCTGGTCTGGAACTCCTGACCTCAGGTGATCCACCCGCCTCGGCCTC CCAAAGTGCTGGGATTACAGGCGTGAGCCACCGCGCCCAGCCTGTTTTTTTGTTTGT GTGTTTTGTTTTTTTTGAGACAGAGTCTTGCTCTGTTTCCCAGGCTGGAGTGAAGTG GTGCCATCTCAGCTCAGAGACAGAGTCTTGCTCTGTTTCCCAGGCTGGAGTGAAGT GGTGCCATCTTGGCTCACTGCAACCTTCACCTCCCAGGTTCAAGTGATTCTCCTGCC TCAGCCTCCCAAGTAGCTGGGACTACAGGCATGTGTCACCACACCCGGCTAATTTTT TTGTATTTTTAGTAGAGACGGGATTTCACCGTGTTGCCCAGGCTGGTCTCGAACTCC TGAGCTCAGGCAGTCTGCCTGCCTCAGCCTCCCAAAGTGCTGGGATTACACGTGTG AACCAACCCGCCCGGCCTGTTGTTTTCTTACATAATTCATTATCATACCTACAAAGTT AACAGTTACTAATATCATCTTACACCTAAATTTCTCTGATAGACTAAGGTTATTTTTTA ACATCTTAATCCAATCAAATGTTTGTATCCTGTAATGCTCTCATTGAAACAGCTATATT TCTTTTTCAGATTAGTGATGATGAACCAGGTTATGACCTTGATTTATTTTGCATACCTA ATCATTATGCTGAGGATTTGGAAAGGGTGTTTATTCCTCATGGACTAATTATGGACAG GTAAGTAAGATCTTAAAATGAGGTTTTTTACTTTTTCTTGTGTTAATTTCAAACATCAG CAGCTGTTCTGAGTACTTGCTATTTGAACATAAACTAGGCCAACTTATTAAATAACTG ATGCTTTCTAAAATCTTCTTTATTAAAAATAAAAGAGGAGGGCCTTACTAATTACTTAG TATCAGTTGTGGTATAGTGGGACTCTGTAGGGACCAGAACAAAGTAAACATTGAAGG GAGATGGAAGAAGGAACTCTAGCCAGAGTCTTGCATTTCTCAGTCCTAAACAGGGTA ATGGACTGGGGCTGAATCACATGAAGGCAAGGTCAGATTTTTATTATTATGCACATCT AGCTTGAAAATTTTCTGTTAAGTCAATTACAGTGAAAAACCTTACCTGGTATTGAATG CTTGCATTGTATGTCTGGCTATTCTGTGTTTTTATTTTAAAATTATAATATCAAAATATT TGTGTTATAAAATATTCTAACTATGGAGGCCATAAACAAGAAGACTAAAGTTCTCTCC TTTCAGCCTTCTGTACACATTTCTTCTCAAGCACTGGCCTATGCATGTATACTATATG CAAAAGTACATATATACATTTATATTTTAACGTATGAGTATAGTTTTAAATGTTATTGGA CACTTTTAATATTAGTGTGTCTAGAGCTATCTAATATATTTTAAAGGTTGCATAGCATT CTGTCTTATGGAGATACCATAACTGATTTAACCAGTCCACTATTGATAGACACTATTTT GTTCTTACCGACTGTACTAGAAGAAACATTCTTTTACATGTTTGGTACTTGTTCAGCTT TATTCAAGTGGAATTTCTGGGTCAAGGGGAAAGAGTTTATTGAATATTTTGGTATTGC CAAATTTTCCTCTAAGAAGTTGAATCATTTTATACTCCTGATGTTATATGAGAGTACCT TTCTCTTCACAATTTGTCTCTTTTTTTTTTTTTTTTGAGACAAGGTCTCTGTTGCCCAG GCTGGGGTGCAGTGCAGCAGAATGATCACAGTTCACTGCAGTCTCAACCTCCTGGG TTCAAGCGATCCTTCCACCTCAGCCTCCTGAGTAGCTGGGACTATAGGTGTGCGCC ACCACTCCCAGCTAATATTTTTATTTTGTAGAAACAGGGTTCGCCATGTTACCCAGCC TCCCAAAGTGCTGGGATTACAGGCATGAGCCACTGGCCCAGTTTCTACAGTCTCTCT TAATATTGTATATTATCCAGAAAATTTCATTTAATCAGAACCTGCCAGTCTGATAGGTG AAAATGGTATCTTGTTTTTATTTGCATTTAAAAAAAATTATGATAGTGGTATGCTTGGT TTTTTTGAAGGTATCAAATTTTTTACCTTATGAAACATGAGGGCAAAGGATGTGATAC GTGGAAGATTTAAAAAAAATTTTTAATGCATTTTTTTGAGACAAGGTCTTGCTCTATTG TCCAGGCTGGAGTGCAGTGGCACAATCACAGTTCACTCCAGCCTCAACATCCTGCA CTAAAGTGATTTTCCCACCTCACCTCTCAAGTAGCTGGGACTACAGGTACATGCTAC CATGCCTGGCTAATTTTTTTTTTTTTGCAGGCATGGGGTCTCACTATATTGCCCAGGT TGGTGTGGAAGTTTAATGACTAAGAGGTGTTTGTTATAAAGTTTAATGTATGAAACTT TCTATTAAATTCCTGATTTTATTTCTGTAGGACTGAACGTCTTGCTCGAGATGTGATG AAGGAGATGGGAGGCCATCACATTGTAGCCCTCTGTGTGCTCAAGGGGGGCTATAA ATTCTTTGCTGACCTGCTGGATTACATCAAAGCACTGAATAGAAATAGTGATAGATCC ATTCCTATGACTGTAGATTTTATCAGACTGAAGAGCTATTGTGTGAGTATATTTAATAT ATGATTCTTTTTAGTGGCAACAGTAGGTTTTCTTATATTTTCTTTGAATCTCTGCAAAC CATACTTGCTTTCATTTCACTTGGTTACAGTGAGATTTTTCTAACATATTCACTAGTAC TTTACATCAAAGCCAATACTGTTTTTTTAAAACTAGTCACCTTGGAGGATATATACTTA TTTTACAGGTGTGTGTGGTTTTTTAAATAAACTCCTTTTAGGAATTGCTGTTGGGACTT GGGATACTTTTTTCACTATACATACTGGTGACAGATACCCTCTCTTGAGCTACATCGG TTTGTGGGGAGTCAAAAGTCCTTTGGAGCTAGGTTTGACAAATAAGGTGGGTTAACA CTTGTTTCCTAGAAAGCACATGGAGAGCTAGAGTATTGGCGAATTGAAGAAATCCCC CTTTTTTTTTAACACACTTAAGAAAGGGGACTGCAGGTATACTCAAGAGAGTAAGTCG CACCAGAAACCACTTTTGATCCACAGTCTGCCTGTGTCACACAATTGAAATGCATCA CAACATTGACACTGTGGATGAAACAAAATCAGTGTGAATTTTAGTAGTGAATTTCATT CATAATTTGATCGTGCAAACGTTTGATTTTTATTACTTTAGACTATTGTTTCTGATTTTA TGTTGGGTTGGTATTTCCTGTGAGTTACTGTTTTACCTTTAAAATAGGAATTTTTCATA CTCTTCAAAGATTAGAACAAATGTCCAGTTTTTGCTGTTTCATGAATGAGTCCTGTCC ATCTTTGTAGAAACTCGCCTTATGTTCACATTTTTATTGAGAATAAGACCACTTATCTA CATTTAACTATCAACCTCATCCTCTCCATTAATCATCTATTTTAGTGACCCAAGTTTTT GACCTTTTCCATGTTTACATCAATCCTGTAGGTGATTGGGCAGCCATTTAAGTATTAT TATAGACATTTTCACTATCCCATTAAAACCCTTTATGCCCATACATCATAACACTACTT CCTACCCATAAGCTCCTTTTAACTTGTTAAAGTCTTGCTTGAATTAAAGACTTGTTTAC GGTATCGATAAGCTTGATATCAAAACGCCAACTTTGACCCGGAACGCGGAAAACACC TGAGAAAAACACCTGGGCGAGTCTCCACGTAAACGGTCAAAGTCCCCGCGGCCCTA GACAAATATTACGCGCTATGAGTAACACAAAATTATTCAGATTTCACTTCCTCTTATTC AGTTTTCCCGCGAAAATGGCCAAATCTTACTCGGTTACGCCCAAATTTACTACAACAT CCGCCTAAAACCGCGCGAAAATTGTCACTTCCTGTGTACACCGGCGCACACCAAAAA CGTCACTTTTGCCACATCCGTCGCTTACATGTGTTCCGCCACACTTGCAACATCACA CTTCCGCCACACTACTACGTCACCCGCCCCGTTCCCACGCCCCGCGCCACGTCACA AACTCCACCCCCTCATTATCATATTGGCTTCAATCCAAAATAAGGTATATTATTGATGA TGTTT |
| 122 | HDAdHER2BiTE | AAACATCATCAATAATATACCTTATTTTGGATTGAAGCCAATATGATAATGAGGGGGT GGAGTTTGTGACGTGGCGCGGGGCGTGGGAACGGGGCGGGTGACGTAGGTTTTAG GGCGGAGTAACTTGTATGTGTTGGGAATTGTAGTTTTCTTAAAATGGGAAGTGACGT AACGTGGGAAAACGGAAGTGACGATTTGAGGAAGTTGTGGGTTTTTTGGCTTTCGTT TCTGGGCGTAGGTTCGCGTGCGGTTTTCTGGGTGTTTTTTGTGGACTTTAACCGTTA CGTCATTTTTTAGTCCTATATATACTCGCTCTGCACTTGGCCCTTTTTTACACTGTGAC TGATTGAGCTGGTGCCGTGTCGAGTGGTGTTTTTTTAATAGGTTTTCTTTTTTACTGG TAGGCGCGCCGTCGACGCGACAACTGTCTTATTACCCTGTTATCCCTACCTTAATTA AGCTAGCCGACGCGTTGGCCAGGAGCTCAGATCTCCGGATCCAAGCTTATCGATTT CGAACCCAAATGGATCTACCACATTTGTAGAGGTTTTACTTGCTTTAAAAAACCTCCC ACACCTCCCCCTGAACCTGAAACATAAAATGAATGCAATTGTTGTTGTTAACTTGTTT ATTGCAGCTTATAATGGTTACAAATAAAGCAATAGCATCACAAATTTCACAAATAAAG CAATAGCATCACAAATTTCACAAATAAAGCATTTTTTTCACTGCATTCTAGTTGTGGTT TGTCCAAACTCATCAATGTATCTTATCATGTCGAGCTAGCTAGTCAGGACTTCAGTTC CAGCTTTGTGCCGGCTCCGAAGGTCAGCGGGTTGCTAGACCATTGCTGACAGTAAT ATGTGGCGGCATCCTCGGCTTCCATAGAGCTGATTGTCAGGCTGTAGCTGGTGCCA GATCCAGATCCGCTGAATCTGTAGGGCACGCCGGAGGCCACTTTGCTTGTGTCGTA GATCCACCGCTTGGGGCTTGTGCCAGACTTCTGCTGATACCAGTTCATGTAGGACAC GCTGCTGCTGGCTCTGCATGTCATGGTCACTTTCTCGCCTGGGGAAGCGCTCATGA TGGCGGGGCTTTGTGTCAGCTGAATATCGGAGCCACCACCACCGCTTCCACCACCA CCTGAACCGCCTCCGCCACTAGAAACTGTCAGGGTTGTGCCCTGGCCCCAGTAATC CAGACAGTAGTGGTCGTCGTAGTACCGGGCACAATAGTACACAGCGCTATCTTCAG AGGTCAGGCTGGACAGCTGCATGTAGGCGGTAGAGCTGCTCTTGTCGGTGGTCAGT GTGGCCTTGTCCTTGAACTTCTGGTTGTAATTGGTGTAGCCCCGGCTGGGGTTGATG TAGCCGATCCATTCCAGTCCTTGGCCAGGCCTCTGCTTGACCCAGTGCATGGTGTA CCGGGTGAATGTGTAGCCGCTGGTCTTGCAGGACATCTTCACAGAGGCGCCAGGTC TAGCCAGTTCGGCGCCAGACTGTTGCAGCTTGATATCGGATCCACCGCCACCAAGG GCTTTGATTTCCAGCTTGGTGCCGCTGCCGAAGGTGAAAGGGGTTCTGAAGTGCTG CTGACAAAAGTACACGGCCAGATCCTCGGCCTGCACGCTGCTGATGGTAAAGGTGA AGTCAGGGCCAGAGCCAGAGCCGGTGAATCTAGAGGGCACGCCTGTGTATCTGCT GCTGGCGCTGTAGATCAGCAGCTTAGGAGACTGGCCAGGCTTCTGCTGATACCAGG CCACGGCGTTGTACACGTCCTGGCTGGCTTTACAGGTGATGGACACTCTGTCGCCC ACGCTGGTGCTCAGAAACTTGTGGCTCTGGGTCAGCTGAATATCAGATCCTCCGCC GCCTGAACCTCCGCCTCCGCTTCCTCCGCCACCAGAAGAGACTGTCACGGTTGTGC CCTGGCCCCAATATGGCACGTAGCCGTGGTACACTTCCCATCTGGCACAAAAGTAG GTGGCCATGTCCTCGGACTTCAGGTTGTTGATCTGCAGGTAGGCGGTGTTGGCGCT GGTTTCCAGGCTGAAGTCGAATCTGCCCTTGAAATCGTCGGCGAAGGTGCTCTCGC CGGTGCTGGTATTGATCCAGCCCATCCATTTCAGGCCCTGTCCAGGGGCCTGCTTG ACCCAGTTCATGCCGTAGTTGGTGAAGGGGTAGCCGCTGGCCTTGCAGGAGATCTT CACTGTCTCGCCAGGTTTCTTCAGCTCGGGGCCAGACTGCTGCAGCTGAACTTCAG AATGGGCGCCTGTGGCGGCTCCCACAAGAAACAGGATGCGCCAGATCCAGTCCATG GTGGCCCTCCTTCGCCGGTGATCTCAGCTGTAGGAAAGAGAAGAAGGTTAGTAGTC GACGTGTCCCTCTCGATGAATCTAAGTATCAATTGTGAGCGCTCACAAGTCAACACT CTTTTTGATAAATCTAGTAGATATCACTTACGTAGGCGCCGGTCACAGCTTGGATCTG TAACGGCGCAGAACAGAAAACGAAACAAAGACGTAGAGTTGAGCAAGCAGGGTCAG GCAAAGCGTGGAGAGCCGGCTGAGTCTAGGTAGGCTCCAAGGGAGCGCCGGACAA AGGCCCGGTCTCGACCTGAGCTTTAAACTTACCTAGACGGCGGACGCAGTTCAGGA GGCACCACAGGCGGGAGGCGGCAGAACGCGACTCAACCGGCGTGGATGGCGGCC TCAGGTAGGGCGGCGGGCGCGTGAAGGAGAGATGCGAGCCCCTCGAAGCTTCAGC TGTGTTCTGGCGGCAAACCCGTTGCGAAAAAGAACGTTCACGGCGACTACTGCACT TATATACGGTTCTCCCCCACCCTCGGGAAAAAGGCGGAGCCAGTACACGACATCAC |
| | | TTTCCCAGTTTACCCCGCGCCACCTTCTCTAGGCACCGGTTCAATTGCCGACCCCTC CCCCCAACTTCTCGGGGACTGTGGGCGATGTGCGCTCTGCCCACTGACGGGCACC GGAGCGATCGCAGATCCTTCGATAGAGAAATGTTCTGGCACCTGCACTTGCACTGG GGACAGCCTATTTTGCTAGTTTGTTTTGTTTCGTTTTGTTTTGATGGAGAGCGTATGT TAGTTACGATTCACACAAAAAACCAACACACAGATGTAATGAAAATAAAGATATTTTAT TGCGGCCGCTCGAGTCTAGAGGTATACATGCCATGGTCCGCGGTATTACCCTGTTAT CCCTAAGACAACTGTCCTGCATGCGACGTCGGCGCGCCATCGAATTCCTGCAGCCC GGGGGATCCCTTTGTTGATTTTTTCCACATAGATTATTTTTGACTGTTTTGGCACTTTA TATAAATGGAATCATATAGTAAATATATACATGTATATATGTATATATACACTATATATG TATATATATAGTGTATATATATACATGTATATATGTATATTTACATATATACTGTATATA TGTATATTTACATATATACTGTATATATGTATATATACACGTATATACTGTATATATACA GTATATACTGTATATATATACTGTATATATGTGTATATATATATACAGTATATATACAGT ATACATATATATACATGTATATATACACAGTATATATACATGTATATATACACAGTATAT ATACATGTATATATACACAGTATATATACATGTATATATACACAGTATATATACACTGT ATATATGTATATATATACTGTGTATATATATACAGTATATATACAGTATATATATACATG TATATGTATATATATACTGTATATATGTGTATATATACGTATATATACTGTATATATATA TAGACACTTTTGTGTCTGGCTTCTTGCTCTCATCATAAAGCACTTGAAATCCATCCAT GTTGTAGCTGGTATCAGTAGCTAGTTTCTTTTCATTGCTGAGAAGTATCACTTTTTATT GATGAGTAGTTTTACATTGTATGAACATGCCCGAGTTTGTTTATGCATTCTACTAATA GACACCTGGGCTGTTTGCAGTTTTTGGCTATTACAAAGAAAATTTCTATATTTTTTTCC AACTTTTATTTTAGGTTCAGCGGGTACATGGGCAGGTTTGTTACATCGGTAAATTGCA TGTCACTGGGGTTTGGTGTACAGATTATTTCACCACCCAGGTAATAAGCATAGTACC TGATAGGCAGGTTTTTGATCCTCACCCTCCTCCCTCAAGTAGGCCCCAGTGTCTATT GTTCTATTCCTTTTTTTTTTTTTTGAGATGGAGTCTCACTCTGTTGCCCAGGCTGGAG TGCAGTGGTGCGATCTCGGCTCACTGCAAGGTCCACCTCCAGGGTTCAAGCGATTC TACTGCCGCAGCCTCCCGAGTAGCTGGGATTACAGGCACCCACCACCACGCCCGG CTAATTTTTGTATTTTTAGTAGAGACTGGGTTTCACCATGTTGGCCAGGATGGTCTCG AACTCCTCACCTCAGGTGATCCGCCCGCTTCGGCCTCGCAAAGTACTGGGATTACA GGCGTGAGCCACGGTGCCTGGCCTATGGTTCCATTCTTTGTGTACACGTGTACTCA GTGTTTAGCTTCCACTTGTAAGTGAAAACATGCAGTATTTGGTTTTCTGAAATTCTTGT CTTCATCTTTTTGTAGACAATCCACTACTTTTATTTTTAAATATTAGGAACAATAGCCT CTGTGTGTCCTGAATTGCAATGTTTTTTCTCTGATTTTGCTGCTCATCTTGTAACCTGT ATGGTGTTTTTCTGTGTGCAAGCTTTTTACTTTACGTGGGCAATTTTATCATTCTTTTC TTTTATGGTTTCTGGGTTTCATGTCATGATTGGAGAAGCTTGATCTACCCTGATACTA TAAAAATATCCACCTTGGCTTTTTTTTTTTTTTTTAGATGGATTTTCACTCTTGTTGCCC AGGCTGGAGTGCAATGGCGTGATCTCGGCTCACCACAACCTCTGCCTCCCAGGTTC AAGTGATTCTCCTGCCTCGGCCTCCTGAGTAGCTGGGATTACAGGCATGCGCCACC ACGCTCAGCTAATTTTGTATTTTTAGTAGAGACAGGATTTCACCATGTTGGTCAGGCT GGTCTTGAACTCCCCACCTCAGGTGATCTGTCTGCCTCGGCCTCCCAAAGTGCTGG GATTACAGGCGTGACCCACCACACCTGGCCCACGTTGTTTTCTGATAGTGTTTTCAC GTGTTATTTTTTCTGTCTTACTCTTTGAATCATCTAAATGTATCTTAATGTCAGGAGTG AGGTAGAGGACCCAGTTGTTTGGTTTAAATGACTAGCCAGTTTTCCCAACACCAGGT GTTGAACATCCCTCACTGATGTGAGATGCTGCCTTTTTCACAGTCTCAATCCCCATGT GCATTTCTATTTATTTTCCTTCCACTTTATTCACATCTTTTTCAGTATCCACAACCATAT TGTTTTAAATAGCTTCATGATATGTTTTAATATTTGGTAGAGCTAGAACCCTTTCTTGG TTCTTTTTCTTTTCTTCTTCTCCTCCTTTTCCTCCTCCTTCTTTCTCCTCCTCCTTCTTC TTTCTTTTTAGAAATTTCCTGGCTATTCTTGTTTTGTAGTCTTCCAGATAAATAATTTTG AATCACTTTGTCATGTTCCAGAAAATAATACTGCCAGAATTTTTATTGTGATCACAGAA GTTTTACAGATTCATCTGGGGGAAAAAAACCCATCTTCCCAGAATACAGAAAGGGTG AGGGAAAGACAAACATCAAGGACAAGTCTCAGGTTCTGGACTTGGAGACCAAGAGG ATTTGGGGCATCCGGGAGCAGGGCAGGGAGGTGTGATGGCTGCCTTTCACTGAGTA GGAGGATGCCCAGACTTGGGGGAACTATGGAAAGTTTGGTTCAGACTTTCTGAGTT GGAGATGTCTATGCGCAGTCCACAGGGCGAAGCCCTGGAGGTAGAGGCCCTCCTTA CTGCCTCCCTCATTCAGTCTGAGGTCCATTCTCCACATGGACACTGAAGTGACTCAA GGCTTCCCTTGTTGGATCAGGATAGTCTCCAGGGCCCTGGGTGGTCTGGGCCTGCC CACCTCCCCATCCCCTCTCCTGCCTGCCCTCCTCTTGCTCACTGGACCCCACCCAC CAGCCTCCTTCTATTCCTCCTGACATTTGCACTTGCTGTTCCGTCTCTGCTTGGAACA TCCTAGCTCTTTGCATGGCTTGCTCCTTCCCATCTTTTAATCTAAGCTCAAATATCACT CCTTCAGATATGCCTTTCCCAAATATCCTAGCTAAAGAGAACTGCTCCCCACTTCCCA CAAGCCCTAGCAAACTGGGACTCTCCTTCTTGCTTTCTTTCTCTCTGCCCCTTCTGTG TTTTTTCCATAGACCTTAACTTGTTACTGTTTTATTTGTTGATGTGTTTACATCATTTGT CCCCCTGTGATGGTTAGTTTTATGTGTCAACTTGGCTAGGCTGTAGGGGCTGTTCGT CAATCAAACACTGATCTATGTGTAGCTGTGAAGGGATTTTGTAGCTGTGATTAATAGT TGACTTTAAGTAAGGAAGATTATCCTGGAGAATGTGGGGGAAGGGGCAGACCTCAG CCAATCAGTTGAAAGGCCTTAAGAACAAAGCTGAGATTTCCCCGAGGAAGAAGAAAT TCTGCCTGTGGACTGCAGCATCAGCTCCTGCTTGAGAGGTTCCAGGCTGCCCTTCC TGACAGCCTGTCCTATGATTTTGGACTTGCCTTGCCAGGTTTTTCCTCTTTCTTGACC CCCCAAAACCATATATATCTCCTGCTGGTTTGTTTCTCTGGTGTAATCCTGAGGGATG AACACTCCTCCCTCCCAGTTCACTCTGGGAGAGGTTGGGGATCATGTTTGTCTATTT CCCCATGCCTGGCCCATGCGTGTTTATTGAATCACTGAACAACCACGCATTAGGAAG |
| | | CCAGTCATATGTGCTTCTTCAGAACCTCATGTCCATTGCCAGATCTCCCTGGCTTTTG TGGCTAGAGGACAAGTGAGAGATAGTAGCTTACCCACAGACCTTGGCCCTGAGGCC CCCAGGGGCCTGAGCCTGCTGTAAGGGAGGAGGGAGCCCCTGAGGTCTCACACAC CTCCCTGGGGATCTGGCATTTTCCCCTGGGGCTGGCCTCAGAGCTGGGCGGGGGC AGATTATGGAGTGGGTTGTAGAGGGAGCACCTCCCCACTAGCAATCATGGTTTTTTC TGCGCCTCTCATAGGGAGGCCTACAGGCCTCCACCTTCAGCTCTTCAGGAGCCACA GGCAGCTGAGTGACTTCTGCATCACAGTCTTCTCAGAGACAAACTTGCAGTAAGAAA GAGGGGCCCGTGAGGGACCCCAGAGAAGGCTGTTGTCAAAGCAGGATGAGAGTGA ACTCTTCCATGGGGGACACCCAGCGTCTCCAAGCTCTTTTATGCTGTCTTCAAGGGG TCTAGAGAGCTTCAGCCCAACATGAGACCCAGTCCAGCAGCATTTCCCTGCGAAGT GAAAGTTAGGACCCTGACTAGATACACCACGCTGACCTCAGCCAGAATATCAAGATG CTGAGGCGCTGAGATGCTGGGATGCTAAGGTGCTAAGGTGCTGGGGTGCTGGGGT GCTAGGATGCTGAGGTTTTGTGATACTGGGTCGCTGAGATGCTGGGATACTGGGGT GCTGAAGTGCTGGGGTACTGGGGTGTTGCCATGCTGAAATGCTGGGGTGTTGGGAT GCTGAGGTGCTGGGTTGCTGGGATGCTGGGATAGTCCTTGGATGCTGCGGTGCTGA GATGCTGGTCTGCTGGTCTGCTGGGGTGTTGGGATGCTGGGATTTTGGGATGCTGG AGTGCTGCAGTGCTGCAGTGCTGAGATGCTGGAGTGCTGGGGTGCTGGAATACTGT AGTACTGGTGTGCTGGAGCATTGAGATGCTAGGGCACTGGGATGCTAAGGTGCTGA GATGCTGCAGTGCTGGGGTGTTGGGATGCCGAGGTGTTGGGATGCTTAAGTGCTGG GGTGTTGGGATGCTGGGGTGCTGGAACACTATGGTGCTGGGGTGCTGGAGTGTTG AGATACTGTAGTGGTGGGATGCTTAAGTGCTGGGGTGCTGGGTGTTGGGATGCCTA GGTGCTGGGGTGCCGAGATGCTGGAGTACTAGTGTGCTGGGATGCTGAAGTGCTG GGGTCCTGAGATGCTGCAGTGCTAGGGTGCTGAGATTCTGGGCTGCTGGAGTGTTG GGGTGCTGGGATGCTGGAGTGCTGAGATGCTTGGACAATGGGGTGCTGGAATACTA TGGTGCTGGGGTGCTGGGGTATTGAGATGCTAGGGTACTGGGATGCTGAAGTGCTG AGATCCTGGAGTGCTGGGCTGCTGGGCCACAGGCTCTTGAATCCATTCGTCTGCCC AGGGGAAGAAACCAGAAGATAAAGAGCTAATGAAGGAGCTTTGGTTGAGAGGGAGG AAGTAATGGAAGGAGCAACATCTTGTGGAGGAGCAGGAGAGAATGGACCTCAGGTT GGGAGAGAGGGCCAGGCTACAGGCCAGAGAGGCAGAAGGATTCCAGCAGAGTGTG GGCTCCAGGAGCCAAGGGGAAACAGGTTTCTGGGAGGAGAGAGTCCAGTACTGCT GAAGTGGCAAGTCCGCTGAGGACCAGGAAGCTTCATTTGGCTTTATGACCAGGAGG AATTTGGAACTGTGACTAGAGTACTTAGGGGGAAGGAGGCAAGACTGGAGCCAGAT TGCTCTGGGTTGAGGGGTGAGTGGGAGGTGAAGCAGGGCACTGTCACTCCTTTGAA GGGTGGCAGAGAGCTGGAATTGGTGCTGGATGGGCTGTGGGGTGACAGGGTCATG TGGAAAGCCCCTGGGGGGCACCTGGAAAAGGAGAAGCTGACAGTACAGTGAGAGG ACAGCTAAGGGAAAGCGGAATGGCAGAACACGCACTGCCAGGAGGAATGAGGATA GGGTCAGGAGTGCCAGGGGCAGTGAGGCCAGCCTGGGGTCAGGTGGCAGGACGT GTCCAGGAAGCTGGTCTGCACTGCAGCCCACACTGGCTCAGCCTTGAGGTTCCCTG TGTGGTTGGGGTAGGAAGTTGAACCCTCTGGGAATGGAAGATGGAACCAGCTCTGC GAGCCAAGCTCAGCTTTTATCTATGGGTCTCTGAGGGCTGGCAGAGCTGAGTGGGG ACAACTGTGATCCGTGAGGCTCTCAGGTTGAGGTGGCCCCTCCGGGAGGGCTTCAT TTTCCCAGCGGGTAGGTTCTAAGCAGCAGTGGCTGGGCAGGTGGGTCCAACACAGA GCCAGAGAAGGGTGAATGGGCCTCCTGGCACCCCACCCCTGCTGCCCCTGAGCTC AGTGATGGAGGGGGACAGCACAGCTGAGCCCAAGTGCTTTGGTGTGGCCCTGAGG GAAAGCTGCAGCCTGCCTGGGGCCTGGCATGGATGGGACACTTGAGGCAGAGGGA CAATAGTGGGCGCTGCAGTGAGGCTGGCTCTTGGAGAGGTTTCCTGAGGAGTGCTG CCTGAGACGGGCAGGGAGAACAGAGACAAAGTTGGTGACAGGGAATGAAAGCTGA CTGAAGGACTTTACCCAGACCTATGAGGATATCTCTCTCAGCAGGAAGCAGGAGGG GACTGTGTGAGGACTGGCCAAGAGCTGGAGTGTTGGGAAAATGACTCTTTCTCCGA CCCCTCTGTCCTAGCTCTGGCCCCTGGACTGCGGAGGTCTGCTTCCACCCCCATTG GTCGATCGTTGTCCCTTGTCACAGCCATTGAGAATTTTGGCAGGGAGCATGTTCTTA GAGCATTTTTAGGCTCTGCGGGACATAACAGCTCTGCCTCAGAGCACATGCCTTTCT CAGCTCCTGAAAGCCACTGATCAAATTGGAACATTTTGTACCTTAGGGATGAGGATA TCAACTCTCCCAGCCACTTAGAGGGATAAATGTGATGATGCATTCAATTGTGACTACA TCTGATCCCAACTGTTGCTTCAGCTGCTCTCCTATAGCACATGGCGGGAGGCGTGC ATCCCAGTAGCTACCTCCCCACTTTTGGGGAGATGTGGTTCCATCCATGAAACCTGG GTACCCGCCTACCAGGTCCTGGCCTATCAGGTGGCAGGGTCTGGTCAAAGAAGGG CATGTGTGGTCTTCAGCAAGGGAGACAGGACGGTGGTGCAGAGCGTCTAGACCCTC AGGGCAAGTCTCCCCCACACCTGCTCCCGGGGCAGTTGTCTTTGTGACCTCCCATC CCCCTCTGTTTCATCCTCTATAAAATGAGGGGCTGAGCCCCAAAATAACAGGCTTCT TTGCCATGATGCAAAACTGCTGAATCTTTCTTTCTGACACACAAGGCATCGAGCAGC CTCTGAAAGAACCAAAGCCACTAGCAGGCTTCCTGACTTGGGTTTGTAGGTACTGAA TACTCCCTTGAAAAATAAAAACATAGAGGCACTTTTCTCCTGGCTGTTTATTACAGAA CGAAGAAAAAACACACTGGCTTGAAACAGACGCCAGATTTCAAATGTAGAGGTGAAA TACGAGGTGGCAATTAAAATGTGATTACAGAAAGTCTGGACACTGAGAAAAGTTTAC AGGACAGTGGGTGTGGGTTTTCTATAACAGACACTTAAATATACATGACGATAATTGC AGATAGAAACCATCAAAGACAAACCCCAAATCAACTAATAATGTTTACAGATGTTCCC CCCCAAACCACAGAGCCTTACATCAAAACAAATACTGAAAGGCTTTAAACCAGGAAC AGCTCGCCTTAACCCCACGAGGGTGCACACAAGCTGGGCTTTTTCTCTCGGTCTGA ATGGTAAAGGGAGGAGGATACTCTAGCTCCTCCAGGTGGATTGCTGAGACAGGGCT |
| | | CGGCTCACACACTGTCTCTGCGCCTCTCCCAAATCTGGAGAACTCTCCCAGCCTCCT GGTAAAGTGTCTCTGTGGGGCACTTAACGATAAAACAGCTTCTGCTGTAAAGCTCAT TAGGAAAGAGCTAGCGGAGACTGAAAGGTTCGCAAAAGAGATTAAGAATCACACAA GGCAATAGGATTTTTAGTGAACATAGAAATAAATGGCCAAGTGGTTTTCTATTTGGCA TTTGTCAACTTGCACAACAACTCTTGGTCATATCCACATTGCTCATTGCATTAAAACC ATAAGCGACTCAGCCACCTAGCTTAACAAGGTATCACTGGAGCAAACAACACGGTCT GCATATTTGTAACATTGTATAATAAACACAAAACAATGCATAGTAAACACAACTCTACT GAAACAAAAGCCGTCGCTTTATTTACAAAGTCACAAAATGAAGTATAAATACTTCTGT CATTAATGTTTAGGAAAACCATTTACAAAATTTTCAAATATGTACACGTAGCTTGAAAA ATCACCAGCTTTCCATTTTGTCACAGGTAGAGAGAGGGATAAGCATGGGCTGACAAC ACCACTCAAATTGTAACGGGAGACAACTGCGGGTATGGATCGACACCACTTCCTAGA GTGATGTCACCATGGGGGTTTCTATGGGCATCCTGCTCAGATTTAAAGTGCCCCAGC ATCCTGGGTGACTTGCCCAGAATTCTGGGCTGTGGCATTTTGAGCAGCAGCATGCT GTTCCAAAATGTCGTCGATCAGCCTCAAGTTGCACACCCAGTCTTCATCTGGGCTCA CACAGGAGCCTTTCAAGAGAGCTTCAATGAAATCTACCTCATTGCAGTCAGGTGACG AAATCAGATCATTTAGTGGGGGTTGGGGCTGGCGCAAAAAGTCGGCAGGTGGCAGC TCAGGGGGAATATCCGTTCTGTCGAACGGACCTGGGAACTGGCTGGCAGCAACGG CAGAAGCAGCAGCAGCGGTGGCAGCAGCAGCCACATAGCTTGGTGGCTCGATGCC CTGTATGGGGCTCAGGGGACTAAAGCTGGCCATACCCTGCTGGAGGAACTTGGTGG TGTTTGCTACAGGCACCGGGCCCTGTACCGGGCTCTGCCTGAGGCTCTGGCTGCCC AGCAGGCTGAAGCTGGGGTTGTTGGCCAGGGGCACTTGTGTTCCCATCGCAGCGG GCACTTGTGCCTCCCAATCAGATGGCCTCTGAAGGCAGGCCTGGCCAGAAGGTGAG TGCTGCTGAACGCTATTATCCACTTGGCTGAGGGGTGTTTTCCCCGAAACTGCTGTG GTCACAGCTGCTGCCGCTGTGACCCATGCAGCATTGTTGAACGCAGTGGGCATTCT TGGCACACTAGGCCGTCTGAGCTGGTGGGGACTCAAGGACTGGGTGCCCAGGGAG CTGGGACAGAACCCAGGCAGGGGCACTTCTGGTGGGGTGGCCTTGGGGCTCTGCA TATGCTGGCAGACAGAGTCAAGTCTGCCCAGGGGAGTCTGGCCTGAGTGTGAGAG GATGGGACACTGGGGGCTGGAGGTGAAAATTCCTTGCCGCTTCCCCAGAGTTGGTG AGATCACTCCCATGCCCTCGCAGCTCTGGTGCCTGGTGAGTGGGATCATTCCTGGA CTCAGATTGTTCTGAAGAAGCCCAGTTCTGGGTGGCATCAAGTGCTTGCTAGATGGG GGGCTTGCCTTGATCCGGCTACACTTGGAGGTGACTTGTTCTTGGACGGCTACATAC AGAAAGAGAGAAGTGGGGATGAGTTCCAAAGGCATCCTCGACTTCGGCTGTGGCCA CCGGAGGGTAGCTCCTGGCCCAACACGGACTTCTCACCTCCCGCCCTTGGCTCTCT ACTGAGCTCCCCCCTGCTCCCCAATTCCTCGCCATTCCCCTCATTTCTCTGCCCTCA GCCTGGACTGCAGTTCTTCTGGGAAGCTGCCCCAACTCCCTAGGTCTGTGCTCACC AAGAGCAGATCACACTGGACTGAAATGCCAGCTGATTTGTCTCTTCAAGAAAATTGG AAGCTCCTGGAGGTCAGGGTCCATGTCTGCTTTTACACTCAGTGCTCTGTATGCAGG CCTGGCACTGCCCACCCTTTGACAGGTGGTGCATATTTTGTAGAAGGAAGGAAGGG GCCAGGTGGGGTGGGCTGGGCTGGTGGCGGGAGCTAGCTCAGCCTCTTAGATTCT CTACCCGATGGATGTGACCTGGGACAGCAAGTGAGTGTGGTGAGTGAGTGCAGACG GTGCTTTGTTCCCCTCTTGTCTCATAGCCTAGATGGCCTCTGAGCCCAGATCTGGGG CTCAGACAACATTTGTTCAACTGAACGGTAATGGGTTTCCTTTCTGAAGGCTGAAATC TGGGAGCTGACATTCTGGACTCCCTGAGTTCTGAAGAGCCTGGGGATGGAGAGACA CGGAGCAGAAGATGGAAGGTAGAGTCCCAGGTGCCTAAGATGGGGAATACATCTCC CCTCATTGTCATGAGAGTCCACTCTAGCTGATATCTACTGTGGCCAATATCTACCGG TACTTTTTTGGGGTGGACACTGAGTCATGCAGCAGTCTTATGGTTTACCCAAGGTCA GGTAGGGGAGACAGTGCAGTCAGAGCACAAGCCCAGTGTGTCTGACCCACCCAAG AATCCATGCTCGTATCTACAAAAATGATTTTTTCTCTTGTAATGGTGCCTAGGTTCTTT TATTATCATGGCATGTGTATGTTTTTCAACTAGGTTACAATCTGGCCTTATAAGGTTAA CCTCCTGGAGGCCACCAGCCTTCCTGAAACTTGTCTGTGCTGTCCCTGCAACTGGA GTGTGCCTGATGTGGCACTCCAGCCTGGACAAGTGGGACACAGACTCCGCTGTTAT CAGGCCCAAAGATGTCTTCCATAAGACCAGAAGAGCAATGGTGTAGAGGTGTCATG GGCTACAATAAAGATGCTGACCTCCTGTCTGAGGGCAAGCAGCCTCTTCTGGCCCT CAGACAAATGCTGAGTGTTCCCAAGACTACCCTCGGCCTGGTCCAATCTCATCCCAC TGGTGCGTAAGGGTTGCTGAACTCATGACTTCTTGGCTAGCCTGCAACCTCCACGG AGTGGGAACTACATCAGGCATTTTGCTAACTGCTGTATCCTAGGCCAATAAATGTTG ATCACATTTATAGCTGCCATGGTAGGGTGGGGACCCCTGCTATCTATCTGTGGAGGC TCTGGGAGCCCCTGACACAAACTTTCTGAAGCAGAGCCTCCCCAACCCCTTTTCCAT TCCCTATACCTGACAGATGGCCCAGGAACCCATTAGAAATGGAAGGTCACTGCAGC AGTATGTGAATGTGCGTGTGGGAGAAGGGCAGGATCAGAGCCCTGGGGGTGTGGC AGCCCCCAAGTGATTCTAATCCAGATCCTAGGGTTGTTTCCCTGTCCCATTGAAATA GCTGCTTTAAGGGGCCTGACTCAGGGAAATCAGTCTCTTGAATTAAGTGGTGATTTT GGAGTCATTTAGACCAGGCCTTCAATTGGGATCCACTAGTTCTAGAGCGGCCGGGC CCAGGGAACCCCGCAGGCGGGGGCGGCCAGTTTCCCGGGTTCGGCTTTACGTCAC GCGAGGGCGGCAGGGAGGACGGAATGGCGGGGTTTGGGGTGGGTCCCTCCTCGG GGGAGCCCTGGGAAAAGAGGACTGCGTGTGGGAAGAGAAGGTGGAAATGGCGTTT TGGTTGACATGTGCCGCCTGCGAGCGTGCTGCGGGGAGGGGCCGAGGGCAGATTC GGGAATGATGGCGCGGGGTGGGGGCGTGGGGGCTTTCTCGGGAGAGGCCCTTCC CTGGAAGTTTGGGGTGCGATGGTGAGGTTCTCGGGGCACCTCTGGAGGGGCCTCG GCACGGAAAGCGACCACCTGGGAGGGCGTGTGGGGACCAGGTTTTGCCTTTAGTTT |
| | | TGCACACACTGTAGTTCATCTTTATGGAGATGCTCATGGCCTCATTGAAGCCCCACT ACAGCTCTGGTAGCGGTAACCATGCGTATTTGACACACGAAGGAACTAGGGAAAAG GCATTAGGTCATTTCAAGCCGAAATTCACATGTGCTAGAATCCAGATTCCATGCTGA CCGATGCCCCAGGATATAGAAAATGAGAATCTGGTCCTTACCTTCAAGAACATTCTTA ACCGTAATCAGCCTCTGGTATCTTAGCTCCACCCTCACTGGTTTTTTCTTGTTTGTTG AACCGGCCAAGCTGCTGGCCTCCCTCCTCAACCGTTCTGATCATGCTTGCTAAAATA GTCAAAACCCCGGCCAGTTAAATATGCTTTAGCCTGCTTTATTATGATTATTTTTGTTG TTTTGGCAATGACCTGGTTACCTGTTGTTTCTCCCACTAAAACTTTTTAAGGGCAGGA ATCACCGCCGTAACTCTAGCACTTAGCACAGTACTTGGCTTGTAAGAGGTCCTCGAT GATGGTTTGTTGAATGAATACATTAAATAATTAACCACTTGAACCCTAAGAAAGAAGC GATTCTATTTCATATTAGGCATTGTAATGACTTAAGGTAAAGAGCAGTGCTATTAACG GAGTCTAACTGGGAATCCAGCTTGTTTGGGCTATTTACTAGTTGTGTGGCTGTGGGC AACTTACTTCACCTCTCTGGGCTTAAGTCATTTTATGTATATCTGAGGTGCTGGCTAC CTCTTGGAGTTATTGAGAGGATTATAAGACAGTCTATGTGAATCAGCAACCCTTGCAT GGCCCCTGGCGGGGAACAGTAATAATAGCCATCATCATGTTTACTTACATAGTCCTA ATTAGTCTTCAAAACAGCCCTGTAGCAATGGTATGATTATTACCATTTTACAGATGAG GAACCTTTGAAGCCTCAGAGAGGCTAACAGACATACCCTAGGTCATACAGTTATTAA GAGAAGGAGCTCTGTCTCGAACCTAGCTCTCTCTCTCTCGAGTAATACCAGTTAAAA AATAGGCTACAAATAGGTACTCAAAAAAATGGTAGTGGCTGTTGTTTTTATTCAGTTG CTGAGGAAAAAATGTTGATTTTTCATCTCTAAACATCAACTTACTTAATTCTGCCAATT TCTTTTTTTTGAGACAGGGTCTCACTCTGTCACCTAGGATGGAGTGCAGTGGCACAA TCACTGCTCACTGCAGCCTCGACTTCCCGGGCTCGGGTGATTCTCCCCAGGCTCAG GGGATTCTCCCACTTCAGCCTCCCAAGTAGCTGGGACTACAGGTGCGCACCACCAT CCCTGGCTAATATTTGTACTTTATTTTATTTATTTATTTATTTATTTTTTGAGATGGAGT TTCGCTCTTGTTGCCCGGGCTGGAGTACAGTGGCATGATCTCGGCTCAGTGCAACC TCTGCCTCCCGGGTTCAAGCGATTCTCCTACCTCATCCCCCTGAGTAGCTGGGATTA CAGGCGCCTGCCACCATGCCTGGCTAATTTTTTGTATTTTTAATAGAGACGAGGTTTC ACCATGTTGGCCAGGCTACTCTCGAACTCCTGATCTCAGGTGATCCACCCGCCTTG GCCTCCCAAAGTGCTGGGATTACAGGCGTGAGCCACTGCGCCCGGCCTAATATTTG TATTTTTTGTAGAGATGGTGTTTTGCCATGTTGTCCAGGCTGGTCTTGAACTCCTGAG CTCAAGCGATCTGCCCGCCTCTGCTTCCCAAAGTGCTGGGATTACAGGCATGAGCC ACCGTGCCTGGCCTAGGTAGACGCTTTTAGCTTTGGGGTGTGATGCCTGCCCCAGT ATATAGTGAATTTAATTATTGCTAGAGCTGGCTGTTTGTTAGTTTTCTTTGAACATAAG ATACTCATTGTTTTTAGTTTGCAAATCCCTCTTCCTTTTTAAAAAATTTCTTTCCCTTAA ATTGTTTGCATGTTAGCAATAACAAATGCTTAAATGGTGCTATGTGCTAGATACTCTT CTAAGCCCTGTTATGTATATTAACTAATTTTTTAAATTACACAAATCAGAGAGGTTAAG TAACTTGCCCAAGATTACCCAACAATACTAGGATTTGAACCTAAGTTTGTCTCACCCC AGATTCTGCTCTTAATCTCTAAACTTTTAAGTTAGTAGTGACAATAGTAGGTATTTATT GAATACTTAACTATGTTTTAGGCGTTGAAGTAAATATTTTGCAGGCATTATCTAATGTA AACACCCTAAAGTTACATAACAGGTACCCTTTAGGTAAATAAACACTAGTATGACCTT GGAGGCACAGATAGTTGAAGTAACTTGCCCAATATCACTTACATGAAATTGGCCCTC AAATGTGTCTGATACAACCCATGCTGCTTGTAACTATCGTTTTAAACTGCCAGGGTAA ACTTGGACACACTTGAGCTAAGAAAAAGCTTTTAGATTTTTGCAAATTAATGTGAAAG ATATGCTTTATGTGGATATAATATCTTCTAAATTTCGGGGATGGTAGTCCTAGAAATG TAATCCTGCCCTAGCCGAGCTTACCCTGCCAATAATTTTTTACAGAATTGGTAAAACG GAGCACCTTTTTTTTGTCCTTGGCCACACTGTTATCAACAGGGTGTAGATTGACATCA ATCTGTAGGTGTAAACCAGAATTACTCTTTGTGACCACCAGGAAATAGAGCAGTTCA GTTCAGGGGTTTCTTTCTGTGAATTTAGCACTGTGACCTGCATACTACAAGTCTACTT TGTTTTCTATCCATTGTTTGTATCTGGGTATTGCAAAAGGTAGGAAAAGGACCAACCA GATCAGCAGAGAAGAGTTGCCTTGGAGTTTTCTTTTAGTTTTCTGCAGTTCATTAGAT AGTAACTAGGCCATGTCATTTTACTCCCTTGTAGTGAAGATATGTTGAAGTTGTACTG GTATACTCTTCTACCTTTCTGTAATTTTATATTGTGTAGACTTGATAAAATTTATGTGTC AATCACCACCATTAATATCAATATTGAGCCTCAATTCTTATTTTTCTGCCCAGTGGCT GCCAAATTACTAACATTTACAATAATTCACTACTACTAAGATAATCTACTAGTTCGATC ACATACTTCAAATTGTTATGGAACTACTGTCTTCAGCATTGTGCTTCTGATAACTGATA AGTATAATTTTTTTTTTGTCCAGAGTGAACATGTCTATTCTTCCACTGTACACACTAAT AAAAGGAAAAATTGTAATATTGGGTAAATTCATGTCCTTACACATGTAGTAGTTATGA GCCCATGTCCCTAGAATGAGTAATAATTTATCCCTCCCTTGGTTGAATAGTCAAGAAT GCTGATTTTAATTCTTCTAACAGCTTTATCCCTCAGAAGGGAAGGCAAGCAAGTTATA TATGTAGTTTATTTGTAAGACTGATATGAAATTGGAAGATGAATCTACTATTAGCTTTA ATTATTTTTACATTTAGGAATATTGCATCAGTAACTCATAATTTTGGTTTTCTGTTATCC TGAGTTAACACAAATTATCCAAGGAGATGGCGGATCATCTGCTTTGAGGTGTTTTTTT TTGAGAATTTTAATGTATCTGAATATAAAAGGTAAAAATATGCCAACTAGCAATTTCTG CCCATTCCAGAAGTTTGGAAATATTACTCATTACTAGGAATTAAATAAAATATGGTTTA TCTATTGTTATACCTCTTTTAATTCACATAGCTCATTTTTATCTTTTATTTTTGTTTGTTT TTTTTGAGATGGAGTCTTGCTCTGTCACCAGGCAGGAGTGCAGTGATGCAAATCTCG GCTCACTCTAGCCACCGACTCCCTGGTTCAAGCGATTCTCCTGCCTGAGCCTTCTGA GTAGCTGGGATTACAGGCAGGCACCACCACGCCCAGCTAATTTTTGTAGAGACAGG ATTTCACCGTGTTGGCCAGGATGGTCTCCATCTCCTGACCTCATGATCTGCCTGCTT CGGCCTCCCAAAGTGCTGGGATTACAGGTGGGAGCCACTACGCCTGGCCCACATAG |
| | | CTCATTTTTAGACTCACTTCCATTAAGTCTTGTTTGGACCCACGAACATTGTCTTTTTT TTTTTAAGATGGAGTTTCACTTTTGTTGCCCAGACTGTAGTGCAATGGTGCAATCTCA GCTCACTGCAATCTCTGCCTCCTGGGTTCTAGCAATTCTCCTGCCTCAGCCTCCCGA GTAGCTGGAATTACAGGCGCCCGCCACCACGCCCAGCTAATTTTTGTGTTTTTAGTA GAGACGGGGTTTCACCATGTTGGGCAGGCCAGGGGTGATCCGCCCACCTCAGCCT CCCAAAGTGCTGGGATTACAGGTGTGAGCCACCGCATCTGGCCAACATGTCTTTTTT TTTTTTTTCCTTTTTAACCACAAAGAGACTTAAGCAGTCCTTGTCACAGATGATGAATT GATGTTGCAAGTATTGTCTTAGCTTGGATTAATTTTCTTGCTTACTGTAATTTTAGATA ATATAGCTTTGTAATTAGAGATTTTATGTGTAAACCACAAAAATGTTTACATGAAGGCC ATTATTACAGATGTGACGTGCATAATTATTAGTAATTTGTATGTTTACATGGGTCAGTC TGGCAAAAAATTATGAAGTTTTAAAAATTAAAAAAAATTATAATGCCAGTTTTACTGGA AAGTAAAATTATTTCAGTAATCGATTATAGCAAAAGTATTGATTTTCATTCCAGACAAA AGTCAGAATGAAAGGTAATTTCTCAATACTCTTTCAGATTAATAAAAGTACCTGTAGC GATTTTTATCATTCACAAGTATATCACAAGTAAGTTAGAATTTGAGAACTGTGTTCTAG ATCTCTGAGGAGATGCAGTCAGATTTCTGAACTGTCTCAGCAAATGGTAAGTAACTTA GAGCTAGTAATTAATAACCTGTCCTTTGATTTCTGATTCAGCCAAGAATGGCCATATT TGGGAAAGGCAGATCTGGAGAGTAACCACGTTTTCATTCATTTACCACTTCTAGGCC CCTCCAGAGCTCTCAGATATTTTGGGGTTGAGCCCTTCCCCAAAGCCATACAGGACC TTTTTTTTGTGATCTGTTCTAGCCATTTTTATGTTGGGTGCTTGTTATGGACTGAGCAT TTATGTCCTCCCACACCCCCCCCATACCTTTTTTGAAGTCCTAACCCCCAGTGTGAT GGTATTTGGAGACAGGGCCTTTGGAAGGTAATTACAGTTAGAAGAAGTCGGGAGGG TTGGGCCCAGGTCTGATTGGATTAGTGCCCTTATATGAAAAGACACCAGGACGGGC GCAGTGGCTCACACCTGTAATCCCAGCACTTTGGGAGGCCAAGGTGGGTGGATCAC GAGGTCAGGAGTTTGAGACCAGCCTGGCCAATGTAGTGAAACACCATCTCTACTAAA AATACAAAAATTAGCTGGGTGTGGTAGCGGGCTCCTGTCATCCAAGCTACTCGGGA GGGTGAGGCATGAGAATCACTTGAACCCGGGAGTTGGAGGTTGCAGTGAGCCCAG ATTGTGCCACTGTACTCCAGCCTGGGTGACAGAGTGAGACTCTGTCTCAAAAAAGAA AAAAAAAAAAAAAGAGACACCAGAGAGCTTGTTAGAAGAGGTCATGTGAGCACACAG TTAGAAGACCTTCAAGCCAAAGAAGAGGCCTGAGATTGAAACCTACCTTGCAGGTAC CTTAATTTTGGACTTCCCAGCCTCCAAAACTGTGAGAAATAAGTTTCTGTTAAGTCAC TCAGTCTGTGGTATTTTGTTATGGCAGCCTGAGCAGGTAGTTGTTCTTTCAGAAGGT GTTGATAATAACCACATGCAACACCAAGTCACAAATAATAAAACAGATGTAACTTATA TTCATACAGAAAGTTGGGCACTGCCATTGCCTTGTTGGTTTACACGGCTGTGCTAGT TCAGTAGCAGAAAGGTGCTGGTCTCCTTTACTCAGTTTACAATCTAGGCAGTAGAAT GTAATCACTGCTTTAAACTTGATACTGCTTAGGGAGAGAATCATTGGTGCTGGGTAA CTTTGGGTTCTAGGTTTACTTTTTGTGTATATATAACTGTTTTTGGTAAATCACAAGTT TCTGGGCTTGTCGAATTAGATTTTGTTACAGATTATGAGCTTTATTATGCTATACAGTT AGTTGTATGTATATATGCCTTTCCCACTAGATTTTAAGCTTTTTTTTTTTTTTTTTTTTT GTGACGGAGTCTTGCTCTTGTCGCCCAGGCTGAAGTGGAGTGCAGTGGCACAATCT CGGCTCACTGCAGCCTCCACCTCCTAGGTTCAAGCGATTCTCCTGCCTCGGCCTCC CAAGTAACTGGGACTACAGGCACGTGCCACCACACCCGGCTAATTTTTGTATTTTTT GTAGAGACAGGGTTTCGCCATGTTGGCTAGGCTGGTCTTGAACTTCTGGCCTCAGG TGATCCACCCGCCTCAGCCTCCCAAAGTGCTGGGATTTACAGGCATGAGCCACCAC GCCCAGCTATAGCTCTTTAAGGGTTGTAAATTTATAATCATTCTTTTACTCTCCTGCAA ATTCTGTTGCACACTGCCTTAATCAAGGTAGATGCTGAATGCATTTTTGTATAATTGA ATATGTTGCAATCCCCAACTCTCTCCAACTGTTCCTGTCAAAGCAGCCACTGGATTGT TAACTAATCCATATTAGATGGGGTTAATTAATATCAGATGGGACAAGTAAGGGCTAAT AAGATTATAGGCCACCAAGTAGATTTCTGTCTAGCTCTTATAGAGATTGAGTTTATTG GACCTGTTTGATAGGAAGTTTTGGTGTTTGGGATGATTAAAACTGAAGTTCCTATTTA TTGAATTATACCTATTTATATTATTTCATATCAGTGGTCCACATGCAAGTGAGGCTTCT GAGACAGAGTTTGAGTTCTCTCTTCAACTACCATAACACTTAACCTGTATCTTTTTTTT TTTTTTTTTTTTTAGACAGGAGTCTCGCTCTGTCACTCAGGCTGGAGTGTAGTGGTAT GATCTCGGCTCACTGTAACCTCTGCCTCCTGGATTCAAGCAGTTCTCCATGTCTCAG CCTCCCTAGTAGCTGGGATTACAGGCCTGTGCCACCATGCCTGGCTAATTTTTTTTTT GTATTTTTAGTAGAGACGGGGTTTTACCACGTTGGCCAGGCTGGTCTCGAACTCTTG ACCTCGAGCGATCAACTTGCCTTGGCCTCCCAAAGTGCTGGGATTACAGGCATGAG CCACAGCGCCCAGCCGTCTTTTTTTTTAAATAGCAATTTAACACTGTTCACAGTTACT CATGTACATGTCATGCCATCTATTACACTGTAAGTTCTGTGAGGGTAGCTGTATCAAA TTTATCTAACTCTCTCTAGTATGCATGACATAGTAAGTATTCAATAAATATTTGCATAT TAGTGATAAGGATACAGGTTCTGAATAGTGGGTCCTTACCATTTAAGAATTAGTATTT GATGGCCGGGCGGGGTGGCTCACGCCTGTAATCCCAGCACTTTGGGAGGCTGAGG CGGGCGGATCATGAGATCAGGAGATCGAGACCATCCTGGCTAACATGGTGAAATCC CGTCTTTACAAAAAAAATACAAAAGAATTAACCAAGTGTGGTGGTGGGTGCCTGTAG TCCCAGCTACTGCTTTGTGAGGCTGAGGCAGGCAGATCACCTGAGGTGGGAAATTC AAGACCAGCCTGACCAACATGGAGAAACCCCATCTCTACTAAAAATACAAAATTAGC CGGGCGTGGTGGCGCATGTCTGTAATCCCAGCTACTCGGGAGGCTGAGGCAGGAG AATGGCGTGAACCCGGGAGGCGGAGCTTGCAGTGAGCCAGGATCGCGCCACTGCA CTCCAGCCTGGGCGACAGAGCGAGACTCCGTCTCAAAAAAAAAAAAAAAAAAAAAAT TAGTATTTGATATTTGATCATTAAATATGAATTAAGAGGACTTAGACTTTTTGTTAAAT GTCAAGCTGGGAAAAGTTGTCATTTAAATGAATTGCCTCTTATTTAATTTCGTCTGAT |
| | | GATACATTTTGTTTTTATTTTGTAAAAAATTATTTTTTTTCTTTTTGGAGACAGGGTCTT GCTCTGTTGCCCAGGCTGGTCACAAACTCCTGACCTCAAGCAATCCTCCTGCCTTAG CCTCCCAAAATGCTGGGATTACAGGCGTGACGACCTCGCCCGGCCTTGTATTATGAT ACATTTTGAACAACTACAAGTAGACTTGGTATAATGAACCTGCACGTACCCATTGCCA AGTTCTGACAACTGTCTGTCTATAGCCAATTATGCATTTCTTAAATTAGAACCCCCCC AATATACCCAAATATATATATATGTGTGCATATATATAGTAAGTTGTAACAAAGTTGTG AATTCATACCTGAAGTATCTCAAGTGATGCAAGTTTTATGAATTTTTGTTTATGCCTTT TGGGAAGAGTTGTATTGACAAATTTTTTATGCTTAAAGTAAACCATAAATCAAAAAAAT AAAATCTAGGATGCAATAAAACAAAACAACTTCTTGACATAAGTATGGTATGTAAATC TGTTTTGATTGGAAATCAATTTGTTATATTGCCAGAATTCCTGTTTTAGAATACATCTC TGCTGATCTGTCTGTATTCTTAGACTGCATATCTGGGATGAACTCTGGGCAGAATTCA CATGGGCTTCCTTTGAAATAAACAAGACTTTTCAAATTCTTAGTCGATCTGCAGAACC TGTAGCCAGGCACTGAACCATTTTGATAGATGCAGTAATCGTTGCAAGTGTATATTTC AAGGGAGTTCTGGCTGGGTCCTAGTTTATGCTTGTGGCAGAAGCAGTGAGTAACTG GGAGGAAGTTGGTGAGTAAGCTTCAAGGAAGAAGTCATTTTTAGTACTCTGGATCTT CCTGATTTTAAAGCACTACAAAATGGTGCATTTTCATTCTTGTCAAGTGATAACAGAT ATATTCTGATGAGCCTGAAATGAATATATATTGTATCATTTTTATAATATCTAGCAAGG TTTGTATTTTCCTAGAACTTGAACTAAATTTCAGTTCATAAAATTTATAAAATACTTAGT TGTTGTAAAATATTTTTGGAATGTTCACATAGGTGACACACAAATGTCCCATTTTCATT CTTTCTATAGTAAATATGTTCTGATATGTGAAGGTTTAGCAGATGCATCAGCATTTAAT CCTAGAGGATCTGGCATAATCTTTTCCCCCAAGAATAGAAATTTTTTCTGCTTATGAA AGTAGTACATGTTTCTTTAAAAACAAATCAATATTGACTTCTGCCTGCTGTATAGCACT ATGCCTCCACCTGGCCATGACCAGGGGCATGTCCTGGTCCACCTACCTGAAAATGT TTGCAACCAGCCTCCTGGCCATGTGCACAGGGGCTGAAGTTGTCCCACAGGTATTA CGGGCCAACCTGACAATACATGAAGTTCCACCAAAGTCTGAGAACTCAGAACTGAGC TTTGGGGACTGAAAGACAGCACAAACCTCAAATTTCTCAGCACTGGAAACCTCAAAA TATAACTGAATTCCATAAATAAGATTTTAAGTCTTAAATATGTATTTTTAAATGTATTAA AAGTCAAGCTGCTTGTATTTAAGCACCTAATACAATGCTTAGGTTGTAAAAGGAGATG CTCAATAGGTACTAACTGATATATTGAGATTTAATTATGGTTTGACCAATATTTATTGG AAACCGCCAAAGCTTAAATCATCAGCTTCTTGAATGTGATTTGAAAGGTAATTTAGTA TTGAATAGCATGTGAGCTAGAGTATTTCATTCTTTCTGGTTTATTTCTTCAAATAGACT TTGAATATAATGGTGAATGGGTATTATAAATTAACTAATAAAAATGACATTGAAAATGA AAAAATATATATATTAAAGTGTAGAAAGTGACCAGGCGTGGTGGCTCACACCTGTAAT CCAAGCACCTTGGGAGGCTGAGGCAGGAGGATCTCTTGATCCCAGGAGTTCAAGAC CAGCCTGGGCAACATAGCGAGACTTCGTCTCTAAAAAAAAAAAAGAGAGAGAAAAAA ATTTTTTTTATTTAAAAAAAGTGTAGAAAGTGTCAAGACCCCACTTCTTACCATTATTT GGTATATTTCTCTATACCCACCCACCCTTCCTCCTTACTCCCTCCCTCCCTTCCCAAT CTTTTTATCTTTTTGTATTCTGATTTTTTGTTTGTATATTTTGCTTTAATTTAATGTATCC TTTAAAAATTTCCCATACATTTTATATGTATATATAAAAACGCATGCTGCCAAAGATAA TTTATAAGAAAGACCATTGAATTTTTTTAAAAGTGATATATATTCATTGAAAAAAATTTA GAATATATAGCAAAGCAATAAAGAACTAAATAAAATTGCTGTAACTCCTCTTTCAAAG ATAAGTGCTTTTATGATTTTGTTGTATTTTTTTCTGTATATAGGTACATATATAGTATTT ATAAAGCTGTACTCATAGTACATTTTCACATCACAGGTACCATATCAGTGTTATTAAAT ATTTTGTATGCCAGGGGCTAGACATACCAAGACAACCAATATGTGGTTCTACTTAAAT AATATTAGAGTATCTTTTATGATGACACTTCATGAGTTGACTATAATAATCTTAGACTT CTAAGAGTTTGGGTTTTCAAAAGATCACTTAGCTTTTTTGGGTGATTTTTCCCCCTTA CTGTGAGATGAGAGAGGCTGTTTGGATTTGGGATTGGGGTAGCGGGGACAGCAACT TTTCTTTTCTTTTTCTTTTTTATTTTGAGGTAGGGTATTGCTGTGTCACCCAGGCTGGA GTGCAGTGGTGTGATCTCGGCTCACTGCAACCTCCACCTCCCGGGCTCAGGTGATC CTCCTGCTTCAGCCTCCCAGTAACTGGGACTACAGGCGCGTGCCACATGCCTGGCT AATTTTGTATTTTTAGTAGAGATGGGGTTTCACCATGTTGGCCAGGCTGGTCTCTAAC TCCTGACCTCAGGTGATACGCCCACCTGGGCCTCCCAAAATACTGGGATTACAGGC ATGAGCCGCTGCATCAGCCAGCAGTTTTTCTTGTGGTTTTTTTTGTTTGTTTTGTTTTG TTTTGTTTTTGAGATAGGGTCTTACTCTGTTGTCCACGCTGGAGTGCTGTGGTATGAT CGTAGCTCACTGCAGCCTCAAACTCCTGGGCTCAAGTGATTCCTTCTGCCTCCGCCT CCCGAGTAGCTGGGACTACAGGTATGCACCACCATACCTGGCAAATTTTTACAAAGT TTTTTGTAGGGACGGGGTCTTGCTACATTCCCCATGTCGGTCTTGAACTCCTGGCCT CAAGCAACTCTCCTGTCTCAGCCTCCCAAAGCACTGGGATTACAAGTGTGAGCCACC ACACCATGCCAGTTTTTCCTGTTCAGTGTGATATTTTATCTTGTTAGACTACAGTGTG TTAAAACTTGTTTTACTAAATTTTCAAACATACTCAAAAGTGGAGAGAATAGTATAATG AATACCCGTATGTTCATCACCCATGTTTAGAATATTATTAAATATAAAGATTTTGCTGC GTTTGTCTTAGCTCTTTAAAATTTTTCTTTTTCTCTTTGTGACCTAAAGGAAATTCCATA TCTTATCACTTTACTTCTACATTCTTGACTAAGATGACTAAGACATATAGTTACATGGT TTTTTGTTTTGTTTTTGTTTTTTAAAGACGAAATCTCGCTCTTGTCCCCCAGGCTGGA GTGCAATGGTGCCATCTCAGCTCAGTGCAACCTCTGCCTTCTGGGTACAAGCGATTC TCCTGCCTCAGCCTCCCAAGTAGCTGGGATTACAGGCTCCTGCCACCACGCCTGGC TAATTTTTGTATTTTTAGTAGAGACGGCGGGGGGAGGTTTCACCATGTTGACAAGGC TGGTCTGGAACTCCTGACCTCAGGTGATCCACCCGCCTCGGCCTCCCAAAGTGCTG GGATTACAGGCGTGAGCCACCGCGCCCAGCCTGTTTTTTTGTTTGTGTGTTTTGTTT TTTTTGAGACAGAGTCTTGCTCTGTTTCCCAGGCTGGAGTGAAGTGGTGCCATCTCA |
| | | GCTCAGAGACAGAGTCTTGCTCTGTTTCCCAGGCTGGAGTGAAGTGGTGCCATCTT GGCTCACTGCAACCTTCACCTCCCAGGTTCAAGTGATTCTCCTGCCTCAGCCTCCCA AGTAGCTGGGACTACAGGCATGTGTCACCACACCCGGCTAATTTTTTTGTATTTTTAG TAGAGACGGGATTTCACCGTGTTGCCCAGGCTGGTCTCGAACTCCTGAGCTCAGGC AGTCTGCCTGCCTCAGCCTCCCAAAGTGCTGGGATTACACGTGTGAACCAACCCGC CCGGCCTGTTGTTTTCTTACATAATTCATTATCATACCTACAAAGTTAACAGTTACTAA TATCATCTTACACCTAAATTTCTCTGATAGACTAAGGTTATTTTTTAACATCTTAATCCA ATCAAATGTTTGTATCCTGTAATGCTCTCATTGAAACAGCTATATTTCTTTTTCAGATT AGTGATGATGAACCAGGTTATGACCTTGATTTATTTTGCATACCTAATCATTATGCTG AGGATTTGGAAAGGGTGTTTATTCCTCATGGACTAATTATGGACAGGTAAGTAAGAT CTTAAAATGAGGTTTTTTACTTTTTCTTGTGTTAATTTCAAACATCAGCAGCTGTTCTG AGTACTTGCTATTTGAACATAAACTAGGCCAACTTATTAAATAACTGATGCTTTCTAAA ATCTTCTTTATTAAAAATAAAAGAGGAGGGCCTTACTAATTACTTAGTATCAGTTGTG GTATAGTGGGACTCTGTAGGGACCAGAACAAAGTAAACATTGAAGGGAGATGGAAG AAGGAACTCTAGCCAGAGTCTTGCATTTCTCAGTCCTAAACAGGGTAATGGACTGGG GCTGAATCACATGAAGGCAAGGTCAGATTTTTATTATTATGCACATCTAGCTTGAAAA TTTTCTGTTAAGTCAATTACAGTGAAAAACCTTACCTGGTATTGAATGCTTGCATTGTA TGTCTGGCTATTCTGTGTTTTTATTTTAAAATTATAATATCAAAATATTTGTGTTATAAA ATATTCTAACTATGGAGGCCATAAACAAGAAGACTAAAGTTCTCTCCTTTCAGCCTTC TGTACACATTTCTTCTCAAGCACTGGCCTATGCATGTATACTATATGCAAAAGTACAT ATATACATTTATATTTTAACGTATGAGTATAGTTTTAAATGTTATTGGACACTTTTAATA TTAGTGTGTCTAGAGCTATCTAATATATTTTAAAGGTTGCATAGCATTCTGTCTTATGG AGATACCATAACTGATTTAACCAGTCCACTATTGATAGACACTATTTTGTTCTTACCGA CTGTACTAGAAGAAACATTCTTTTACATGTTTGGTACTTGTTCAGCTTTATTCAAGTGG AATTTCTGGGTCAAGGGGAAAGAGTTTATTGAATATTTTGGTATTGCCAAATTTTCCT CTAAGAAGTTGAATCATTTTATACTCCTGATGTTATATGAGAGTACCTTTCTCTTCACA ATTTGTCTCTTTTTTTTTTTTTTTTGAGACAAGGTCTCTGTTGCCCAGGCTGGGGTGC AGTGCAGCAGAATGATCACAGTTCACTGCAGTCTCAACCTCCTGGGTTCAAGCGATC CTTCCACCTCAGCCTCCTGAGTAGCTGGGACTATAGGTGTGCGCCACCACTCCCAG CTAATATTTTTATTTTGTAGAAACAGGGTTCGCCATGTTACCCAGCCTCCCAAAGTGC TGGGATTACAGGCATGAGCCACTGGCCCAGTTTCTACAGTCTCTCTTAATATTGTATA TTATCCAGAAAATTTCATTTAATCAGAACCTGCCAGTCTGATAGGTGAAAATGGTATC TTGTTTTTATTTGCATTTAAAAAAAATTATGATAGTGGTATGCTTGGTTTTTTTGAAGG TATCAAATTTTTTACCTTATGAAACATGAGGGCAAAGGATGTGATACGTGGAAGATTT AAAAAAAATTTTTAATGCATTTTTTTGAGACAAGGTCTTGCTCTATTGTCCAGGCTGG AGTGCAGTGGCACAATCACAGTTCACTCCAGCCTCAACATCCTGCACTAAAGTGATT TTCCCACCTCACCTCTCAAGTAGCTGGGACTACAGGTACATGCTACCATGCCTGGCT AATTTTTTTTTTTTTGCAGGCATGGGGTCTCACTATATTGCCCAGGTTGGTGTGGAAG TTTAATGACTAAGAGGTGTTTGTTATAAAGTTTAATGTATGAAACTTTCTATTAAATTC CTGATTTTATTTCTGTAGGACTGAACGTCTTGCTCGAGATGTGATGAAGGAGATGGG AGGCCATCACATTGTAGCCCTCTGTGTGCTCAAGGGGGGCTATAAATTCTTTGCTGA CCTGCTGGATTACATCAAAGCACTGAATAGAAATAGTGATAGATCCATTCCTATGACT GTAGATTTTATCAGACTGAAGAGCTATTGTGTGAGTATATTTAATATATGATTCTTTTT AGTGGCAACAGTAGGTTTTCTTATATTTTCTTTGAATCTCTGCAAACCATACTTGCTTT CATTTCACTTGGTTACAGTGAGATTTTTCTAACATATTCACTAGTACTTTACATCAAAG CCAATACTGTTTTTTTAAAACTAGTCACCTTGGAGGATATATACTTATTTTACAGGTGT GTGTGGTTTTTTAAATAAACTCCTTTTAGGAATTGCTGTTGGGACTTGGGATACTTTTT TCACTATACATACTGGTGACAGATACCCTCTCTTGAGCTACATCGGTTTGTGGGGAG TCAAAAGTCCTTTGGAGCTAGGTTTGACAAATAAGGTGGGTTAACACTTGTTTCCTAG AAAGCACATGGAGAGCTAGAGTATTGGCGAATTGAAGAAATCCCCCTTTTTTTTTAAC ACACTTAAGAAAGGGGACTGCAGGTATACTCAAGAGAGTAAGTCGCACCAGAAACC ACTTTTGATCCACAGTCTGCCTGTGTCACACAATTGAAATGCATCACAACATTGACAC TGTGGATGAAACAAAATCAGTGTGAATTTTAGTAGTGAATTTCATTCATAATTTGATCG TGCAAACGTTTGATTTTTATTACTTTAGACTATTGTTTCTGATTTTATGTTGGGTTGGT ATTTCCTGTGAGTTACTGTTTTACCTTTAAAATAGGAATTTTTCATACTCTTCAAAGAT TAGAACAAATGTCCAGTTTTTGCTGTTTCATGAATGAGTCCTGTCCATCTTTGTAGAA ACTCGCCTTATGTTCACATTTTTATTGAGAATAAGACCACTTATCTACATTTAACTATC AACCTCATCCTCTCCATTAATCATCTATTTTAGTGACCCAAGTTTTTGACCTTTTCCAT GTTTACATCAATCCTGTAGGTGATTGGGCAGCCATTTAAGTATTATTATAGACATTTT CACTATCCCATTAAAACCCTTTATGCCCATACATCATAACACTACTTCCTACCCATAA GCTCCTTTTAACTTGTTAAAGTCTTGCTTGAATTAAAGACTTGTTTACGGTATCGATAA GCTTGATATCAAAACGCCAACTTTGACCCGGAACGCGGAAAACACCTGAGAAAAACA CCTGGGCGAGTCTCCACGTAAACGGTCAAAGTCCCCGCGGCCCTAGACAAATATTA CGCGCTATGAGTAACACAAAATTATTCAGATTTCACTTCCTCTTATTCAGTTTTCCCG CGAAAATGGCCAAATCTTACTCGGTTACGCCCAAATTTACTACAACATCCGCCTAAAA CCGCGCGAAAATTGTCACTTCCTGTGTACACCGGCGCACACCAAAAACGTCACTTTT GCCACATCCGTCGCTTACATGTGTTCCGCCACACTTGCAACATCACACTTCCGCCAC ACTACTACGTCACCCGCCCCGTTCCCACGCCCCGCGCCACGTCACAAACTCCACCC CCTCATTATCATATTGGCTTCAATCCAAAATAAGGTATATTATTGATGATGTTT |
| 123 | HDAdCD19BiTE | AAACATCATCAATAATATACCTTATTTTGGATTGAAGCCAATATGATAATGAGGGGGT GGAGTTTGTGACGTGGCGCGGGGCGTGGGAACGGGGCGGGTGACGTAGGTTTTAG GGCGGAGTAACTTGTATGTGTTGGGAATTGTAGTTTTCTTAAAATGGGAAGTGACGT AACGTGGGAAAACGGAAGTGACGATTTGAGGAAGTTGTGGGTTTTTTGGCTTTCGTT TCTGGGCGTAGGTTCGCGTGCGGTTTTCTGGGTGTTTTTTGTGGACTTTAACCGTTA CGTCATTTTTTAGTCCTATATATACTCGCTCTGCACTTGGCCCTTTTTTACACTGTGAC TGATTGAGCTGGTGCCGTGTCGAGTGGTGTTTTTTTAATAGGTTTTCTTTTTTACTGG TAGGCGCGCCGTCGACGCTTATTACCCTGTTATCCCTACTAGCTAGCCGACGCGTT GGGAGCTCTCCGGATCCAAGCTTATCGATTTCGAACCCAAATGGATCTACCACATTT GTAGAGGTTTTACTTGCTTTAAAAAACCTCCCACACCTCCCCCTGAACCTGAAACATA AAATGAATGCAATTGTTGTTGTTAACTTGTTTATTGCAGCTTATAATGGTTACAAATAA AGCAATAGCATCACAAATTTCACAAATAAAGCAATAGCATCACAAATTTCACAAATAA AGCATTTTTTTCACTGCATTCTAGTTGTGGTTTGTCCAAACTCATCAATGTATCTTATC ATGTCGAGCTAGCTAGTCAGGACTTCAGTTCCAGCTTTGTGCCGGCTCCGAAGGTC AGCGGGTTGCTAGACCATTGCTGACAGTAATATGTGGCGGCATCCTCGGCTTCCATA GAGCTGATTGTCAGGCTGTAGCTGGTGCCAGATCCAGATCCGCTGAATCTGTAGGG CACGCCGGAGGCCACTTTGCTTGTGTCGTAGATCCACCGCTTGGGGCTTGTGCCAG ACTTCTGCTGATACCAGTTCATGTAGGACACGCTGCTGCTGGCTCTGCATGTCATGG TCACTTTCTCGCCTGGGGAAGCGCTCATGATGGCGGGGCTTTGTGTCAGCTGAATA TCGGAGCCACCACCACCGCTTCCACCACCACCTGAACCGCCTCCGCCACTAGAAAC TGTCAGGGTTGTGCCCTGGCCCCAGTAATCCAGACAGTAGTGGTCGTCGTAGTACC GGGCACAATAGTACACAGCGCTATCTTCAGAGGTCAGGCTGGACAGCTGCATGTAG GCGGTAGAGCTGCTCTTGTCGGTGGTCAGTGTGGCCTTGTCCTTGAACTTCTGGTT GTAATTGGTGTAGCCCCGGCTGGGGTTGATGTAGCCGATCCATTCCAGTCCTTGGC CAGGCCTCTGCTTGACCCAGTGCATGGTGTACCGGGTGAATGTGTAGCCGCTGGTC TTGCAGGACATCTTCACAGAGGCGCCAGGTCTAGCCAGTTCGGCGCCAGACTGTTG CAGCTTGATATCGGATCCACCACCTCCGCCAGAAGAAACTGTCACGCTGGTGCCCT GGCCCCAATAGTCCATAGCGTAGCTGCCGCCGTAGTAGTAGTGCTTGGCGCAATAG TAGATGGCGGTGTCGTCGGTCTGCAGGCTGTTCATCTTCAGGAACACCTGGCTCTT GGAGTTGTCCTTGATGATGGTCAGCCGGGACTTCAGGGCGCTGTTGTAGTAGGTTG TCTCGCTGCCCCAGATCACTCCCAGCCATTCCAGGCCTTTCCGAGGAGGCTGTCTG ATCCAGGACACGCCATAATCAGGCAGGGACACTCCGCTGACGGTACAGGTCACGCT CAGAGATTGAGATGGGGCCACCAGGCCAGGGCCAGACTCTTGCAGTTTCACTTCAG ATCCTCCGCCACCAGAACCTCCGCCTCCGCTTCCTCCGCCGCCTGTGATTTCCAGC TTGGTGCCTCCGCCAAAGGTGTAAGGCAGGGTGTTGCCTTGCTGGCAGAAGTAGGT AGCGATATCCTCTTGTTCCAGGTTGCTGATTGTCAGGCTGTAGTCGGTGCCAGAGCC GCTGCCAGAAAATCTGCTTGGCACGCCGCTGTGCAGTCTGCTGGTGTGGTAGATCA GCAGCTTCACGGTGCCGTCGGGTTTCTGCTGATACCAGTTCAGGTACTTGCTGATGT CCTGGCTGGCTCTACAGCTGATGGTCACTCTATCGCCCAGAGAGGCAGACAGGCTG CTGGTGGTCTGGGTCATCTGAATATCGCTGTGGGCGCCTGTGGCAGCTCCCACAAG GAACAGGATGCGCCAGATCCAATCCATGGTGGCCCTCCTTCGCCGGTGATCTCAGC TGTAGGAAAGAGAAGAAGGTTAGTAGTCGACGTGTCCCTCTCGATGAATCTAAGTAT CAATTGTGAGCGCTCACAAGTCAACACTCTTTTTGATAAATCTAGTAGATATCACTTA CGTAGGCGCCGGTCACAGCTTGGATCTGTAACGGCGCAGAACAGAAAACGAAACAA AGACGTAGAGTTGAGCAAGCAGGGTCAGGCAAAGCGTGGAGAGCCGGCTGAGTCT AGGTAGGCTCCAAGGGAGCGCCGGACAAAGGCCCGGTCTCGACCTGAGCTTTAAA CTTACCTAGACGGCGGACGCAGTTCAGGAGGCACCACAGGCGGGAGGCGGCAGAA CGCGACTCAACCGGCGTGGATGGCGGCCTCAGGTAGGGCGGCGGGCGCGTGAAG GAGAGATGCGAGCCCCTCGAAGCTTCAGCTGTGTTCTGGCGGCAAACCCGTTGCGA AAAAGAACGTTCACGGCGACTACTGCACTTATATACGGTTCTCCCCCACCCTCGGGA AAAAGGCGGAGCCAGTACACGACATCACTTTCCCAGTTTACCCCGCGCCACCTTCTC TAGGCACCGGTTCAATTGCCGACCCCTCCCCCCAACTTCTCGGGGACTGTGGGCGA TGTGCGCTCTGCCCACTGACGGGCACCGGAGCGATCGCAGATCCTTCGATAGAGAA ATGTTCTGGCACCTGCACTTGCACTGGGGACAGCCTATTTTGCTAGTTTGTTTTGTTT CGTTTTGTTTTGATGGAGAGCGTATGTTAGTTACGATTCACACAAAAAACCAACACAC AGATGTAATGAAAATAAAGATATTTTATTGCGGCCGCTCGAGTCTAGAGGACATGCC ATGGTCGTATTACCCTGTTATCCCTAACTGCATGCGACGTCGGCGCGCCATCGAATT CCTGCAGCCCGGGGGATCCCTTTGTTGATTTTTTCCACATAGATTATTTTTGACTGTT TTGGCACTTTATATAAATGGAATCATATAGTAAATATATACATGTATATATGTATATATA CACTATATATGTATATATATAGTGTATATATATACATGTATATATGTATATTTACATATA TACTGTATATATGTATATTTACATATATACTGTATATATGTATATATACACGTATATACT GTATATATACAGTATATACTGTATATATATACTGTATATATGTGTATATATATATACAGT ATATATACAGTATACATATATATACATGTATATATACACAGTATATATACATGTATATAT ACACAGTATATATACATGTATATATACACAGTATATATACATGTATATATACACAGTAT ATATACACTGTATATATGTATATATATACTGTGTATATATATACAGTATATATACAGTAT ATATATACATGTATATGTATATATATACTGTATATATGTGTATATATACGTATATATACT GTATATATATATAGACACTTTTGTGTCTGGCTTCTTGCTCTCATCATAAAGCACTTGAA ATCCATCCATGTTGTAGCTGGTATCAGTAGCTAGTTTCTTTTCATTGCTGAGAAGTAT CACTTTTTATTGATGAGTAGTTTTACATTGTATGAACATGCCCGAGTTTGTTTATGCAT TCTACTAATAGACACCTGGGCTGTTTGCAGTTTTTGGCTATTACAAAGAAAATTTCTA |
| | | TATTTTTTTCCAACTTTTATTTTAGGTTCAGCGGGTACATGGGCAGGTTTGTTACATC GGTAAATTGCATGTCACTGGGGTTTGGTGTACAGATTATTTCACCACCCAGGTAATA AGCATAGTACCTGATAGGCAGGTTTTTGATCCTCACCCTCCTCCCTCAAGTAGGCCC CAGTGTCTATTGTTCTATTCCTTTTTTTTTTTTTTGAGATGGAGTCTCACTCTGTTGCC CAGGCTGGAGTGCAGTGGTGCGATCTCGGCTCACTGCAAGGTCCACCTCCAGGGTT CAAGCGATTCTACTGCCGCAGCCTCCCGAGTAGCTGGGATTACAGGCACCCACCAC CACGCCCGGCTAATTTTTGTATTTTTAGTAGAGACTGGGTTTCACCATGTTGGCCAG GATGGTCTCGAACTCCTCACCTCAGGTGATCCGCCCGCTTCGGCCTCGCAAAGTAC TGGGATTACAGGCGTGAGCCACGGTGCCTGGCCTATGGTTCCATTCTTTGTGTACAC GTGTACTCAGTGTTTAGCTTCCACTTGTAAGTGAAAACATGCAGTATTTGGTTTTCTG AAATTCTTGTCTTCATCTTTTTGTAGACAATCCACTACTTTTATTTTTAAATATTAGGAA CAATAGCCTCTGTGTGTCCTGAATTGCAATGTTTTTTCTCTGATTTTGCTGCTCATCTT GTAACCTGTATGGTGTTTTTCTGTGTGCAAGCTTTTTACTTTACGTGGGCAATTTTAT CATTCTTTTCTTTTATGGTTTCTGGGTTTCATGTCATGATTGGAGAAGCTTGATCTACC CTGATACTATAAAAATATCCACCTTGGCTTTTTTTTTTTTTTTTAGATGGATTTTCACTC TTGTTGCCCAGGCTGGAGTGCAATGGCGTGATCTCGGCTCACCACAACCTCTGCCT CCCAGGTTCAAGTGATTCTCCTGCCTCGGCCTCCTGAGTAGCTGGGATTACAGGCA TGCGCCACCACGCTCAGCTAATTTTGTATTTTTAGTAGAGACAGGATTTCACCATGTT GGTCAGGCTGGTCTTGAACTCCCCACCTCAGGTGATCTGTCTGCCTCGGCCTCCCA AAGTGCTGGGATTACAGGCGTGACCCACCACACCTGGCCCACGTTGTTTTCTGATA GTGTTTTCACGTGTTATTTTTTCTGTCTTACTCTTTGAATCATCTAAATGTATCTTAATG TCAGGAGTGAGGTAGAGGACCCAGTTGTTTGGTTTAAATGACTAGCCAGTTTTCCCA ACACCAGGTGTTGAACATCCCTCACTGATGTGAGATGCTGCCTTTTTCACAGTCTCA ATCCCCATGTGCATTTCTATTTATTTTCCTTCCACTTTATTCACATCTTTTTCAGTATCC ACAACCATATTGTTTTAAATAGCTTCATGATATGTTTTAATATTTGGTAGAGCTAGAAC CCTTTCTTGGTTCTTTTTCTTTTCTTCTTCTCCTCCTTTTCCTCCTCCTTCTTTCTCCTC CTCCTTCTTCTTTCTTTTTAGAAATTTCCTGGCTATTCTTGTTTTGTAGTCTTCCAGAT AAATAATTTTGAATCACTTTGTCATGTTCCAGAAAATAATACTGCCAGAATTTTTATTG TGATCACAGAAGTTTTACAGATTCATCTGGGGGAAAAAAACCCATCTTCCCAGAATAC AGAAAGGGTGAGGGAAAGACAAACATCAAGGACAAGTCTCAGGTTCTGGACTTGGA GACCAAGAGGATTTGGGGCATCCGGGAGCAGGGCAGGGAGGTGTGATGGCTGCCT TTCACTGAGTAGGAGGATGCCCAGACTTGGGGGAACTATGGAAAGTTTGGTTCAGA CTTTCTGAGTTGGAGATGTCTATGCGCAGTCCACAGGGCGAAGCCCTGGAGGTAGA GGCCCTCCTTACTGCCTCCCTCATTCAGTCTGAGGTCCATTCTCCACATGGACACTG AAGTGACTCAAGGCTTCCCTTGTTGGATCAGGATAGTCTCCAGGGCCCTGGGTGGT CTGGGCCTGCCCACCTCCCCATCCCCTCTCCTGCCTGCCCTCCTCTTGCTCACTGG ACCCCACCCACCAGCCTCCTTCTATTCCTCCTGACATTTGCACTTGCTGTTCCGTCT CTGCTTGGAACATCCTAGCTCTTTGCATGGCTTGCTCCTTCCCATCTTTTAATCTAAG CTCAAATATCACTCCTTCAGATATGCCTTTCCCAAATATCCTAGCTAAAGAGAACTGC TCCCCACTTCCCACAAGCCCTAGCAAACTGGGACTCTCCTTCTTGCTTTCTTTCTCTC TGCCCCTTCTGTGTTTTTTCCATAGACCTTAACTTGTTACTGTTTTATTTGTTGATGTG TTTACATCATTTGTCCCCCTGTGATGGTTAGTTTTATGTGTCAACTTGGCTAGGCTGT AGGGGCTGTTCGTCAATCAAACACTGATCTATGTGTAGCTGTGAAGGGATTTTGTAG CTGTGATTAATAGTTGACTTTAAGTAAGGAAGATTATCCTGGAGAATGTGGGGGAAG GGGCAGACCTCAGCCAATCAGTTGAAAGGCCTTAAGAACAAAGCTGAGATTTCCCC GAGGAAGAAGAAATTCTGCCTGTGGACTGCAGCATCAGCTCCTGCTTGAGAGGTTC CAGGCTGCCCTTCCTGACAGCCTGTCCTATGATTTTGGACTTGCCTTGCCAGGTTTT TCCTCTTTCTTGACCCCCCAAAACCATATATATCTCCTGCTGGTTTGTTTCTCTGGTG TAATCCTGAGGGATGAACACTCCTCCCTCCCAGTTCACTCTGGGAGAGGTTGGGGA TCATGTTTGTCTATTTCCCCATGCCTGGCCCATGCGTGTTTATTGAATCACTGAACAA CCACGCATTAGGAAGCCAGTCATATGTGCTTCTTCAGAACCTCATGTCCATTGCCAG ATCTCCCTGGCTTTTGTGGCTAGAGGACAAGTGAGAGATAGTAGCTTACCCACAGAC CTTGGCCCTGAGGCCCCCAGGGGCCTGAGCCTGCTGTAAGGGAGGAGGGAGCCCC TGAGGTCTCACACACCTCCCTGGGGATCTGGCATTTTCCCCTGGGGCTGGCCTCAG AGCTGGGCGGGGGCAGATTATGGAGTGGGTTGTAGAGGGAGCACCTCCCCACTAG CAATCATGGTTTTTTCTGCGCCTCTCATAGGGAGGCCTACAGGCCTCCACCTTCAGC TCTTCAGGAGCCACAGGCAGCTGAGTGACTTCTGCATCACAGTCTTCTCAGAGACAA ACTTGCAGTAAGAAAGAGGGGCCCGTGAGGGACCCCAGAGAAGGCTGTTGTCAAAG CAGGATGAGAGTGAACTCTTCCATGGGGGACACCCAGCGTCTCCAAGCTCTTTTATG CTGTCTTCAAGGGGTCTAGAGAGCTTCAGCCCAACATGAGACCCAGTCCAGCAGCA TTTCCCTGCGAAGTGAAAGTTAGGACCCTGACTAGATACACCACGCTGACCTCAGCC AGAATATCAAGATGCTGAGGCGCTGAGATGCTGGGATGCTAAGGTGCTAAGGTGCT GGGGTGCTGGGGTGCTAGGATGCTGAGGTTTTGTGATACTGGGTCGCTGAGATGCT GGGATACTGGGGTGCTGAAGTGCTGGGGTACTGGGGTGTTGCCATGCTGAAATGCT GGGGTGTTGGGATGCTGAGGTGCTGGGTTGCTGGGATGCTGGGATAGTCCTTGGAT GCTGCGGTGCTGAGATGCTGGTCTGCTGGTCTGCTGGGGTGTTGGGATGCTGGGA TTTTGGGATGCTGGAGTGCTGCAGTGCTGCAGTGCTGAGATGCTGGAGTGCTGGGG TGCTGGAATACTGTAGTACTGGTGTGCTGGAGCATTGAGATGCTAGGGCACTGGGA TGCTAAGGTGCTGAGATGCTGCAGTGCTGGGGTGTTGGGATGCCGAGGTGTTGGG ATGCTTAAGTGCTGGGGTGTTGGGATGCTGGGGTGCTGGAACACTATGGTGCTGGG |
| | | GTGCTGGAGTGTTGAGATACTGTAGTGGTGGGATGCTTAAGTGCTGGGGTGCTGGG TGTTGGGATGCCTAGGTGCTGGGGTGCCGAGATGCTGGAGTACTAGTGTGCTGGGA TGCTGAAGTGCTGGGGTCCTGAGATGCTGCAGTGCTAGGGTGCTGAGATTCTGGGC TGCTGGAGTGTTGGGGTGCTGGGATGCTGGAGTGCTGAGATGCTTGGACAATGGG GTGCTGGAATACTATGGTGCTGGGGTGCTGGGGTATTGAGATGCTAGGGTACTGGG ATGCTGAAGTGCTGAGATCCTGGAGTGCTGGGCTGCTGGGCCACAGGCTCTTGAAT CCATTCGTCTGCCCAGGGGAAGAAACCAGAAGATAAAGAGCTAATGAAGGAGCTTT GGTTGAGAGGGAGGAAGTAATGGAAGGAGCAACATCTTGTGGAGGAGCAGGAGAG AATGGACCTCAGGTTGGGAGAGAGGGCCAGGCTACAGGCCAGAGAGGCAGAAGGA TTCCAGCAGAGTGTGGGCTCCAGGAGCCAAGGGGAAACAGGTTTCTGGGAGGAGA GAGTCCAGTACTGCTGAAGTGGCAAGTCCGCTGAGGACCAGGAAGCTTCATTTGGC TTTATGACCAGGAGGAATTTGGAACTGTGACTAGAGTACTTAGGGGGAAGGAGGCA AGACTGGAGCCAGATTGCTCTGGGTTGAGGGGTGAGTGGGAGGTGAAGCAGGGCA CTGTCACTCCTTTGAAGGGTGGCAGAGAGCTGGAATTGGTGCTGGATGGGCTGTGG GGTGACAGGGTCATGTGGAAAGCCCCTGGGGGGCACCTGGAAAAGGAGAAGCTGA CAGTACAGTGAGAGGACAGCTAAGGGAAAGCGGAATGGCAGAACACGCACTGCCA GGAGGAATGAGGATAGGGTCAGGAGTGCCAGGGGCAGTGAGGCCAGCCTGGGGT CAGGTGGCAGGACGTGTCCAGGAAGCTGGTCTGCACTGCAGCCCACACTGGCTCA GCCTTGAGGTTCCCTGTGTGGTTGGGGTAGGAAGTTGAACCCTCTGGGAATGGAAG ATGGAACCAGCTCTGCGAGCCAAGCTCAGCTTTTATCTATGGGTCTCTGAGGGCTG GCAGAGCTGAGTGGGGACAACTGTGATCCGTGAGGCTCTCAGGTTGAGGTGGCCC CTCCGGGAGGGCTTCATTTTCCCAGCGGGTAGGTTCTAAGCAGCAGTGGCTGGGCA GGTGGGTCCAACACAGAGCCAGAGAAGGGTGAATGGGCCTCCTGGCACCCCACCC CTGCTGCCCCTGAGCTCAGTGATGGAGGGGGACAGCACAGCTGAGCCCAAGTGCT TTGGTGTGGCCCTGAGGGAAAGCTGCAGCCTGCCTGGGGCCTGGCATGGATGGGA CACTTGAGGCAGAGGGACAATAGTGGGCGCTGCAGTGAGGCTGGCTCTTGGAGAG GTTTCCTGAGGAGTGCTGCCTGAGACGGGCAGGGAGAACAGAGACAAAGTTGGTGA CAGGGAATGAAAGCTGACTGAAGGACTTTACCCAGACCTATGAGGATATCTCTCTCA GCAGGAAGCAGGAGGGGACTGTGTGAGGACTGGCCAAGAGCTGGAGTGTTGGGAA AATGACTCTTTCTCCGACCCCTCTGTCCTAGCTCTGGCCCCTGGACTGCGGAGGTCT GCTTCCACCCCCATTGGTCGATCGTTGTCCCTTGTCACAGCCATTGAGAATTTTGGC AGGGAGCATGTTCTTAGAGCATTTTTAGGCTCTGCGGGACATAACAGCTCTGCCTCA GAGCACATGCCTTTCTCAGCTCCTGAAAGCCACTGATCAAATTGGAACATTTTGTAC CTTAGGGATGAGGATATCAACTCTCCCAGCCACTTAGAGGGATAAATGTGATGATGC ATTCAATTGTGACTACATCTGATCCCAACTGTTGCTTCAGCTGCTCTCCTATAGCACA TGGCGGGAGGCGTGCATCCCAGTAGCTACCTCCCCACTTTTGGGGAGATGTGGTTC CATCCATGAAACCTGGGTACCCGCCTACCAGGTCCTGGCCTATCAGGTGGCAGGGT CTGGTCAAAGAAGGGCATGTGTGGTCTTCAGCAAGGGAGACAGGACGGTGGTGCA GAGCGTCTAGACCCTCAGGGCAAGTCTCCCCCACACCTGCTCCCGGGGCAGTTGTC TTTGTGACCTCCCATCCCCCTCTGTTTCATCCTCTATAAAATGAGGGGCTGAGCCCC AAAATAACAGGCTTCTTTGCCATGATGCAAAACTGCTGAATCTTTCTTTCTGACACAC AAGGCATCGAGCAGCCTCTGAAAGAACCAAAGCCACTAGCAGGCTTCCTGACTTGG GTTTGTAGGTACTGAATACTCCCTTGAAAAATAAAAACATAGAGGCACTTTTCTCCTG GCTGTTTATTACAGAACGAAGAAAAAACACACTGGCTTGAAACAGACGCCAGATTTC AAATGTAGAGGTGAAATACGAGGTGGCAATTAAAATGTGATTACAGAAAGTCTGGAC ACTGAGAAAAGTTTACAGGACAGTGGGTGTGGGTTTTCTATAACAGACACTTAAATAT ACATGACGATAATTGCAGATAGAAACCATCAAAGACAAACCCCAAATCAACTAATAAT GTTTACAGATGTTCCCCCCCAAACCACAGAGCCTTACATCAAAACAAATACTGAAAG GCTTTAAACCAGGAACAGCTCGCCTTAACCCCACGAGGGTGCACACAAGCTGGGCT TTTTCTCTCGGTCTGAATGGTAAAGGGAGGAGGATACTCTAGCTCCTCCAGGTGGAT TGCTGAGACAGGGCTCGGCTCACACACTGTCTCTGCGCCTCTCCCAAATCTGGAGA ACTCTCCCAGCCTCCTGGTAAAGTGTCTCTGTGGGGCACTTAACGATAAAACAGCTT CTGCTGTAAAGCTCATTAGGAAAGAGCTAGCGGAGACTGAAAGGTTCGCAAAAGAG ATTAAGAATCACACAAGGCAATAGGATTTTTAGTGAACATAGAAATAAATGGCCAAGT GGTTTTCTATTTGGCATTTGTCAACTTGCACAACAACTCTTGGTCATATCCACATTGC TCATTGCATTAAAACCATAAGCGACTCAGCCACCTAGCTTAACAAGGTATCACTGGA GCAAACAACACGGTCTGCATATTTGTAACATTGTATAATAAACACAAAACAATGCATA GTAAACACAACTCTACTGAAACAAAAGCCGTCGCTTTATTTACAAAGTCACAAAATGA AGTATAAATACTTCTGTCATTAATGTTTAGGAAAACCATTTACAAAATTTTCAAATATG TACACGTAGCTTGAAAAATCACCAGCTTTCCATTTTGTCACAGGTAGAGAGAGGGAT AAGCATGGGCTGACAACACCACTCAAATTGTAACGGGAGACAACTGCGGGTATGGA TCGACACCACTTCCTAGAGTGATGTCACCATGGGGGTTTCTATGGGCATCCTGCTCA GATTTAAAGTGCCCCAGCATCCTGGGTGACTTGCCCAGAATTCTGGGCTGTGGCATT TTGAGCAGCAGCATGCTGTTCCAAAATGTCGTCGATCAGCCTCAAGTTGCACACCCA GTCTTCATCTGGGCTCACACAGGAGCCTTTCAAGAGAGCTTCAATGAAATCTACCTC ATTGCAGTCAGGTGACGAAATCAGATCATTTAGTGGGGGTTGGGGCTGGCGCAAAA AGTCGGCAGGTGGCAGCTCAGGGGGAATATCCGTTCTGTCGAACGGACCTGGGAA CTGGCTGGCAGCAACGGCAGAAGCAGCAGCAGCGGTGGCAGCAGCAGCCACATAG CTTGGTGGCTCGATGCCCTGTATGGGGCTCAGGGGACTAAAGCTGGCCATACCCTG CTGGAGGAACTTGGTGGTGTTTGCTACAGGCACCGGGCCCTGTACCGGGCTCTGCC |
| | | TGAGGCTCTGGCTGCCCAGCAGGCTGAAGCTGGGGTTGTTGGCCAGGGGCACTTG TGTTCCCATCGCAGCGGGCACTTGTGCCTCCCAATCAGATGGCCTCTGAAGGCAGG CCTGGCCAGAAGGTGAGTGCTGCTGAACGCTATTATCCACTTGGCTGAGGGGTGTT TTCCCCGAAACTGCTGTGGTCACAGCTGCTGCCGCTGTGACCCATGCAGCATTGTT GAACGCAGTGGGCATTCTTGGCACACTAGGCCGTCTGAGCTGGTGGGGACTCAAG GACTGGGTGCCCAGGGAGCTGGGACAGAACCCAGGCAGGGGCACTTCTGGTGGG GTGGCCTTGGGGCTCTGCATATGCTGGCAGACAGAGTCAAGTCTGCCCAGGGGAGT CTGGCCTGAGTGTGAGAGGATGGGACACTGGGGGCTGGAGGTGAAAATTCCTTGC CGCTTCCCCAGAGTTGGTGAGATCACTCCCATGCCCTCGCAGCTCTGGTGCCTGGT GAGTGGGATCATTCCTGGACTCAGATTGTTCTGAAGAAGCCCAGTTCTGGGTGGCAT CAAGTGCTTGCTAGATGGGGGGCTTGCCTTGATCCGGCTACACTTGGAGGTGACTT GTTCTTGGACGGCTACATACAGAAAGAGAGAAGTGGGGATGAGTTCCAAAGGCATC CTCGACTTCGGCTGTGGCCACCGGAGGGTAGCTCCTGGCCCAACACGGACTTCTCA CCTCCCGCCCTTGGCTCTCTACTGAGCTCCCCCCTGCTCCCCAATTCCTCGCCATTC CCCTCATTTCTCTGCCCTCAGCCTGGACTGCAGTTCTTCTGGGAAGCTGCCCCAACT CCCTAGGTCTGTGCTCACCAAGAGCAGATCACACTGGACTGAAATGCCAGCTGATTT GTCTCTTCAAGAAAATTGGAAGCTCCTGGAGGTCAGGGTCCATGTCTGCTTTTACAC TCAGTGCTCTGTATGCAGGCCTGGCACTGCCCACCCTTTGACAGGTGGTGCATATTT TGTAGAAGGAAGGAAGGGGCCAGGTGGGGTGGGCTGGGCTGGTGGCGGGAGCTA GCTCAGCCTCTTAGATTCTCTACCCGATGGATGTGACCTGGGACAGCAAGTGAGTGT GGTGAGTGAGTGCAGACGGTGCTTTGTTCCCCTCTTGTCTCATAGCCTAGATGGCCT CTGAGCCCAGATCTGGGGCTCAGACAACATTTGTTCAACTGAACGGTAATGGGTTTC CTTTCTGAAGGCTGAAATCTGGGAGCTGACATTCTGGACTCCCTGAGTTCTGAAGAG CCTGGGGATGGAGAGACACGGAGCAGAAGATGGAAGGTAGAGTCCCAGGTGCCTA AGATGGGGAATACATCTCCCCTCATTGTCATGAGAGTCCACTCTAGCTGATATCTAC TGTGGCCAATATCTACCGGTACTTTTTTGGGGTGGACACTGAGTCATGCAGCAGTCT TATGGTTTACCCAAGGTCAGGTAGGGGAGACAGTGCAGTCAGAGCACAAGCCCAGT GTGTCTGACCCACCCAAGAATCCATGCTCGTATCTACAAAAATGATTTTTTCTCTTGT AATGGTGCCTAGGTTCTTTTATTATCATGGCATGTGTATGTTTTTCAACTAGGTTACAA TCTGGCCTTATAAGGTTAACCTCCTGGAGGCCACCAGCCTTCCTGAAACTTGTCTGT GCTGTCCCTGCAACTGGAGTGTGCCTGATGTGGCACTCCAGCCTGGACAAGTGGGA CACAGACTCCGCTGTTATCAGGCCCAAAGATGTCTTCCATAAGACCAGAAGAGCAAT GGTGTAGAGGTGTCATGGGCTACAATAAAGATGCTGACCTCCTGTCTGAGGGCAAG CAGCCTCTTCTGGCCCTCAGACAAATGCTGAGTGTTCCCAAGACTACCCTCGGCCT GGTCCAATCTCATCCCACTGGTGCGTAAGGGTTGCTGAACTCATGACTTCTTGGCTA GCCTGCAACCTCCACGGAGTGGGAACTACATCAGGCATTTTGCTAACTGCTGTATCC TAGGCCAATAAATGTTGATCACATTTATAGCTGCCATGGTAGGGTGGGGACCCCTGC TATCTATCTGTGGAGGCTCTGGGAGCCCCTGACACAAACTTTCTGAAGCAGAGCCTC CCCAACCCCTTTTCCATTCCCTATACCTGACAGATGGCCCAGGAACCCATTAGAAAT GGAAGGTCACTGCAGCAGTATGTGAATGTGCGTGTGGGAGAAGGGCAGGATCAGA GCCCTGGGGGTGTGGCAGCCCCCAAGTGATTCTAATCCAGATCCTAGGGTTGTTTC CCTGTCCCATTGAAATAGCTGCTTTAAGGGGCCTGACTCAGGGAAATCAGTCTCTTG AATTAAGTGGTGATTTTGGAGTCATTTAGACCAGGCCTTCAATTGGGATCCACTAGTT CTAGAGCGGCCGGGCCCAGGGAACCCCGCAGGCGGGGGCGGCCAGTTTCCCGGG TTCGGCTTTACGTCACGCGAGGGCGGCAGGGAGGACGGAATGGCGGGGTTTGGGG TGGGTCCCTCCTCGGGGGAGCCCTGGGAAAAGAGGACTGCGTGTGGGAAGAGAAG GTGGAAATGGCGTTTTGGTTGACATGTGCCGCCTGCGAGCGTGCTGCGGGGAGGG GCCGAGGGCAGATTCGGGAATGATGGCGCGGGGTGGGGGCGTGGGGGCTTTCTC GGGAGAGGCCCTTCCCTGGAAGTTTGGGGTGCGATGGTGAGGTTCTCGGGGCACC TCTGGAGGGGCCTCGGCACGGAAAGCGACCACCTGGGAGGGCGTGTGGGGACCA GGTTTTGCCTTTAGTTTTGCACACACTGTAGTTCATCTTTATGGAGATGCTCATGGCC TCATTGAAGCCCCACTACAGCTCTGGTAGCGGTAACCATGCGTATTTGACACACGAA GGAACTAGGGAAAAGGCATTAGGTCATTTCAAGCCGAAATTCACATGTGCTAGAATC CAGATTCCATGCTGACCGATGCCCCAGGATATAGAAAATGAGAATCTGGTCCTTACC TTCAAGAACATTCTTAACCGTAATCAGCCTCTGGTATCTTAGCTCCACCCTCACTGGT TTTTTCTTGTTTGTTGAACCGGCCAAGCTGCTGGCCTCCCTCCTCAACCGTTCTGAT CATGCTTGCTAAAATAGTCAAAACCCCGGCCAGTTAAATATGCTTTAGCCTGCTTTAT TATGATTATTTTTGTTGTTTTGGCAATGACCTGGTTACCTGTTGTTTCTCCCACTAAAA CTTTTTAAGGGCAGGAATCACCGCCGTAACTCTAGCACTTAGCACAGTACTTGGCTT GTAAGAGGTCCTCGATGATGGTTTGTTGAATGAATACATTAAATAATTAACCACTTGA ACCCTAAGAAAGAAGCGATTCTATTTCATATTAGGCATTGTAATGACTTAAGGTAAAG AGCAGTGCTATTAACGGAGTCTAACTGGGAATCCAGCTTGTTTGGGCTATTTACTAG TTGTGTGGCTGTGGGCAACTTACTTCACCTCTCTGGGCTTAAGTCATTTTATGTATAT CTGAGGTGCTGGCTACCTCTTGGAGTTATTGAGAGGATTATAAGACAGTCTATGTGA ATCAGCAACCCTTGCATGGCCCCTGGCGGGGAACAGTAATAATAGCCATCATCATGT TTACTTACATAGTCCTAATTAGTCTTCAAAACAGCCCTGTAGCAATGGTATGATTATTA CCATTTTACAGATGAGGAACCTTTGAAGCCTCAGAGAGGCTAACAGACATACCCTAG GTCATACAGTTATTAAGAGAAGGAGCTCTGTCTCGAACCTAGCTCTCTCTCTCTCGA GTAATACCAGTTAAAAAATAGGCTACAAATAGGTACTCAAAAAAATGGTAGTGGCTGT TGTTTTTATTCAGTTGCTGAGGAAAAAATGTTGATTTTTCATCTCTAAACATCAACTTA |
| | | CTTAATTCTGCCAATTTCTTTTTTTTGAGACAGGGTCTCACTCTGTCACCTAGGATGG AGTGCAGTGGCACAATCACTGCTCACTGCAGCCTCGACTTCCCGGGCTCGGGTGAT TCTCCCCAGGCTCAGGGGATTCTCCCACTTCAGCCTCCCAAGTAGCTGGGACTACA GGTGCGCACCACCATCCCTGGCTAATATTTGTACTTTATTTTATTTATTTATTTATTTA TTTTTTGAGATGGAGTTTCGCTCTTGTTGCCCGGGCTGGAGTACAGTGGCATGATCT CGGCTCAGTGCAACCTCTGCCTCCCGGGTTCAAGCGATTCTCCTACCTCATCCCCCT GAGTAGCTGGGATTACAGGCGCCTGCCACCATGCCTGGCTAATTTTTTGTATTTTTA ATAGAGACGAGGTTTCACCATGTTGGCCAGGCTACTCTCGAACTCCTGATCTCAGGT GATCCACCCGCCTTGGCCTCCCAAAGTGCTGGGATTACAGGCGTGAGCCACTGCGC CCGGCCTAATATTTGTATTTTTTGTAGAGATGGTGTTTTGCCATGTTGTCCAGGCTGG TCTTGAACTCCTGAGCTCAAGCGATCTGCCCGCCTCTGCTTCCCAAAGTGCTGGGAT TACAGGCATGAGCCACCGTGCCTGGCCTAGGTAGACGCTTTTAGCTTTGGGGTGTG ATGCCTGCCCCAGTATATAGTGAATTTAATTATTGCTAGAGCTGGCTGTTTGTTAGTT TTCTTTGAACATAAGATACTCATTGTTTTTAGTTTGCAAATCCCTCTTCCTTTTTAAAAA ATTTCTTTCCCTTAAATTGTTTGCATGTTAGCAATAACAAATGCTTAAATGGTGCTATG TGCTAGATACTCTTCTAAGCCCTGTTATGTATATTAACTAATTTTTTAAATTACACAAAT CAGAGAGGTTAAGTAACTTGCCCAAGATTACCCAACAATACTAGGATTTGAACCTAA GTTTGTCTCACCCCAGATTCTGCTCTTAATCTCTAAACTTTTAAGTTAGTAGTGACAAT AGTAGGTATTTATTGAATACTTAACTATGTTTTAGGCGTTGAAGTAAATATTTTGCAGG CATTATCTAATGTAAACACCCTAAAGTTACATAACAGGTACCCTTTAGGTAAATAAAC ACTAGTATGACCTTGGAGGCACAGATAGTTGAAGTAACTTGCCCAATATCACTTACAT GAAATTGGCCCTCAAATGTGTCTGATACAACCCATGCTGCTTGTAACTATCGTTTTAA ACTGCCAGGGTAAACTTGGACACACTTGAGCTAAGAAAAAGCTTTTAGATTTTTGCAA ATTAATGTGAAAGATATGCTTTATGTGGATATAATATCTTCTAAATTTCGGGGATGGTA GTCCTAGAAATGTAATCCTGCCCTAGCCGAGCTTACCCTGCCAATAATTTTTTACAGA ATTGGTAAAACGGAGCACCTTTTTTTTGTCCTTGGCCACACTGTTATCAACAGGGTGT AGATTGACATCAATCTGTAGGTGTAAACCAGAATTACTCTTTGTGACCACCAGGAAAT AGAGCAGTTCAGTTCAGGGGTTTCTTTCTGTGAATTTAGCACTGTGACCTGCATACTA CAAGTCTACTTTGTTTTCTATCCATTGTTTGTATCTGGGTATTGCAAAAGGTAGGAAA AGGACCAACCAGATCAGCAGAGAAGAGTTGCCTTGGAGTTTTCTTTTAGTTTTCTGC AGTTCATTAGATAGTAACTAGGCCATGTCATTTTACTCCCTTGTAGTGAAGATATGTT GAAGTTGTACTGGTATACTCTTCTACCTTTCTGTAATTTTATATTGTGTAGACTTGATA AAATTTATGTGTCAATCACCACCATTAATATCAATATTGAGCCTCAATTCTTATTTTTCT GCCCAGTGGCTGCCAAATTACTAACATTTACAATAATTCACTACTACTAAGATAATCT ACTAGTTCGATCACATACTTCAAATTGTTATGGAACTACTGTCTTCAGCATTGTGCTT CTGATAACTGATAAGTATAATTTTTTTTTTGTCCAGAGTGAACATGTCTATTCTTCCAC TGTACACACTAATAAAAGGAAAAATTGTAATATTGGGTAAATTCATGTCCTTACACAT GTAGTAGTTATGAGCCCATGTCCCTAGAATGAGTAATAATTTATCCCTCCCTTGGTTG AATAGTCAAGAATGCTGATTTTAATTCTTCTAACAGCTTTATCCCTCAGAAGGGAAGG CAAGCAAGTTATATATGTAGTTTATTTGTAAGACTGATATGAAATTGGAAGATGAATCT ACTATTAGCTTTAATTATTTTTACATTTAGGAATATTGCATCAGTAACTCATAATTTTGG TTTTCTGTTATCCTGAGTTAACACAAATTATCCAAGGAGATGGCGGATCATCTGCTTT GAGGTGTTTTTTTTTGAGAATTTTAATGTATCTGAATATAAAAGGTAAAAATATGCCAA CTAGCAATTTCTGCCCATTCCAGAAGTTTGGAAATATTACTCATTACTAGGAATTAAAT AAAATATGGTTTATCTATTGTTATACCTCTTTTAATTCACATAGCTCATTTTTATCTTTT ATTTTTGTTTGTTTTTTTTGAGATGGAGTCTTGCTCTGTCACCAGGCAGGAGTGCAGT GATGCAAATCTCGGCTCACTCTAGCCACCGACTCCCTGGTTCAAGCGATTCTCCTGC CTGAGCCTTCTGAGTAGCTGGGATTACAGGCAGGCACCACCACGCCCAGCTAATTT TTGTAGAGACAGGATTTCACCGTGTTGGCCAGGATGGTCTCCATCTCCTGACCTCAT GATCTGCCTGCTTCGGCCTCCCAAAGTGCTGGGATTACAGGTGGGAGCCACTACGC CTGGCCCACATAGCTCATTTTTAGACTCACTTCCATTAAGTCTTGTTTGGACCCACGA ACATTGTCTTTTTTTTTTTAAGATGGAGTTTCACTTTTGTTGCCCAGACTGTAGTGCAA TGGTGCAATCTCAGCTCACTGCAATCTCTGCCTCCTGGGTTCTAGCAATTCTCCTGC CTCAGCCTCCCGAGTAGCTGGAATTACAGGCGCCCGCCACCACGCCCAGCTAATTT TTGTGTTTTTAGTAGAGACGGGGTTTCACCATGTTGGGCAGGCCAGGGGTGATCCG CCCACCTCAGCCTCCCAAAGTGCTGGGATTACAGGTGTGAGCCACCGCATCTGGCC AACATGTCTTTTTTTTTTTTTTCCTTTTTAACCACAAAGAGACTTAAGCAGTCCTTGTC ACAGATGATGAATTGATGTTGCAAGTATTGTCTTAGCTTGGATTAATTTTCTTGCTTAC TGTAATTTTAGATAATATAGCTTTGTAATTAGAGATTTTATGTGTAAACCACAAAAATG TTTACATGAAGGCCATTATTACAGATGTGACGTGCATAATTATTAGTAATTTGTATGTT TACATGGGTCAGTCTGGCAAAAAATTATGAAGTTTTAAAAATTAAAAAAAATTATAATG CCAGTTTTACTGGAAAGTAAAATTATTTCAGTAATCGATTATAGCAAAAGTATTGATTT TCATTCCAGACAAAAGTCAGAATGAAAGGTAATTTCTCAATACTCTTTCAGATTAATAA AAGTACCTGTAGCGATTTTTATCATTCACAAGTATATCACAAGTAAGTTAGAATTTGA GAACTGTGTTCTAGATCTCTGAGGAGATGCAGTCAGATTTCTGAACTGTCTCAGCAA ATGGTAAGTAACTTAGAGCTAGTAATTAATAACCTGTCCTTTGATTTCTGATTCAGCC AAGAATGGCCATATTTGGGAAAGGCAGATCTGGAGAGTAACCACGTTTTCATTCATT TACCACTTCTAGGCCCCTCCAGAGCTCTCAGATATTTTGGGGTTGAGCCCTTCCCCA AAGCCATACAGGACCTTTTTTTTGTGATCTGTTCTAGCCATTTTTATGTTGGGTGCTT GTTATGGACTGAGCATTTATGTCCTCCCACACCCCCCCCATACCTTTTTTGAAGTCCT |
| | | AACCCCCAGTGTGATGGTATTTGGAGACAGGGCCTTTGGAAGGTAATTACAGTTAGA AGAAGTCGGGAGGGTTGGGCCCAGGTCTGATTGGATTAGTGCCCTTATATGAAAAG ACACCAGGACGGGCGCAGTGGCTCACACCTGTAATCCCAGCACTTTGGGAGGCCAA GGTGGGTGGATCACGAGGTCAGGAGTTTGAGACCAGCCTGGCCAATGTAGTGAAAC ACCATCTCTACTAAAAATACAAAAATTAGCTGGGTGTGGTAGCGGGCTCCTGTCATC CAAGCTACTCGGGAGGGTGAGGCATGAGAATCACTTGAACCCGGGAGTTGGAGGTT GCAGTGAGCCCAGATTGTGCCACTGTACTCCAGCCTGGGTGACAGAGTGAGACTCT GTCTCAAAAAAGAAAAAAAAAAAAAAAGAGACACCAGAGAGCTTGTTAGAAGAGGTC ATGTGAGCACACAGTTAGAAGACCTTCAAGCCAAAGAAGAGGCCTGAGATTGAAACC TACCTTGCAGGTACCTTAATTTTGGACTTCCCAGCCTCCAAAACTGTGAGAAATAAGT TTCTGTTAAGTCACTCAGTCTGTGGTATTTTGTTATGGCAGCCTGAGCAGGTAGTTGT TCTTTCAGAAGGTGTTGATAATAACCACATGCAACACCAAGTCACAAATAATAAAACA GATGTAACTTATATTCATACAGAAAGTTGGGCACTGCCATTGCCTTGTTGGTTTACAC GGCTGTGCTAGTTCAGTAGCAGAAAGGTGCTGGTCTCCTTTACTCAGTTTACAATCT AGGCAGTAGAATGTAATCACTGCTTTAAACTTGATACTGCTTAGGGAGAGAATCATTG GTGCTGGGTAACTTTGGGTTCTAGGTTTACTTTTTGTGTATATATAACTGTTTTTGGTA AATCACAAGTTTCTGGGCTTGTCGAATTAGATTTTGTTACAGATTATGAGCTTTATTAT GCTATACAGTTAGTTGTATGTATATATGCCTTTCCCACTAGATTTTAAGCTTTTTTTTTT TTTTTTTTTTTGTGACGGAGTCTTGCTCTTGTCGCCCAGGCTGAAGTGGAGTGCAGT GGCACAATCTCGGCTCACTGCAGCCTCCACCTCCTAGGTTCAAGCGATTCTCCTGC CTCGGCCTCCCAAGTAACTGGGACTACAGGCACGTGCCACCACACCCGGCTAATTT TTGTATTTTTTGTAGAGACAGGGTTTCGCCATGTTGGCTAGGCTGGTCTTGAACTTCT GGCCTCAGGTGATCCACCCGCCTCAGCCTCCCAAAGTGCTGGGATTTACAGGCATG AGCCACCACGCCCAGCTATAGCTCTTTAAGGGTTGTAAATTTATAATCATTCTTTTAC TCTCCTGCAAATTCTGTTGCACACTGCCTTAATCAAGGTAGATGCTGAATGCATTTTT GTATAATTGAATATGTTGCAATCCCCAACTCTCTCCAACTGTTCCTGTCAAAGCAGCC ACTGGATTGTTAACTAATCCATATTAGATGGGGTTAATTAATATCAGATGGGACAAGT AAGGGCTAATAAGATTATAGGCCACCAAGTAGATTTCTGTCTAGCTCTTATAGAGATT GAGTTTATTGGACCTGTTTGATAGGAAGTTTTGGTGTTTGGGATGATTAAAACTGAAG TTCCTATTTATTGAATTATACCTATTTATATTATTTCATATCAGTGGTCCACATGCAAGT GAGGCTTCTGAGACAGAGTTTGAGTTCTCTCTTCAACTACCATAACACTTAACCTGTA TCTTTTTTTTTTTTTTTTTTTTTAGACAGGAGTCTCGCTCTGTCACTCAGGCTGGAGTG TAGTGGTATGATCTCGGCTCACTGTAACCTCTGCCTCCTGGATTCAAGCAGTTCTCC ATGTCTCAGCCTCCCTAGTAGCTGGGATTACAGGCCTGTGCCACCATGCCTGGCTA ATTTTTTTTTTGTATTTTTAGTAGAGACGGGGTTTTACCACGTTGGCCAGGCTGGTCT CGAACTCTTGACCTCGAGCGATCAACTTGCCTTGGCCTCCCAAAGTGCTGGGATTAC AGGCATGAGCCACAGCGCCCAGCCGTCTTTTTTTTTAAATAGCAATTTAACACTGTTC ACAGTTACTCATGTACATGTCATGCCATCTATTACACTGTAAGTTCTGTGAGGGTAGC TGTATCAAATTTATCTAACTCTCTCTAGTATGCATGACATAGTAAGTATTCAATAAATA TTTGCATATTAGTGATAAGGATACAGGTTCTGAATAGTGGGTCCTTACCATTTAAGAA TTAGTATTTGATGGCCGGGCGGGGTGGCTCACGCCTGTAATCCCAGCACTTTGGGA GGCTGAGGCGGGCGGATCATGAGATCAGGAGATCGAGACCATCCTGGCTAACATG GTGAAATCCCGTCTTTACAAAAAAAATACAAAAGAATTAACCAAGTGTGGTGGTGGG TGCCTGTAGTCCCAGCTACTGCTTTGTGAGGCTGAGGCAGGCAGATCACCTGAGGT GGGAAATTCAAGACCAGCCTGACCAACATGGAGAAACCCCATCTCTACTAAAAATAC AAAATTAGCCGGGCGTGGTGGCGCATGTCTGTAATCCCAGCTACTCGGGAGGCTGA GGCAGGAGAATGGCGTGAACCCGGGAGGCGGAGCTTGCAGTGAGCCAGGATCGC GCCACTGCACTCCAGCCTGGGCGACAGAGCGAGACTCCGTCTCAAAAAAAAAAAAA AAAAAAAAATTAGTATTTGATATTTGATCATTAAATATGAATTAAGAGGACTTAGACTT TTTGTTAAATGTCAAGCTGGGAAAAGTTGTCATTTAAATGAATTGCCTCTTATTTAATT TCGTCTGATGATACATTTTGTTTTTATTTTGTAAAAAATTATTTTTTTTCTTTTTGGAGA CAGGGTCTTGCTCTGTTGCCCAGGCTGGTCACAAACTCCTGACCTCAAGCAATCCTC CTGCCTTAGCCTCCCAAAATGCTGGGATTACAGGCGTGACGACCTCGCCCGGCCTT GTATTATGATACATTTTGAACAACTACAAGTAGACTTGGTATAATGAACCTGCACGTA CCCATTGCCAAGTTCTGACAACTGTCTGTCTATAGCCAATTATGCATTTCTTAAATTA GAACCCCCCCAATATACCCAAATATATATATATGTGTGCATATATATAGTAAGTTGTAA CAAAGTTGTGAATTCATACCTGAAGTATCTCAAGTGATGCAAGTTTTATGAATTTTTGT TTATGCCTTTTGGGAAGAGTTGTATTGACAAATTTTTTATGCTTAAAGTAAACCATAAA TCAAAAAAATAAAATCTAGGATGCAATAAAACAAAACAACTTCTTGACATAAGTATGG TATGTAAATCTGTTTTGATTGGAAATCAATTTGTTATATTGCCAGAATTCCTGTTTTAG AATACATCTCTGCTGATCTGTCTGTATTCTTAGACTGCATATCTGGGATGAACTCTGG GCAGAATTCACATGGGCTTCCTTTGAAATAAACAAGACTTTTCAAATTCTTAGTCGAT CTGCAGAACCTGTAGCCAGGCACTGAACCATTTTGATAGATGCAGTAATCGTTGCAA GTGTATATTTCAAGGGAGTTCTGGCTGGGTCCTAGTTTATGCTTGTGGCAGAAGCAG TGAGTAACTGGGAGGAAGTTGGTGAGTAAGCTTCAAGGAAGAAGTCATTTTTAGTAC TCTGGATCTTCCTGATTTTAAAGCACTACAAAATGGTGCATTTTCATTCTTGTCAAGT GATAACAGATATATTCTGATGAGCCTGAAATGAATATATATTGTATCATTTTTATAATA TCTAGCAAGGTTTGTATTTTCCTAGAACTTGAACTAAATTTCAGTTCATAAAATTTATA AAATACTTAGTTGTTGTAAAATATTTTTGGAATGTTCACATAGGTGACACACAAATGTC CCATTTTCATTCTTTCTATAGTAAATATGTTCTGATATGTGAAGGTTTAGCAGATGCAT |
| | | CAGCATTTAATCCTAGAGGATCTGGCATAATCTTTTCCCCCAAGAATAGAAATTTTTT CTGCTTATGAAAGTAGTACATGTTTCTTTAAAAACAAATCAATATTGACTTCTGCCTGC TGTATAGCACTATGCCTCCACCTGGCCATGACCAGGGGCATGTCCTGGTCCACCTA CCTGAAAATGTTTGCAACCAGCCTCCTGGCCATGTGCACAGGGGCTGAAGTTGTCC CACAGGTATTACGGGCCAACCTGACAATACATGAAGTTCCACCAAAGTCTGAGAACT CAGAACTGAGCTTTGGGGACTGAAAGACAGCACAAACCTCAAATTTCTCAGCACTGG AAACCTCAAAATATAACTGAATTCCATAAATAAGATTTTAAGTCTTAAATATGTATTTTT AAATGTATTAAAAGTCAAGCTGCTTGTATTTAAGCACCTAATACAATGCTTAGGTTGT AAAAGGAGATGCTCAATAGGTACTAACTGATATATTGAGATTTAATTATGGTTTGACC AATATTTATTGGAAACCGCCAAAGCTTAAATCATCAGCTTCTTGAATGTGATTTGAAA GGTAATTTAGTATTGAATAGCATGTGAGCTAGAGTATTTCATTCTTTCTGGTTTATTTC TTCAAATAGACTTTGAATATAATGGTGAATGGGTATTATAAATTAACTAATAAAAATGA CATTGAAAATGAAAAAATATATATATTAAAGTGTAGAAAGTGACCAGGCGTGGTGGCT CACACCTGTAATCCAAGCACCTTGGGAGGCTGAGGCAGGAGGATCTCTTGATCCCA GGAGTTCAAGACCAGCCTGGGCAACATAGCGAGACTTCGTCTCTAAAAAAAAAAAAG AGAGAGAAAAAAATTTTTTTTATTTAAAAAAAGTGTAGAAAGTGTCAAGACCCCACTT CTTACCATTATTTGGTATATTTCTCTATACCCACCCACCCTTCCTCCTTACTCCCTCCC TCCCTTCCCAATCTTTTTATCTTTTTGTATTCTGATTTTTTGTTTGTATATTTTGCTTTAA TTTAATGTATCCTTTAAAAATTTCCCATACATTTTATATGTATATATAAAAACGCATGCT GCCAAAGATAATTTATAAGAAAGACCATTGAATTTTTTTAAAAGTGATATATATTCATT GAAAAAAATTTAGAATATATAGCAAAGCAATAAAGAACTAAATAAAATTGCTGTAACTC CTCTTTCAAAGATAAGTGCTTTTATGATTTTGTTGTATTTTTTTCTGTATATAGGTACAT ATATAGTATTTATAAAGCTGTACTCATAGTACATTTTCACATCACAGGTACCATATCAG TGTTATTAAATATTTTGTATGCCAGGGGCTAGACATACCAAGACAACCAATATGTGGT TCTACTTAAATAATATTAGAGTATCTTTTATGATGACACTTCATGAGTTGACTATAATA ATCTTAGACTTCTAAGAGTTTGGGTTTTCAAAAGATCACTTAGCTTTTTTGGGTGATTT TTCCCCCTTACTGTGAGATGAGAGAGGCTGTTTGGATTTGGGATTGGGGTAGCGGG GACAGCAACTTTTCTTTTCTTTTTCTTTTTTATTTTGAGGTAGGGTATTGCTGTGTCAC CCAGGCTGGAGTGCAGTGGTGTGATCTCGGCTCACTGCAACCTCCACCTCCCGGGC TCAGGTGATCCTCCTGCTTCAGCCTCCCAGTAACTGGGACTACAGGCGCGTGCCAC ATGCCTGGCTAATTTTGTATTTTTAGTAGAGATGGGGTTTCACCATGTTGGCCAGGCT GGTCTCTAACTCCTGACCTCAGGTGATACGCCCACCTGGGCCTCCCAAAATACTGG GATTACAGGCATGAGCCGCTGCATCAGCCAGCAGTTTTTCTTGTGGTTTTTTTTGTTT GTTTTGTTTTGTTTTGTTTTTGAGATAGGGTCTTACTCTGTTGTCCACGCTGGAGTGC TGTGGTATGATCGTAGCTCACTGCAGCCTCAAACTCCTGGGCTCAAGTGATTCCTTC TGCCTCCGCCTCCCGAGTAGCTGGGACTACAGGTATGCACCACCATACCTGGCAAA TTTTTACAAAGTTTTTTGTAGGGACGGGGTCTTGCTACATTCCCCATGTCGGTCTTGA ACTCCTGGCCTCAAGCAACTCTCCTGTCTCAGCCTCCCAAAGCACTGGGATTACAAG TGTGAGCCACCACACCATGCCAGTTTTTCCTGTTCAGTGTGATATTTTATCTTGTTAG ACTACAGTGTGTTAAAACTTGTTTTACTAAATTTTCAAACATACTCAAAAGTGGAGAGA ATAGTATAATGAATACCCGTATGTTCATCACCCATGTTTAGAATATTATTAAATATAAA GATTTTGCTGCGTTTGTCTTAGCTCTTTAAAATTTTTCTTTTTCTCTTTGTGACCTAAA GGAAATTCCATATCTTATCACTTTACTTCTACATTCTTGACTAAGATGACTAAGACATA TAGTTACATGGTTTTTTGTTTTGTTTTTGTTTTTTAAAGACGAAATCTCGCTCTTGTCC CCCAGGCTGGAGTGCAATGGTGCCATCTCAGCTCAGTGCAACCTCTGCCTTCTGGG TACAAGCGATTCTCCTGCCTCAGCCTCCCAAGTAGCTGGGATTACAGGCTCCTGCCA CCACGCCTGGCTAATTTTTGTATTTTTAGTAGAGACGGCGGGGGGAGGTTTCACCAT GTTGACAAGGCTGGTCTGGAACTCCTGACCTCAGGTGATCCACCCGCCTCGGCCTC CCAAAGTGCTGGGATTACAGGCGTGAGCCACCGCGCCCAGCCTGTTTTTTTGTTTGT GTGTTTTGTTTTTTTTGAGACAGAGTCTTGCTCTGTTTCCCAGGCTGGAGTGAAGTG GTGCCATCTCAGCTCAGAGACAGAGTCTTGCTCTGTTTCCCAGGCTGGAGTGAAGT GGTGCCATCTTGGCTCACTGCAACCTTCACCTCCCAGGTTCAAGTGATTCTCCTGCC TCAGCCTCCCAAGTAGCTGGGACTACAGGCATGTGTCACCACACCCGGCTAATTTTT TTGTATTTTTAGTAGAGACGGGATTTCACCGTGTTGCCCAGGCTGGTCTCGAACTCC TGAGCTCAGGCAGTCTGCCTGCCTCAGCCTCCCAAAGTGCTGGGATTACACGTGTG AACCAACCCGCCCGGCCTGTTGTTTTCTTACATAATTCATTATCATACCTACAAAGTT AACAGTTACTAATATCATCTTACACCTAAATTTCTCTGATAGACTAAGGTTATTTTTTA ACATCTTAATCCAATCAAATGTTTGTATCCTGTAATGCTCTCATTGAAACAGCTATATT TCTTTTTCAGATTAGTGATGATGAACCAGGTTATGACCTTGATTTATTTTGCATACCTA ATCATTATGCTGAGGATTTGGAAAGGGTGTTTATTCCTCATGGACTAATTATGGACAG GTAAGTAAGATCTTAAAATGAGGTTTTTTACTTTTTCTTGTGTTAATTTCAAACATCAG CAGCTGTTCTGAGTACTTGCTATTTGAACATAAACTAGGCCAACTTATTAAATAACTG ATGCTTTCTAAAATCTTCTTTATTAAAAATAAAAGAGGAGGGCCTTACTAATTACTTAG TATCAGTTGTGGTATAGTGGGACTCTGTAGGGACCAGAACAAAGTAAACATTGAAGG GAGATGGAAGAAGGAACTCTAGCCAGAGTCTTGCATTTCTCAGTCCTAAACAGGGTA ATGGACTGGGGCTGAATCACATGAAGGCAAGGTCAGATTTTTATTATTATGCACATCT AGCTTGAAAATTTTCTGTTAAGTCAATTACAGTGAAAAACCTTACCTGGTATTGAATG CTTGCATTGTATGTCTGGCTATTCTGTGTTTTTATTTTAAAATTATAATATCAAAATATT TGTGTTATAAAATATTCTAACTATGGAGGCCATAAACAAGAAGACTAAAGTTCTCTCC TTTCAGCCTTCTGTACACATTTCTTCTCAAGCACTGGCCTATGCATGTATACTATATG CAAAAGTACATATATACATTTATATTTTAACGTATGAGTATAGTTTTAAATGTTATTGGA CACTTTTAATATTAGTGTGTCTAGAGCTATCTAATATATTTTAAAGGTTGCATAGCATT CTGTCTTATGGAGATACCATAACTGATTTAACCAGTCCACTATTGATAGACACTATTTT GTTCTTACCGACTGTACTAGAAGAAACATTCTTTTACATGTTTGGTACTTGTTCAGCTT TATTCAAGTGGAATTTCTGGGTCAAGGGGAAAGAGTTTATTGAATATTTTGGTATTGC CAAATTTTCCTCTAAGAAGTTGAATCATTTTATACTCCTGATGTTATATGAGAGTACCT TTCTCTTCACAATTTGTCTCTTTTTTTTTTTTTTTTGAGACAAGGTCTCTGTTGCCCAG GCTGGGGTGCAGTGCAGCAGAATGATCACAGTTCACTGCAGTCTCAACCTCCTGGG TTCAAGCGATCCTTCCACCTCAGCCTCCTGAGTAGCTGGGACTATAGGTGTGCGCC ACCACTCCCAGCTAATATTTTTATTTTGTAGAAACAGGGTTCGCCATGTTACCCAGCC TCCCAAAGTGCTGGGATTACAGGCATGAGCCACTGGCCCAGTTTCTACAGTCTCTCT TAATATTGTATATTATCCAGAAAATTTCATTTAATCAGAACCTGCCAGTCTGATAGGTG AAAATGGTATCTTGTTTTTATTTGCATTTAAAAAAAATTATGATAGTGGTATGCTTGGT TTTTTTGAAGGTATCAAATTTTTTACCTTATGAAACATGAGGGCAAAGGATGTGATAC GTGGAAGATTTAAAAAAAATTTTTAATGCATTTTTTTGAGACAAGGTCTTGCTCTATTG TCCAGGCTGGAGTGCAGTGGCACAATCACAGTTCACTCCAGCCTCAACATCCTGCA CTAAAGTGATTTTCCCACCTCACCTCTCAAGTAGCTGGGACTACAGGTACATGCTAC CATGCCTGGCTAATTTTTTTTTTTTTGCAGGCATGGGGTCTCACTATATTGCCCAGGT TGGTGTGGAAGTTTAATGACTAAGAGGTGTTTGTTATAAAGTTTAATGTATGAAACTT TCTATTAAATTCCTGATTTTATTTCTGTAGGACTGAACGTCTTGCTCGAGATGTGATG AAGGAGATGGGAGGCCATCACATTGTAGCCCTCTGTGTGCTCAAGGGGGGCTATAA ATTCTTTGCTGACCTGCTGGATTACATCAAAGCACTGAATAGAAATAGTGATAGATCC ATTCCTATGACTGTAGATTTTATCAGACTGAAGAGCTATTGTGTGAGTATATTTAATAT ATGATTCTTTTTAGTGGCAACAGTAGGTTTTCTTATATTTTCTTTGAATCTCTGCAAAC CATACTTGCTTTCATTTCACTTGGTTACAGTGAGATTTTTCTAACATATTCACTAGTAC TTTACATCAAAGCCAATACTGTTTTTTTAAAACTAGTCACCTTGGAGGATATATACTTA TTTTACAGGTGTGTGTGGTTTTTTAAATAAACTCCTTTTAGGAATTGCTGTTGGGACTT GGGATACTTTTTTCACTATACATACTGGTGACAGATACCCTCTCTTGAGCTACATCGG TTTGTGGGGAGTCAAAAGTCCTTTGGAGCTAGGTTTGACAAATAAGGTGGGTTAACA CTTGTTTCCTAGAAAGCACATGGAGAGCTAGAGTATTGGCGAATTGAAGAAATCCCC CTTTTTTTTTAACACACTTAAGAAAGGGGACTGCAGGTATACTCAAGAGAGTAAGTCG CACCAGAAACCACTTTTGATCCACAGTCTGCCTGTGTCACACAATTGAAATGCATCA CAACATTGACACTGTGGATGAAACAAAATCAGTGTGAATTTTAGTAGTGAATTTCATT CATAATTTGATCGTGCAAACGTTTGATTTTTATTACTTTAGACTATTGTTTCTGATTTTA TGTTGGGTTGGTATTTCCTGTGAGTTACTGTTTTACCTTTAAAATAGGAATTTTTCATA CTCTTCAAAGATTAGAACAAATGTCCAGTTTTTGCTGTTTCATGAATGAGTCCTGTCC ATCTTTGTAGAAACTCGCCTTATGTTCACATTTTTATTGAGAATAAGACCACTTATCTA CATTTAACTATCAACCTCATCCTCTCCATTAATCATCTATTTTAGTGACCCAAGTTTTT GACCTTTTCCATGTTTACATCAATCCTGTAGGTGATTGGGCAGCCATTTAAGTATTAT TATAGACATTTTCACTATCCCATTAAAACCCTTTATGCCCATACATCATAACACTACTT CCTACCCATAAGCTCCTTTTAACTTGTTAAAGTCTTGCTTGAATTAAAGACTTGTTTAC GGTATCGATAAGCTTGATATCAAAACGCCAACTTTGACCCGGAACGCGGAAAACACC TGAGAAAAACACCTGGGCGAGTCTCCACGTAAACGGTCAAAGTCCCCGCGGCCCTA GACAAATATTACGCGCTATGAGTAACACAAAATTATTCAGATTTCACTTCCTCTTATTC AGTTTTCCCGCGAAAATGGCCAAATCTTACTCGGTTACGCCCAAATTTACTACAACAT CCGCCTAAAACCGCGCGAAAATTGTCACTTCCTGTGTACACCGGCGCACACCAAAAA CGTCACTTTTGCCACATCCGTCGCTTACATGTGTTCCGCCACACTTGCAACATCACA CTTCCGCCACACTACTACGTCACCCGCCCCGTTCCCACGCCCCGCGCCACGTCACA AACTCCACCCCCTCATTATCATATTGGCTTCAATCCAAAATAAGGTATATTATTGATGA TGTTT |
| 124 | HDAd2xBITEs | AAACATCATCAATAATATACCTTATTTTGGATTGAAGCCAATATGATAATGAGGGGGT GGAGTTTGTGACGTGGCGCGGGGCGTGGGAACGGGGCGGGTGACGTAGGTTTTAG GGCGGAGTAACTTGTATGTGTTGGGAATTGTAGTTTTCTTAAAATGGGAAGTGACGT AACGTGGGAAAACGGAAGTGACGATTTGAGGAAGTTGTGGGTTTTTTGGCTTTCGTT TCTGGGCGTAGGTTCGCGTGCGGTTTTCTGGGTGTTTTTTGTGGACTTTAACCGTTA CGTCATTTTTTAGTCCTATATATACTCGCTCTGCACTTGGCCCTTTTTTACACTGTGAC TGATTGAGCTGGTGCCGTGTCGAGTGGTGTTTTTTTAATAGGTTTTCTTTTTTACTGG TAGGCGCGCCGTCGACGCGACAACTGTCTTATTACCCTGTTATCCCTACCTTAATTA AGCTAGCCGACGCGTTGGCCAGGAGCTCAGATCTCCGGATCCAAGCTTATCGATTT CGAACCCAAATGGATCTACCACATTTGTAGAGGTTTTACTTGCTTTAAAAAACCTCCC ACACCTCCCCCTGAACCTGAAACATAAAATGAATGCAATTGTTGTTGTTAACTTGTTT ATTGCAGCTTATAATGGTTACAAATAAAGCAATAGCATCACAAATTTCACAAATAAAG CAATAGCATCACAAATTTCACAAATAAAGCATTTTTTTCACTGCATTCTAGTTGTGGTT TGTCCAAACTCATCAATGTATCTTATCATGTCGAGCTAGCTAGTCAGGACTTCAGTTC CAGCTTTGTGCCGGCTCCGAAGGTCAGCGGGTTGCTAGACCATTGCTGACAGTAAT ATGTGGCGGCATCCTCGGCTTCCATAGAGCTGATTGTCAGGCTGTAGCTGGTGCCA GATCCAGATCCGCTGAATCTGTAGGGCACGCCGGAGGCCACTTTGCTTGTGTCGTA GATCCACCGCTTGGGGCTTGTGCCAGACTTCTGCTGATACCAGTTCATGTAGGACAC GCTGCTGCTGGCTCTGCATGTCATGGTCACTTTCTCGCCTGGGGAAGCGCTCATGA TGGCGGGGCTTTGTGTCAGCTGAATATCGGAGCCACCACCACCGCTTCCACCACCA |
| | | CCTGAACCGCCTCCGCCACTAGAAACTGTCAGGGTTGTGCCCTGGCCCCAGTAATC CAGACAGTAGTGGTCGTCGTAGTACCGGGCACAATAGTACACAGCGCTATCTTCAG AGGTCAGGCTGGACAGCTGCATGTAGGCGGTAGAGCTGCTCTTGTCGGTGGTCAGT GTGGCCTTGTCCTTGAACTTCTGGTTGTAATTGGTGTAGCCCCGGCTGGGGTTGATG TAGCCGATCCATTCCAGTCCTTGGCCAGGCCTCTGCTTGACCCAGTGCATGGTGTA CCGGGTGAATGTGTAGCCGCTGGTCTTGCAGGACATCTTCACAGAGGCGCCAGGTC TAGCCAGTTCGGCGCCAGACTGTTGCAGCTTGATATCGGATCCACCGCCACCAAGG GCTTTGATTTCCAGCTTGGTGCCGCTGCCGAAGGTGAAAGGGGTTCTGAAGTGCTG CTGACAAAAGTACACGGCCAGATCCTCGGCCTGCACGCTGCTGATGGTAAAGGTGA AGTCAGGGCCAGAGCCAGAGCCGGTGAATCTAGAGGGCACGCCTGTGTATCTGCT GCTGGCGCTGTAGATCAGCAGCTTAGGAGACTGGCCAGGCTTCTGCTGATACCAGG CCACGGCGTTGTACACGTCCTGGCTGGCTTTACAGGTGATGGACACTCTGTCGCCC ACGCTGGTGCTCAGAAACTTGTGGCTCTGGGTCAGCTGAATATCAGATCCTCCGCC GCCTGAACCTCCGCCTCCGCTTCCTCCGCCACCAGAAGAGACTGTCACGGTTGTGC CCTGGCCCCAATATGGCACGTAGCCGTGGTACACTTCCCATCTGGCACAAAAGTAG GTGGCCATGTCCTCGGACTTCAGGTTGTTGATCTGCAGGTAGGCGGTGTTGGCGCT GGTTTCCAGGCTGAAGTCGAATCTGCCCTTGAAATCGTCGGCGAAGGTGCTCTCGC CGGTGCTGGTATTGATCCAGCCCATCCATTTCAGGCCCTGTCCAGGGGCCTGCTTG ACCCAGTTCATGCCGTAGTTGGTGAAGGGGTAGCCGCTGGCCTTGCAGGAGATCTT CACTGTCTCGCCAGGTTTCTTCAGCTCGGGGCCAGACTGCTGCAGCTGAACTTCAG AATGGGCGCCTGTGGCGGCTCCCACAAGAAACAGGATGCGCCAGATCCAGTCCATG GAAGGTCCGGGGTTCTCTTCCACGTCGCCACATGTCAGCAGGCTGCCTCTGCCTTC GGACTTCAGTTCCAGCTTTGTGCCGGCTCCGAAGGTCAGCGGGTTGCTAGACCATT GCTGACAGTAATATGTGGCGGCATCCTCGGCTTCCATGCTGGAGATTGTCAGGCTG TAGCTGGTTCCGCTGCCGCTTCCGGAAAATCTGTAGGGCACGCCGGAGGCCACTTT GCTTGTGTCGTAGATCCACCGCTTGGGGCTTGTGCCAGACTTCTGCTGATACCAGTT CATGTAGGACACGCTGGAGCTGGCTCTACAGGTCATGGTCACTTTCTCGCCTGGGC TAGCGCTCATGATGGCAGGAGACTGGGTCAGCTGAATATCAGAACCGCCACCACCG CTTCCGCCGCCACCTGAACCACCACCACCTGAAGAAACTGTCAGTGTGGTGCCCTG GCCCCAGTAATCCAGACAGTAGTGGTCGTCGTAGTACCGGGCACAATAGTACACAG CGCTATCTTCAGAGGTCAGGCTGCTCAGCTGCATGTAGGCGGTAGAGCTGCTCTTG TCGGTGGTCAGAGTGGCCTTGTCCTTGAACTTCTGGTTGTAATTGGTGTAGCCCCGG CTGGGGTTGATGTAGCCGATCCATTCCAGTCCTTGGCCAGGCCTCTGCTTGACCCA GTGCATGGTGTACCGGGTGAATGTGTATCCGCTGGTCTTGCAGCTCATCTTCACAGA AGCGCCAGGTCTAGCCAGCTCGGCGCCAGACTGTTGCAGCTTAATATCGCTGCCTC CGCCTCCGCCACTAGACACTGTGACCAGTGTTCCTCTGCCCCAGTAGTCGAGGCCC TGTCTGGCGCAATAATACACGGCGGTGTCCTCGGCTCTCAGGGAGTTCATCTGCAG GTACAGGCTGTTCTTGGCGTTGTCCCGGCTGATGGTGAATCTGCCCTTGATGCTGTC CAGGTAGTAGGTGTAGCTGCCGCCGCTGCTGATTGTGGACACCCATTCAAGGCCTT TGCCAGGAGCCTGTCGGACCCAGCTCATATCGTAGCTGCTGAAGGTGAAGCCGCTG GCGGCACAAGACAGTCTCAGAGATCCGCCAGGCTTAACCAGGCCTCCGCCTGATTC CACCAGCTGAACTTCAGATCCACCACCGCCGGATCCGCCGCCTCCGCTTCCTCCGC CGCCAGATCCTCCGCCACCTCTCTTGATTTCCACCTTGGTGCCTCCGCCAAATGTCA GGGGATTGCTGGACCACTGCAGGCAGTAGTACACGGCGAAGTCCTCAGGTTCCAG GCTGCTTATGGTCAGGGTGAAATCGGTGCCGCTGCCAGAGCCAGAAAATCTGGCAG GCACTCCGCTGGCCAGATTGCTGGTCAGGTAGATCAGGATTCTAGGGGCCTGTCCG GGCTTCTGCTGCAGCCAGTAGATGTAGTTGATGGAGCTGCTGGCGCTACAGCTCAG TGTGGCTCTTTCGCCAGGGCTAAGAGACAGTGTGGCAGGGCTCTGTGTCAGCACAA TCTCGCCGGTGGTATCAGGCAGCCACAGCAGCAGCAGAAACAGCAGTTGAGCTGGA GCCTCCATGGTGGCCCTCCTTCGCCGGTGATCTCAGCTGTAGGAAAGAGAAGAAGG TTAGTAGTCGACGTGTCCCTCTCGATGAATCTAAGTATCAATTGTGAGCGCTCACAA GTCAACACTCTTTTTGATAAATCTAGTAGATATCACTTACGTAGGCGCCGGTCACAGC TTGGATCTGTAACGGCGCAGAACAGAAAACGAAACAAAGACGTAGAGTTGAGCAAG CAGGGTCAGGCAAAGCGTGGAGAGCCGGCTGAGTCTAGGTAGGCTCCAAGGGAGC GCCGGACAAAGGCCCGGTCTCGACCTGAGCTTTAAACTTACCTAGACGGCGGACGC AGTTCAGGAGGCACCACAGGCGGGAGGCGGCAGAACGCGACTCAACCGGCGTGGA TGGCGGCCTCAGGTAGGGCGGCGGGCGCGTGAAGGAGAGATGCGAGCCCCTCGA AGCTTCAGCTGTGTTCTGGCGGCAAACCCGTTGCGAAAAAGAACGTTCACGGCGAC TACTGCACTTATATACGGTTCTCCCCCACCCTCGGGAAAAAGGCGGAGCCAGTACA CGACATCACTTTCCCAGTTTACCCCGCGCCACCTTCTCTAGGCACCGGTTCAATTGC CGACCCCTCCCCCCAACTTCTCGGGGACTGTGGGCGATGTGCGCTCTGCCCACTGA CGGGCACCGGAGCGATCGCAGATCCTTCGATAGAGAAATGTTCTGGCACCTGCACT TGCACTGGGGACAGCCTATTTTGCTAGTTTGTTTTGTTTCGTTTTGTTTTGATGGAGA GCGTATGTTAGTTACGATTCACACAAAAAACCAACACACAGATGTAATGAAAATAAAG ATATTTTATTGCGGCCGCTCGAGTCTAGAGGTATACATGCCATGGTCCGCGGTATTA CCCTGTTATCCCTAAGACAACTGTCCTGCATGCGACGTCGGCGCGCCATCGAATTC CTGCAGCCCGGGGGATCCCTTTGTTGATTTTTTCCACATAGATTATTTTTGACTGTTT TGGCACTTTATATAAATGGAATCATATAGTAAATATATACATGTATATATGTATATATA CACTATATATGTATATATATAGTGTATATATATACATGTATATATGTATATTTACATATA TACTGTATATATGTATATTTACATATATACTGTATATATGTATATATACACGTATATACT |
| | | GTATATATACAGTATATACTGTATATATATACTGTATATATGTGTATATATATATACAGT ATATATACAGTATACATATATATACATGTATATATACACAGTATATATACATGTATATAT ACACAGTATATATACATGTATATATACACAGTATATATACATGTATATATACACAGTAT ATATACACTGTATATATGTATATATATACTGTGTATATATATACAGTATATATACAGTAT ATATATACATGTATATGTATATATATACTGTATATATGTGTATATATACGTATATATACT GTATATATATATAGACACTTTTGTGTCTGGCTTCTTGCTCTCATCATAAAGCACTTGAA ATCCATCCATGTTGTAGCTGGTATCAGTAGCTAGTTTCTTTTCATTGCTGAGAAGTAT CACTTTTTATTGATGAGTAGTTTTACATTGTATGAACATGCCCGAGTTTGTTTATGCAT TCTACTAATAGACACCTGGGCTGTTTGCAGTTTTTGGCTATTACAAAGAAAATTTCTA TATTTTTTTCCAACTTTTATTTTAGGTTCAGCGGGTACATGGGCAGGTTTGTTACATC GGTAAATTGCATGTCACTGGGGTTTGGTGTACAGATTATTTCACCACCCAGGTAATA AGCATAGTACCTGATAGGCAGGTTTTTGATCCTCACCCTCCTCCCTCAAGTAGGCCC CAGTGTCTATTGTTCTATTCCTTTTTTTTTTTTTTGAGATGGAGTCTCACTCTGTTGCC CAGGCTGGAGTGCAGTGGTGCGATCTCGGCTCACTGCAAGGTCCACCTCCAGGGTT CAAGCGATTCTACTGCCGCAGCCTCCCGAGTAGCTGGGATTACAGGCACCCACCAC CACGCCCGGCTAATTTTTGTATTTTTAGTAGAGACTGGGTTTCACCATGTTGGCCAG GATGGTCTCGAACTCCTCACCTCAGGTGATCCGCCCGCTTCGGCCTCGCAAAGTAC TGGGATTACAGGCGTGAGCCACGGTGCCTGGCCTATGGTTCCATTCTTTGTGTACAC GTGTACTCAGTGTTTAGCTTCCACTTGTAAGTGAAAACATGCAGTATTTGGTTTTCTG AAATTCTTGTCTTCATCTTTTTGTAGACAATCCACTACTTTTATTTTTAAATATTAGGAA CAATAGCCTCTGTGTGTCCTGAATTGCAATGTTTTTTCTCTGATTTTGCTGCTCATCTT GTAACCTGTATGGTGTTTTTCTGTGTGCAAGCTTTTTACTTTACGTGGGCAATTTTAT CATTCTTTTCTTTTATGGTTTCTGGGTTTCATGTCATGATTGGAGAAGCTTGATCTACC CTGATACTATAAAAATATCCACCTTGGCTTTTTTTTTTTTTTTTAGATGGATTTTCACTC TTGTTGCCCAGGCTGGAGTGCAATGGCGTGATCTCGGCTCACCACAACCTCTGCCT CCCAGGTTCAAGTGATTCTCCTGCCTCGGCCTCCTGAGTAGCTGGGATTACAGGCA TGCGCCACCACGCTCAGCTAATTTTGTATTTTTAGTAGAGACAGGATTTCACCATGTT GGTCAGGCTGGTCTTGAACTCCCCACCTCAGGTGATCTGTCTGCCTCGGCCTCCCA AAGTGCTGGGATTACAGGCGTGACCCACCACACCTGGCCCACGTTGTTTTCTGATA GTGTTTTCACGTGTTATTTTTTCTGTCTTACTCTTTGAATCATCTAAATGTATCTTAATG TCAGGAGTGAGGTAGAGGACCCAGTTGTTTGGTTTAAATGACTAGCCAGTTTTCCCA ACACCAGGTGTTGAACATCCCTCACTGATGTGAGATGCTGCCTTTTTCACAGTCTCA ATCCCCATGTGCATTTCTATTTATTTTCCTTCCACTTTATTCACATCTTTTTCAGTATCC ACAACCATATTGTTTTAAATAGCTTCATGATATGTTTTAATATTTGGTAGAGCTAGAAC CCTTTCTTGGTTCTTTTTCTTTTCTTCTTCTCCTCCTTTTCCTCCTCCTTCTTTCTCCTC CTCCTTCTTCTTTCTTTTTAGAAATTTCCTGGCTATTCTTGTTTTGTAGTCTTCCAGAT AAATAATTTTGAATCACTTTGTCATGTTCCAGAAAATAATACTGCCAGAATTTTTATTG TGATCACAGAAGTTTTACAGATTCATCTGGGGGAAAAAAACCCATCTTCCCAGAATAC AGAAAGGGTGAGGGAAAGACAAACATCAAGGACAAGTCTCAGGTTCTGGACTTGGA GACCAAGAGGATTTGGGGCATCCGGGAGCAGGGCAGGGAGGTGTGATGGCTGCCT TTCACTGAGTAGGAGGATGCCCAGACTTGGGGGAACTATGGAAAGTTTGGTTCAGA CTTTCTGAGTTGGAGATGTCTATGCGCAGTCCACAGGGCGAAGCCCTGGAGGTAGA GGCCCTCCTTACTGCCTCCCTCATTCAGTCTGAGGTCCATTCTCCACATGGACACTG AAGTGACTCAAGGCTTCCCTTGTTGGATCAGGATAGTCTCCAGGGCCCTGGGTGGT CTGGGCCTGCCCACCTCCCCATCCCCTCTCCTGCCTGCCCTCCTCTTGCTCACTGG ACCCCACCCACCAGCCTCCTTCTATTCCTCCTGACATTTGCACTTGCTGTTCCGTCT CTGCTTGGAACATCCTAGCTCTTTGCATGGCTTGCTCCTTCCCATCTTTTAATCTAAG CTCAAATATCACTCCTTCAGATATGCCTTTCCCAAATATCCTAGCTAAAGAGAACTGC TCCCCACTTCCCACAAGCCCTAGCAAACTGGGACTCTCCTTCTTGCTTTCTTTCTCTC TGCCCCTTCTGTGTTTTTTCCATAGACCTTAACTTGTTACTGTTTTATTTGTTGATGTG TTTACATCATTTGTCCCCCTGTGATGGTTAGTTTTATGTGTCAACTTGGCTAGGCTGT AGGGGCTGTTCGTCAATCAAACACTGATCTATGTGTAGCTGTGAAGGGATTTTGTAG CTGTGATTAATAGTTGACTTTAAGTAAGGAAGATTATCCTGGAGAATGTGGGGGAAG GGGCAGACCTCAGCCAATCAGTTGAAAGGCCTTAAGAACAAAGCTGAGATTTCCCC GAGGAAGAAGAAATTCTGCCTGTGGACTGCAGCATCAGCTCCTGCTTGAGAGGTTC CAGGCTGCCCTTCCTGACAGCCTGTCCTATGATTTTGGACTTGCCTTGCCAGGTTTT TCCTCTTTCTTGACCCCCCAAAACCATATATATCTCCTGCTGGTTTGTTTCTCTGGTG TAATCCTGAGGGATGAACACTCCTCCCTCCCAGTTCACTCTGGGAGAGGTTGGGGA TCATGTTTGTCTATTTCCCCATGCCTGGCCCATGCGTGTTTATTGAATCACTGAACAA CCACGCATTAGGAAGCCAGTCATATGTGCTTCTTCAGAACCTCATGTCCATTGCCAG ATCTCCCTGGCTTTTGTGGCTAGAGGACAAGTGAGAGATAGTAGCTTACCCACAGAC CTTGGCCCTGAGGCCCCCAGGGGCCTGAGCCTGCTGTAAGGGAGGAGGGAGCCCC TGAGGTCTCACACACCTCCCTGGGGATCTGGCATTTTCCCCTGGGGCTGGCCTCAG AGCTGGGCGGGGGCAGATTATGGAGTGGGTTGTAGAGGGAGCACCTCCCCACTAG CAATCATGGTTTTTTCTGCGCCTCTCATAGGGAGGCCTACAGGCCTCCACCTTCAGC TCTTCAGGAGCCACAGGCAGCTGAGTGACTTCTGCATCACAGTCTTCTCAGAGACAA ACTTGCAGTAAGAAAGAGGGGCCCGTGAGGGACCCCAGAGAAGGCTGTTGTCAAAG CAGGATGAGAGTGAACTCTTCCATGGGGGACACCCAGCGTCTCCAAGCTCTTTTATG CTGTCTTCAAGGGGTCTAGAGAGCTTCAGCCCAACATGAGACCCAGTCCAGCAGCA TTTCCCTGCGAAGTGAAAGTTAGGACCCTGACTAGATACACCACGCTGACCTCAGCC |
| | | AGAATATCAAGATGCTGAGGCGCTGAGATGCTGGGATGCTAAGGTGCTAAGGTGCT GGGGTGCTGGGGTGCTAGGATGCTGAGGTTTTGTGATACTGGGTCGCTGAGATGCT GGGATACTGGGGTGCTGAAGTGCTGGGGTACTGGGGTGTTGCCATGCTGAAATGCT GGGGTGTTGGGATGCTGAGGTGCTGGGTTGCTGGGATGCTGGGATAGTCCTTGGAT GCTGCGGTGCTGAGATGCTGGTCTGCTGGTCTGCTGGGGTGTTGGGATGCTGGGA TTTTGGGATGCTGGAGTGCTGCAGTGCTGCAGTGCTGAGATGCTGGAGTGCTGGGG TGCTGGAATACTGTAGTACTGGTGTGCTGGAGCATTGAGATGCTAGGGCACTGGGA TGCTAAGGTGCTGAGATGCTGCAGTGCTGGGGTGTTGGGATGCCGAGGTGTTGGG ATGCTTAAGTGCTGGGGTGTTGGGATGCTGGGGTGCTGGAACACTATGGTGCTGGG GTGCTGGAGTGTTGAGATACTGTAGTGGTGGGATGCTTAAGTGCTGGGGTGCTGGG TGTTGGGATGCCTAGGTGCTGGGGTGCCGAGATGCTGGAGTACTAGTGTGCTGGGA TGCTGAAGTGCTGGGGTCCTGAGATGCTGCAGTGCTAGGGTGCTGAGATTCTGGGC TGCTGGAGTGTTGGGGTGCTGGGATGCTGGAGTGCTGAGATGCTTGGACAATGGG GTGCTGGAATACTATGGTGCTGGGGTGCTGGGGTATTGAGATGCTAGGGTACTGGG ATGCTGAAGTGCTGAGATCCTGGAGTGCTGGGCTGCTGGGCCACAGGCTCTTGAAT CCATTCGTCTGCCCAGGGGAAGAAACCAGAAGATAAAGAGCTAATGAAGGAGCTTT GGTTGAGAGGGAGGAAGTAATGGAAGGAGCAACATCTTGTGGAGGAGCAGGAGAG AATGGACCTCAGGTTGGGAGAGAGGGCCAGGCTACAGGCCAGAGAGGCAGAAGGA TTCCAGCAGAGTGTGGGCTCCAGGAGCCAAGGGGAAACAGGTTTCTGGGAGGAGA GAGTCCAGTACTGCTGAAGTGGCAAGTCCGCTGAGGACCAGGAAGCTTCATTTGGC TTTATGACCAGGAGGAATTTGGAACTGTGACTAGAGTACTTAGGGGGAAGGAGGCA AGACTGGAGCCAGATTGCTCTGGGTTGAGGGGTGAGTGGGAGGTGAAGCAGGGCA CTGTCACTCCTTTGAAGGGTGGCAGAGAGCTGGAATTGGTGCTGGATGGGCTGTGG GGTGACAGGGTCATGTGGAAAGCCCCTGGGGGGCACCTGGAAAAGGAGAAGCTGA CAGTACAGTGAGAGGACAGCTAAGGGAAAGCGGAATGGCAGAACACGCACTGCCA GGAGGAATGAGGATAGGGTCAGGAGTGCCAGGGGCAGTGAGGCCAGCCTGGGGT CAGGTGGCAGGACGTGTCCAGGAAGCTGGTCTGCACTGCAGCCCACACTGGCTCA GCCTTGAGGTTCCCTGTGTGGTTGGGGTAGGAAGTTGAACCCTCTGGGAATGGAAG ATGGAACCAGCTCTGCGAGCCAAGCTCAGCTTTTATCTATGGGTCTCTGAGGGCTG GCAGAGCTGAGTGGGGACAACTGTGATCCGTGAGGCTCTCAGGTTGAGGTGGCCC CTCCGGGAGGGCTTCATTTTCCCAGCGGGTAGGTTCTAAGCAGCAGTGGCTGGGCA GGTGGGTCCAACACAGAGCCAGAGAAGGGTGAATGGGCCTCCTGGCACCCCACCC CTGCTGCCCCTGAGCTCAGTGATGGAGGGGGACAGCACAGCTGAGCCCAAGTGCT TTGGTGTGGCCCTGAGGGAAAGCTGCAGCCTGCCTGGGGCCTGGCATGGATGGGA CACTTGAGGCAGAGGGACAATAGTGGGCGCTGCAGTGAGGCTGGCTCTTGGAGAG GTTTCCTGAGGAGTGCTGCCTGAGACGGGCAGGGAGAACAGAGACAAAGTTGGTGA CAGGGAATGAAAGCTGACTGAAGGACTTTACCCAGACCTATGAGGATATCTCTCTCA GCAGGAAGCAGGAGGGGACTGTGTGAGGACTGGCCAAGAGCTGGAGTGTTGGGAA AATGACTCTTTCTCCGACCCCTCTGTCCTAGCTCTGGCCCCTGGACTGCGGAGGTCT GCTTCCACCCCCATTGGTCGATCGTTGTCCCTTGTCACAGCCATTGAGAATTTTGGC AGGGAGCATGTTCTTAGAGCATTTTTAGGCTCTGCGGGACATAACAGCTCTGCCTCA GAGCACATGCCTTTCTCAGCTCCTGAAAGCCACTGATCAAATTGGAACATTTTGTAC CTTAGGGATGAGGATATCAACTCTCCCAGCCACTTAGAGGGATAAATGTGATGATGC ATTCAATTGTGACTACATCTGATCCCAACTGTTGCTTCAGCTGCTCTCCTATAGCACA TGGCGGGAGGCGTGCATCCCAGTAGCTACCTCCCCACTTTTGGGGAGATGTGGTTC CATCCATGAAACCTGGGTACCCGCCTACCAGGTCCTGGCCTATCAGGTGGCAGGGT CTGGTCAAAGAAGGGCATGTGTGGTCTTCAGCAAGGGAGACAGGACGGTGGTGCA GAGCGTCTAGACCCTCAGGGCAAGTCTCCCCCACACCTGCTCCCGGGGCAGTTGTC TTTGTGACCTCCCATCCCCCTCTGTTTCATCCTCTATAAAATGAGGGGCTGAGCCCC AAAATAACAGGCTTCTTTGCCATGATGCAAAACTGCTGAATCTTTCTTTCTGACACAC AAGGCATCGAGCAGCCTCTGAAAGAACCAAAGCCACTAGCAGGCTTCCTGACTTGG GTTTGTAGGTACTGAATACTCCCTTGAAAAATAAAAACATAGAGGCACTTTTCTCCTG GCTGTTTATTACAGAACGAAGAAAAAACACACTGGCTTGAAACAGACGCCAGATTTC AAATGTAGAGGTGAAATACGAGGTGGCAATTAAAATGTGATTACAGAAAGTCTGGAC ACTGAGAAAAGTTTACAGGACAGTGGGTGTGGGTTTTCTATAACAGACACTTAAATAT ACATGACGATAATTGCAGATAGAAACCATCAAAGACAAACCCCAAATCAACTAATAAT GTTTACAGATGTTCCCCCCCAAACCACAGAGCCTTACATCAAAACAAATACTGAAAG GCTTTAAACCAGGAACAGCTCGCCTTAACCCCACGAGGGTGCACACAAGCTGGGCT TTTTCTCTCGGTCTGAATGGTAAAGGGAGGAGGATACTCTAGCTCCTCCAGGTGGAT TGCTGAGACAGGGCTCGGCTCACACACTGTCTCTGCGCCTCTCCCAAATCTGGAGA ACTCTCCCAGCCTCCTGGTAAAGTGTCTCTGTGGGGCACTTAACGATAAAACAGCTT CTGCTGTAAAGCTCATTAGGAAAGAGCTAGCCTGCAACCTCCACGGAGTGGGAACT ACATCAGGCATTTTGCTAACTGCTGTATCCTAGGCCAATAAATGTTGATCACATTTAT AGCTGCCATGGTAGGGTGGGGACCCCTGCTATCTATCTGTGGAGGCTCTGGGAGCC CCTGACACAAACTTTCTGAAGCAGAGCCTCCCCAACCCCTTTTCCATTCCCTATACCT GACAGATGGCCCAGGAACCCATTAGAAATGGAAGGTCACTGCAGCAGTATGTGAAT GTGCGTGTGGGAGAAGGGCAGGATCAGAGCCCTGGGGGTGTGGCAGCCCCCAAGT GATTCTAATCCAGATCCTAGGGTTGTTTCCCTGTCCCATTGAAATAGCTGCTTTAAGG GGCCTGACTCAGGGAAATCAGTCTCTTGAATTAAGTGGTGATTTTGGAGTCATTTAG ACCAGGCCTTCAATTGGGATCCACTAGTTCTAGAGCGGCCGGGCCCAGGGAACCCC |
| | | GCAGGCGGGGGCGGCCAGTTTCCCGGGTTCGGCTTTACGTCACGCGAGGGCGGCA GGGAGGACGGAATGGCGGGGTTTGGGGTGGGTCCCTCCTCGGGGGAGCCCTGGG AAAAGAGGACTGCGTGTGGGAAGAGAAGGTGGAAATGGCGTTTTGGTTGACATGTG CCGCCTGCGAGCGTGCTGCGGGGAGGGGCCGAGGGCAGATTCGGGAATGATGGC GCGGGGTGGGGGCGTGGGGGCTTTCTCGGGAGAGGCCCTTCCCTGGAAGTTTGGG GTGCGATGGTGAGGTTCTCGGGGCACCTCTGGAGGGGCCTCGGCACGGAAAGCGA CCACCTGGGAGGGCGTGTGGGGACCAGGTTTTGCCTTTAGTTTTGCACACACTGTA GTTCATCTTTATGGAGATGCTCATGGCCTCATTGAAGCCCCACTACAGCTCTGGTAG CGGTAACCATGCGTATTTGACACACGAAGGAACTAGGGAAAAGGCATTAGGTCATTT CAAGCCGAAATTCACATGTGCTAGAATCCAGATTCCATGCTGACCGATGCCCCAGGA TATAGAAAATGAGAATCTGGTCCTTACCTTCAAGAACATTCTTAACCGTAATCAGCCT CTGGTATCTTAGCTCCACCCTCACTGGTTTTTTCTTGTTTGTTGAACCGGCCAAGCTG CTGGCCTCCCTCCTCAACCGTTCTGATCATGCTTGCTAAAATAGTCAAAACCCCGGC CAGTTAAATATGCTTTAGCCTGCTTTATTATGATTATTTTTGTTGTTTTGGCAATGACC TGGTTACCTGTTGTTTCTCCCACTAAAACTTTTTAAGGGCAGGAATCACCGCCGTAAC TCTAGCACTTAGCACAGTACTTGGCTTGTAAGAGGTCCTCGATGATGGTTTGTTGAA TGAATACATTAAATAATTAACCACTTGAACCCTAAGAAAGAAGCGATTCTATTTCATAT TAGGCATTGTAATGACTTAAGGTAAAGAGCAGTGCTATTAACGGAGTCTAACTGGGA ATCCAGCTTGTTTGGGCTATTTACTAGTTGTGTGGCTGTGGGCAACTTACTTCACCTC TCTGGGCTTAAGTCATTTTATGTATATCTGAGGTGCTGGCTACCTCTTGGAGTTATTG AGAGGATTATAAGACAGTCTATGTGAATCAGCAACCCTTGCATGGCCCCTGGCGGG GAACAGTAATAATAGCCATCATCATGTTTACTTACATAGTCCTAATTAGTCTTCAAAAC AGCCCTGTAGCAATGGTATGATTATTACCATTTTACAGATGAGGAACCTTTGAAGCCT CAGAGAGGCTAACAGACATACCCTAGGTCATACAGTTATTAAGAGAAGGAGCTCTGT CTCGAACCTAGCTCTCTCTCTCTCGAGTAATACCAGTTAAAAAATAGGCTACAAATAG GTACTCAAAAAAATGGTAGTGGCTGTTGTTTTTATTCAGTTGCTGAGGAAAAAATGTT GATTTTTCATCTCTAAACATCAACTTACTTAATTCTGCCAATTTCTTTTTTTTGAGACAG GGTCTCACTCTGTCACCTAGGATGGAGTGCAGTGGCACAATCACTGCTCACTGCAG CCTCGACTTCCCGGGCTCGGGTGATTCTCCCCAGGCTCAGGGGATTCTCCCACTTC AGCCTCCCAAGTAGCTGGGACTACAGGTGCGCACCACCATCCCTGGCTAATATTTGT ACTTTATTTTATTTATTTATTTATTTATTTTTTGAGATGGAGTTTCGCTCTTGTTGCCCG GGCTGGAGTACAGTGGCATGATCTCGGCTCAGTGCAACCTCTGCCTCCCGGGTTCA AGCGATTCTCCTACCTCATCCCCCTGAGTAGCTGGGATTACAGGCGCCTGCCACCA TGCCTGGCTAATTTTTTGTATTTTTAATAGAGACGAGGTTTCACCATGTTGGCCAGGC TACTCTCGAACTCCTGATCTCAGGTGATCCACCCGCCTTGGCCTCCCAAAGTGCTGG GATTACAGGCGTGAGCCACTGCGCCCGGCCTAATATTTGTATTTTTTGTAGAGATGG TGTTTTGCCATGTTGTCCAGGCTGGTCTTGAACTCCTGAGCTCAAGCGATCTGCCCG CCTCTGCTTCCCAAAGTGCTGGGATTACAGGCATGAGCCACCGTGCCTGGCCTAGG TAGACGCTTTTAGCTTTGGGGTGTGATGCCTGCCCCAGTATATAGTGAATTTAATTAT TGCTAGAGCTGGCTGTTTGTTAGTTTTCTTTGAACATAAGATACTCATTGTTTTTAGTT TGCAAATCCCTCTTCCTTTTTAAAAAATTTCTTTCCCTTAAATTGTTTGCATGTTAGCA ATAACAAATGCTTAAATGGTGCTATGTGCTAGATACTCTTCTAAGCCCTGTTATGTAT ATTAACTAATTTTTTAAATTACACAAATCAGAGAGGTTAAGTAACTTGCCCAAGATTAC CCAACAATACTAGGATTTGAACCTAAGTTTGTCTCACCCCAGATTCTGCTCTTAATCT CTAAACTTTTAAGTTAGTAGTGACAATAGTAGGTATTTATTGAATACTTAACTATGTTT TAGGCGTTGAAGTAAATATTTTGCAGGCATTATCTAATGTAAACACCCTAAAGTTACA TAACAGGTACCCTTTAGGTAAATAAACACTAGTATGACCTTGGAGGCACAGATAGTT GAAGTAACTTGCCCAATATCACTTACATGAAATTGGCCCTCAAATGTGTCTGATACAA CCCATGCTGCTTGTAACTATCGTTTTAAACTGCCAGGGTAAACTTGGACACACTTGA GCTAAGAAAAAGCTTTTAGATTTTTGCAAATTAATGTGAAAGATATGCTTTATGTGGAT ATAATATCTTCTAAATTTCGGGGATGGTAGTCCTAGAAATGTAATCCTGCCCTAGCCG AGCTTACCCTGCCAATAATTTTTTACAGAATTGGTAAAACGGAGCACCTTTTTTTTGT CCTTGGCCACACTGTTATCAACAGGGTGTAGATTGACATCAATCTGTAGGTGTAAAC CAGAATTACTCTTTGTGACCACCAGGAAATAGAGCAGTTCAGTTCAGGGGTTTCTTT CTGTGAATTTAGCACTGTGACCTGCATACTACAAGTCTACTTTGTTTTCTATCCATTGT TTGTATCTGGGTATTGCAAAAGGTAGGAAAAGGACCAACCAGATCAGCAGAGAAGA GTTGCCTTGGAGTTTTCTTTTAGTTTTCTGCAGTTCATTAGATAGTAACTAGGCCATG TCATTTTACTCCCTTGTAGTGAAGATATGTTGAAGTTGTACTGGTATACTCTTCTACCT TTCTGTAATTTTATATTGTGTAGACTTGATAAAATTTATGTGTCAATCACCACCATTAA TATCAATATTGAGCCTCAATTCTTATTTTTCTGCCCAGTGGCTGCCAAATTACTAACAT TTACAATAATTCACTACTACTAAGATAATCTACTAGTTCGATCACATACTTCAAATTGT TATGGAACTACTGTCTTCAGCATTGTGCTTCTGATAACTGATAAGTATAATTTTTTTTT TGTCCAGAGTGAACATGTCTATTCTTCCACTGTACACACTAATAAAAGGAAAAATTGT AATATTGGGTAAATTCATGTCCTTACACATGTAGTAGTTATGAGCCCATGTCCCTAGA ATGAGTAATAATTTATCCCTCCCTTGGTTGAATAGTCAAGAATGCTGATTTTAATTCTT CTAACAGCTTTATCCCTCAGAAGGGAAGGCAAGCAAGTTATATATGTAGTTTATTTGT AAGACTGATATGAAATTGGAAGATGAATCTACTATTAGCTTTAATTATTTTTACATTTA GGAATATTGCATCAGTAACTCATAATTTTGGTTTTCTGTTATCCTGAGTTAACACAAAT TATCCAAGGAGATGGCGGATCATCTGCTTTGAGGTGTTTTTTTTTGAGAATTTTAATG TATCTGAATATAAAAGGTAAAAATATGCCAACTAGCAATTTCTGCCCATTCCAGAAGT |
| | | TTGGAAATATTACTCATTACTAGGAATTAAATAAAATATGGTTTATCTATTGTTATACCT CTTTTAATTCACATAGCTCATTTTTATCTTTTATTTTTGTTTGTTTTTTTTGAGATGGAG TCTTGCTCTGTCACCAGGCAGGAGTGCAGTGATGCAAATCTCGGCTCACTCTAGCCA CCGACTCCCTGGTTCAAGCGATTCTCCTGCCTGAGCCTTCTGAGTAGCTGGGATTAC AGGCAGGCACCACCACGCCCAGCTAATTTTTGTAGAGACAGGATTTCACCGTGTTG GCCAGGATGGTCTCCATCTCCTGACCTCATGATCTGCCTGCTTCGGCCTCCCAAAGT GCTGGGATTACAGGTGGGAGCCACTACGCCTGGCCCACATAGCTCATTTTTAGACT CACTTCCATTAAGTCTTGTTTGGACCCACGAACATTGTCTTTTTTTTTTTAAGATGGAG TTTCACTTTTGTTGCCCAGACTGTAGTGCAATGGTGCAATCTCAGCTCACTGCAATCT CTGCCTCCTGGGTTCTAGCAATTCTCCTGCCTCAGCCTCCCGAGTAGCTGGAATTAC AGGCGCCCGCCACCACGCCCAGCTAATTTTTGTGTTTTTAGTAGAGACGGGGTTTCA CCATGTTGGGCAGGCCAGGGGTGATCCGCCCACCTCAGCCTCCCAAAGTGCTGGG ATTACAGGTGTGAGCCACCGCATCTGGCCAACATGTCTTTTTTTTTTTTTTCCTTTTTA ACCACAAAGAGACTTAAGCAGTCCTTGTCACAGATGATGAATTGATGTTGCAAGTATT GTCTTAGCTTGGATTAATTTTCTTGCTTACTGTAATTTTAGATAATATAGCTTTGTAATT AGAGATTTTATGTGTAAACCACAAAAATGTTTACATGAAGGCCATTATTACAGATGTG ACGTGCATAATTATTAGTAATTTGTATGTTTACATGGGTCAGTCTGGCAAAAAATTAT GAAGTTTTAAAAATTAAAAAAAATTATAATGCCAGTTTTACTGGAAAGTAAAATTATTT CAGTAATCGATTATAGCAAAAGTATTGATTTTCATTCCAGACAAAAGTCAGAATGAAA GGTAATTTCTCAATACTCTTTCAGATTAATAAAAGTACCTGTAGCGATTTTTATCATTC ACAAGTATATCACAAGTAAGTTAGAATTTGAGAACTGTGTTCTAGATCTCTGAGGAGA TGCAGTCAGATTTCTGAACTGTCTCAGCAAATGGTAAGTAACTTAGAGCTAGTAATTA ATAACCTGTCCTTTGATTTCTGATTCAGCCAAGAATGGCCATATTTGGGAAAGGCAG ATCTGGAGAGTAACCACGTTTTCATTCATTTACCACTTCTAGGCCCCTCCAGAGCTCT CAGATATTTTGGGGTTGAGCCCTTCCCCAAAGCCATACAGGACCTTTTTTTTGTGATC TGTTCTAGCCATTTTTATGTTGGGTGCTTGTTATGGACTGAGCATTTATGTCCTCCCA CACCCCCCCCATACCTTTTTTGAAGTCCTAACCCCCAGTGTGATGGTATTTGGAGAC AGGGCCTTTGGAAGGTAATTACAGTTAGAAGAAGTCGGGAGGGTTGGGCCCAGGTC TGATTGGATTAGTGCCCTTATATGAAAAGACACCAGGACGGGCGCAGTGGCTCACA CCTGTAATCCCAGCACTTTGGGAGGCCAAGGTGGGTGGATCACGAGGTCAGGAGTT TGAGACCAGCCTGGCCAATGTAGTGAAACACCATCTCTACTAAAAATACAAAAATTAG CTGGGTGTGGTAGCGGGCTCCTGTCATCCAAGCTACTCGGGAGGGTGAGGCATGA GAATCACTTGAACCCGGGAGTTGGAGGTTGCAGTGAGCCCAGATTGTGCCACTGTA CTCCAGCCTGGGTGACAGAGTGAGACTCTGTCTCAAAAAAGAAAAAAAAAAAAAAAG AGACACCAGAGAGCTTGTTAGAAGAGGTCATGTGAGCACACAGTTAGAAGACCTTCA AGCCAAAGAAGAGGCCTGAGATTGAAACCTACCTTGCAGGTACCTTAATTTTGGACT TCCCAGCCTCCAAAACTGTGAGAAATAAGTTTCTGTTAAGTCACTCAGTCTGTGGTAT TTTGTTATGGCAGCCTGAGCAGGTAGTTGTTCTTTCAGAAGGTGTTGATAATAACCAC ATGCAACACCAAGTCACAAATAATAAAACAGATGTAACTTATATTCATACAGAAAGTT GGGCACTGCCATTGCCTTGTTGGTTTACACGGCTGTGCTAGTTCAGTAGCAGAAAG GTGCTGGTCTCCTTTACTCAGTTTACAATCTAGGCAGTAGAATGTAATCACTGCTTTA AACTTGATACTGCTTAGGGAGAGAATCATTGGTGCTGGGTAACTTTGGGTTCTAGGT TTACTTTTTGTGTATATATAACTGTTTTTGGTAAATCACAAGTTTCTGGGCTTGTCGAA TTAGATTTTGTTACAGATTATGAGCTTTATTATGCTATACAGTTAGTTGTATGTATATAT GCCTTTCCCACTAGATTTTAAGCTTTTTTTTTTTTTTTTTTTTTGTGACGGAGTCTTGCT CTTGTCGCCCAGGCTGAAGTGGAGTGCAGTGGCACAATCTCGGCTCACTGCAGCCT CCACCTCCTAGGTTCAAGCGATTCTCCTGCCTCGGCCTCCCAAGTAACTGGGACTAC AGGCACGTGCCACCACACCCGGCTAATTTTTGTATTTTTTGTAGAGACAGGGTTTCG CCATGTTGGCTAGGCTGGTCTTGAACTTCTGGCCTCAGGTGATCCACCCGCCTCAG CCTCCCAAAGTGCTGGGATTTACAGGCATGAGCCACCACGCCCAGCTATAGCTCTTT AAGGGTTGTAAATTTATAATCATTCTTTTACTCTCCTGCAAATTCTGTTGCACACTGCC TTAATCAAGGTAGATGCTGAATGCATTTTTGTATAATTGAATATGTTGCAATCCCCAA CTCTCTCCAACTGTTCCTGTCAAAGCAGCCACTGGATTGTTAACTAATCCATATTAGA TGGGGTTAATTAATATCAGATGGGACAAGTAAGGGCTAATAAGATTATAGGCCACCA AGTAGATTTCTGTCTAGCTCTTATAGAGATTGAGTTTATTGGACCTGTTTGATAGGAA GTTTTGGTGTTTGGGATGATTAAAACTGAAGTTCCTATTTATTGAATTATACCTATTTA TATTATTTCATATCAGTGGTCCACATGCAAGTGAGGCTTCTGAGACAGAGTTTGAGTT CTCTCTTCAACTACCATAACACTTAACCTGTATCTTTTTTTTTTTTTTTTTTTTTAGACA GGAGTCTCGCTCTGTCACTCAGGCTGGAGTGTAGTGGTATGATCTCGGCTCACTGT AACCTCTGCCTCCTGGATTCAAGCAGTTCTCCATGTCTCAGCCTCCCTAGTAGCTGG GATTACAGGCCTGTGCCACCATGCCTGGCTAATTTTTTTTTTGTATTTTTAGTAGAGA CGGGGTTTTACCACGTTGGCCAGGCTGGTCTCGAACTCTTGACCTCGAGCGATCAA CTTGCCTTGGCCTCCCAAAGTGCTGGGATTACAGGCATGAGCCACAGCGCCCAGCC GTCTTTTTTTTTAAATAGCAATTTAACACTGTTCACAGTTACTCATGTACATGTCATGC CATCTATTACACTGTAAGTTCTGTGAGGGTAGCTGTATCAAATTTATCTAACTCTCTCT AGTATGCATGACATAGTAAGTATTCAATAAATATTTGCATATTAGTGATAAGGATACA GGTTCTGAATAGTGGGTCCTTACCATTTAAGAATTAGTATTTGATGGCCGGGCGGGG TGGCTCACGCCTGTAATCCCAGCACTTTGGGAGGCTGAGGCGGGCGGATCATGAGA TCAGGAGATCGAGACCATCCTGGCTAACATGGTGAAATCCCGTCTTTACAAAAAAAA TACAAAAGAATTAACCAAGTGTGGTGGTGGGTGCCTGTAGTCCCAGCTACTGCTTTG |
| | | TGAGGCTGAGGCAGGCAGATCACCTGAGGTGGGAAATTCAAGACCAGCCTGACCAA CATGGAGAAACCCCATCTCTACTAAAAATACAAAATTAGCCGGGCGTGGTGGCGCAT GTCTGTAATCCCAGCTACTCGGGAGGCTGAGGCAGGAGAATGGCGTGAACCCGGG AGGCGGAGCTTGCAGTGAGCCAGGATCGCGCCACTGCACTCCAGCCTGGGCGACA GAGCGAGACTCCGTCTCAAAAAAAAAAAAAAAAAAAAAATTAGTATTTGATATTTGAT CATTAAATATGAATTAAGAGGACTTAGACTTTTTGTTAAATGTCAAGCTGGGAAAAGT TGTCATTTAAATGAATTGCCTCTTATTTAATTTCGTCTGATGATACATTTTGTTTTTATT TTGTAAAAAATTATTTTTTTTCTTTTTGGAGACAGGGTCTTGCTCTGTTGCCCAGGCT GGTCACAAACTCCTGACCTCAAGCAATCCTCCTGCCTTAGCCTCCCAAAATGCTGGG ATTACAGGCGTGACGACCTCGCCCGGCCTTGTATTATGATACATTTTGAACAACTAC AAGTAGACTTGGTATAATGAACCTGCACGTACCCATTGCCAAGTTCTGACAACTGTC TGTCTATAGCCAATTATGCATTTCTTAAATTAGAACCCCCCCAATATACCCAAATATAT ATATATGTGTGCATATATATAGTAAGTTGTAACAAAGTTGTGAATTCATACCTGAAGTA TCTCAAGTGATGCAAGTTTTATGAATTTTTGTTTATGCCTTTTGGGAAGAGTTGTATTG ACAAATTTTTTATGCTTAAAGTAAACCATAAATCAAAAAAATAAAATCTAGGATGCAAT AAAACAAAACAACTTCTTGACATAAGTATGGTATGTAAATCTGTTTTGATTGGAAATCA ATTTGTTATATTGCCAGAATTCCTGTTTTAGAATACATCTCTGCTGATCTGTCTGTATT CTTAGACTGCATATCTGGGATGAACTCTGGGCAGAATTCACATGGGCTTCCTTTGAA ATAAACAAGACTTTTCAAATTCTTAGTCGATCTGCAGAACCTGTAGCCAGGCACTGAA CCATTTTGATAGATGCAGTAATCGTTGCAAGTGTATATTTCAAGGGAGTTCTGGCTG GGTCCTAGTTTATGCTTGTGGCAGAAGCAGTGAGTAACTGGGAGGAAGTTGGTGAG TAAGCTTCAAGGAAGAAGTCATTTTTAGTACTCTGGATCTTCCTGATTTTAAAGCACT ACAAAATGGTGCATTTTCATTCTTGTCAAGTGATAACAGATATATTCTGATGAGCCTG AAATGAATATATATTGTATCATTTTTATAATATCTAGCAAGGTTTGTATTTTCCTAGAAC TTGAACTAAATTTCAGTTCATAAAATTTATAAAATACTTAGTTGTTGTAAAATATTTTTG GAATGTTCACATAGGTGACACACAAATGTCCCATTTTCATTCTTTCTATAGTAAATATG TTCTGATATGTGAAGGTTTAGCAGATGCATCAGCATTTAATCCTAGAGGATCTGGCAT AATCTTTTCCCCCAAGAATAGAAATTTTTTCTGCTTATGAAAGTAGTACATGTTTCTTT AAAAACAAATCAATATTGACTTCTGCCTGCTGTATAGCACTATGCCTCCACCTGGCCA TGACCAGGGGCATGTCCTGGTCCACCTACCTGAAAATGTTTGCAACCAGCCTCCTG GCCATGTGCACAGGGGCTGAAGTTGTCCCACAGGTATTACGGGCCAACCTGACAAT ACATGAAGTTCCACCAAAGTCTGAGAACTCAGAACTGAGCTTTGGGGACTGAAAGAC AGCACAAACCTCAAATTTCTCAGCACTGGAAACCTCAAAATATAACTGAATTCCATAA ATAAGATTTTAAGTCTTAAATATGTATTTTTAAATGTATTAAAAGTCAAGCTGCTTGTAT TTAAGCACCTAATACAATGCTTAGGTTGTAAAAGGAGATGCTCAATAGGTACTAACTG ATATATTGAGATTTAATTATGGTTTGACCAATATTTATTGGAAACCGCCAAAGCTTAAA TCATCAGCTTCTTGAATGTGATTTGAAAGGTAATTTAGTATTGAATAGCATGTGAGCT AGAGTATTTCATTCTTTCTGGTTTATTTCTTCAAATAGACTTTGAATATAATGGTGAAT GGGTATTATAAATTAACTAATAAAAATGACATTGAAAATGAAAAAATATATATATTAAA GTGTAGAAAGTGACCAGGCGTGGTGGCTCACACCTGTAATCCAAGCACCTTGGGAG GCTGAGGCAGGAGGATCTCTTGATCCCAGGAGTTCAAGACCAGCCTGGGCAACATA GCGAGACTTCGTCTCTAAAAAAAAAAAAGAGAGAGAAAAAAATTTTTTTTATTTAAAAA AAGTGTAGAAAGTGTCAAGACCCCACTTCTTACCATTATTTGGTATATTTCTCTATAC CCACCCACCCTTCCTCCTTACTCCCTCCCTCCCTTCCCAATCTTTTTATCTTTTTGTAT TCTGATTTTTTGTTTGTATATTTTGCTTTAATTTAATGTATCCTTTAAAAATTTCCCATA CATTTTATATGTATATATAAAAACGCATGCTGCCAAAGATAATTTATAAGAAAGACCAT TGAATTTTTTTAAAAGTGATATATATTCATTGAAAAAAATTTAGAATATATAGCAAAGCA ATAAAGAACTAAATAAAATTGCTGTAACTCCTCTTTCAAAGATAAGTGCTTTTATGATT TTGTTGTATTTTTTTCTGTATATAGGTACATATATAGTATTTATAAAGCTGTACTCATAG TACATTTTCACATCACAGGTACCATATCAGTGTTATTAAATATTTTGTATGCCAGGGG CTAGACATACCAAGACAACCAATATGTGGTTCTACTTAAATAATATTAGAGTATCTTTT ATGATGACACTTCATGAGTTGACTATAATAATCTTAGACTTCTAAGAGTTTGGGTTTTC AAAAGATCACTTAGCTTTTTTGGGTGATTTTTCCCCCTTACTGTGAGATGAGAGAGGC TGTTTGGATTTGGGATTGGGGTAGCGGGGACAGCAACTTTTCTTTTCTTTTTCTTTTT TATTTTGAGGTAGGGTATTGCTGTGTCACCCAGGCTGGAGTGCAGTGGTGTGATCTC GGCTCACTGCAACCTCCACCTCCCGGGCTCAGGTGATCCTCCTGCTTCAGCCTCCC AGTAACTGGGACTACAGGCGCGTGCCACATGCCTGGCTAATTTTGTATTTTTAGTAG AGATGGGGTTTCACCATGTTGGCCAGGCTGGTCTCTAACTCCTGACCTCAGGTGATA CGCCCACCTGGGCCTCCCAAAATACTGGGATTACAGGCATGAGCCGCTGCATCAGC CAGCAGTTTTTCTTGTGGTTTTTTTTGTTTGTTTTGTTTTGTTTTGTTTTTGAGATAGG GTCTTACTCTGTTGTCCACGCTGGAGTGCTGTGGTATGATCGTAGCTCACTGCAGCC TCAAACTCCTGGGCTCAAGTGATTCCTTCTGCCTCCGCCTCCCGAGTAGCTGGGACT ACAGGTATGCACCACCATACCTGGCAAATTTTTACAAAGTTTTTTGTAGGGACGGGG TCTTGCTACATTCCCCATGTCGGTCTTGAACTCCTGGCCTCAAGCAACTCTCCTGTC TCAGCCTCCCAAAGCACTGGGATTACAAGTGTGAGCCACCACACCATGCCAGTTTTT CCTGTTCAGTGTGATATTTTATCTTGTTAGACTACAGTGTGTTAAAACTTGTTTTACTA AATTTTCAAACATACTCAAAAGTGGAGAGAATAGTATAATGAATACCCGTATGTTCAT CACCCATGTTTAGAATATTATTAAATATAAAGATTTTGCTGCGTTTGTCTTAGCTCTTT AAAATTTTTCTTTTTCTCTTTGTGACCTAAAGGAAATTCCATATCTTATCACTTTACTTC TACATTCTTGACTAAGATGACTAAGACATATAGTTACATGGTTTTTTGTTTTGTTTTTG |
| | | TTTTTTAAAGACGAAATCTCGCTCTTGTCCCCCAGGCTGGAGTGCAATGGTGCCATC TCAGCTCAGTGCAACCTCTGCCTTCTGGGTACAAGCGATTCTCCTGCCTCAGCCTCC CAAGTAGCTGGGATTACAGGCTCCTGCCACCACGCCTGGCTAATTTTTGTATTTTTA GTAGAGACGGCGGGGGGAGGTTTCACCATGTTGACAAGGCTGGTCTGGAACTCCTG ACCTCAGGTGATCCACCCGCCTCGGCCTCCCAAAGTGCTGGGATTACAGGCGTGAG CCACCGCGCCCAGCCTGTTTTTTTGTTTGTGTGTTTTGTTTTTTTTGAGACAGAGTCT TGCTCTGTTTCCCAGGCTGGAGTGAAGTGGTGCCATCTCAGCTCAGAGACAGAGTC TTGCTCTGTTTCCCAGGCTGGAGTGAAGTGGTGCCATCTTGGCTCACTGCAACCTTC ACCTCCCAGGTTCAAGTGATTCTCCTGCCTCAGCCTCCCAAGTAGCTGGGACTACAG GCATGTGTCACCACACCCGGCTAATTTTTTTGTATTTTTAGTAGAGACGGGATTTCAC CGTGTTGCCCAGGCTGGTCTCGAACTCCTGAGCTCAGGCAGTCTGCCTGCCTCAGC CTCCCAAAGTGCTGGGATTACACGTGTGAACCAACCCGCCCGGCCTGTTGTTTTCTT ACATAATTCATTATCATACCTACAAAGTTAACAGTTACTAATATCATCTTACACCTAAA TTTCTCTGATAGACTAAGGTTATTTTTTAACATCTTAATCCAATCAAATGTTTGTATCCT GTAATGCTCTCATTGAAACAGCTATATTTCTTTTTCAGATTAGTGATGATGAACCAGG TTATGACCTTGATTTATTTTGCATACCTAATCATTATGCTGAGGATTTGGAAAGGGTG TTTATTCCTCATGGACTAATTATGGACAGGTAAGTAAGATCTTAAAATGAGGTTTTTTA CTTTTTCTTGTGTTAATTTCAAACATCAGCAGCTGTTCTGAGTACTTGCTATTTGAACA TAAACTAGGCCAACTTATTAAATAACTGATGCTTTCTAAAATCTTCTTTATTAAAAATAA AAGAGGAGGGCCTTACTAATTACTTAGTATCAGTTGTGGTATAGTGGGACTCTGTAG GGACCAGAACAAAGTAAACATTGAAGGGAGATGGAAGAAGGAACTCTAGCCAGAGT CTTGCATTTCTCAGTCCTAAACAGGGTAATGGACTGGGGCTGAATCACATGAAGGCA AGGTCAGATTTTTATTATTATGCACATCTAGCTTGAAAATTTTCTGTTAAGTCAATTAC AGTGAAAAACCTTACCTGGTATTGAATGCTTGCATTGTATGTCTGGCTATTCTGTGTT TTTATTTTAAAATTATAATATCAAAATATTTGTGTTATAAAATATTCTAACTATGGAGGC CATAAACAAGAAGACTAAAGTTCTCTCCTTTCAGCCTTCTGTACACATTTCTTCTCAA GCACTGGCCTATGCATGTATACTATATGCAAAAGTACATATATACATTTATATTTTAAC GTATGAGTATAGTTTTAAATGTTATTGGACACTTTTAATATTAGTGTGTCTAGAGCTAT CTAATATATTTTAAAGGTTGCATAGCATTCTGTCTTATGGAGATACCATAACTGATTTA ACCAGTCCACTATTGATAGACACTATTTTGTTCTTACCGACTGTACTAGAAGAAACAT TCTTTTACATGTTTGGTACTTGTTCAGCTTTATTCAAGTGGAATTTCTGGGTCAAGGG GAAAGAGTTTATTGAATATTTTGGTATTGCCAAATTTTCCTCTAAGAAGTTGAATCATT TTATACTCCTGATGTTATATGAGAGTACCTTTCTCTTCACAATTTGTCTCTTTTTTTTTT TTTTTTGAGACAAGGTCTCTGTTGCCCAGGCTGGGGTGCAGTGCAGCAGAATGATCA CAGTTCACTGCAGTCTCAACCTCCTGGGTTCAAGCGATCCTTCCACCTCAGCCTCCT GAGTAGCTGGGACTATAGGTGTGCGCCACCACTCCCAGCTAATATTTTTATTTTGTA GAAACAGGGTTCGCCATGTTACCCAGCCTCCCAAAGTGCTGGGATTACAGGCATGA GCCACTGGCCCAGTTTCTACAGTCTCTCTTAATATTGTATATTATCCAGAAAATTTCAT TTAATCAGAACCTGCCAGTCTGATAGGTGAAAATGGTATCTTGTTTTTATTTGCATTTA AAAAAAATTATGATAGTGGTATGCTTGGTTTTTTTGAAGGTATCAAATTTTTTACCTTA TGAAACATGAGGGCAAAGGATGTGATACGTGGAAGATTTAAAAAAAATTTTTAATGCA TTTTTTTGAGACAAGGTCTTGCTCTATTGTCCAGGCTGGAGTGCAGTGGCACAATCA CAGTTCACTCCAGCCTCAACATCCTGCACTAAAGTGATTTTCCCACCTCACCTCTCAA GTAGCTGGGACTACAGGTACATGCTACCATGCCTGGCTAATTTTTTTTTTTTTGCAGG CATGGGGTCTCACTATATTGCCCAGGTTGGTGTGGAAGTTTAATGACTAAGAGGTGT TTGTTATAAAGTTTAATGTATGAAACTTTCTATTAAATTCCTGATTTTATTTCTGTAGGA CTGAACGTCTTGCTCGAGATGTGATGAAGGAGATGGGAGGCCATCACATTGTAGCC CTCTGTGTGCTCAAGGGGGGCTATAAATTCTTTGCTGACCTGCTGGATTACATCAAA GCACTGAATAGAAATAGTGATAGATCCATTCCTATGACTGTAGATTTTATCAGACTGA AGAGCTATTGTGTGAGTATATTTAATATATGATTCTTTTTAGTGGCAACAGTAGGTTTT CTTATATTTTCTTTGAATCTCTGCAAACCATACTTGCTTTCATTTCACTTGGTTACAGT GAGATTTTTCTAACATATTCACTAGTACTTTACATCAAAGCCAATACTGTTTTTTTAAA ACTAGTCACCTTGGAGGATATATACTTATTTTACAGGTGTGTGTGGTTTTTTAAATAAA CTCCTTTTAGGAATTGCTGTTGGGACTTGGGATACTTTTTTCACTATACATACTGGTG ACAGATACCCTCTCTTGAGCTACATCGGTTTGTGGGGAGTCAAAAGTCCTTTGGAGC TAGGTTTGACAAATAAGGTGGGTTAACACTTGTTTCCTAGAAAGCACATGGAGAGCT AGAGTATTGGCGAATTGAAGAAATCCCCCTTTTTTTTTAACACACTTAAGAAAGGGGA CTGCAGGTATACTCAAGAGAGTAAGTCGCACCAGAAACCACTTTTGATCCACAGTCT GCCTGTGTCACACAATTGAAATGCATCACAACATTGACACTGTGGATGAAACAAAAT CAGTGTGAATTTTAGTAGTGAATTTCATTCATAATTTGATCGTGCAAACGTTTGATTTT TATTACTTTAGACTATTGTTTCTGATTTTATGTTGGGTTGGTATTTCCTGTGAGTTACT GTTTTACCTTTAAAATAGGAATTTTTCATACTCTTCAAAGATTAGAACAAATGTCCAGT TTTTGCTGTTTCATGAATGAGTCCTGTCCATCTTTGTAGAAACTCGCCTTATGTTCAC ATTTTTATTGAGAATAAGACCACTTATCTACATTTAACTATCAACCTCATCCTCTCCAT TAATCATCTATTTTAGTGACCCAAGTTTTTGACCTTTTCCATGTTTACATCAATCCTGT AGGTGATTGGGCAGCCATTTAAGTATTATTATAGACATTTTCACTATCCCATTAAAAC CCTTTATGCCCATACATCATAACACTACTTCCTACCCATAAGCTCCTTTTAACTTGTTA AAGTCTTGCTTGAATTAAAGACTTGTTTACGGTATCGATAAGCTTGATATCAAAACGC CAACTTTGACCCGGAACGCGGAAAACACCTGAGAAAAACACCTGGGCGAGTCTCCA CGTAAACGGTCAAAGTCCCCGCGGCCCTAGACAAATATTACGCGCTATGAGTAACAC AAAATTATTCAGATTTCACTTCCTCTTATTCAGTTTTCCCGCGAAAATGGCCAAATCTT ACTCGGTTACGCCCAAATTTACTACAACATCCGCCTAAAACCGCGCGAAAATTGTCA CTTCCTGTGTACACCGGCGCACACCAAAAACGTCACTTTTGCCACATCCGTCGCTTA CATGTGTTCCGCCACACTTGCAACATCACACTTCCGCCACACTACTACGTCACCCGC CCCGTTCCCACGCCCCGCGCCACGTCACAAACTCCACCCCCTCATTATCATATTGGC TTCAATCCAAAATAAGGTATATTATTGATGATGTTT |
| 125 | HDAdTrio | AAACATCATCAATAATATACCTTATTTTGGATTGAAGCCAATATGATAATGAGGGGGT GGAGTTTGTGACGTGGCGCGGGGCGTGGGAACGGGGCGGGTGACGTAGGTTTTAG GGCGGAGTGATGCCCCCCCTCGAGGTTCGACGGTATCGATAAGCTTGATTTAATTAA GGCCGGCCCCTAGGGGCGCGCCGTCGACGCGACAACTGTCTTATTACCCTGTTATC CCTACCTTAATTAACATTTCCATACCACATTTGTAGAGGTTTTACTTGCTTTAAAAAAC CTCCCACACCTCCCCCTGAACCTGAAACATAAAATGAATGCAATTGTTGTTGTTAACT TGTTTATTGCAGCTTATAATGGTTACAAATAAAGCAATAGCATCACAAATTTCACAAAT AAAGCATTTTTTTCACTGCATTCTAGTTGTGGTTTGTCCAAACTCATCAATGTATCTTA TCATGTCTGGCCAGCTAGCTAGTCAAGCGTAATCAGGCACATCGTATGGATAGCCG GCATAATCTGGCACGTCATAAGGGTAGCCGGCGTAGTCGGGCACATCATAGGGGTA GCTGCCCCCTCCGCCCTTGCCGGGGCTCAGGCTCAGGGACTTCTGGGTGTAGTGG TTGTGCAGGGCCTCGTGCATCACGCTGCAGCTGAACACGTTGCCCTGCTGCCACCG GCTCTTGTCCACGGTCAGCTTGGAGTACAGGAAGAATGAGCCGTCGCTGTCCAGCA CAGGGGGGGTGGTCTTGTAGTTGTTCTCGGGCTGGCCGTTGCTCTCCCATTCCACG GCGATATCGCTGGGGTAGAAGCCTTTCACCAGGCAGGTCAGGGACACCTGGTTCTT GGTCAGCTCGTCCCGGCTGGGGGGCAGTGTGTACACCTGGGGCTCGCGGGGCTG GCCCTTGGCCTTGCTGATGGTTTTCTCGATGGGGGCAGGCAGAGCCTTGTTGGACA CCTTGCACTTGTACTCTTTGCCGTTCAGCCAGTCCTGGTGCAGCACGGTCAGCACG GACACCACCCGGTAGGTGCTGTTGTACTGTTCCTCTCTGGGCTTGGTCTTGGCGTT GTGCACTTCCACGCCGTCCACGTACCAATTAAACTTCACTTCAGGGTCCTCGTGGGA CACGTCCACCACCACGCAGGTCACTTCGGGGGTCCGGCTGATCATCAGGGTGTCCT TGGGCTTTGGGGGGAACAGGAACACGCTAGGTCCGCCCAGCAGCTCAGGGGCAGG GCAGGGGGGACAGGTGTGGGTCTTGTCGCAGCTCTTGGCCTCTCTCTTGATTTCCA CCTTGGTGCCCTGTCCGAAGGTGGCGGGGTGGTACAGGTACTGCTGGCAGTAGTA GGTGGCGAAGTCTTCGGGCTGCAGGGAGCTGATGGTCAGGGTGAAGTCGGTGCCG GAGCCGCTGCCGCTGAATCTGCTGGGCACGCCGCTGTACAGGAAGCTGGCGCTGT AGATCAGCAGCTTGGGGGCCTTGCCGGGCTTCTGCTGATACCAAGCCACGGCGGT GGACACGTCCTGGCTGGCCCGACAGGTGATGGTCACTCTGTCGCCCACGCTGGCG CTCAGGCTGCTGGGGCTCTGGGTCATCTGGATGTCGCTGCCTCCGCCGCCTGATCC TCCCCCTCCGGATCCGCCTCCGCCAGCAGACACAGTCACCAGTGTGCCCTGGCCC CAGTAATCGAAGCCGCCAGGCCAGTGCCTTCTGGCGCAGTAGTACACAGCGGTGTC CTCGGCCCGCAGGCTGTTCATCTGCAGGTAGGCGGTGTTCTTGCTGGTGTCGGCGC TGATGGTGAACCGGCCCTTCACGCTGTCGGCGTAGTAGGTGCTGCCGCCGTAGGG GCTGATCCAGGCCACCCATTCCAGTCCCTTGCCAGGGGCCTGGCGCACCCAGTGG ATCCAGCTGTCGCTGAAGGTGAAGCCGCTGGCGGCGCAAGACAGTCTCAGGCTGC CGCCAGGCTGCACCAGTCCGCCGCCAGATTCCACCAGCTGCACCTCAGAGTGGGC GCCTGTGGCGGCTCCGACCAGGAACAGGATCCGCCAGATCCAGTCCATGGTGCCG GCGCTGAGGACAGTGCTCAGCGGTCCTTCGGCAGTCTCCGGGCCCAGCAACAGGC TTGTAGTCCCGGAGCGCGACGGCGTTCCGACCGATCTGAGGGCGACAGGAGCCCC CGATCAGGCGGAGCCACGAGACGGGCTCCGAGTTGGCCTGGAAAGGGCAGGCCG CGGCGCTTACCTCTCGGCCCCGCAGAGGCAGACGCTCCCGTCGCTGTGGTCTCGG CCCGTGTGGAGCGCGCCGCCGCCGACTCGGCTTATATACCCTCCCCCCAGCCCCG TCGTGGAGGCCTCCGATTGGCGAAGGCGCTCCTCGTTGGAGGACGCTGATTGGTC CATGCCACCAAGCTGGCCGCTGCCGATTCGAAGCGGCCTCCTCCGCAACTTACGTC ACTGCTACGCCCCCCGGGCTGACTCATTGGCTCTTGGTCACTCTCAACGGCCGCCA ATGAGGGGCAGCATCCGCCCTGTGCGCCGGTCCTATTGGTTCAGCAGCTGCCTGTC CCTCGGGTGACCTCTCAACCCGAGACCGTTTCCGCCGGTAACTAGTCAAAACAAACT CCCATTGACGTCAATGGGGTGGAGACTTGGAAATCCCCGTGAGTCAAACCGCTATC CACGCCCATTGATGTACTGCCAAAACCGCATCATCATGGTAATAGCGATGACTAATA CGTAGATGTACTGCCAAGTAGGAAAGTCCCATAAGGTCATGTACTGGGCATAATGCC AGGCGGGCCATTTACCGTCATTGACGTCAATAGGGGGCGTACTTGGCATATGATAC ACTTGATGTACTGCCAAGTGGGCAGTTTACCGTAAATACTCCACCCATTGACGTCAA TGGAAAGTCCCTATTGGCGTTACTATGGGAACATACGTCATTATTGACGTCAATGGG CGGGGGTCGTTGGGCGGTCAGCCAGGCGGGCCATTTACCGTAAGTTATGTAACGC CTGCAGGCAGCTTTCTATGCAACCCAAGGACTCAGTTTTTGGCCTGTTTTAGTGACA GGCAATCAGCAACATGCTGCATTTCTCTCCAGTGTTGTAATCAAAGAAACCCTCCCA TAGCTTTAAATGATATTCCTTCCCCTTCCAATTATGTGGGGGGAAAACAACCCTATTC TCCACCCAGAAGTGTTAACTCAAGAATTACATTTTCAAGAAGTTTCCAGATTCGTAAA ACCAGAATTAGATGTCTTTCACCTAAATGTCTCGGTGTTGACCAAAGGAACACACAG GTTTCTCATTTAACTTTTTTAATGGGTCTCAAAATTCTGTGACAAATTTTTGGTCAAGT TGTTTCCATTAAAAAGTACTGATTTTAAAAACTAATAACTTAAAACTGCCACACGCAAA AAAGAAAACCAAAGTGGTCCACAAAACATTCTCCTTTCCTTCTGAAGGTTTTACGATG CATTGTTATCATTAACCAGTCTTTTACTACTAAACTTAAATGGCCAATTGAAACAAACA GTTCTGAGACCGTTCTTCCACCACTGATTAAGAGTGGGGTGGCAGGTATTAGGGATA |
| | | ATCCTAGGTTAGGAAGCATTCAGATAGCTCATCACTCTATCAATAGTCACTGCCCGA ATTCTGAAAGCATGAAGAAGTATGCAGAGCTTGATTTTAGTTTTATAAAAATCCGGTT CTTCAAGGGAGGATTTTTGTGGCACAGTCTCACTGTTGAAATTCAGGGCCTGCATCA GCTCATCAATAACTGCCAGCATGTTTTGATCTAGAAAGATCTGCCTCTTAGGATCCAT CAGCAGCTTTGCATTCATGGTCTTGAACTCCACCTGGTACATCTTCAAGTCTTCATAA ATACTACTAAGGCACAGGGCCATCATAAAAGAGGTCTTTCTGGAGGCCAGGCAACTC CCATTAGTTATGAAAGAGGTCTCTCTGGAATTTAGGCAACTCTCATTCTTGGTTAATT CCAATGGTAAACAGGCCTCCACTGTGCTGGTTTTATCTTTTGTGATATCTTCATGATC AATCTCTTCAGAAGTGCAAGGGTAAAATTCTAGAGTTTGTCTGGCCTTCTGGAGCAT GTTGCTGACGGCCCTCAGCAGGTTTTGGGAGTGGTGAAGGCATGGGAACATTCCTG GGTCTGGAGTGGCCACGGGGAGGTTTCTGGCGCCCACCCCAGGTACCCCTACTCC AGGAACACTGCAGGGCACAGATGCCCATTCGCTCCAAGATGAGCTATAGTAGCGGT CCTGGGCCCGCACGCTAATGCTGGCATTTTTGCGGCAGATGACCGTGGCTGAGGTC TTGTCCGTGAAGACTCTATCTTTCTTTTCTCTCTTGCTCTTGCCCTGGACCTGAACGC AGAATGTCAGGGAGAAGTAGGAATGTGGAGTACTCCAGGTGTCAGGGTACTCCCAG CTGACCTCCACCTGCCGAGAATTCTTTAATGGCTTCAGCTGCAAGTTCTTGGGTGGG TCAGGTTTGATGATGTCCCTGATGAAGAAGCTGCTGGTGTAGTTTTCATACTTGAGC TTGTGAACGGCATCCACCATGACCTCAATGGGCAGACTCTCCTCAGCAGCTGGGCA GGCACTGTCCTCCTGGCACTCCACTGAGTACTCATACTCCTTGTTGTCCCCTCTGAC TCTCTCTGCAGAGAGTGTAGCAGCTCCGCACGTCACCCCTTGGGGGTCAGAAGAGC CTCTGCTGCTTTTGACACTGAATGTCAAATCAGTACTGATTGTCGTCAGCCACCAGC AGGTGAAACGTCCAGAATAATTCTTGGCCTCGCATCTTAGAAAGGTCTTATTTTTGGG TTCTTTCTGGTCCTTTAAAATATCAGTGGACCAAATTCCATCTTCCTTTTTGTGAAGCA GCAGGAGCGAATGGCTTAGAACCTCGCCTCCTTTGTGACAGGTGTACTGGCCAGCA TCTCCAAACTCTTTGACTTGGATGGTCAGGGTTTTGCCAGAGCCTAAGACCTCACTG CTCTGGTCCAAGGTCCAGGTGATACCATCTTCTTCAGGGGTGTCACAGGTGAGGAC CACCATTTCTCCAGGGGCATCCGGATACCAATCCAATTCTACGACATAAACATCTTTC TTCAGTTCCCATATGGCCACGAGGGGAGATGCCAGAAAAACCAGGGAAAACCAAGA GATGACCAACTGCTGGTGACCCATGGTAAGCTTTCTAGATTCATGACGTGCGACCG GTGAGTCTCAAGTCCCCTTCGATCGCAGGAGCCGCCTCCACCCCCCACCCCTGGGT TCGGATCCTCACACCTCCAGCCGCGCGGTCCGCCCACTACACCCCAAGCAGGTCG GGCCGCTTTTCACCCCCACTTCTCGCGGGCGGGGGACGGGAAACATGAACCCACC AATCGCGCCGCACCACGCACCCACGGCACCCAATGAGGATTCGTGAGGGGGGGCG GAAGTCCACCAATCGGAGCTGTCCAGGTGCGGTGACCGATGCCCGAAGCGTGGCT CCTTCCGATTGGTCAGGGTTTGCAGCTTTTCTCCAATCAAATGGCTCCGCGGGCCCC TCACGCGTGGGACCGCCCCTTTGCCTGGGCGGCCTTGGCCTCTAAGGGAAAACGT CCAATGAATGTAAGCAGAGAACACATCCATCCTTTTAGAGAGGTCGCGGCGGAACTA CTTCCGGATCCTAGAGAGCGTTTATTCCGTGACCGGGGAAACGGTAGCCATGACAA CGTGTTCTTCACCCTTTCCCATCTATGCCTGGGGTTTCGTTGGAAGTGATTCCAGCA CGACTTTCGCGGCCAGCTTTCTCGTGTGGCGGCGCTTCTCGGAAAGAAGAAACACA CGAGAGAGGTTGCAAATCCCCTAGAACCCTTTCAGATGGCGGGGGGGCGGGGGGG TGGCGGTGGTGATGAAACTAGTGGGACTATGGTTGCTGACTAATTGAGATGCATGCT TTGCATACTTCTGCCTGCTGGGGAGCCTGGGGACTTTCCACACCTGGTTGCTGACTA ATTGAGATGCATGCTTTGCATACTTCTGCCTGCTGGGGAGCCTGGGGACTTTCCACA CCCTAACTGACACACATTCCACAGCTGGTTCTTTCAGCCTCAGAAGGTACCTAACCA AGTTCCTCTTTCAGAGGTTATTTCAGGCCCTGCAGGTTAATTAAGCTAGCCGACGCG TTGGCCAGGAGCTCAGATCTCCGGATCCAAGCTTATCGATTTCGAACCCAAATGGAT CTACCACATTTGTAGAGGTTTTACTTGCTTTAAAAAACCTCCCACACCTCCCCCTGAA CCTGAAACATAAAATGAATGCAATTGTTGTTGTTAACTTGTTTATTGCAGCTTATAATG GTTACAAATAAAGCAATAGCATCACAAATTTCACAAATAAAGCAATAGCATCACAAAT TTCACAAATAAAGCATTTTTTTCACTGCATTCTAGTTGTGGTTTGTCCAAACTCATCAA TGTATCTTATCATGTCGAGCTAGCTAGTCAGGACTTCAGTTCCAGCTTTGTGCCGGC TCCGAAGGTCAGCGGGTTGCTAGACCATTGCTGACAGTAATATGTGGCGGCATCCT CGGCTTCCATAGAGCTGATTGTCAGGCTGTAGCTGGTGCCAGATCCAGATCCGCTG AATCTGTAGGGCACGCCGGAGGCCACTTTGCTTGTGTCGTAGATCCACCGCTTGGG GCTTGTGCCAGACTTCTGCTGATACCAGTTCATGTAGGACACGCTGCTGCTGGCTCT GCATGTCATGGTCACTTTCTCGCCTGGGGAAGCGCTCATGATGGCGGGGCTTTGTG TCAGCTGAATATCGGAGCCACCACCACCGCTTCCACCACCACCTGAACCGCCTCCG CCACTAGAAACTGTCAGGGTTGTGCCCTGGCCCCAGTAATCCAGACAGTAGTGGTC GTCGTAGTACCGGGCACAATAGTACACAGCGCTATCTTCAGAGGTCAGGCTGGACA GCTGCATGTAGGCGGTAGAGCTGCTCTTGTCGGTGGTCAGTGTGGCCTTGTCCTTG AACTTCTGGTTGTAATTGGTGTAGCCCCGGCTGGGGTTGATGTAGCCGATCCATTCC AGTCCTTGGCCAGGCCTCTGCTTGACCCAGTGCATGGTGTACCGGGTGAATGTGTA GCCGCTGGTCTTGCAGGACATCTTCACAGAGGCGCCAGGTCTAGCCAGTTCGGCGC CAGACTGTTGCAGCTTGATATCGGATCCGCCACCTCCGCCAGATGACACTGTGACC AGTGTTCCTCTGCCCCAGTAGTCGAGGCCCTGTCTGGCGCAATAATACACGGCGGT GTCCTCGGCTCTCAGGGAGTTCATCTGCAGGTACAGGCTGTTCTTGGCGTTGTCCC GGCTGATGGTGAATCTGCCCTTGATGCTGTCCAGGTAGTAGGTGTAGCTGCCGCCG CTGCTGATTGTGGACACCCATTCAAGGCCTTTGCCAGGAGCCTGTCGGACCCAGCT CATATCGTAGCTGCTGAAGGTGAAGCCGGAAGCGGCACAGCTCAGTCTCAGAGAGC |
| | | CGCCAGGTTTCACAAGTCCGCCGCCTGATTCCACCAGCTGCACTTCAGATCCACCA CCGCCACTACCTCCGCCTCCGCTTCCTCCGCCGCCAGATCCTCCGCCACCTCTCTT GATTTCCACCTTGGTGCCTCCGCCAAATGTCAGGGGATTGCTGGACCACTGCAGGC AGTAGTACACGGCGAAGTCCTCAGGTTCCAGGCTGCTTATGGTCAGGGTGAAATCG GTGCCGCTGCCAGAGCCAGAAAATCTGGCAGGCACTCCGCTGGCCAGATTGCTGGT CAGGTAGATCAGGATTCTAGGGGCCTGTCCGGGCTTCTGCTGCAGCCAGTAGATGT AGTTGATGGAGCTGCTGGCGCTACAGCTCAGGGTAGCTCTCTCGCCAGGGCTCAGA GACAGTGTGGCTGGAGACTGTGTCAGCACGATCTCGCCGGTGGTATCAGGCAGCCA AAGCAGCAGCAGGAACAGCAGCTGAGCAGGGGCTTCCATGGTGGCCCTCCTTCGC CGGTGATCTCAGCTGTAGGAAAGAGAAGGTTAGTAGTCGACGTGTCCCTCTCG ATGAATCTAAGTATCAATTGTGAGCGCTCACAAGTCAACACTCTTTTTGATAAATCTA GTAGATATCACTTACGTAGGCGCCGGTCACAGCTTGGATCTGTAACGGCGCAGAAC AGAAAACGAAACAAAGACGTAGAGTTGAGCAAGCAGGGTCAGGCAAAGCGTGGAGA GCCGGCTGAGTCTAGGTAGGCTCCAAGGGAGCGCCGGACAAAGGCCCGGTCTCGA CCTGAGCTTTAAACTTACCTAGACGGCGGACGCAGTTCAGGAGGCACCACAGGCGG GAGGCGGCAGAACGCGACTCAACCGGCGTGGATGGCGGCCTCAGGTAGGGCGGC GGGCGCGTGAAGGAGAGATGCGAGCCCCTCGAAGCTTCAGCTGTGTTCTGGCGGC AAACCCGTTGCGAAAAAGAACGTTCACGGCGACTACTGCACTTATATACGGTTCTCC CCCACCCTCGGGAAAAAGGCGGAGCCAGTACACGACATCACTTTCCCAGTTTACCC CGCGCCACCTTCTCTAGGCACCGGTTCAATTGCCGACCCCTCCCCCCAACTTCTCG GGGACTGTGGGCGATGTGCGCTCTGCCCACTGACGGGCACCGGAGCGATCGCAGA TCCTTCGATAGAGAAATGTTCTGGCACCTGCACTTGCACTGGGGACAGCCTATTTTG CTAGTTTGTTTTGTTTCGTTTTGTTTTGATGGAGAGCGTATGTTAGTTACGATTCACAC AAAAAACCAACACACAGATGTAATGAAAATAAAGATATTTTATTGCGGCCGCTCGAGT CTAGAGGTATACATGCCATGGTCCGCGGTATTACCCTGTTATCCCTAAGACAACTGT CCTGCATGCGACGTCGGCGCGCCATCGAATTCCTGCAGCCCGGGGGATCCCTTTGT TGATTTTTTCCACATAGATTATTTTTGACTGTTTTGGCACTTTATATAAATGGAATCATA TAGTAAATATATACATGTATATATGTATATATACACTATATATGTATATATATAGTGTAT ATATATACATGTATATATGTATATTTACATATATACTGTATATATGTATATTTACATATAT ACTGTATATATGTATATATACACGTATATACTGTATATATACAGTATATACTGTATATAT ATACTGTATATATGTGTATATATATATACAGTATATATACAGTATACATATATATACATG TATATATACACAGTATATATACATGTATATATACACAGTATATATACATGTATATATACA CAGTATATATACATGTATATATACACAGTATATATACACTGTATATATGTATATATATAC TGTGTATATATATACAGTATATATACAGTATATATATACATGTATATGTATATATATACT GTATATATGTGTATATATACGTATATATACTGTATATATATATAGACACTTTTGTGTCT GGCTTCTTGCTCTCATCATAAAGCACTTGAAATCCATCCATGTTGTAGCTGGTATCAG TAGCTAGTTTCTTTTCATTGCTGAGAAGTATCACTTTTTATTGATGAGTAGTTTTACAT TGTATGAACATGCCCGAGTTTGTTTATGCATTCTACTAATAGACACCTGGGCTGTTTG CAGTTTTTGGCTATTACAAAGAAAATTTCTATATTTTTTTCCAACTTTTATTTTAGGTTC AGCGGGTACATGGGCAGGTTTGTTACATCGGTAAATTGCATGTCACTGGGGTTTGGT GTACAGATTATTTCACCACCCAGGTAATAAGCATAGTACCTGATAGGCAGGTTTTTGA TCCTCACCCTCCTCCCTCAAGTAGGCCCCAGTGTCTATTGTTCTATTCCTTTTTTTTTT TTTTGAGATGGAGTCTCACTCTGTTGCCCAGGCTGGAGTGCAGTGGTGCGATCTCG GCTCACTGCAAGGTCCACCTCCAGGGTTCAAGCGATTCTACTGCCGCAGCCTCCCG AGTAGCTGGGATTACAGGCACCCACCACCACGCCCGGCTAATTTTTGTATTTTTAGT AGAGACTGGGTTTCACCATGTTGGCCAGGATGGTCTCGAACTCCTCACCTCAGGTG ATCCGCCCGCTTCGGCCTCGCAAAGTACTGGGATTACAGGCGTGAGCCACGGTGCC TGGCCTATGGTTCCATTCTTTGTGTACACGTGTACTCAGTGTTTAGCTTCCACTTGTA AGTGAAAACATGCAGTATTTGGTTTTCTGAAATTCTTGTCTTCATCTTTTTGTAGACAA TCCACTACTTTTATTTTTAAATATTAGGAACAATAGCCTCTGTGTGTCCTGAATTGCAA TGTTTTTTCTCTGATTTTGCTGCTCATCTTGTAACCTGTATGGTGTTTTTCTGTGTGCA AGCTTTTTACTTTACGTGGGCAATTTTATCATTCTTTTCTTTTATGGTTTCTGGGTTTC ATGTCATGATTGGAGAAGCTTGATCTACCCTGATACTATAAAAATATCCACCTTGGCT TTTTTTTTTTTTTTTAGATGGATTTTCACTCTTGTTGCCCAGGCTGGAGTGCAATGGC GTGATCTCGGCTCACCACAACCTCTGCCTCCCAGGTTCAAGTGATTCTCCTGCCTCG GCCTCCTGAGTAGCTGGGATTACAGGCATGCGCCACCACGCTCAGCTAATTTTGTAT TTTTAGTAGAGACAGGATTTCACCATGTTGGTCAGGCTGGTCTTGAACTCCCCACCT CAGGTGATCTGTCTGCCTCGGCCTCCCAAAGTGCTGGGATTACAGGCGTGACCCAC CACACCTGGCCCACGTTGTTTTCTGATAGTGTTTTCACGTGTTATTTTTTCTGTCTTAC TCTTTGAATCATCTAAATGTATCTTAATGTCAGGAGTGAGGTAGAGGACCCAGTTGTT TGGTTTAAATGACTAGCCAGTTTTCCCAACACCAGGTGTTGAACATCCCTCACTGAT GTGAGATGCTGCCTTTTTCACAGTCTCAATCCCCATGTGCATTTCTATTTATTTTCCTT CCACTTTATTCACATCTTTTTCAGTATCCACAACCATATTGTTTTAAATAGCTTCATGA TATGTTTTAATATTTGGTAGAGCTAGAACCCTTTCTTGGTTCTTTTTCTTTTCTTCTTCT CCTCCTTTTCCTCCTCCTTCTTTCTCCTCCTCCTTCTTCTTTCTTTTTAGAAATTTCCT GGCTATTCTTGTTTTGTAGTCTTCCAGATAAATAATTTTGAATCACTTTGTCATGTTCC AGAAAATAATACTGCCAGAATTTTTATTGTGATCACAGAAGTTTTACAGATTCATCTG GGGGAAAAAAACCCATCTTCCCAGAATACAGAAAGGGTGAGGGAAAGACAAACATC AAGGACAAGTCTCAGGTTCTGGACTTGGAGACCAAGAGGATTTGGGGCATCCGGGA GCAGGGCAGGGAGGTGTGATGGCTGCCTTTCACTGAGTAGGAGGATGCCCAGACTT |
| | | GGGGGAACTATGGAAAGTTTGGTTCAGACTTTCTGAGTTGGAGATGTCTATGCGCAG TCCACAGGGCGAAGCCCTGGAGGTAGAGGCCCTCCTTACTGCCTCCCTCATTCAGT CTGAGGTCCATTCTCCACATGGACACTGAAGTGACTCAAGGCTTCCCTTGTTGGATC AGGATAGTCTCCAGGGCCCTGGGTGGTCTGGGCCTGCCCACCTCCCCATCCCCTCT CCTGCCTGCCCTCCTCTTGCTCACTGGACCCCACCCACCAGCCTCCTTCTATTCCTC CTGACATTTGCACTTGCTGTTCCGTCTCTGCTTGGAACATCCTAGCTCTTTGCATGG CTTGCTCCTTCCCATCTTTTAATCTAAGCTCAAATATCACTCCTTCAGATATGCCTTTC CCAAATATCCTAGCTAAAGAGAACTGCTCCCCACTTCCCACAAGCCCTAGCAAACTG GGACTCTCCTTCTTGCTTTCTTTCTCTCTGCCCCTTCTGTGTTTTTTCCATAGACCTTA ACTTGTTACTGTTTTATTTGTTGATGTGTTTACATCATTTGTCCCCCTGTGATGGTTAG TTTTATGTGTCAACTTGGCTAGGCTGTAGGGGCTGTTCGTCAATCAAACACTGATCTA TGTGTAGCTGTGAAGGGATTTTGTAGCTGTGATTAATAGTTGACTTTAAGTAAGGAAG ATTATCCTGGAGAATGTGGGGGAAGGGGCAGACCTCAGCCAATCAGTTGAAAGGCC TTAAGAACAAAGCTGAGATTTCCCCGAGGAAGAAGAAATTCTGCCTGTGGACTGCAG CATCAGCTCCTGCTTGAGAGGTTCCAGGCTGCCCTTCCTGACAGCCTGTCCTATGAT TTTGGACTTGCCTTGCCAGGTTTTTCCTCTTTCTTGACCCCCCAAAACCATATATATC TCCTGCTGGTTTGTTTCTCTGGTGTAATCCTGAGGGATGAACACTCCTCCCTCCCAG TTCACTCTGGGAGAGGTTGGGGATCATGTTTGTCTATTTCCCCATGCCTGGCCCATG CGTGTTTATTGAATCACTGAACAACCACGCATTAGGAAGCCAGTCATATGTGCTTCTT CAGAACCTCATGTCCATTGCCAGATCTCCCTGGCTTTTGTGGCTAGAGGACAAGTGA GAGATAGTAGCTTACCCACAGACCTTGGCCCTGAGGCCCCCAGGGGCCTGAGCCT GCTGTAAGGGAGGAGGGAGCCCCTGAGGTCTCACACACCTCCCTGGGGATCTGGC ATTTTCCCCTGGGGCTGGCCTCAGAGCTGGGCGGGGGCAGATTATGGAGTGGGTT GTAGAGGGAGCACCTCCCCACTAGCAATCATGGTTTTTTCTGCGCCTCTCATAGGGA GGCCTACAGGCCTCCACCTTCAGCTCTTCAGGAGCCACAGGCAGCTGAGTGACTTC TGCATCACAGTCTTCTCAGAGACAAACTTGCAGTAAGAAAGAGGGGCCCGTGAGGG ACCCCAGAGAAGGCTGTTGTCAAAGCAGGATGAGAGTGAACTCTTCCATGGGGGAC ACCCAGCGTCTCCAAGCTCTTTTATGCTGTCTTCAAGGGGTCTAGAGAGCTTCAGCC CAACATGAGACCCAGTCCAGCAGCATTTCCCTGCGAAGTGAAAGTTAGGACCCTGA CTAGATACACCACGCTGACCTCAGCCAGAATATCAAGATGCTGAGGCGCTGAGATG CTGGGATGCTAAGGTGCTAAGGTGCTGGGGTGCTGGGGTGCTAGGATGCTGAGGT TTTGTGATACTGGGTCGCTGAGATGCTGGGATACTGGGGTGCTGAAGTGCTGGGGT ACTGGGGTGTTGCCATGCTGAAATGCTGGGGTGTTGGGATGCTGAGGTGCTGGGTT GCTGGGATGCTGGGATAGTCCTTGGATGCTGCGGTGCTGAGATGCTGGTCTGCTGG TCTGCTGGGGTGTTGGGATGCTGGGATTTTGGGATGCTGGAGTGCTGCAGTGCTGC AGTGCTGAGATGCTGGAGTGCTGGGGTGCTGGAATACTGTAGTACTGGTGTGCTGG AGCATTGAGATGCTAGGGCACTGGGATGCTAAGGTGCTGAGATGCTGCAGTGCTGG GGTGTTGGGATGCCGAGGTGTTGGGATGCTTAAGTGCTGGGGTGTTGGGATGCTG GGGTGCTGGAACACTATGGTGCTGGGGTGCTGGAGTGTTGAGATACTGTAGTGGTG GGATGCTTAAGTGCTGGGGTGCTGGGTGTTGGGATGCCTAGGTGCTGGGGTGCCG AGATGCTGGAGTACTAGTGTGCTGGGATGCTGAAGTGCTGGGGTCCTGAGATGCTG CAGTGCTAGGGTGCTGAGATTCTGGGCTGCTGGAGTGTTGGGGTGCTGGGATGCT GGAGTGCTGAGATGCTTGGACAATGGGGTGCTGGAATACTATGGTGCTGGGGTGCT GGGGTATTGAGATGCTAGGGTACTGGGATGCTGAAGTGCTGAGATCCTGGAGTGCT GGGCTGCTGGGCCACAGGCTCTTGAATCCATTCGTCTGCCCAGGGGAAGAAACCAG AAGATAAAGAGCTAATGAAGGAGCTTTGGTTGAGAGGGAGGAAGTAATGGAAGGAG CAACATCTTGTGGAGGAGCAGGAGAGAATGGACCTCAGGTTGGGAGAGAGGGCCA GGCTACAGGCCAGAGAGGCAGAAGGATTCCAGCAGAGTGTGGGCTCCAGGAGCCA AGGGGAAACAGGTTTCTGGGAGGAGAGAGTCCAGTACTGCTGAAGTGGCAAGTCCG CTGAGGACCAGGAAGCTTCATTTGGCTTTATGACCAGGAGGAATTTGGAACTGTGAC TAGAGTACTTAGGGGGAAGGAGGCAAGACTGGAGCCAGATTGCTCTGGGTTGAGG GGTGAGTGGGAGGTGAAGCAGGGCACTGTCACTCCTTTGAAGGGTGGCAGAGAGC TGGAATTGGTGCTGGATGGGCTGTGGGGTGACAGGGTCATGTGGAAAGCCCCTGG GGGGCACCTGGAAAAGGAGAAGCTGACAGTACAGTGAGAGGACAGCTAAGGGAAA GCGGAATGGCAGAACACGCACTGCCAGGAGGAATGAGGATAGGGTCAGGAGTGCC AGGGGCAGTGAGGCCAGCCTGGGGTCAGGTGGCAGGACGTGTCCAGGAAGCTGGT CTGCACTGCAGCCCACACTGGCTCAGCCTTGAGGTTCCCTGTGTGGTTGGGGTAGG AAGTTGAACCCTCTGGGAATGGAAGATGGAACCAGCTCTGCGAGCCAAGCTCAGCT TTTATCTATGGGTCTCTGAGGGCTGGCAGAGCTGAGTGGGGACAACTGTGATCCGT GAGGCTCTCAGGTTGAGGTGGCCCCTCCGGGAGGGCTTCATTTTCCCAGCGGGTA GGTTCTAAGCAGCAGTGGCTGGGCAGGTGGGTCCAACACAGAGCCAGAGAAGGGT GAATGGGCCTCCTGGCACCCCACCCCTGCTGCCCCTGAGCTCAGTGATGGAGGGG GACAGCACAGCTGAGCCCAAGTGCTTTGGTGTGGCCCTGAGGGAAAGCTGCAGCCT GCCTGGGGCCTGGCATGGATGGGACACTTGAGGCAGAGGGACAATAGTGGGCGCT GCAGTGAGGCTGGCTCTTGGAGAGGTTTCCTGAGGAGTGCTGCCTGAGACGGGCA GGGAGAACAGAGACAAAGTTGGTGACAGGGAATGAAAGCTGACTGAAGGACTTTAC CCAGACCTATGAGGATATCTCTCTCAGCAGGAAGCAGGAGGGGACTGTGTGAGGAC TGGCCAAGAGCTGGAGTGTTGGGAAAATGACTCTTTCTCCGACCCCTCTGTCCTAGC TCTGGCCCCTGGACTGCGGAGGTCTGCTTCCACCCCCATTGGTCGATCGTTGTCCC TTGTCACAGCCATTGAGAATTTTGGCAGGGAGCATGTTCTTAGAGCATTTTTAGGCT |
| | | CTGCGGGACATAACAGCTCTGCCTCAGAGCACATGCCTTTCTCAGCTCCTGAAAGC CACTGATCAAATTGGAACATTTTGTACCTTAGGGATGAGGATATCAACTCTCCCAGC CACTTAGAGGGATAAATGTGATGATGCATTCAATTGTGACTACATCTGATCCCAACTG TTGCTTCAGCTGCTCTCCTATAGCACATGGCGGGAGGCGTGCATCCCAGTAGCTAC CTCCCCACTTTTGGGGAGATGTGGTTCCATCCATGAAACCTGGGTACCCGCCTACCA GGTCCTGGCCTATCAGGTGGCAGGGTCTGGTCAAAGAAGGGCATGTGTGGTCTTCA GCAAGGGAGACAGGACGGTGGTGCAGAGCGTCTAGACCCTCAGGGCAAGTCTCCC CCACACCTGCTCCCGGGGCAGTTGTCTTTGTGACCTCCCATCCCCCTCTGTTTCATC CTCTATAAAATGAGGGGCTGAGCCCCAAAATAACAGGCTTCTTTGCCATGATGCAAA ACTGCTGAATCTTTCTTTCTGACACACAAGGCATCGAGCAGCCTCTGAAAGAACCAA AGCCACTAGCAGGCTTCCTGACTTGGGTTTGTAGGTACTGAATACTCCCTTGAAAAA TAAAAACATAGAGGCACTTTTCTCCTGGCTGTTTATTACAGAACGAAGAAAAAACACA CTGGCTTGAAACAGACGCCAGATTTCAAATGTAGAGGTGAAATACGAGGTGGCAATT AAAATGTGATTACAGAAAGTCTGGACACTGAGAAAAGTTTACAGGACAGTGGGTGTG GGTTTTCTATAACAGACACTTAAATATACATGACGATAATTGCAGATAGAAACCATCA AAGACAAACCCCAAATCAACTAATAATGTTTACAGATGTTCCCCCCCAAACCACAGA GCCTTACATCAAAACAAATACTGAAAGGCTTTAAACCAGGAACAGCTCGCCTTAACC CCACGAGGGTGCACACAAGCTGGGCTTTTTCTCTCGGTCTGAATGGTAAAGGGAGG AGGATACTCTAGCTCCTCCAGGTGGATTGCTGAGACAGGGCTCGGCTCACACACTG TCTCTGCGCCTCTCCCAAATCTGGAGAACTCTCCCAGCCTCCTGGTAAAGTGTCTCT GTGGGGCACTTAACGATAAAACAGCTTCTGCTGTAAAGCTCATTAGGAAAGAGCTAG CCTGCAACCTCCACGGAGTGGGAACTACATCAGGCATTTTGCTAACTGCTGTATCCT AGGCCAATAAATGTTGATCACATTTATAGCTGCCATGGTAGGGTGGGGACCCCTGCT ATCTATCTGTGGAGGCTCTGGGAGCCCCTGACACAAACTTTCTGAAGCAGAGCCTC CCCAACCCCTTTTCCATTCCCTATACCTGACAGATGGCCCAGGAACCCATTAGAAAT GGAAGGTCACTGCAGCAGTATGTGAATGTGCGTGTGGGAGAAGGGCAGGATCAGA GCCCTGGGGGTGTGGCAGCCCCCAAGTGATTCTAATCCAGATCCTAGGGTTGTTTC CCTGTCCCATTGAAATAGCTGCTTTAAGGGGCCTGACTCAGGGAAATCAGTCTCTTG AATTAAGTGGTGATTTTGGAGTCATTTAGACCAGGCCTTCAATTGGGATCCACTAGTT CTAGAGCGGCCGGGCCCAGGGAACCCCGCAGGCGGGGGCGGCCAGTTTCCCGGG TTCGGCTTTACGTCACGCGAGGGCGGCAGGGAGGACGGAATGGCGGGGTTTGGGG TGGGTCCCTCCTCGGGGGAGCCCTGGGAAAAGAGGACTGCGTGTGGGAAGAGAAG GTGGAAATGGCGTTTTGGTTGACATGTGCCGCCTGCGAGCGTGCTGCGGGGAGGG GCCGAGGGCAGATTCGGGAATGATGGCGCGGGGTGGGGGCGTGGGGGCTTTCTC GGGAGAGGCCCTTCCCTGGAAGTTTGGGGTGCGATGGTGAGGTTCTCGGGGCACC TCTGGAGGGGCCTCGGCACGGAAAGCGACCACCTGGGAGGGCGTGTGGGGACCA GGTTTTGCCTTTAGTTTTGCACACACTGTAGTTCATCTTTATGGAGATGCTCATGGCC TCATTGAAGCCCCACTACAGCTCTGGTAGCGGTAACCATGCGTATTTGACACACGAA GGAACTAGGGAAAAGGCATTAGGTCATTTCAAGCCGAAATTCACATGTGCTAGAATC CAGATTCCATGCTGACCGATGCCCCAGGATATAGAAAATGAGAATCTGGTCCTTACC TTCAAGAACATTCTTAACCGTAATCAGCCTCTGGTATCTTAGCTCCACCCTCACTGGT TTTTTCTTGTTTGTTGAACCGGCCAAGCTGCTGGCCTCCCTCCTCAACCGTTCTGAT CATGCTTGCTAAAATAGTCAAAACCCCGGCCAGTTAAATATGCTTTAGCCTGCTTTAT TATGATTATTTTTGTTGTTTTGGCAATGACCTGGTTACCTGTTGTTTCTCCCACTAAAA CTTTTTAAGGGCAGGAATCACCGCCGTAACTCTAGCACTTAGCACAGTACTTGGCTT GTAAGAGGTCCTCGATGATGGTTTGTTGAATGAATACATTAAATAATTAACCACTTGA ACCCTAAGAAAGAAGCGATTCTATTTCATATTAGGCATTGTAATGACTTAAGGTAAAG AGCAGTGCTATTAACGGAGTCTAACTGGGAATCCAGCTTGTTTGGGCTATTTACTAG TTGTGTGGCTGTGGGCAACTTACTTCACCTCTCTGGGCTTAAGTCATTTTATGTATAT CTGAGGTGCTGGCTACCTCTTGGAGTTATTGAGAGGATTATAAGACAGTCTATGTGA ATCAGCAACCCTTGCATGGCCCCTGGCGGGGAACAGTAATAATAGCCATCATCATGT TTACTTACATAGTCCTAATTAGTCTTCAAAACAGCCCTGTAGCAATGGTATGATTATTA CCATTTTACAGATGAGGAACCTTTGAAGCCTCAGAGAGGCTAACAGACATACCCTAG GTCATACAGTTATTAAGAGAAGGAGCTCTGTCTCGAACCTAGCTCTCTCTCTCTCGA GTAATACCAGTTAAAAAATAGGCTACAAATAGGTACTCAAAAAAATGGTAGTGGCTGT TGTTTTTATTCAGTTGCTGAGGAAAAAATGTTGATTTTTCATCTCTAAACATCAACTTA CTTAATTCTGCCAATTTCTTTTTTTTGAGACAGGGTCTCACTCTGTCACCTAGGATGG AGTGCAGTGGCACAATCACTGCTCACTGCAGCCTCGACTTCCCGGGCTCGGGTGAT TCTCCCCAGGCTCAGGGGATTCTCCCACTTCAGCCTCCCAAGTAGCTGGGACTACA GGTGCGCACCACCATCCCTGGCTAATATTTGTACTTTATTTTATTTATTTATTTA TTTTTTGAGATGGAGTTTCGCTCTTGTTGCCCGGGCTGGAGTACAGTGGCATGATCT CGGCTCAGTGCAACCTCTGCCTCCCGGGTTCAAGCGATTCTCCTACCTCATCCCCCT GAGTAGCTGGGATTACAGGCGCCTGCCACCATGCCTGGCTAATTTTTTGTATTTTTA ATAGAGACGAGGTTTCACCATGTTGGCCAGGCTACTCTCGAACTCCTGATCTCAGGT GATCCACCCGCCTTGGCCTCCCAAAGTGCTGGGATTACAGGCGTGAGCCACTGCGC CCGGCCTAATATTTGTATTTTTTGTAGAGATGGTGTTTTGCCATGTTGTCCAGGCTGG TCTTGAACTCCTGAGCTCAAGCGATCTGCCCGCCTCTGCTTCCCAAAGTGCTGGGAT TACAGGCATGAGCCACCGTGCCTGGCCTAGGTAGACGCTTTTAGCTTTGGGGTGTG ATGCCTGCCCCAGTATATAGTGAATTTAATTATTGCTAGAGCTGGCTGTTTGTTAGTT TTCTTTGAACATAAGATACTCATTGTTTTTAGTTTGCAAATCCCTCTTCCTTTTTAAAAA |
| | | ATTTCTTTCCCTTAAATTGTTTGCATGTTAGCAATAACAAATGCTTAAATGGTGCTATG TGCTAGATACTCTTCTAAGCCCTGTTATGTATATTAACTAATTTTTTAAATTACACAAAT CAGAGAGGTTAAGTAACTTGCCCAAGATTACCCAACAATACTAGGATTTGAACCTAA GTTTGTCTCACCCCAGATTCTGCTCTTAATCTCTAAACTTTTAAGTTAGTAGTGACAAT AGTAGGTATTTATTGAATACTTAACTATGTTTTAGGCGTTGAAGTAAATATTTTGCAGG CATTATCTAATGTAAACACCCTAAAGTTACATAACAGGTACCCTTTAGGTAAATAAAC ACTAGTATGACCTTGGAGGCACAGATAGTTGAAGTAACTTGCCCAATATCACTTACAT GAAATTGGCCCTCAAATGTGTCTGATACAACCCATGCTGCTTGTAACTATCGTTTTAA ACTGCCAGGGTAAACTTGGACACACTTGAGCTAAGAAAAAGCTTTTAGATTTTTGCAA ATTAATGTGAAAGATATGCTTTATGTGGATATAATATCTTCTAAATTTCGGGGATGGTA GTCCTAGAAATGTAATCCTGCCCTAGCCGAGCTTACCCTGCCAATAATTTTTTACAGA ATTGGTAAAACGGAGCACCTTTTTTTTGTCCTTGGCCACACTGTTATCAACAGGGTGT AGATTGACATCAATCTGTAGGTGTAAACCAGAATTACTCTTTGTGACCACCAGGAAAT AGAGCAGTTCAGTTCAGGGGTTTCTTTCTGTGAATTTAGCACTGTGACCTGCATACTA CAAGTCTACTTTGTTTTCTATCCATTGTTTGTATCTGGGTATTGCAAAAGGTAGGAAA AGGACCAACCAGATCAGCAGAGAAGAGTTGCCTTGGAGTTTTCTTTTAGTTTTCTGC AGTTCATTAGATAGTAACTAGGCCATGTCATTTTACTCCCTTGTAGTGAAGATATGTT GAAGTTGTACTGGTATACTCTTCTACCTTTCTGTAATTTTATATTGTGTAGACTTGATA AAATTTATGTGTCAATCACCACCATTAATATCAATATTGAGCCTCAATTCTTATTTTTCT GCCCAGTGGCTGCCAAATTACTAACATTTACAATAATTCACTACTACTAAGATAATCT ACTAGTTCGATCACATACTTCAAATTGTTATGGAACTACTGTCTTCAGCATTGTGCTT CTGATAACTGATAAGTATAATTTTTTTTTTGTCCAGAGTGAACATGTCTATTCTTCCAC TGTACACACTAATAAAAGGAAAAATTGTAATATTGGGTAAATTCATGTCCTTACACAT GTAGTAGTTATGAGCCCATGTCCCTAGAATGAGTAATAATTTATCCCTCCCTTGGTTG AATAGTCAAGAATGCTGATTTTAATTCTTCTAACAGCTTTATCCCTCAGAAGGGAAGG CAAGCAAGTTATATATGTAGTTTATTTGTAAGACTGATATGAAATTGGAAGATGAATCT ACTATTAGCTTTAATTATTTTTACATTTAGGAATATTGCATCAGTAACTCATAATTTTGG TTTTCTGTTATCCTGAGTTAACACAAATTATCCAAGGAGATGGCGGATCATCTGCTTT GAGGTGTTTTTTTTTGAGAATTTTAATGTATCTGAATATAAAAGGTAAAAATATGCCAA CTAGCAATTTCTGCCCATTCCAGAAGTTTGGAAATATTACTCATTACTAGGAATTAAAT AAAATATGGTTTATCTATTGTTATACCTCTTTTAATTCACATAGCTCATTTTTATCTTTT ATTTTTGTTTGTTTTTTTTGAGATGGAGTCTTGCTCTGTCACCAGGCAGGAGTGCAGT GATGCAAATCTCGGCTCACTCTAGCCACCGACTCCCTGGTTCAAGCGATTCTCCTGC CTGAGCCTTCTGAGTAGCTGGGATTACAGGCAGGCACCACCACGCCCAGCTAATTT TTGTAGAGACAGGATTTCACCGTGTTGGCCAGGATGGTCTCCATCTCCTGACCTCAT GATCTGCCTGCTTCGGCCTCCCAAAGTGCTGGGATTACAGGTGGGAGCCACTACGC CTGGCCCACATAGCTCATTTTTAGACTCACTTCCATTAAGTCTTGTTTGGACCCACGA ACATTGTCTTTTTTTTTTTAAGATGGAGTTTCACTTTTGTTGCCCAGACTGTAGTGCAA TGGTGCAATCTCAGCTCACTGCAATCTCTGCCTCCTGGGTTCTAGCAATTCTCCTGC CTCAGCCTCCCGAGTAGCTGGAATTACAGGCGCCCGCCACCACGCCCAGCTAATTT TTGTGTTTTTAGTAGAGACGGGGTTTCACCATGTTGGGCAGGCCAGGGGTGATCCG CCCACCTCAGCCTCCCAAAGTGCTGGGATTACAGGTGTGAGCCACCGCATCTGGCC AACATGTCTTTTTTTTTTTTTTCCTTTTTAACCACAAAGAGACTTAAGCAGTCCTTGTC ACAGATGATGAATTGATGTTGCAAGTATTGTCTTAGCTTGGATTAATTTTCTTGCTTAC TGTAATTTTAGATAATATAGCTTTGTAATTAGAGATTTTATGTGTAAACCACAAAAATG TTTACATGAAGGCCATTATTACAGATGTGACGTGCATAATTATTAGTAATTTGTATGTT TACATGGGTCAGTCTGGCAAAAAATTATGAAGTTTTAAAAATTAAAAAAAATTATAATG CCAGTTTTACTGGAAAGTAAAATTATTTCAGTAATCGATTATAGCAAAAGTATTGATTT TCATTCCAGACAACTGTCTGTCTATAGCCAATTATGCATTTCTTAAATTAGAACCCCC CCAATATACCCAAATATATATATATGTGTGCATATATATAGTAAGTTGTAACAAAGTTG TGAATTCATACCTGAAGTATCTCAAGTGATGCAAGTTTTATGAATTTTTGTTTATGCCT TTTGGGAAGAGTTGTATTGACAAATTTTTTATGCTTAAAGTAAACCATAAATCAAAAAA ATAAAATCTAGGATGCAATAAAACAAAACAACTTCTTGACATAAGTATGGTATGTAAAT CTGTTTTGATTGGAAATCAATTTGTTATATTGCCAGAATTCCTGTTTTAGAATACATCT CTGCTGATCTGTCTGTATTCTTAGACTGCATATCTGGGATGAACTCTGGGCAGAATT CACATGGGCTTCCTTTGAAATAAACAAGACTTTTCAAATTCTTAGTCGATCTGCAGAA CCTGTAGCCAGGCACTGAACCATTTTGATAGATGCAGTAATCGTTGCAAGTGTATATT TCAAGGGAGTTCTGGCTGGGTCCTAGTTTATGCTTGTGGCAGAAGCAGTGAGTAACT GGGAGGAAGTTGGTGAGTAAGCTTCAAGGAAGAAGTCATTTTTAGTACTCTGGATCT TCCTGATTTTAAAGCACTACAAAATGGTGCATTTTCATTCTTGTCAAGTGATAACAGAT ATATTCTGATGAGCCTGAAATGAATATATATTGTATCATTTTTATAATATCTAGCAAGG TTTGTATTTTCCTAGAACTTGAACTAAATTTCAGTTCATAAAATTTATAAAATACTTAGT TGTTGTAAAATATTTTTGGAATGTTCACATAGGTGACACACAAATGTCCCATTTTCATT CTTTCTATAGTAAATATGTTCTGATATGTGAAGGTTTAGCAGATGCATCAGCATTTAAT CCTAGAGGATCTGGCATAATCTTTTCCCCCAAGAATAGAAATTTTTTCTGCTTATGAA AGTAGTACATGTTTCTTTAAAAACAAATCAATATTGACTTCTGCCTGCTGTATAGCACT ATGCCTCCACCTGGCCATGACCAGGGGCATGTCCTGGTCCACCTACCTGAAAATGT TTGCAACCAGCCTCCTGGCCATGTGCACAGGGGCTGAAGTTGTCCCACAGGTATTA CGGGCCAACCTGACAATACATGAAGTTCCACCAAAGTCTGAGAACTCAGAACTGAGC TTTGGGGACTGAAAGACAGCACAAACCTCAAATTTCTCAGCACTGGAAACCTCAAAA |
| | | TATAACTGAATTCCATAAATAAGATTTTAAGTCTTAAATATGTATTTTTAAATGTATTAA AAGTCAAGCTGCTTGTATTTAAGCACCTAATACAATGCTTAGGTTGTAAAAGGAGATG CTCAATAGGTACTAACTGATATATTGAGATTTAATTATGGTTTGACCAATATTTATTGG AAACCGCCAAAGCTTAAATCATCAGCTTCTTGAATGTGATTTGAAAGGTAATTTAGTA TTGAATAGCATGTGAGCTAGAGTATTTCATTCTTTCTGGTTTATTTCTTCAAATAGACT TTGAATATAATGGTGAATGGGTATTATAAATTAACTAATAAAAATGACATTGAAAATGA AAAAATATATATATTAAAGTGTAGAAAGTGACCAGGCGTGGTGGCTCACACCTGTAAT CCAAGCACCTTGGGAGGCTGAGGCAGGAGGATCTCTTGATCCCAGGAGTTCAAGAC CAGCCTGGGCAACATAGCGAGACTTCGTCTCTAAAAAAAAAAAAGAGAGAGAAAAAA ATTTTTTTTATTTAAAAAAAGTGTAGAAAGTGTCAAGACCCCACTTCTTACCATTATTT GGTATATTTCTCTATACCCACCCACCCTTCCTCCTTACTCCCTCCCTCCCTTCCCAAT CTTTTTATCTTTTTGTATTCTGATTTTTTGTTTGTATATTTTGCTTTAATTTAATGTATCC TTTAAAAATTTCCCATACATTTTATATGTATATATAAAAACGCATGCTGCCAAAGATAA TTTATAAGAAAGACCATTGAATTTTTTTAAAAGTGATATATATTCATTGAAAAAAATTTA GAATATATAGCAAAGCAATAAAGAACTAAATAAAATTGCTGTAACTCCTCTTTCAAAG ATAAGTGCTTTTATGATTTTGTTGTATTTTTTTCTGTATATAGGTACATATATAGTATTT ATAAAGCTGTACTCATAGTACATTTTCACATCACAGGTACCATATCAGTGTTATTAAAT ATTTTGTATGCCAGGGGCTAGACATACCAAGACAACCAATATGTGGTTCTACTTAAAT AATATTAGAGTATCTTTTATGATGACACTTCATGAGTTGACTATAATAATCTTAGACTT CTAAGAGTTTGGGTTTTCAAAAGATCACTTAGCTTTTTTGGGTGATTTTTCCCCCTTA CTGTGAGATGAGAGAGGCTGTTTGGATTTGGGATTGGGGTAGCGGGGACAGCAACT TTTCTTTTCTTTTTCTTTTTTATTTTGAGGTAGGGTATTGCTGTGTCACCCAGGCTGGA GTGCAGTGGTGTGATCTCGGCTCACTGCAACCTCCACCTCCCGGGCTCAGGTGATC CTCCTGCTTCAGCCTCCCAGTAACTGGGACTACAGGCGCGTGCCACATGCCTGGCT AATTTTGTATTTTTAGTAGAGATGGGGTTTCACCATGTTGGCCAGGCTGGTCTCTAAC TCCTGACCTCAGGTGATACGCCCACCTGGGCCTCCCAAAATACTGGGATTACAGGC ATGAGCCGCTGCATCAGCCAGCAGTTTTTCTTGTGGTTTTTTTTGTTTGTTTTGTTTTG TTTTGTTTTTGAGATAGGGTCTTACTCTGTTGTCCACGCTGGAGTGCTGTGGTATGAT CGTAGCTCACTGCAGCCTCAAACTCCTGGGCTCAAGTGATTCCTTCTGCCTCCGCCT CCCGAGTAGCTGGGACTACAGGTATGCACCACCATACCTGGCAAATTTTTACAAAGT TTTTTGTAGGGACGGGGTCTTGCTACATTCCCCATGTCGGTCTTGAACTCCTGGCCT CAAGCAACTCTCCTGTCTCAGCCTCCCAAAGCACTGGGATTACAAGTGTGAGCCACC ACACCATGCCAGTTTTTCCTGTTCAGTGTGATATTTTATCTTGTTAGACTACAGTGTG TTAAAACTTGTTTTACTAAATTTTCAAACATACTCAAAAGTGGAGAGAATAGTATAATG AATACCCGTATGTTCATCACCCATGTTTAGAATATTATTAAATATAAAGATTTTGCTGC GTTTGTCTTAGCTCTTTAAAATTTTTCTTTTTCTCTTTGTGACCTAAAGGAAATTCCATA TCTTATCACTTTACTTCTACATTCTTGACTAAGATGACTAAGACATATAGTTACATGGT TTTTTGTTTTGTTTTTGTTTTTTAAAGACGAAATCTCGCTCTTGTCCCCCAGGCTGGA GTGCAATGGTGCCATCTCAGCTCAGTGCAACCTCTGCCTTCTGGGTACAAGCGATTC TCCTGCCTCAGCCTCCCAAGTAGCTGGGATTACAGGCTCCTGCCACCACGCCTGGC TAATTTTTGTATTTTTAGTAGAGACGGCGGGGGGAGGTTTCACCATGTTGACAAGGC TGGTCTGGAACTCCTGACCTCAGGTGATCCACCCGCCTCGGCCTCCCAAAGTGCTG GGATTACAGGCGTGAGCCACCGCGCCCAGCCTGTTTTTTTGTTTGTGTGTTTTGTTT TTTTTGAGACAGAGTCTTGCTCTGTTTCCCAGGCTGGAGTGAAGTGGTGCCATCTCA GCTCAGAGACAGAGTCTTGCTCTGTTTCCCAGGCTGGAGTGAAGTGGTGCCATCTT GGCTCACTGCAACCTTCACCTCCCAGGTTCAAGTGATTCTCCTGCCTCAGCCTCCCA AGTAGCTGGGACTACAGGCATGTGTCACCACACCCGGCTAATTTTTTTGTATTTTTAG TAGAGACGGGATTTCACCGTGTTGCCCAGGCTGGTCTCGAACTCCTGAGCTCAGGC AGTCTGCCTGCCTCAGCCTCCCAAAGTGCTGGGATTACACGTGTGAACCAACCCGC CCGGCCTGTTGTTTTCTTACATAATTCATTATCATACCTACAAAGTTAACAGTTACTAA TATCATCTTACACCTAAATTTCTCTGATAGACTAAGGTTATTTTTTAACATCTTAATCCA ATCAAATGTTTGTATCCTGTAATGCTCTCATTGAAACAGCTATATTTCTTTTTCAGATT AGTGATGATGAACCAGGTTATGACCTTGATTTATTTTGCATACCTAATCATTATGCTG AGGATTTGGAAAGGGTGTTTATTCCTCATGGACTAATTATGGACAGGTAAGTAAGAT CTTAAAATGAGGTTTTTTACTTTTTCTTGTGTTAATTTCAAACATCAGCAGCTGTTCTG AGTACTTGCTATTTGAACATAAACTAGGCCAACTTATTAAATAACTGATGCTTTCTAAA ATCTTCTTTATTAAAAATAAAAGAGGAGGGCCTTACTAATTACTTAGTATCAGTTGTG GTATAGTGGGACTCTGTAGGGACCAGAACAAAGTAAACATTGAAGGGAGATGGAAG AAGGAACTCTAGCCAGAGTCTTGCATTTCTCAGTCCTAAACAGGGTAATGGACTGGG GCTGAATCACATGAAGGCAAGGTCAGATTTTTATTATTATGCACATCTAGCTTGAAAA TTTTCTGTTAAGTCAATTACAGTGAAAAACCTTACCTGGTATTGAATGCTTGCATTGTA TGTCTGGCTATTCTGTGTTTTTATTTTAAAATTATAATATCAAAATATTTGTGTTATAAA ATATTCTAACTATGGAGGCCATAAACAAGAAGACTAAAGTTCTCTCCTTTCAGCCTTC TGTACACATTTCTTCTCAAGCACTGGCCTATGCATGTATACTATATGCAAAAGTACAT ATATACATTTATATTTTAACGTATGAGTATAGTTTTAAATGTTATTGGACACTTTTAATA TTAGTGTGTCTAGAGCTATCTAATATATTTTAAAGGTTGCATAGCATTCTGTCTTATGG AGATACCATAACTGATTTAACCAGTCCACTATTGATAGACACTATTTTGTTCTTACCGA CTGTACTAGAAGAAACATTCTTTTACATGTTTGGTACTTGTTCAGCTTTATTCAAGTGG AATTTCTGGGTCAAGGGGAAAGAGTTTATTGAATATTTTGGTATTGCCAAATTTTCCT CTAAGAAGTTGAATCATTTTATACTCCTGATGTTATATGAGAGTACCTTTCTCTTCACA ATTTGTCTCTTTTTTTTTTTTTTTTGAGACAAGGTCTCTGTTGCCCAGGCTGGGGTGC AGTGCAGCAGAATGATCACAGTTCACTGCAGTCTCAACCTCCTGGGTTCAAGCGATC CTTCCACCTCAGCCTCCTGAGTAGCTGGGACTATAGGTGTGCGCCACCACTCCCAG CTAATATTTTTATTTTGTAGAAACAGGGTTCGCCATGTTACCCAGCCTCCCAAAGTGC TGGGATTACAGGCATGAGCCACTGGCCCAGTTTCTACAGTCTCTCTTAATATTGTATA TTATCCAGAAAATTTCATTTAATCAGAACCTGCCAGTCTGATAGGTGAAAATGGTATC TTGTTTTTATTTGCATTTAAAAAAAATTATGATAGTGGTATGCTTGGTTTTTTTGAAGG TATCAAATTTTTTACCTTATGAAACATGAGGGCAAAGGATGTGATACGTGGAAGATTT AAAAAAAATTTTTAATGCATTTTTTTGAGACAAGGTCTTGCTCTATTGTCCAGGCTGG AGTGCAGTGGCACAATCACAGTTCACTCCAGCCTCAACATCCTGCACTAAAGTGATT TTCCCACCTCACCTCTCAAGTAGCTGGGACTACAGGTACATGCTACCATGCCTGGCT AATTTTTTTTTTTTTGCAGGCATGGGGTCTCACTATATTGCCCAGGTTGGTGTGGAAG TTTAATGACTAAGAGGTGTTTGTTATAAAGTTTAATGTATGAAACTTTCTATTAAATTC CTGATTTTATTTCTGTAGGACTGAACGTCTTGCTCGAGATGTGATGAAGGAGATGGG AGGCCATCACATTGTAGCCCTCTGTGTGCTCAAGGGGGGCTATAAATTCTTTGCTGA CCTGCTGGATTACATCAAAGCACTGAATAGAAATAGTGATAGATCCATTCCTATGACT GTAGATTTTATCAGACTGAAGAGCTATTGTGTGAGTATATTTAATATATGATTCTTTTT AGTGGCAACAGTAGGTTTTCTTATATTTTCTTTGAATCTCTGCAAACCATACTTGCTTT CATTTCACTTGGTTACAGTGAGATTTTTCTAACATATTCACTAGTACTTTACATCAAAG CCAATACTGTTTTTTTAAAACTAGTCACCTTGGAGGATATATACTTATTTTACAGGTGT GTGTGGTTTTTTAAATAAACTCCTTTTAGGAATTGCTGTTGGGACTTGGGATACTTTTT TCACTATACATACTGGTGACAGATACCCTCTCTTGAGCTACATCGGTTTGTGGGGAG TCAAAAGTCCTTTGGAGCTAGGTTTGACAAATAAGGTGGGTTAACACTTGTTTCCTAG AAAGCACATGGAGAGCTAGAGTATTGGCGAATTGAAGAAATCCCCCTTTTTTTTTAAC ACACTTAAGAAAGGGGACTGCAGGTATACTCAAGAGAGTAAGTCGCACCAGAAACC ACTTTTGATCCACAGTCTGCCTGTGTCACACAATTGAAATGCATCACAACATTGACAC TGTGGATGAAACAAAATCAGTGTGAATTTTAGTAGTGAATTTCATTCATAATTTGATCG TGCAAACGTTTGATTTTTATTACTTTAGACTATTGTTTCTGATTTTATGTTGGGTTGGT ATTTCCTGTGAGTTACTGTTTTACCTTTAAAATAGGAATTTTTCATACTCTTCAAAGAT TAGAACAAATGTCCAGTTTTTGCTGTTTCATGAATGAGTCCTGTCCATCTTTGTAGAA ACTCGCCTTATGTTCACATTTTTATTGAGAATAAGACCACTTATCTACATTTAACTATC AACCTCATCCTCTCCATTAATCATCTATTTTAGTGACCCAAGTTTTTGACCTTTTCCAT GTTTACATCAATCCTGTAGGTGATTGGGCAGCCATTTAAGTATTATTATAGACATTTT CACTATCCCATTAAAACCCTTTATGCCCATACATCATAACACTACTTCCTACCCATAA GCTCCTTTTAACTTGTTAAAGTCTTGCTTGAATTAAAGACTTGTTTACGGTATCGATAA GCTTGATATCAAAACGCCAACTTTGACCCGGAACGCGGAAAACACCTGAGAAAAACA CCTGGGCGAGTCTCCACGTAAACGGTCAAAGTCCCCGCGGCCCTAGACAAATATTA CGCGCTATGAGTAACACAAAATTATTCAGATTTCACTTCCTCTTATTCAGTTTTCCCG CGAAAATGGCCAAATCTTACTCGGTTACGCCCAAATTTACTACAACATCCGCCTAAAA CCGCGCGAAAATTGTCACTTCCTGTGTACACCGGCGCACACCAAAAACGTCACTTTT GCCACATCCGTCGCTTACATGTGTTCCGCCACACTTGCAACATCACACTTCCGCCAC ACTACTACGTCACCCGCCCCGTTCCCACGCCCCGCGCCACGTCACAAACTCCACCC CCTCATTATCATATTGGCTTCAATCCAAAATAAGGTATATTATTGATGATGTTT |
| 126 | Ad5/3Ad2E1AΔ24 | TAACATCATCAATAATATACCTTATTTTGGATTGAAGCCAATATGATAATGAGGGGGT GGAGTTTGTGACGTGGCGCGGGGCGTGGGAACGGGGCGGGTGACGTAGTAGTGT GGCGGAAGTGTGATGTTGCAAGTGTGGCGGAACACATGTAAGCGACGGATGTGGCA AAAGTGACGTTTTTGGTGTGCGCCGGTGTACACAGGAAGTGACAATTTTCGCGCGG TTTTAGGCGGATGTTGTAGTAAATTTGGGCGTAACCGAGTAAGATTTGGCCATTTTC GCGGGAAAACTGAATAAGAGGAAGTGAAATCTGAATAATTTTGTGTTACTCATAGCG CGTAATATTTGTCTAGGGCCGCGGGGACTTTGACCGTTTACGTGGAGACTCGCCCA GGTGTTTTTCTCAGGTGTTTTCCGCGTTCCGGGTCAAAGTTGGCGTTTTATTATTATA GTCAGCTGACGTGTAGTGTATTTATACCCGGTGAGTTCCTCAAGAGGCCACTCTTGA GTGCCAGCGAGTAGAGTTTTCTCCTCCGAGCCGCTCCGACACCGGGACTGAAAATG AGACATATTATCTGCCACGGAGGTGTTATTACCGAAGAAATGGCCGCCAGTCTTTTG GACCAGCTGATCGAAGAGGTACTGGCTGATAATCTTCCACCTCCTAGCCATTTTGAA CCACCTACCCTTCACGAACTGTATGATTTAGACGTGACGGCCCCCGAAGATCCCAAC GAGGAGGCGGTTTCGCAGATTTTTCCCGAGTCTGTAATGTTGGCGGTGCAGGAAGG GATTGACTTATTCACTTTTCCGCCGGCGCCCGGTTCTCCGGAGCCGCCTCACCTTTC CCGGCAGCCCGAGCAGCCGGAGCAGAGAGCCTTGGGTCCGGTTTCTATGCCAAAC CTTGTGCCGGAGGTGATCGATCCACCCAGTGACGACGAGGATGAAGAGGGTGAGG AGTTTGTGTTAGATTATGTGGAGCACCCCGGGCACGGTTGCAGGTCTTGTCATTATC ACCGGAGGAATACGGGGGACCCAGATATTATGTGTTCGCTTTGCTATATGAGGACCT GTGGCATGTTTGTCTACAGTAAGTGAAAATTATGGGCAGTCGGTGATAGAGTGGTGG GTTTGGTGTGGTAATTTTTTTTTAATTTTTACAGTTTTGTGGTTTAAAGAATTTTGTATT GTGATTTTTTAAAAGGTCCTGTGTCTGAACCTGAGCCTGAGCCCGAGCCAGAACCG GAGCCTGCAAGACCTACCCGGCGTCCTAAATTGGTGCCTGCTATCCTGAGACGCCC GACATCACCTGTGTCTAGAGAATGCAATAGTAGTACGGATAGCTGTGACTCCGGTCC TTCTAACACACCTCCTGAGATACACCCGGTGGTCCCGCTGTGCCCCATTAAACCAGT TGCCGTGAGAGTTGGTGGGCGTCGCCAGGCTGTGGAATGTATCGAGGACTTGCTTA ACGAGTCTGGGCAACCTTTGGACTTGAGCTGTAAACGCCCCAGGCCATAAGGTGTA |
| | | AACCTGTGATTGCGTGTGTGGTTAACGCCTTTGTTTGCTGAATGAGTTGATGTAAGTT TAATAAAGGGTGAGATAATGTTTAACTTGCATGGCGTGTTAAATGGGGCGGGGCTTA AAGGGTATATAATGCGCCGTGGGCTAATCTTGGTTACATCTGACCTCATGGAGGCTT GGGAGTGTTTGGAAGATTTTTCTGCTGTGCGTAACTTGCTGGAACAGAGCTCTAACA GTACCTCTTGGTTTTGGAGGTTTCTGTGGGGCTCATCCCAGGCAAAGTTAGTCTGCA GAATTAAGGAGGATTACAAGTGGGAATTTGAAGAGCTTTTGAAATCCTGTGGTGAGC TGTTTGATTCTTTGAATCTGGGTCACCAGGCGCTTTTCCAAGAGAAGGTCATCAAGA CTTTGGATTTTTCCACACCGGGGCGCGCTGCGGCTGCTGTTGCTTTTTTGAGTTTTA TAAAGGATAAATGGAGCGAAGAAACCCATCTGAGCGGGGGGTACCTGCTGGATTTT CTGGCCATGCATCTGTGGAGAGCGGTTGTGAGACACAAGAATCGCCTGCTACTGTT GTCTTCCGTCCGCCCGGCGATAATACCGACGGAGGAGCAGCAGCAGCAGCAGGAG GAAGCCAGGCGGCGGCGGCAGGAGCAGAGCCCATGGAACCCGAGAGCCGGCCTG GACCCTCGGGAATGAATGTTGTTCAGGTGGCTGAACTGTATCCAGAACTGAGACGC ATTTTGACAATTACAGAGGATGGGCAGGGGCTAAAGGGGGTAAAGAGGGAGCGGG GGGCTTGTGAGGCTACAGAGGAGGCTAGGAATCTAGCTTTTAGCTTAATGACCAGA CACCGTCCTGAGTGTATTACTTTTCAACAGATCAAGGATAATTGCGCTAATGAGCTTG ATCTGCTGGCGCAGAAGTATTCCATAGAGCAGCTGACCACTTACTGGCTGCAGCCA GGGGATGATTTTGAGGAGGCTATTAGGGTATATGCAAAGGTGGCACTTAGGCCAGA TTGCAAGTACAAGATCAGCAAACTTGTAAATATCAGGAATTGTTGCTACATTTCTGGG AACGGGGCCGAGGTGGAGATAGATACGGAGGATAGGGTGGCCTTTAGATGTAGCAT GATAAATATGTGGCCGGGGGTGCTTGGCATGGACGGGGTGGTTATTATGAATGTAA GGTTTACTGGCCCCAATTTTAGCGGTACGGTTTTCCTGGCCAATACCAACCTTATCC TACACGGTGTAAGCTTCTATGGGTTTAACAATACCTGTGTGGAAGCCTGGACCGATG TAAGGGTTCGGGGCTGTGCCTTTTACTGCTGCTGGAAGGGGGTGGTGTGTCGCCCC AAAAGCAGGGCTTCAATTAAGAAATGCCTCTTTGAAAGGTGTACCTTGGGTATCCTG TCTGAGGGTAACTCCAGGGTGCGCCACAATGTGGCCTCCGACTGTGGTTGCTTCAT GCTAGTGAAAAGCGTGGCTGTGATTAAGCATAACATGGTATGTGGCAACTGCGAGG ACAGGGCCTCTCAGATGCTGACCTGCTCGGACGGCAACTGTCACCTTCTGAAGACC ATTCACGTAGCCAGCCACTCTCGCAAGGCCTGGCCAGTGTTTGAGCATAACATACTG ACCCGCTGTTCCTTGCATTTGGGTAACAGGAGGGGGGTGTTCCTACCTTACCAATGC AATTTGAGTCACACTAAGATATTGCTTGAGCCCGAGAGCATGTCCAAGGTGAACCTG AACGGGGTGTTTGACATGACCATGAAGATCTGGAAGGTGCTGAGGTACGATGAGAC CCGCACCAGGTGCAGACCCTGCGAGTGTGGCGGTAAACATATTAGGAACCAGCCTG TGATGCTGGATGTGACCGAGGAGCTGAGGCCCGATCACTTGGTGCTGGCCTGCACC CGCGCTGAGTTTGGCTCTAGCGATGAAGATACAGATTGAGGTACTGAAATGTGTGG GCGTGGCTTAAGGGTGGGAAAGAATATATAAGGTGGGGGTCTTATGTAGTTTTGTAT CTGTTTTGCAGCAGCCGCCGCCGCCATGAGCACCAACTCGTTTGATGGAAGCATTG TGAGCTCATATTTGACAACGCGCATGCCCCCATGGGCCGGGGTGCGTCAGAATGTG ATGGGCTCCAGCATTGATGGTCGCCCCGTCCTGCCCGCAAACTCTACTACCTTGAC CTACGAGACCGTGTCTGGAACGCCGTTGGAGACTGCAGCCTCCGCCGCCGCTTCA GCCGCTGCAGCCACCGCCCGCGGGATTGTGACTGACTTTGCTTTCCTGAGCCCGCT TGCAAGCAGTGCAGCTTCCCGTTCATCCGCCCGCGATGACAAGTTGACGGCTCTTTT GGCACAATTGGATTCTTTGACCCGGGAACTTAATGTCGTTTCTCAGCAGCTGTTGGA TCTGCGCCAGCAGGTTTCTGCCCTGAAGGCTTCCTCCCCTCCCAATGCGGTTTAAAA CATAAATAAAAAACCAGACTCTGTTTGGATTTGGATCAAGCAAGTGTCTTGCTGTCTT TATTTAGGGGTTTTGCGCGCGCGGTAGGCCCGGGACCAGCGGTCTCGGTCGTTGA GGGTCCTGTGTATTTTTTCCAGGACGTGGTAAAGGTGACTCTGGATGTTCAGATACA TGGGCATAAGCCCGTCTCTGGGGTGGAGGTAGCACCACTGCAGAGCTTCATGCTGC GGGGTGGTGTTGTAGATGATCCAGTCGTAGCAGGAGCGCTGGGCGTGGTGCCTAA AAATGTCTTTCAGTAGCAAGCTGATTGCCAGGGGCAGGCCCTTGGTGTAAGTGTTTA CAAAGCGGTTAAGCTGGGATGGGTGCATACGTGGGGATATGAGATGCATCTTGGAC TGTATTTTTAGGTTGGCTATGTTCCCAGCCATATCCCTCCGGGGATTCATGTTGTGCA GAACCACCAGCACAGTGTATCCGGTGCACTTGGGAAATTTGTCATGTAGCTTAGAAG GAAATGCGTGGAAGAACTTGGAGACGCCCTTGTGACCTCCAAGATTTTCCATGCATT CGTCCATAATGATGGCAATGGGCCCACGGGCGGCGGCCTGGGCGAAGATATTTCTG GGATCACTAACGTCATAGTTGTGTTCCAGGATGAGATCGTCATAGGCCATTTTTACAA AGCGCGGGCGGAGGGTGCCAGACTGCGGTATAATGGTTCCATCCGGCCCAGGGGC GTAGTTACCCTCACAGATTTGCATTTCCCACGCTTTGAGTTCAGATGGGGGGATCAT GTCTACCTGCGGGGCGATGAAGAAAACGGTTTCCGGGGTAGGGGAGATCAGCTGG GAAGAAAGCAGGTTCCTGAGCAGCTGCGACTTACCGCAGCCGGTGGGCCCGTAAAT CACACCTATTACCGGGTGCAACTGGTAGTTAAGAGAGCTGCAGCTGCCGTCATCCC TGAGCAGGGGGGCCACTTCGTTAAGCATGTCCCTGACTCGCATGTTTTCCCTGACC AAATCCGCCAGAAGGCGCTCGCCGCCCAGCGATAGCAGTTCTTGCAAGGAAGCAAA GTTTTTCAACGGTTTGAGACCGTCCGCCGTAGGCATGCTTTTGAGCGTTTGACCAAG CAGTTCCAGGCGGTCCCACAGCTCGGTTACCTGCTCTACGGCATCTCGATCCAGCA TATCTCCTCGTTTCGCGGGTTGGGGCGGCTTTCGCTGTACGGCAGTAGTCGGTGCT CGTCCAGACGGGCCAGGGTCATGTCTTTCCACGGGCGCAGGGTCCTCGTCAGCGT AGTCTGGGTCACGGTGAAGGGGTGCGCTCCGGGCTGCGCGCTGGCCAGGGTGCG CTTGAGGCTGGTCCTGCTGGTGCTGAAGCGCTGCCGGTCTTCGCCCTGCGCGTCG GCCAGGTAGCATTTGACCATGGTGTCATAGTCCAGCCCCTCCGCGGCGTGGCCCTT |
| | | GGCGCGCAGCTTGCCCTTGGAGGAGGCGCCGCACGAGGGGCAGTGCAGACTTTTG AGGGCGTAGAGCTTGGGCGCGAGAAATACCGATTCCGGGGAGTAGGCATCCGCGC CGCAGGCCCCGCAGACGGTCTCGCATTCCACGAGCCAGGTGAGCTCTGGCCGTTC GGGGTCAAAAACCAGGTTTCCCCCATGCTTTTTGATGCGTTTCTTACCTCTGGTTTCC ATGAGCCGGTGTCCACGCTCGGTGACGAAAAGGCTGTCCGTGTCCCCGTATACAGA CTTGAGAGGCCTGTCCTCGAGCGGTGTTCCGCGGTCCTCCTCGTATAGAAACTCGG ACCACTCTGAGACAAAGGCTCGCGTCCAGGCCAGCACGAAGGAGGCTAAGTGGGA GGGGTAGCGGTCGTTGTCCACTAGGGGGTCCACTCGCTCCAGGGTGTGAAGACAC ATGTCGCCCTCTTCGGCATCAAGGAAGGTGATTGGTTTGTAGGTGTAGGCCACGTG ACCGGGTGTTCCTGAAGGGGGGCTATAAAAGGGGGTGGGGGCGCGTTCGTCCTCA CTCTCTTCCGCATCGCTGTCTGCGAGGGCCAGCTGTTGGGGTGAGTACTCCCTCTG AAAAGCGGGCATGACTTCTGCGCTAAGATTGTCAGTTTCCAAAAACGAGGAGGATTT GATATTCACCTGGCCCGCGGTGATGCCTTTGAGGGTGGCCGCATCCATCTGGTCAG AAAAGACAATCTTTTTGTTGTCAAGCTTGGTGGCAAACGACCCGTAGAGGGCGTTGG ACAGCAACTTGGCGATGGAGCGCAGGGTTTGGTTTTTGTCGCGATCGGCGCGCTCC TTGGCCGCGATGTTTAGCTGCACGTATTCGCGCGCAACGCACCGCCATTCGGGAAA GACGGTGGTGCGCTCGTCGGGCACCAGGTGCACGCGCCAACCGCGGTTGTGCAGG GTGACAAGGTCAACGCTGGTGGCTACCTCTCCGCGTAGGCGCTCGTTGGTCCAGCA GAGGCGGCCGCCCTTGCGCGAGCAGAATGGCGGTAGGGGGTCTAGCTGCGTCTCG TCCGGGGGGTCTGCGTCCACGGTAAAGACCCCGGGCAGCAGGCGCGCGTCGAAGT AGTCTATCTTGCATCCTTGCAAGTCTAGCGCCTGCTGCCATGCGCGGGCGGCAAGC GCGCGCTCGTATGGGTTGAGTGGGGGACCCCATGGCATGGGGTGGGTGAGCGCG GAGGCGTACATGCCGCAAATGTCGTAAACGTAGAGGGGCTCTCTGAGTATTCCAAG ATATGTAGGGTAGCATCTTCCACCGCGGATGCTGGCGCGCACGTAATCGTATAGTTC GTGCGAGGGAGCGAGGAGGTCGGGACCGAGGTTGCTACGGGCGGGCTGCTCTGC TCGGAAGACTATCTGCCTGAAGATGGCATGTGAGTTGGATGATATGGTTGGACGCT GGAAGACGTTGAAGCTGGCGTCTGTGAGACCTACCGCGTCACGCACGAAGGAGGC GTAGGAGTCGCGCAGCTTGTTGACCAGCTCGGCGGTGACCTGCACGTCTAGGGCG CAGTAGTCCAGGGTTTCCTTGATGATGTCATACTTATCCTGTCCCTTTTTTTTCCACA GCTCGCGGTTGAGGACAAACTCTTCGCGGTCTTTCCAGTACTCTTGGATCGGAAACC CGTCGGCCTCCGAACGGTAAGAGCCTAGCATGTAGAACTGGTTGACGGCCTGGTAG GCGCAGCATCCCTTTTCTACGGGTAGCGCGTATGCCTGCGCGGCCTTCCGGAGCGA GGTGTGGGTGAGCGCAAAGGTGTCCCTGACCATGACTTTGAGGTACTGGTATTTGA AGTCAGTGTCGTCGCATCCGCCCTGCTCCCAGAGCAAAAAGTCCGTGCGCTTTTTG GAACGCGGATTTGGCAGGGCGAAGGTGACATCGTTGAAGAGTATCTTTCCCGCGCG AGGCATAAAGTTGCGTGTGATGCGGAAGGGTCCCGGCACCTCGGAACGGTTGTTAA TTACCTGGGCGGCGAGCACGATCTCGTCAAAGCCGTTGATGTTGTGGCCCACAATG TAAAGTTCCAAGAAGCGCGGGATGCCCTTGATGGAAGGCAATTTTTTAAGTTCCTCG TAGGTGAGCTCTTCAGGGGAGCTGAGCCCGTGCTCTGAAAGGGCCCAGTCTGCAAG ATGAGGGTTGGAAGCGACGAATGAGCTCCACAGGTCACGGGCCATTAGCATTTGCA GGTGGTCGCGAAAGGTCCTAAACTGGCGACCTATGGCCATTTTTTCTGGGGTGATG CAGTAGAAGGTAAGCGGGTCTTGTTCCCAGCGGTCCCATCCAAGGTTCGCGGCTAG GTCTCGCGCGGCAGTCACTAGAGGCTCATCTCCGCCGAACTTCATGACCAGCATGA AGGGCACGAGCTGCTTCCCAAAGGCCCCCATCCAAGTATAGGTCTCTACATCGTAG GTGACAAAGAGACGCTCGGTGCGAGGATGCGAGCCGATCGGGAAGAACTGGATCT CCCGCCACCAATTGGAGGAGTGGCTATTGATGTGGTGAAAGTAGAAGTCCCTGCGA CGGGCCGAACACTCGTGCTGGCTTTTGTAAAAACGTGCGCAGTACTGGCAGCGGTG CACGGGCTGTACATCCTGCACGAGGTTGACCTGACGACCGCGCACAAGGAAGCAG AGTGGGAATTTGAGCCCCTCGCCTGGCGGGTTTGGCTGGTGGTCTTCTACTTCGGC TGCTTGTCCTTGACCGTCTGGCTGCTCGAGGGGAGTTACGGTGGATCGGACCACCA CGCCGCGCGAGCCCAAAGTCCAGATGTCCGCGCGCGGCGGTCGGAGCTTGATGAC AACATCGCGCAGATGGGAGCTGTCCATGGTCTGGAGCTCCCGCGGCGTCAGGTCA GGCGGGAGCTCCTGCAGGTTTACCTCGCATAGACGGGTCAGGGCGCGGGCTAGAT CCAGGTGATACCTAATTTCCAGGGGCTGGTTGGTGGCGGCGTCGATGGCTTGCAAG AGGCCGCATCCCCGCGGCGCGACTACGGTACCGCGCGGCGGGCGGTGGGCCGCG GGGGTGTCCTTGGATGATGCATCTAAAAGCGGTGACGCGGGCGAGCCCCCGGAGG TAGGGGGGGCTCCGGACCCGCCGGGAGAGGGGGCAGGGGCACGTCGGCGCCGC GCGCGGGCAGGAGCTGGTGCTGCGCGCGTAGGTTGCTGGCGAACGCGACGACGC GGCGGTTGATCTCCTGAATCTGGCGCCTCTGCGTGAAGACGACGGGCCCGGTGAG CTTGAGCCTGAAAGAGAGTTCGACAGAATCAATTTCGGTGTCGTTGACGGCGGCCT GGCGCAAAATCTCCTGCACGTCTCCTGAGTTGTCTTGATAGGCGATCTCGGCCATGA ACTGCTCGATCTCTTCCTCCTGGAGATCTCCGCGTCCGGCTCGCTCCACGGTGGCG GCGAGGTCGTTGGAAATGCGGGCCATGAGCTGCGAGAAGGCGTTGAGGCCTCCCT CGTTCCAGACGCGGCTGTAGACCACGCCCCCTTCGGCATCGCGGGCGCGCATGAC CACCTGCGCGAGATTGAGCTCCACGTGCCGGGCGAAGACGGCGTAGTTTCGCAGG CGCTGAAAGAGGTAGTTGAGGGTGGTGGCGGTGTGTTCTGCCACGAAGAAGTACAT AACCCAGCGTCGCAACGTGGATTCGTTGATATCCCCCAAGGCCTCAAGGCGCTCCA TGGCCTCGTAGAAGTCCACGGCGAAGTTGAAAAACTGGGAGTTGCGCGCCGACACG GTTAACTCCTCCTCCAGAAGACGGATGAGCTCGGCGACAGTGTCGCGCACCTCGCG CTCAAAGGCTACAGGGGCCTCTTCTTCTTCTTCAATCTCCTCTTCCATAAGGGCCTC |
| | | CCCTTCTTCTTCTTCTGGCGGCGGTGGGGGAGGGGGGACACGGCGGCGACGACGG CGCACCGGGAGGCGGTCGACAAAGCGCTCGATCATCTCCCCGCGGCGACGGCGCA TGGTCTCGGTGACGGCGCGGCCGTTCTCGCGGGGGCGCAGTTGGAAGACGCCGC CCGTCATGTCCCGGTTATGGGTTGGCGGGGGGCTGCCATGCGGCAGGGATACGGC GCTAACGATGCATCTCAACAATTGTTGTGTAGGTACTCCGCCGCCGAGGGACCTGA GCGAGTCCGCATCGACCGGATCGGAAAACCTCTCGAGAAAGGCGTCTAACCAGTCA CAGTCGCAAGGTAGGCTGAGCACCGTGGCGGGCGGCAGCGGGCGGCGGTCGGGG TTGTTTCTGGCGGAGGTGCTGCTGATGATGTAATTAAAGTAGGCGGTCTTGAGACGG CGGATGGTCGACAGAAGCACCATGTCCTTGGGTCCGGCCTGCTGAATGCGCAGGC GGTCGGCCATGCCCCAGGCTTCGTTTTGACATCGGCGCAGGTCTTTGTAGTAGTCTT GCATGAGCCTTTCTACCGGCACTTCTTCTTCTCCTTCCTCTTGTCCTGCATCTCTTGC ATCTATCGCTGCGGCGGCGGCGGAGTTTGGCCGTAGGTGGCGCCCTCTTCCTCCC ATGCGTGTGACCCCGAAGCCCCTCATCGGCTGAAGCAGGGCTAGGTCGGCGACAA CGCGCTCGGCTAATATGGCCTGCTGCACCTGCGTGAGGGTAGACTGGAAGTCATCC ATGTCCACAAAGCGGTGGTATGCGCCCGTGTTGATGGTGTAAGTGCAGTTGGCCAT AACGGACCAGTTAACGGTCTGGTGACCCGGCTGCGAGAGCTCGGTGTACCTGAGAC GCGAGTAAGCCCTCGAGTCAAATACGTAGTCGTTGCAAGTCCGCACCAGGTACTGG TATCCCACCAAAAAGTGCGGCGGCGGCTGGCGGTAGAGGGGCCAGCGTAGGGTGG CCGGGGCTCCGGGGGCGAGATCTTCCAACATAAGGCGATGATATCCGTAGATGTAC CTGGACATCCAGGTGATGCCGGCGGCGGTGGTGGAGGCGCGCGGAAAGTCGCGG ACGCGGTTCCAGATGTTGCGCAGCGGCAAAAAGTGCTCCATGGTCGGGACGCTCTG GCCGGTCAGGCGCGCGCAATCGTTGACGCTCTAGACCGTGCAAAAGGAGAGCCTG TAAGCGGGCACTCTTCCGTGGTCTGGTGGATAAATTCGCAAGGGTATCATGGCGGA CGACCGGGGTTCGAGCCCCGTATCCGGCCGTCCGCCGTGATCCATGCGGTTACCG CCCGCGTGTCGAACCCAGGTGTGCGACGTCAGACAACGGGGGAGTGCTCCTTTTG GCTTCCTTCCAGGCGCGGCGGCTGCTGCGCTAGCTTTTTTGGCCACTGGCCGCGC GCAGCGTAAGCGGTTAGGCTGGAAAGCGAAAGCATTAAGTGGCTCGCTCCCTGTAG CCGGAGGGTTATTTTCCAAGGGTTGAGTCGCGGGACCCCCGGTTCGAGTCTCGGAC CGGCCGGACTGCGGCGAACGGGGGTTTGCCTCCCCGTCATGCAAGACCCCGCTTG CAAATTCCTCCGGAAACAGGGACGAGCCCCTTTTTTGCTTTTCCCAGATGCATCCGG TGCTGCGGCAGATGCGCCCCCCTCCTCAGCAGCGGCAAGAGCAAGAGCAGCGGCA GACATGCAGGGCACCCTCCCCTCCTCCTACCGCGTCAGGAGGGGCGACATCCGCG GTTGACGCGGCAGCAGATGGTGATTACGAACCCCCGCGGCGCCGGGCCCGGCACT ACCTGGACTTGGAGGAGGGCGAGGGCCTGGCGCGGCTAGGAGCGCCCTCTCCTGA GCGGTACCCAAGGGTGCAGCTGAAGCGTGATACGCGTGAGGCGTACGTGCCGCGG CAGAACCTGTTTCGCGACCGCGAGGGAGAGGAGCCCGAGGAGATGCGGGATCGAA AGTTCCACGCAGGGCGCGAGCTGCGGCATGGCCTGAATCGCGAGCGGTTGCTGCG CGAGGAGGACTTTGAGCCCGACGCGCGAACCGGGATTAGTCCCGCGCGCGCACAC GTGGCGGCCGCCGACCTGGTAACCGCATACGAGCAGACGGTGAACCAGGAGATTA ACTTTCAAAAAAGCTTTAACAACCACGTGCGTACGCTTGTGGCGCGCGAGGAGGTG GCTATAGGACTGATGCATCTGTGGGACTTTGTAAGCGCGCTGGAGCAAAACCCAAAT AGCAAGCCGCTCATGGCGCAGCTGTTCCTTATAGTGCAGCACAGCAGGGACAACGA GGCATTCAGGGATGCGCTGCTAAACATAGTAGAGCCCGAGGGCCGCTGGCTGCTC GATTTGATAAACATCCTGCAGAGCATAGTGGTGCAGGAGCGCAGCTTGAGCCTGGC TGACAAGGTGGCCGCCATCAACTATTCCATGCTTAGCCTGGGCAAGTTTTACGCCCG CAAGATATACCATACCCCTTACGTTCCCATAGACAAGGAGGTAAAGATCGAGGGGTT CTACATGCGCATGGCGCTGAAGGTGCTTACCTTGAGCGACGACCTGGGCGTTTATC GCAACGAGCGCATCCACAAGGCCGTGAGCGTGAGCCGGCGGCGCGAGCTCAGCG ACCGCGAGCTGATGCACAGCCTGCAAAGGGCCCTGGCTGGCACGGGCAGCGGCGA TAGAGAGGCCGAGTCCTACTTTGACGCGGGCGCTGACCTGCGCTGGGCCCCAAGC CGACGCGCCCTGGAGGCAGCTGGGGCCGGACCTGGGCTGGCGGTGGCACCCGCG CGCGCTGGCAACGTCGGCGGCGTGGAGGAATATGACGAGGACGATGAGTACGAGC CAGAGGACGGCGAGTACTAAGCGGTGATGTTTCTGATCAGATGATGCAAGACGCAA CGGACCCGGCGGTGCGGGCGGCGCTGCAGAGCCAGCCGTCCGGCCTTAACTCCA CGGACGACTGGCGCCAGGTCATGGACCGCATCATGTCGCTGACTGCGCGCAATCCT GACGCGTTCCGGCAGCAGCCGCAGGCCAACCGGCTCTCCGCAATTCTGGAAGCGG TGGTCCCGGCGCGCGCAAACCCCACGCACGAGAAGGTGCTGGCGATCGTAAACGC GCTGGCCGAAAACAGGGCCATCCGGCCCGACGAGGCCGGCCTGGTCTACGACGCG CTGCTTCAGCGCGTGGCTCGTTACAACAGCGGCAACGTGCAGACCAACCTGGACCG GCTGGTGGGGGATGTGCGCGAGGCCGTGGCGCAGCGTGAGCGCGCGCAGCAGCA GGGCAACCTGGGCTCCATGGTTGCACTAAACGCCTTCCTGAGTACACAGCCCGCCA ACGTGCCGCGGGGACAGGAGGACTACACCAACTTTGTGAGCGCACTGCGGCTAAT GGTGACTGAGACACCGCAAAGTGAGGTGTACCAGTCTGGGCCAGACTATTTTTTCCA GACCAGTAGACAAGGCCTGCAGACCGTAAACCTGAGCCAGGCTTTCAAAAACTTGC AGGGGCTGTGGGGGGTGCGGGCTCCCACAGGCGACCGCGCGACCGTGTCTAGCTT GCTGACGCCCAACTCGCGCCTGTTGCTGCTGCTAATAGCGCCCTTCACGGACAGTG GCAGCGTGTCCCGGGACACATACCTAGGTCACTTGCTGACACTGTACCGCGAGGCC ATAGGTCAGGCGCATGTGGACGAGCATACTTTCCAGGAGATTACAAGTGTCAGCCG CGCGCTGGGGCAGGAGGACACGGGCAGCCTGGAGGCAACCCTAAACTACCTGCTG ACCAACCGGCGGCAGAAGATCCCCTCGTTGCACAGTTTAAACAGCGAGGAGGAGCG |
| | | CATTTTGCGCTACGTGCAGCAGAGCGTGAGCCTTAACCTGATGCGCGACGGGGTAA CGCCCAGCGTGGCGCTGGACATGACCGCGCGCAACATGGAACCGGGCATGTATGC CTCAAACCGGCCGTTTATCAACCGCCTAATGGACTACTTGCATCGCGCGGCCGCCG TGAACCCCGAGTATTTCACCAATGCCATCTTGAACCCGCACTGGCTACCGCCCCCTG GTTTCTACACCGGGGGATTCGAGGTGCCCGAGGGTAACGATGGATTCCTCTGGGAC GACATAGACGACAGCGTGTTTTCCCCGCAACCGCAGACCCTGCTAGAGTTGCAACA GCGCGAGCAGGCAGAGGCGGCGCTGCGAAAGGAAAGCTTCCGCAGGCCAAGCAG CTTGTCCGATCTAGGCGCTGCGGCCCCGCGGTCAGATGCTAGTAGCCCATTTCCAA GCTTGATAGGGTCTCTTACCAGCACTCGCACCACCCGCCCGCGCCTGCTGGGCGA GGAGGAGTACCTAAACAACTCGCTGCTGCAGCCGCAGCGCGAAAAAAACCTGCCTC CGGCATTTCCCAACAACGGGATAGAGAGCCTAGTGGACAAGATGAGTAGATGGAAG ACGTACGCGCAGGAGCACAGGGACGTGCCAGGCCCGCGCCCGCCCACCCGTCGT CAAAGGCACGACCGTCAGCGGGGTCTGGTGTGGGAGGACGATGACTCGGCAGACG ACAGCAGCGTCCTGGATTTGGGAGGGAGTGGCAACCCGTTTGCGCACCTTCGCCCC AGGCTGGGGAGAATGTTTTAAAAAAAAAAAAGCATGATGCAAAATAAAAAACTCACCA AGGCCATGGCACCGAGCGTTGGTTTTCTTGTATTCCCCTTAGTATGCGGCGCGCGG CGATGTATGAGGAAGGTCCTCCTCCCTCCTACGAGAGTGTGGTGAGCGCGGCGCCA GTGGCGGCGGCGCTGGGTTCTCCCTTCGATGCTCCCCTGGACCCGCCGTTTGTGC CTCCGCGGTACCTGCGGCCTACCGGGGGGAGAAACAGCATCCGTTACTCTGAGTTG GCACCCCTATTCGACACCACCCGTGTGTACCTGGTGGACAACAAGTCAACGGATGT GGCATCCCTGAACTACCAGAACGACCACAGCAACTTTCTGACCACGGTCATTCAAAA CAATGACTACAGCCCGGGGGAGGCAAGCACACAGACCATCAATCTTGACGACCGGT CGCACTGGGGCGGCGACCTGAAAACCATCCTGCATACCAACATGCCAAATGTGAAC GAGTTCATGTTTACCAATAAGTTTAAGGCGCGGGTGATGGTGTCGCGCTTGCCTACT AAGGACAATCAGGTGGAGCTGAAATACGAGTGGGTGGAGTTCACGCTGCCCGAGG GCAACTACTCCGAGACCATGACCATAGACCTTATGAACAACGCGATCGTGGAGCACT ACTTGAAAGTGGGCAGACAGAACGGGGTTCTGGAAAGCGACATCGGGGTAAAGTTT GACACCCGCAACTTCAGACTGGGGTTTGACCCCGTCACTGGTCTTGTCATGCCTGG GGTATATACAAACGAAGCCTTCCATCCAGACATCATTTTGCTGCCAGGATGCGGGGT GGACTTCACCCACAGCCGCCTGAGCAACTTGTTGGGCATCCGCAAGCGGCAACCCT TCCAGGAGGGCTTTAGGATCACCTACGATGATCTGGAGGGTGGTAACATTCCCGCA CTGTTGGATGTGGACGCCTACCAGGCGAGCTTGAAAGATGACACCGAACAGGGCG GGGGTGGCGCAGGCGGCAGCAACAGCAGTGGCAGCGGCGCGGAAGAGAACTCCA ACGCGGCAGCCGCGGCAATGCAGCCGGTGGAGGACATGAACGATCATGCCATTCG CGGCGACACCTTTGCCACACGGGCTGAGGAGAAGCGCGCTGAGGCCGAAGCAGCG GCCGAAGCTGCCGCCCCCGCTGCGCAACCCGAGGTCGAGAAGCCTCAGAAGAAAC CGGTGATCAAACCCCTGACAGAGGACAGCAAGAAACGCAGTTACAACCTAATAAGC AATGACAGCACCTTCACCCAGTACCGCAGCTGGTACCTTGCATACAACTACGGCGA CCCTCAGACCGGAATCCGCTCATGGACCCTGCTTTGCACTCCTGACGTAACCTGCG GCTCGGAGCAGGTCTACTGGTCGTTGCCAGACATGATGCAAGACCCCGTGACCTTC CGCTCCACGCGCCAGATCAGCAACTTTCCGGTGGTGGGCGCCGAGCTGTTGCCCG TGCACTCCAAGAGCTTCTACAACGACCAGGCCGTCTACTCCCAACTCATCCGCCAGT TTACCTCTCTGACCCACGTGTTCAATCGCTTTCCCGAGAACCAGATTTTGGCGCGCC CGCCAGCCCCCACCATCACCACCGTCAGTGAAAACGTTCCTGCTCTCACAGATCAC GGGACGCTACCGCTGCGCAACAGCATCGGAGGAGTCCAGCGAGTGACCATTACTG ACGCCAGACGCCGCACCTGCCCCTACGTTTACAAGGCCCTGGGCATAGTCTCGCCG CGCGTCCTATCGAGCCGCACTTTTTGAGCAAGCATGTCCATCCTTATATCGCCCAGC AATAACACAGGCTGGGGCCTGCGCTTCCCAAGCAAGATGTTTGGCGGGGCCAAGAA GCGCTCCGACCAACACCCAGTGCGCGTGCGCGGGCACTACCGCGCGCCCTGGGG CGCGCACAAACGCGGCCGCACTGGGCGCACCACCGTCGATGACGCCATCGACGCG GTGGTGGAGGAGGCGCGCAACTACACGCCCACGCCGCCACCAGTGTCCACAGTGG ACGCGGCCATTCAGACCGTGGTGCGCGGAGCCCGGCGCTATGCTAAAATGAAGAG ACGGCGGAGGCGCGTAGCACGTCGCCACCGCCGCCGACCCGGCACTGCCGCCCA ACGCGCGGCGGCGGCCCTGCTTAACCGCGCACGTCGCACCGGCCGACGGGCGGC CATGCGGGCCGCTCGAAGGCTGGCCGCGGGTATTGTCACTGTGCCCCCCAGGTCC AGGCGACGAGCGGCCGCCGCAGCAGCCGCGGCCATTAGTGCTATGACTCAGGGTC GCAGGGGCAACGTGTATTGGGTGCGCGACTCGGTTAGCGGCCTGCGCGTGCCCGT GCGCACCCGCCCCCCGCGCAACTAGATTGCAAGAAAAAACTACTTAGACTCGTACT GTTGTATGTATCCAGCGGCGGCGGCGCGCAACGAAGCTATGTCCAAGCGCAAAATC AAAGAAGAGATGCTCCAGGTCATCGCGCCGGAGATCTATGGCCCCCCGAAGAAGGA AGAGCAGGATTACAAGCCCCGAAAGCTAAAGCGGGTCAAAAAGAAAAAGAAAGATG ATGATGATGAACTTGACGACGAGGTGGAACTGCTGCACGCTACCGCGCCCAGGCGA CGGGTACAGTGGAAAGGTCGACGCGTAAAACGTGTTTTGCGACCCGGCACCACCGT AGTCTTTACGCCCGGTGAGCGCTCCACCCGCACCTACAAGCGCGTGTATGATGAGG TGTACGGCGACGAGGACCTGCTTGAGCAGGCCAACGAGCGCCTCGGGGAGTTTGC CTACGGAAAGCGGCATAAGGACATGCTGGCGTTGCCGCTGGACGAGGGCAACCCA ACACCTAGCCTAAAGCCCGTAACACTGCAGCAGGTGCTGCCCGCGCTTGCACCGTC CGAAGAAAAGCGCGGCCTAAAGCGCGAGTCTGGTGACTTGGCACCCACCGTGCAG CTGATGGTACCCAAGCGCCAGCGACTGGAAGATGTCTTGGAAAAAATGACCGTGGA ACCTGGGCTGGAGCCCGAGGTCCGCGTGCGGCCAATCAAGCAGGTGGCGCCGGG |
| | | ACTGGGCGTGCAGACCGTGGACGTTCAGATACCCACTACCAGTAGCACCAGTATTG CCACCGCCACAGAGGGCATGGAGACACAAACGTCCCCGGTTGCCTCAGCGGTGGC GGATGCCGCGGTGCAGGCGGTCGCTGCGGCCGCGTCCAAGACCTCTACGGAGGTG CAAACGGACCCGTGGATGTTTCGCGTTTCAGCCCCCCGGCGCCCGCGCGGTTCGA GGAAGTACGGCGCCGCCAGCGCGCTACTGCCCGAATATGCCCTACATCCTTCCATT GCGCCTACCCCCGGCTATCGTGGCTACACCTACCGCCCCAGAAGACGAGCAACTAC CCGACGCCGAACCACCACTGGAACCCGCCGCCGCCGTCGCCGTCGCCAGCCCGTG CTGGCCCCGATTTCCGTGCGCAGGGTGGCTCGCGAAGGAGGCAGGACCCTGGTGC TGCCAACAGCGCGCTACCACCCCAGCATCGTTTAAAAGCCGGTCTTTGTGGTTCTTG CAGATATGGCCCTCACCTGCCGCCTCCGTTTCCCGGTGCCGGGATTCCGAGGAAGA ATGCACCGTAGGAGGGGCATGGCCGGCCACGGCCTGACGGGCGGCATGCGTCGT GCGCACCACCGGCGGCGGCGCGCGTCGCACCGTCGCATGCGCGGCGGTATCCTG CCCCTCCTTATTCCACTGATCGCCGCGGCGATTGGCGCCGTGCCCGGAATTGCATC CGTGGCCTTGCAGGCGCAGAGACACTGATTAAAAACAAGTTGCATGTGGAAAAATCA AAATAAAAAGTCTGGACTCTCACGCTCGCTTGGTCCTGTAACTATTTTGTAGAATGGA AGACATCAACTTTGCGTCTCTGGCCCCGCGACACGGCTCGCGCCCGTTCATGGGAA ACTGGCAAGATATCGGCACCAGCAATATGAGCGGTGGCGCCTTCAGCTGGGGCTCG CTGTGGAGCGGCATTAAAAATTTCGGTTCCACCGTTAAGAACTATGGCAGCAAGGCC TGGAACAGCAGCACAGGCCAGATGCTGAGGGATAAGTTGAAAGAGCAAAATTTCCA ACAAAAGGTGGTAGATGGCCTGGCCTCTGGCATTAGCGGGGTGGTGGACCTGGCC AACCAGGCAGTGCAAAATAAGATTAACAGTAAGCTTGATCCCCGCCCTCCCGTAGAG GAGCCTCCACCGGCCGTGGAGACAGTGTCTCCAGAGGGGCGTGGCGAAAAGCGTC CGCGCCCCGACAGGGAAGAAACTCTGGTGACGCAAATAGACGAGCCTCCCTCGTAC GAGGAGGCACTAAAGCAAGGCCTGCCCACCACCCGTCCCATCGCGCCCATGGCTA CCGGAGTGCTGGGCCAGCACACACCCGTAACGCTGGACCTGCCTCCCCCCGCCGA CACCCAGCAGAAACCTGTGCTGCCAGGCCCGACCGCCGTTGTTGTAACCCGTCCTA GCCGCGCGTCCCTGCGCCGCGCCGCCAGCGGTCCGCGATCGTTGCGGCCCGTAG CCAGTGGCAACTGGCAAAGCACACTGAACAGCATCGTGGGTCTGGGGGTGCAATCC CTGAAGCGCCGACGATGCTTCTGAATAGCTAACGTGTCGTATGTGTGTCATGTATGC GTCCATGTCGCCGCCAGAGGAGCTGCTGAGCCGCCGCGCGCCCGCTTTCCAAGAT GGCTACCCCTTCGATGATGCCGCAGTGGTCTTACATGCACATCTCGGGCCAGGACG CCTCGGAGTACCTGAGCCCCGGGCTGGTGCAGTTTGCCCGCGCCACCGAGACGTA CTTCAGCCTGAATAACAAGTTTAGAAACCCCACGGTGGCGCCTACGCACGACGTGA CCACAGACCGGTCCCAGCGTTTGACGCTGCGGTTCATCCCTGTGGACCGTGAGGAT ACTGCGTACTCGTACAAGGCGCGGTTCACCCTAGCTGTGGGTGATAACCGTGTGCT GGACATGGCTTCCACGTACTTTGACATCCGCGGCGTGCTGGACAGGGGCCCTACTT TTAAGCCCTACTCTGGCACTGCCTACAACGCCCTGGCTCCCAAGGGTGCCCCAAAT CCTTGCGAATGGGATGAAGCTGCTACTGCTCTTGAAATAAACCTAGAAGAAGAGGAC GATGACAACGAAGACGAAGTAGACGAGCAAGCTGAGCAGCAAAAAACTCACGTATTT GGGCAGGCGCCTTATTCTGGTATAAATATTACAAAGGAGGGTATTCAAATAGGTGTC GAAGGTCAAACACCTAAATATGCCGATAAAACATTTCAACCTGAACCTCAAATAGGA GAATCTCAGTGGTACGAAACTGAAATTAATCATGCAGCTGGGAGAGTCCTTAAAAAG ACTACCCCAATGAAACCATGTTACGGTTCATATGCAAAACCCACAAATGAAAATGGA GGGCAAGGCATTCTTGTAAAGCAACAAAATGGAAAGCTAGAAAGTCAAGTGGAAATG CAATTTTTCTCAACTACTGAGGCGACCGCAGGCAATGGTGATAACTTGACTCCTAAA GTGGTATTGTACAGTGAAGATGTAGATATAGAAACCCCAGACACTCATATTTCTTACA TGCCCACTATTAAGGAAGGTAACTCACGAGAACTAATGGGCCAACAATCTATGCCCA ACAGGCCTAATTACATTGCTTTTAGGGACAATTTTATTGGTCTAATGTATTACAACAG CACGGGTAATATGGGTGTTCTGGCGGGCCAAGCATCGCAGTTGAATGCTGTTGTAG ATTTGCAAGACAGAAACACAGAGCTTTCATACCAGCTTTTGCTTGATTCCATTGGTGA TAGAACCAGGTACTTTTCTATGTGGAATCAGGCTGTTGACAGCTATGATCCAGATGTT AGAATTATTGAAAATCATGGAACTGAAGATGAACTTCCAAATTACTGCTTTCCACTGG GAGGTGTGATTAATACAGAGACTCTTACCAAGGTAAAACCTAAAACAGGTCAGGAAA ATGGATGGGAAAAAGATGCTACAGAATTTTCAGATAAAAATGAAATAAGAGTTGGAAA TAATTTTGCCATGGAAATCAATCTAAATGCCAACCTGTGGAGAAATTTCCTGTACTCC AACATAGCGCTGTATTTGCCCGACAAGCTAAAGTACAGTCCTTCCAACGTAAAAATTT CTGATAACCCAAACACCTACGACTACATGAACAAGCGAGTGGTGGCTCCCGGGTTA GTGGACTGCTACATTAACCTTGGAGCACGCTGGTCCCTTGACTATATGGACAACGTC AACCCATTTAACCACCACCGCAATGCTGGCCTGCGCTACCGCTCAATGTTGCTGGG CAATGGTCGCTATGTGCCCTTCCACATCCAGGTGCCTCAGAAGTTCTTTGCCATTAA AAACCTCCTTCTCCTGCCGGGCTCATACACCTACGAGTGGAACTTCAGGAAGGATGT TAACATGGTTCTGCAGAGCTCCCTAGGAAATGACCTAAGGGTTGACGGAGCCAGCA TTAAGTTTGATAGCATTTGCCTTTACGCCACCTTCTTCCCCATGGCCCACAACACCG CCTCCACGCTTGAGGCCATGCTTAGAAACGACACCAACGACCAGTCCTTTAACGACT ATCTCTCCGCCGCCAACATGCTCTACCCTATACCCGCCAACGCTACCAACGTGCCCA TATCCATCCCCTCCCGCAACTGGGCGGCTTTCCGCGGCTGGGCCTTCACGCGCCTT AAGACTAAGGAAACCCCATCACTGGGCTCGGGCTACGACCCTTATTACACCTACTCT GGCTCTATACCCTACCTAGATGGAACCTTTTACCTCAACCACACCTTTAAGAAGGTG GCCATTACCTTTGACTCTTCTGTCAGCTGGCCTGGCAATGACCGCCTGCTTACCCCC AACGAGTTTGAAATTAAGCGCTCAGTTGACGGGGAGGGTTACAACGTTGCCCAGTG |
| | | TAACATGACCAAAGACTGGTTCCTGGTACAAATGCTAGCTAACTACAACATTGGCTA CCAGGGCTTCTATATCCCAGAGAGCTACAAGGACCGCATGTACTCCTTCTTTAGAAA CTTCCAGCCCATGAGCCGTCAGGTGGTGGATGATACTAAATACAAGGACTACCAACA GGTGGGCATCCTACACCAACACAACAACTCTGGATTTGTTGGCTACCTTGCCCCCAC CATGCGCGAAGGACAGGCCTACCCTGCTAACTTCCCCTATCCGCTTATAGGCAAGA CCGCAGTTGACAGCATTACCCAGAAAAAGTTTCTTTGCGATCGCACCCTTTGGCGCA TCCCATTCTCCAGTAACTTTATGTCCATGGGCGCACTCACAGACCTGGGCCAAAACC TTCTCTACGCCAACTCCGCCCACGCGCTAGACATGACTTTTGAGGTGGATCCCATGG ACGAGCCCACCCTTCTTTATGTTTTGTTTGAAGTCTTTGACGTGGTCCGTGTGCACC GGCCGCACCGCGGCGTCATCGAAACCGTGTACCTGCGCACGCCCTTCTCGGCCGG CAACGCCACAACATAAAGAAGCAAGCAACATCAACAACAGCTGCCGCCATGGGCTC CAGTGAGCAGGAACTGAAAGCCATTGTCAAAGATCTTGGTTGTGGGCCATATTTTTT GGGCACCTATGACAAGCGCTTTCCAGGCTTTGTTTCTCCACACAAGCTCGCCTGCG CCATAGTCAATACGGCCGGTCGCGAGACTGGGGGCGTACACTGGATGGCCTTTGCC TGGAACCCGCACTCAAAAACATGCTACCTCTTTGAGCCCTTTGGCTTTTCTGACCAG CGACTCAAGCAGGTTTACCAGTTTGAGTACGAGTCACTCCTGCGCCGTAGCGCCATT GCTTCTTCCCCCGACCGCTGTATAACGCTGGAAAAGTCCACCCAAAGCGTACAGGG GCCCAACTCGGCCGCCTGTGGACTATTCTGCTGCATGTTTCTCCACGCCTTTGCCAA CTGGCCCCAAACTCCCATGGATCACAACCCCACCATGAACCTTATTACCGGGGTACC CAACTCCATGCTCAACAGTCCCCAGGTACAGCCCACCCTGCGTCGCAACCAGGAAC AGCTCTACAGCTTCCTGGAGCGCCACTCGCCCTACTTCCGCAGCCACAGTGCGCAG ATTAGGAGCGCCACTTCTTTTTGTCACTTGAAAAACATGTAAAAATAATGTACTAGAG ACACTTTCAATAAAGGCAAATGCTTTTATTTGTACACTCTCGGGTGATTATTTACCCC CACCCTTGCCGTCTGCGCCGTTTAAAAATCAAAGGGGTTCTGCCGCGCATCGCTAT GCGCCACTGGCAGGGACACGTTGCGATACTGGTGTTTAGTGCTCCACTTAAACTCA GGCACAACCATCCGCGGCAGCTCGGTGAAGTTTTCACTCCACAGGCTGCGCACCAT CACCAACGCGTTTAGCAGGTCGGGCGCCGATATCTTGAAGTCGCAGTTGGGGCCTC CGCCCTGCGCGCGCGAGTTGCGATACACAGGGTTGCAGCACTGGAACACTATCAGC GCCGGGTGGTGCACGCTGGCCAGCACGCTCTTGTCGGAGATCAGATCCGCGTCCA GGTCCTCCGCGTTGCTCAGGGCGAACGGAGTCAACTTTGGTAGCTGCCTTCCCAAA AAGGGCGCGTGCCCAGGCTTTGAGTTGCACTCGCACCGTAGTGGCATCAAAAGGTG ACCGTGCCCGGTCTGGGCGTTAGGATACAGCGCCTGCATAAAAGCCTTGATCTGCT TAAAAGCCACCTGAGCCTTTGCGCCTTCAGAGAAGAACATGCCGCAAGACTTGCCG GAAAACTGATTGGCCGGACAGGCCGCGTCGTGCACGCAGCACCTTGCGTCGGTGTT GGAGATCTGCACCACATTTCGGCCCCACCGGTTCTTCACGATCTTGGCCTTGCTAGA CTGCTCCTTCAGCGCGCGCTGCCCGTTTTCGCTCGTCACATCCATTTCAATCACGTG CTCCTTATTTATCATAATGCTTCCGTGTAGACACTTAAGCTCGCCTTCGATCTCAGCG CAGCGGTGCAGCCACAACGCGCAGCCCGTGGGCTCGTGATGCTTGTAGGTCACCT CTGCAAACGACTGCAGGTACGCCTGCAGGAATCGCCCCATCATCGTCACAAAGGTC TTGTTGCTGGTGAAGGTCAGCTGCAACCCGCGGTGCTCCTCGTTCAGCCAGGTCTT GCATACGGCCGCCAGAGCTTCCACTTGGTCAGGCAGTAGTTTGAAGTTCGCCTTTA GATCGTTATCCACGTGGTACTTGTCCATCAGCGCGCGCGCAGCCTCCATGCCCTTC TCCCACGCAGACACGATCGGCACACTCAGCGGGTTCATCACCGTAATTTCACTTTCC GCTTCGCTGGGCTCTTCCTCTTCCTCTTGCGTCCGCATACCACGCGCCACTGGGTC GTCTTCATTCAGCCGCCGCACTGTGCGCTTACCTCCTTTGCCATGCTTGATTAGCAC CGGTGGGTTGCTGAAACCCACCATTTGTAGCGCCACATCTTCTCTTTCTTCCTCGCT GTCCACGATTACCTCTGGTGATGGCGGGCGCTCGGGCTTGGGAGAAGGGCGCTTC TTTTTCTTCTTGGGCGCAATGGCCAAATCCGCCGCCGAGGTCGATGGCCGCGGGCT GGGTGTGCGCGGCACCAGCGCGTCTTGTGATGAGTCTTCCTCGTCCTCGGACTCGA TACGCCGCCTCATCCGCTTTTTTGGGGGCGCCCGGGGAGGCGGCGGCGACGGGG ACGGGGACGACACGTCCTCCATGGTTGGGGGACGTCGCGCCGCACCGCGTCCGCG CTCGGGGGTGGTTTCGCGCTGCTCCTCTTCCCGACTGGCCATTTCCTTCTCCTATAG GCAGAAAAAGATCATGGAGTCAGTCGAGAAGAAGGACAGCCTAACCGCCCCCTCTG AGTTCGCCACCACCGCCTCCACCGATGCCGCCAACGCGCCTACCACCTTCCCCGTC GAGGCACCCCCGCTTGAGGAGGAGGAAGTGATTATCGAGCAGGACCCAGGTTTTGT AAGCGAAGACGACGAGGACCGCTCAGTACCAACAGAGGATAAAAAGCAAGACCAGG ACAACGCAGAGGCAAACGAGGAACAAGTCGGGCGGGGGGACGAAAGGCATGGCGA CTACCTAGATGTGGGAGACGACGTGCTGTTGAAGCATCTGCAGCGCCAGTGCGCCA TTATCTGCGACGCGTTGCAAGAGCGCAGCGATGTGCCCCTCGCCATAGCGGATGTC AGCCTTGCCTACGAACGCCACCTATTCTCACCGCGCGTACCCCCCAAACGCCAAGA AAACGGCACATGCGAGCCCAACCCGCGCCTCAACTTCTACCCCGTATTTGCCGTGC CAGAGGTGCTTGCCACCTATCACATCTTTTTCCAAAACTGCAAGATACCCCTATCCTG CCGTGCCAACCGCAGCCGAGCGGACAAGCAGCTGGCCTTGCGGCAGGGCGCTGTC ATACCTGATATCGCCTCGCTCAACGAAGTGCCAAAAATCTTTGAGGGTCTTGGACGC GACGAGAAGCGCGCGGCAAACGCTCTGCAACAGGAAAACAGCGAAAATGAAAGTCA CTCTGGAGTGTTGGTGGAACTCGAGGGTGACAACGCGCGCCTAGCCGTACTAAAAC GCAGCATCGAGGTCACCCACTTTGCCTACCCGGCACTTAACCTACCCCCCAAGGTC ATGAGCACAGTCATGAGTGAGCTGATCGTGCGCCGTGCGCAGCCCCTGGAGAGGG ATGCAAATTTGCAAGAACAAACAGAGGAGGGCCTACCCGCAGTTGGCGACGAGCAG CTAGCGCGCTGGCTTCAAACGCGCGAGCCTGCCGACTTGGAGGAGCGACGCAAAC |
| | | TAATGATGGCCGCAGTGCTCGTTACCGTGGAGCTTGAGTGCATGCAGCGGTTCTTT GCTGACCCGGAGATGCAGCGCAAGCTAGAGGAAACATTGCACTACACCTTTCGACA GGGCTACGTACGCCAGGCCTGCAAGATCTCCAACGTGGAGCTCTGCAACCTGGTCT CCTACCTTGGAATTTTGCACGAAAACCGCCTTGGGCAAAACGTGCTTCATTCCACGC TCAAGGGCGAGGCGCGCCGCGACTACGTCCGCGACTGCGTTTACTTATTTCTATGC TACACCTGGCAGACGGCCATGGGCGTTTGGCAGCAGTGCTTGGAGGAGTGCAACCT CAAGGAGCTGCAGAAACTGCTAAAGCAAAACTTGAAGGACCTATGGACGGCCTTCA ACGAGCGCTCCGTGGCCGCGCACCTGGCGGACATCATTTTCCCCGAACGCCTGCTT AAAACCCTGCAACAGGGTCTGCCAGACTTCACCAGTCAAAGCATGTTGCAGAACTTT AGGAACTTTATCCTAGAGCGCTCAGGAATCTTGCCCGCCACCTGCTGTGCACTTCCT AGCGACTTTGTGCCCATTAAGTACCGCGAATGCCCTCCGCCGCTTTGGGGCCACTG CTACCTTCTGCAGCTAGCCAACTACCTTGCCTACCACTCTGACATAATGGAAGACGT GAGCGGTGACGGTCTACTGGAGTGTCACTGTCGCTGCAACCTATGCACCCCGCACC GCTCCCTGGTTTGCAATTCGCAGCTGCTTAACGAAAGTCAAATTATCGGTACCTTTG AGCTGCAGGGTCCCTCGCCTGACGAAAAGTCCGCGGCTCCGGGGTTGAAACTCACT CCGGGGCTGTGGACGTCGGCTTACCTTCGCAAATTTGTACCTGAGGACTACCACGC CCACGAGATTAGGTTCTACGAAGACCAATCCCGCCCGCCAAATGCGGAGCTTACCG CCTGCGTCATTACCCAGGGCCACATTCTTGGCCAATTGCAAGCCATCAACAAAGCCC GCCAAGAGTTTCTGCTACGAAAGGGACGGGGGGTTTACTTGGACCCCCAGTCCGGC GAGGAGCTCAACCCAATCCCCCCGCCGCCGCAGCCCTATCAGCAGCAGCCGCGGG CCCTTGCTTCCCAGGATGGCACCCAAAAAGAAGCTGCAGCTGCCGCCGCCACCCAC GGACGAGGAGGAATACTGGGACAGTCAGGCAGAGGAGGTTTTGGACGAGGAGGAG GAGGACATGATGGAAGACTGGGAGAGCCTAGACGAGGAAGCTTCCGAGGTCGAAG AGGTGTCAGACGAAACACCGTCACCCTCGGTCGCATTCCCCTCGCCGGCGCCCCA GAAATCGGCAACCGGTTCCAGCATGGCTACAACCTCCGCTCCTCAGGCGCCGCCG GCACTGCCCGTTCGCCGACCCAACCGTAGATGGGACACCACTGGAACCAGGGCCG GTAAGTCCAAGCAGCCGCCGCCGTTAGCCCAAGAGCAACAACAGCGCCAAGGCTAC CGCTCATGGCGCGGGCACAAGAACGCCATAGTTGCTTGCTTGCAAGACTGTGGGGG CAACATCTCCTTCGCCCGCCGCTTTCTTCTCTACCATCACGGCGTGGCCTTCCCCCG TAACATCCTGCATTACTACCGTCATCTCTACAGCCCATACTGCACCGGCGGCAGCGG CAGCGGCAGCAACAGCAGCGGCCACACAGAAGCAAAGGCGACCGGATAGCAAGAC TCTGACAAAGCCCAAGAAATCCACAGCGGCGGCAGCAGCAGGAGGAGGAGCGCTG CGTCTGGCGCCCAACGAACCCGTATCGACCCGCGAGCTTAGAAACAGGATTTTTCC CACTCTGTATGCTATATTTCAACAGAGCAGGGGCCAAGAACAAGAGCTGAAAATAAA AAACAGGTCTCTGCGATCCCTCACCCGCAGCTGCCTGTATCACAAAAGCGAAGATCA GCTTCGGCGCACGCTGGAAGACGCGGAGGCTCTCTTCAGTAAATACTGCGCGCTGA CTCTTAAGGACTAGTTTCGCGCCCTTTCTCAAATTTAAGCGCGAAAACTACGTCATCT CCAGCGGCCACACCCGGCGCCAGCACCTGTCGTCAGCGCCATTATGAGCAAGGAA ATTCCCACGCCCTACATGTGGAGTTACCAGCCACAAATGGGACTTGCGGCTGGAGC TGCCCAAGACTACTCAACCCGAATAAACTACATGAGCGCGGGACCCCACATGATATC CCGGGTCAACGGAATCCGCGCCCACCGAAACCGAATTCTCTTGGAACAGGCGGCTA TTACCACCACACCTCGTAATAACCTTAATCCCCGTAGTTGGCCCGCTGCCCTGGTGT ACCAGGAAAGTCCCGCTCCCACCACTGTGGTACTTCCCAGAGACGCCCAGGCCGAA GTTCAGATGACTAACTCAGGGGCGCAGCTTGCGGGCGGCTTTCGTCACAGGGTGC GGTCGCCCGGGCAGGGTATAACTCACCTGACAATCAGAGGGCGAGGTATTCAGCTC AACGACGAGTCGGTGAGCTCCTCGCTTGGTCTCCGTCCGGACGGGACATTTCAGAT CGGCGGCGCCGGCCGCTCTTCATTCACGCCTCGTCAGGCAATCCTAACTCTGCAGA CCTCGTCCTCTGAGCCGCGCTCTGGAGGCATTGGAACTCTGCAATTTATTGAGGAGT TTGTGCCATCGGTCTACTTTAACCCCTTCTCGGGACCTCCCGGCCACTATCCGGATC AATTTATTCCTAACTTTGACGCGGTAAAGGACTCGGCGGATGGCTACGACTGAATGT TAAGTGGAGAGGCAGAGCAACTGCGCCTGAAACACCTGGTCCACTGTCGCCGCCAC AAGTGCTTTGCCCGCGACTCCGGTGAGTTTTGCTACTTTGAATTGCCCGAGGATCAT ATCGAGGGCCCGGCGCACGGCGTCCGGCTTACCGCCCAGGGAGAGCTTGCCCGTA GCCTGATTCGGGAGTTTACCCAGCGCCCCCTGCTAGTTGAGCGGGACAGGGGACC CTGTGTTCTCACTGTGATTTGCAACTGTCCTAACCCTGGATTACATCAAGATCTTTGT TGCCATCTCTGTGCTGAGTATAATAAATACAGAAATTAAAATATACTGGGGCTCCTAT CGCCATCCTGTAAACGCCACCGTCTTCACCCGCCCAAGCAAACCAAGGCGAACCTT ACCTGGTACTTTTAACATCTCTCCCTCTGTGATTTACAACAGTTTCAACCCAGACGGA GTGAGTCTACGAGAGAACCTCTCCGAGCTCAGCTACTCCATCAGAAAAAACACCACC CTCCTTACCTGCCGGGAACGTACGACCTAGGGATAACAGGGTAATAAGCAATTGACT CTATGTGGGATATGCTCCAGCGCTACAACCTTGAAGTCAGGCTTCCTGGATGTCAGC ATCTGACTTTGGCCAGCACCTGTCCCGCGGATTTGTTCCAGTCCAACTACAGCGACC CACCCTAACAGAGATGACCAACACAACCAACGCGGCCGCCGCTACCGGACTTACAT CTACCACAAATACACCCCAAGTTTCTGCCTTTGTCAATAACTGGGATAACTTGGGCAT GTGGTGGTTCTCCATAGCGCTTATGTTTGTATGCCTTATTATTATGTGGCTCATCTGC TGCCTAAAGCGCAAACGCGCCCGACCACCCATCTATAGTCCCATCATTGTGCTACAC CCAAACAATGATGGAATCCATAGATTGGACGGACTGAAACACATGTTCTTTTCTCTTA CAGTATGATTAAATGAGACATGATTCCTCGAGTTTTTATATTACTGACCCTTGTTGCG CTTTTTTGTGCGTGCTCCACATTGGCTGCGGTTTCTCACATCGAAGTAGACTGCATT CCAGCCTTCACAGTCTATTTGCTTTACGGATTTGTCACCCTCACGCTCATCTGCAGC |
| | | CTCATCACTGTGGTCATCGCCTTTATCCAGTGCATTGACTGGGTCTGTGTGCGCTTT GCATATCTCAGACACCATCCCCAGTACAGGGACAGGACTATAGCTGAGCTTCTTAGA ATTCTTTAATTATGAAATTTACTGTGACTTTTCTGCTGATTATTTGCACCCTATCTGCG TTTTGTTCCCCGACCTCCAAGCCTCAAAGACATATATCATGCAGATTCACTCGTATAT GGAATATTCCAAGTTGCTACAATGAAAAAAGCGATCTTTCCGAAGCCTGGTTATATGC AATCATCTCTGTTATGGTGTTCTGCAGTACCATCTTAGCCCTAGCTATATATCCCTAC CTTGACATTGGCTGGAAACGAATAGATGCCATGAACCACCCAACTTTCCCCGCGCCC GCTATGCTTCCACTGCAACAAGTTGTTGCCGGCGGCTTTGTCCCAGCCAATCAGCCT CGCCCCACTTCTCCCACCCCCACTGAAATCAGCTACTTTAATCTAACAGGAGGAGAT GACTGACACCCTAGATCTAGAAATGGACGGAATTATTACAGAGCAGCGCCTGCTAGA AAGACGCAGGGCAGCGGCCGAGCAACAGCGCATGAATCAAGAGCTCCAAGACATG GTTAACTTGCACCAGTGCAAAAGGGGTATCTTTTGTCTGGTAAAGCAGGCCAAAGTC ACCTACGACAGTAATACCACCGGACACCGCCTTAGCTACAAGTTGCCAACCAAGCGT CAGAAATTGGTGGTCATGGTGGGAGAAAAGCCCATTACCATAACTCAGCACTCGGTA GAAACCGAAGGCTGCATTCACTCACCTTGTCAAGGACCTGAGGATCTCTGCACCCTT ATTAAGACCCTGTGCGGTCTCAAAGATCTTATTCCCTTTAACTAATAAAAAAAAATAAT AAAGCATCACTTACTTAAAATCAGTTAGCAAATTTCTGTCCAGTTTATTCAGCAGCAC CTCCTTGCCCTCCTCCCAGCTCTGGTATTGCAGCTTCCTCCTGGCTGCAAACTTTCT CCACAATCTAAATGGAATGTCAGTTTCCTCCTGTTCCTGTCCATCCGCACCCACTATC TTCATGTTGTTGCAGATGAAGCGCGCAAGACCGTCTGAAGATACCTTCAACCCCGTG TATCCATATGACACGGAAACCGGTCCTCCAACTGTGCCTTTTCTTACTCCTCCCTTTG TATCCCCCAATGGGTTTCAAGAGAGTCCCCCTGGGGTACTCTCTTTGCGCCTATCCG AACCTCTAGTTACCTCCAATGGCATGCTTGCGCTCAAAATGGGCAACGGCCTCTCTC TGGACGAGGCCGGCAACCTTACCTCCCAAAATGTAACCACTGTGAGCCCACCTCTC AAAAAAACCAAGTCAAACATAAACCTGGAAATATCTGCACCCCTCACAGTTACCTCAG AAGCCCTAACTGTGGCTGCCGCCGCACCTCTAATGGTCGCGGGCAACACACTCACC ATGCAATCACAGGCCCCGCTAACCGTGCACGACTCCAAACTTAGCATTGCCACCCAA GGACCCCTCACAGTGTCAGAAGGAAAGCTAGCCCTGCAAACATCAGGCCCCCTCAC CACCACCGATAGCAGTACCCTTACTATCACTGCCTCACCCCCTCTAACTACTGCCAC TGGTAGCTTGGGCATTGACTTGAAAGAGCCCATTTATACACAAAATGGAAAACTAGG ACTAAAGTACGGGGCTCCTTTGCATGTAACAGACGACCTAAACACTTTGACCGTAGC AACTGGTCCAGGTGTGACTATTAATAATACTTCCTTGCAAACTAAAGTTACTGGAGCC TTGGGTTTTGATTCACAAGGCAATATGCAACTTAATGTAGCAGGAGGACTAAGGATT GATTCTCAAAACAGACGCCTTATACTTGATGTTAGTTATCCGTTTGATGCTCAAAACC AACTAAATCTAAGACTAGGACAGGGCCCTCTTTTTATAAACTCAGCCCACAACTTGGA TATTAACTACAACAAAGGCCTTTACTTGTTTACAGCTTCAAACAATTCCAAAAAGCTTG AGGTTAACCTAAGCACTGCCAAGGGGTTGATGTTTGACGCTACAGCCATAGCCATTA ATGCAGGAGATGGGCTTGAATTTGGTTCACCTAATGCACCAAACACAAATCCCCTCA AAACAAAAATTGGCCATGGCCTAGAATTTGATTCAAACAAGGCTATGGTTCCTAAACT AGGAACTGGCCTTAGTTTTGACAGCACAGGTGCCATTACAGTAGGAAACAAAAATAA TGATAAGCTAACCCTATGGACAGGTCCAAAACCAGAAGCCAACTGCATAATTGAATA CGGGAAACAAAACCCAGATAGCAAACTAACTTTAATCCTTGTAAAAAATGGAGGAATT GTTAATGGATATGTAACGCTAATGGGAGCCTCAGACTACGTTAACACCTTATTTAAAA ACAAAAATGTCTCCATTAATGTAGAACTATACTTTGATGCCACTGGTCATATATTACCA GACTCATCTTCTCTTAAAACAGATCTAGAACTAAAATACAAGCAAACCGCTGACTTTA GTGCAAGAGGTTTTATGCCAAGTACTACAGCGTATCCATTTGTCCTTCCTAATGCGG GAACACATAATGAAAATTATATTTTTGGTCAATGCTACTACAAAGCAAGCGATGGTGC CCTTTTTCCGTTGGAAGTTACTGTTATGCTTAATAAACGCCTGCCAGATAGTCGCACA TCCTATGTTATGACTTTTTTATGGTCCTTGAATGCTGGTCTAGCTCCAGAAACTACTC AGGCAACCCTCATAACCTCCCCATTTACCTTTTCCTATATTAGAGAAGATGACTAATA AACTCTAAAGAATCGTTTGTGTTATGTTTCAACGTGTTTATTTTTCAATTGCAGAAAAT TTCAAGTCATTTTTCATTCAGTAGTATAGCCCCACCACCACATAGCTTATACAGATCA CCGTACCTTAATCAAACTCACAGAACCCTAGTATTCAACCTGCCACCTCCCTCCCAA CACACAGAGTACACAGTCCTTTCTCCCCGGCTGGCCTTAAAAAGCATCATATCATGG GTAACAGACATATTCTTAGGTGTTATATTCCACACGGTTTCCTGTCGAGCCAAACGCT CATCAGTGATATTAATAAACTCCCCGGGCAGCTCACTTAAGTTCATGTCGCTGTCCA GCTGCTGAGCCACAGGCTGCTGTCCAACTTGCGGTTGCTTAACGGGCGGCGAAGG AGAAGTCCACGCCTACATGGGGGTAGAGTCATAATCGTGCATCAGGATAGGGCGGT GGTGCTGCAGCAGCGCGCGAATAAACTGCTGCCGCCGCCGCTCCGTCCTGCAGGA ATACAACATGGCAGTGGTCTCCTCAGCGATGATTCGCACCGCCCGCAGCATAAGGC GCCTTGTCCTCCGGGCACAGCAGCGCACCCTGATCTCACTTAAATCAGCACAGTAA CTGCAGCACAGCACCACAATATTGTTCAAAATCCCACAGTGCAAGGCGCTGTATCCA AAGCTCATGGCGGGGACCACAGAACCCACGTGGCCATCATACCACAAGCGCAGGTA GATTAAGTGGCGACCCCTCATAAACACGCTGGACATAAACATTACCTCTTTTGGCAT GTTGTAATTCACCACCTCCCGGTACCATATAAACCTCTGATTAAACATGGCGCCATC CACCACCATCCTAAACCAGCTGGCCAAAACCTGCCCGCCGGCTATACACTGCAGGG AACCGGGACTGGAACAATGACAGTGGAGAGCCCAGGACTCGTAACCATGGATCATC ATGCTCGTCATGATATCAATGTTGGCACAACACAGGCACACGTGCATACACTTCCTC AGGATTACAAGCTCCTCCCGCGTTAGAACCATATCCCAGGGAACAACCCATTCCTGA ATCAGCGTAAATCCCACACTGCAGGGAAGACCTCGCACGTAACTCACGTTGTGCATT GTCAAAGTGTTACATTCGGGCAGCAGCGGATGATCCTCCAGTATGGTAGCGCGGGT TTCTGTCTCAAAAGGAGGTAGACGATCCCTACTGTACGGAGTGCGCCGAGACAACC GAGATCGTGTTGGTCGTAGTGTCATGCCAAATGGAACGCCGGACGTAGTCATATTTC CTGAAGCAAAACCAGGTGCGGGCGTGACAAACAGATCTGCGTCTCCGGTCTCGCCG CTTAGATCGCTCTGTGTAGTAGTTGTAGTATATCCACTCTCTCAAAGCATCCAGGCG CCCCCTGGCTTCGGGTTCTATGTAAACTCCTTCATGCGCCGCTGCCCTGATAACATC CACCACCGCAGAATAAGCCACACCCAGCCAACCTACACATTCGTTCTGCGAGTCACA CACGGGAGGAGCGGGAAGAGCTGGAAGAACCATGTTTTTTTTTTTATTCCAAAAGAT TATCCAAAACCTCAAAATGAAGATCTATTAAGTGAACGCGCTCCCCTCCGGTGGCGT GGTCAAACTCTACAGCCAAAGAACAGATAATGGCATTTGTAAGATGTTGCACAATGG CTTCCAAAAGGCAAACGGCCCTCACGTCCAAGTGGACGTAAAGGCTAAACCCTTCA GGGTGAATCTCCTCTATAAACATTCCAGCACCTTCAACCATGCCCAAATAATTCTCAT CTCGCCACCTTCTCAATATATCTCTAAGCAAATCCCGAATATTAAGTCCGGCCATTGT AAAAATCTGCTCCAGAGCGCCCTCCACCTTCAGCCTCAAGCAGCGAATCATGATTGC AAAAATTCAGGTTCCTCACAGACCTGTATAAGATTCAAAAGCGGAACATTAACAAAAA TACCGCGATCCCGTAGGTCCCTTCGCAGGGCCAGCTGAACATAATCGTGCAGGTCT GCACGGACCAGCGCGGCCACTTCCCCGCCAGGAACCTTGACAAAAGAACCCACACT GATTATGACACGCATACTCGGAGCTATGCTAACCAGCGTAGCCCCGATGTAAGCTTT GTTGCATGGGCGGCGATATAAAATGCAAGGTGCTGCTCAAAAAATCAGGCAAAGCC TCGCGCAAAAAAGAAAGCACATCGTAGTCATGCTCATGCAGATAAAGGCAGGTAAGC TCCGGAACCACCACAGAAAAAGACACCATTTTTCTCTCAAACATGTCTGCGGGTTTC TGCATAAACACAAAATAAAATAACAAAAAAACATTTAAACATTAGAAGCCTGTCTTACA ACAGGAAAAACAACCCTTATAAGCATAAGACGGACTACGGCCATGCCGGCGTGACC GTAAAAAAACTGGTCACCGTGATTAAAAAGCACCACCGACAGCTCCTCGGTCATGTC CGGAGTCATAATGTAAGACTCGGTAAACACATCAGGTTGATTCATCGGTCAGTGCTA AAAAGCGACCGAAATAGCCCGGGGGAATACATACCCGCAGGCGTAGAGACAACATT ACAGCCCCCATAGGAGGTATAACAAAATTAATAGGAGAGAAAAACACATAAACACCT GAAAAACCCTCCTGCCTAGGCAAAATAGCACCCTCCCGCTCCAGAACAACATACAGC GCTTCACAGCGGCAGCCTAACAGTCAGCCTTACCAGTAAAAAAGAAAACCTATTAAA AAAACACCACTCGACACGGCACCAGCTCAATCAGTCACAGTGTAAAAAAGGGCCAA GTGCAGAGCGAGTATATATAGGACTAAAAAATGACGTAACGGTTAAAGTCCACAAAA AACACCCAGAAAACCGCACGCGAACCTACGCCCAGAAACGAAAGCCAAAAAACCCA CAACTTCCTCAAATCGTCACTTCCGTTTTCCCACGTTACGTAACTTCCCATTTTAAGA AAACTACAATTCCCAACACATACAAGTTACTCCGCCCTAAAACCTACGTCACCCGCC CCGTTCCCACGCCCCGCGCCACGTCACAAACTCCACCCCCTCATTATCATATTGGCT TCAATCCAAAATAAGGTATATTATTGATGATGTTAAT |
| 127 | ICOSTAT | TAACATCATCAATTATACCTTCCATTTTGGATTGAAGCCAATATGATAATGAGGGGGT GGAGTTTGTGACGTGGCGCGGGGCGTGGGAACGGGGCGGGTGACGTAGTAGTGT GGCGGAAGTGTGATGTTGCAAGTGTGGCGGAACACATGTAAGCGACGGATGTGGCA AAAGTGACGTTTTTGGTGTGCGCCGGTGTACACAGGAAGTGACAATTTTCGCGCGG TTTTAGGCGGATGTTGTAGTAAATTTGGGCGTAACCGAGTAAGATTTGGCCATTTTC GCGGGAAAACTGAATAAGAGGAAGTGAAATCTGAATAATTTTGTGTTACTCATAGCG CGTAATATTTGTCTAGGGCCGCGGGGACTTTGACCGTTTACGTGGAGACTCGCCCA GGTGTTTTTCTCAGGTGTTTTCCGCGTACGTCGGCGGCTCGTGGCTCTTCCGGGAA AAGGATTCTCGGAAAGTGGTTCGAGTACGTCGGCGGCTCGTGGCTCTTCCGGGAAA AGGATTCTCGGAAAGTGGTTCGAAGTACGTCGACCACAAACCCCGCCCAGCGTCTT GTCATTGGCGTCGACGCTGTACGGGGTCAAAGTTGGCGTTTTATTATTATAGTCAGC TGACGTGTAGTGTATTTATACCCGGTGAGTTCCTCAAGAGGCCACTCTTGAGTGCCA GCGAGTAGAGTTTTCTCCTCCGAGCCGCTCCGACACCGGGACTGAAAATGAGACAT ATTATCTGCCACGGAGGTGTTATTACCGAAGAAATGGCCGCCAGTCTTTTGGACCAG CTGATCGAAGAGGTACTGGCTGATAATCTTCCACCTCCTAGCCATTTTGAACCACCT ACCCTTCACGAACTGTATGATTTAGACGTGACGGCCCCCGAAGATCCCAACGAGGA GGCGGTTTCGCAGATTTTTCCCGACTCTGTAATGTTGGCGGTGCAGGAAGGGATTG ACTTACTCACTTTTCCGCCGGCGCCCGGTTCTCCGGAGCCGCCTCACCTTTCCCGG CAGCCCGAGCAGCCGGAGCAGAGAGCCTTGGGTCCGGTTTCTATGCCAAACCTTGT ACCGGAGGTGATCGATCCACCCAGTGACGACGAGGATGAAGAGGGTGAGGAGTTT GTGTTAGATTATGTGGAGCACCCCGGGCACGGTTGCAGGTCTTGTCATTATCACCG GAGGAATACGGGGGACCCAGATATTATGTGTTCGCTTTGCTATATGAGGACCTGTGG CATGTTTGTCTACAGTAAGTGAAAATTATGGGCAGTGGGTGATAGAGTGGTGGGTTT GGTGTGGTAATTTTTTTTTTAATTTTTACAGTTTTGTGGTTTAAAGAATTTTGTATTGTG ATTTTTTTAAAAGGTCCTGTGTCTGAACCTGAGCCTGAGCCCGAGCCAGAACCGGAG CCTGCAAGACCTACCCGCCGTCCTAAAATGGCGCCTGCTATCCTGAGACGCCCGAC ATCACCTGTGTCTAGAGAATGCAATAGTAGTACGGATAGCTGTGACTCCGGTCCTTC TAACACACCTCCTGAGATACACCCGGTGGTCCCGCTGTGCCCCATTAAACCAGTTGC CGTGAGAGTTGGTGGGCGTCGCCAGGCTGTGGAATGTATCGAGGACTTGCTTAACG AGCCTGGGCAACCTTTGGACTTGAGCTGTAAACGCCCCAGGCCATAAGGTGTAAAC CTGTGATTGCGTGTGTGGTTAACGCCTTTGTTTGCTGAATGAGTTGATGTAAGTTTAA TAAAGGGTGAGATAATGTTTAACTTGCATGGCGTGTTAAATGGGGCGGGGCTTAAAG GGTATATAATGCGCCGTGGGCTAATCTTGGTTACATCTGACCTCATGGAGGCTTGGG AGTGTTTGGAAGATTTTTCTGCTGTGCGTAACTTGCTGGAACAGAGCTCTAACAGTA |
| | | CCTCTTGGTTTTGGAGGTTTCTGTGGGGCTCATCCCAGGCAAAGTTAGTCTGCAGAA TTAAGGAGGATTACAAGTGGGAATTTGAAGAGCTTTTGAAATCCTGTGGTGAGCTGT TTGATTCTTTGAATCTGGGTCACCAGGCGCTTTTCCAAGAGAAGGTCATCAAGACTTT GGATTTTTCCACACCGGGGCGCGCTGCGGCTGCTGTTGCTTTTTTGAGTTTTATAAA GGATAAATGGAGCGAAGAAACCCATCTGAGCGGGGGGTACCTGCTGGATTTTCTGG CCATGCATCTGTGGAGAGCGGTTGTGAGACACAAGAATCGCCTGCTACTGTTGTCTT CCGTCCGCCCGGCGATAATACCGACGGAGGAGCAGCAGCAGCAGCAGGAGGAAGC CAGGCGGCGGCGGCAGGAGCAGAGCCCATGGAACCCGAGAGCCGGCCTGGACCC TCGGGAATGAATGTTGTACAGGTGGCTGAACTGTATCCAGAACTGAGACGCATTTTG ACAATTACAGAGGATGGGCAGGGGCTAAAGGGGGTAAAGAGGGAGCGGGGGGCTT GTGAGGCTACAGAGGAGGCTAGGAATCTAGCTTTTAGCTTAATGACCAGACACCGTC CTGAGTGTATTACTTTTCAACAGATCAAGGATAATTGCGCTAATGAGCTTGATCTGCT GGCGCAGAAGTATTCCATAGAGCAGCTGACCACTTACTGGCTGCAGCCAGGGGATG ATTTTGAGGAGGCTATTAGGGTATATGCAAAGGTGGCACTTAGGCCAGATTGCAAGT ACAAGATCAGCAAACTTGTAAATATCAGGAATTGTTGCTACATTTCTGGGAACGGGG CCGAGGTGGAGATAGATACGGAGGATAGGGTGGCCTTTAGATGTAGCATGATAAAT ATGTGGCCGGGGGTGCTTGGCATGGACGGGGTGGTTATTATGAATGTAAGGTTTAC TGGCCCCAATTTTAGCGGTACGGTTTTCCTGGCCAATACCAACCTTATCCTACACGG TGTAAGCTTCTATGGGTTTAACAATACCTGTGTGGAAGCCTGGACCGATGTAAGGGT TCGGGGCTGTGCCTTTTACTGCTGCTGGAAGGGGGTGGTGTGTCGCCCCAAAAGCA GGGCTTCAATTAAGAAATGCCTCTTTGAAAGGTGTACCTTGGGTATCCTGTCTGAGG GTAACTCCAGGGTGCGCCACAATGTGGCCTCCGACTGTGGTTGCTTCATGCTAGTG AAAAGCGTGGCTGTGATTAAGCATAACATGGTATGTGGCAACTGCGAGGACAGGGC CTCTCAGATGCTGACCTGCTCGGACGGCAACTGTCACCTGCTGAAGACCATTCACG TAGCCAGCCACTCTCGCAAGGCCTGGCCAGTGTTTGAGCATAACATACTGACCCGC TGTTCCTTGCATTTGGGTAACAGGAGGGGGGTGTTCCTACCTTACCAATGCAATTTG AGTCACACTAAGATATTGCTTGAGCCCGAGAGCATGTCCAAGGTGAACCTGAACGG GGTGTTTGACATGACCATGAAGATCTGGAAGGTGCTGAGGTACGATGAGACCCGCA CCAGGTGCAGACCCTGCGAGTGTGGCGGTAAACATATTAGGAACCAGCCTGTGATG CTGGATGTGACCGAGGAGCTGAGGCCCGATCACTTGGTGCTGGCCTGCACCCGCG CTGAGTTTGGCTCTAGCGATGAAGATACAGATTGAGGTACTGAAATGTGTGGGCGTG GCTTAAGGGTGGGAAAGAATATATAAGGTGGGGGTCTTATGTAGTTTTGTATCTGTTT TGCAGCAGCCGCCGCCGCCATGAGCACCAACTCGTTTGATGGAAGCATTGTGAGCT CATATTTGACAACGCGCATGCCCCCATGGGCCGGGGTGCGTCAGAATGTGATGGGC TCCAGCATTGATGGTCGCCCCGTCCTGCCCGCAAACTCTACTACCTTGACCTACGAG ACCGTGTCTGGAACGCCGTTGGAGACTGCAGCCTCCGCCGCCGCTTCAGCCGCTG CAGCCACCGCCCGCGGGATTGTGACTGACTTTGCTTTCCTGAGCCCGCTTGCAAGC AGTGCAGCTTCCCGTTCATCCGCCCGCGATGACAAGTTGACGGCTCTTTTGGCACA ATTGGATTCTTTGACCCGGGAACTTAATGTCGTTTCTCAGCAGCTGTTGGATCTGCG CCAGCAGGTTTCTGCCCTGAAGGCTTCCTCCCCTCCCAATGCGGTTTAAAACATAAA TAAAAAACCAGACTCTGTTTGGATTTGGATCAAGCAAGTGTCTTGCTGTCTTTATTTA GGGGTTTTGCGCGCGCGGTAGGCCCGGGACCAGCGGTCTCGGTCGTTGAGGGTCC TGTGTATTTTTTCCAGGACGTGGTAAAGGTGACTCTGGATGTTCAGATACATGGGCA TAAGCCCGTCTCTGGGGTGGAGGTAGCACCACTGCAGAGCTTCATGCTGCGGGGT GGTGTTGTAGATGATCCAGTCGTAGCAGGAGCGCTGGGCGTGGTGCCTAAAAATGT CTTTCAGTAGCAAGCTGATTGCCAGGGGCAGGCCCTTGGTGTAAGTGTTTACAAAGC GGTTAAGCTGGGATGGGTGCATACGTGGGGATATGAGATGCATCTTGGACTGTATTT TTAGGTTGGCTATGTTCCCAGCCATATCCCTCCGGGGATTCATGTTGTGCAGAACCA CCAGCACAGTGTATCCGGTGCACTTGGGAAATTTGTCATGTAGCTTAGAAGGAAATG CGTGGAAGAACTTGGAGACGCCCTTGTGACCTCCAAGATTTTCCATGCATTCGTCCA TAATGATGGCAATGGGCCCACGGGCGGCGGCCTGGGCGAAGATATTTCTGGGATCA CTAACGTCATAGTTGTGTTCCAGGATGAGATCGTCATAGGCCATTTTTACAAAGCGC GGGCGGAGGGTGCCAGACTGCGGTATAATGGTTCCATCCGGCCCAGGGGCGTAGT TACCCTCACAGATTTGCATTTCCCACGCTTTGAGTTCAGATGGGGGGATCATGTCTA CCTGCGGGGCGATGAAGAAAACGGTTTCCGGGGTAGGGGAGATCAGCTGGGAAGA AAGCAGGTTCCTGAGCAGCTGCGACTTACCGCAGCCGGTGGGCCCGTAAATCACAC CTATTACCGGGTGCAACTGGTAGTTAAGAGAGCTGCAGCTGCCGTCATCCCTGAGC AGGGGGGCCACTTCGTTAAGCATGTCCCTGACTCGCATGTTTTCCCTGACCAAATCC GCCAGAAGGCGCTCGCCGCCCAGCGATAGCAGTTCTTGCAAGGAAGCAAAGTTTTT CAACGGTTTGAGACCGTCCGCCGTAGGCATGCTTTTGAGCGTTTGACCAAGCAGTT CCAGGCGGTCCCACAGCTCGGTCACCTGCTCTACGGCATCTCGATCCAGCATATCT CCTCGTTTCGCGGGTTGGGGCGGCTTTCGCTGTACGGCAGTAGTCGGTGCTCGTCC AGACGGGCCAGGGTCATGTCTTTCCACGGGCGCAGGGTCCTCGTCAGCGTAGTCT GGGTCACGGTGAAGGGGTGCGCTCCGGGCTGCGCGCTGGCCAGGGTGCGCTTGA GGCTGGTCCTGCTGGTGCTGAAGCGCTGCCGGTCTTCGCCCTGCGCGTCGGCCAG GTAGCATTTGACCATGGTGTCATAGTCCAGCCCCTCCGCGGCGTGGCCCTTGGCGC GCAGCTTGCCCTTGGAGGAGGCGCCGCACGAGGGGCAGTGCAGACTTTTGAGGGC GTAGAGCTTGGGCGCGAGAAATACCGATTCCGGGGAGTAGGCATCCGCGCCGCAG GCCCCGCAGACGGTCTCGCATTCCACGAGCCAGGTGAGCTCTGGCCGTTCGGGGT CAAAAACCAGGTTTCCCCCATGCTTTTTGATGCGTTTCTTACCTCTGGTTTCCATGAG |
| | | CCGGTGTCCACGCTCGGTGACGAAAAGGCTGTCCGTGTCCCCGTATACAGACTNNN GTTTTGAGAGGCCTGTCCTCGAGCGGTGTTCCGCGGTCCTCCTCGTATAGAAACTC GGACCACTCTGAGACAAAGGCTCGCGTCCAGGCCAGCACGAAGGAGGCTAAGTGG GAGGGGTAGCGGTCGTTGTCCACTAGGGGGTCCACTCGCTCCAGGGTGTGAAGAC ACATGTCGCCCTCTTCGGCATCAAGGAAGGTGATTGGTTTGTAGGTGTAGGCCACG TGACCGGGTGTTCCTGAAGGGGGGCTATAAAAGGGGGTGGGGGCGCGTTCGTCCT CACTCTCTTCCGCATCGCTGTCTGCGAGGGCCAGCTGTTGGGGTGAGTACTCCCTC TGAAAAGCGGGCATGACTTCTGCGCTAAGATTGTCAGTTTCCAAAAACGAGGAGGAT TTGATATTCACCTGGCCCGCGGTGATGCCTTTGAGGGTGGCCGCATCCATCTGGTC AGAAAAGACAATCTTTTTGTTGTCAAGCTTGGTGGCAAACGACCCGTAGAGGGCGTT GGACAGCAACTTGGCGATGGAGCGCAGGGTTTGGTTTTTGTCGCGATCGGCGCGCT CCTTGGCCGCGATGTTTAGCTGCACGTATTCGCGCGCAACGCACCGCCATTCGGGA AAGACGGTGGTGCGCTCGTCGGGCACCAGGTGCACGCGCCAACCGCGGTTGTGCA GGGTGACAAGGTCAACGCTGGTGGCTACCTCTCCGCGTAGGCGCTCGTTGGTCCA GCAGAGGCGGCCGCCCTTGCGCGAGCAGAATGGCGGTAGGGGGTCTAGCTGCGTC TCGTCCGGGGGGTCTGCGTCCACGGTAAAGACCCCGGGCAGCAGGCGCGCGTCGA AGTAGTCTATCTTGCATCCTTGCAAGTCTAGCGCCTGCTGCCATGCGCGGGCGGCA AGCGCGCGCTCGTATGGGTTGAGTGGGGGACCCCATGGCATGGGGTGGGTGAGCG CGGAGGCGTACATGCCGCAAATGTCGTAAACGTAGAGGGGCTCTCTGAGTATTCCA AGATATGTAGGGTAGCATCTTCCACCGCGGATGCTGGCGCGCACGTAATCGTATAG TTCGTGCGAGGGAGCGAGGAGGTCGGGACCGAGGTTGCTACGGGCGGGCTGCTCT GCTCGGAAGACTATCTGCCTGAAGATGGCATGTGAGTTGGATGATATGGTTGGACG CTGGAAGACGTTGAAGCTGGCGTCTGTGAGACCTACCGCGTCACGCACGAAGGAG GCGTAGGAGTCGCGCAGCTTGTTGACCAGCTCGGCGGTGACCTGCACGTCTAGGG CGCAGTAGTCCAGGGTTTCCTTGATGATGTCATACTTATCCTGTCCCTTTTTTTTCCA CAGCTCGCGGTTGAGGACAAACTCTTCGCGGTCTTTCCAGTACTCTTGGATCGGAAA CCCGTCGGCCTCCGAACGGTAAGAGCCTAGCATGTAGAACTGGTTGACGGCCTGGT AGGCGCAGCATCCCTTTTCTACGGGTAGCGCGTATGCCTGCGCGGCCTTCCGGAGC GAGGTGTGGGTGAGCGCAAAGGTGTCCCTGACCATGACTTTGAGGTACTGGTATTT GAAGTCAGTGTCGTCGCATCCGCCCTGCTCCCAGAGCAAAAAGTCCGTGCGCTTTT TGGAACGCGGATTTGGCAGGGCGAAGGTGACATCGTTGAAGAGTATCTTTCCCGCG CGAGGCATAAAGTTGCGTGTGATGCGGAAGGGTCCCGGCACCTCGGAACGGTTGTT AATTACCTGGGCGGCGAGCACGATCTCGTCAAAGCCGTTGATGTTGTGGCCCACAA TGTAAAGTTCCAAGAAGCGCGGGATGCCCTTGATGGAAGGCAATTTTTTAAGTTCCT CGTAGGTGAGCTCTTCAGGGGAGCTGAGCCCGTGCTCTGAAAGGGCCCAGTCTGC AAGATGAGGGTTGGAAGCGACGAATGAGCTCCACAGGTCACGGGCCATTAGCATTT GCAGGTGGTCGCGAAAGGTCCTAAACTGGCGACCTATGGCCATTTTTTCTGGGGTG ATGCAGTAGAAGGTAAGCGGGTCTTGTTCCCAGCGGTCCCATCCAAGGTTCGCGGC TAGGTCTCGCGCGGCAGTCACTAGAGGCTCATCTCCGCCGAACTTCATGACCAGCA TGAAGGGCACGAGCTGCTTCCCAAAGGCCCCCATCCAAGTATAGGTCTCTACATCG TAGGTGACAAAGAGACGCTCGGTGCGAGGATGCGAGCCGATCGGGAAGAACTGGA TCTCCCGCCACCAATTGGAGGAGTGGCTATTGATGTGGTGAAAGTAGAAGTCCCTG CGACGGGCCGAACACTCGTGCTGGCTTTTGTAAAAACGTGCGCAGTACTGGCAGCG GTGCACGGGCTGTACATCCTGCACGAGGTTGACCTGACGACCGCGCACAAGGAAG CAGAGTGGGAATTTGAGCCCCTCGCCTGGCGGGTTTGGCTGGTGGTCTTCTACTTC GGCTGCTTGTCCTTGACCGTCTGGCTGCTCGAGGGGAGTTACGGTGGATCGGACCA CCACGCCGCGCGAGCCCAAAGTCCAGATGTCCGCGCGCGGCGGTCGGAGCTTGAT GACAACATCGCGCAGATGGGAGCTGTCCATGGTCTGGAGCTCCCGCGGCGTCAGG TCAGGCGGGAGCTCCTGCAGGTTTACCTCGCATAGACGGGTCAGGGCGCGGGCTA GATCCAGGTGATACCTAATTTCCAGGGGCTGGTTGGTGGCGGCGTCGATGGCTTGC AAGAGGCCGCATCCCCGCGGCGCGACTACGGTACCGCGCGGCGGGCGGTGGGCC GCGGGGGTGTCCTTGGATGATGCATCTAAAAGCGGTGACGCGGGCGAGCCCCCGG AGGTAGGGGGGGCTCCGGACCCGCCGGGAGAGGGGGCAGGGGCACGTCGGCGC CGCGCGCGGGCAGGAGCTGGTGCTGCGCGCGTAGGTTGCTGGCGAACGCGACGA CGCGGCGGTTGATCTCCTGAATCTGGCGCCTCTGCGTGAAGACGACGGGCCCGGT GAGCTTGAGCCTGAAAGAGAGTTCGACAGAATCAATTTCGGTGTCGTTGACGGCGG CCTGGCGCAAAATCTCCTGCACGTCTCCTGAGTTGTCTTGATAGGCGATCTCGGCCA TGAACTGCTCGATCTCTTCCTCCTGGAGATCTCCGCGTCCGGCTCGCTCCACGGTG GCGGCGAGGTCGTTGGAAATGCGGGCCATGAGCTGCGAGAAGGCGTTGAGGCCTC CCTCGTTCCAGACGCGGCTGTAGACCACGCCCCCTTCGGCATCGCGGGCGCGCAT GACCACCTGCGCGAGATTGAGCTCCACGTGCCGGGCGAAGACGGCGTAGTTTCGC AGGCGCTGAAAGAGGTAGTTGAGGGTGGTGGCGGTGTGTTCTGCCACGAAGAAGTA CATAACCCAGCGTCGCAACGTGGATTCGTTGATATCCCCCAAGGCCTCAAGGCGCT CCATGGCCTCGTAGAAGTCCACGGCGAAGTTGAAAAACTGGGAGTTGCGCGCCGAC ACGGTTAACTCCTCCTCCAGAAGACGGATGAGCTCGGCGACAGTGTCGCGCACCTC GCGCTCAAAGGCTACAGGGGCCTCTTCTTCTTCTTCAATCTCCTCTTCCATAAGGGC CTCCCCTTCTTCTTCTTCTGGCGGCGGTGGGGGAGGGGGGACACGGCGGCGACGA CGGCGCACCGGGAGGCGGTCGACAAAGCGCTCGATCATCTCCCCGCGGCGACGG CGCATGGTCTCGGTGACGGCGCGGCCGTTCTCGCGGGGGCGCAGTTGGAAGACGC CGCCCGTCATGTCCCGGTTATGGGTTGGCGGGGGGCTGCCATGCGGCAGGGATAC |
| | | GGCGCTAACGATGCATCTCAACAATTGTTGTGTAGGTACTCCGCCGCCGAGGGACC TGAGCGAGTCCGCATCGACCGGATCGGAAAACCTCTCGAGAAAGGCGTCTAACCAG TCACAGTCGCAAGGTAGGCTGAGCACCGTGGCGGGCGGCAGCGGGCGGCGGTCG GGGTTGTTTCTGGCGGAGGTGCTGCTGATGATGTAATTAAAGTAGGCGGTCTTGAG ACGGCGGATGGTCGACAGAAGCACCATGTCCTTGGGTCCGGCCTGCTGAATGCGC AGGCGGTCGGCCATGCCCCAGGCTTCGTTTTGACATCGGCGCAGGTCTTTGTAGTA GTCTTGCATGAGCCTTTCTACCGGCACTTCTTCTTCTCCTTCCTCTTGTCCTGCATCT CTTGCATCTATCGCTGCGGCGGCGGCGGAGTTTGGCCGTAGGTGGCGCCCTCTTC CTCCCATGCGTGTGACCCCGAAGCCCCTCATCGGCTGAAGCAGGGCTAGGTCGGC GACAACGCGCTCGGCTAATATGGCCTGCTGCACCTGCGTGAGGGTAGACTGGAAGT CATCCATGTCCACAAAGCGGTGGTATGCGCCCGTGTTGATGGTGTAAGTGCAGTTG GCCATAACGGACCAGTTAACGGTCTGGTGACCCGGCTGCGAGAGCTCGGTGTACCT GAGACGCGAGTAAGCCCTCGAGTCAAATACGTAGTCGTTGCAAGTCCGCACCAGGT ACTGGTATCCCACCAAAAAGTGCGGCGGCGGCTGGCGGTAGAGGGGCCAGCGTAG GGTGGCCGGGGCTCCGGGGGCGAGATCTTCCAACATAAGGCGATGATATCCGTAG ATGTACCTGGACATCCAGGTGATGCCGGCGGCGGTGGTGGAGGCGCGCGGAAAGT CGCGGACGCGGTTCCAGATGTTGCGCAGCGGCAAAAAGTGCTCCATGGTCGGGAC GCTCTGGCCGGTCAGGCGCGCGCAATCGTTGACGCTCTACCGTGCAAAAGGAGAG CCTGTAAGCGGGCACTCTTCCGTGGTCTGGTGGATAAATTCGCAAGGGTATCATGG CGGACGACCGGGGTTCGAGCCCCGTATCCGGCCGTCCGCCGTGATCCATGCGGTT ACCGCCCGCGTGTCGAACCCAGGTGTGCGACGTCAGACAACGGGGGAGTGCTCCT TTTGGCTTCCTTCCAGGCGCGGCGGCTGCTGCGCTAGCTTTTTTGGCCACTGGCCG CGCGCAGCGTAAGCGGTTAGGCTGGAAAGCGAAAGCATTAAGTGGCTCGCTCCCTG TAGCCGGAGGGTTATTTTCCAAGGGTTGAGTCGCGGGACCCCCGGTTCGAGTCTCG GACCGGCCGGACTGCGGCGAACGGGGGTTTGCCTCCCCGTCATGCAAGACCCCGC TTGCAAATTCCTCCGGAAACAGGGACGAGCCCCTTTTTTGCTTTTCCCAGATGCATC CGGTGCTGCGGCAGATGCGCCCCCCTCCTCAGCAGCGGCAAGAGCAAGAGCAGCG GCAGACATGCAGGGCACCCTCCCCTCCTCCTACCGCGTCAGGAGGGGCGACATCC GCGGTTGACGCGGCAGCAGATGGTGATTACGAACCCCCGCGGCGCCGGGCCCGG CACTACCTGGACTTGGAGGAGGGCGAGGGCCTGGCGCGGCTAGGAGCGCCCTCTC CTGAGCGGTACCCAAGGGTGCAGCTGAAGCGTGATACGCGTGAGGCGTACGTGCC GCGGCAGAACCTGTTTCGCGACCGCGAGGGAGAGGAGCCCGAGGAGATGCGGGAT CGAAAGTTCCACGCAGGGCGCGAGCTGCGGCATGGCCTGAATCGCGAGCGGTTGC TGCGCGAGGAGGACTTTGAGCCCGACGCGCGAACCGGGATTAGTCCCGCGCGCGC ACACGTGGCGGCCGCCGACCTGGTAACCGCATACGAGCAGACGGTGAACCAGGAG ATTAACTTTCAAAAAAGCTTTAACAACCACGTGCGTACGCTTGTGGCGCGCGAGGAG GTGGCTATAGGACTGATGCATCTGTGGGACTTTGTAAGCGCGCTGGAGCAAAACCC AAATAGCAAGCCGCTCATGGCGCAGCTGTTCCTTATAGTGCAGCACAGCAGGGACA ACGAGGCATTCAGGGATGCGCTGCTAAACATAGTAGAGCCCGAGGGCCGCTGGCT GCTCGATTTGATAAACATCCTGCAGAGCATAGTGGTGCAGGAGCGCAGCTTGAGCC TGGCTGACAAGGTGGCCGCCATCAACTATTCCATGCTTAGCCTGGGCAAGTTTTACG CCCGCAAGATATACCATACCCCTTACGTTCCCATAGACAAGGAGGTAAAGATCGAGG GGTTCTACATGCGCATGGCGCTGAAGGTGCTTACCTTGAGCGACGACCTGGGCGTT TATCGCAACGAGCGCATCCACAAGGCCGTGAGCGTGAGCCGGCGGCGCGAGCTCA GCGACCGCGAGCTGATGCACAGCCTGCAAAGGGCCCTGGCTGGCACGGGCAGCG GCGATAGAGAGGCCGAGTCCTACTTTGACGCGGGCGCTGACCTGCGCTGGGCCCC AAGCCGACGCGCCCTGGAGGCAGCTGGGGCCGGACCTGGGCTGGCGGTGGCACC CGCGCGCGCTGGCAACGTCGGCGGCGTGGAGGAATATGACGAGGACGATGAGTAC GAGCCAGAGGACGGCGAGTACTAAGCGGTGATGTTTCTGATCAGATGATGCAAGAC GCAACGGACCCGGCGGTGCGGGCGGCGCTGCAGAGCCAGCCGTCCGGCCTTAAC TCCACGGACGACTGGCGCCAGGTCATGGACCGCATCATGTCGCTGACTGCGCGCA ATCCTGACGCGTTCCGGCAGCAGCCGCAGGCCAACCGGCTCTCCGCAATTCTGGAA GCGGTGGTCCCGGCGCGCGCAAACCCCACGCACGAGAAGGTGCTGGCGATCGTAA ACGCGCTGGCCGAAAACAGGGCCATCCGGCCCGACGAGGCCGGCCTGGTCTACGA CGCGCTGCTTCAGCGCGTGGCTCGTTACAACAGCGGCAACGTGCAGACCAACCTG GACCGGCTGGTGGGGGATGTGCGCGAGGCCGTGGCGCAGCGTGAGCGCGCGCAG CAGCAGGGCAACCTGGGCTCCATGGTTGCACTAAACGCCTTCCTGAGTACACAGCC CGCCAACGTGCCGCGGGGACAGGAGGACTACACCAACTTTGTGAGCGCACTGCGG CTAATGGTGACTGAGACACCGCAAAGTGAGGTGTACCAGTCTGGGCCAGACTATTTT TTCCAGACCAGTAGACAAGGCCTGCAGACCGTAAACCTGAGCCAGGCTTTCAAAAA CTTGCAGGGGCTGTGGGGGGTGCGGGCTCCCACAGGCGACCGCGCGACCGTGTC TAGCTTGCTGACGCCCAACTCGCGCCTGTTGCTGCTGCTAATAGCGCCCTTCACGG ACAGTGGCAGCGTGTCCCGGGACACATACCTAGGTCACTTGCTGACACTGTACCGC GAGGCCATAGGTCAGGCGCATGTGGACGAGCATACTTTCCAGGAGATTACAAGTGT CAGCCGCGCGCTGGGGCAGGAGGACACGGGCAGCCTGGAGGCAACCCTAAACTAC CTGCTGACCAACCGGCGGCAGAAGATCCCCTCGTTGCACAGTTTAAACAGCGAGGA GGAGCGCATTTTGCGCTACGTGCAGCAGAGCGTGAGCCTTAACCTGATGCGCGACG GGGTAACGCCCAGCGTGGCGCTGGACATGACCGCGCGCAACATGGAACCGGGCAT GTATGCCTCAAACCGGCCGTTTATCAACCGCCTAATGGACTACTTGCATCGCGCGG CCGCCGTGAACCCCGAGTATTTCACCAATGCCATCTTGAACCCGCACTGGCTACCG |
| | | CCCCCTGGTTTCTACACCGGGGGATTCGAGGTGCCCGAGGGTAACGATGGATTCCT CTGGGACGACATAGACGACAGCGTGTTTTCCCCGCAACCGCAGACCCTGCTAGAGT TGCAACAGCGCGAGCAGGCAGAGGCGGCGCTGCGAAAGGAAAGCTTCCGCAGGCC AAGCAGCTTGTCCGATCTAGGCGCTGCGGCCCCGCGGTCAGATGCTAGTAGCCCAT TTCCAAGCTTGATAGGGTCTCTTACCAGCACTCGCACCACCCGCCCGCGCCTGCTG GGCGAGGAGGAGTACCTAAACAACTCGCTGCTGCAGCCGCAGCGCGAAAAAAACCT GCCTCCGGCATTTCCCAACAACGGGATAGAGAGCCTAGTGGACAAGATGAGTAGAT GGAAGACGTACGCGCAGGAGCACAGGGACGTGCCAGGCCCGCGCCCGCCCACCC GTCGTCAAAGGCACGACCGTCAGCGGGGTCTGGTGTGGGAGGACGATGACTCGGC AGACGACAGCAGCGTCCTGGATTTGGGAGGGAGTGGCAACCCGTTTGCGCACCTTC GCCCCAGGCTGGGGAGAATGTTTTAAAAAAAAAAAAGCATGATGCAAAATAAAAAAC TCACCAAGGCCATGGCACCGAGCGTTGGTTTTCTTGTATTCCCCTTAGTATGCGGCG CGCGGCGATGTATGAGGAAGGTCCTCCTCCCTCCTACGAGAGTGTGGTGAGCGCG GCGCCAGTGGCGGCGGCGCTGGGTTCTCCCTTCGATGCTCCCCTGGACCCGCCGT TTGTGCCTCCGCGGTACCTGCGGCCTACCGGGGGGAGAAACAGCATCCGTTACTCT GAGTTGGCACCCCTATTCGACACCACCCGTGTGTACCTGGTGGACAACAAGTCAAC GGATGTGGCATCCCTGAACTACCAGAACGACCACAGCAACTTTCTGACCACGGTCAT TCAAAACAATGACTACAGCCCGGGGGAGGCAAGCACACAGACCATCAATCTTGACG ACCGGTCGCACTGGGGCGGCGACCTGAAAACCATCCTGCATACCAACATGCCAAAT GTGAACGAGTTCATGTTTACCAATAAGTTTAAGGCGCGGGTGATGGTGTCGCGCTTG CCTACTAAGGACAATCAGGTGGAGCTGAAATACGAGTGGGTGGAGTTCACGCTGCC CGAGGGCAACTACTCCGAGACCATGACCATAGACCTTATGAACAACGCGATCGTGG AGCACTACTTGAAAGTGGGCAGACAGAACGGGGTTCTGGAAAGCGACATCGGGGTA AAGTTTGACACCCGCAACTTCAGACTGGGGTTTGACCCCGTCACTGGTCTTGTCATG CCTGGGGTATATACAAACGAAGCCTTCCATCCAGACATCATTTTGCTGCCAGGATGC GGGGTGGACTTCACCCACAGCCGCCTGAGCAACTTGTTGGGCATCCGCAAGCGGC AACCCTTCCAGGAGGGCTTTAGGATCACCTACGATGATCTGGAGGGTGGTAACATTC CCGCACTGTTGGATGTGGACGCCTACCAGGCGAGCTTGAAAGATGACACCGAACAG GGCGGGGGTGGCGCAGGCGGCAGCAACAGCAGTGGCAGCGGCGCGGAAGAGAAC TCCAACGCGGCAGCCGCGGCAATGCAGCCGGTGGAGGACATGAACGATCATGCCA TTCGCGGCGACACCTTTGCCACACGGGCTGAGGAGAAGCGCGCTGAGGCCGAAGC AGCGGCCGAAGCTGCCGCCCCCGCTGCGCAACCCGAGGTCGAGAAGCCTCAGAAG AAACCGGTGATCAAACCCCTGACAGAGGACAGCAAGAAACGCAGTTACAACCTAATA AGCAATGACAGCACCTTCACCCAGTACCGCAGCTGGTACCTTGCATACAACTACGG CGACCCTCAGACCGGAATCCGCTCATGGACCCTGCTTTGCACTCCTGACGTAACCT GCGGCTCGGAGCAGGTCTACTGGTCGTTGCCAGACATGATGCAAGACCCCGTGACC TTCCGCTCCACGCGCCAGATCAGCAACTTTCCGGTGGTGGGCGCCGAGCTGTTGCC CGTGCACTCCAAGAGCTTCTACAACGACCAGGCCGTCTACTCCCAACTCATCCGCC AGTTTACCTCTCTGACCCACGTGTTCAATCGCTTTCCCGAGAACCAGATTTTGGCGC GCCCGCCAGCCCCCACCATCACCACCGTCAGTGAAAACGTTCCTGCTCTCACAGAT CACGGGACGCTACCGCTGCGCAACAGCATCGGAGGAGTCCAGCGAGTGACCATTA CTGACGCCAGACGCCGCACCTGCCCCTACGTTTACAAGGCCCTGGGCATAGTCTCG CCGCGCGTCCTATCGAGCCGCACTTTTTGAGCAAGCATGTCCATCCTTATATCGCCC AGCAATAACACAGGCTGGGGCCTGCGCTTCCCAAGCAAGATGTTTGGCGGGGCCAA GAAGCGCTCCGACCAACACCCAGTGCGCGTGCGCGGGCACTACCGCGCGCCCTGG GGCGCGCACAAACGCGGCCGCACTGGGCGCACCACCGTCGATGACGCCATCGACG CGGTGGTGGAGGAGGCGCGCAACTACACGCCCACGCCGCCACCAGTGTCCACAGT GGACGCGGCCATTCAGACCGTGGTGCGCGGAGCCCGGCGCTATGCTAAAATGAAG AGACGGCGGAGGCGCGTAGCACGTCGCCACCGCCGCCGACCCGGCACTGCCGCC CAACGCGCGGCGGCGGCCCTGCTTAACCGCGCACGTCGCACCGGCCGACGGGCG GCCATGCGGGCCGCTCGAAGGCTGGCCGCGGGTATTGTCACTGTGCCCCCCAGGT CCAGGCGACGAGCGGCCGCCGCAGCAGCCGCGGCCATTAGTGCTATGACTCAGGG TCGCAGGGGCAACGTGTATTGGGTGCGCGACTCGGTTAGCGGCCTGCGCGTGCCC GTGCGCACCCGCCCCCCGCGCAACTAGATTGCAAGAAAAAACTACTTAGACTCGTA CTGTTGTATGTATCCAGCGGCGGCGGCGCGCAACGAAGCTATGTCCAAGCGCAAAA TCAAAGAAGAGATGCTCCAGGTCATCGCGCCGGAGATCTATGGCCCCCCGAAGAAG GAAGAGCAGGATTACAAGCCCCGAAAGCTAAAGCGGGTCAAAAAGAAAAAGAAAGA TGATGATGATGAACTTGACGACGAGGTGGAACTGCTGCACGCTACCGCGCCCAGGC GACGGGTACAGTGGAAAGGTCGACGCGTAAAACGTGTTTTGCGACCCGGCACCACC GTAGTCTTTACGCCCGGTGAGCGCTCCACCCGCACCTACAAGCGCGTGTATGATGA GGTGTACGGCGACGAGGACCTGCTTGAGCAGGCCAACGAGCGCCTCGGGGAGTTT GCCTACGGAAAGCGGCATAAGGACATGCTGGCGTTGCCGCTGGACGAGGGCAACC CAACACCTAGCCTAAAGCCCGTAACACTGCAGCAGGTGCTGCCCGCGCTTGCACCG TCCGAAGAAAAGCGCGGCCTAAAGCGCGAGTCTGGTGACTTGGCACCCACCGTGCA GCTGATGGTACCCAAGCGCCAGCGACTGGAAGATGTCTTGGAAAAAATGACCGTGG AACCTGGGCTGGAGCCCGAGGTCCGCGTGCGGCCAATCAAGCAGGTGGCGCCGG GACTGGGCGTGCAGACCGTGGACGTTCAGATACCCACTACCAGTAGCACCAGTATT GCCACCGCCACAGAGGGCATGGAGACACAAACGTCCCCGGTTGCCTCAGCGGTGG CGGATGCCGCGGTGCAGGCGGTCGCTGCGGCCGCGTCCAAGACCTCTACGGAGGT GCAAACGGACCCGTGGATGTTTCGCGTTTCAGCCCCCCGGCGCCCGCGCGGTTCG |
| | | AGGAAGTACGGCGCCGCCAGCGCGCTACTGCCCGAATATGCCCTACATCCTTCCAT TGCGCCTACCCCCGGCTATCGTGGCTACACCTACCGCCCCAGAAGACGAGCAACTA CCCGACGCCGAACCACCACTGGAACCCGCCGCCGCCGTCGCCGTCGCCAGCCCGT GCTGGCCCCGATTTCCGTGCGCAGGGTGGCTCGCGAAGGAGGCAGGACCCTGGTG CTGCCAACAGCGCGCTACCACCCCAGCATCGTTTAAAAGCCGGTCTTTGTGGTTCTT GCAGATATGGCCCTCACCTGCCGCCTCCGTTTCCCGGTGCCGGGATTCCGAGGAAG AATGCACCGTAGGAGGGGCATGGCCGGCCACGGCCTGACGGGCGGCATGCGTCGT GCGCACCACCGGCGGCGGCGCGCGTCGCACCGTCGCATGCGCGGCGGTATCCTG CCCCTCCTTATTCCACTGATCGCCGCGGCGATTGGCGCCGTGCCCGGAATTGCATC CGTGGCCTTGCAGGCGCAGAGACACTGATTAAAAACAAGTTGCATGTGGAAAAATCA AAATAAAAAGTCTGGACTCTCACGCTCGCTTGGTCCTGTAACTATTTTGTAGAATGGA AGACATCAACTTTGCGTCTCTGGCCCCGCGACACGGCTCGCGCCCGTTCATGGGAA ACTGGCAAGATATCGGCACCAGCAATATGAGCGGTGGCGCCTTCAGCTGGGGCTCG CTGTGGAGCGGCATTAAAAATTTCGGTTCCACCGTTAAGAACTATGGCAGCAAGGCC TGGAACAGCAGCACAGGCCAGATGCTGAGGGATAAGTTGAAAGAGCAAAATTTCCA ACAAAAGGTGGTAGATGGCCTGGCCTCTGGCATTAGCGGGGTGGTGGACCTGGCC AACCAGGCAGTGCAAAATAAGATTAACAGTAAGCTTGATCCCCGCCCTCCCGTAGAG GAGCCTCCACCGGCCGTGGAGACAGTGTCTCCAGAGGGGCGTGGCGAAAAGCGTC CGCGCCCCGACAGGGAAGAAACTCTGGTGACGCAAATAGACGAGCCTCCCTCGTAC GAGGAGGCACTAAAGCAAGGCCTGCCCACCACCCGTCCCATCGCGCCCATGGCTA CCGGAGTGCTGGGCCAGCACACACCCGTAACGCTGGACCTGCCTCCCCCCGCCGA CACCCAGCAGAAACCTGTGCTGCCAGGCCCGACCGCCGTTGTTGTAACCCGTCCTA GCCGCGCGTCCCTGCGCCGCGCCGCCAGCGGTCCGCGATCGTTGCGGCCCGTAG CCAGTGGCAACTGGCAAAGCACACTGAACAGCATCGTGGGTCTGGGGGTGCAATCC CTGAAGCGCCGACGATGCTTCTGAATAGCTAACGTGTCGTATGTGTGTCATGTATGC GTCCATGTCGCCGCCAGAGGAGCTGCTGAGCCGCCGCGCGCCCGCTTTCCAAGAT GGCTACCCCTTCGATGATGCCGCAGTGGTCTTACATGCACATCTCGGGCCAGGACG CCTCGGAGTACCTGAGCCCCGGGCTGGTGCAGTTTGCCCGCGCCACCGAGACGTA CTTCAGCCTGAATAACAAGTTTAGAAACCCCACGGTGGCGCCTACGCACGACGTGA CCACAGACCGGTCCCAGCGTTTGACGCTGCGGTTCATCCCTGTGGACCGTGAGGAT ACTGCGTACTCGTACAAGGCGCGGTTCACCCTAGCTGTGGGTGATAACCGTGTGCT GGACATGGCTTCCACGTACTTTGACATCCGCGGCGTGCTGGACAGGGGCCCTACTT TTAAGCCCTACTCTGGCACTGCCTACAACGCCCTGGCTCCCAAGGGTGCCCCAAAT CCTTGCGAATGGGATGAAGCTGCTACTGCTCTTGAAATAAACCTAGAAGAAGAGGAC GATGACAACGAAGACGAAGTAGACGAGCAAGCTGAGCAGCAAAAAACTCACGTATTT GGGCAGGCGCCTTATTCTGGTATAAATATTACAAAGGAGGGTATTCAAATAGGTGTC GAAGGTCAAACACCTAAATATGCCGATAAAACATTTCAACCTGAACCTCAAATAGGA GAATCTCAGTGGTACGAAACTGAAATTAATCATGCAGCTGGGAGAGTCCTTAAAAAG ACTACCCCAATGAAACCATGTTACGGTTCATATGCAAAACCCACAAATGAAAATGGA GGGCAAGGCATTCTTGTAAAGCAACAAAATGGAAAGCTAGAAAGTCAAGTGGAAATG CAATTTTTCTCAACTACTGAGGCGACCGCAGGCAATGGTGATAACTTGACTCCTAAA GTGGTATTGTACAGTGAAGATGTAGATATAGAAACCCCAGACACTCATATTTCTTACA TGCCCACTATTAAGGAAGGTAACTCACGAGAACTAATGGGCCAACAATCTATGCCCA ACAGGCCTAATTACATTGCTTTTAGGGACAATTTTATTGGTCTAATGTATTACAACAG CACGGGTAATATGGGTGTTCTGGCGGGCCAAGCATCGCAGTTGAATGCTGTTGTAG ATTTGCAAGACAGAAACACAGAGCTTTCATACCAGCTTTTGCTTGATTCCATTGGTGA TAGAACCAGGTACTTTTCTATGTGGAATCAGGCTGTTGACAGCTATGATCCAGATGTT AGAATTATTGAAAATCATGGAACTGAAGATGAACTTCCAAATTACTGCTTTCCACTGG GAGGTGTGATTAATACAGAGACTCTTACCAAGGTAAAACCTAAAACAGGTCAGGAAA ATGGATGGGAAAAAGATGCTACAGAATTTTCAGATAAAAATGAAATAAGAGTTGGAAA TAATTTTGCCATGGAAATCAATCTAAATGCCAACCTGTGGAGAAATTTCCTGTACTCC AACATAGCGCTGTATTTGCCCGACAAGCTAAAGTACAGTCCTTCCAACGTAAAAATTT CTGATAACCCAAACACCTACGACTACATGAACAAGCGAGTGGTGGCTCCCGGGTTA GTGGACTGCTACATTAACCTTGGAGCACGCTGGTCCCTTGACTATATGGACAACGTC AACCCATTTAACCACCACCGCAATGCTGGCCTGCGCTACCGCTCAATGTTGCTGGG CAATGGTCGCTATGTGCCCTTCCACATCCAGGTGCCTCAGAAGTTCTTTGCCATTAA AAACCTCCTTCTCCTGCCGGGCTCATACACCTACGAGTGGAACTTCAGGAAGGATGT TAACATGGTTCTGCAGAGCTCCCTAGGAAATGACCTAAGGGTTGACGGAGCCAGCA TTAAGTTTGATAGCATTTGCCTTTACGCCACCTTCTTCCCCATGGCCCACAACACCG CCTCCACGCTTGAGGCCATGCTTAGAAACGACACCAACGACCAGTCCTTTAACGACT ATCTCTCCGCCGCCAACATGCTCTACCCTATACCCGCCAACGCTACCAACGTGCCCA TATCCATCCCCTCCCGCAACTGGGCGGCTTTCCGCGGCTGGGCCTTCACGCGCCTT AAGACTAAGGAAACCCCATCACTGGGCTCGGGCTACGACCCTTATTACACCTACTCT GGCTCTATACCCTACCTAGATGGAACCTTTTACCTCAACCACACCTTTAAGAAGGTG GCCATTACCTTTGACTCTTCTGTCAGCTGGCCTGGCAATGACCGCCTGCTTACCCCC AACGAGTTTGAAATTAAGCGCTCAGTTGACGGGGAGGGTTACAACGTTGCCCAGTG TAACATGACCAAAGACTGGTTCCTGGTACAAATGCTAGCTAACTACAACATTGGCTA CCAGGGCTTCTATATCCCAGAGAGCTACAAGGACCGCATGTACTCCTTCTTTAGAAA CTTCCAGCCCATGAGCCGTCAGGTGGTGGATGATACTAAATACAAGGACTACCAACA GGTGGGCATCCTACACCAACACAACAACTCTGGATTTGTTGGCTACCTTGCCCCCAC |
| | | CATGCGCGAAGGACAGGCCTACCCTGCTAACTTCCCCTATCCGCTTATAGGCAAGA CCGCAGTTGACAGCATTACCCAGAAAAAGTTTCTTTGCGATCGCACCCTTTGGCGCA TCCCATTCTCCAGTAACTTTATGTCCATGGGCGCACTCACAGACCTGGGCCAAAACC TTCTCTACGCCAACTCCGCCCACGCGCTAGACATGACTTTTGAGGTGGATCCCATGG ACGAGCCCACCCTTCTTTATGTTTTGTTTGAAGTCTTTGACGTGGTCCGTGTGCACC GGCCGCACCGCGGCGTCATCGAAACCGTGTACCTGCGCACGCCCTTCTCGGCCGG CAACGCCACAACATAAAGAAGCAAGCAACATCAACAACAGCTGCCGCCATGGGCTC CAGTGAGCAGGAACTGAAAGCCATTGTCAAAGATCTTGGTTGTGGGCCATATTTTTT GGGCACCTATGACAAGCGCTTTCCAGGCTTTGTTTCTCCACACAAGCTCGCCTGCG CCATAGTCAATACGGCCGGTCGCGAGACTGGGGGCGTACACTGGATGGCCTTTGCC TGGAACCCGCACTCAAAAACATGCTACCTCTTTGAGCCCTTTGGCTTTTCTGACCAG CGACTCAAGCAGGTTTACCAGTTTGAGTACGAGTCACTCCTGCGCCGTAGCGCCATT GCTTCTTCCCCCGACCGCTGTATAACGCTGGAAAAGTCCACCCAAAGCGTACAGGG GCCCAACTCGGCCGCCTGTGGACTATTCTGCTGCATGTTTCTCCACGCCTTTGCCAA CTGGCCCCAAACTCCCATGGATCACAACCCCACCATGAACCTTATTACCGGGGTACC CAACTCCATGCTCAACAGTCCCCAGGTACAGCCCACCCTGCGTCGCAACCAGGAAC AGCTCTACAGCTTCCTGGAGCGCCACTCGCCCTACTTCCGCAGCCACAGTGCGCAG ATTAGGAGCGCCACTTCTTTTTGTCACTTGAAAAACATGTAAAAATAATGTACTAGAG ACACTTTCAATAAAGGCAAATGCTTTTATTTGTACACTCTCGGGTGATTATTTACCCC CACCCTTGCCGTCTGCGCCGTTTAAAAATCAAAGGGGTTCTGCCGCGCATCGCTAT GCGCCACTGGCAGGGACACGTTGCGATACTGGTGTTTAGTGCTCCACTTAAACTCA GGCACAACCATCCGCGGCAGCTCGGTGAAGTTTTCACTCCACAGGCTGCGCACCAT CACCAACGCGTTTAGCAGGTCGGGCGCCGATATCTTGAAGTCGCAGTTGGGGCCTC CGCCCTGCGCGCGCGAGTTGCGATACACAGGGTTGCAGCACTGGAACACTATCAGC GCCGGGTGGTGCACGCTGGCCAGCACGCTCTTGTCGGAGATCAGATCCGCGTCCA GGTCCTCCGCGTTGCTCAGGGCGAACGGAGTCAACTTTGGTAGCTGCCTTCCCAAA AAGGGCGCGTGCCCAGGCTTTGAGTTGCACTCGCACCGTAGTGGCATCAAAAGGTG ACCGTGCCCGGTCTGGGCGTTAGGATACAGCGCCTGCATAAAAGCCTTGATCTGCT TAAAAGCCACCTGAGCCTTTGCGCCTTCAGAGAAGAACATGCCGCAAGACTTGCCG GAAAACTGATTGGCCGGACAGGCCGCGTCGTGCACGCAGCACCTTGCGTCGGTGTT GGAGATCTGCACCACATTTCGGCCCCACCGGTTCTTCACGATCTTGGCCTTGCTAGA CTGCTCCTTCAGCGCGCGCTGCCCGTTTTCGCTCGTCACATCCATTTCAATCACGTG CTCCTTATTTATCATAATGCTTCCGTGTAGACACTTAAGCTCGCCTTCGATCTCAGCG CAGCGGTGCAGCCACAACGCGCAGCCCGTGGGCTCGTGATGCTTGTAGGTCACCT CTGCAAACGACTGCAGGTACGCCTGCAGGAATCGCCCCATCATCGTCACAAAGGTC TTGTTGCTGGTGAAGGTCAGCTGCAACCCGCGGTGCTCCTCGTTCAGCCAGGTCTT GCATACGGCCGCCAGAGCTTCCACTTGGTCAGGCAGTAGTTTGAAGTTCGCCTTTA GATCGTTATCCACGTGGTACTTGTCCATCAGCGCGCGCGCAGCCTCCATGCCCTTC TCCCACGCAGACACGATCGGCACACTCAGCGGGTTCATCACCGTAATTTCACTTTCC GCTTCGCTGGGCTCTTCCTCTTCCTCTTGCGTCCGCATACCACGCGCCACTGGGTC GTCTTCATTCAGCCGCCGCACTGTGCGCTTACCTCCTTTGCCATGCTTGATTAGCAC CGGTGGGTTGCTGAAACCCACCATTTGTAGCGCCACATCTTCTCTTTCTTCCTCGCT GTCCACGATTACCTCTGGTGATGGCGGGCGCTCGGGCTTGGGAGAAGGGCGCTTC TTTTTCTTCTTGGGCGCAATGGCCAAATCCGCCGCCGAGGTCGATGGCCGCGGGCT GGGTGTGCGCGGCACCAGCGCGTCTTGTGATGAGTCTTCCTCGTCCTCGGACTCGA TACGCCGCCTCATCCGCTTTTTTGGGGGCGCCCGGGGAGGCGGCGGCGACGGGG ACGGGGACGACACGTCCTCCATGGTTGGGGGACGTCGCGCCGCACCGCGTCCGCG CTCGGGGGTGGTTTCGCGCTGCTCCTCTTCCCGACTGGCCATTTCCTTCTCCTATAG GCAGAAAAAGATCATGGAGTCAGTCGAGAAGAAGGACAGCCTAACCGCCCCCTCTG AGTTCGCCACCACCGCCTCCACCGATGCCGCCAACGCGCCTACCACCTTCCCCGTC GAGGCACCCCCGCTTGAGGAGGAGGAAGTGATTATCGAGCAGGACCCAGGTTTTGT AAGCGAAGACGACGAGGACCGCTCAGTACCAACAGAGGATAAAAAGCAAGACCAGG ACAACGCAGAGGCAAACGAGGAACAAGTCGGGCGGGGGGACGAAAGGCATGGCGA CTACCTAGATGTGGGAGACGACGTGCTGTTGAAGCATCTGCAGCGCCAGTGCGCCA TTATCTGCGACGCGTTGCAAGAGCGCAGCGATGTGCCCCTCGCCATAGCGGATGTC AGCCTTGCCTACGAACGCCACCTATTCTCACCGCGCGTACCCCCCAAACGCCAAGA AAACGGCACATGCGAGCCCAACCCGCGCCTCAACTTCTACCCCGTATTTGCCGTGC CAGAGGTGCTTGCCACCTATCACATCTTTTTCCAAAACTGCAAGATACCCCTATCCTG CCGTGCCAACCGCAGCCGAGCGGACAAGCAGCTGGCCTTGCGGCAGGGCGCTGTC ATACCTGATATCGCCTCGCTCAACGAAGTGCCAAAAATCTTTGAGGGTCTTGGACGC GACGAGAAGCGCGCGGCAAACGCTCTGCAACAGGAAAACAGCGAAAATGAAAGTCA CTCTGGAGTGTTGGTGGAACTCGAGGGTGACAACGCGCGCCTAGCCGTACTAAAAC GCAGCATCGAGGTCACCCACTTTGCCTACCCGGCACTTAACCTACCCCCCAAGGTC ATGAGCACAGTCATGAGTGAGCTGATCGTGCGCCGTGCGCAGCCCCTGGAGAGGG ATGCAAATTTGCAAGAACAAACAGAGGAGGGCCTACCCGCAGTTGGCGACGAGCAG CTAGCGCGCTGGCTTCAAACGCGCGAGCCTGCCGACTTGGAGGAGCGACGCAAAC TAATGATGGCCGCAGTGCTCGTTACCGTGGAGCTTGAGTGCATGCAGCGGTTCTTT GCTGACCCGGAGATGCAGCGCAAGCTAGAGGAAACATTGCACTACACCTTTCGACA GGGCTACGTACGCCAGGCCTGCAAGATCTCCAACGTGGAGCTCTGCAACCTGGTCT CCTACCTTGGAATTTTGCACGAAAACCGCCTTGGGCAAAACGTGCTTCATTCCACGC |
| | | TCAAGGGCGAGGCGCGCCGCGACTACGTCCGCGACTGCGTTTACTTATTTCTATGC TACACCTGGCAGACGGCCATGGGCGTTTGGCAGCAGTGCTTGGAGGAGTGCAACCT CAAGGAGCTGCAGAAACTGCTAAAGCAAAACTTGAAGGACCTATGGACGGCCTTCA ACGAGCGCTCCGTGGCCGCGCACCTGGCGGACATCATTTTCCCCGAACGCCTGCTT AAAACCCTGCAACAGGGTCTGCCAGACTTCACCAGTCAAAGCATGTTGCAGAACTTT AGGAACTTTATCCTAGAGCGCTCAGGAATCTTGCCCGCCACCTGCTGTGCACTTCCT AGCGACTTTGTGCCCATTAAGTACCGCGAATGCCCTCCGCCGCTTTGGGGCCACTG CTACCTTCTGCAGCTAGCCAACTACCTTGCCTACCACTCTGACATAATGGAAGACGT GAGCGGTGACGGTCTACTGGAGTGTCACTGTCGCTGCAACCTATGCACCCCGCACC GCTCCCTGGTTTGCAATTCGCAGCTGCTTAACGAAAGTCAAATTATCGGTACCTTTG AGCTGCAGGGTCCCTCGCCTGACGAAAAGTCCGCGGCTCCGGGGTTGAAACTCACT CCGGGGCTGTGGACGTCGGCTTACCTTCGCAAATTTGTACCTGAGGACTACCACGC CCACGAGATTAGGTTCTACGAAGACCAATCCCGCCCGCCAAATGCGGAGCTTACCG CCTGCGTCATTACCCAGGGCCACATTCTTGGCCAATTGCAAGCCATCAACAAAGCCC GCCAAGAGTTTCTGCTACGAAAGGGACGGGGGGTTTACTTGGACCCCCAGTCCGGC GAGGAGCTCAACCCAATCCCCCCGCCGCCGCAGCCCTATCAGCAGCAGCCGCGGG CCCTTGCTTCCCAGGATGGCACCCAAAAAGAAGCTGCAGCTGCCGCCGCCACCCAC GGACGAGGAGGAATACTGGGACAGTCAGGCAGAGGAGGTTTTGGACGAGGAGGAG GAGGACATGATGGAAGACTGGGAGAGCCTAGACGAGGAAGCTTCCGAGGTCGAAG AGGTGTCAGACGAAACACCGTCACCCTCGGTCGCATTCCCCTCGCCGGCGCCCCA GAAATCGGCAACCGGTTCCAGCATGGCTACAACCTCCGCTCCTCAGGCGCCGCCG GCACTGCCCGTTCGCCGACCCAACCGTAGATGGGACACCACTGGAACCAGGGCCG GTAAGTCCAAGCAGCCGCCGCCGTTAGCCCAAGAGCAACAACAGCGCCAAGGCTAC CGCTCATGGCGCGGGCACAAGAACGCCATAGTTGCTTGCTTGCAAGACTGTGGGGG CAACATCTCCTTCGCCCGCCGCTTTCTTCTCTACCATCACGGCGTGGCCTTCCCCCG TAACATCCTGCATTACTACCGTCATCTCTACAGCCCATACTGCACCGGCGGCAGCGG CAGCGGCAGCAACAGCAGCGGCCACACAGAAGCAAAGGCGACCGGATAGCAAGAC TCTGACAAAGCCCAAGAAATCCACAGCGGCGGCAGCAGCAGGAGGAGGAGCGCTG CGTCTGGCGCCCAACGAACCCGTATCGACCCGCGAGCTTAGAAACAGGATTTTTCC CACTCTGTATGCTATATTTCAACAGAGCAGGGGCCAAGAACAAGAGCTGAAAATAAA AAACAGGTCTCTGCGATCCCTCACCCGCAGCTGCCTGTATCACAAAAGCGAAGATCA GCTTCGGCGCACGCTGGAAGACGCGGAGGCTCTCTTCAGTAAATACTGCGCGCTGA CTCTTAAGGACTAGTTTCGCGCCCTTTCTCAAATTTAAGCGCGAAAACTACGTCATCT CCAGCGGCCACACCCGGCGCCAGCACCTGTCGTCAGCGCCATTATGAGCAAGGAA ATTCCCACGCCCTACATGTGGAGTTACCAGCCACAAATGGGACTTGCGGCTGGAGC TGCCCAAGACTACTCAACCCGAATAAACTACATGAGCGCGGGACCCCACATGATATC CCGGGTCAACGGAATCCGCGCCCACCGAAACCGAATTCTCTTGGAACAGGCGGCTA TTACCACCACACCTCGTAATAACCTTAATCCCCGTAGTTGGCCCGCTGCCCTGGTGT ACCAGGAAAGTCCCGCTCCCACCACTGTGGTACTTCCCAGAGACGCCCAGGCCGAA GTTCAGATGACTAACTCAGGGGCGCAGCTTGCGGGCGGCTTTCGTCACAGGGTGC GGTCGCCCGGGCAGGGTATAACTCACCTGACAATCAGAGGGCGAGGTATTCAGCTC AACGACGAGTCGGTGAGCTCCTCGCTTGGTCTCCGTCCGGACGGGACATTTCAGAT CGGCGGCGCCGGCCGCTCTTCATTCACGCCTCGTCAGGCAATCCTAACTCTGCAGA CCTCGTCCTCTGAGCCGCGCTCTGGAGGCATTGGAACTCTGCAATTTATTGAGGAGT TTGTGCCATCGGTCTACTTTAACCCCTTCTCGGGACCTCCCGGCCACTATCCGGATC AATTTATTCCTAACTTTGACGCGGTAAAGGACTCGGCGGATGGCTACGACTGAATGT TAAGTGGAGAGGCAGAGCAACTGCGCCTGAAACACCTGGTCCACTGTCGCCGCCAC AAGTGCTTTGCCCGCGACTCCGGTGAGTTTTGCTACTTTGAATTGCCCGAGGATCAT ATCGAGGGCCCGGCGCACGGCGTCCGGCTTACCGCCCAGGGAGAGCTTGCCCGTA GCCTGATTCGGGAGTTTACCCAGCGCCCCCTGCTAGTTGAGCGGGACAGGGGACC CTGTGTTCTCACTGTGATTTGCAACTGTCCTAACCCTGGATTACATCAAGATCTTTGT TGCCATCTCTGTGCTGAGTATAATAAATACAGAAATTAAAATATACTGGGGCTCCTAT CGCCATCCTGTAAACGCCACCGTCTTCACCCGCCCAAGCAAACCAAGGCGAACCTT ACCTGGTACTTTTAACATCTCTCCCTCTGTGATTTACAACAGTTTCAACCCAGACGGA GTGAGTCTACGAGAGAACCTCTCCGAGCTCAGCTACTCCATCAGAAAAAACACCACC CTCCTTACCTGCCGGGAACGTACGACCTAGGGATAACAGGGTAATAAGCAATTGACT CTATGTGGGATATGCTCCAGCGCTACAACCTTGAAGTCAGGCTTCCTGGATGTCAGC ATCTGACTTTGGCCAGCACCTGTCCCGCGGATTTGTTCCAGTCCAACTACAGCGACC CACCCTAACAGAGATGACCAACACAACCAACGCGGCCGCCGCTACCGGACTTACAT CTACCACAAATACACCCCAAGTTTCTGCCTTTGTCAATAACTGGGATAACTTGGGCAT GTGGTGGTTCTCCATAGCGCTTATGTTTGTATGCCTTATTATTATGTGGCTCATCTGC TGCCTAAAGCGCAAACGCGCCCGACCACCCATCTATAGTCCCATCATTGTGCTACAC CCAAACAATGATGGAATCCATAGATTGGACGGACTGAAACACATGTTCTTTTCTCTTA CAGTATGATTAAATGAGACATGATTCCTCGAGTTTTTATATTACTGACCCTTGTTGCG CTTTTTTGTGCGTGCTCCACATTGGCTGCGGTTTCTCACATCGAAGTAGACTGCATT CCAGCCTTCACAGTCTATTTGCTTTACGGATTTGTCACCCTCACGCTCATCTGCAGC CTCATCACTGTGGTCATCGCCTTTATCCAGTGCATTGACTGGGTCTGTGTGCGCTTT GCATATCTCAGACACCATCCCCAGTACAGGGACAGGACTATAGCTGAGCTTCTTAGA ATTCTTTAATTATGAAATTTACTGTGACTTTTCTGCTGATTATTTGCACCCTATCTGCG TTTTGTTCCCCGACCTCCAAGCCTCAAAGACATATATCATGCAGATTCACTCGTATAT |
| | | GGAATATTCCAAGTTGCTACAATGAAAAAAGCGATCTTTCCGAAGCCTGGTTATATGC AATCATCTCTGTTATGGTGTTCTGCAGTACCATCTTAGCCCTAGCTATATATCCCTAC CTTGACATTGGCTGGAAACGAATAGATGCCATGAACCACCCAACTTTCCCCGCGCCC GCTATGCTTCCACTGCAACAAGTTGTTGCCGGCGGCTTTGTCCCAGCCAATCAGCCT CGCCCCACTTCTCCCACCCCCACTGAAATCAGCTACTTTAATCTAACAGGAGGAGAT GACTGACACCCTAGATCTAGAAATGGACGGAATTATTACAGAGCAGCGCCTGCTAGA AAGACGCAGGGCAGCGGCCGAGCAACAGCGCATGAATCAAGAGCTCCAAGACATG GTTAACTTGCACCAGTGCAAAAGGGGTATCTTTTGTCTGGTAAAGCAGGCCAAAGTC ACCTACGACAGTAATACCACCGGACACCGCCTTAGCTACAAGTTGCCAACCAAGCGT CAGAAATTGGTGGTCATGGTGGGAGAAAAGCCCATTACCATAACTCAGCACTCGGTA GAAACCGAAGGCTGCATTCACTCACCTTGTCAAGGACCTGAGGATCTCTGCACCCTT ATTAAGACCCTGTGCGGTCTCAAAGATCTTATTCCCTTTAACTAATAAAAAAAAATAAT AAAGCATCACTTACTTAAAATCAGTTAGCAAATTTCTGTCCAGTTTATTCAGCAGCAC CTCCTTGCCCTCCTCCCAGCTCTGGTATTGCAGCTTCCTCCTGGCTGCAAACTTTCT CCACAATCTAAATGGAATGTCAGTTTCCTCCTGTTCCTGTCCATCCGCACCCACTATC TTCATGTTGTTGCAGATGAAGCGCGCAAGACCGTCTGAAGATACCTTCAACCCCGTG TATCCATATGACACGGAAACCGGTCCTCCAACTGTGCCTTTTCTTACTCCTCCCTTTG TATCCCCCAATGGGTTTCAAGAGAGTCCCCCTGGGGTACTCTCTTTGCGCCTATCCG AACCTCTAGTTACCTCCAATGGCATGCTTGCGCTCAAAATGGGCAACGGCCTCTCTC TGGACGAGGCCGGCAACCTTACCTCCCAAAATGTAACCACTGTGAGCCCACCTCTC AAAAAAACCAAGTCAAACATAAACCTGGAAATATCTGCACCCCTCACAGTTACCTCAG AAGCCCTAACTGTGGCTGCCGCCGCACCTCTAATGGTCGCGGGCAACACACTCACC ATGCAATCACAGGCCCCGCTAACCGTGCACGACTCCAAACTTAGCATTGCCACCCAA GGACCCCTCACAGTGTCAGAAGGAAAGCTAGCCCTGCAAACATCAGGCCCCCTCAC CACCACCGATAGCAGTACCCTTACTATCACTGCCTCACCCCCTCTAACTACTGCCAC TGGTAGCTTGGGCATTGACTTGAAAGAGCCCATTTATACACAAAATGGAAAACTAGG ACTAAAGTACGGGGCTCCTTTGCATGTAACAGACGACCTAAACACTTTGACCGTAGC AACTGGTCCAGGTGTGACTATTAATAATACTTCCTTGCAAACTAAAGTTACTGGAGCC TTGGGTTTTGATTCACAAGGCAATATGCAACTTAATGTAGCAGGAGGACTAAGGATT GATTCTCAAAACAGACGCCTTATACTTGATGTTAGTTATCCGTTTGATGCTCAAAACC AACTAAATCTAAGACTAGGACAGGGCCCTCTTTTTATAAACTCAGCCCACAACTTGGA TATTAACTACAACAAAGGCCTTTACTTGTTTACAGCTTCAAACAATTCCAAAAAGCTTG AGGTTAACCTAAGCACTGCCAAGGGGTTGATGTTTGACGCTACAGCCATAGCCATTA ATGCAGGAGATGGGCTTGAATTTGGTTCACCTAATGCACCAAACACAAATCCCCTCA AAACAAAAATTGGCCATGGCCTAGAATTTGATTCAAACAAGGCTATGGTTCCTAAACT AGGAACTGGCCTTAGTTTTGACAGCACAGGTGCCATTACAGTAGGAAACAAAAATAA TGATAAGCTAACCCTATGGACAGGTCCAAAACCAGAAGCCAACTGCATAATTGAATA CGGGAAACAAAACCCAGATAGCAAACTAACTTTAATCCTTGTAAAAAATGGAGGAATT GTTAATGGATATGTAACGCTAATGGGAGCCTCAGACTACGTTAACACCTTATTTAAAA ACAAAAATGTCTCCATTAATGTAGAACTATACTTTGATGCCACTGGTCATATATTACCA GACTCATCTTCTCTTAAAACAGATCTAGAACTAAAATACAAGCAAACCGCTGACTTTA GTGCAAGAGGTTTTATGCCAAGTACTACAGCGTATCCATTTGTCCTTCCTAATGCGG GAACACATAATGAAAATTATATTTTTGGTCAATGCTACTACAAAGCAAGCGATGGTGC CCTTTTTCCGTTGGAAGTTACTGTTATGCTTAATAAACGCCTGCCAGATAGTCGCACA TCCTATGTTATGACTTTTTTATGGTCCTTGAATGCTGGTCTAGCTCCAGAAACTACTC AGGCAACCCTCATAACCTCCCCATTTACCTTTTCCTATATTAGAGAAGATGACTAATA AACTCTAAAGAATCGTTTGTGTTATGTTTCAACGTGTTTATTTTTCAATTGCAGAAAAT TTCAAGTCATTTTTCATTCAGTAGTATAGCCCCACCACCACATAGCTTATACAGATCA CCGTACCTTAATCAAACTCACAGAACCCTAGTATTCAACCTGCCACCTCCCTCCCAA CACACAGAGTACACAGTCCTTTCTCCCCGGCTGGCCTTAAAAAGCATCATATCATGG GTAACAGACATATTCTTAGGTGTTATATTCCACACGGTTTCCTGTCGAGCCAAACGCT CATCAAGTGATATTAATAAACTCCCCGGGCAGCTCACTTAAGTTCATGTCGCTGTCC AGCTGCTGAGCCACAGGCTGCTGTCCAACTTGCGGTTGCTTAACGGGCGGCGAAG GAGAAGTCCACGCCTACATGGGGGGAGAGTCATAATCGTGCATCAGGATAGGGCG GTGGTGCTGCAGCAGCGCGCGAATAAACTGCTGCCGCCGCCGCTCCGTCCTGCAG GAATACAACATGGCAGTGGTCTCCTCAGCGATGATTCGCACCGCCCGCAGCATAAG GCGCTTGTCCTCCGGGCACAGCAGCGCACCCTGATCTCACTTAAATCAGCACAGTA ACTGCAGCACAGCACCACAATATTGTTCAAAATCCCACAGTGCAAGGCGCTGTATCC AAAGCTCATGGCGGGGACCACAGAACCCACGTGGCCATCATACCACAAGCGCAGGT AGATTAAGTGGCGACCCCTCATAAACACGCTGGACATAAACATTACCTCTTTTGGCA TGTTGTAATTCACCACCTCCCGGTACCATATAAACCTCTGATTAAACATGGCGCCATC CACCACCATCCTAAACCAGCTGGCCAAAACCTGCCCCGCCGGGNTATACACTGCAG GGAACCGGGACTTGGACAATGACAAGTGGGAGAGCCCAGGACTCGTAACCATGGAT CATCATGCTCGTCATGATATCAATGTTGGCACAACACAGGCACACGTGCATACACTT CCTCAGGATTACAAGCTCCTCCCGCGTTAGAACCATATCCCAGGGAACAACCCATTC CTGAATCAGCGTAAATCCCACACTGCAGGGAAGACCTCGCACGTAACTCACGTTGT GCATTGTCAAAGTGTTACATTCGGGCAGCAGCGGATGATCCTCCAGTATGGTAGCG CGGGTTTCTGTCTCAAAAGGAGGTAGACGATCCCTACTGTACGGAGTGCGCCGAGA CAACCGAGATCGTGTTGGTCGTAGTGTCATGCCAAATGGAACGCCGGACGTAGTCA TATTTCCTGAAGCAAAACCAGGTGCGGGCGTGACAAACAGATCTGCGTCTCCGGTC |
| | | TCGCCGCTTAGATCGCTCTGTGTAGTAGTTGTAGTATATCCACTCTCTCAAAGCATCC AGGCGCCCCCTGGCTTCGGGTTCTATGTAAACTCCTTCATGCGCCGCTGCCCTGAT AACATCCACCACCGCAGAATAAGCCACACCCAGCCAACCTACACATTCGTTCTGCGA GTCACACACGGGAGGAGCGGGAAGAGCTGGAAGAACCATGTTTTTTTTTTTATTCCA AAAGATTATCCAAAACCTCAAAATGAAGATCTATTAAGTGAACGCGCTCCCCTCCGG TGGCGTGGTCAAACTCTACAGCCAAAGAACAGATAATGGCATTTGTAAGATGTTGCA CAATGGCTTCCAAAAGGCAAACGGCCCTCACGTCCAAGTGGACGTAAAGGCTAAAC CCTTCAGGGTGAATCTCCTCTATAAACATTCCAGCACCTTCAACCATGCCCAAATAAT TCTCATCTCGCCACCTTCTCAATATATCTCTAAGCAAATCCCGAATATTTAAGTCCGG GCCATTGTAAAAAATTTGGCTCCAGAGCGCCCTCCACCTTCAGCCTCAAGCAGCGAA TCATGATTGCAAAAATTCAGGTTCCTCACAGACCTGTATAAGATTCAAAAGCGGAACA TTAACAAAAATACCGCGATCCCGTAGGTCCCTTCGCAGGGCCAGCTGAACATAATCG TGCAGGTCTGCACGGACCAGCGCGGCCACTTCCCCGCCAGGAACCATGACAAAAG AACCCACACTGATTATGACACGCATACTCGGAGCTATGCTAACCAGCGTAGCCCCGA TGTAAGCTTGTTGCATGGGCGGCGATATAAAATGCAAGGTGCTGCTCAAAAAATCAG GCAAAGCCTCGCGCAAAAAAGAAAGCACATCGTAGTCATGCTCATGCAGATAAAGG CAGGTAAGCTCCGGAACCACCACAGAAAAAGACACCATTTTTCTCTCAAACATGTCT GCGGGTTTCTGCATAAACACAAAATAAAATAACAAAAAAACATTTAAACATTAGAAGC CTGTCTTACAACAGGAAAAACAACCCTTATAAGCATAAGACGGACTACGGCCATGCC GGCGTGACCGTAAAAAAACTGGTCACCGTGATTAAAAAGCACCACCGACAGCTCCT CGGTCAGTCCGGAGTCATAATGTAAGACTCGGTAAACACATCAGGTTGATTCACATC GGTCAGTGTTAAAAAGCGACCGAAATAGCCNGGGGGAATACAATACCCGCAGGCGT AGAGACAACATTACAGCCCCCATAGGAGGTATAACAAAATTAATAGGAGAGAAAAAC ACATAAACACCTGAAAAACCCTCCTGCCTAGGCAAAATAGCACCCTCCCGCTCCAGA ACAACATACAGCGCTTCCACAGCGGCAGCCATAACAGTCAGCCTTACCAGTAAAAAA GAAAACCTATTAAAAAAACACCACTCGACACGGCACCAGCTCAATCAGTCACAGTGT AAAAAAGGGCCAAGTGCAGAGCGAGTATATATAGGACTAAAAAATGACGGTAACGGT TAAAGTCCACAAAAAACACCCAGAAAACCGCACGCGAACCTACGCCCAGAAACGAA AGCCAAAAAACCCACAACTTCCTCAAATCGTCACTTCCGTTTTCCCACGTTACGTCAC TTCCCATTTTAAGAAAACTACAATTCCCAACACATACAAGTTACTCCGCCCTAAAACC TACGTCACCCGCCCCGTTCCCACGCCCCGCGCCACGTCACAAACTCCACCCCCTCA TTATCATATTGGCTTCAATCCAAAATAAGGTATATTATTGATGATGTTAATTAACATGC ATGGATCCTCGTCTCGACGATGCCCTTGAGAGCCTTCAACCCAGTCAGCTCCTTCCG GTGGGCGCGGGGCATGACTATCGTCGCCGCACTTATGACTGTCTTCTTTATCATGCA ACTCGTAGGACAGGTGCCGGCAGCGCTCTGGGTCATTTTCGGCGAGGACCGCTTTC GCTGGAGCGCGACGATGATCGGCCTGTCGCTTGCGGTATTCGGAATCTTGCACGCC CTCGCTCAAGCCTTCGTCACTGGTCCCGCCACCAAACGTTTCGGCGAGAAGCAGGC CATTATCGCCGGCATGGCGGCCGACGCGCTGGGCTACGTCTTGCTGGCGTTCGCG ACGCGAGGCTGGATGGCCTTCCCCATTATGATTCTTCTCGCTTCCGGCGGCATCGG GATGCCCGCGTTGCAGGCCATGCTGTCCAGGCAGGTAGATGACGACCATCAGGGA CAGCTTCAAGGATCGCTCGCGGCTCTTACCAGCCTAACTTCGATCACTGGACCGCT GATCGTCACGGCGATTTATGCCGCCTCGGCGAGCACATGGAACGGGTTGGCATGGA TTGTAGGCGCCGCCCTATACCTTGTCTGCCTCCCCGCGTTGCGTCGCGGTGCATGG AGCCGGGCCACCTCGACCTGAATGGAAGCCGGCGGCACCTCGCTAACGGATTCAC CACTCCAAGAATTGGAGCCAATCAATTCTTGCGGAGAACTGTGAATGCGCAAACCAA CCCTTGGCAGAACATATCCATCGCGTCCGCCATCTCCAGCAGCCGCACGCGGCGCA TCTCGGGCAGCGTTGGGTCCTGGCCACGGGTGCGCATGATCGTGCTCCTGTCGTTG AGGACCCGGCTAGGCTGGCGGGGTTGCCTTACTGGTTAGCAGAATGAATCACCGAT ACGCGAGCGAACGTGAAGCGACTGCTGCTGCAAAACGTCTGCGACCTGAGCAACAA CATGAATGGTCTTCGGTTTCCGTGTTTCGTAAAGTCTGGAAACGCGGAAGTCAGCGC CCTGCACCATTATGTTCCGGATCTGCATCGCAGGATGCTGCTGGCTACCCTGTGGA ACACCTACATCTGTATTAACGAAGCGCTGGCATTGACCCTGAGTGATTTTTCTCTGGT CCCGCCGCATCCATACCGCCAGTTGTTTACCCTCACAACGTTCCAGTAACCGGGCAT GTTCATCATCAGTAACCCGTATCGTGAGCATCCTCTCTCGTTTCATCGGTATCATTAC CCCCATGAACAGAAATTCCCCCTTACACGGAGGCATCAAGTGACCAAACAGGAAAAA ACCGCCCTTAACATGGCCCGCTTTATCAGAAGCCAGACATTAACGCTTCTGGAGAAA CTCAACGAGCTGGACGCGGATGAACAGGCAGACATCTGTGAATCGCTTCACGACCA CGCTGATGAGCTTTACCGCAGCTGCCTCGCGCGTTTCGGTGATGACGGTGAAAACC TCTGACACATGCAGCTCCCGGAGACGGTCACAGCTTGTCTGTAAGCGGATGCCGGG AGCAGACAAGCCCGTCAGGGCGCGTCAGCGGGTGTTGGCGGGTGTCGGGGCGCA GCCATGACCCAGTCACGTAGCGATAGCGGAGTGTATACTGGCTTAACTATGCGGCA TCAGAGCAGATTGTACTGAGAGTGCACCATATGCGGTGTGAAATACCGCACAGATG CGTAAGGAGAAAATACCGCATCAGGCGCTCTTCCGCTTCCTCGCTCACTGACTCGCT GCGCTCGGTCGTTCGGCTGCGGCGAGCGGTATCAGCTCACTCAAAGGCGGTAATAC GGTTATCCACAGAATCAGGGGATAACGCAGGAAAGAACATGTGAGCAAAAGGCCAG CAAAAGGCCAGGAACCGTAAAAAGGCCGCGTTGCTGGCGTTTTTCCATAGGCTCCG CCCCCCTGACGAGCATCACAAAAATCGACGCTCAAGTCAGAGGTGGCGAAACCCGA CAGGACTATAAAGATACCAGGCGTTTCCCCCTGGAAGCTCCCTCGTGCGCTCTCCT GTTCCGACCCTGCCGCTTACCGGATACCTGTCCGCCTTTCTCCCTTCGGGAAGCGT GGCGCTTTCTCAATGCTCACGCTGTAGGTATCTCAGTTCGGTGTAGGTCGTTCGCTC CAAGCTGGGCTGTGTGCACGAACCCCCCGTTCAGCCCGACCGCTGCGCCTTATCCG GTAACTATCGTCTTGAGTCCAACCCGGTAAGACACGACTTATCGCCACTGGCAGCAG CCACTGGTAACAGGATTAGCAGAGCGAGGTATGTAGGCGGTGCTACAGAGTTCTTG AAGTGGTGGCCTAACTACGGCTACACTAGAAGGACAGTATTTGGTATCTGCGCTCTG CTGAAGCCAGTTACCTTCGGAAAAAGAGTTGGTAGCTCTTGATCCGGCAAACAAACC ACCGCTGGTAGCGGTGGTTTTTTTGTTTGCAAGCAGCAGATTACGCGCAGAAAAAAA GGATCTCAAGAAGATCCTTTGATCTTTTCTACGGGGTCTGACGCTCAGTGGAACGAA AACTCACGTTAAGGGATTTTGGTCATGAGATTATCAAAAAGGATCTTCACCTAGATCC TTTTAAATTAAAAATGAAGTTTTAAATCAATCTAAAGTATATATGAGTAAACTTGGTCT GACAGTTACCAATGCTTAATCAGTGAGGCACCTATCTCAGCGATCTGTCTATTTCGTT CATCCATAGTTGCCTGACTCCCCGTCGTGTAGATAACTACGATACGGGAGGGCTTAC CATCTGGCCCCAGTGCTGCAATGATACCGCGAGACCCACGCTCACCGGCTCCAGAT TTATCAGCAATAAACCAGCCAGCCGGAAGGGCCGAGCGCAGAAGTGGTCCTGCAAC TTTATCCGCCTCCATCCAGTCTATTAATTGTTGCCGGGAAGCTAGAGTAAGTAGTTC GCCAGTTAATAGTTTGCGCAACGTTGGTTGNNNNNNAAAAAGGATCTTCACCTAGAT CCTTTTCACGTAGAAAGCCAGTCCGCAGAAACGGTGCTGACCCCGGATGAATGTCA GCTACTGGGCTATCTGGACAAGGGAAAACGCAAGCGCAAAGAGAAAGCAGGTAGCT TGCAGTGGGCTTACATGGCGATAGCTAGACTGGGCGGTTTTATGGACAGCAAGCGA ACCGGAATTGCCAGCTGGGGCGCCCTCTGGTAAGGTTGGGAAGCCCTGCAAAGTAA ACTGGATGGCTTTCTCGCCGCCAAGGATCTGATGGCGCAGGGGATCAAGCTCTGAT CAAGAGACAGGATGAGGATCGTTTCGCATGATTGAACAAGATGGATTGCACGCAGG TTCTCCGGCCGCTTGGGTGGAGAGGCTATTCGGCTATGACTGGGCACAACAGACAA TCGGCTGCTCTGATGCCGCCGTGTTCCGGCTGTCAGCGCAGGGGCGCCCGGTTCT TTTTGTCAAGACCGACCTGTCCGGTGCCCTGAATGAACTGCAAGACGAGGCAGCGC GGCTATCGTGGCTGGCCACGACGGGCGTTCCTTGCGCAGCTGTGCTCGACGTTGTC ACTGAAGCGGGAAGGGACTGGCTGCTATTGGGCGAAGTGCCGGGGCAGGATCTCC TGTCATCTCACCTTGCTCCTGCCGAGAAAGTATCCATCATGGCTGATGCAATGCGGC GGCTGCATACGCTTGATCCGGCTACCTGCCCATTCGACCACCAAGCGAAACATCGC ATCGAGCGAGCACGTACTCGGATGGAAGCCGGTCTTGTCGATCAGGATGATCTGGA CGAAGAGCATCAGGGGCTCGCGCCAGCCGAACTGTTCGCCAGGCTCAAGGCGAGC ATGCCCGACGGCGAGGATCTCGTCGTGACCCATGGCGATGCCTGCTTGCCGAATAT CATGGTGGAAAATGGCCGCTTTTCTGGATTCATCGACTGTGGCCGGCTGGGTGTGG CGGACCGCTATCAGGACATAGCGTTGGCTACCCGTGATATTGCTGAAGAGCTTGGC GGCGAATGGGCTGACCGCTTCCTCGTGCTTTACGGTATCGCCGCTCCCGATTCGCA GCGCATCGCCTTCTATCGCCTTCTTGACGAGTTCTTCTGAATTTTGTTAAAATTTTTGT TAAATCAGCTCATTTTTTAACCAATAGGCCGAAATCGGCAACATCCCTTATAAATCAA AAGAATAGACCGCGATAGGGTTGAGTGTTGTTCCAGTTTGGAACAAGAGTCCACTAT TAAAGAACGTGGACTCCAACGTCAAAGGGCGAAAAACCGTCTATCAGGGCGATGGC CCACTACGTGAACCATCACCCAAATCAAGTTTTTTGCGGTCGAGGTGCCGTAAAGCT CTAAATCGGAACCCTAAAGGGAGCCCCCGATTTAGAGCTTGACGGGGAAAGCCGGC GAACGTGGCGAGAAAGGAAGGGAAGAAAGCGAAAGGAGCGGGCGCTAGGGCGCT GGCAAGTGTAGCGGTCACGCTGCGCGTAACCACCACACCCGCGCGCTTAATGCGC CGCTACAGGGCGCGTCCATTCGCCATTCAGGATCGAATTAATTCTTAAT |
| 128 | STAT1 binding site (1) | TTCCGGGAA |
| 129 | STAT1 binding site (2) | TTCTCGGAA |
| 130 | Human CD44v6 | MDKFWWHAAWGLCLVPLSLAQIDLNITCRFAGVFHVEKNGRYSISRTEAADLCKAFNST LPTMAQMEKALSIGFETCRYGFIEGHVVIPRIHPNSICAANNTGVYILTSNTSQYDTYCFN ASAPPEEDCTSVTDLPNAFDGPITITIVNRDGTRYVQKGEYRTNPEDIYPSNPTDDDVSS GSSSERSSTSGGYIFYTFSTVHPIPDEDSPWITDSTDRIPATIQATPSSTTEETATQKEQW FGNRWHEGYRQTPKEDSHSTTGTAAASAHTSHPMQGRTTPSPEDSSWTDFFNPISHP MGRGHQAGRRMDMDSSHSITLQPTANPNTGLVEDLDRTGPLSMTTQQSNSQSFSTSH EGLEEDKDHPTTSTLTSSNRNDVTGGRRDPNHSEGSTTLLEGYTSHYPHTKESRTFIPV TSAKTGSFGVTAVTVGDSNSNVNRSLSGDQDTFHPSGGSHTTHGSESDGHSHGSQEG GANTTSGPIRTPQIPEWLIILASLLALALILAVCIAVNSRRRCGQKKKLVINSGNGAVEDRK PSGLNGEASKSQEMVHLVNKESSETPDQFMTADETRNLQNVDMKIGV |
| 131 | HER2(FRP5)-CD28TM,ICD-CD3Z CAR | EVQLQQSGPELKKPGETVKISCKASGYPFTNYGMNWVKQAPGQGLKWMGWINTSTGE STFADDFKGRFDFSLETSANTAYLQINNLKSEDMATYFCARWEVYHGYVPYWGQGTTV TVSSGGGGSGGGGSGGGGSDIQLTQSHKFLSTSVGDRVSITCKASQDVYNAVAWYQQ KPGQSPKLLIYSASSRYTGVPSRFTGSGSGPDFTFTISSVQAEDLAVYFCQQHFRTPFTF GSGTKLEIKEPKSCDKTHTCPTRFWVLVVVGGVLACYSLLVTVAFIIFWVRSKRSRLLHS DYMNMTPRRPGPTRKHYQPYAPPRDFAAYRSRVKFSRSADAPAYQQGQNQLYNELNL GRREEYDVLDKRRGRDPEMGGKPQRRKNPQEGLYNELQKDKMAEAYSEIGMKGERR RGKGHDGLYQGLSTATKDTYDALHMQALPPR |

The disclosure includes the combination of the aspects and preferred features described except where such a combination is clearly impermissible or expressly avoided.

The section headings used herein are for organizational purposes only and are not to be construed as limiting the subject matter described.

Aspects and embodiments of the present disclosure will now be illustrated, by way of example, with reference to the accompanying figures. Further aspects and embodiments will be apparent to those skilled in the art. All documents mentioned in this text are incorporated herein by reference.

Throughout this specification, including the claims which follow, unless the context requires otherwise, the word "comprise," and variations such as "comprises" and "comprising," will be understood to imply the inclusion of a stated integer or step or group of integers or steps but not the exclusion of any other integer or step or group of integers or steps.

It must be noted that, as used in the specification and the appended claims, the singular forms "a," "an," and "the" include plural referents unless the context clearly dictates otherwise. Ranges may be expressed herein as from "about" one particular value, and/or to "about" another particular value. When such a range is expressed, another embodiment includes from the one particular value and/or to the other particular value. Similarly, when values are expressed as approximations, by the use of the antecedent "about," it will be understood that the particular value forms another embodiment.

Where a nucleic acid sequence in disclosed the reverse complement thereof is also expressly contemplated.

### Brief Description of the Figures

Embodiments and studies illustrating the principles of the disclosure will now be discussed with reference to the accompanying figures.

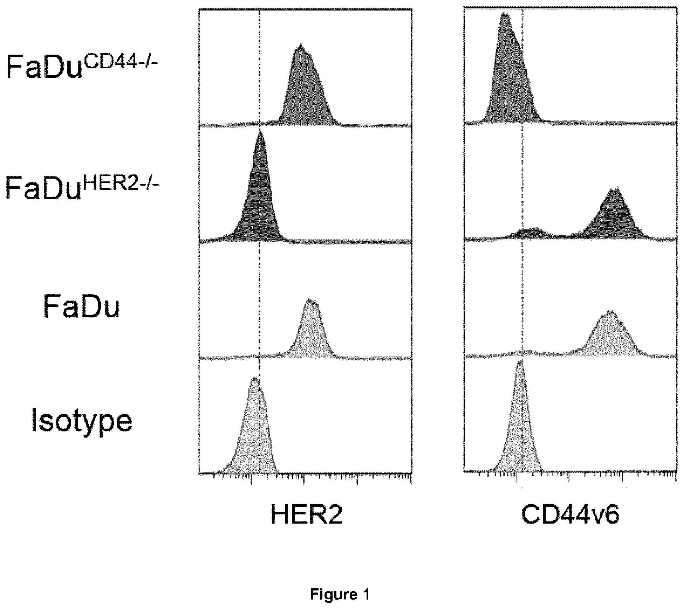

**Figure 1****.** Histograms showing HER2 and CD44v6 expression by FaDu cells, FaDu^{HER2-/-} and FaDu^{CD44-/-}cells as determined by flow cytometry.
**Figure 2****.** Histograms showing HER2 and CD44v6 expression by FaDu cells analysed prior to administration to mice, and FaDu cells obtained from lymph nodes at 20 weeks post-administration.

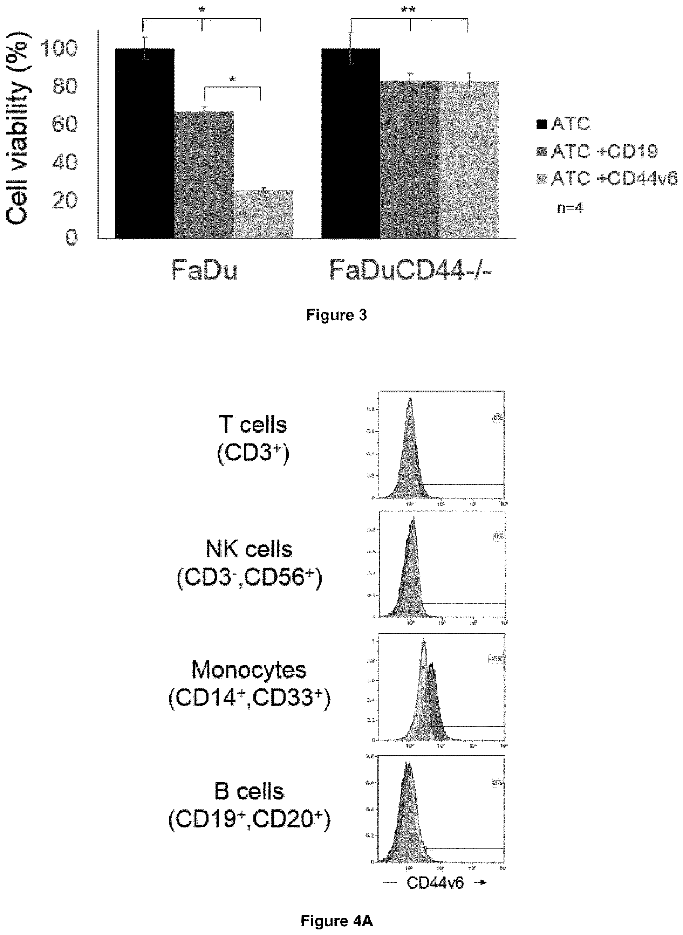

**Figure 3****.** Bar chart showing *in vitro* cell killing of FaDu and FaDu^{CD44-/-} cells by activated T cells (ATCs), either alone (ATC), or in the presence of cell culture supernatant containing anti-CD19 BiTE (ATC + CD19) or cell culture supernatant containing anti-CD44v6 BiTE (ATC + CD44v6). Data are presented as mean ± SD (n=4). **P <* 0.001; ***P* < 0.01.

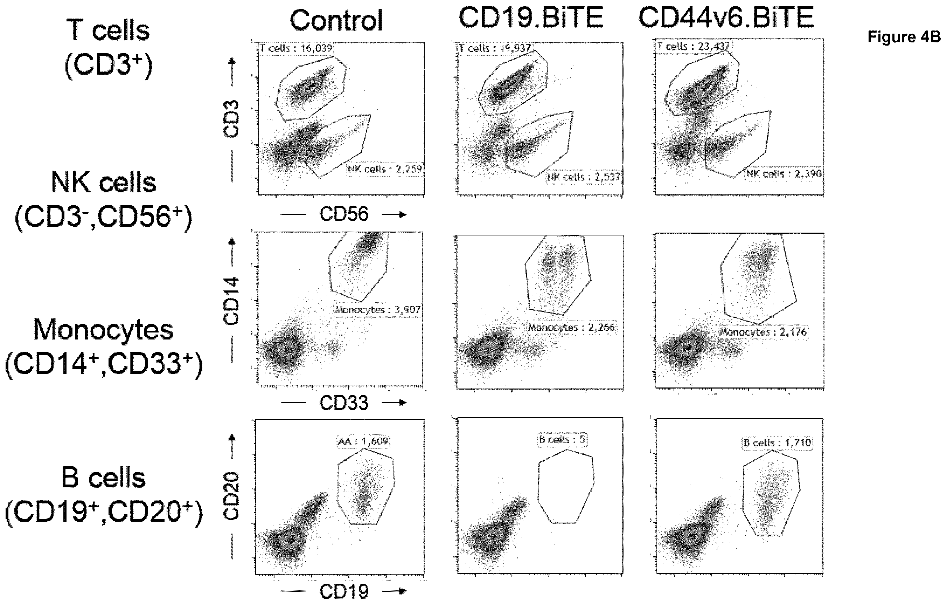

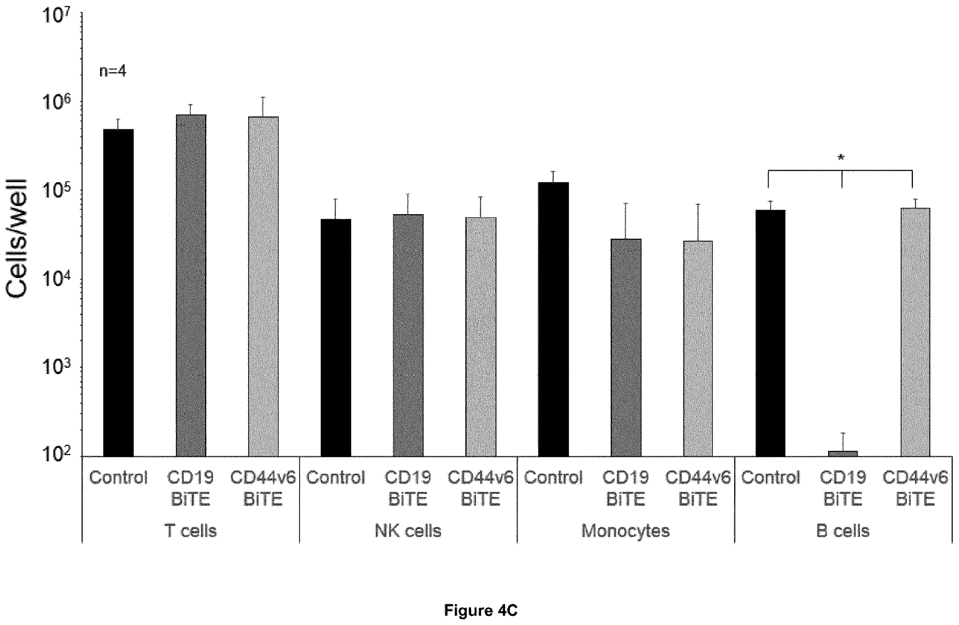

**Figures 4A to 4C****.** Histograms, scatterplots and bar chart showing the results of characterisation by flow cytometry of the immune cell population comprising activated T cells (ATCs) used in *in vitro* analysis of the anti-cancer activity of ATCs in the presence of cell culture supernatant containing anti-CD19 BiTE or cell culture supernatant containing anti-CD44v6 BiTE. **4A** shows CD44v6 expression by different immune cell subsets. **4B** and **4C** show the numbers of the different immune cell subsets following incubation of the population comprising ATCs in the presence of FaDu cells (Control), and in the presence of cell culture supernatant containing anti-CD19 BiTE (CD19 BiTE), or cell culture supernatant containing anti-CD44v6 BiTE (CD44v6 BiTE). Data are presented as mean ± SD (n=4). **P* < 0.001.

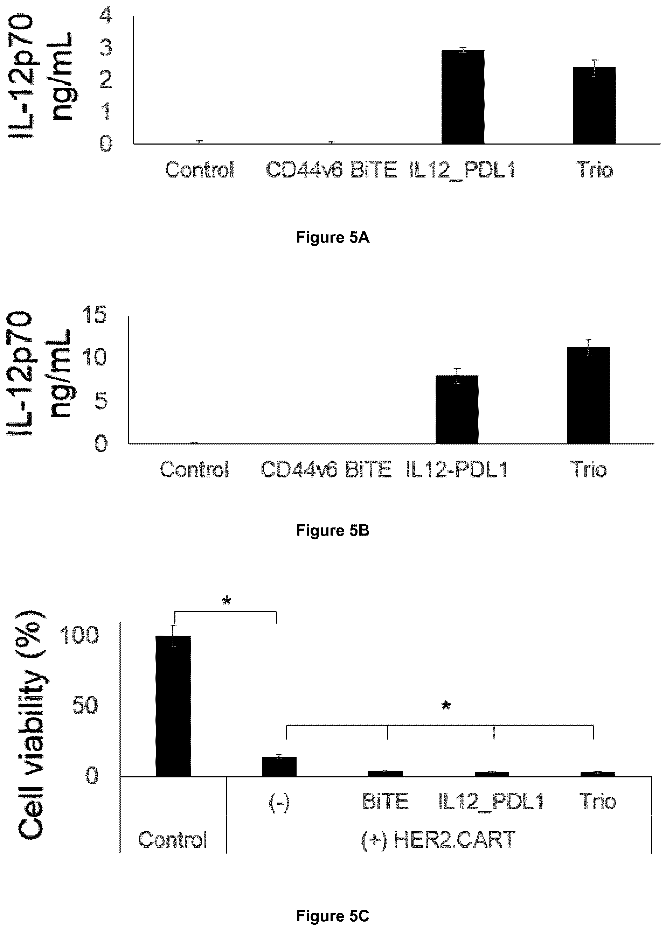

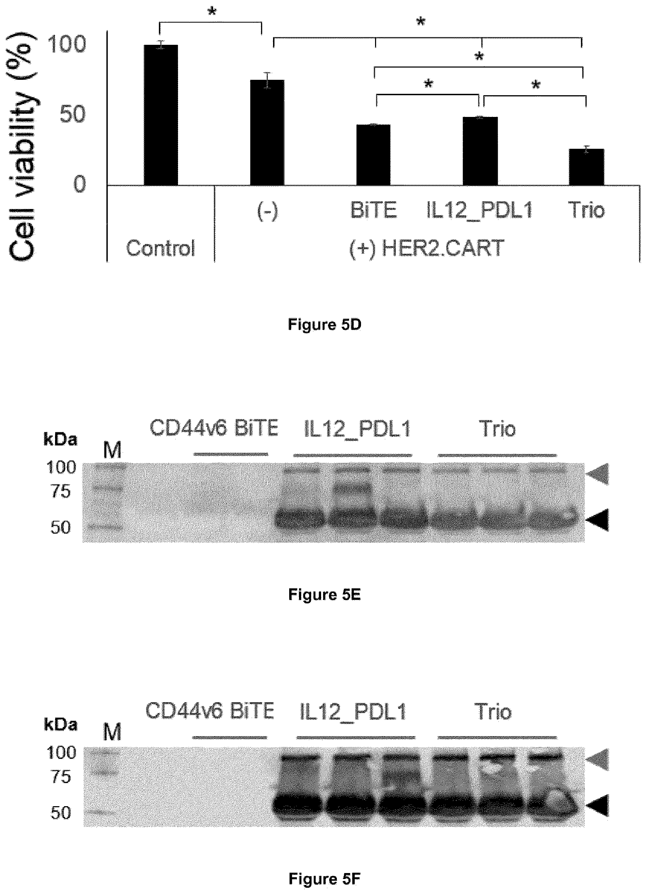

**Figures 5A to 5F****.** Bar charts and images showing the results of *in vitro* analysis of cell killing and HDAd transgene expression. Firefly luciferase-labelled FaDu and FaDu^{HER2-/-} cells were infected with HD*AdCD44v6BiTE* (BiTE), HDA*dIL-12_PO-L1* (IL-12_PDL1) or HD*AdTrio* (Trio). 24 hours post-infection, HER2-specific CAR-T cells were added at an effector:target cell ratio of 1:10. Cell culture supernatant was collected and analysed for IL-12p70 by ELISA and anti-PD-L1 minibody by western blot, and cell viability was evaluated. Results obtained using FaDu cells are shown in **5A**, **5C** and **5E**. Results obtained using FaDu^{HER2-/-} cells are shown in **5B**, **5D** and **5F**. **5A** and **5B** show IL-12p70 detected in the cell culture supernatant of uninfected cells (Control) or cells infected with the indicated HdAd. **5C** and **5D** show the cell viability of uninfected cells in the absence of co-culture with HER2-specific CAR-T cells (Control), or following co-culture with HER2-specific CAR-T cells (-), and cell viability of cells infected with the indicated HdAd following co-culture with HER2-specific CAR-T cells. **5E** and **5F** show anti-PD-L1 minibody detected in the cell culture supernatant of cells infected with the indicated HdAd. Data are presented as mean ± SD (n=4) **P* < 0.001.

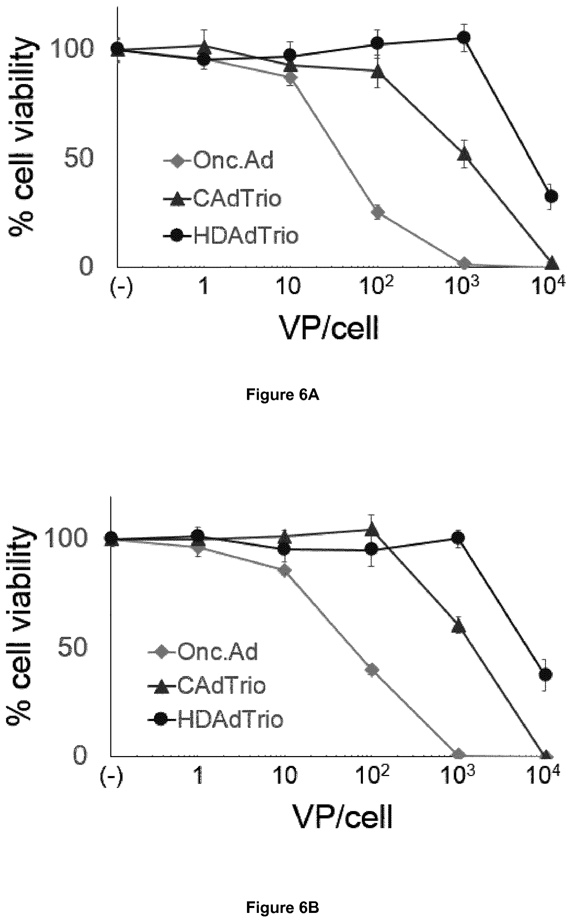

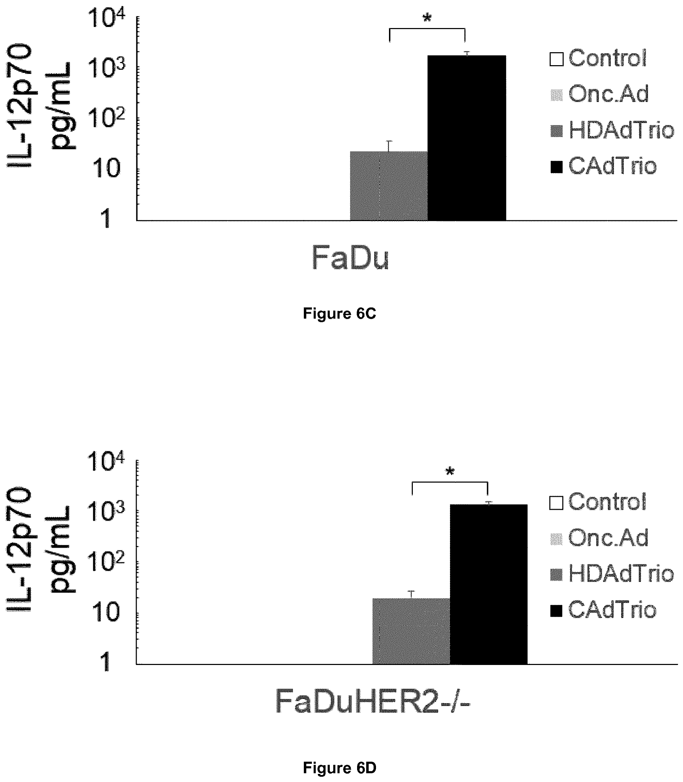

**Figures 6A to 6D****.** Graphs and bar charts showing the results of *in vitro* analysis of cell killing and HDAd transgene expression. FaDu and FaDu^{HER2-/-} cells were infected with Onc.Ad alone, HDAd*Trio* alone or Onc5/3Ad2E1Δ24 + HDAd*Trio* (CAdTrio), at various different viral particle/cell concentrations. Cell culture supernatant was collected was analysed for IL-12p70 by ELISA, and cell viability was evaluated. Results obtained using FaDu cells are shown in **6A** and **6C,** and results obtained using FaDu^{HER2-/-} cells are shown in **6B** and **6D. 6A** and **6B** show the cell viability of cells infected with the indicated virus/combination of viruses, at the indicated viral particle/cell concentrations. **6C** and **6D** show IL-12p70 detected in the cell culture supernatant of cells infected with the indicated virus/combination of viruses. **P* < 0.001.

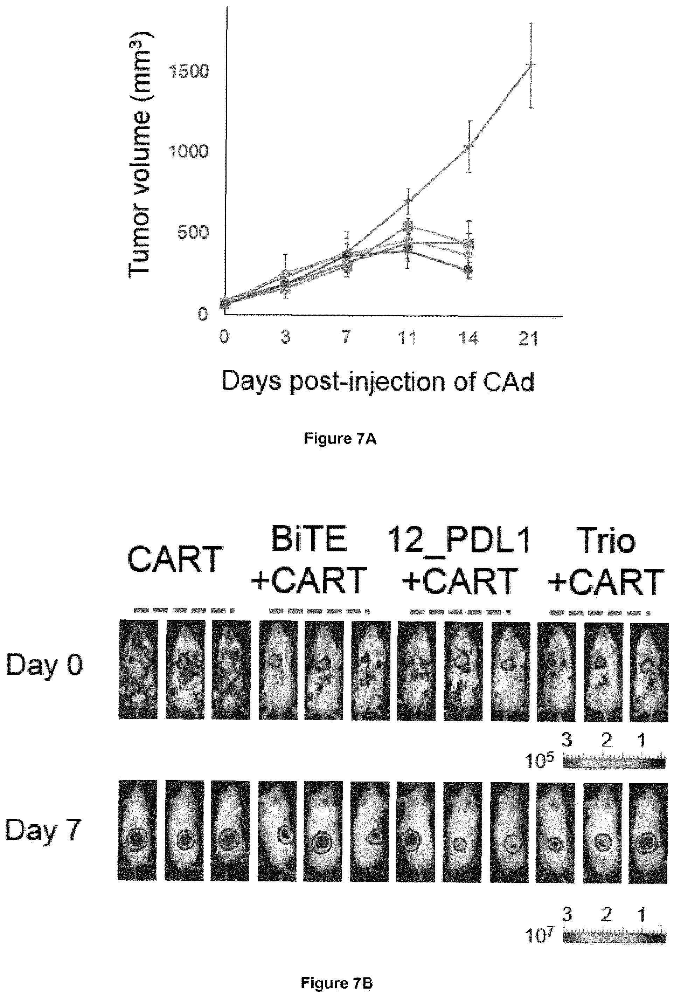

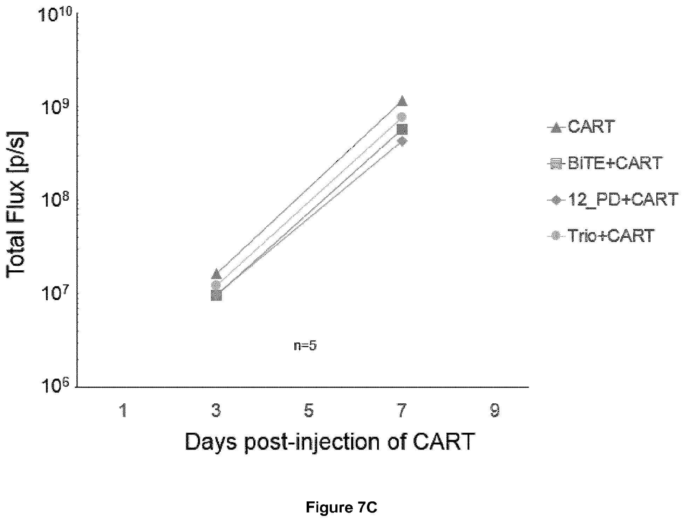

**Figures 7A to 7C****.** Graphs and images showing the results of *in vivo* analysis of the anti-cancer activity of the combination of HER2-specific CAR-T cell therapy with different OncAd + HDAd combinations in an ectopic FaDu cell-derived model of squamous cell head and neck carcinoma. FaDu cells were transplanted subcutaneously into the right flank of NSG mice, and mice were untreated (Control), or administered with firefly luciferase-labelled HER2-specific CAR-T cells alone (CART), or in combination with Onc5/3Ad2E1Δ24 and HDAd*CD44v6BiTE* (BiTE + CART), Onc5/3Ad2E1Δ24 and HDAd*IL-12_PD-L1* (12_PD + CART) or Onc5/3Ad2E1Δ24 and *HDAdTrio* (Trio + CART). **7A** shows tumor volumes at the indicated number of days after administration of the OncAd + HDAd combinations. **7B** and **7C** show the total flux (in photons per second; p/s) of ventral surface for mice of the different treatment groups at the indicated number of days after infusion of the CAR-T cells.

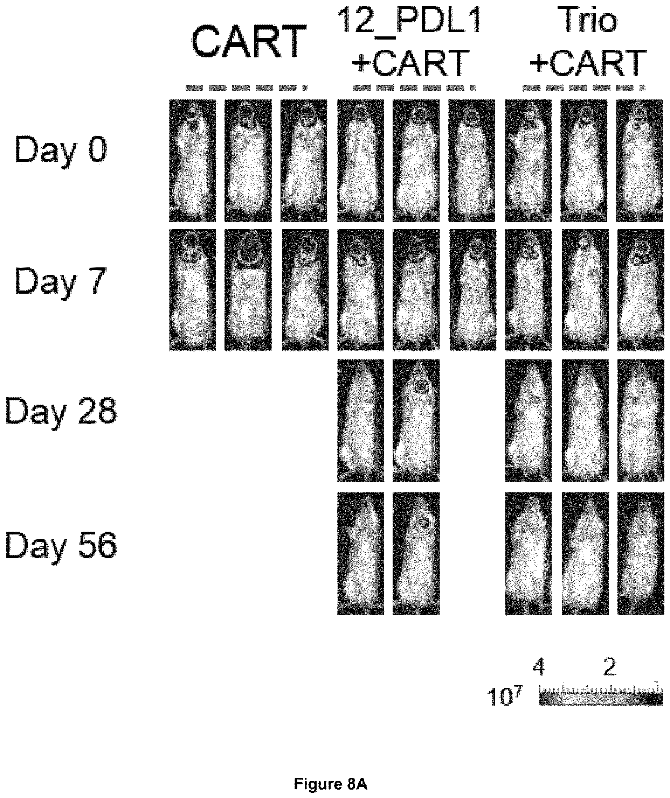

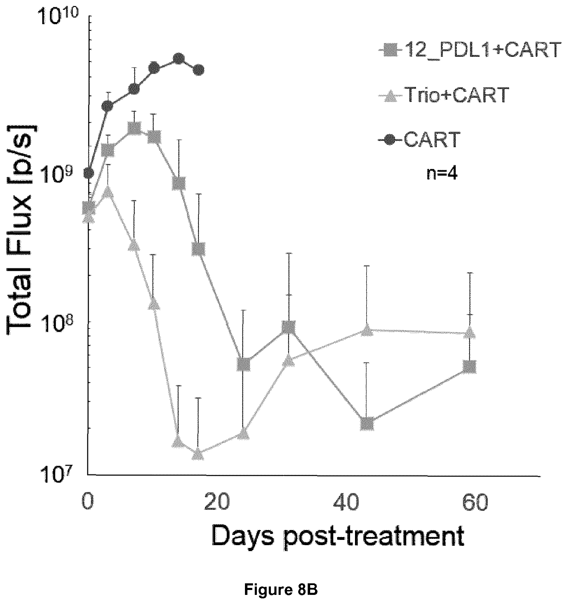

**Figures 8A** **and** **8B****.** Images and graphs showing the results of *in vivo* analysis of the anti-cancer activity of the combination of HER2-specific CAR-T cell therapy with different OncAd + HDAd combinations in an orthotopic FaDu cell-derived model of squamous cell head and neck carcinoma. Firefly luciferase-labelled FaDu cells were transplanted orthotopically into NSG mice, and mice were untreated, or administered with HER2-specific CAR-T cells alone (CART), or in combination with Onc5/3Ad2E1Δ24 and HDAd*IL-12_PD-L1* (12_PDL1 + CART) or Onc5/3Ad2E1Δ24 and *HDAdTrio* (Trio + CART). **8A** and **8B** show the total flux (in photons per second; p/s) of ventral surface for mice of the different treatment groups at the indicated number of days after infusion of the CAR-T cells.

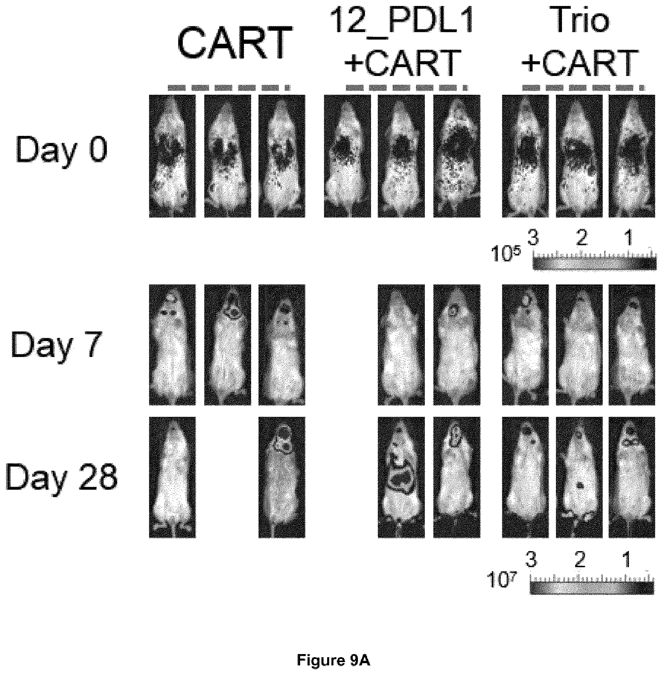

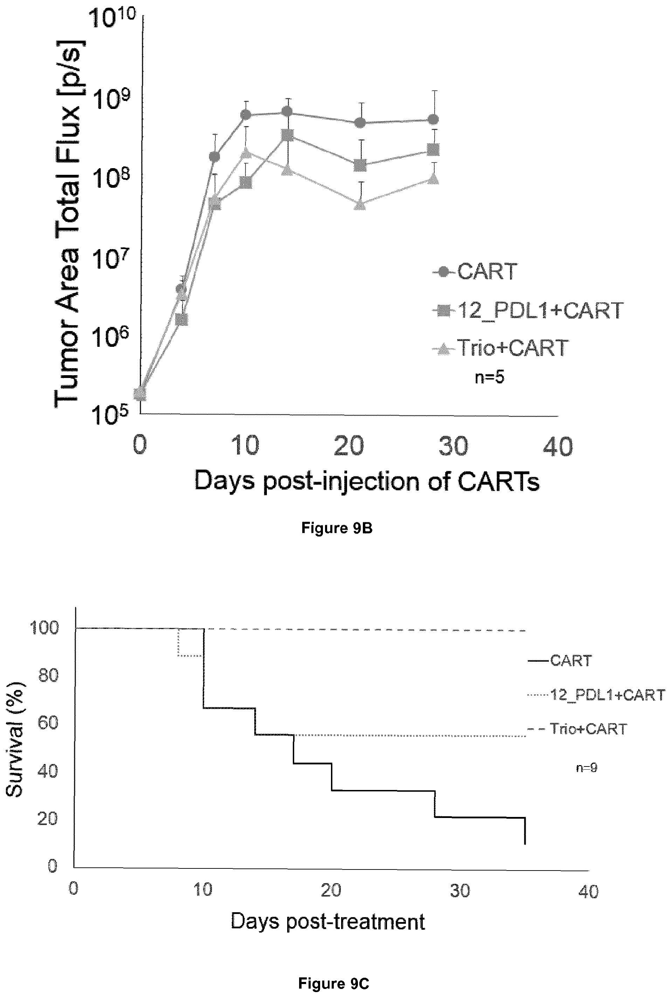

**Figures 9A to 9C****.** Images and graphs showing the results of *in vivo* analysis of the anti-cancer activity of the combination of HER2-specific CAR-T cell therapy with different OncAd + HDAd combinations in an orthotopic FaDu cell-derived model of squamous cell head and neck carcinoma. FaDu cells were transplanted orthotopically into NSG mice, and mice were untreated, or administered with firefly luciferase-labelled HER2-specific CAR-T cells alone (CART), or in combination with Onc5/3Ad2E1Δ24 and HDAd*IL-12_PD-L1* (12_PDL1 + CART) or Onc5/3Ad2E1Δ24 and HDAd*Trio* (Trio + CART). **9A** and **9B** show the total flux (in photons per second; p/s) of ventral surface for mice of the different treatment groups at the indicated number of days after infusion of the CAR-T cells. **9C** shows percentage of surviving subjects in the different treatment groups at the indicated number of days after infusion of the CAR-T cells.

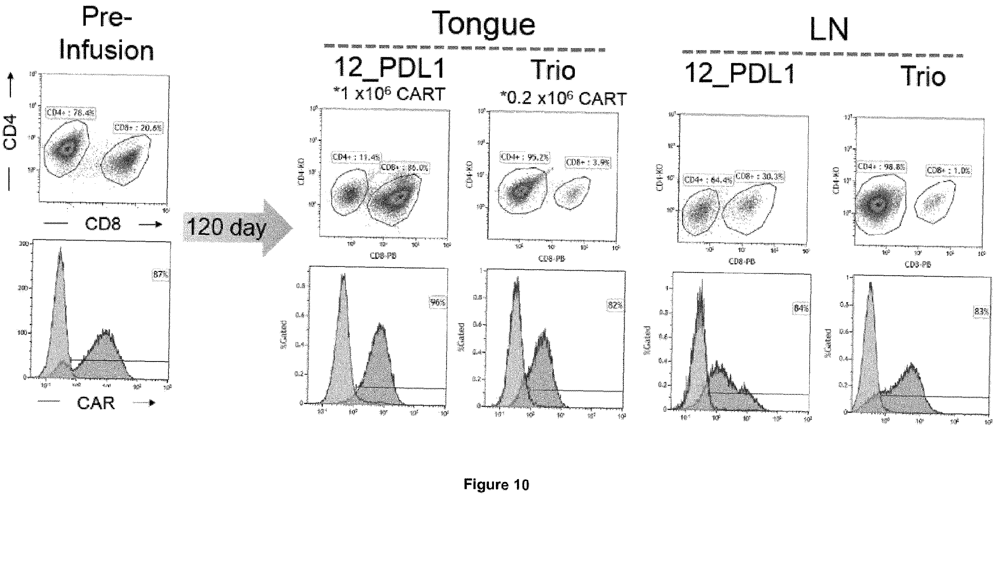

**Figure 10****.** Scatterplots and histograms showing the results of characterisation by flow cytometry of firefly luciferase-labelled HER2-specific CAR-T cells prior to infusion, and HER2-specific CAR-T cells obtained from the tongue and lymph nodes of mice at 120 days post-infusion, from mice treated with Onc5/3Ad2E1Δ24 and HDAd*IL-12 PD-L1* (12_PDL1) or Onc5/3Ad2E1Δ24 and HDAd*Trio* (Trio). The CAR-T cells were analysed for CD4, CD8 and CAR expression.

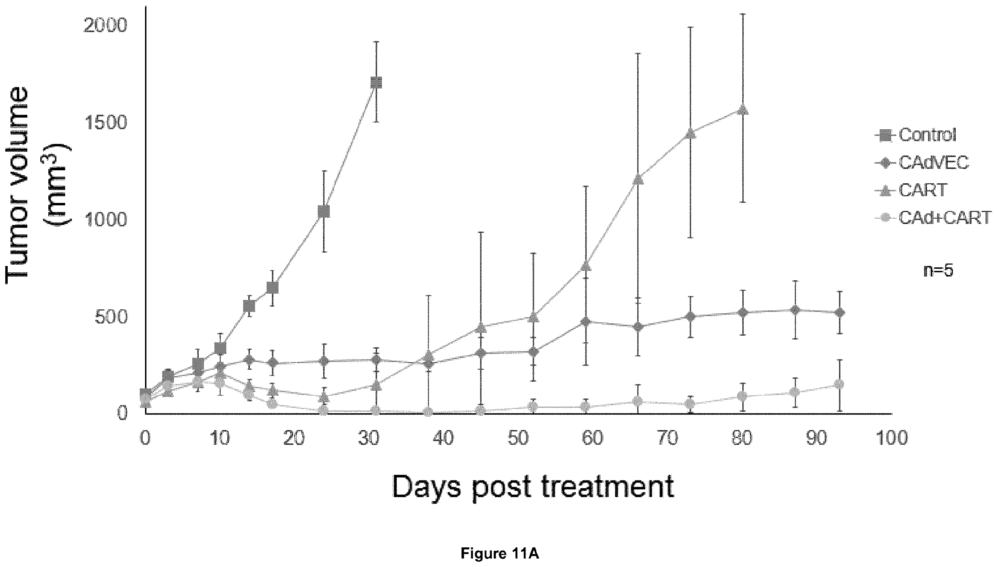

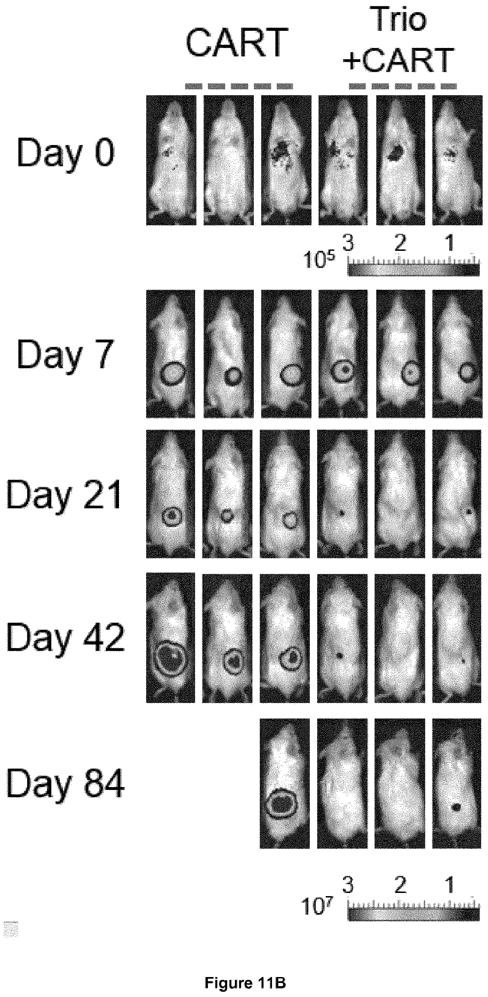

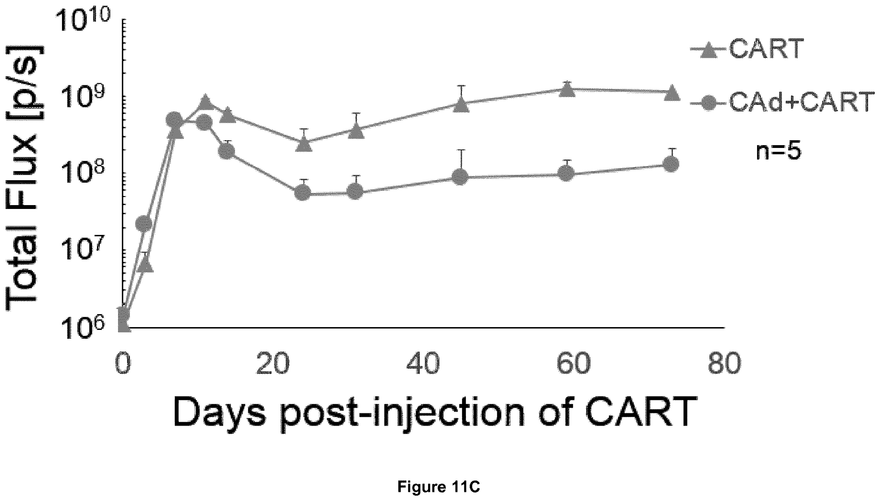

**Figures 11A to 11C****.** Graphs and images showing the results of *in vivo* analysis of the anti-cancer activity of HER2-specific CAR-T cell therapy and OncAd + HDAd in an ectopic PC-3 cell-derived model of prostate adenocarcinoma. PC-3 cells were transplanted subcutaneously into the right flank of NSG mice, and mice were untreated (Control), administered with firefly luciferase-labelled HER2-specific CAR-T cells alone (CART), the combination of Onc5/3Ad2E1Δ24 and HDAd*Trio* (CAdVEC), or firefly luciferase-labelled HER2-specific CAR-T cells in combination with Onc5/3Ad2E1Δ24 and HDAd*Trio* (CAd + CART; Trio + CART). **11A** shows tumor volumes at the indicated number of days after administration of the OncAd + HDAd combinations. **11B** and **11C** show the total flux (in photons per second; p/s) of ventral surface for mice of the different treatment groups at the indicated number of days after infusion of the CAR-T cells.

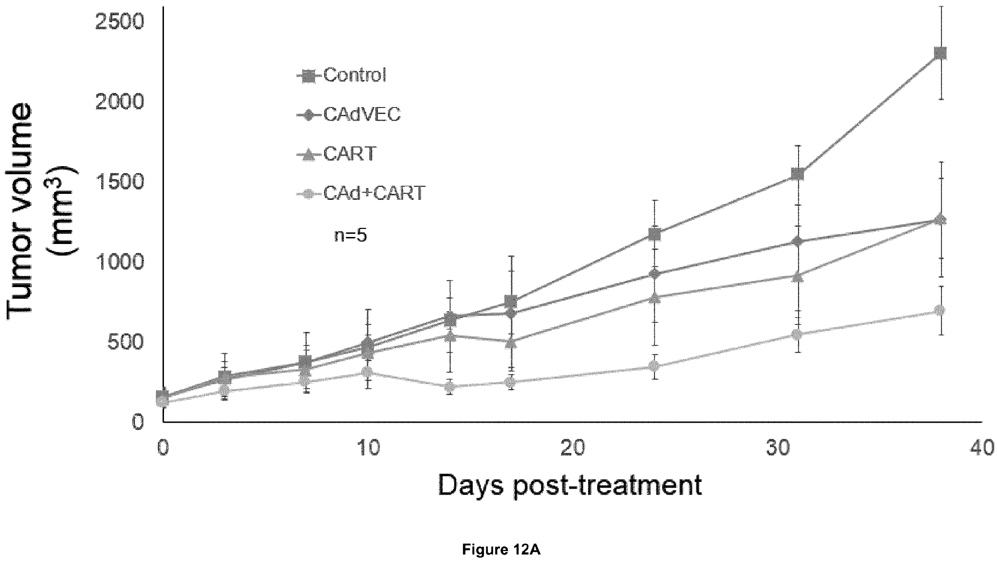

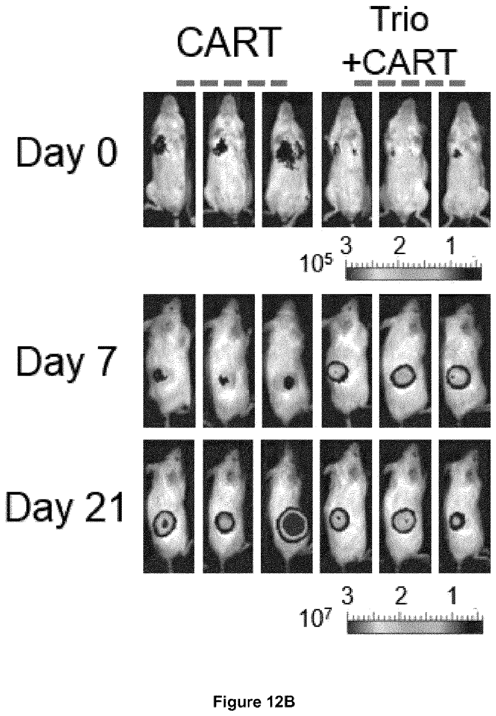

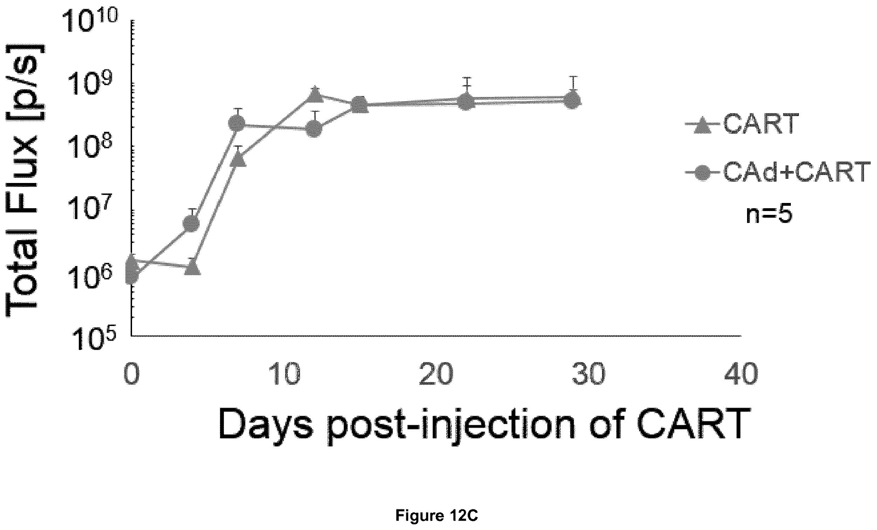

**Figures 12A to 12C****.** Graphs and images showing the results of *in vivo* analysis of the anti-cancer activity of HER2-specific CAR-T cell therapy and OncAd + HDAd in an ectopic CAPAN-1 cell-derived model of pancreatic adenocarcinoma. CAPAN-1 cells were transplanted subcutaneously into the right flank of NSG mice, and mice were untreated (Control), administered with firefly luciferase-labelled HER2-specific CAR-T cells alone (CART), the combination of Onc5/3Ad2E1Δ24 and HDAd*Trio* (CAdVEC), or firefly luciferase-labelled HER2-specific CAR-T cells in combination with Onc5/3Ad2E1Δ24 and HDAd*Trio* (CAd + CART; Trio + CART). **12A** shows tumor volumes at the indicated number of days after administration of the OncAd + HDAd combinations. **12B** and **12C** show the total flux (in photons per second; p/s) of ventral surface for mice of the different treatment groups at the indicated number of days after infusion of the CAR-T cells.

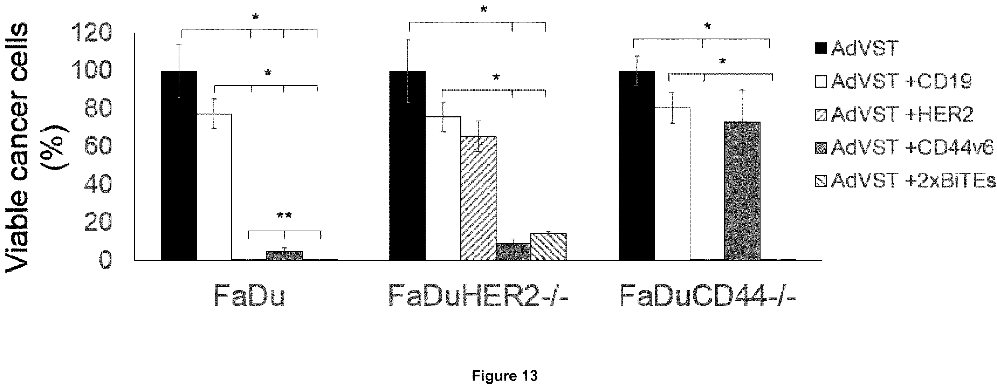

**Figure 13****.** Bar chart showing the results of *in vitro* analysis of cell killing of FaDu, FaDu^{HER2-/-} and FaDu^{CD44v6-/-} cells in the presence of AdVSTs alone (AdVST), or in the presence of cell culture supernatant containing anti-CD19 BiTE (AdVST + CD19), cell culture supernatant containing anti-HER2 BiTE (AdVST + HER2), cell culture supernatant containing anti-CD44v6 BiTE (AdVST + CD44v6) or cell culture supernatant containing anti-HER2 BiTE and anti-CD44v6 BiTE (AdVST + 2xBiTEs). Data are presented as mean ± SD (n=5). **P* < 0.001; ***P* < 0.01.

### Examples

### Example 1: Materials and Methods

### 1.1 Generation of antigen-specific CAR-T cells

HER2-binding CAR constructs were prepared. Briefly, DNA encoding scFv (*i.e*. VL domain and VH domain joined by a linker sequence) for the anti-HER2 antibody clone FRP5 was cloned into a CAR construct backbone comprising a 5' signal peptide (SP), and CD28 transmembrane (TM) and intracellular domain sequence, with a 3' CD3ζ intracellular domain sequence. The encoded CAR is shown in SEQ ID NO:131.

HER2 specific CAR-T cells were subsequently generated. Briefly, human PBMCs were isolated from blood samples by Ficoll density gradient centrifugation. Cells were stimulated with anti-CD3(OKT3)/anti-CD28 in the presence of IL-2 to promote T cell activation and proliferation, and the cells were transduced with retrovirus encoding the HER2 CAR construct. T-cells were expanded by culture in the presence of 100 IU/mL recombinant human IL-2, and were frozen at 6 days post-transduction.

PSCA-specific CAR-T cells were generated in the same way, using the PCSA-specific CAR construct "2G.CAR.PSCA" described in Watanabe et al., Oncoimmunology (2016) 5(12): e1253656, which is hereby incorporated by reference in its entirety (represented schematically in Figure 1A of Watanabe et al., Oncoimmunology (2016) 5(12): e1253656).

T-cells were thawed and expanded in the presence of 100 IU/mL of recombinant human IL-2 for 5 days and used for *in vitro*/*in vivo* experiments and phenotypic analysis.

### 1.2 Helper-dependent Ad (HDAd) constructs

Novel constructs encoding a helper-dependent adenovirus were prepared using recombinant DNA techniques.

*HDAdCD19BiTE* contains sequence encoding an anti-CD19 bispecific T cell engager (BiTE), which comprises scFv specific for CD19 joined via a linker to scFv specific for CD3 (clone OKT3). The CD19 scFv comprises the VH and VL of clone FMC63. The nucleotide sequence for HDAd*CD19BiTE* is shown in SEQ ID NO:123, and the encoded BiTE is shown in SEQ ID NO:93.

HDAd*CD44v6BiTE* contains sequence encoding an anti-CD44v6 BiTE, which comprises scFv specific for CD44v6 joined via a linker to scFv specific for CD3 (clone OKT3). The CD44v6 scFv comprises the VH and VL of clone BIWA8 described e.g. in US 2005/0214212 A1. The nucleotide sequence for HDAd*CD44v6BiTE* is shown in SEQ ID NO:121, and the encoded BiTE is shown in SEQ ID NO:64.

HDAd*HER2BiTE* contains sequence encoding an anti-HER2 BiTE, which comprises scFv specific for HER2 joined via a linker to scFv specific for CD3 (clone OKT3). The HER2 scFv comprises the VH and VL of clone FRP5. The nucleotide sequence for HDAd*CD44v6BiTE* is shown in SEQ ID NO:122, and the encoded BiTE is shown in SEQ ID NO:83.

HDAd*Trio* contains sequence encoding expression cassettes for (i) an anti-CD44v6 BiTE, which comprises scFv specific for CD44v6 joined via a linker to scFv specific for CD3 (clone OKT3), (ii) human IL-12p70 (sequence encoding alpha and beta chains), and (iii) an anti-PD-L1 minibody derived from YW243.55.S70 (atezolizumab). The three coding sequences each have their own polyA signal sequences. The nucleotide sequence for HDAd*Trio* is shown in SEQ ID NO:125.

*HD2xBiTEs* contains sequence encoding (i) an anti-HER2 BiTE, which comprises scFv specific for HER2 joined via a linker to scFv specific for CD3, and (ii) an anti-CD44v6 BiTE, which comprises scFv specific for CD44v6 joined via a linker to scFv specific for CD3. The anti-HER2 BiTE and anti-CD44v6 BiTE are encoded by the same expression cassette, joined by a T2A autocleavage linker sequence. The nucleotide sequence for HD*2xBiTEs* is shown in SEQ ID NO:122, and the encoded BiTE is shown in SEQ ID NO:103.

HDAd*IL-12*_*TK_PD-L1* contains sequence encoding expression cassettes for (i) human IL-12p70 (sequence encoding alpha and beta chains), (ii) HSV-1 thymidine kinase, and (iii) an anti-PD-L1 minibody (comprising the CDRs of anti-PD-L1 clone H12_gl described e.g. in WO 2016111645 A1). The three coding sequences each have their own polyA signal sequences. The nucleotide sequence for HDAd/L-*12_TK_PD-L1* is shown in SEQ ID NO:120.

HDAd*IL-12 PD-L1* contains sequence encoding human IL-12p70 protein and anti-PD-L1 minibody derived from YW243.55.S70 (atezolizumab). The anti-PD-L1 minibody of this construct consists of scFv for YW243.55.S70 fused with a hinge, CH2 and CH3 regions of human IgG1 and a C-terminal HA tag (as described e.g. in Tanoue et al. Cancer Res. (2017) 77(8):2040-2051).

### 1.3 OncAd construct

Constructs encoding oncolytic adenovirus were prepared using recombinant DNA techniques.

Onc5/3Ad2E1Δ24 (also referred to herein as "Onc5/2E1Δ24") shown in SEQ ID NO:126 was prepared by using recombinant DNA techniques. Onc5/3Ad2E1Δ24 has a similar structure as Onc5Δ24 disclosed e.g. in Fueyo et al. 2000 Oncogene 19:2-12 (hereby incorporated by reference in its entirety; Onc5Δ24 is also referred to in Fueyo et al. as "Δ24"), but differs in that Onc5/3Ad2E1Δ24 encodes E1A protein from adenovirus type 2 (Ad2) lacking the sequence LTCHEACF (SEQ ID NO:105), rather than E1A protein from adenovirus type 5 (Ad5) lacking the sequence LTCHEACF (SEQ ID NO:105).

### 1.4 CAdTrio and CAdIL-12 PD-L1

"CAd*Trio*" as used in the present Examples refers to the combination of Onc5/3Ad2E1Δ24 (described in Example 1.3) and HDAd*Trio* described in Example 1.2.

"CAd*IL-12*_*PD-L1* "as used in the present Examples refers to the combination of Onc5/3Ad2E1Δ24 (described in Example 1.3) and HDAd/*L-12_PD-L1* described in Example 1.2.

### 1.5 Cell Lines

The following cell lines are used in the experiments described in the present Examples:
FaDu - cell line derived from human pharynx squamous cell carcinoma.

FaDu^{CD44-/-} - cell line obtained by CRISPR/Cas9-KO modification of FaDu cells to specifically knockout the gene encoding CD44.

FaDu^{HER2-/-} - cell line obtained by CRISPR/Cas9-KO modification of FaDu cells to specifically knockout the gene encoding HER2.

PC-3 - cell line derived from metastatic human prostate adenocarcinoma.

CAPAN-1 - cell line derived from metastatic human pancreatic adenocarcinoma.

### 1.6 Generation of activated T cells (ATCs)

Activated T cells (ATCs) were prepared as follows.

Anti-CD3 (clone OKT3) and anti-CD28 agonist antibodies were coated onto wells of tissue culture plates by addition of 0.5 ml of 1:1000 dilution of 1 mg/ml antibodies, and incubation for 2-4 hr at 37°C, or at 4°C overnight.

PBMCs were isolated from blood samples obtained from healthy donors according to the standard Ficoll-Paque method.

1 x 10⁶ PBMCs (in 2 ml of cell culture medium) were stimulated by culture on the anti-CD3/CD28 agonist antibody-coated plates in CTL cell culture medium (containing 50% Advanced RPMI, 50% Click's medium, 10% FBS, 1% GlutaMax, 1% Pen/Strep) supplemented with 10 ng/ml IL-7 and 5 ng/ml IL-15. The cells were maintained at 37°C in a 5% CO₂ atmosphere. The next day, 1 ml of the cell culture medium was replaced with fresh CTL medium containing 20 ng/ml IL-7 and 10 ng/ml IL-15.

ATCs were maintained in culture, and subsequently harvested and used in experiments or cryopreserved between days 5-7.

### 1.7 Generation of oncolytic virus-specific T cells

Adenovirus-specific T cells (AdVSTs) were prepared as follows.

Anti-CD3 (clone OKT3) and anti-CD28 agonist antibodies were coated onto wells of tissue culture plates by addition of 0.5 ml of 1:1000 dilution of 1 mg/ml antibodies, and incubation for 2-4 hr at 37°C, or at 4°C overnight.

PBMCs were isolated from blood samples obtained from healthy donors according to the standard Ficoll-Paque method.

1 x 10⁶ PBMCs (in 2 ml of cell culture medium) were stimulated by culture on the anti-CD3/CD28 agonist antibody-coated plates in CTL cell culture medium supplemented with 10 ng/ml IL-7 and 100 ng/ml IL-15.

20 µl of a 200-fold dilution of Adenovirus-specific Hexon Pepmix (JPT Cat# PM-HAdV3) or Penton PepMix (JPT Cat# PM-HAdV5) was added to the wells. The cells were maintained at 37°C in a 5% CO₂ atmosphere. After 48 hours cells were fed with CTL medium, with added IL-7 and IL-15 to a final concentration of 10 ng/ml IL-7 and 100 ng/ml IL-15.

### 1.8 Generation of CAR-expressing oncolytic virus-specific T cells

On day 3, AdVSTs were resuspended at a concentration of 0.125 x 10⁶ cells/ml in CTL cell culture medium containing 10 ng/ml IL-7 and 100 ng/ml IL-15.

Retronectin coated plates were prepared by incubation of RetroNectin (Clontech) diluted 1:100 in PBS for 2-4 hr at 37°C, or at 4°C overnight. The wells were washed with CTL medium, 1 ml of retroviral supernatant of HER2-specific CAR retrovirus was added to wells, and plates were centrifuged at 2000g for 1.5 hr. At the end of the centrifugation step retroviral supernatant was aspirated, and 2 ml of AdVST suspension (i.e. 0.25 x 10⁶ cells) was added to wells of the plate. Plates were centrifuged at 400g for 5 min, and incubated at 37°C in a 5% CO₂ atmosphere.

After 48 hrs (i.e. on day 6) the cell culture medium was aspirated and replaced with CTL cell culture medium containing 10 ng/ml IL-7 and 100 ng/ml IL-15.

On day 9 cells were harvested and used in experiments or cryopreserved, or subjected to a second stimulation to expand CAR-expressing AdVSTs.

### 1.9 Expansion of AdVSTs and CAR-AdVSTs

AdVSTs and CAR-expressing AdVSTs were expanded by further stimulations as desired, as follows.

Pepmix-pulsed autologous ATCs were used as APCs, and K562cs cells (see e.g. Ngo et al., J Immunother. (2014) 37(4):193-203) were used as costimulatory cells. The final ratio of AdVSTs or CAR-AdVSTs:ATCs:K562cs cells in the stimulation cultures was 1:1:3-5.

AdVSTs or CAR-AdVSTs were resuspended to a concentration of 0.2 x 10⁶ cells/ml in CTL medium.

1 x 10⁶ ATCs were incubated with 10 µl of 200-fold dilution of Adenovirus-specific Hexon Pepmix (JPT Cat# PM-HAdV3) or Penton PepMix (JPT Cat# PM-HAdV5) at 37°C for 30 min. The ATCs were subsequently irradiated at 30Gy and harvested. 3-5 x 10⁶ K562cs cells were irradiated at 100Gy.

The ATCs and K562cs cells were then mixed in a total volume of 5 ml CTL medium, and 20 ng/ml IL-7 and 200 ng/ml IL-15 was added, 1 ml of this mixture was added to wells of a 24 well plate, and 1 ml of AdVST suspension or CAR-AdVST suspension was added to the wells.

Cells were maintained at 37°C in a 5% CO₂ atmosphere. After 3-4 days cell culture medium was added as necessary, and after 6-7 days cells the expanded AdVSTs or CAR-AdVSTs were harvested for use in experiments.

### Example 2: Analysis of CD44v6 and HER2 expression in head and neck cancer.

FaDu cells, FaOu^{CD44-/-} cells and FaDu^{HER2-/-} cells were analysed for expression of HER2 and CD44v6 by flow cytometry using antibodies specific for the respective targets.

The results are shown in Figure 1. FaDu cells were found to express both of HER2 and CD44v6. FaDu^{CD44-/-} cells were found to express HER2, but did not express CD44v6. FaDu^{HER2-/-} cells were found to express CD44v6, but did not express HER2.

### Example 3: Analysis of FaDu cell phenotype following treatment with HER2-specfic CAR-T cells and CAd12 PD-L1

The phenotype of cells of a FaDu cell-derived xenograft model of squamous cell head and neck cancer was investigated following treatment with CAd *12*_*PD-L1* and HER2-specific CAR-T cells.

Briefly, 0.5 x 10⁶ FaDu cells engineered to express firefly luciferase were injected orthotopically into NSG male mice. After 6 days groups of mice were injected intratumorally with 1 x 10⁸ viral particles of CAd*12_PD-L1,* at a ratio of Onc5/3Ad2E1Δ24:HDAd*IL-12_PD-L1* of 1:20; and three days later, mice were injected via the tail vein with HER2-specific CAR-T cells (see Example 1.1).

20 weeks post injection FaDu cells were obtained from the lymph node by FACS sorting of luciferase-expressing cells, and analysed by flow cytometry for expression of HER2 and CD44v6. Expression of HER2 and CD44v6 was also analysed in FaDu cells engineered to express firefly luciferase prior to administration.

The results are shown in Figure 2. The FaDu cells that persisted following treatment with CAd*12*_*PD*-L1 and HER2-specific CAR-T cells did not express HER2, but did express CD44v6.

### Example 4: T cell mediated cell killing of cancer cells facilitated by BiTEs specific for cancer cell antigens

Constructs encoding CD19-specific and CD44v6-specific BiTEs were analysed for their ability to promote cell killing of FaDu cells by activated T cells (ATCs).

Briefly, FaDu cells were infected with 200 viral particles/cell of HDAd*CD19BiTE* or *HDAdCD44v6BiTE* (see Example 1.2) by addition of viral particles to cell culture medium of the cells in culture. Cell culture supernatant was collected at 48 hours post-infection.

ATCs (see Example 1.6) were co-cultured with firefly Luciferase (ffLuc)-labelled FaDu cells or FaDU^{CD44-/-}cells (see Example 1.5) at an effector:target cell ratio of 1:10, in the presence of cell culture supernatant containing CD19-specific BiTE or CD44v6-specific BiTE.

After 72 hours, cell killing was analysed by Luciferase assay. Briefly, cells were washed with PBS, and lysis buffer was added. Cell lysates were collected, and the residual cancer cells were determined by measuring ffLuc activity using plate reader. Readings were normalised using the readings for wells containing FaDu cells or FaDu^{CD44-/-} + ATCs without addition of cell culture media containing BiTE (= 100% cell viability), and wells lacking cells (= 0% cell viability).

The results are shown in Figure 3. Culture in the presence of CD44v6-specific BiTE was found to increase the cell killing of FaDu cells substantially more than FaDu^{C144-/-} cells.

To determine whether circulating CD44v6 BiTE induces "on target, off tumor" toxicity to immune cells in blood, cell culture media containing BiTE was added to PBMCs from healthy donors in culture *in vitro.* After 72 hours in culture, PBMCs were analysed by flow cytometry. Briefly, cells were stained with antibodies specific for CD3, CD56, CD33, CD14, CD19 in order to permit the delineation of different immune cell subsets within the PBMC population.

The results are shown in Figures 4A to 4C. Culture in the presence of CD44v6-specific BiTE was found not to influence affect the numbers of T cells, NK cells, monocytes of B cells. Culture in the presence of CD19-specific BiTE was found not to influence affect the numbers of T cells, NK cells or monocytes, but significantly reduced the number of B cells.

### Example 5: Characterisation of HDAds in vitro

### 5.1 Analysis of HDAd transgene expression

Firefly luciferase-labelled FaDu and FaDu^{HER2-/-} cells were infected with 200 viral particles/cell with HDAd*CD44v6BiTE,* HDAd*IL-12 PD-L1* or HDAd*Trio* (see Example 1.2) by addition of viral particles to cell culture medium of the cells in culture. At 24 hours post-infection, HER2-specific CAR-T cells (see Example 1.1) were added at an effector:target cell ratio of 1:10. Cell culture supernatant was collected at 48 hours post-infection.

Secretion of IL-12 into the cell culture supernatant was analysed by ELISA, and secretion of anti-PD-L1 minibody was analysed by western blot using an anti-HA antibody (the anti-PD-L1 minibody comprises a C-terminal HA-tag).

At 72 hours after initiation of the co-culture, the residual FaDu cells were detected by analysis of firefly luciferase activity.

The results are shown in Figures 5A to 5F.

IL-12 was detected in the cell culture supernatant of cells infected with HDAd*IL*-*12*_*PD-L1* or HDAd*Trio.* Anti-PD-L1 minibody was also detected in the cell culture supernatant of cells infected with HDAdIL-*12_PD-L1* or HDA*dTrio.*

The HER2-specific CAR-T cells killed significantly more FaDu cells than FaDu^{HER2-/-} cells. Cell killing of FaDu cells and FaDu^{HER2-/-} cells was greater in the presence of CD44v6BiTE. Cell killing of FaDu cells and FaDu^{HER2-/-} cells was greater in the presence of IL-12 and anti-PD-L1 minibody. Cell killing of FaDu cells and FaDu^{HER2-/-} cells was greatest in the presence of CD44v6BiTE, IL-12 and anti-PD-L1 minibody.

The ability of Onc5/3Ad2E1Δ24 (Onc.Ad), the combination of Onc5/3Ad2E1Δ24 + HDAd*Trio* (referred to in the Figures as "CAd*Trio*") and HDAd*Trio* to cause cell killing of FaDu cells and FaDu^{HER2-/-} cells was analysed.

Briefly, FaDu cells or FaDu^{HER2-/-} cells were seeded in wells of 96-well plates and infected with Onc.Ad (alone), Onc5/3Ad2E1Δ24 + HDAd*Trio* (at a ratio of Onc5/3Ad2E1Δ24 to HDAd*Trio* of 1:20) or HDAd*Trio* (alone) at various different viral particle/cell concentrations. Cells were cultured for 4 days, and then MTS reagents (Promega) were added to each well, with cells being incubated at 37°C for 2 hours. Live cells were then detected by measuring the absorbance at 490nm with a plate reader. Readings were normalised using the readings for untreated cells (= 100% cell viability), and wells lacking cells (= 0% cell viability).

Cell culture supernatants from 100 viral particles/cell conditions were also collected and secretion of IL-12 into the cell culture supernatant was analysed by ELISA.

The results are shown in Figures 6A to 6D.

### Example 6: Analysis of the anticancer effects of OncAd and HDAds in vivo

### 6.1 Ectopic FaDu cell-derived model of squamous cell head and neck carcinoma

FaDu cells were transplanted subcutaneously into the right flank of NSG mice, and mice were untreated (control), or administered with:
(i) 1 x 10⁸ viral particles of Onc5/3Ad2E1Δ24 and HDAd*CD44v6BiTE,* at a ratio of Onc5/3Ad2E1Δ24 to HDAd*CD44v6BiTE* of 1:20 (referred to as "BiTE + CART" in the Figures);
(ii) 1 x 10⁸ viral particles of Onc5/3Ad2E1Δ24 and HDAd*IL-12_PD-L1,* at a ratio of Onc5/3Ad2E1Δ24 to HDAd*IL-12 PD-L1* of 1:20 (referred to as "12_PD + CART" in the Figures); or
(iii) 1 x 10⁸ viral particles of Onc5/3Ad2E1Δ24 and HDAd*Trio,* at a ratio of Onc5/3Ad2E1Δ24 to HDAd*Trio* of 1:20 (referred to as "Trio + CART" in the Figures).

Three days later, the mice were administered with 1 x 10⁶ firefly luciferase-labelled HER2-specific CAR-T cells.

Tumor volumes were measured on days 3, 7, 11, 14 and 21 after viral particle administration. The end point was established at tumor volume of >1,500 mm³. The expansion and localisation of the HER-2 specific CAR-T cells was monitored by analysis of luciferase activity by intraperitoneal injection of D-Luciferin (1.5 mg per mouse), and imaging of the mice 10 min later using an IVIS imager (Xenogen).

The results are shown in Figures 7A to 7C.

The HER2-specific CAR-T cells were shown to localise to the FaDu tumors.

### 6.2 Orthotopic FaDu cell-derived model of squamous cell head and neck carcinoma

In a first experiment, 0.5 x 10⁶ firefly luciferase-labelled FaDu cells were injected orthotopically into NSG male mice, and six days later mice were untreated, or administered with:
(i) 1 x 10⁸ viral particles of Onc5/3Ad2E1Δ24 and HDAd*IL-12_PD-L1,* at a ratio of Onc5/3Ad2E1Δ24 to HDAd*IL-12_PD-L1* of 1:20 (referred to as "12_PDL1 + CART" in the Figures); or
(ii) 1 x 10⁸ viral particles of Onc5/3Ad2E1Δ24 and HDAd*Trio,* at a ratio of Onc5/3Ad2E1Δ24 to HDAd*Trio* of 1:20 (referred to as "Trio + CART" in the Figures).

Three days later, the mice were administered with 0.2 x 10⁶ HER2-specific CAR-T cells.

Tumors were monitored by analysis of luciferase activity by intraperitoneal injection of D-Luciferin (1.5 mg per mouse), and imaging of the mice 10 min later using an IVIS imager (Xenogen).

The results are shown in Figures 8A and 8B. Treatment with the combination of Onc5/3Ad2E1Δ24 + HDAd*Trio* plus HER2-specific CAR-T cells was found to lead to earlier tumor control as compared to treatment with HER2-specific CAR-T cells only, and also as compared to treatment with the combination of Onc5/3Ad2E1Δ24 + HDAd/*L-12_PD-L1* plus HER2-specific CAR-T cells.

In a separate experiment, 0.5 x 10⁶ FaDu cells were injected orthotopically into NSG male mice, and six days later mice were untreated (control), or administered with:
(i) 1 x 10⁸ viral particles of Onc5/3Ad2E1Δ24 and HDAd*IL-12_PD-L1,* at a ratio of Onc5/3Ad2E1Δ24 to HDAd/*L-12_PD-L1* of 1:20 (referred to as "12_PDL1 + CART" in the Figures); or
(ii) 1 x 10⁸ viral particles of Onc5/3Ad2E1Δ24 and *HDAdTrio,* at a ratio of Onc5/3Ad2E1Δ24 to HDAd*Trio* of 1:20 (referred to as "Trio + CART" in the Figures).

Three days later, the mice were administered with 0.2 x 10⁶ or 1 x 10⁶ firefly luciferase-labelled HER2-specific CAR-T cells.

Tumor volumes and survival were monitored over time. The end point was established at tumor volume of >1,500 mm³. The expansion and localisation of the HER-2 specific CAR-T cells was monitored by analysis of luciferase activity by intraperitoneal injection of D-Luciferin (1.5 mg per mouse), and imaging of the mice 10 min later using an IVIS imager (Xenogen).

The results are shown in Figures 9A to 9C. The combination of Onc5/3Ad2E1Δ24 + HDAd*Trio* was found to restrict CAR-T cell expansion, and attenuate early mouse death.

The phenotype of the HER2-specific CAR-T cells was analysed by flow cytometry prior to infusion into mice, and at day 120 after being harvested from the tongue and lymph nodes of mice that had been treated according to (i) or (ii) above.

The results are shown in Figure 10. CD4+ CAR-T cells were found to persist more than CD8+ CAR-T cells.

### Example 7: Analysis of the OncAd and HDAdTrio in vivo in prostate and pancreatic cancers

### 7.1 Ectopic PC-3 cell-derived model of prostate adenocarcinoma

4 x 10⁶ PC-3 cells were injected subcutaneously into the right flank of NSG mice, and six days later mice were untreated (control), or administered with:
(i) 1 x 10⁸ viral particles of Onc5/3Ad2E1Δ24 and HDAd*Trio,* at a ratio of Onc5/3Ad2E1Δ24 to HDAd*Trio* of 1:20 (referred to as "CAdVEC" in the Figures).

Three days later, the mice were administered with 1 x 10⁶ firefly luciferase-labelled HER2-specific CAR-T cells, or were not administered with luciferase-labelled HER2-specific CAR-T cells.

Tumor volumes and survival were monitored over time. The end point was established at tumor volume of >1,500 mm³. The expansion and localisation of the HER-2 specific CAR-T cells was monitored by analysis of luciferase activity by intraperitoneal injection of D-Luciferin (1.5 mg per mouse), and imaging of the mice 10 min later using an IVIS imager (Xenogen).

The results are shown in Figures 11A to 11C. Treatment with Onc5/3Ad2E1Δ24 + HDAd*Trio* + HER-2 specific CAR-T cells (referred to in the Figures as "CAd + CART") was found to reduced tumor volumes and increase survival to a greater extent than treatment with Onc5/3Ad2E1Δ24 + HDAd*Trio* (referred to in the Figures as "CAdVEC"), or treatment with HER2-specific CAR-T cells only (referred to in the Figures as "CART").

### 7.2 Ectopic CAPAN-1 cell-derived model of pancreatic adenocarcinoma

In a separate experiment, 5 x 10⁶ CAPAN-1 cells were injected subcutaneously into the right flank of NSG mice, and six days later mice were untreated (control), or administered with:
(i) 1 x 10⁸ viral particles of Onc5/3Ad2E1Δ24 and HDAd*Trio,* at a ratio of Onc5/3Ad2E1Δ24 to HDAd*Trio* of 1:20 (referred to as "CAdVEC" in the Figures).

Three days later, the mice were administered with 1 x 10⁶ firefly luciferase-labelled PSCA-specific CAR-T cells (see Example 1.1), or were not administered with luciferase-labelled PSCA-specific CAR-T cells.

Tumor volumes and survival were monitored over time. The end point was established at tumor volume of >1,500 mm³. The expansion and localisation of the PSCA specific CAR-T cells was monitored by analysis of luciferase activity by intraperitoneal injection of D-Luciferin (1.5 mg per mouse), and imaging of the mice 10 min later using an IVIS imager (Xenogen).

The results are shown in Figures 12A to 12C. Treatment with Onc5/3Ad2E1Δ24 + HDAd*Trio* + PSCA specific CAR-T cells (referred to in the Figures as "CAd + CART") was found to reduced tumor volumes and increase survival to a greater extent than treatment with Onc5/3Ad2E1Δ24 + HDAd*Trio* (referred to in the Figures as "CAdVEC"), or treatment with PSCA-specific CAR-T cells only (referred to in the Figures as "CART").

### Example 8: Analysis of the ability of HDAd-encoded BiTEs to induce cell killing of cancer cells by Adenovirus specific T cells in vitro

Firefly luciferase-labelled FaDu cells FaDu^{cD44-/-} cells or FaDu^{HER2-/-} cells (see Example 1.5) were infected with 100 viral particles/cell of HDAd*CD19BiTE,* HDAd*HER2BiTE,* HDAd*CD44v6BiTE* or HD2*xBiTEs* (see Example 1.2) by addition of viral particles to cell culture medium of the cells in culture.

At 24 hours post-infection, AdVSTs (see Example 1.7) were added at an effector:target cell ratio of 1:10.

After 72 hours, cell killing was analysed by Luciferase assay. Briefly, cells were washed with PBS, and lysis buffer was added. Cell lysates were collected, and the residual cancer cells were determined by measuring ffLuc activity using plate reader. Readings were normalised using the readings for wells containing FaDu cells, FaDu^{CD44-/-} or FaDu^{HER2-/-} + AdVSTs without infection by HDAds (= 100% cell viability), and wells lacking cells (= 0% cell viability).

The results are shown in Figure 13. HER2-specific and CD44v6-specific BiTEs were found to be very effective at inducing cell killing of FaDu cells by AdVSTs, irrespective of adenovirus infection.

The following are numbered aspects of the invention:
1. A method of treating a cancer, comprising administering to a subject:
   (i) a virus comprising nucleic acid encoding an antigen-binding molecule comprising: (a) an antigen-binding moiety specific for an immune cell surface molecule, and (b) an antigen-binding moiety specific for a cancer cell antigen; and
   (ii) an oncolytic virus, and/or (iii) at least one cell comprising a chimeric antigen receptor (CAR) specific for a cancer cell antigen.
2. A combination of:
   (i) a virus comprising nucleic acid encoding an antigen-binding molecule comprising: (a) an antigen-binding moiety specific for an immune cell surface molecule, and (b) an antigen-binding moiety specific for a cancer cell antigen; and
   (ii) an oncolytic virus, and/or (iii) at least one cell comprising a chimeric antigen receptor (CAR) specific for a cancer cell antigen for use in a method of treating a cancer.
3. Use of:
   (i) a virus comprising nucleic acid encoding an antigen-binding molecule comprising: (a) an antigen-binding moiety specific for an immune cell surface molecule, and (b) an antigen-binding moiety specific for a cancer cell antigen; and
   (ii) an oncolytic virus, and/or (iii) at least one cell comprising a chimeric antigen receptor (CAR) specific for a cancer cell antigen, in the manufacture of a medicament for use in a method of treating a cancer.
4. The method according to aspect 1, the combination for use according to aspect 2, or the use according to aspect 3, wherein the CAR and the antigen-binding moiety capable of binding to a cancer cell antigen are specific for non-identical cancer cell antigens.
5. The method, the combination for use or the use according to any one of aspects 1 to 4, wherein the antigen-binding molecule comprises (a) a heavy chain variable region (VH) and a light chain variable region (VL) specific for an immune cell surface molecule associated via a linker sequence to (b) VH and a VL specific for a cancer cell antigen.
6. The method, the combination for use or the use according to any one of aspects 1 to 5, wherein the immune cell surface molecule is a CD3-TCR complex polypeptide.
7. The method, the combination for use or the use according to any one of aspects 1 to 6, wherein the cancer cell antigen is selected from CD44v6, HER2, CD19, PSCA, p53, CEA, GP100, EGFR, hTERT, NY-ES01, MAGE-A3, mesothelin and MUC-1.
8. The method, the combination for use or the use according to any one of aspects 1 to 7, wherein the virus comprising nucleic acid encoding an antigen-binding molecule additionally comprises nucleic acid encoding an immunomodulatory factor which is an agonist of an effector immune response or an antagonist of an immunoregulatory response.
9. The method, the combination for use or the use according to any one of aspects 1 to 8, wherein the virus comprising nucleic acid encoding an antigen-binding molecule additionally comprises nucleic acid encoding IL-12 and/or an antagonist anti-PD-L1 antibody.
10. The method, the combination for use or the use according to any one of aspects 1 to 9, wherein the virus comprising nucleic acid encoding an antigen-binding molecule is a helper-dependent adenovirus (HDAd).
11. The method, the combination for use or the use according to any one of aspects 1 to 10, wherein the virus comprising nucleic acid encoding an antigen-binding molecule comprises nucleic acid encoding an enzyme capable of catalysing conversion of a non-toxic factor to a cytotoxic form.
12. The method, the combination for use or the use according to aspect 11, wherein the enzyme is selected from: thymidine kinase, cytosine deaminase, nitroreductase, cytochrome P450, carboxypeptidase G2, purine nucleoside phosphorylase, horseradish peroxidase and carboxylesterase.
13. The method, the combination for use or the use according to any one of aspects 1 to 12, wherein the cell comprising a CAR is specific for the oncolytic virus.
14. The method, the combination for use or the use according to any one of aspects 1 to 13, wherein the cell comprising a CAR is a T cell.
15. The method, the combination for use or the use according to any one of aspects 1 to 15, wherein the oncolytic virus is an oncolytic adenovirus (OncAd).
16. The method, combination or use according to any one of aspects 1 to 15, wherein the oncolytic virus is derived from adenovirus 5 (Ad5).
17. The method, the combination for use or the use according to any one of aspects 1 to 16, wherein the oncolytic virus encodes an E1A protein which displays reduced binding to Rb protein as compared to E1A protein encoded by Ad5.
18. The method, the combination for use or the use according to any one of aspects 1 to 17, wherein the oncolytic virus encodes an E1A protein lacking the amino acid sequence LTCHEACF (SEQ ID NO: 105).
19. The method, the combination for use or the use according to any one of aspects 1 to 18, wherein the oncolytic virus encodes an E1 A protein comprising, or consisting of, the amino acid sequence SEQ ID NO:104.
20. The method, the combination for use or the use according to any one of aspects 1 to 19, wherein the oncolytic virus comprises nucleic acid having one or more binding sites for one or more transcription factors.
21. The method, the combination for use or the use according to any one of aspects 1 to 20, wherein the oncolytic virus comprises nucleic acid having one or more binding sites for STAT 1.
22. The method, combination or use according to any one of aspects 1 to 21, wherein the method of treating a cancer comprises:
   (a) isolating at least one cell from a subject;
   (b) modifying the at least one cell to express or comprise a CAR specific for a cancer cell antigen, or a nucleic acid encoding a CAR specific for a cancer cell antigen,
   (c) optionally expanding the modified at least one cell, and;
   (d) administering the modified at least one cell to a subject.
23. The method, combination or use according to any one of aspects 1 to 22, wherein the method of treating a cancer comprises:
   (a) isolating immune cells from a subject;
   (b) generating or expanding a population of immune cells specific for an oncolytic virus by a method comprising: stimulating the immune cells by culture in the presence of antigen presenting cells (APCs) presenting a peptide of the oncolytic virus, and;
   (c) administering at least one immune cell specific for the oncolytic virus to a subject.
24. The method, combination or use according to any one of aspects 1 to 23, wherein the cancer is selected from head and neck cancer, head and neck squamous cell carcinoma (HNSCC), nasopharyngeal carcinoma (NPC), oropharyngeal carcinoma (OPC), prostate carcinoma, pancreatic carcinoma, cervical carcinoma (CC), gastric carcinoma (GC), hepatocellular carcinoma (HCC), osteosarcoma (OS), ovarian cancer, colorectal cancer, breast cancer, HER2-positive breast cancer and lung cancer.
25. A combination, comprising:
   (i) a helper-dependent adenovirus (HDAd) comprising nucleic acid encoding an antigen-binding molecule comprising: (a) an antigen-binding moiety specific for an immune cell surface molecule, and (b) an antigen-binding moiety specific for a cancer cell antigen; and
   (ii) an oncolytic virus, and/or (iii) at least one cell comprising a chimeric antigen receptor (CAR) specific for a cancer cell antigen.
26. The combination according to aspect 25, wherein the antigen-binding molecule comprises (a) a single-chain variable fragment (scFv) specific for an immune cell surface molecule associated via a linker to (b) a scFv specific for a cancer cell antigen.
27. The combination according to aspect 25 or aspect 26, wherein the immune cell surface molecule is a CD3-TCR complex polypeptide.
28. The combination according to any one of aspects 25 to 27, wherein the cancer cell antigen is selected from CD44v6, CD19, HER2, PSCA, p53, CEA, GP100, EGFR, hTERT, NY-ES01, MAGE-A3, mesothelin and MUC-1.
29. The combination according to any one of aspects 25 to 28, additionally comprising nucleic acid encoding an immunomodulatory factor which is an agonist of an effector immune response or an antagonist of an immunoregulatory response.
30. The combination according to any one of aspects 25 to 29, additionally comprising nucleic acid encoding IL-12 and/or an antagonist anti-PD-L1 antibody.
31. The combination according to any one of aspects 25 to 30, additionally comprising nucleic acid encoding an enzyme capable of catalysing conversion of a non-toxic factor to a cytotoxic form.
32. The combination according to aspect 31, wherein the enzyme is selected from: thymidine kinase, cytosine deaminase, nitroreductase, cytochrome P450, carboxypeptidase G2, purine nucleoside phosphorylase, horseradish peroxidase and carboxylesterase.
33. An antigen-binding molecule, optionally isolated or man-made, comprising: (a) an antigen-binding moiety specific for an immune cell surface molecule, and (b) an antigen-binding moiety specific for a cancer cell antigen.
34. The antigen-binding molecule according to aspect 33, wherein the antigen-binding molecule comprises (a) a heavy chain variable region (VH) and a light chain variable region (VL) specific for an immune cell surface molecule associated via a linker sequence to (b) VH and a VL specific for a cancer cell antigen.
35. The antigen-binding molecule according to aspect 33 or aspect 34, wherein the immune cell surface molecule is a CD3-TCR complex polypeptide.
36. The antigen-binding molecule according to any one of aspects 33 to 35, wherein the cancer cell antigen is selected from CD44v6, HER2, CD19, PSCA, p53, CEA, GP100, EGFR, hTERT, NY-ES01, MAGE-A3, mesothelin and MUC-1.
37. A nucleic acid, or a plurality of nucleic acids, optionally isolated or man-made, encoding the components of the combination according to any one of aspects 25 to 32, or the antigen-binding molecule according to any one of aspects 33 to 36.
38. A nucleic acid, or a plurality of nucleic acids, optionally isolated or man-made, encoding: (i) an antigen-binding molecule comprising: (a) an antigen-binding moiety specific for an immune cell surface molecule, and (b) an antigen-binding moiety specific for a cancer cell antigen, (ii) IL-12, and/or an antagonist anti-PD-L1 antibody.
39. A cell comprising the components of the combination according to any one of aspects 25 to 32, the antigen-binding molecule according to any one of aspects 33 to 36, or the nucleic acid or plurality of nucleic acids according to aspect 37 or aspect 38.
40. A pharmaceutical composition comprising the components of the combination according to any one of aspects 25 to 32, the antigen-binding molecule according to any one of aspects 33 to 36, the nucleic acid or plurality of nucleic acids according to aspect 37 or aspect 38, or the cell according to aspect 39, and a pharmaceutically acceptable carrier, diluent, excipient or adjuvant.
41. A method of treating cancer comprising administering to a subject the combination according to any one of aspects 25 to 32, the antigen-binding molecule according to any one of aspects 33 to 36, the nucleic acid or plurality of nucleic acids according to aspect 37 or aspect 38, the cell according to aspect 39, or the pharmaceutical composition according to aspect 40.
42. The combination according to any one of aspects 25 to 32, the antigen-binding molecule according to any one of aspects 33 to 36, the nucleic acid or plurality of nucleic acids according to aspect 37 or aspect 38, the cell according to aspect 39, or the pharmaceutical composition according to aspect 40, for use in a method of treating a cancer.
43. Use of the combination according to any one of aspects 25 to 32, the antigen-binding molecule according to any one of aspects 33 to 36, the nucleic acid or plurality of nucleic acids according to aspect 37 or aspect 38, the cell according to aspect 39, or the pharmaceutical composition according to aspect 40, in the manufacture of a medicament for treating a cancer.
44. The method according to aspect 41 , the combination, nucleic acid or plurality of nucleic acids, cell, or pharmaceutical composition for use according to aspect 42, or the use according to aspect 43, wherein the cancer is selected from head and neck cancer, head and neck squamous cell carcinoma (HNSCC), nasopharyngeal carcinoma (NPC), prostate carcinoma, pancreatic carcinoma, cervical carcinoma (CC), oropharyngeal carcinoma (OPC), gastric carcinoma (GC), hepatocellular carcinoma (HCC), osteosarcoma (OS), ovarian cancer, colorectal cancer, breast cancer, HER2-positive breast cancer and lung cancer.
45. A kit of parts comprising a predetermined quantity of the components of the combination according to any one of aspects 25 to 32, the antigen-binding molecule according to any one of aspects 33 to 36, the nucleic acid or plurality of nucleic acids according to aspect 37 or aspect 38, the cell according to aspect 39, or the pharmaceutical composition according to aspect 40.

## Claims

1. A combination of:
(i) a helper-dependent adenovirus (HDAd) comprising nucleic acid encoding an antigen-binding molecule comprising: (a) an antigen-binding moiety specific for a CD3-TCR complex polypeptide, and (b) an antigen-binding moiety specific for a cancer cell antigen; and
(ii) an oncolytic adenovirus (OncAd),
for use in a method of treating a cancer.

2. The combination for use according to claim 1, further comprising at least one T cell comprising a chimeric antigen receptor (CAR) specific for a cancer cell antigen.

3. The combination for use according to claim 2, wherein the CAR and the antigen-binding moiety capable of binding to a cancer cell antigen are specific for non-identical cancer cell antigens.

4. The combination for use according to any one of claim 1 to 3, wherein the antigen-binding molecule comprises (a) a heavy chain variable region (VH) and a light chain variable region (VL) specific for an immune cell surface molecule associated via a linker sequence to (b) VH and a VL specific for a cancer cell antigen.

5. The combination for use according to any one of claims 1 to 4, wherein the virus comprising nucleic acid encoding an antigen-binding molecule additionally comprises nucleic acid encoding an immunomodulatory factor which is an agonist of an effector immune response or an antagonist of an immunoregulatory response, optionally wherein the virus comprising nucleic acid encoding an antigen-binding molecule additionally comprises nucleic acid encoding IL-12 and/or an antagonist anti-PD-L1 antibody.

6. The combination for use according to any one of claims 1 to 5, wherein the HDAd comprising nucleic acid encoding an antigen-binding molecule comprises nucleic acid encoding an enzyme capable of catalysing conversion of a non-toxic factor to a cytotoxic form, and wherein the enzyme is selected from: thymidine kinase, cytosine deaminase, nitroreductase, cytochrome P450, carboxypeptidase G2, purine nucleoside phosphorylase, horseradish peroxidase and carboxylesterase.

7. The combination for use according to any one of claims 1 to 6, wherein the oncolytic adenovirus is derived from adenovirus 5 (Ad5); and/or
wherein the oncolytic adenovirus encodes an E1A protein which displays reduced binding to Rb protein as compared to E1A protein encoded by Ad5; and/or
wherein the oncolytic adenovirus encodes an E1A protein lacking the amino acid sequence LTCHEACF (SEQ ID NO:105); and/or
wherein the oncolytic adenovirus encodes an E1A protein comprising, or consisting of, the amino acid sequence SEQ ID NO:104; and/or
wherein the oncolytic adenovirus comprises nucleic acid having one or more binding sites for STAT1.

8. The combination for use according to any one of claims 1 to 7, wherein the method of treating a cancer comprises:
(a) isolating at least one T cell from a subject;
(b) modifying the at least one T cell to express or comprise a CAR specific for a cancer cell antigen, or a nucleic acid encoding a CAR specific for a cancer cell antigen,
(c) optionally expanding the modified at least one T cell, and;
(d) administering the modified at least one T cell to a subject, and/or
wherein the method of treating a cancer comprises:
(a) isolating immune cells from a subject;
(b) generating or expanding a population of immune cells specific for an oncolytic virus by a method comprising: stimulating the immune cells by culture in the presence of antigen presenting cells (ARCs) presenting a peptide of the oncolytic virus, and;
(c) administering at least one immune cell specific for the oncolytic virus to a subject.

9. The combination for use according to any one of claims 1 to 8, wherein the cancer is selected from head and neck cancer, head and neck squamous cell carcinoma (HNSCC), nasopharyngeal carcinoma (NFC), oropharyngeal carcinoma (OPC), prostate carcinoma, pancreatic carcinoma, cervical carcinoma (CC), gastric carcinoma (GC), hepatocellular carcinoma (HCC), osteosarcoma (OS), ovarian cancer, colorectal cancer, breast cancer, HER2-positive breast cancer and lung cancer.

10. A combination, comprising:
(i) a helper-dependent adenovirus (HDAd) comprising nucleic acid encoding an antigen-binding molecule comprising: (a) an antigen-binding moiety specific for a CD3-TCR complex polypeptide, and (b) an antigen-binding moiety specific for a cancer cell antigen; and
(ii) an oncolytic adenovirus (OncAd).

11. The combination according to claim 10, wherein the antigen-binding molecule comprises (a) a single-chain variable fragment (scFv) specific for an immune cell surface molecule associated via a linker to (b) a scFv specific for a cancer cell antigen.

12. The combination according to claim 10 or claim 11, additionally comprising nucleic acid encoding an immunomodulatory factor which is an agonist of an effector immune response or an antagonist of an immunoregulatory response.

13. The combination according to any one of claims 10 to 12, additionally comprising nucleic acid encoding IL-12 and/or an antagonist anti-PD-L1 antibody.

14. The combination according to any one of claims 10 to 13, additionally comprising nucleic acid encoding an enzyme capable of catalysing conversion of a non-toxic factor to a cytotoxic form, wherein the enzyme is selected from: thymidine kinase, cytosine deaminase, nitroreductase, cytochrome P450, carboxypeptidase G2, purine nucleoside phosphorylase, horseradish peroxidase and carboxylesterase.

15. A pharmaceutical composition comprising the components of the combination according to any one of claims 10 to 14, and a pharmaceutically acceptable carrier, diluent, excipient or adjuvant.
